(19) Europäisches Patentamt
European Patent Office
Office européen des brevets

(11) EP 4 667 466 A1

# (12) EUROPEAN PATENT APPLICATION

(43) Date of publication:
**24.12.2025 Bulletin 2025/52**

(21) Application number: **24183691.5**

(22) Date of filing: **21.06.2024**

(51) International Patent Classification (IPC):
***C07D 471/04** (2006.01)* ***A61K 47/55** (2017.01)*
***A61P 35/00** (2006.01)* ***A61K 31/4745** (2006.01)*

(52) Cooperative Patent Classification (CPC):
**C07D 471/04; A61K 47/55; A61P 35/00**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **Dark Blue Therapeutics Ltd
Oxford, Oxfordshire OX4 4GB (GB)**

(72) Inventor: **The designation of the inventor has not yet been filed**

(74) Representative: **J A Kemp LLP
80 Turnmill Street
London EC1M 5QU (GB)**

## (54) PROTAC DEGRADERS OF MLLT1 AND/OR MLLT3

(57) The invention relates to a compound which is a Proteolysis Targeting Chimera (PROTAC) or a pharmaceutically acceptable salt thereof, wherein the PROTAC has the structure:

**M-LINK-U**

wherein U is an E3 ubiquitin ligase binding moiety, LINK is a moiety that covalently links M and U, and M is an MLLT1 and/or MLLT3 binder of formula (I):

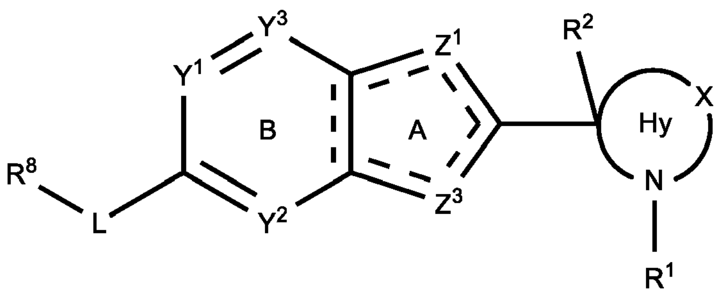

(I)

wherein:
wherein $Z^1$, $Z^2$, $Y^1$, $Y^2$, $Y^3$, $R^1$, $R^2$, $R^8$, X, L and Hy are as defined herein, and either $R^8$ is a bond to LINK, or M is bonded to LINK via a C or N atom within group $R^8$ or ring Hy such that a hydrogen atom on the C or N atom within group $R^8$ or ring Hy is replaced with a bond to LINK. The compounds are useful in the treatment of cancer.

EP 4 667 466 A1

## Description

### Field of the Invention

The present invention relates to compounds that find use in the treatment of cancer by inducing selective degradation of MLLT1 and/or MLLT3. The invention also provides such compounds *per se,* pharmaceutical compositions comprising such compounds, and methods of treating cancer by administering such compounds.

### Background

The processes controlling gene transcription are highly regulated during development and normal homeostasis. Dysregulation of gene transcription is a common driver of cancer with somatic defects in proteins that control gene transcription a frequent occurrence (Cell, 2013, 153, 17; Cell, 2017, 168, 629). Transcriptional elongation is a central step in gene transcription, carried out by the RNA polymerase II (PolII), which itself is kept under tight control by regulatory protein complexes. During development PolII is recruited to sites of the genome proximal to the transcription start site for target genes and is kept in a paused state. Such target genes are often immediate response genes such as heat-shock genes and key developmental genes. Productive elongation is initiated by the coordinated interplay of regulatory protein complexes including the super elongation complex (SEC). The SEC includes multiple proteins with diverse functions including protein phosphorylation, histone reader, histone modification and regulatory activities. Critical to the recruitment of the SEC to target genes are the histone reader proteins MLLT1 (mixed lineage leukaemia translocated to 1, also known as eleven-nineteen leukaemia, ENL) and MLLT3 (mixed lineage leukaemia translocated to 3, also known as AF-9). MLLT1 and 3 contain essential YEATS domains that bind acetylated histones. Mutations in the YEATS domains reduce loading of PolII on to SEC target genes and suppression of gene transcription. (Cell Mol Life Sci 2018, 75, 3931; Nat Rev Mol Cell Biol 2012, 13, 543).

Gain-of-function mutations in the MLLT1 Yeats domain have been causally associated with Wilm's tumor (also known as nephroblastoma) a kidney cancer most commonly observed in children (Nature 2020, 577, 121). Additionally, it has been shown that for certain acute leukaemias MLLT1 represents a critical dependency (Nature 2017, 543, 270). Such leukaemias include mixed-lineage leukaemia (MLL) rearranged leukaemia. MLL rearrangements arise from in frame fusions of the MLL gene with more than 80 different partner genes, many of which are involved in the regulation of transcription elongation including components of the SEC (Front. Pediatr. 5:4. doi: 10.3389/fped.2017.00004). MLL rearrangements are observed in approximately 10% of all acute leukaemias including a high frequency in infantile ALL where it accounts for 70-80% of all cases (Front. Pediatr. 5:4. doi: 10.3389/fped.2017.00004). Notably it is reported that patients with MLL rearranged leukaemias have an especially poor prognosis (New Engl J Med 2016, 374, 2209). Target genes for MLL-SEC complexes include potent oncogenes such as BCL-2, Myc and CDK6 along with many other genes implicated in maintaining cancer cell self-renewal, growth and survival, such as the HOX family genes and MEIS1 (Front. Pediatr. 5:4. doi: 10.3389/fped.2017.00004). Consistent with a role in the regulation of multiple oncogenes, it has been demonstrated that the SEC can play a critical role in the transcriptional addiction of both hematopoietic and solid cancers. For example, data supports potential in non-MLL rearranged leukaemia (Cancer Disc 2022, 12, 2684) and it is reported that with some breast cancer cells, growth and survival is dependent on a SEC mediated transcription of the Myc oncogene (Cell Rep. 2021 Feb 16;34(7): 108749. doi: 10.1016/j.celrep.2021.108749. PMID: 33596420; PMCID: PMC8006859).

Taken together the evidence supports MLLT1 as an attractive therapeutic target across acute leukaemias and solid cancers.

Proteolysis targeting chimeras (PROTACs) have been proposed as a small molecule-based platform technology capable of inducing proteolysis of a target protein in the body. The PROTAC is a bifunctional compound in which a molecule that binds to a disease-related target protein and an E3 ubiquitin ligase binding moiety are linked by a chemical linker. Theoretically, the PROTAC compound is capable of inducing degradation of the target protein by placing the disease-related target protein near the E3 ubiquitin ligase.

Drug discovery efforts have resulted in agents that either bind the YEATS domain to block the MLLT1 and/or MLLT3 protein to histone interaction or that result in degradation of the MLLT1 and/or MLLT3 protein (using a PROTAC approach). In all cases however, the reported activity has been weak (ACS Cent Sci 2021, 7, 815; Cancer Disc 2022, 12, 2684; Angew. Chem. Int. Ed. 2018, 57, 16302). Accordingly, there remains a high need to identify potent modulators of MLLT cell function.

### Summary of the Invention

The inventors have discovered a series of compounds that induce selective degradation of MLLT1 and/or MLLT3. Accordingly, the invention provides a compound which is a Proteolysis Targeting Chimera (PROTAC) or a pharmaceutically acceptable salt thereof, wherein the PROTAC has the structure:

**N-LINK-U** wherein U is an E3 ubiquitin ligase binding moiety, LINK is a moiety that covalently links M and U, and M is an MLLT1 and/or MLLT3 binder of formula (I):

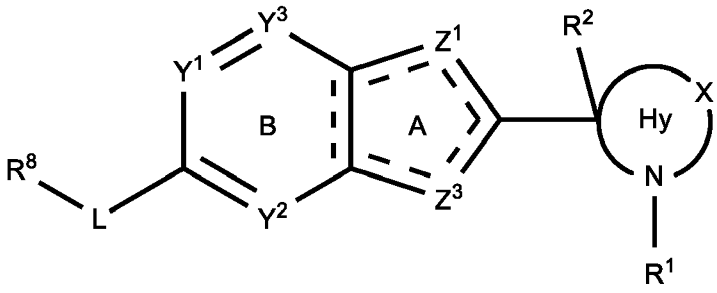

(I)

wherein:

one of $Z^1$ and $Z^3$ is -N(H)- and the other is N or -C($R^4$)-, $Y^1$ is N, $Y^2$ is N or -C($R^6$)-, and $Y^3$ is N or -C($R^5$)-;

Hy is a 4- to 7-membered heterocyclic ring containing X and at least one N atom, wherein: ring Hy is linked to ring A via a C atom within ring Hy, said C atom also being linked to $R^2$; a N atom within ring Hy is substituted by $R^1$; X is a bond, -N($R^{11}$)-, O, S, -S(O)$_2$-, -S(O)($NR^{11}$)-, or -C($R^{11}$)$_2$-; and the rest of ring Hy is unsubstituted or substituted by one or two $R^3$;

L is -C(O)N(H)-, wherein the C atom of L is bonded to $R^8$, and the N atom of L is bonded to ring B;

$R^1$ is H, $C_{1-4}$ cycloalkyl, or $C_{1-4}$ alkyl which is itself unsubstituted or substituted with one $C_{1-4}$ alkoxy or one, two or three halo;

$R^2$ is H or methyl;

each $R^3$ is independently selected from $C_{1-4}$ alkyl, $C_{1-4}$ alkoxy, phenyl, a 5- to 6-membered heteroaryl ring and halo, or (i) two $R^3$ linked to adjacent C atoms in ring Hy form, together with the C atoms to which they are attached, a $C_{5-6}$ cycloalkyl ring, or (ii) two $R^3$ linked to the same C atom in ring Hy form, together with the C atom to which they are attached, a $C_{3-6}$ cycloalkyl ring or a $C_{3-6}$ heterocycloalkyl ring;

$R^4$ and $R^6$ are independently selected from H, halo, CN, $C_{1-4}$ alkoxy, and $C_{1-4}$ alkyl which is itself unsubstituted or substituted by one, two or three halo;

$R^5$ is selected from H, halo, $C_{1-4}$ alkoxy, $C_{3-5}$ cycloalkyl and $C_{1-4}$ alkyl which is itself unsubstituted or substituted by one, two or three halo;

$R^8$ is an 8- to 10-membered heteroaryl ring, the group $R^8$ being unsubstituted or substituted by one, two or three substituents independently selected from halo, $C_{1-4}$ alkoxy, $R^{10}$, -($C_{1-4}$ alkylene)-$R^{10}$, =O, -CN, and $C_{1-4}$ alkyl which is itself unsubstituted or substituted by one or two $R^9$;

each $R^9$ is independently selected from halo and $C_{1-4}$ alkoxy;

$R^{10}$ is a group selected from a 5- to 6-membered heteroaryl ring, a 3- to 6-membered cycloalkyl ring, a 4- to 6-membered heterocyclyl ring, and a phenyl ring, the group $R^{10}$ being unsubstituted or substituted by one or two substituents independently selected from $C_{1-4}$ alkyl, $C_{1-4}$ alkoxy, and halo; and

each $R^{11}$ is independently selected from H, $C_{1-4}$ alkyl, and $C_{1-4}$ cycloalkyl;

wherein M is bonded to LINK via a C or N atom within group $R^8$ such that a hydrogen atom on the C or N atom within group $R^8$ is replaced with a bond to LINK.

In a preferred embodiment, M is of formula (II):

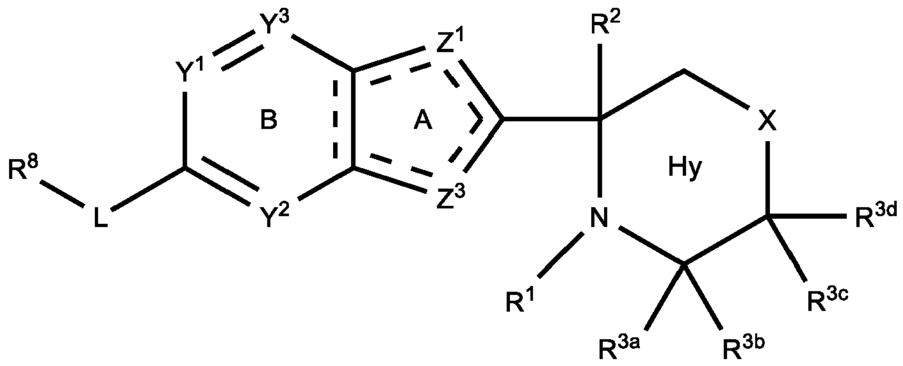

(II)

wherein:

$Z^1$, $Z^3$, $Y^1$, $Y^2$, $Y^3$, $R^1$, $R^2$, $R^8$, X and L are as defined herein;
$R^{3a}$ and $R^{3b}$ are independently selected from H, $C_{1-4}$ alkyl, phenyl, a 5- to 6-membered heteroaryl ring and $C_{1-4}$ alkoxy, or $R^{3a}$ and $R^{3b}$ form, together with the C atom to which they are attached, a $C_{3-6}$ cycloalkyl ring or a $C_{3-6}$ heterocycloalkyl ring,
$R^{3c}$ and $R^{3d}$ are independently selected from H, $C_{1-4}$ alkyl, halo, phenyl, a 5- to 6-membered heteroaryl ring and $C_{1-4}$ alkoxy, or $R^{3c}$ and $R^{3d}$ form, together with the C atom to which they are attached, a $C_{3-6}$ cycloalkyl ring or a $C_{3-6}$ heterocycloalkyl ring, or
$R^{3a}$ and $R^{3c}$ are H, and $R^{3b}$ and $R^{3d}$ form, together with the C atoms to which they are attached, a $C_{5-6}$ cycloalkyl ring, with the proviso that when X is -N($R^{11}$)-, O, S, -S(O)$_2$- or -S(O)($NR^{11}$)-, then neither $R^{3c}$ nor $R^{3d}$ are halo;
wherein at least two of $R^{3a}$, $R^{3b}$, $R^{3c}$ and $R^{3d}$ are H; and
wherein M is bonded to LINK via a C or N atom within group $R^8$ such that a hydrogen atom on the C or N atom within group $R^8$ is replaced with a bond to LINK.

In a more preferred embodiment, M is of formula (III):

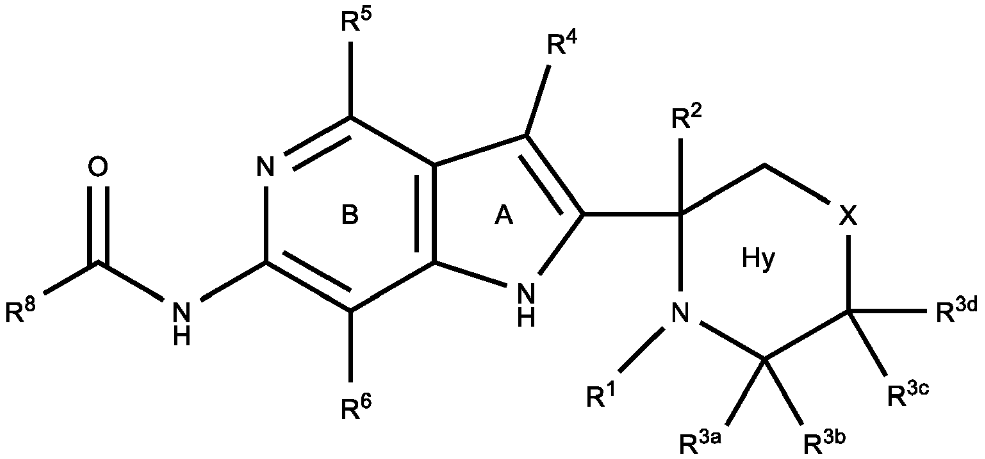

(III)

wherein:

$R^1$ is H or $C_{1-4}$ alkyl which is itself unsubstituted or substituted with one $C_{1-4}$ alkoxy or one, two or three halo, preferably with one $C_{1-4}$ alkoxy;
$R^2$ is H or methyl;
$R^{3a}$ and $R^{3b}$ are independently selected from H, $C_{1-4}$ alkyl, and $C_{1-4}$ alkoxy, or $R^{3a}$ and $R^{3b}$ form, together with the C atom to which they are attached, a $C_{3-6}$ cycloalkyl ring, $R^{3c}$ and $R^{3d}$ are independently selected from H, $C_{1-4}$ alkyl, halo and $C_{1-4}$ alkoxy, or $R^{3c}$ and $R^{3d}$ form, together with the C atom to which they are attached, a $C_{3-6}$ cycloalkyl ring, or $R^{3a}$ and $R^{3c}$ are H, and $R^{3b}$ and $R^{3d}$ form, together with the C atoms to which they are attached, a $C_{5-6}$ cycloalkyl ring, with the proviso that when X is -N(Me)- or O, then neither $R^{3c}$ nor $R^{3d}$ are halo; wherein at least two of $R^{3a}$, $R^{3b}$, $R^{3c}$ and

$R^{3d}$ are H;

X is a bond, -N(Me)-, O or $-CH_2-$;

$R^4$ is selected from H, CN, halo, $C_{1-4}$ alkoxy, and $C_{1-4}$ alkyl which is itself unsubstituted or substituted by one, two or three halo;

$R^5$ and $R^6$ are independently selected from H, halo, $C_{1-4}$ alkoxy, and $C_{1-4}$ alkyl which is itself unsubstituted or substituted by one, two or three halo;

$R^8$ is an 8- to 10-membered heteroaryl ring, the group $R^8$ being unsubstituted or substituted by one, two or three substituents independently selected from halo, $C_{1-4}$ alkoxy, $R^{10}$, -($C_{1-4}$ alkylene)-$R^{10}$, =O, -CN, and $C_{1-4}$ alkyl which is itself unsubstituted or substituted by one or two $R^9$;

each $R^9$ is independently selected from halo and $C_{1-4}$ alkoxy;

$R^{10}$ is a group selected from a 5- to 6-membered heteroaryl ring, a 3- to 6-membered cycloalkyl ring, a 4- to 6-membered heterocyclyl ring, and a phenyl ring, the group $R^{10}$ being unsubstituted or substituted by one or two substituents independently selected from $C_{1-4}$ alkyl, $C_{1-4}$ alkoxy, and halo; and

wherein M is bonded to LINK via a C or N atom within group $R^8$ such that a hydrogen atom on the C or N atom within group $R^8$ is replaced with a bond to LINK.

In a particularly preferred embodiment, M is of formula (IV):

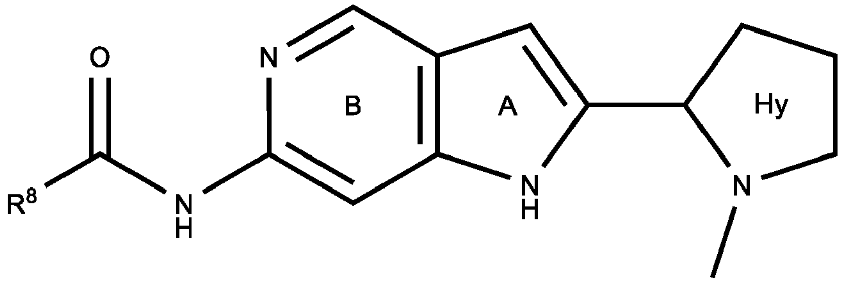

(IV)

wherein $R^8$ is an 8- to 10-membered heteroaryl ring, the group $R^8$ being unsubstituted or substituted by one, two or three substituents independently selected from halo, $C_{1-4}$ alkyl and $R^{10}$;

$R^{10}$ is a 3-membered cycloalkyl ring;

wherein M is bonded to LINK via a C or N atom within group $R^8$ such that a hydrogen atom on the C or N atom within group $R^8$ is replaced with a bond to LINK. As discussed elsewhere herein, the stereochemistry of the compounds of the invention at the ring Hy is preferably such that the bond from ring Hy to the core ring A is in the "up" position and the bond from ring Hy to $R^2$ is the down position. Thus, formula (IV) preferably has the stereochemistry depicted below:

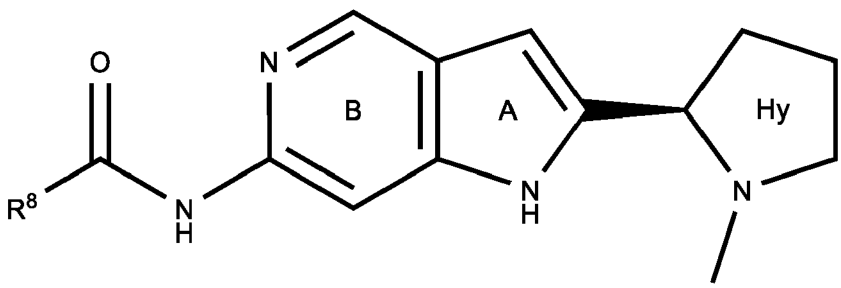

(IV)

The inventors have also surprisingly found that compounds of formula (I') are potent inhibitors of MLLT1 and MLLT3. Accordingly, the invention provides a compound which is a bicyclic compound of formula (I') or a pharmaceutically acceptable salt thereof:

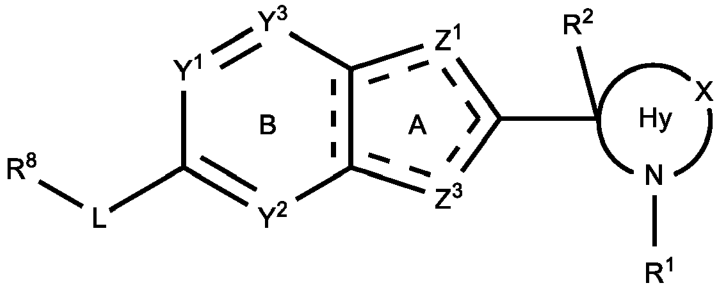

(I')

wherein:

one of $Z^1$ and $Z^3$ is -N(H)- and the other is N or -C($R^4$)-, $Y^1$ is N, $Y^2$ is N or -C($R^6$)-, and $Y^3$ is N or -C($R^5$)-;
Hy is a 4- to 7-membered heterocyclic ring containing X and at least one N atom, wherein: ring Hy is linked to ring A via a C atom within ring Hy, said C atom also being linked to $R^2$; a N atom within ring Hy is substituted by $R^1$; X is a bond, -N($R^{11}$)-, O, S, -S(O)$_2$-, -S(O)($NR^{11}$)-, or -C($R^{11}$)$_2$-; and the rest of ring Hy is unsubstituted or substituted by one or two $R^3$;
L is -C(O)N(H)-, wherein the C atom of L is bonded to $R^8$, and the N atom of L is bonded to ring B;
$R^1$ is H, $C_{1-4}$ cycloalkyl, or $C_{1-4}$ alkyl which is itself unsubstituted or substituted with one $C_{1-4}$ alkoxy or one, two or three halo;
$R^2$ is H or methyl;
each $R^3$ is independently selected from $C_{1-4}$ alkyl, $C_{1-4}$ alkoxy, phenyl, a 5- to 6-membered heteroaryl ring and halo, or (i) two $R^3$ linked to adjacent C atoms in ring Hy form, together with the C atoms to which they are attached, a $C_{5-6}$ cycloalkyl ring, or (ii) two $R^3$ linked to the same C atom in ring Hy form, together with the C atom to which they are attached, a $C_{3-6}$ cycloalkyl ring or a $C_{3-6}$ heterocycloalkyl ring;
$R^4$ and $R^6$ are independently selected from H, halo, CN, $C_{1-4}$ alkoxy, and $C_{1-4}$ alkyl which is itself unsubstituted or substituted by one, two or three halo;
$R^5$ is selected from H, halo, $C_{1-4}$ alkoxy, $C_{3-5}$ cycloalkyl and $C_{1-4}$ alkyl which is itself unsubstituted or substituted by one, two or three halo;
at least one of $R^4$, $R^5$ and $R^6$ is not H;
$R^8$ is an 8- to 10-membered heteroaryl ring, the group $R^8$ being unsubstituted or substituted by one, two or three substituents independently selected from halo, $C_{1-4}$ alkoxy, $R^{10}$, -($C_{1-4}$ alkylene)-$R^{10}$, =O, -CN, and $C_{1-4}$ alkyl which is itself unsubstituted or substituted by one or two $R^9$;
each $R^9$ is independently selected from halo and $C_{1-4}$ alkoxy;
$R^{10}$ is a group selected from a 5- to 6-membered heteroaryl ring, a 3- to 6-membered cycloalkyl ring, a 4- to 6-membered heterocyclyl ring, and a phenyl ring, the group $R^{10}$ being unsubstituted or substituted by one or two substituents independently selected from $C_{1-4}$ alkyl, $C_{1-4}$ alkoxy, and halo;
each $R^{11}$ is independently selected from H, $C_{1-4}$ alkyl, and $C_{1-4}$ cycloalkyl.

In a preferred embodiment, the compound is a bicyclic compound of formula (II') or a pharmaceutically acceptable salt thereof:

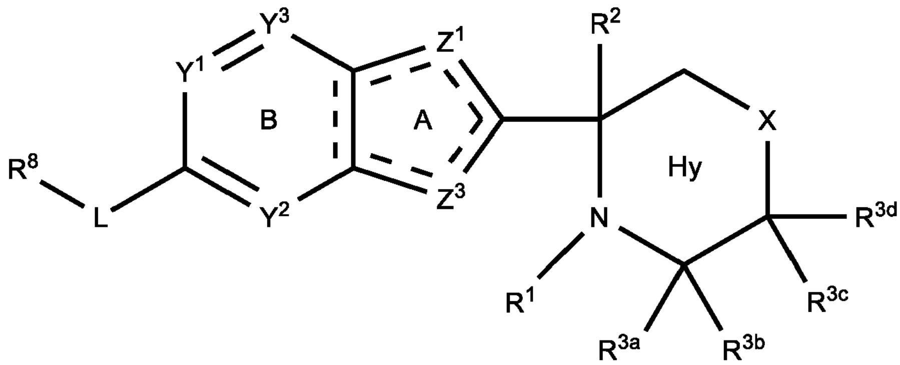

(II')

wherein:

$Z^1$, $Z^3$, $Y^1$, $Y^2$, $Y^3$, $R^1$, $R^2$, $R^8$, X and L are as defined in any one of claims 1 to 18;
$R^{3a}$ and $R^{3b}$ are independently selected from H, $C_{1-4}$ alkyl, phenyl, a 5- to 6-membered heteroaryl ring and $C_{1-4}$ alkoxy, or $R^{3a}$ and $R^{3b}$ form, together with the C atom to which they are attached, a $C_{3-6}$ cycloalkyl ring or a $C_{3-6}$ heterocycloalkyl ring,
$R^{3c}$ and $R^{3d}$ are independently selected from H, $C_{1-4}$ alkyl, halo, phenyl, a 5- to 6-membered heteroaryl ring and $C_{1-4}$ alkoxy, or $R^{3c}$ and $R^{3d}$ form, together with the C atom to which they are attached, a $C_{3-6}$ cycloalkyl ring or a $C_{3-6}$ heterocycloalkyl ring, with the proviso that when X is -N($R^{11}$)-, O, S, -S(O)$_2$- or -S(O)(N$R^{11}$)-, then neither $R^{3c}$ nor $R^{3d}$ are halo;
or
$R^{3a}$ and $R^{3c}$ are H, and $R^{3b}$ and $R^{3d}$ form, together with the C atoms to which they are attached, a $C_{5-6}$ cycloalkyl ring;
wherein at least two of $R^{3a}$, $R^{3b}$, $R^{3c}$ and $R^{3d}$ are H.

In a more preferred embodiment, the compound is a bicyclic compound of formula (III') or a pharmaceutically acceptable salt thereof:

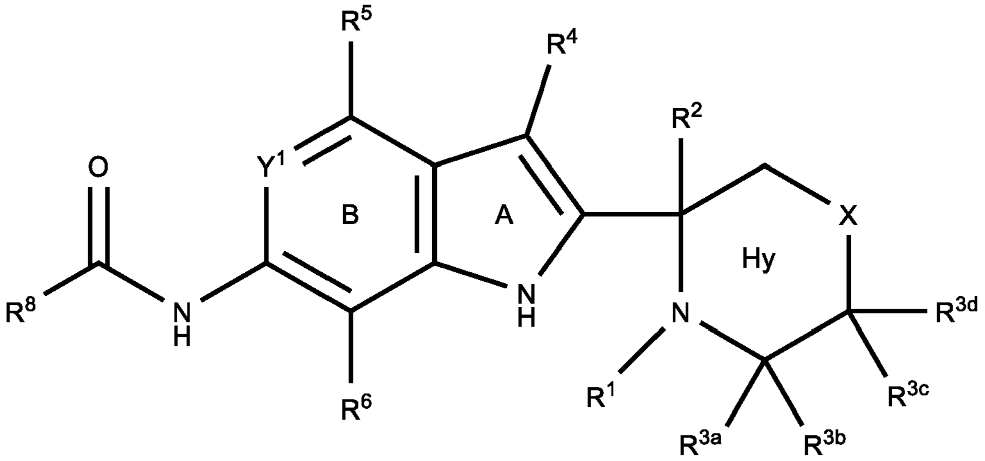

(III')

wherein:
X, $Y^1$, $R^1$, $R^2$, $R^4$, $R^5$, $R^6$, $R^{3a}$, $R^{3b}$, $R^{3c}$, $R^{3d}$ and $R^8$ are as defined herein.
The present invention also provides a compound as described herein for use in a method of treating cancer in a subject in need thereof. Also provided is a method for treating cancer in a subject, which method comprises administering to said subject an effective amount of a compound as described herein. Further provided is the use of a compound as described herein in the manufacture of a medicament for use in treating cancer in a subj ect.

## Detailed Description of the Invention

*Definitions*

As used herein, a $C_{1-4}$ alkyl group is a linear or branched alkyl group containing from 1 to 4 carbon atoms. A $C_{1-4}$ alkyl group is often a $C_{1-3}$ alkyl group. Examples of $C_{1-4}$ alkyl groups include methyl, ethyl, n-propyl, iso-propyl, n-butyl, sec-butyl, and tert-butyl. A $C_{1-3}$ alkyl group is typically a $C_{1-2}$ alkyl group. A $C_{1-2}$ alkyl group is methyl or ethyl, typically methyl. For the avoidance of doubt, where multiple alkyl groups are present, the alkyl groups may be the same or different.
As used herein, a $C_{1-4}$ alkoxy group is typically a said $C_{1-4}$ alkyl group which is joined to the rest of the molecule via an oxygen atom. Typically, a $C_{1-4}$ alkoxy group is a $C_{1-3}$ alkoxy group. Examples of $C_{1-4}$ alkoxy groups include methoxy, ethoxy, propoxy and butoxy. Typically, a $C_{1-3}$ alkoxy group is a $C_{1-2}$ alkoxy group such as a methoxy or ethoxy group. For the avoidance of doubt, where two alkoxy groups are present, the alkoxy groups may be the same or different.
As used herein, a $C_{1-30}$ alkylene group is a linear or branched divalent alkyl group that contains from 1 to 30 carbon atoms. A $C_{1-30}$ alkylene group is sometimes a $C_{1-20}$ alkylene group, and often a $C_{1-10}$ alkylene group. $C_{1-30}$ alkylene groups, $C_{1-20}$ alkylene groups and $C_{1-10}$ alkylene groups are preferably linear.
A $C_{1-30}$ alkylene group is sometimes a $C_{1-6}$ alkylene group, typically a $C_{1-4}$ alkylene group or a $C_{1-3}$ alkylene group. Examples of $C_{1-4}$ alkylene groups include methylene, ethylene, n-propylene, iso-propylene, n-butylene, sec-butylene, and tert-butylene. A $C_{1-3}$ alkylene group is typically a $C_{1-2}$ alkylene group. A $C_{1-2}$ alkylene group is methylene or ethylene, typically methylene. For the avoidance of doubt, where multiple alkylene groups are present, the alkylene groups may be the same or different.

As used herein, a $C_{2-6}$ alkenylene group is a linear or branched divalent alkenyl group containing from 2 to 6 carbon atoms and having one or more, e.g. one or two, typically one double bonds. Typically, a $C_{2-6}$ alkenylene group is a $C_{2-4}$ alkenylene group.

Examples of $C_{2-4}$ alkenylene groups include divalent ethenylene, propenylene and butenylene. For the avoidance of doubt, where multiple alkenylene groups are present, the alkenylene groups may be the same or different.

An alkyl, alkoxy, alkylene or alkenylene group as used herein may be unsubstituted or substituted. Unless otherwise stated, substituted alkyl, alkoxy, alkylene or alkenylene groups typically carry one or more, e.g. one, two or three e.g. one, or two, e.g. one substituent selected from halo, OH, and unsubstituted $C_{1-4}$ alkoxy. Preferred substituents are halo and $C_{1-4}$ alkoxy unless otherwise stated. The substituents on a substituted alkyl, alkoxy, alkylene or alkenylene group are typically themselves unsubstituted. Where more than one substituent is present, these may be the same or different.

As used herein, a halo typically refers to chlorine, fluorine, bromine or iodine, preferably chlorine, bromine or fluorine, more preferably chorine or fluorine, most preferably fluorine unless otherwise stated.

A $C_{3-8}$ cycloalkyl ring is a cyclic hydrocarbon containing from 3 to 8 carbon atoms. A cycloalkyl ring may be saturated or partially unsaturated, but is typically saturated. A $C_{3-8}$ cycloalkyl ring is typically a $C_{3-6}$ cycloalkyl ring. A partially unsaturated cycloalkyl ring is a cyclic hydrocarbon containing 1 or 2, e.g. 1 double bond. $C_{3-6}$ cycloalkyl and $C_{5-6}$ cycloalkyl rings may also be referred to herein as 3- to 6-membered cycloalkyl rings and 5- to 6-membered cycloalkyl rings respectively.

A $C_{3-6}$ cycloalkyl ring may be a saturated $C_{3-6}$ cycloalkyl ring. A $C_{3-6}$ cycloalkyl ring may be a $C_{5-6}$ cycloalkyl ring, in particular a saturated $C_{5-6}$ cycloalkyl ring. Examples of $C_{3-6}$ cycloalkyl rings are cyclopropyl, cyclobutyl cyclopentyl and cyclohexyl groups.

A $C_{3-8}$ cycloalkyl ring may be a $C_{7-8}$ cycloalkyl ring, in particular a saturated $C_{7-8}$ cycloalkyl ring. Examples of $C_{7-8}$ cycloalkyl rings are cycloheptanly, cyclooctanyl, bicyclo[2.2.1]heptanyl and bicyclo[2.2.2]octanyl groups.

A 5- to 10-membered heterocyclyl ring is a cyclic group containing from 5 to 10 atoms selected from C, O, N and S in the ring, including at least one heteroatom, and typically one or two heteroatoms unless otherwise stated. The heteroatom or heteroatoms are typically selected from O, N, and S, most typically from S and N, especially N. For example, where the heterocyclyl ring is denoted a nitrogen-containing heterocyclyl group, it contains one nitrogen atom and optionally a further heteroatom selected from O, N and S. A heterocyclyl ring may be saturated or partially unsaturated, but is typically saturated. A 5- to 10- membered partially unsaturated heterocyclyl ring is a cyclic group containing from 5 to 10 atoms selected from C, O, N and S in the ring and containing 1 or 2, e.g. 1 double bond.

A 5- to 10- membered heterocyclyl ring is typically a 4- to 7- membered heterocyclyl ring or a 5- to 6-membered ring. A 4- to 7- membered heterocyclyl ring is typically a monocyclic ring and may be a monocyclic 5- or 6- membered heterocyclyl ring. Alternatively, a 5- to 10- membered heterocyclyl ring may be a 9- or 10- membered fused bicyclic heterocyclyl ring (i.e. a fused heterobicyclic group). In some compounds described herein, a 5- to 10- membered heterocyclyl group is a 4- to 7- membered nitrogen-containing heterocyclyl ring which is unsubstituted or is substituted as described herein. Preferred 4- to 7- membered nitrogen-containing heterocyclyl rings include morpholine, pyrrolidine, piperidine and piperazine.

Examples of 5- and 6- membered saturated heterocyclyl rings include piperazine, piperidine, morpholine, diazinane and pyrrolidine. Diazinane is typically 1,4-diazinane.

Examples of 9- and 10- membered bicyclic heterocyclyl rings include azaspiro[4.4]nonane and octahydro-1H-indole. Preferably, the bicyclic heterocyclyl ring comprises 1, 2 or 3, preferably 1 or 2 nitrogen atoms.

For the avoidance of doubt, references to a heterocyclyl ring also include fused polycyclic ring systems, including for instance fused bicyclic systems in which a heterocyclyl ring is fused to an aryl group. When the heterocyclyl ring is such a fused heterocyclyl group, preferred examples are fused ring systems wherein a 5- to 6-membered heterocyclyl group is fused to a phenyl group.

As used herein, a 6- to 10-membered aryl ring is a substituted or unsubstituted, monocyclic or fused polycyclic aromatic group containing from 6 to 10 carbon atoms in the ring portion. Examples include monocyclic groups such as phenyl and fused bicyclic groups such as naphthyl and indenyl. Phenyl (benzene) is preferred.

As used herein, a 5- to 10- membered heteroaryl ring is a substituted or unsubstituted monocyclic or fused polycyclic aromatic group containing from 5 to 10 atoms in the ring portion, including at least one heteroatom, for example 1, 2 or 3 heteroatoms, typically selected from O, S and N. A heteroaryl ring is typically a 5- or 6-membered heteroaryl ring or an 8- to 10- membered heteroaryl ring. Preferably, the heteroaryl ring comprises 1, 2 or 3, preferably 1 or 2 nitrogen atoms.

Examples of 5- and 6- membered heteroaryl rings include thiazole, pyrazole, pyrimidine, triazole, 1,2,4-oxadiazole and pyrazine.

Examples of 8-, 9- and 10- membered heteroaryl rings include indazole, thieno[2,3-c]pyrazole, imidazo[4,5-b]pyridine, pyrazolo[3,4-b]pyridine, furo[2,3-c]pyridine, indole, benzoxazole, benzothiazole, [1,2,4]triazolo[4,3-a]pyridine,

thieno[2,3-d]pyrimidine, 1,2,3-benzotriazole, imidazo[1,5-a]pyridine, imidazo[1,2-a]pyrazine, oxazolo[5,4-b]pyridine, quinoline, naphthyridine, isoquinoline, quinazoline, and quinoxaline. 8-, 9- and 10- membered heteroaryl rings as used herein are typically fused bicyclic groups.

For the avoidance of doubt, references to a heteroaryl ring also include fused polycyclic ring systems, including for instance fused bicyclic systems in which a heteroaryl ring is fused to an aryl group. When the heteroaryl ring is such a fused heteroaryl group, preferred examples are fused ring systems wherein a 5- to 6-membered heteroaryl group is fused to a phenyl group. Indazole is preferred.

As used herein, a fused bicyclic group is a group comprising two cyclic moieties sharing a common bond between two atoms.

A cycloalkyl, heterocyclyl, aryl or heteroaryl ring may be unsubstituted or substituted as described herein unless otherwise stated. For example, a cycloalkyl, heterocyclyl, aryl or heteroaryl ring may be unsubstituted or substituted with 1, 2 or 3, typically 1 or 2 such as e.g. 1 substituent. Suitable substituents include halo, $C_{1-4}$ alkoxy, $R^{10}$, -($C_{1-4}$ alkylene)-$R^{10}$, =O, -CN, and $C_{1-4}$ alkyl, wherein $R^{10}$ is as defined herein). The substituents on a substituted cycloalkyl, heterocyclyl, aryl or heteroaryl ring are typically themselves unsubstituted, unless otherwise stated.

The compounds described herein comprise at least one heterocyclyl ring comprising at least one nitrogen atom. Said nitrogen atom(s) are independently selected from secondary, tertiary and quaternary nitrogen atom(s). A quaternary nitrogen atom is present when the compound comprises a quaternised derivative of one or more monocyclic groups or fused bicyclic groups. As used herein, a quaternised derivative of a moiety such as a cyclic moiety is formed by bonding an additional alkyl group to a nitrogen atom in the moiety such that the valency of the said nitrogen atom increases from 3 to 4 and the nitrogen atom is positively charged.

The compounds described herein comprise a heterocyclyl ring identified as ring Hy, which is a 4- to 7-membered heterocyclyl ring. Ring Hy contains X and at least one N atom in the ring portion, wherein X is a bond, -N($R^{11}$)-, O, S, -S(O)$_2$-, -S(O)(N$R^{11}$)-, or - C($R^{11}$)$_2$-, and $R^{11}$ is as defined herein. Ring Hy is linked to ring A (as identified in formula (I) above) via a C atom within ring Hy, said C atom also being linked to $R^2$ which is as defined herein. A N atom within ring Hy is substituted by $R^1$ which is as defined herein.

The rest of ring Hy is unsubstituted or substituted by one or two $R^3$. Each $R^3$ is independently selected from $C_{1-4}$ alkyl, $C_{1-4}$ alkoxy and halo, or (i) two $R^3$ linked to adjacent C atoms in ring Hy form, together with the C atoms to which they are attached, a $C_{5-6}$ cycloalkyl ring, or (ii) two $R^3$ linked to the same C atom in ring Hy form, together with the C atom to which they are attached, a $C_{3-6}$ cycloalkyl ring or a $C_{3-6}$ heterocycloalkyl ring, as defined herein. In option (ii), the skilled person would understand that the C atom to which the two $R^3$ are attached is a spiro atom (i.e. the common atom that connects the two rings of a spiro compound).

As used herein, the terms "monovalent" or "monovalent moiety" are used to describe a chemical group obtainable by removing a hydrogen atom from the corresponding compound. As used herein, the terms "divalent" or "divalent moiety" are used to describe a chemical group obtainable by removing a hydrogen atom from the corresponding monovalent moiety. Thus, as used herein, the terms "divalent" and "divalent moiety" are used to describe a chemical group obtainable by removing two hydrogen atoms from the corresponding compound.

It is to be understood that each individual atom present in the formulae depicted herein may be present in the form of any of its naturally occurring isotopes, with the most abundant isotope(s) being preferred. Thus, by way of example, each individual hydrogen atom present in the formulae depicted herein may be present as a 1H, 2H (deuterium) or 3H (tritium) atom, preferably 1H. Similarly, by way of example, each individual carbon atom present in the formulae depicted herein may be present as a 12C, 13C or 14C atom, preferably 12C.

In the compounds of the invention, the stereochemistry is not limited. In particular, where moiety M of formula (I) and/or moiety U contains one or more chiral centre, the compounds may be used in enantiomerically or diastereoisomerically pure form, or in the form of a mixture of isomers. Further, for the avoidance of doubt, the compounds of the invention may be used in any tautomeric form. Typically, the agent or substance described herein contains at least 50%, preferably at least 60, 75%, 90% or 95% of a compound which is enantiomerically or diasteriomerically pure. Thus, the compound is preferably substantially optically pure.

The moiety M of formula (I) typically contains at least one chiral centre at the carbon atom of ring Hy which is linked to $R^2$ and to ring A. Moiety M may be provided in the form of the R-enantiomer at said carbon atom, in the form of the S-enantiomer at said carbon atom, or in the form of a mixture of the two enantiomers. The substance or agent described herein may contain at least 50%, preferably at least 60, 75%, 90% or 95% of a compound which is enantiomerically or diasteriomerically pure at moiety M. A pure enantiomeric form of either the R- or the S-enantiomer may be preferred. In some embodiments, the R-enantiomer at said carbon atom is preferred, and in particular when $R^2$ is H then the R-enantiomer at said carbon atom is preferred. The preferred stereochemistry at said carbon atom is also depicted by the following illustrative structure of formula (I) where the bond from ring Hy to the core ring A is in the "up" position and the bond from ring Hy to $R^2$ is the down position:

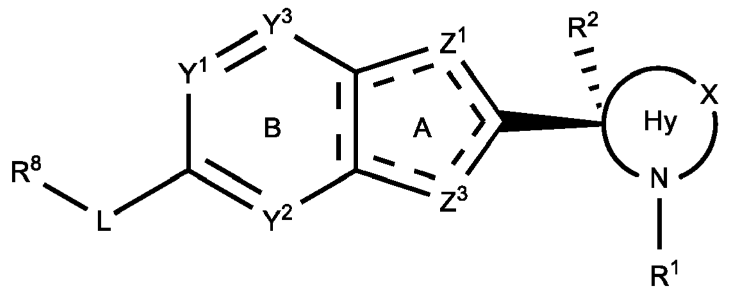

(I)

This steroechemsitry at the carbon atom of ring Hy which is linked to $R^2$ and to ring A, where the bond from ring Hy to the core ring A is in the "up" position and the bond from ring Hy to $R^2$ is the down position (as depicted above), is also preferred for the moiety M of formulae (II), (III), (IV), and for the compounds of formulae (I'), (II') and (III').

As discussed further herein, where the compounds of the invention contain a chiral centre, and in particular where moiety M and/or moiety U contains a chiral centre, the individual stereoisomers may be obtained by chiral synthesis, or by separation of stereoisomers. Stereoisomers may be separated, for example, using chiral chromatography. The individual stereoisomers may be identified using the IUPAC labels R and S as appropriate. Alternatively, the individual stereoisomers may be identified by the order in which they elute, for example the first, second, third or fourth stereisomer respectively, as obtained by separation using chiral chromatography. Where two or more stereocentres are present in a compound or moiety, a combination of IUPAC nomenclature for stereocentres where absolute stereochemistry has been defined, and rate of elution, may be used.

For example, where two or more individual stereoisomers are unidentified by IUPAC nomenclature, these stereoisomers may be identified as the first- and second-eluting isomers, as obtained by separation using chiral chromatography. Therefore, the stereoisomers at a chiral carbon atom of ring Hy which is linked to $R^2$ and to ring A may be defined as the first-eluting and second-eluting stereoisomers, as obtained by separation using chiral chromatography. Chiral chromatography is typically reverse phase HPLC or SFC.

The compounds of the present invention may exist in unsolvated as well as solvated forms with pharmaceutically acceptable solvents such as water, ethanol, and the like, and it is intended that the invention embrace both solvated and unsolvated forms.

A compound of the present invention can be converted into a pharmaceutically acceptable salt thereof, and a salt can be converted into the free compound, by conventional methods. For instance, a compound of the present invention can be contacted with a pharmaceutically acceptable acid to form a pharmaceutically acceptable salt. A pharmaceutically acceptable salt is a salt with a pharmaceutically acceptable acid or base.

Pharmaceutically acceptable acids include both inorganic acids such as hydrochloric, sulphuric, phosphoric, diphosphoric, hydrobromic or nitric acid and organic acids such as oxalic, citric, fumaric, maleic, malic, ascorbic, succinic, tartaric, benzoic, acetic, methanesulphonic, ethanesulphonic, benzenesulphonic orp-toluenesulphonic acid. Pharmaceutically acceptable bases include alkali metal (e.g. sodium or potassium) and alkali earth metal (e.g. calcium or magnesium) hydroxides and organic bases such as alkyl amines, aralkyl amines and heterocyclic amines. Hydrochloride salts and acetate salts are preferred, in particular hydrochloride salts.

*Proteolysis Targeting Chimera (PROTAC)s*

The compounds of the invention comprise a binder of MLLT1 and/or MLLT3 (M) that is covalently attached via a linker moiety (LINK) to an E3 ubiquitin ligase binding moiety (U). The inventors have surpisingly discovered that such compounds may have substantially enhanced efficacy compared with a corresponding MLLT1 and/or MLLT3 inhibitor that is not attached to an E3 ubiquitin ligase binding moiety. For example, the antiproliferative effects of the compounds may be substantially enhanced by covalent attachment to the E3 ubiquitin ligase binding moiety.

Thus, the invention provides a compound which is a Proteolysis Targeting Chimera (PROTAC) or a pharmaceutically acceptable salt thereof, wherein the PROTAC has the structure:

**M-LINK-U**

wherein U is an E3 ubiquitin ligase binding moiety, LINK is a moiety that covalently links M and U, and M is an MLLT1 and/or MLLT3 binder of formula (I) as defined herein.

In the compounds of the present invention, the MLLT 1 and/or MLLT3 binder is covalently attached to the E3 ubiquitin ligase binding moiety via the moiety labelled LINK. LINK is either a single bond or a chemical group that covalently

attaches the MLLT1 and/or MLLT3 binder to the E3 ubiquitin ligase binding moiety.

The MLLT1 and/or MLLT3 binder is attached to LINK via a C or N atom within group $R^8$ or ring Hy (as described herein) such that a hydrogen atom on the C or N atom within group $R^8$ or ring Hy is replaced with a bond to LINK. A hydrogen atom on the C or N atom within group $R^8$ or ring Hy is replaced by either a direct single bond to the E3 ubiquitin ligase binding moiety or by a chemical group that covalently attaches the MLLT1 and/or MLLT3 binder to the E3 ubiquitin ligase binding moiety. It is preferred for the MLLT1 and/or MLLT3 binder to be attached to LINK via a C or N atom within group $R^8$.

The description herein discusses the substitution of group $R^8$ and ring Hy by various chemical groups aside from LINK. For completeness, the skilled person would readily appreciate that where group $R^8$ and/or ring Hy are defined as being unsubstituted, this is intended as a reference to the absence of substituents other than a bond to LINK. Therefore, such a definition retains the option that group $R^8$ or ring Hy is attached to LINK via a C or N atom within group $R^8$ or ring Hy such that a hydrogen atom on the C or N atom within group $R^8$ or ring Hy is replaced with a bond to LINK. Furthermore, the skilled person would readily appreciate that where group $R^8$ and/or ring Hy are defined as being substituted by one or more chemical groups (suitable such chemical groups are defined herein), this is intended as a reference to substituents other than a bond to LINK. Therefore, such a definition includes group $R^8$ or ring Hy being attached to LINK via a C or N atom within group $R^8$ or ring Hy such that a hydrogen atom on the C or N atom within group $R^8$ or ring Hy is replaced with a bond to LINK. Therefore, where group $R^8$ and/or ring Hy are defined as being unsubstituted or substituted by one or more chemical groups (suitable such chemical groups are defined herein), then one of group $R^8$ or ring Hy may also be attached to LINK via a C or N atom within group $R^8$ or ring Hy such that a hydrogen atom on the C or N atom within group $R^8$ or ring Hy is replaced with a bond to LINK. A bond to LINK may optionally be positioned on a carbon atom which carries a further substituent (where chemically possible). Typically, a carbon or nitrogen atom which is bonded to LINK does not carry a further substituent.

For the avoidance of doubt, where group $R^8$ and/or ring Hy are defined as being substituted by a hydrogen atom (e.g. where $R^1$, $R^2$, $R^{11}$, $R^{3a}$, $R^{3b}$, $R^{3c}$ and $R^{3d}$ are defined as being a hydrogen atom), then a reference herein to a hydrogen atom within group $R^8$ or ring Hy being replaced with a bond to LINK, includes the option that said hydrogen atom (e.g. a hydrogen atom at $R^1$, $R^2$, $R^{11}$, $R^{3a}$, $R^{3b}$, $R^{3c}$ and $R^{3d}$) is replaced with a bond to LINK. Therefore, where $R^1$, $R^2$, $R^{11}$, $R^{3a}$, $R^{3b}$, $R^{3c}$ and $R^{3d}$ are defined as being a hydrogen atom, then said hydrogen atom may be replaced with a bond to LINK.

For the avoidance of doubt, where ring Hy or group $R^8$ is substituted, a C or N atom which is bonded to LINK may be within the aryl, heteroaryl, heterocyclyl, or cycloalkyl ring of ring Hy or group $R^8$, or it may be a C or N atom of a substituent on ring Hy or group $R^8$. Preferably, a C or N atom which is bonded to LINK is a C or N atom on the heterocyclyl ring of ring Hy (i.e. directly to the ring and not via a substituent), a C or N atom of the heteroaryl ring of group $R^8$, or the heteroaryl, cycloalkyl, heterocyclyl or phenyl ring of $R^{10}$ in the case that a group $R^{10}$ is present as, or as part of, a substituent on $R^8$.

As the skilled person would readily appreciate, methods for preparation of compounds (in which two discrete chemical entities contribute discrete biological functions to the overall compound) are very well known in the art. A hugely diverse range of techniques for covalently attaching the respective chemical entities, via a vast number of chemical linker moieties, is well established. Such is the ubiquity of these methodologies, that standard text books devoted entirely to this topic have long been available. One such textbook is "Bioconjugate Techniques" (Greg T. Hermanson, Academic Press Inc., 1996), the content of which is herein incorporated by reference in its entirety.

*MLLT1 and/or MLLT3 binder group M*

In the compounds of the invention, the MLLT1 and/or MLLT3 binder M is a monovalent moiety of formula (I) as defined herein.

In the description that follows, the skilled person will readily understand that M is a monovalent moiety that is bonded to LINK via a C or N atom within group $R^8$ or ring Hy (as described herein) such that a hydrogen atom on the C or N atom within group $R^8$ or ring Hy is replaced with a bond to LINK. Thus, in the description that follows, any of the hydrogen atoms on any of the C or N atoms within group $R^8$ or ring Hy may be removed to form the point of attachment of the monovalent moiety M to LINK.

Typically, ring Hy is a 5- to 6- membered heterocyclyl ring containing X and at least one N atom, for instance one or two N atoms. X is typically a bond, $-N(R^{11})-$, O or $-C(R^{11})_2-$. Preferably, X is a bond, -N(Me)-, O or $-CH_2-$, most preferably X is a bond. In particular, when ring Hy is a 4- or 5-membered heterocyclyl ring, X is most preferably a bond. For example, ring Hy may be selected from a pyrrolidinyl, piperidinyl, morpholinyl or diazinanyl ring. Preferably, ring Hy is a 5-membered heterocyclyl ring containing X and at least one N atom, for instance one N atom. For example, ring Hy may be a pyrrolidinyl ring.

Ring Hy is linked to ring A via a C atom within ring Hy, said C atom also being linked to $R^2$. A N atom within ring Hy is substituted by $R^1$. Typically, the N atom of ring Hy which is substituted by $R^1$ is adjacent to the C atom of ring Hy that is bonded to ring A.

A C or N atom within ring Hy may be bonded to LINK by a hydrogen atom on that C or N atom being replaced by a bond to LINK. Typically, where ring Hy is bonded to LINK, this is via a C atom on Hy. Preferably, the bond to LINK is via replacement of a hydrogen atom on a C or N within group $R^8$, i.e. the bond to LINK is preferably not via ring Hy.

Typically, $R^1$ is H, $C_{1-4}$ cycloalkyl, or $C_{1-4}$ alkyl which is itself unsubstituted or substituted with one $C_{1-4}$ alkoxy or one, two or three halo, preferably with one $C_{1-4}$ alkoxy, for example $R^1$ may be H or $C_{1-2}$ alkyl which is unsubstituted or substituted with one $C_{1-2}$ alkoxy or one, two or three halo, preferably with one $C_{1-2}$ alkoxy, or $R^1$ may be H or $C_{1-2}$ alkyl which is unsubstituted or substituted with one $C_{1-2}$ alkoxy. Preferably, $R^1$ is H, methyl, ethyl or methoxyethyl, more preferably methyl or ethyl. Most preferably, $R^1$ is methyl.

$R^2$ is H or methyl. Preferably, $R^2$ is H.

Typically, each $R^{11}$ is independently selected from H and methyl. When X is -N($R^{11}$)-or -S(O)($NR^{11}$)-, $R^{11}$ is preferably methyl. When X is -C($R^{11}$)$_2$-, optionally one $R^{11}$ is replaced with a single bond to LINK and the other $R^{11}$ is H. When X is -C($R^{11}$)$_2$-, each $R^{11}$ is preferably H.

Aside from groups $R^1$ and $R^2$, ring Hy may further be unsubstituted or substituted by one or two $R^3$. Typically, each $R^3$ is independently selected from $C_{1-4}$ alkyl, $C_{1-4}$ alkoxy and halo, or (i) two $R^3$ linked to adjacent C atoms in ring Hy form, together with the C atoms to which they are attached, a $C_{5-6}$ cycloalkyl ring, or (ii) two $R^3$ linked to the same C atom in ring Hy form, together with the C atom to which they are attached, a $C_{3-6}$ cycloalkyl ring. More typically, each $R^3$ is independently selected from methyl, ethyl, t-butyl methoxy and fluoro, or (i) two $R^3$ linked to adjacent C atoms in ring Hy form, together with the C atoms to which they are attached, a cyclohexyl ring, or (ii) two $R^3$ linked to the same C atom in ring Hy form, together with the C atom to which they are attached, a cyclopentyl ring. Preferably, ring Hy is substituted by no $R^3$ groups.

Typically, therefore, ring Hy is a 5- to 6- membered heterocyclyl ring containing X and at least one N atom, for example a pyrrolidinyl, piperidinyl, morpholinyl or diazinanyl ring; the N atom of ring Hy which is substituted by $R^1$ is adjacent to the C atom of ring Hy that is bonded to ring A; $R^1$ is H or $C_{1-2}$ alkyl which is unsubstituted or substituted with one $C_{1-2}$ alkoxy or one, two or three halo, preferably with one $C_{1-2}$ alkoxy; $R^2$ is H or methyl; X is a bond, -N($R^{11}$)-, O or -C($R^{11}$)$_2$-; $R^{11}$ is H or methyl; ring Hy is further unsubstituted or substituted by one or two $R^3$; and each $R^3$ is independently selected from $C_{1-4}$ alkyl, $C_{1-4}$ alkoxy and halo, or (i) two $R^3$ linked to adjacent C atoms in ring Hy form, together with the C atoms to which they are attached, a $C_{5-6}$ cycloalkyl ring, or (ii) two $R^3$ linked to the same C atom in ring Hy form, together with the C atom to which they are attached, a $C_{3-6}$ cycloalkyl ring.

Preferably, ring Hy is a pyrrolidinyl ring; the N atom of ring Hy which is substituted by $R^1$ is adjacent to the C atom of ring Hy that is bonded to ring A; $R^1$ is methyl or ethyl, more preferably methyl; $R^2$ is H; and ring Hy is substituted by no $R^3$ groups, i.e. ring Hy is not further substituted and carries substituent $R^1$ only. In this embodiment, ring Hy may be referred to as being an unsubstituted pyrrolidine ring, wherein the skilled person would understand that ring Hy still carries substituent $R^1$.

Typically, no more than two of $Y^1$, $Y^2$ and $Y^3$ are N. Preferably $Y^1$ is N, $Y^2$ is -C($R^6$)-, and $Y^3$ is -C($R^5$)-.

Typically, $Z^1$ is -C($R^4$)-, $Z^3$ is -N(H)-, and $Y^1$ is N. Thus, the bicyclic structure formed by rings A and B has the structure:

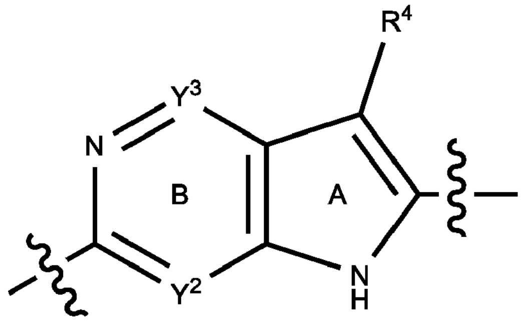

In one preferred embodiment, $Z^1$ is -C($R^4$)-, $Z^3$ is -N(H)-, $Y^1$ is N, $Y^2$ is -C($R^6$)-, and $Y^3$ is -C($R^5$)-, such that the bicyclic structure formed by rings A and B has the structure:

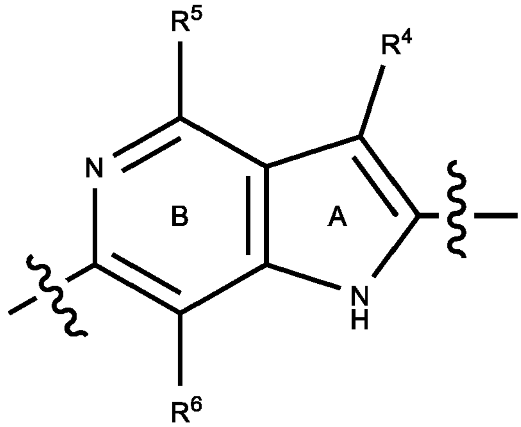

Typically, $R^4$, $R^5$ and $R^6$ are independently selected from H, CN, halo, $C_{1-4}$ alkoxy, and $C_{1-4}$ alkyl which is itself unsubstituted or substituted by one, two or three halo, with the proviso that $R^5$ and $R^6$ are not CN. Typically, $R^4$, $R^5$ and $R^6$ are independently selected from H, CN, halo (e.g. fluoro), methoxy, and methyl, with the proviso that $R^5$ and $R^6$ are not CN. Preferably, either $R^4$, $R^5$ and $R^6$ are H, or one of $R^4$, $R^5$ and $R^6$ is selected from halo, CN, $C_{1-4}$ alkoxy, and $C_{1-4}$ alkyl which is itself unsubstituted or substituted by one, two or three halo, and the rest are H, with the proviso that $R^5$ and $R^6$ are not CN. More preferably, either $R^4$, $R^5$ and $R^6$ are H, or one of $R^4$, $R^5$ and $R^6$ is selected from halo (e.g. fluoro), CN, methoxy, and methyl, and the rest are H, with the proviso that $R^5$ and $R^6$ are not CN. More preferably, $R^4$, $R^5$ and $R^6$ are H.

In one preferred embodiment, $R^5$ and $R^6$ are as defined herein and $R^4$ is selected from H, halo, CN, $C_{1-4}$ alkoxy, and $C_{1-4}$ alkyl which is itself unsubstituted or substituted by one, two or three halo. More preferably, $R^5$ and $R^6$ are as defined herein and $R^4$ is selected from H, halo, CN, $C_{1-4}$ alkyl and $CF_3$. Most preferably, $R^5$ and $R^6$ are as defined herein and $R^4$ is selected from H, F, Cl, CN, Me and $CF_3$.

In one preferred embodiment, $R^5$ is selected from H, cyclopropyl, $C_{1-4}$ alkoxy, and $C_{1-4}$ alkyl, for example from H, $C_{1-4}$ alkoxy, and $C_{1-4}$ alkyl. More preferably, $R^5$ is selected from H, cyclopropyl, methoxy and methyl, for example H, methoxy and methyl.

In one preferred embodiment, $R^6$ is H.

Typically, therefore, in formula (I):

- $R^4$ is selected from H, halo, CN, $C_{1-4}$ alkoxy, and $C_{1-4}$ alkyl which is itself unsubstituted or substituted by one, two or three halo, and $R^5$ and $R^6$ when present are H; or
- $R^5$ is selected from H, cyclopropyl, $C_{1-4}$ alkoxy, and $C_{1-4}$ alkyl, typically from H, $C_{1-4}$ alkoxy, and $C_{1-4}$ alkyl, and $R^4$ and $R^6$ when present are H; or
- $R^4$, $R^5$ and $R^6$ are H.

Preferably, in formula (I):

- $R^4$ is selected from H, F, Cl, Me, CN and $CF_3$, and $R^5$ and $R^6$ when present are H;
- $R^5$ is selected from H, cyclopropyl, methoxy and methyl, typically from H, methoxy and methyl, and $R^4$ and $R^6$ when present are H; or
- $R^4$, $R^5$ and $R^6$ are H.

L is -C(O)N(H)-, wherein the C atom of L is bonded to $R^8$, and the N atom of L is bonded to ring B.

$R^8$ is an 8- to 10-membered heteroaryl ring, the group $R^8$ being unsubstituted or substituted by one, two or three substituents independently selected from halo, $C_{1-4}$ alkoxy, $R^{10}$, -($C_{1-4}$ alkylene)-$R^{10}$, =O, -CN, and $C_{1-4}$ alkyl which is itself unsubstituted or substituted by one or two $R^9$. $R^9$ and $R^{10}$ are as defined herein.

Typically, $R^8$ is selected from indazolyl, thieno[2,3-c]pyrazolyl, imidazo[4,5-b]pyridinyl, pyrazolo[3,4-b]pyridinyl, furo[2,3-c]pyridinyl, indolyl, benzoxazolyl, benzothiazolyl, [1,2,4]triazolo[4,3-a]pyridinyl, thieno[2,3-d]pyrimidinyl, 1,2,3-benzotriazolyl, imidazo[1,5-a]pyridinyl, imidazo[1,2-a]pyrazinyl, oxazolo[5,4-b]pyridinyl, quinolinyl, naphthyridinyl, isoquinolinyl, quinazolinyl, and quinoxalinyl. Preferably, $R^8$ is a 9- to 10-membered heteroaryl ring containing one, two or three N atoms. More preferably, $R^8$ is an indazolyl group.

In some preferred embodiments, $R^8$ is a group selected from the following structures. For the avoidance of doubt, the following structures may be unsubstituted or substituted by one, two or three substituents as defined herein. [see below]

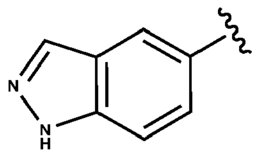
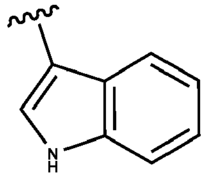
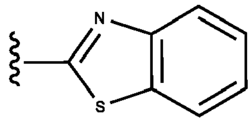
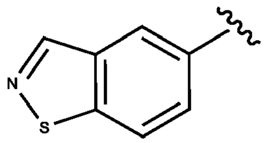
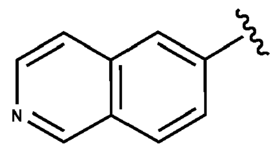
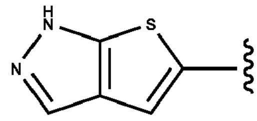
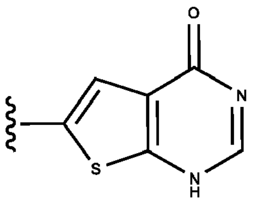
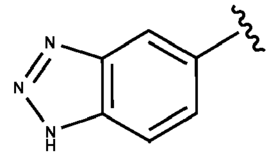
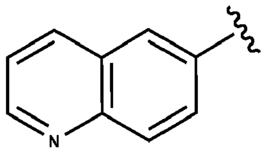
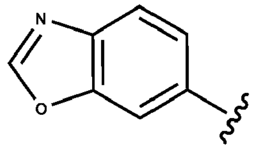
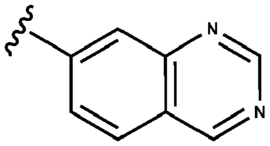
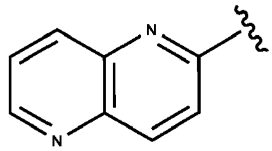
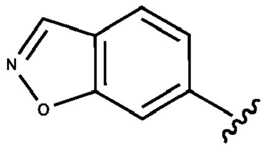
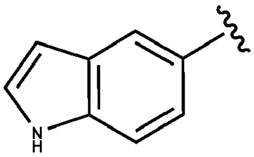
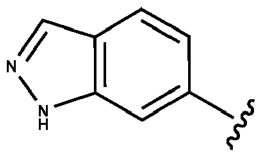
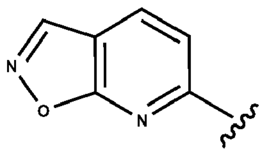
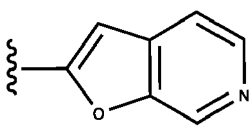
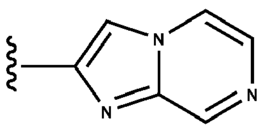
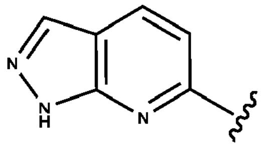
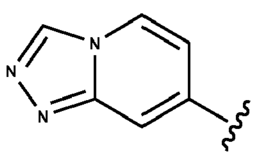
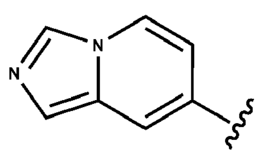
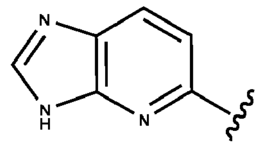
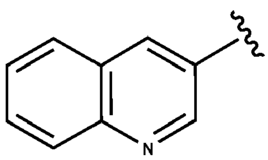
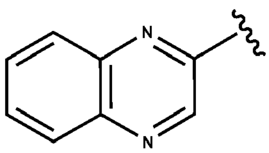
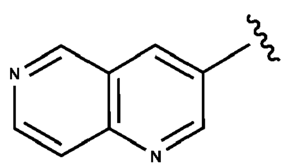
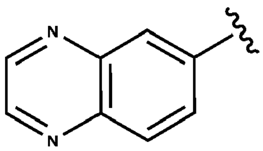
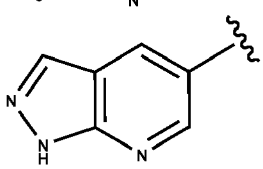

A C or N atom within group $R^8$ may be bonded to LINK by a hydrogen atom on that C or N atom being replaced by a bond to LINK. Typically, where group $R^8$ is bonded to LINK, this is via a C atom on $R^8$. Preferably, a C or N atom within group $R^8$ is bonded to LINK such that a hydrogen atom on that C or N atom is replaced by a bond to LINK. Preferably, the C or N atom that is bonded to LINK is a C or N atom of the heteroaryl ring of $R^8$ or a C or N atom of the heteroaryl, cycloalkyl, heterocyclyl or phenyl ring of $R^{10}$. More preferably, the C or N atom that is bonded to LINK is a C or N atom of the heteroaryl ring of $R^8$.

In addition to any bond to LINK (where present) $R^8$ is unsubstituted or substituted by one, two or three substituents, for instance one or two substituents. Typically, each substituent is independently selected from halo, $C_{1-4}$ alkoxy, $R^{10}$,
$-(CH_2)-R^{10}$, =O, -CN, and $C_{1-4}$ alkyl which is itself unsubstituted or substituted by one or two groups selected from selected from halo and $C_{1-2}$ alkoxy. More typically, each substituent is independently selected from fluoro, chloro, methoxy, $R^{10}$, $-(CH_2)-R^{10}$, =O, -CN, and $C_{1-2}$ alkyl which is itself unsubstituted or substituted by one or two groups selected from selected from halo (e.g. fluoro) and methoxy. Preferably, each substituent is independently selected from fluoro, $R^{10}$ and methyl. Most preferably, each substituent is independently selected from fluoro and methyl. $R^{10}$ is as defined herein.

Typically, $R^{10}$ is a group selected from a 5- to 6-membered heteroaryl ring, a 3- to 6-membered cycloalkyl ring, a 4- to 5-membered heterocyclyl ring, and a phenyl ring. The cycloalkyl, heteroaryl and heterocyclyl rings are as defined herein. Preferably, $R^{10}$ is selected from cyclopropyl, cyclobutyl, cyclopentyl, cyclohexyl, imidazolyl, oxadiazolyl, oxetanyl, phenyl, pyrazinyl, pyrazolyl, pyridazinyl, pyridinyl, pyrimidinyl, pyrrolidinyl, thiazinyl, thiazolyl and triazolyl, more preferably pyrazolyl.

Typically, in addition to any bond to LINK (where present), $R^{10}$ is unsubstituted or substituted by one or two substituents independently selected from fluoro, methyl and methoxy. Preferably, $R^{10}$ is unsubstituted.

Typically, therefore, $R^8$ is an 8- to 10-membered heteroaryl ring; the group $R^8$ is unsubstituted or substituted by one, two or three substituents, for instance one or two substituents, each independently selected from halo, $C_{1-4}$ alkoxy, $R^{10}$, $-(CH_2)-R^{10}$, =O, - CN, and $C_{1-4}$ alkyl which is itself unsubstituted or substituted by one or two groups selected from selected from halo and $C_{1-2}$ alkoxy; wherein $R^{10}$ is a group selected from a 5- to 6-membered heteroaryl ring, a 3- to 6-membered cycloalkyl ring, a 4- to 5-membered heterocyclyl ring, and a phenyl ring; and $R^{10}$ is unsubstituted or substituted by one or two substituents independently selected from fluoro, methyl and methoxy. Typically, where a C or N atom within group $R^8$ is bonded to LINK, the C or N atom that is bonded to LINK is a C or N atom of the heteroaryl ring of $R^8$ or a C or N atom of the heteroaryl, cycloalkyl, heterocyclyl or phenyl ring of $R^{10}$.

Preferably, $R^8$ is an 8- to 10-membered heteroaryl ring containing one, two or three N atoms, for example an indazolyl group; the group $R^8$ is unsubstituted or substituted by one or two substituents, each independently selected from fluoro, $R^{10}$ and methyl; $R^{10}$ is selected from cyclopropyl, cyclobutyl, cyclopentyl, cyclohexyl, imidazolyl, oxadiazolyl, oxetanyl, phenyl, pyrazinyl, pyrazolyl, pyridazinyl, pyridinyl, pyrimidinyl, pyrrolidinyl, thiazinyl, thiazolyl and triazolyl; and $R^{10}$ is unsubstituted.

Preferably, a C or N atom within group $R^8$ is bonded to LINK, wherein the C or N atom that is bonded to LINK is a C or N atom of the heteroaryl ring of $R^8$.

In a typical embodiment of formula (I), the bicyclic structure formed by rings A and B has the structure:

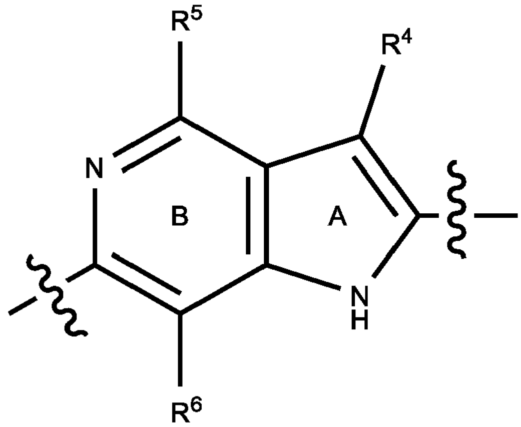

and:

ring Hy is a 5- to 6- membered heterocyclyl ring containing X and at least one N atom, for example a pyrrolidinyl, piperidinyl, morpholinyl or diazinanyl ring; wherein the N
atom of ring Hy which is substituted by $R^1$ is adjacent to the C atom of ring Hy that is bonded to ring A;

$R^1$ is H or $C_{1-2}$ alkyl which is unsubstituted or substituted with one $C_{1-2}$ alkoxy or one, two or three halo, preferably with one $C_{1-2}$ alkoxy;
$R^2$ is H or methyl;
X is a bond, -N(Me)-, O or $-CH_2-$;
ring Hy is further unsubstituted or substituted by one or two $R^3$; wherein each $R^3$ is independently selected from $C_{1-4}$ alkyl, $C_{1-4}$ alkoxy and halo, or (i) two $R^3$ linked to adjacent C atoms in ring Hy form, together with the C atoms to which they are attached, a $C_{5-6}$ cycloalkyl ring, or (ii) two $R^3$ linked to the same C atom in ring Hy form, together with the C atom to which they are attached, a $C_{3-6}$ cycloalkyl ring;
$R^4$ is selected from H, halo (e.g. fluoro or chloro), methoxy, methyl and trifluoromethyl; $R^5$ and $R^6$ are independently selected from H, halo (e.g. fluoro or chloro), methoxy, methyl and trifluoromethyl;
the C atom of L is bonded to $R^8$, and the N atom of L is bonded to ring B;
$R^8$ is an 8- to 10-membered heteroaryl ring; wherein the group $R^8$ is unsubstituted or substituted by one, two or three substituents, for instance one or two substituents, each independently selected from halo, $C_{1-4}$ alkoxy, $R^{10}$, $-(CH_2)-R^{10}$, =O, -CN, and $C_{1-4}$ alkyl which is itself unsubstituted or substituted by one or two groups selected from halo and $C_{1-2}$ alkoxy; and
$R^{10}$ is a group selected from a 5- to 6-membered heteroaryl ring, a 3- to 6-membered cycloalkyl ring, a 4- to 5-membered heterocyclyl ring, and a phenyl ring; wherein $R^{10}$ is unsubstituted or substituted by one or two substituents independently selected from fluoro, methyl and methoxy;
wherein M is bonded to LINK via a C or N atom within group $R^8$ or ring Hy such that a hydrogen atom on the C or N atom within group $R^8$ or ring Hy is replaced with a bond to LINK.

In a preferred embodiment of formula (I), the bicyclic structure formed by rings A and B has the structure:

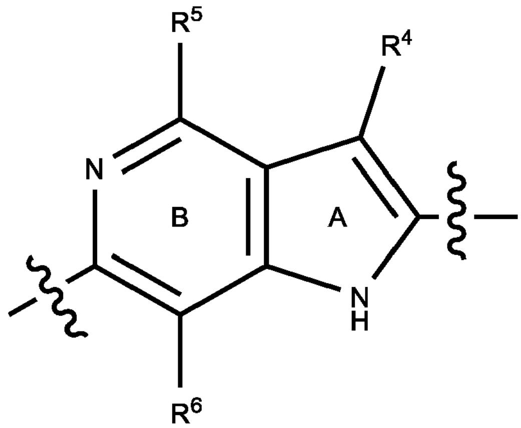

and:

ring Hy is a pyrrolidinyl ring; wherein the N atom of ring Hy which is substituted by $R^1$ is adjacent to the C atom of ring Hy that is bonded to ring A; $R^1$ is H, methyl, ethyl or
methoxyethyl, more preferably methyl; $R^2$ is H; and ring Hy is substituted by no $R^3$ groups;
either $R^4$, $R^5$ and $R^6$ are H, or one of $R^4$, $R^5$ and $R^6$ is selected from halo (e.g. fluoro or chloro), CN, methoxy, methyl and trifluoromethyl, and the rest are H, with the poviso that $R^5$ and $R^6$ are not CN;
the C atom of L is bonded to $R^8$, and the N atom of L is bonded to ring B;
$R^8$ is an 8- to 10-membered heteroaryl ring containing one, two or three N atoms, for example an indazolyl group; the group $R^8$ is unsubstituted or substituted by one or two substituents, each independently selected from fluoro, $R^{10}$ and methyl; $R^{10}$ is selected from cyclopropyl, cyclobutyl, cyclopentyl, cyclohexyl, imidazolyl, oxadiazolyl, oxetanyl, phenyl, pyrazinyl, pyrazolyl, pyridazinyl, pyridinyl, pyrimidinyl, pyrrolidinyl, thiazinyl, thiazolyl and triazolyl; and $R^{10}$ is unsubstituted;
wherein M is bonded to LINK via a C or N atom within group $R^8$ or ring Hy such that a hydrogen atom on the C or N atom within group $R^8$ or ring Hy is replaced with a bond to LINK.

In a particular embodiment of the compounds of the invention, the MLLT1 and/or MLLT3 binder group M is a monovalent moiety of formula (II):

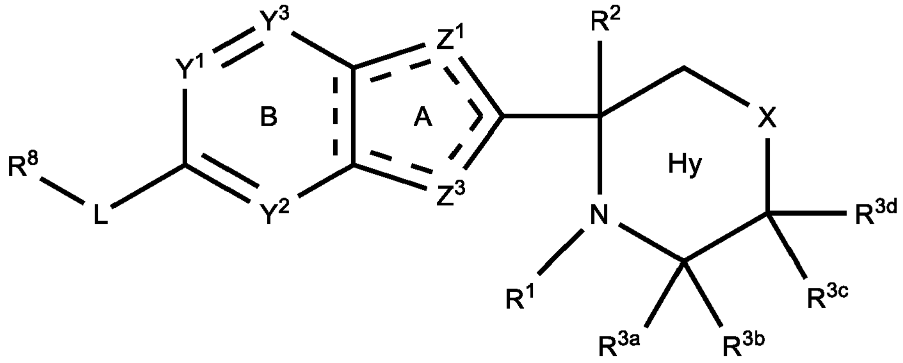

(II)

wherein:

$Z^1$, $Z^3$, $Y^1$, $Y^2$, $Y^3$, $R^1$, $R^2$, $R^8$, X and L are as defined herein;
$R^{3a}$ and $R^{3b}$ are independently selected from H, $C_{1-4}$ alkyl, phenyl, a 5- to 6-membered heteroaryl ring and $C_{1-4}$ alkoxy, or $R^{3a}$ and $R^{3b}$ form, together with the C atom to which they are attached, a $C_{3-6}$ cycloalkyl ring or a $C_{3-6}$ heterocycloalkyl ring,
$R^{3c}$ and $R^{3d}$ are independently selected from H, $C_{1-4}$ alkyl, halo, phenyl, a 5- to 6-membered heteroaryl ring and $C_{1-4}$ alkoxy, or $R^{3c}$ and $R^{3d}$ form, together with the C atom to which they are attached, a $C_{3-6}$ cycloalkyl ring or a $C_{3-6}$ heterocycloalkyl ring, or
$R^{3a}$ and $R^{3c}$ are H, and $R^{3b}$ and $R^{3d}$ form, together with the C atoms to which they are attached, a $C_{5-6}$ cycloalkyl ring, with the proviso that when X is -N($R^{11}$)-, O, S, -S(O)$_2$-or -S(O)(N$R^{11}$)-, then neither $R^{3c}$ nor $R^{3d}$ are halo;
wherein at least two of $R^{3a}$, $R^{3b}$, $R^{3c}$ and $R^{3d}$ are H; and
wherein M is bonded to LINK via a C or N atom within group $R^8$ or ring Hy such that a hydrogen atom on the C or N atom within group $R^8$ or ring Hy is replaced with a bond to LINK.

Typically, in formula (II): $R^{3a}$ and $R^{3b}$ are independently selected from H and methyl; $R^{3c}$ and $R^{3d}$ are independently selected from H, $C_{1-4}$ alkyl, halo and $C_{1-4}$ alkoxy, in particular from H, methyl, ethyl, fluoro, methoxy and t-butyl, or $R^{3c}$ and $R^{3d}$ form, together with the C atom to which they are attached, a $C_{5-6}$ cycloalkyl ring (in particular a cyclopentyl ring), with the proviso that when X is -N($R^{11}$)-, O, S, -S(O)$_2$- or - S(O)(N$R^{11}$)-, then neither $R^{3c}$ nor $R^{3d}$ are fluoro; or $R^{3a}$ and $R^{3c}$ are H, and $R^{3b}$ and $R^{3d}$ form, together with the C atoms to which they are attached, a $C_{5-6}$ cycloalkyl ring (in particular a cyclohexyl ring); wherein at least two of $R^{3a}$, $R^{3b}$, $R^{3c}$ and $R^{3d}$ are H.

Optionally, one of $R^{3a}$, $R^{3b}$, $R^{3c}$ and $R^{3d}$ is replaced with a single bond to LINK.

Preferably, in formula (II), $R^{3a}$, $R^{3b}$, $R^{3c}$ and $R^{3d}$ are all H.

In a typical embodiment of formula (II), the bicyclic structure formed by rings A and B has the structure:

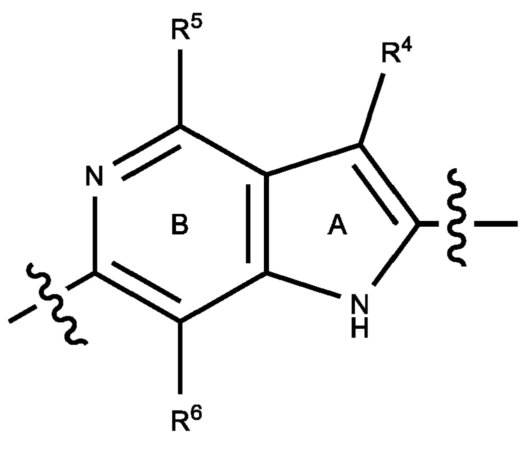

and:

$R^1$ is H or $C_{1-2}$ alkyl which is unsubstituted or substituted with one $C_{1-2}$ alkoxy or one, two or three halo, preferably with one $C_{1-2}$ alkoxy;

$R^2$ is H or methyl;
X is a bond, -N(Me)-, O or $-CH_2-$;
$R^{3a}$ and $R^{3b}$ are independently selected from H and methyl; $R^{3c}$ and $R^{3d}$ are independently selected from H, $C_{1-4}$ alkyl, halo and $C_{1-4}$ alkoxy, in particular from H, methyl, ethyl, fluoro, methoxy and t-butyl, or $R^{3c}$ and $R^{3d}$ form, together with the C atom to which they are attached, a $C_{5-6}$ cycloalkyl ring (in particular a cyclopentyl ring), with the proviso that when X is $NR^{11}$ or O, then neither $R^{3c}$ nor $R^{3d}$ are fluoro; or $R^{3a}$ and $R^{3c}$ are H, and $R^{3b}$ and $R^{3d}$ form, together with the C atoms to which they are attached, a $C_{5-6}$ cycloalkyl ring (in particular a cyclohexyl ring); wherein at least two of $R^{3a}$, $R^{3b}$, $R^{3c}$ and $R^{3d}$ are H;
$R^4$ is selected from H, CN, halo (e.g. fluoro or chloro), methoxy, methyl and trifluoromethyl;
$R^5$ and $R^6$ are independently selected from H, halo (e.g. fluoro or chloro), methoxy, methyl and trifluoromethyl;
the C atom of L is bonded to $R^8$, and the N atom of L is bonded to ring B;
$R^8$ is an 8- to 10-membered heteroaryl ring; wherein the group $R^8$ is unsubstituted or substituted by one, two or three substituents, for instance one or two substituents, each independently selected from halo, $C_{1-4}$ alkoxy, $R^{10}$, $-(CH_2)-R^{10}$, =O, -CN, and $C_{1-4}$ alkyl which is itself unsubstituted or substituted by one or two groups selected from selected from halo and $C_{1-2}$ alkoxy; and
$R^{10}$ is a group selected from a 5- to 6-membered heteroaryl ring, a 3- to 6-membered cycloalkyl ring, a 4- to 5-membered heterocyclyl ring, and a phenyl ring; wherein $R^{10}$ is unsubstituted or substituted by one or two substituents independently selected from fluoro, methyl and methoxy;
wherein M is bonded to LINK via a C or N atom within group $R^8$ or ring Hy such that a hydrogen atom on the C or N atom within group $R^8$ or ring Hy is replaced with a bond to LINK.

In a preferred embodiment of formula (II), the bicyclic structure formed by rings A and B has the structure:

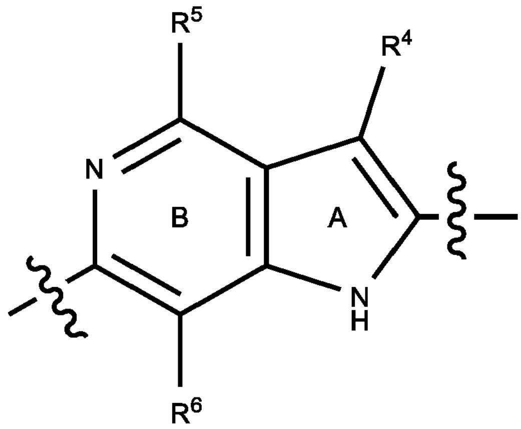

and:

X is absent;
$R^1$ is H, methyl, ethyl or methoxyethyl, more preferably methyl;
$R^2$ is H;
$R^{3a}$, $R^{3b}$ $R^{3c}$ and $R^{3d}$ are all H;
either $R^4$, $R^5$, and $R^6$, are H, or one of $R^4$, $R^5$, and $R^6$ is selected from fluoro, CN, methoxy, methyl and trifluoromethyl, and the rest are H, with the proviso that $R^5$ and $R^6$ are not H;
the C atom of L is bonded to $R^8$, and the N atom of L is bonded to ring B;
$R^8$ is an 8- to 10-membered heteroaryl ring containing one, two or three N atoms, for example an indazolyl group; the group $R^8$ is unsubstituted or substituted by one or two substituents, each independently selected from fluoro, $R^{10}$ and methyl; $R^{10}$ is selected from cyclopropyl, cyclobutyl, cyclopentyl, cyclohexyl, imidazolyl, oxadiazolyl, oxetanyl, phenyl, pyrazinyl, pyrazolyl, pyridazinyl, pyridinyl, pyrimidinyl, pyrrolidinyl, thiazinyl, thiazolyl and triazolyl; and $R^{10}$ is unsubstituted; and
wherein M is bonded to LINK via a C or N atom within group $R^8$ or ring Hy such that a hydrogen atom on the C or N atom within group $R^8$ or ring Hy is replaced with a bond to LINK.

In a particularly preferred embodiment of the compounds of the invention, the MLLT1 and/or MLLT3 binder M is a monovalent moiety of formula (III):

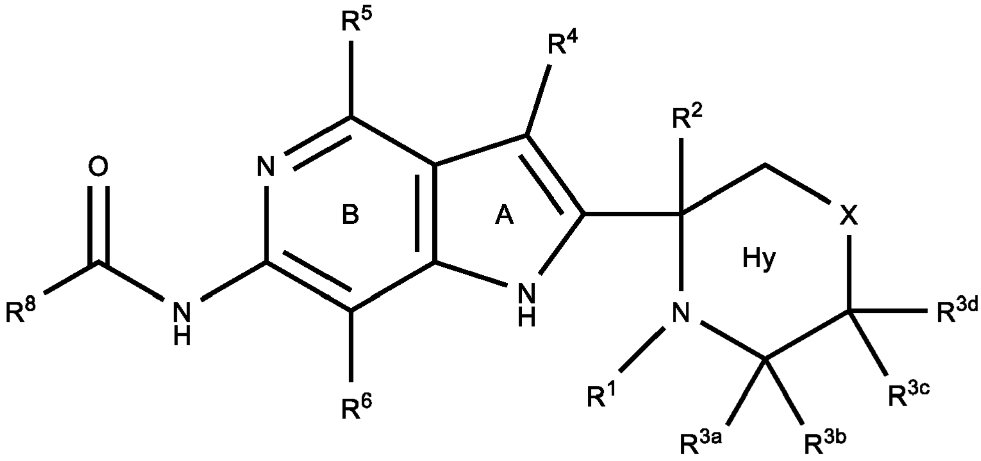

(III)

wherein $R^1$, $R^2$, $R^{3a}$, $R^{3b}$, $R^{3c}$, $R^{3d}$, X, $R^4$, $R^5$, $R^6$ and $R^8$ are as defined herein.

Typically, in the compounds of formula (III):

$R^1$ is H or $C_{1-4}$ alkyl which is itself unsubstituted or substituted with $C_{1-4}$ alkoxy;
$R^2$ is H or methyl;
$R^{3a}$ and $R^{3b}$ are independently selected from H, $C_{1-4}$ alkyl, and $C_{1-4}$ alkoxy, or $R^{3a}$ and $R^{3b}$ form, together with the C atom to which they are attached, a $C_{3-6}$ cycloalkyl ring, $R^{3c}$ and $R^{3d}$ are independently selected from H, $C_{1-4}$ alkyl, halo and $C_{1-4}$ alkoxy, or $R^{3c}$ and $R^{3d}$ form, together with the C atom to which they are attached, a $C_{3-6}$ cycloalkyl ring, or $R^{3a}$ and $R^{3c}$ are H, and $R^{3b}$ and $R^{3d}$ form, together with the C atoms to which they are attached, a $C_{5-6}$ cycloalkyl ring, with the proviso that when X is -N(Me)- or O, then neither $R^{3c}$ nor $R^{3d}$ are halo;
wherein at least two of $R^{3a}$, $R^{3b}$, $R^{3c}$ and $R^{3d}$ are H;
X is a bond, -N(Me)-, O or $-CH_2-$;
$R^4$ is selected from H, CN, halo $C_{1-4}$ alkoxy, and $C_{1-4}$ alkyl which is itself unsubstituted or substituted by one, two or three halo;
$R^5$ and $R^6$ are independently selected from H, halo $C_{1-4}$ alkoxy, and $C_{1-4}$ alkyl which is itself unsubstituted or substituted by one, two or three halo;
$R^8$ is an 8- to 10-membered heteroaryl ring, the group $R^8$ being unsubstituted or substituted by one, two or three substituents independently selected from halo, $C_{1-4}$ alkoxy, $R^{10}$, $-(C_{1-4}$ alkylene$)-R^{10}$, =O, -CN, and $C_{1-4}$ alkyl which is itself unsubstituted or substituted by one or two $R^9$;
each $R^9$ is independently selected from halo and $C_{1-4}$ alkoxy; and $R^{10}$ is a group selected from a 5- to 6-membered heteroaryl ring, a 3- to 6-membered cycloalkyl ring, a 4- to 6-membered heterocyclyl ring, and a phenyl ring, the group $R^{10}$ being unsubstituted or substituted by one or two substituents independently selected from $C_{1-4}$ alkyl, $C_{1-4}$ alkoxy, and halo;
wherein M is bonded to LINK via a C or N atom within group $R^8$ or ring Hy (as described herein) such that a hydrogen atom on the C or N atom within group $R^8$ or ring Hy is replaced with a bond to LINK.

Typically, in formula (III), $R^1$ is H, or $C_{1-4}$ alkyl which is itself unsubstituted or substituted with one $C_{1-4}$ alkoxy or one, two or three halo, preferably with one $C_{1-4}$ alkoxy, for example $R^1$ may be H or $C_{1-2}$ alkyl which is unsubstituted or substituted with one $C_{1-2}$ alkoxy or one, two or three halo, preferably with one $C_{1-2}$ alkoxy. Preferably, $R^1$ is H, methyl, ethyl or methoxyethyl. Most preferably, $R^1$ is methyl.

In formula (III), $R^2$ is H or methyl. Preferably, $R^2$ is H.

Typically, in formula (III), X is a bond, $-N(R^{11})-$, O or $-C(R^{11})_2-$. Preferably, X is a bond, -N(Me)-, O or $-CH_2-$, most preferably X is a bond.

Typically, in formula (III), each $R^{11}$ is independently selected from H and methyl. When X is $-N(R^{11})-$, $R^{11}$ is preferably methyl. When X is $-C(R^{11})_2-$, one $R^{11}$ is optionally replaced with a single bond to LINK and the other $R^{11}$ is H. When X is $-C(R^{11})_2-$, each $R^{11}$ is preferably H.

Typically, in formula (III), $R^{3a}$ and $R^{3b}$ are independently selected from H, $C_{1-4}$ alkyl, halo and $C_{1-4}$ alkoxy, in particular from H and methyl; $R^{3c}$ and $R^{3d}$ are independently selected from H, methyl, ethyl, fluoro, methoxy and t-butyl, or $R^{3c}$ and $R^{3d}$ form, together with the C atom to which they are attached, a $C_{5-6}$ cycloalkyl ring (in particular a cyclopentyl ring), with the proviso that when X is $NR^{11}$ or O, then neither $R^{3c}$ nor $R^{3d}$ are fluoro; or $R^{3a}$ and $R^{3c}$ are H, and $R^{3b}$ and $R^{3d}$ form, together with the C atoms to which they are attached, a $C_{5-6}$ cycloalkyl ring (in particular a cyclohexyl ring); wherein at

least two of $R^{3a}$, $R^{3b}$, $R^{3c}$ and $R^{3d}$ are H.

Preferably, in formula (III), $R^{3a}$, $R^{3b}$, $R^{3c}$ and $R^{3d}$ are all H.

Typically, in formula (III), $R^4$ is selected from H, CN, halo, $C_{1-4}$ alkoxy, and $C_{1-4}$ alkyl which is itself unsubstituted or substituted by one, two or three halo. Preferably, $R^4$ is selected from H, CN, F, Cl, Me and $CF_3$.

Typically, in formula (III), $R^5$ is selected from Hand $C_{1-4}$ alkyl. Preferably, $R^5$ is selected from H and methyl, more preferably H.

Typically, in formula (III), $R^6$ is H.

Typically, therefore, in formula (III):

- $R^4$ is selected from H, CN, halo, $C_{1-4}$ alkoxy, and $C_{1-4}$ alkyl which is itself unsubstituted or substituted by one, two or three halo, and $R^5$ and $R^6$ are H; or
- $R^5$ is selected from H and $C_{1-4}$ alkyl, and $R^4$ and $R^6$ when present are H; or
- $R^4$, $R^5$, and $R^6$ when present are H.

Preferably, in in formula (III):

- $R^4$ is selected from selected from H, CN, F, Cl, Me and $CF_3$, and $R^5$ and $R^6$ are H; or
- $R^5$ is selected from H and methyl, and $R^4$ and $R^6$ when present are H; or
- $R^4$, $R^5$, and $R^6$ when present are H.

In one preferred embodiment of formula (III), $R^8$ is a group selected from indazolyl, thieno[2,3-c]pyrazolyl, imidazo[4,5-b]pyridinyl, pyrazolo[3,4-b]pyridinyl, furo[2,3-c]pyridinyl, indolyl, benzoxazolyl, benzothiazolyl, [1,2,4]triazolo[4,3-a]pyridinyl, thieno[2,3-d]pyrimidinyl, 1,2,3-benzotriazolyl, imidazo[1,5-a]pyridinyl, imidazo[1,2-a]pyrazinyl, oxazolo[5,4-b]pyridinyl, quinolinyl, naphthyridinyl, isoquinolinyl, quinazolinyl, and quinoxalinyl. Preferably, $R^8$ is an 8- to 10-membered heteroaryl ring containing one, two or three N atoms. Most preferably, $R^8$ is an indazolyl group.

Typically, in formula (III), $R^8$ is a group selected from the following structures. For the avoidance of doubt, the following structures may be unsubstituted or substituted by one, two or three substituents as defined herein.

[see below]

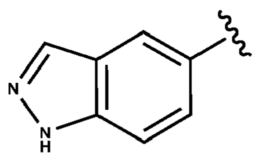
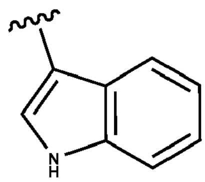
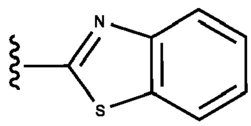

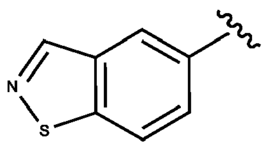
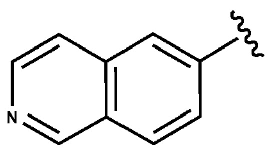
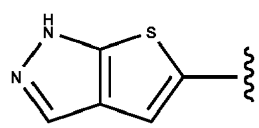

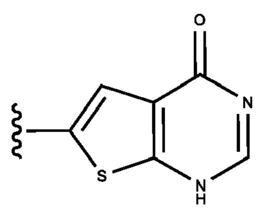
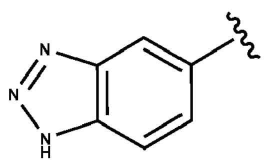
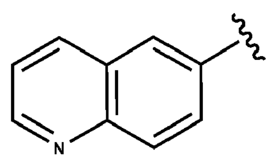

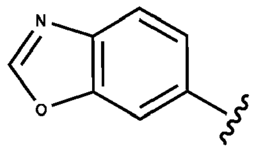
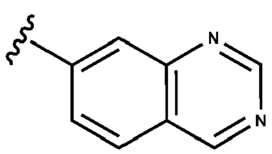
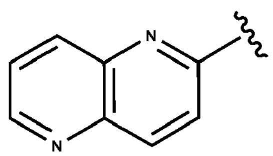

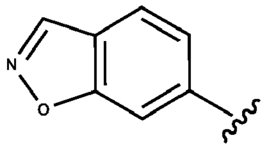
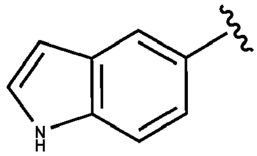
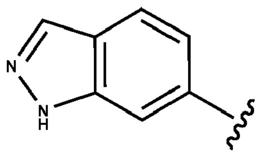
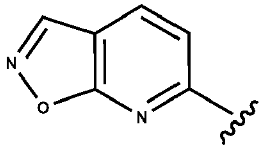

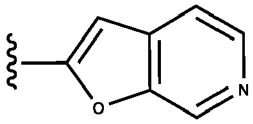
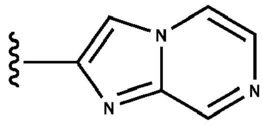
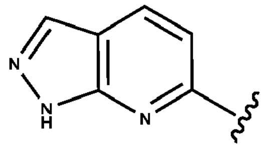

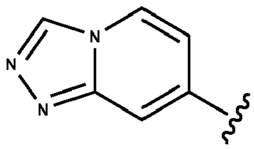
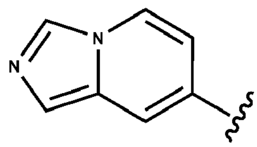
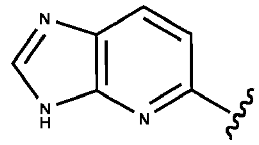

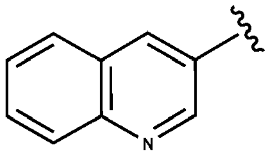
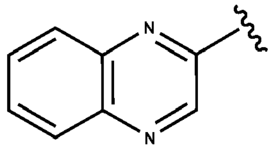
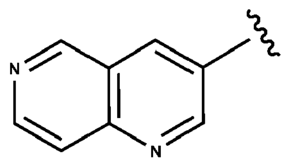

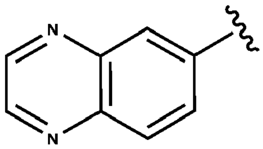
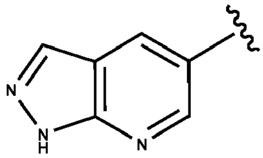

Preferably, $R^8$ is an indazolyl group.

Typically, in formula (III), $R^8$ is unsubstituted or substituted by one, two or three substituents, for instance one or two substituents, independently selected from halo, $C_{1-4}$ alkoxy, $R^{10}$, $-(CH_2)-R^{10}$, =O, -CN, and $C_{1-4}$ alkyl which is itself unsubstituted or substituted by one or two groups selected from selected from halo and $C_{1-2}$ alkoxy. More typically, each substituent is independently selected from fluoro, chloro, methoxy, $R^{10}$, $-(CH_2)-R^{10}$, =O, -CN, and $C_{1-2}$ alkyl which is itself unsubstituted or substituted by one or two groups selected from selected from halo (e.g. fluoro) and methoxy.

Preferably, each substituent is independently selected from fluoro, $R^{10}$ and methyl. Most preferably, each substituent is independently selected from fluoro and methyl. $R^{10}$ is as defined herein.

Typically, in formula (III), $R^{10}$ is a group selected from a 5- to 6-membered heteroaryl ring, a 3- to 6-membered cycloalkyl ring, a 4- to 5-membered heterocyclyl ring, and a phenyl ring. The aryl, heteroaryl and heterocyclyl rings are as defined herein. Preferably, $R^{10}$ is selected from cyclopropyl, cyclobutyl, cyclopentyl, cyclohexyl, imidazolyl, oxadiazolyl, oxetanyl, phenyl, pyrazinyl, pyrazolyl, pyridazinyl, pyridinyl, pyrimidinyl, pyrrolidinyl, thiazinyl, thiazolyl and triazolyl, more preferably pyrazolyl.

Typically, in in formula (III), $R^{10}$ is unsubstituted or substituted by one or two substituents independently selected from fluoro, methyl and methoxy. Preferably, $R^{10}$ is unsubstituted.

In typical embodiments of formula (III):

$R^1$ is H or $C_{1-2}$ alkyl which is unsubstituted or substituted with one $C_{1-2}$ alkoxy or one, two or three halo, preferably with one $C_{1-2}$ alkoxy;

$R^2$ is H or methyl;

X is a bond, -N(Me)- O or $-C(H)_2-$;

$R^{3a}$ and $R^{3b}$ are independently selected from H and methyl; $R^{3c}$ and $R^{3d}$ are independently selected from H, $C_{1-4}$ alkyl, halo and $C_{1-4}$ alkoxy, in particular from H, methyl, ethyl, fluoro, methoxy and t-butyl, or $R^{3c}$ and $R^{3d}$ form, together with the C atom to which they are attached, a $C_{5-6}$ cycloalkyl ring (in particular a cyclopentyl ring), with the proviso that when X is $NR^{11}$ or O, then neither $R^{3c}$ nor $R^{3d}$ are fluoro; or $R^{3a}$ and $R^{3c}$ are H, and $R^{3b}$ and $R^{3d}$ form, together with the C atoms to which they are attached, a $C_{5-6}$ cycloalkyl ring (in particular a cyclohexyl ring); wherein at least two of $R^{3a}$, $R^{3b}$, $R^{3c}$ and $R^{3d}$ are H;

one of the following options applies: $R^4$ is selected from H, CN, halo, $C_{1-4}$ alkoxy, and $C_{1-4}$ alkyl which is itself unsubstituted or substituted by one, two or three halo, and $R^5$ and $R^6$ are H; or $R^5$ is selected from H and $C_{1-4}$ alkyl, and $R^4$ and $R^6$ when present are H; or

$R^4$, $R^5$, and $R^6$ when present are H;

the group $R^8$ is unsubstituted or substituted by one, two or three substituents, for instance one or two substituents, each independently selected from halo, $C_{1-4}$ alkoxy, $R^{10}$, $-(CH_2)-R^{10}$, =O, -CN, and $C_{1-4}$ alkyl which is itself unsubstituted or substituted by one or two groups selected from selected from halo and $C_{1-2}$ alkoxy; and

$R^{10}$ is a group selected from a 5- to 6-membered heteroaryl ring, a 3- to 6-membered cycloalkyl ring, a 4- to 5-membered heterocyclyl ring, and a phenyl ring; wherein $R^{10}$ is unsubstituted or substituted by one or two substituents independently selected from fluoro, methyl and methoxy; and $R^8$ is a group selected from the following structures:

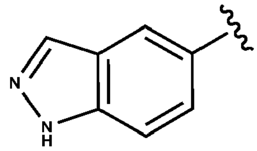
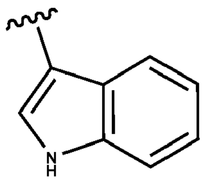
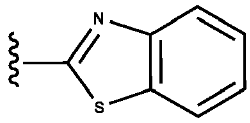

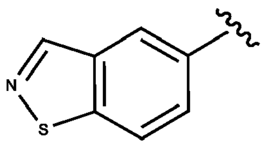
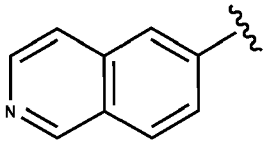
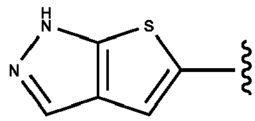

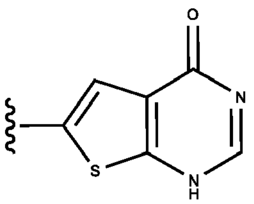
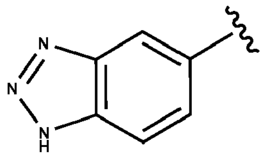
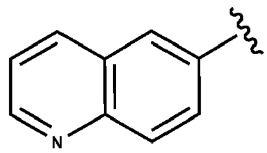

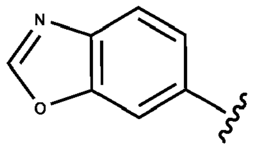
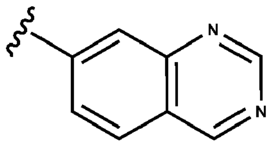
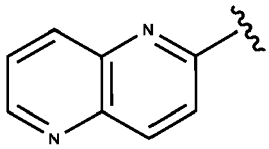

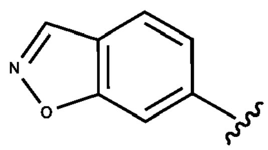
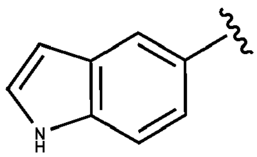
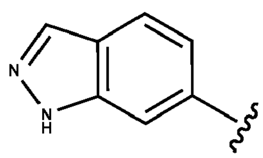
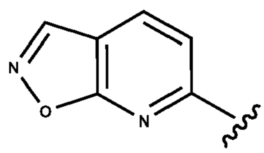

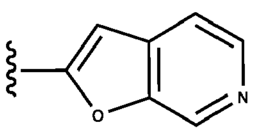
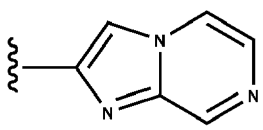
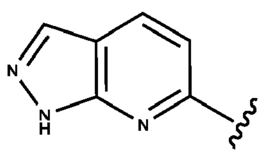

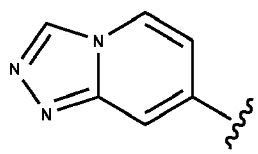
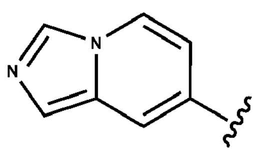
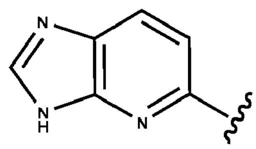

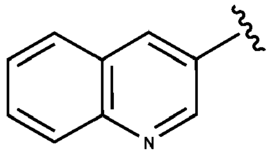
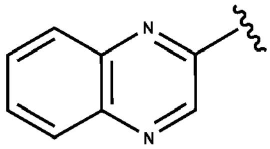
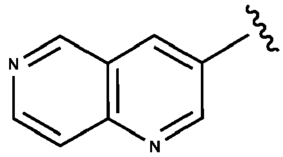
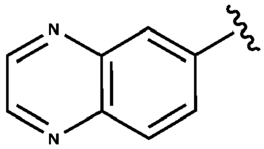
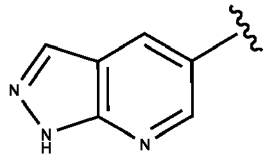

wherein M is bonded to LINK via a C or N atom within group $R^8$ or ring Hy such that a hydrogen atom on the C or N atom within group $R^8$ or ring Hy is replaced with a bond to LINK.

In preferred embodiments of formula (III),

X is absent;
$R^1$ is H, methyl, ethyl or methoxyethyl, more preferably methyl;
$R^2$ is H;
$R^{3a}$, $R^{3b}$, $R^{3c}$ and $R^{3d}$ are all H;
$R^5$ and $R^6$ are H, and $R^4$ is selected from H, CN, F, Cl, Me and $CF_3$, preferably H or Cl, more preferably H;
$R^8$ is an 8- to 10-membered heteroaryl ring containing one, two or three N atoms, for example an indazolyl group; the group $R^8$ is unsubstituted or substituted by one or two substituents, each independently selected from fluoro, $R^{10}$ and methyl;
$R^{10}$ is selected from cyclopropyl, cyclobutyl, cyclopentyl, cyclohexyl, imidazolyl, oxadiazolyl, oxetanyl, phenyl, pyrazinyl, pyrazolyl, pyridazinyl, pyridinyl, pyrimidinyl, pyrrolidinyl, thiazinyl, thiazolyl and triazolyl, preferably pyrazolyl; and $R^{10}$ is unsubstituted;
wherein M is bonded to LINK via a C or N atom within group $R^8$ or ring Hy such that a hydrogen atom on the C or N atom within group $R^8$ or ring Hy is replaced with a bond to LINK.

In a most preferred embodiment of the compounds of the invention, the MLLT1 and/or MLLT3 binder M is a monovalent moiety of formula (IV):

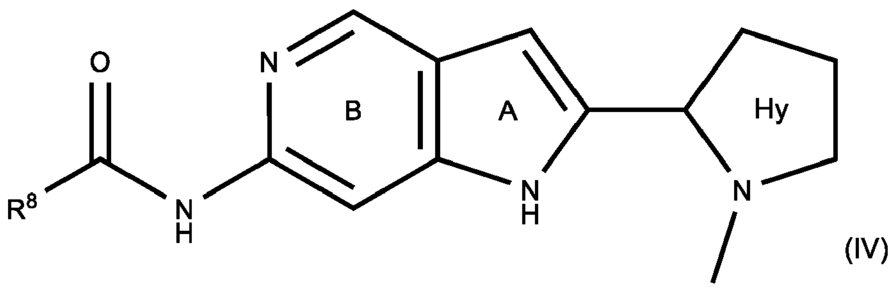

(IV)

wherein $R^8$ is as defined herein. In particular in formula (IV), the group $R^8$ may be unsubstituted or substituted by one, two or three substituents independently selected from $R^{10}$, halo and $C_{1-4}$ alkyl;
wherein M is bonded to LINK via a C or N atom within group $R^8$ such that a hydrogen atom on the C or N atom within group $R^8$ is replaced with a bond to LINK.

Typically, in formula (IV), $R^8$ is a group selected from indazolyl, thieno[2,3-c]pyrazolyl, imidazo[4,5-b]pyridinyl, pyrazolo[3,4-b]pyridinyl, furo[2,3-c]pyridinyl, indolyl, benzoxazolyl, benzothiazolyl, [1,2,4]triazolo[4,3-a]pyridinyl, thieno[2,3-d]pyrimidinyl, 1,2,3-benzotriazolyl, imidazo[1,5-a]pyridinyl, imidazo[1,2-a]pyrazinyl, oxazolo[5,4-b]pyridinyl, quinolinyl, naphthyridinyl, isoquinolinyl, quinazolinyl, and quinoxalinyl.

More typically, in formula (IV), $R^8$ is a group selected from the following structures. For the avoidance of doubt, the following structures may be unsubstituted or substituted by one, two or three substituents as defined herein. [see below]

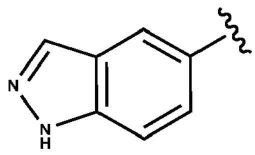
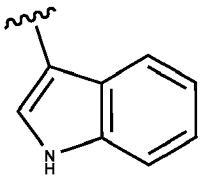
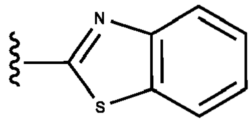

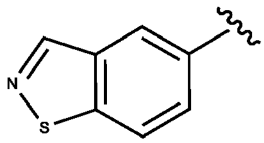
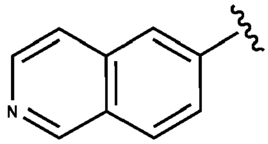
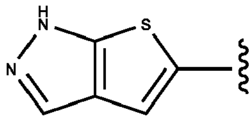

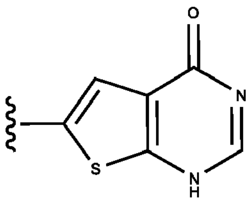
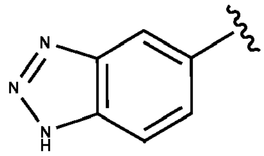
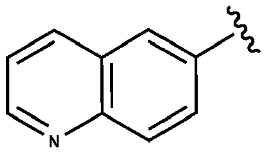

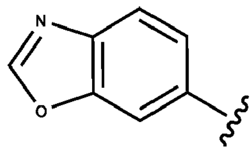
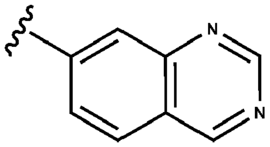
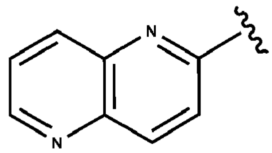

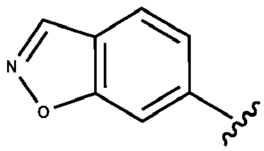
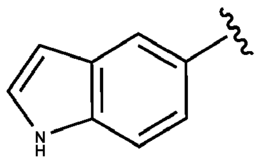
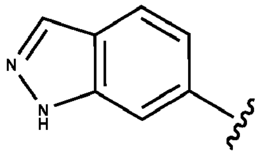
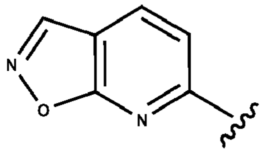

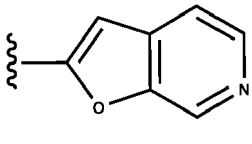
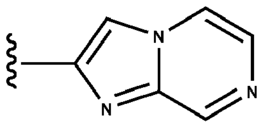
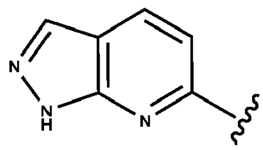

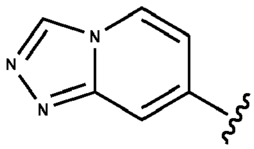
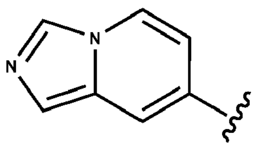
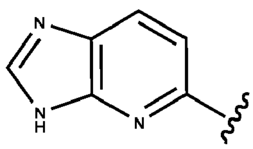

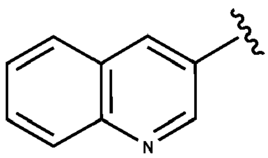
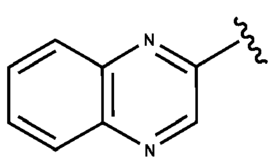
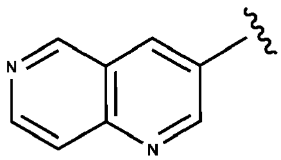

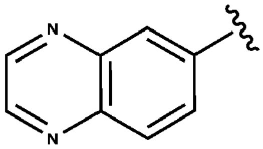
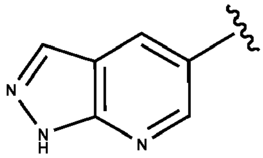

Preferably, $R^8$ is an indazolyl group.

Typically, in formula (IV), $R^8$ is unsubstituted or substituted by one, two or three substituents, for instance one or two substituents, independently selected from halo and $C_{1-4}$ alkyl. Preferably, each substituent is independently selected from fluoro and methyl.

In a particularly preferred embodiment, in formula (IV), $R^8$ is selected from one of the following structures:

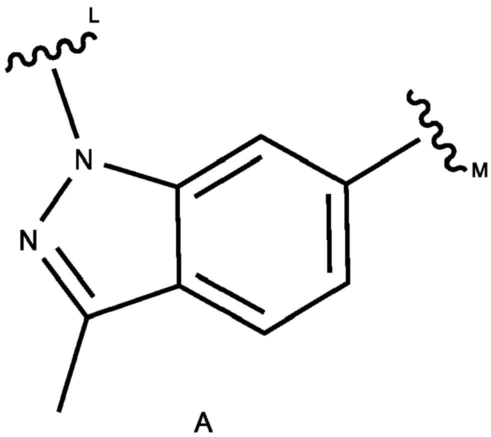

A

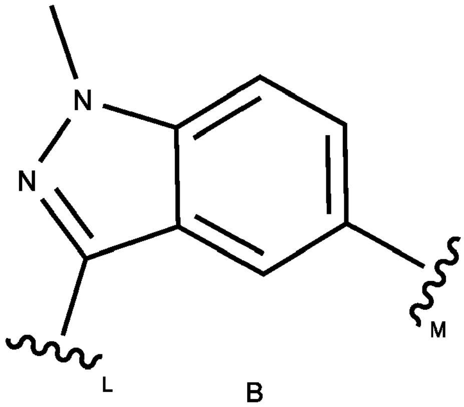

B

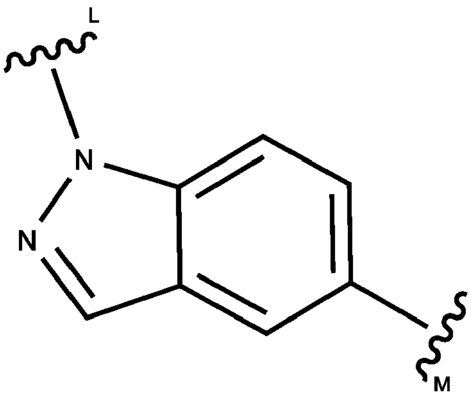

C

wherein:

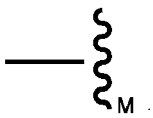

represents the point of attachment of $R^8$ to the rest of moiety M; and

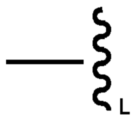

represents the point of attachment of $R^8$ to LINK.

For the avoidance of doubt, the group $R^8$ is further unsubstituted or substituted as described above. Preferably, the group $R^8$ is further unsubstituted or substituted by one or two substituents independently selected from fluorine and methyl.

For the avoidance of doubt, $R^8$ may be selected from one of structures A, B and C in any of formulae (I), (II) and (III).

*E3 ubiquitin ligase binding moieties*

The presence of the E3 ubiquitin ligase binding moiety U in the compound of the invention means that the compound is a so-called "PROTAC".

The term PROTAC is an acronym for proteolysis targeting chimera. In general, PROTACs are, as is known in the art, heterobifunctional molecules that comprise two active moieties attached covalently by a linker group. In a PROTAC, the first active moiety (the MLLT1 and/or MLLT3 binder M in the compound of the present invention) binds to a target protein that is intended for degradation (target proteins for the compound of the present invention are MLLT1 and/or MLLT3). The second active moiety (U in the compound of the present invention) is capable of binding to an E3 ubiquitin ligase, thereby inducing selective intracellular proteolysis. Recruitment of the E3 ligase to the target protein results in ubiquitination and subsequent degradation of the target protein by the proteasome.

E3 ubiquitin ligase moities are known in the art. Substantially any such moiety can be used. The sole limitation on the moiety is that it be capable of binding to an E3 ubiquitin ligase. Those skilled in the art would appreciate that entirely routine laboratory methods can be used to determine whether a given substance binds to an E3 ubiquitin ligase (including but not limited to any of those disclosed specifically herein). Thus, those skilled in the art would have no difficulty in identifying E3 ubiquitin ligase moieties, nor in establishing whether any existing chemical moiety falls within the bounds of this definition. In certain embodiments, the moiety shows activity or binds to the E3 ubiquitin ligase with an $IC_{50}$ of less than about 200 mM. The $IC_{50}$ can be determined according to any method known in the art, e.g., a fluorescent polarization assay.

Merely by way of example of the extensive disclosure in the field concerning PROTACs, and hence E3 ubiquitin ligase binding moieties, reference can be made to Gu et al. (BioEssays 2018, 40, 1700247), Sun et al. (Signal Transduction and Targeted Therapy (2019) 4:64), WO 2020/041331, and WO 2019/140003, the contents of all of which are herein incorporated by reference in their entireties. Any of the numerous E3 ubiquitin ligase binding moieties disclosed in these documents can be used as an E3 ubiquitin ligase binding moiety in the compounds of the present invention. For the avoidance of doubt, Gu et al. refer to such moieties as ligands to recruit E3 ubiquitin ligase, Sun et al. refer to such moieties as E3 ubiquitin ligase (E3) recruiting ligands, in WO 2020/041331 such moieties are referred to as a "ULM" or (small molecule) E3 ubiquitin ligase binding moiety (that binds an E3 ubiquitin ligase) (and noting that the term "ULM" includes each of "ILM", "CLM", "VLM" and "MLM", any of which can be used in the present compounds), and in WO 2019/140003 such moieties (labelled "B" in WO 2019/140003's formula (I)) are referred to as a ubiquitin ligase ligand/binder.

Further examples of documents disclosing suitable moieties that can be used in the compounds of the present invention are WO2013/106643, US2016/0045607, WO2014187777, US20140356322 and US 9,249, 153, US2016/0058872, US2015/0291562 and Winter et al (Science, June 19, 2015, p. 1376), the contents of all of which are herein incorporated by reference in their entireties. Thalidomide, lenalidomide, pomalidomide and analogs thereof are still further examples of suitable moieties.

Examples of E3 ubiquitin ligases include von Hippel-Lindau (VHL) and cereblon (CRBN).

Preferably the E3 ubiquitin ligase binding moiety (U) is capable of binding to CRBN or VHL. Such moieties are referred to herein as CRBN or VHL E3 ubiquitin ligase binding moieties.

In one embodiment, U is a CRBN E3 ubiquitin ligase binding moiety. In another embodiment, U is a VHL E3 ubiquitin ligase binding moiety.

In some embodiments, the E3 ubiquitin ligase binding moiety (U) is:

(a) a CRBN E3 ubiquitin ligase binding moiety of formula (U1):

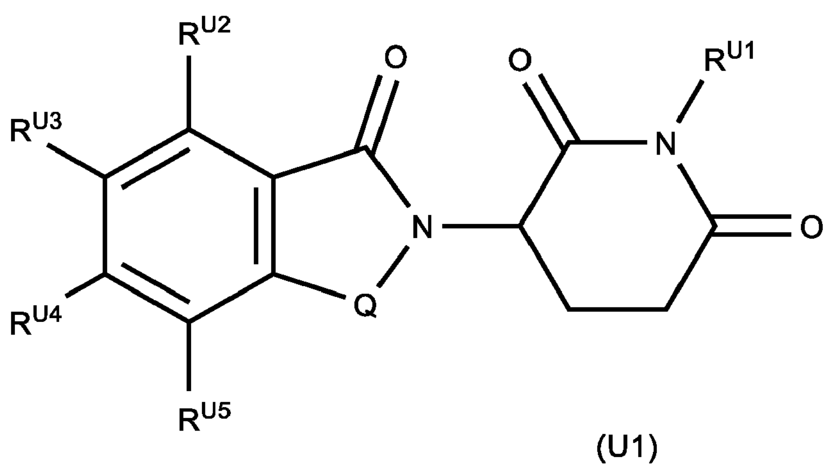

(U1)

wherein:

$R^{U1}$ is H;
Q is selected from -CH($R^{U6}$)-, -N($R^{U6}$)-, -O-, -C(O)-, -NH-CH($R^{U6}$)-, -N=C($R^{U6}$)-, or -N=N-;
$R^{U6}$ is H or $C_{1-4}$ alkyl;
$R^{U2}$ and $R^{U5}$ are each independently selected from H, halogen, $C_{1-4}$ alkyl, $NH_2$, $NO_2$, OH, COOH, CN, $CF_3$, and a single bond to LINK;
$R^{U3}$ and $R^{U4}$ are each independently selected from H, halogen, $C_{1-4}$ alkyl, $NH_2$, $NO_2$, OH, COOH, CN, $CF_3$, and a single bond to LINK;
wherein one and only one of $R^{U2}$, $R^{U3}$, $R^{U4}$ and $R^{U5}$ is a single bond to LINK;

(b) a CRBN E3 ubiquitin ligase binding moiety of formula (U4):

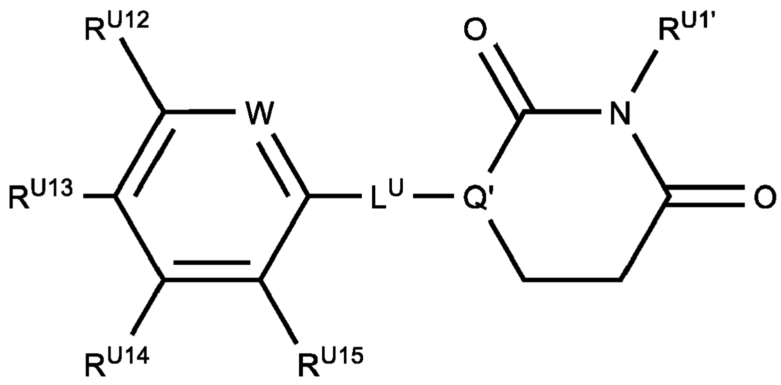

(U4)

wherein:

$R^{U1'}$ is H;
W is N or $CR^{U16}$.,
Q' is N and $L^U$ is a single bond, or Q' is CH and $L^U$ is selected from a single bond or -C(O)N(H)-, wherein either (i) the C atom of $L^U$ is bonded to phenyl, and the N atom of $L^U$ is bonded to Q'; or (ii) the C atom of $L^U$ is bonded to Q', and the N atom of $L^U$ is bonded to phenyl;
$R^{U12}$, $R^{U13}$ and $R^{U14}$ are each independently selected from H, halogen, $C_{1-4}$ alkyl, $NH_2$, $NO_2$, OH, COOH, CN, $CF_3$, and a single bond to LINK;
wherein one and only one of $R^{U12}$, $R^{U13}$ and $R^{U14}$ is a single bond to LINK;
$R^{U15}$ and $R^{U16}$ are each independently selected from H, halogen, $C_{1-4}$ alkyl, $NH_2$, $NO_2$, OH, COOH, CN and $CF_3$;

(c) a CRBN E3 ubiquitin ligase binding moiety of formula (U5):

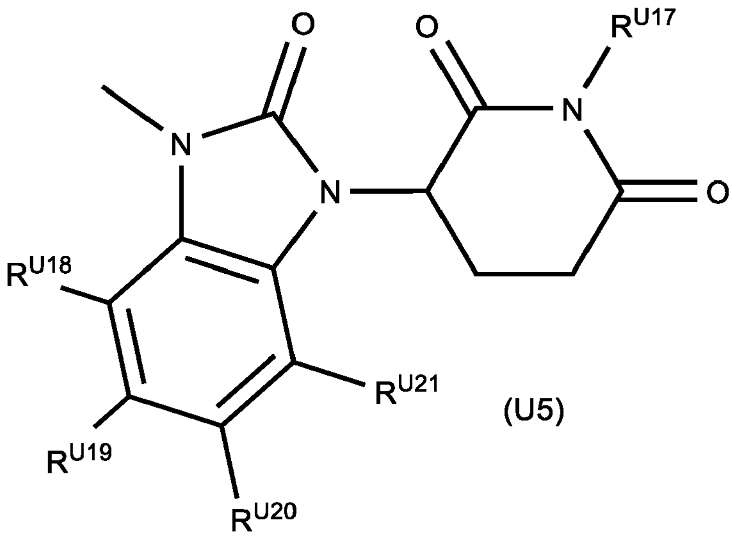

(U5)

wherein:

$R^{U17}$ is H;

$R^{U18}$, $R^{U19}$, $R^{U20}$ and $R^{U21}$ are each independently selected from H, halogen, $C_{1-4}$ alkyl, $NH_2$, $NO_2$, OH, COOH, CN, $CF_3$, and a single bond to LINK;

wherein one and only one of $R^{U18}$, $R^{U19}$, $R^{U20}$ and $R^{U21}$ is a single bond to LINK;

or (d) a VHL E3 ubiquitin ligase binding moiety of formula (U2):

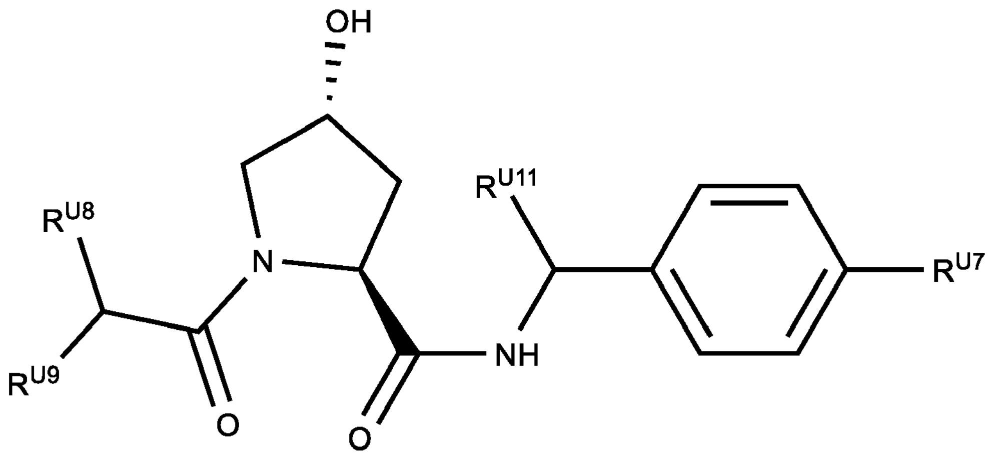

(U2)

wherein:

$R^{U7}$ is a group selected from phenyl, a 5- to 6-membered heteroaryl ring, a 5- to 6-membered heterocyclyl ring, and a 5- to 6-membered cycloalkyl ring, the group $R^{U7}$ being unsubstituted or substituted by $C_{1-4}$ alkyl;

$R^{U11}$ is H or $C_{1-4}$ alkyl;

$R^{U8}$ is selected from $C_{1-4}$ alkyl, phenyl and a 3- to 8-membered cycloalkyl ring; and $R^{U9}$ is a single bond to LINK.

For the avoidance of doubt, in formula (U1), when Q is -NH-CH($R^{U6}$)- or -N=C($R^{U6}$)-, the N atom of Q is bonded to the phenyl ring in U, and the C atom of Q (not including any C atom that may be in $R^{U6}$) is bonded to the N atom in U that is adjacent to group Q.

Typically, in formula (U1), Q is selected from -CH($R^{U6}$)- and -C(O)-. Preferably, Q is selected from -$CH_2$- and -C(O)-.

Typically, in formula (U1), $R^{U6}$ is H or methyl. Preferably, $R^{U6}$ is H.

Typically, in formula (U1), $R^{U2}$ and $R^{U5}$ are each independently selected from H, fluoro, methyl, $NH_2$, $NO_2$, OH,

COOH, CN, $CF_3$, and a single bond to LINK. Preferably, $R^{U2}$ and $R^{U5}$ are each independently selected from H and a single bond to LINK.

Typically, in formula (U1), $R^{U3}$ and $R^{U4}$ are each independently selected from H, fluoro, methyl, $NH_2$, $NO_2$, OH, COOH, CN, $CF_3$ and a single bond to LINK. Preferably, $R^{U3}$ and $R^{U4}$ are each independently selected from H and a single bond to LINK.

Typically, in formula (U1), two or three of $R^{U2}$, $R^{U3}$, $R^{U4}$ and $R^{U5}$ are H. Preferably, three of $R^{U2}$, $R^{U3}$, $R^{U4}$ and $R^{U5}$ are H. More preferably, $R^{U2}$ and $R^{U3}$ are H, one of $R^{U4}$ and $R^{U5}$ is a single bond to LINK, and the other of $R^{U4}$ and $R^{U5}$ is H.

Typically, therefore, in formula (U1):

Q is selected from -CH($R^{U6}$)- and -C(O)-;
$R^{U6}$ is H or methyl;
$R^{U2}$ and $R^{U5}$ are each independently selected from H, fluoro, methyl, $NH_2$, $NO_2$, OH, COOH, CN, $CF_3$, and a single bond to LINK;
$R^{U3}$ and $R^{U4}$ are each independently selected from H, fluoro, methyl, $NH_2$, $NO_2$, OH, COOH, CN, $CF_3$ and a single bond to LINK; and
two or three of $R^{U2}$, $R^{U3}$, $R^{U4}$ and $R^{U5}$ are H.

Preferably, in formula (U1):

Q is selected from $-CH_2-$ and -C(O)-;
$R^{U6}$ is H;
one of $R^{U2}$, $R^{U3}$, $R^{U4}$ and $R^{U5}$ is a single bond to LINK, and the rest are H, for instance wherein $R^{U2}$ and $R^{U3}$ are H, one of $R^{U4}$ and $R^{U5}$ is a single bond to LINK, and the other of $R^{U4}$ and $R^{U5}$ is H.

Typically, in formula (U4), Q' is N and $L^U$ is a single bond, or Q' is CH and $L^U$ is - C(O)N(H)-. Preferably, Q' is N and $L^U$ is a single bond.

Typically, in formula (U4), when $L^U$ is -C(O)N(H)-, then the C atom of $L^U$ is bonded to phenyl, and the N atom of $L^U$ is bonded to Q'.

Typically, in formula (U4), $R^{U12}$, $R^{U13}$ and $R^{U14}$ are each independently selected from H, fluoro, methyl, $NH_2$, $NO_2$, OH, COOH, CN, $CF_3$, and a single bond to LINK (wherein one and only one of $R^{U12}$, $R^{U13}$ and $R^{U14}$ is a single bond to LINK). Preferably, one of $R^{U12}$, $R^{U13}$ and $R^{U14}$ is a single bond to LINK, one of $R^{U12}$, $R^{U13}$ and $R^{U14}$ is H and the other of $R^{U12}$, $R^{U13}$ and $R^{U14}$ is selected from H, fluoro, methyl, $NH_2$, $NO_2$, OH, COOH, CN, $CF_3$, preferably from H, fluoro and methyl. More preferably, one of $R^{U12}$, $R^{U13}$ and $R^{U14}$ is a single bond to LINK and the other two are H, for instance wherein $R^{U12}$ and $R^{U14}$ are H, and $R^{U13}$ is a single bond to LINK.

Typically, in formula (U4), $R^{U15}$ and $R^{U16}$ (when present) are each independently selected from H, fluoro, methyl, $NH_2$, $NO_2$, OH, COOH, CN and $CF_3$. Preferably, $R^{U15}$ and $R^{U16}$ (when present) are each independently selected from H, F and Me.

In one embodiment of formula (U4), $R^{U15}$ and $R^{U16}$ (when present) are H, and two of $R^{U12}$, $R^{U13}$ and $R^{U14}$ are H. Preferably, $R^{U13}$, $R^{U14}$ and $R^{U15}$ are H, $R^{U12}$ is a single bond to LINK, and $R^{U16}$ (when present) is selected from H, F and Me.

Typically, therefore, in formula (U4):

Q' is N and $L^U$ is a single bond, or Q' is CH and $L^U$ is -C(O)N(H)-;
$R^{U12}$, $R^{U13}$ and $R^{U14}$ are each independently selected from H, fluoro, methyl, $NH_2$, $NO_2$, OH, COOH, CN, $CF_3$, and a single bond to LINK; and
$R^{U15}$ and $R^{U16}$ (when present) are each independently selected from H, fluoro, methyl, $NH_2$, $NO_2$, OH, COOH, CN and $CF_3$;
wherein one and only one of $R^{U12}$, $R^{U13}$ and $R^{U14}$ is a single bond to LINK.

Preferably, in formula (U4):

Q' is N and $L^U$ is a single bond;
one of $R^{U12}$, $R^{U13}$ and $R^{U14}$ is a single bond to LINK and the other two are H, for instance wherein $R^{U12}$ and $R^{U14}$ are H, and $R^{U13}$ is a single bond to LINK, or $R^{U13}$, $R^{U14}$
and $R^{U15}$ are H, $R^{U12}$ is a single bond to LINK, and $R^{U16}$ (when present) is selected from H, F and Me.

Typically, in formula (U5), $R^{U18}$, $R^{U19}$, $R^{U20}$ and $R^{U21}$ are each independently selected from H, fluoro, methyl, $NH_2$, $NO_2$, OH, COOH, CN, $CF_3$, and a single bond to LINK (wherein one and only one of $R^{U18}$, $R^{U19}$, $R^{U20}$ and $R^{U21}$ is a

single bond to LINK). Preferably, one of $R^{U18}$, $R^{U19}$, $R^{U20}$ and $R^{U21}$ is a single bond to LINK, one or two, preferably two of $R^{U18}$, $R^{U19}$, $R^{U20}$ and $R^{U21}$ are H and the other one or two, preferably one of $R^{U18}$, $R^{U19}$, $R^{U20}$ and $R^{U21}$ are selected from H, fluoro, methyl, $NH_2$, $NO_2$, OH, COOH, CN, $CF_3$, preferably from H, fluoro and methyl. More preferably, one of $R^{U18}$, $R^{U19}$, $R^{U20}$ and $R^{U21}$ is a single bond to LINK and the other three are H, for instance wherein $R^{U19}$, $R^{U20}$ and $R^{U21}$ are H, and $R^{U18}$ is a single bond to LINK.

Typically, in formula (U2), $R^{U7}$ is a 5- to 6-membered heteroaryl ring. Preferably, $R^{U7}$ is a 5-membered heteroaryl ring containing one S atom and optionally one N atom.

Typically, in formula (U2), $R^{U7}$ is unsubstituted or substituted by $C_{1-4}$ alkyl. Preferably, $R^{U7}$ is unsubstituted or substituted by methyl.

Typically, in formula (U2), $R^{U11}$ is H or methyl. Preferably, $R^{U11}$ is H.

Typically, in formula (U2), $R^{U8}$ is $C_{1-4}$ alkyl or phenyl. Preferably, $R^{U8}$ is t-butyl or phenyl, more preferably t-butyl.

Typically, therefore, in formula (U2), $R^{U7}$ is a 5- to 6-membered heteroaryl ring, the group $R^{U7}$ being unsubstituted or substituted by $C_{1-4}$ alkyl, $R^{U11}$ is H or methyl, and $R^{U8}$ is $C_{1-4}$ alkyl or phenyl.

Preferably, in formula (U2), $R^{U7}$ is a 5-membered heteroaryl ring containing one S atom and optionally one N atom, the group $R^{U7}$ being unsubstituted or substituted by methyl, $R^{U11}$ is H, and $R^{U8}$ is *t*-butyl or phenyl, more preferably *t*-butyl.

In a preferred embodiment, the E3 ubiquitin ligase binding moiety (U) is a VHL E3 ubiquitin ligase binding moiety of formula (U3):

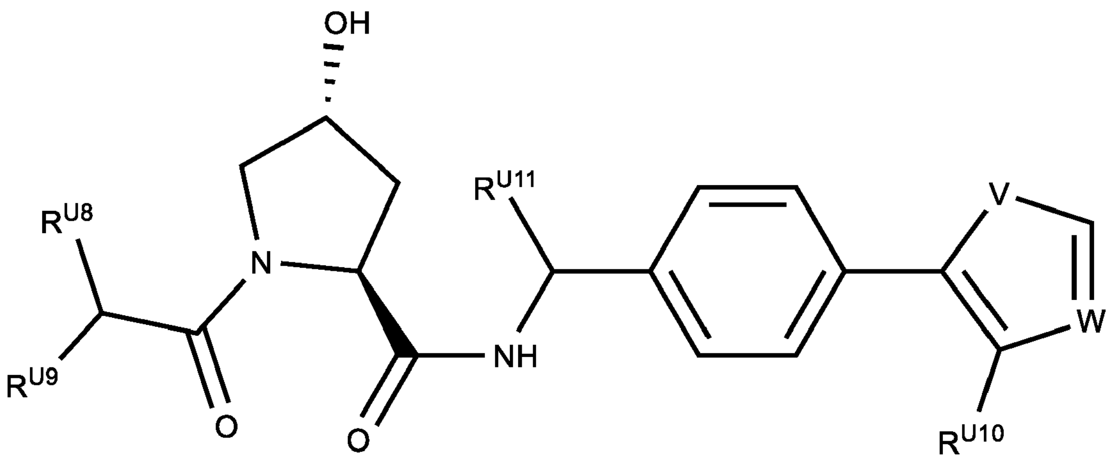

(U3)

wherein:

V is S or O;
W is N or CH;
$R^{U10}$ is H or $C_{1-4}$ alkyl;
$R^{U11}$ is H or $C_{1-4}$ alkyl;
$R^{U8}$ is selected from $C_{1-4}$ alkyl, phenyl and a 3- to 8-membered cycloalkyl ring; and
$R^{U9}$ is a single bond to LINK.

Typically, in formula (U3), V is S.

Typically, in formula (U3), W is N or CH. Preferably, W is N.

Typically, in formula (U3), $R^{U10}$ is H or methyl. Preferably, $R^{U10}$ is methyl.

Typically, in formula (U3), $R^{U11}$ is H or methyl. Preferably, $R^{U11}$ is H.

Typically, in formula (U3), $R^{U8}$ is $C_{1-4}$ alkyl or phenyl. Preferably, $R^{U8}$ is *t*-butyl or phenyl, more preferably *t*-butyl.

Typically, therefore, in formula (U3), V is S, W is N or CH, $R^{U10}$ is H or methyl, $R^{U11}$ is H or methyl, and $R^{U8}$ is $C_{1-4}$ alkyl or phenyl. Preferably, V is S, W is N, $R^{U10}$ is methyl, $R^{U11}$ is H, $R^{U8}$ is *t*-butyl or phenyl, more preferably *t*-butyl.

The moieties U of formulae (U1), (U4) when Q' is CH and (U5) typically contain at least one chiral centre at the carbon atoms marked with an asterisk as depicted below:

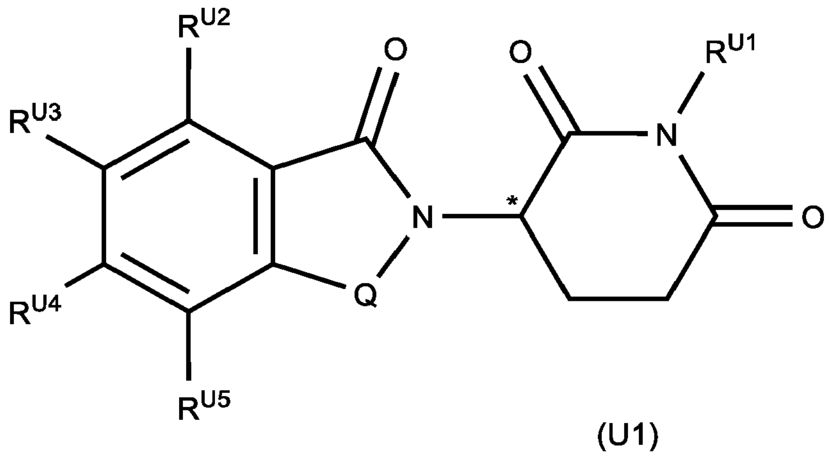

(U1) ;

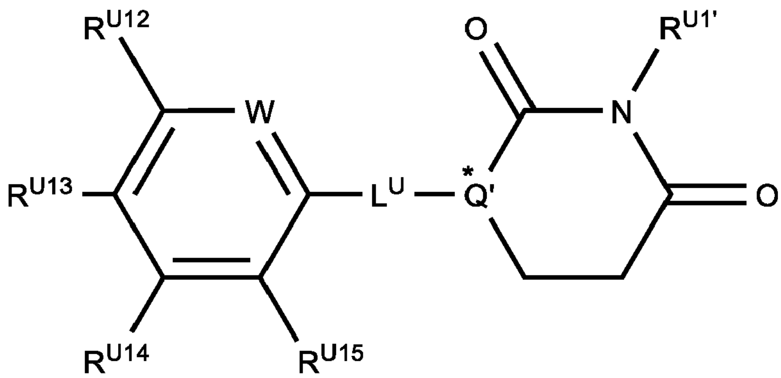

(U4) ;

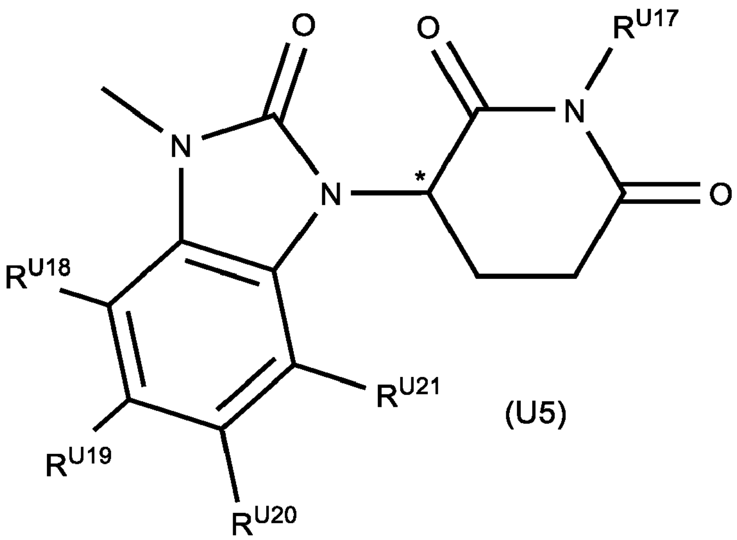

(U5) .

Moiety U may therefore be provided in the form of the R-enantiomer at said carbon atom, in the form of the S-enantiomer at said carbon atom, or in the form of a mixture (for example a 1:1 mixture) of the two enantiomers. A pure enantiomeric form of either the R- or the S-enantiomer may be preferred. In some embodiments, the S-enantiomer at said carbon atom is preferred. In other embodiments, the R-enantiomer at said carbon atom is preferred. The substance or agent described herein may contain at least 50%, preferably at least 60%, 75%, 90% or 95% of a compound which is enantiomerically or diasteriomerically pure at moiety U. Unless otherwise stated, the moiety U may typically be provided in the form of a 1:1 mixture of the two enantiomers.

*Linkers*

As discussed herein, in the compounds of the invention, the binder of MLLT1 and/or MLLT3 (M) is covalently attached to an E3 ubiquitin ligase binding moiety (U). This covalent attachment is labelled LINK and may be a direct single bond or a divalent chemical linker group that forms covalent bonds both to the binder of MLLT1 and/or MLLT3 (M), and to the E3 ubiquitin ligase binding moiety (U). Typically, LINK is a divalent chemical linker group that forms covalent bonds both to the binder of MLLT1 and/or MLLT3 (M), and to the E3 ubiquitin ligase binding moiety (U).

There is no particular limitation on the nature of LINK in the compounds of the present invention (beyond that the respective active moieties, e.g., U and M, are able to exert their desired function and LINK is capable of covalently attaching them together). Those skilled in the art would recognise that chemical linker groups are routinely used in the construction of bifunctional molecules and would be able routinely to provide appropriate chemical linker groups for attaching particular U and M moieties together. Typically, a chemical linker group for use in the present invention is an organic group.

When LINK is a divalent chemical linker group, it may be represented by formula (L):

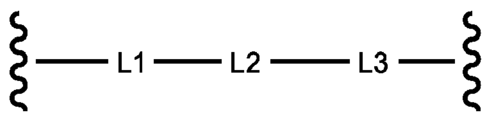

(L)

wherein:

LINK is attached to M via L1;
LINK is attached to U via L3;
L1 and L3 are each independently selected from a single bond, -N(R')-, -C(O)N(R')-, -O-, -N(R')C(O)-, -C(O)-, -S($O_2$)N(R')-, -N(R')S($O_2$)-, -($C_{1-6}$ alkylene)-, ethynyl, -($C_{2-6}$ alkenylene)-, a divalent 3- to 6-membered cycloalkyl ring and a divalent 4- to 7-membered heterocyclyl ring;
L2 is represented by the formula $-(L4)_m-$;
each L4 is independently selected from a divalent 3- to 6-membered cycloalkyl ring, a divalent 4- to 7-membered heterocyclyl ring and a unit of formula:

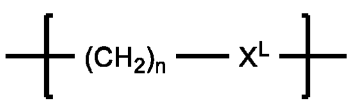 or 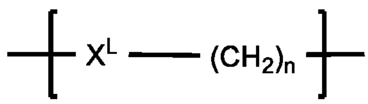 ;

each $X^L$ is independently selected from a single bond, -N(R')-, -O-, -C(O)-, -S-, -SO-, - $SO_2$-, -C(R")=C(R")- and -C(H)≡C(H)-;
n is selected from 1 to 4;
m is selected from 1 to 30;
each R' is independently selected from Hand $C_{1-4}$ alkyl; and
each R" is independently selected from H and halo.

In one embodiment, L1 and L3 are each independently selected from a single bond, - N(R')-, -C(O)N(R')-, -O-, -N(R')C(O)-, -C(O)-, -S($O_2$)N(R')-, -N(R')S($O_2$)-, -($C_{1-6}$ alkylene)-, -($C_{2-6}$ alkenylene)-, ethynyl, a divalent 3- to 6-membered cycloalkyl ring and a divalent 4- to 7-membered heterocyclyl ring;

L2 is represented by the formula $-(L4)_m-$; and
each L4 is independently selected from a divalent 3- to 6-membered cycloalkyl ring, a divalent 4- to 7-membered heterocyclyl ring and a unit of formula:

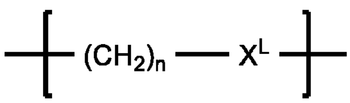

When m is not 1 (i.e. when there are two or more L4 groups present), then the $X^L$ group of any L4 group is not

directly bonded to the $X^L$ group of any other L4 group that is present.

Typically, in formula (L), L1 is selected from a single bond, branched or straight-chain - ($C_{1-6}$ alkylene)-, -C(O)N(R')-, -N(R')C(O)-, -O-, -S($O_2$)N(R')-, -N(R')S($O_2$)-, and a divalent 4- to 7-membered heterocyclyl ring. When present, the divalent 4- to 7-membered heterocyclyl ring of L1 typically contains at least one N atom, for instance one or two N atoms. Preferably, when L1 is a divalent 4- to 7-membered heterocyclyl ring, it is a divalent 5- to 6-membered heterocyclyl ring, more preferably a saturated divalent 5- to 6-membered heterocyclyl ring, for instance a divalent moiety of piperidine or diazinane. Preferably, in formula (L), L1 is selected from a single bond, branched or straight-chain -($C_{1-6}$ alkylene)-, -C(O)N(H)-, -N(H)C(O)-, and a divalent moiety of piperidine or diazinane.

Typically, in formula (L), L3 is selected from a single bond, -N(R')-, -C(O)N(R')-, -O-, -N(R')C(O)-, -S($O_2$)N(R')-, -N(R')S($O_2$)-, ethynyl and a divalent 4- to 7-membered heterocyclyl ring. When present, the divalent 4- to 7-membered heterocyclyl ring of L3 typically contains at least one N atom, for instance one or two N atoms. Preferably, when L3 is a divalent 4- to 7-membered heterocyclyl ring, it is a divalent 5- to 6-membered heterocyclyl ring, more preferably a saturated divalent 5- to 6-membered heterocyclyl ring, for instance a divalent moiety of piperidine or diazinane, preferably 1,4-diazinane. Preferably, in formula (L), L3 is selected from a single bond, -N(H)-, - C(O)N(H)-, -O-, -N(H)C(O)-, ethynyl and a divalent moiety of piperidine or diazinane.

L2 is represented by the formula -$(L4)_m$-. Typically, in formula (L), each L4 is independently selected from a divalent 4- to 7-membered heterocyclyl ring and a unit of formula

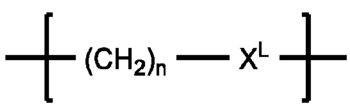

or

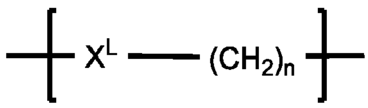

as described above.

Typically, each $X^L$ is independently selected from a single bond, -O-, -S-, - C(R")=C(R")- and -C(H)≡C(H)-, for example a single bond, -O- and -S-, and n is selected from 1 or 2. In one embodiment, each L4 is the same. Alternatively, L2 may comprise two or more blocks, wherein in each block, each L4 is the same. For instance, L2 may be represented by the formula -$(L4')_p$-$(L4")_q$-$(L4''')_r$-, wherein L4', L4" and L4''' are selected from those moieties described above for L4, and wherein each L4' is the same, each L4" is the same and each L4''' is the same. Preferably, L4', L4" and L4''' are selected from -$CH_2$-, -$CH_2CH_2O$-, -$OCH_2CH_2$-, -$CH_2CH_2S$- and -$SCH_2CH_2$-. More preferably, L4', L4" and L4''' are selected from -$CH_2$-, -$CH_2CH_2O$- and - $OCH_2CH_2$-. p, q and r are integers of from 0 to 30, wherein p+q+r=m.

In one embodiment, each L4 unit is the same. For instance, each L4 in formula (L) may be -$CH_2$-, each L4 in formula (L) may be -$CH_2CH_2O$-, each L4 in formula (L) may be - $OCH_2CH_2$-, each L4 in formula (L) may be -$CH_2CH_2S$-, or each L4 in formula (L) may be -$SCH_2CH_2$-. More preferably, each L4 in formula (L) is -$CH_2$-, each L4 in formula (L) is -$CH_2CH_2O$- or each L4 in formula (L) is -$OCH_2CH_2$-. Most preferably, each L4 in formula (L) is -$CH_2$-.

Typically, in formula (L), m is selected from 1 to 10.

Typically, in formula (L), each R' is independently selected from H and methyl. Preferably, each R' is H.

Typically, in formula (L), each R" is independently selected from H, fluoro and chloro. Preferably, each R" is H and chloro, for example H.

When one or more L4 is a divalent 4- to 7-membered heterocyclyl ring, typically only one or two, e.g. one L4 is a divalent 4- to 7-membered heterocyclyl ring. Typically, therefore, none, one or two L4 is a divalent 4- to 7-membered heterocyclyl ring, for example none or one L4 is a divalent 4- to 7-membered heterocyclyl ring.

Typically, therefore, in formula (L):

L1 is selected from a single bond, branched or straight-chain -($C_{1-6}$ alkylene)-, - C(O)N(R')-, -N(R')C(O)-, -O-, -S($O_2$)N(R')-, -N(R')S($O_2$)-, and a divalent 4- to 7-membered heterocyclyl ring typically containing at least one N atom, for instance one or two N atoms;
L3 is selected from a single bond, -N(R')-, -C(O)N(R')-, -O-, -N(R')C(O)-, -S($O_2$)N(R')-, -N(R')S($O_2$)-, ethynyl and a divalent 4- to 7-membered heterocyclyl ring typically containing at least one N atom, for instance one or two N atoms;
each L4 is independently selected from -$CH_2$-, -$CH_2CH_2O$-, -$OCH_2CH_2$-, -$CH_2CH_2S$-, -$SCH_2CH_2$-, and a divalent 4- to 7-membered heterocyclyl ring, wherein when one or more L4 is a divalent 4- to 7-membered heterocyclyl ring, only one or two, e.g. one L4 is a divalent 4- to 7-membered heterocyclyl ring;
m is selected from 1 to 10;
each R' is independently selected from H and methyl; and
each R" is independently selected from H, fluro and chloro, for instance Hand chloro.

Preferably, in formula (L):

L1 is selected from a single bond, branched or straight-chain -($C_{1-6}$ alkylene)-, - C(O)N(H)-, -N(H)C(O)-, and a divalent moiety of piperidine or diazinane;
L3 is selected from a single bond, -N(H)-, -C(O)N(H)-, -O-, -N(H)C(O)-, ethynyl and a divalent moiety of piperidine or diazinane;
each L4 in formula (L) is -$CH_2$-, each L4 in formula (L) is -$CH_2CH_2O$-, each L4 in formula (L) is -$OCH_2CH_2$-, each L4 in formula (L) is -$CH_2CH_2S$-, or each L4 in formula (L) is -$SCH_2CH_2$-, with each L4 preferably being -$CH_2$-;
m is selected from 1 to 10;
each R' is H; and
each R" is H or chloro, for instance H.

In a typical embodiment, L2 is -($C_{1-10}$ alkylene)- or -($C_{1-10}$ alkylene)-C(O)-, preferably - ($C_{1-10}$ alkylene)-. In this embodiment, L1 is preferably selected from a single bond, - C(O)N(H)-, -N(H)C(O)-, and a divalent moiety of piperidine or diazinane. In this embodiment, L3 is preferably selected from a single bond, -N(H)-, -C(O)N(H)-, -O-, - N(H)C(O)-, ethynyl and a divalent moiety of piperidine or diazinane. More preferably, L1 and L3 are selected from the following options:

- L1 is a single bond and L3 is -N(H)-;
- L1 is a divalent moiety of piperidine and L3 is -N(H)-;
- L1 is -C(O)N(H)- or -N(H)C(O)-, and L3 is -C(O)N(H)- or -N(H)C(O)-;
- L1 is a divalent moiety of piperidine and L3 is a divalent moiety of diazinane;
- L1 is -C(O)N(H)- or -N(H)C(O)-, and L3 is a divalent moiety of diazinane;
- L1 is a single bond and L3 is a divalent moiety of diazinane;
- L1 is a single bond and L3 is a divalent moiety of piperidine;
- L1 is a single bond and L3 is -O-;
- L1 is a single bond and L3 is ethynyl;
- L1 is a single bond and L3 is a single bond; and
- L1 is -C(O)N(H)- or -N(H)C(O)-, and L3 is -O-.

For the avoidance of doubt, the skilled person would readily appreciate that each of the definitions of moieties U, M and LINK provided herein may be taken together in any combination to arrive at a definition of the compound of the present invention. As examples that are in no way limiting, definitions of moieties U, M and LINK labelled "typically" may be combined to arrive at a definition of a typical compound of the present invention, and definitions of moieties U, M and LINK labelled "preferably" may be combined to arrive at a definition of a preferred compound of the present invention. However, the skilled person would appreciate that a definition labelled "typically" of one moiety may be combined with a definition labelled "preferably" of another moiety to arrive at a definition of a compound of the present invention.
In preferred compounds of the present invention, M is of formula (III):

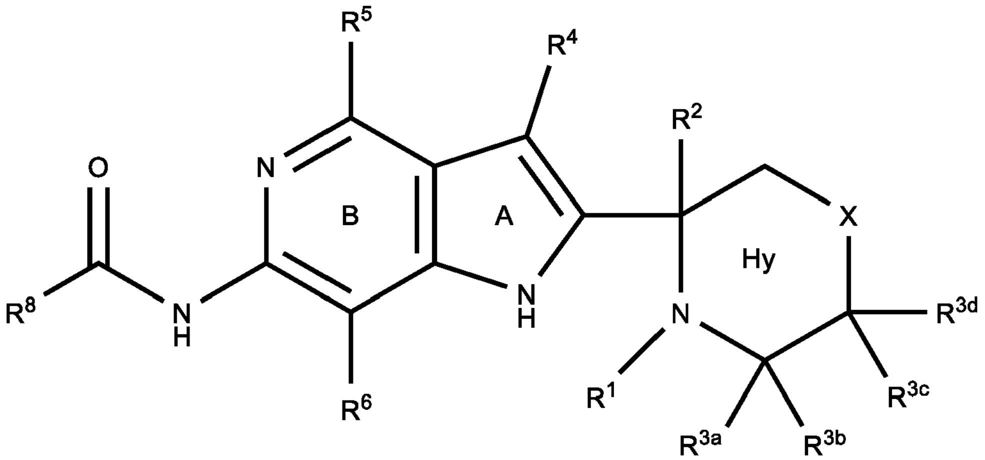

(III)

wherein $R^1$, $R^2$, $R^{3a}$, $R^{3b}$, $R^{3c}$, $R^{3d}$, X, $R^4$, $R^5$, $R^6$ and $R^8$ are as defined herein.
Typically, in such preferred compounds:

$R^1$ is H or $C_{1-4}$ alkyl which is itself unsubstituted or substituted with $C_{1-4}$ alkoxy;
$R^2$ is H or methyl;

$R^{3a}$ and $R^{3b}$ are independently selected from H, $C_{1-4}$ alkyl, and $C_{1-4}$ alkoxy, or $R^{3a}$ and $R^{3b}$ form, together with the C atom to which they are attached, a $C_{3-6}$ cycloalkyl ring,
$R^{3c}$ and $R^{3d}$ are independently selected from H, $C_{1-4}$ alkyl, halo and $C_{1-4}$ alkoxy, or $R^{3c}$ and $R^{3d}$ form, together with the C atom to which they are attached, a $C_{3-6}$ cycloalkyl ring, or
$R^{3a}$ and $R^{3c}$ are H, and $R^{3b}$ and $R^{3d}$ form, together with the C atoms to which they are attached, a $C_{5-6}$ cycloalkyl ring, with the proviso that when X is O or -N(Me)-, then neither $R^{3c}$ nor $R^{3d}$ are halo;
wherein at least two of $R^{3a}$, $R^{3b}$, $R^{3c}$ and $R^{3d}$ are H;
X is a bond, -N(Me)-, O or $-CH_2-$;
$R^4$ is selected from H, CN, halo $C_{1-4}$ alkoxy, and $C_{1-4}$ alkyl which is itself unsubstituted or substituted by one, two or three halo;
$R^5$ and $R^6$ are independently selected from H, halo $C_{1-4}$ alkoxy, and $C_{1-4}$ alkyl which is itself unsubstituted or substituted by one, two or three halo;
$R^8$ is an 8- to 10-membered heteroaryl ring, the group $R^8$ being unsubstituted or substituted by one, two or three substituents independently selected from halo, $C_{1-4}$ alkoxy, $R^{10}$, -($C_{1-4}$ alkylene)-$R^{10}$, =O, -CN, and $C_{1-4}$ alkyl which is itself unsubstituted or substituted by one or two $R^9$;
each $R^9$ is independently selected from halo and $C_{1-4}$ alkoxy; and $R^{10}$ is a group selected from a 5- to 6-membered heteroaryl ring, a 3- to 6-membered cycloalkyl ring, a 4- to 6-membered heterocyclyl ring, and a phenyl ring, the group $R^{10}$ being unsubstituted or substituted by one or two substituents independently selected from $C_{1-4}$ alkyl, $C_{1-4}$ alkoxy, and halo;
wherein M is bonded to LINK via a C or N atom within group $R^8$ or ring Hy (as described herein) such that a hydrogen atom on the C or N atom within group $R^8$ or ring Hy is replaced with a bond to LINK; and
LINK is (a) a single bond, or (b) a chemical linker group represented by formula (L):

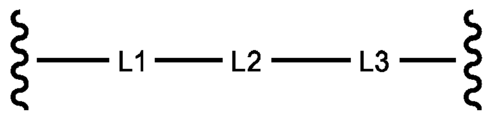

(L)

wherein:

LINK is attached to M via L1;
LINK is attached to U via L3;
L1 and L3 are each independently selected from a single bond, branched or straight-chain -($C_{1-6}$ alkylene)-, -N(R')-, -C(O)N(R')-, -O-, -N(R')C(O)-, -C(O)-, $-S(O_2)N(R')-$, $-N(R')S(O_2)-$, -($C_{1-6}$ alkylene)-, ethynyl, -($C_{2-6}$ alkenylene)-, a divalent 3- to 6-membered cycloalkyl ring and a divalent 4- to 7-membered heterocyclyl ring;
L2 is represented by the formula $-(L4)_m-$;
each L4 is independently selected from a divalent 3- to 6-membered cycloalkyl ring, a divalent 4- to 7-membered heterocyclyl ring and a unit of formula:

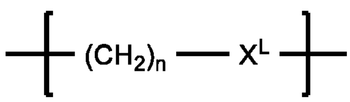 or 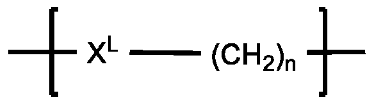 ;

each $X^L$ is independently selected from a single bond, -N(R')-, -O-, -C(O)-, -S-, -SO-, $- SO_2-$, -C(R")=C(R")- and -C(H)≡C(H)-;
n is selected from 1 to 4;
m is selected from 1 to 30;
each R' is independently selected from H and $C_{1-4}$ alkyl;
each R" is independently selected from H, fluoro and chloro; and
U is:

(a) a CRBN E3 ubiquitin ligase binding moiety of formula (U1):

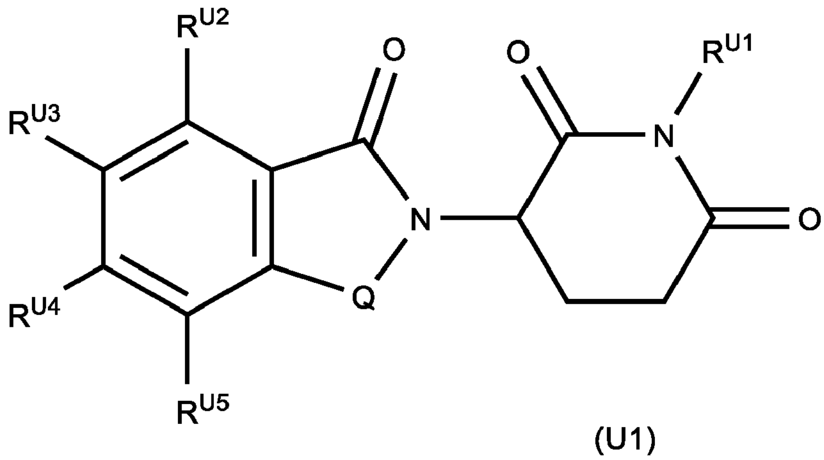

(U1)

wherein:

$R^{U1}$ is H;
Q is selected from -CH($R^{U6}$)-, -N($R^{U6}$)-, -O-, -C(O)-, -NH-CH($R^{U6}$)-, -N=C($R^{U6}$)-, or -N=N-;
$R^{U6}$ is H or $C_{1-4}$ alkyl;
$R^{U2}$ and $R^{U5}$ are each independently selected from H, halogen, $C_{1-4}$ alkyl, $NH_2$, $NO_2$, OH, COOH, CN, $CF_3$, and a single bond to LINK;
$R^{U3}$ and $R^{U4}$ are each independently selected from H, halogen, $C_{1-4}$ alkyl, $NH_2$, $NO_2$, OH, COOH, CN, $CF_3$, and a single bond to LINK;
wherein one and only one of $R^{U2}$, $R^{U3}$, $R^{U4}$ and $R^{U5}$ is a single bond to LINK;

(b) a CRBN E3 ubiquitin ligase binding moiety of formula (U4):

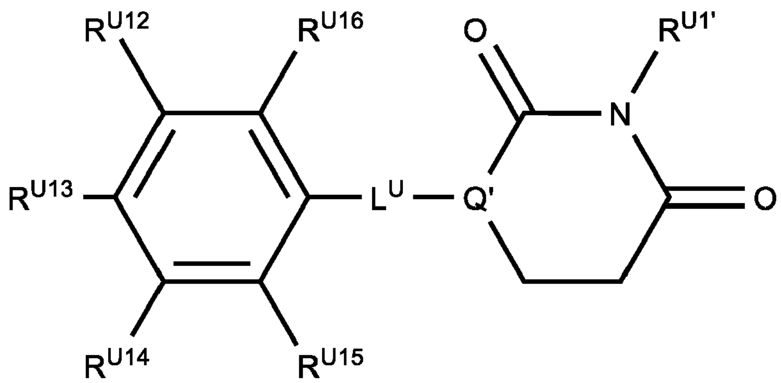

(U4)

wherein:

$R^{U1'}$ is H;
Q' is N and $L^U$ is a single bond, or Q' is CH and $L^U$ is selected from a single bond or -C(O)N(H)-, wherein either (i) the C atom of $L^U$ is bonded to phenyl, and the N atom of $L^U$ is bonded to Q'; or (ii) the C atom of $L^U$ is bonded to Q', and the N atom of $L^U$ is bonded to phenyl;
$R^{U12}$, $R^{U13}$ and $R^{U14}$ are each independently selected from H, halogen, $C_{1-4}$ alkyl, $NH_2$, $NO_2$, OH, COOH, CN, $CF_3$, and a single bond to LINK;
wherein one and only one of $R^{U12}$, $R^{U13}$ and $R^{U14}$ is a single bond to LINK;
$R^{U15}$ and $R^{U16}$ are each independently selected from H, halogen, $C_{1-4}$ alkyl, $NH_2$, $NO_2$, OH, COOH, CN and $CF_3$;

(c) a CRBN E3 ubiquitin ligase binding moiety of formula (U5):

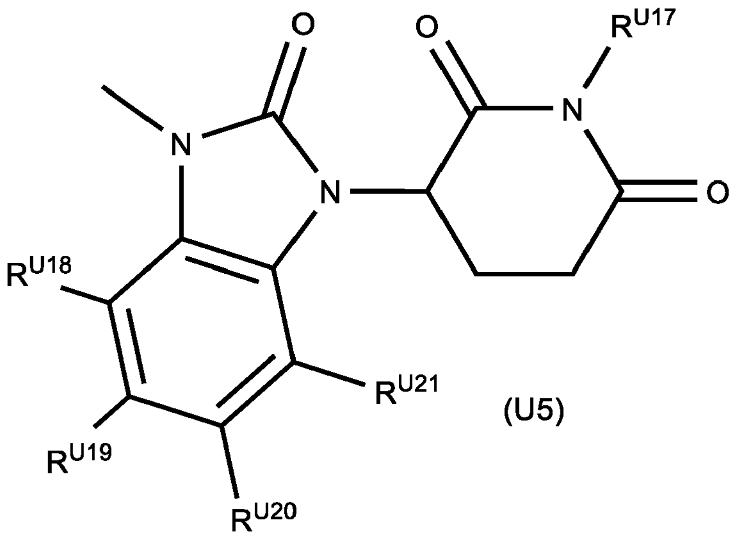

(U5)

wherein:

$R^{U17}$ is H;
$R^{U18}$, $R^{U19}$, $R^{U20}$ and $R^{U21}$ are each independently selected from H, halogen, $C_{1-4}$ alkyl, $NH_2$, $NO_2$, OH, COOH, CN, $CF_3$, and a single bond to LINK;
wherein one and only one of $R^{U18}$, $R^{U19}$, $R^{U20}$ and $R^{U21}$ is a single bond to LINK;
or (d) a VHL E3 ubiquitin ligase binding moiety of formula (U2):

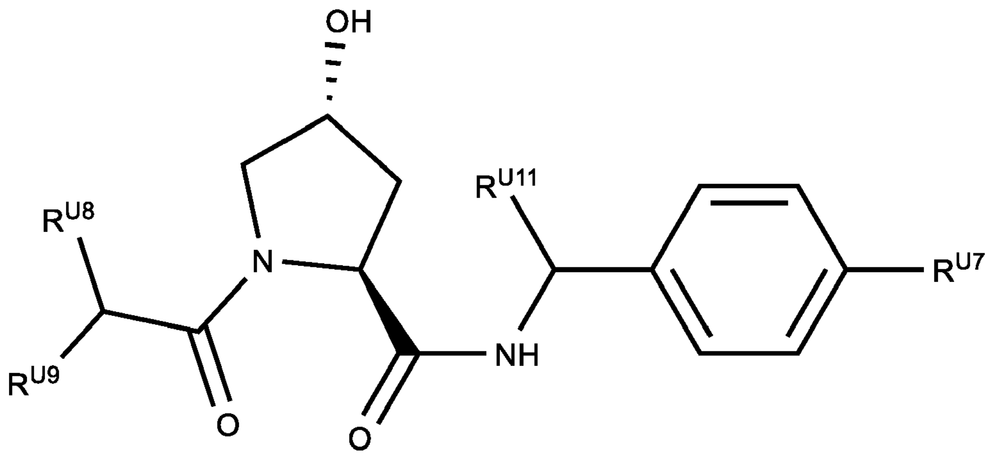

(U2)

wherein:

$R^{U7}$ is a group selected from phenyl, a 5- to 6-membered heteroaryl ring, a 5- to 6-membered heterocyclyl ring, and a 5- to 6-membered cycloalkyl ring, the group $R^{U7}$ being unsubstituted or substituted by $C_{1-4}$ alkyl;
$R^{U11}$ is H or $C_{1-4}$ alkyl;
$R^{U8}$ is selected from $C_{1-4}$ alkyl, phenyl and a 3- to 8-membered cycloalkyl ring; and
$R^{U9}$ is a single bond to LINK.

In one preferred aspect of this embodiment, M is of formula (IV):

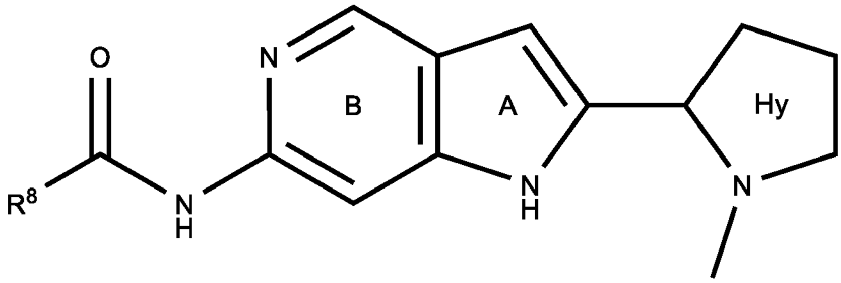

(IV)

wherein $R^8$ is as described herein, the group $R^8$ being unsubstituted or substituted by one, two or three substituents independently selected from halo and $C_{1-4}$ alkyl; wherein M is bonded to LINK via a C or N atom within group $R^8$ such that a hydrogen atom on the C or N atom within group $R^8$ is replaced with a bond to LINK.

In this embodiment, $R^8$ is preferably selected from one of the following structures:

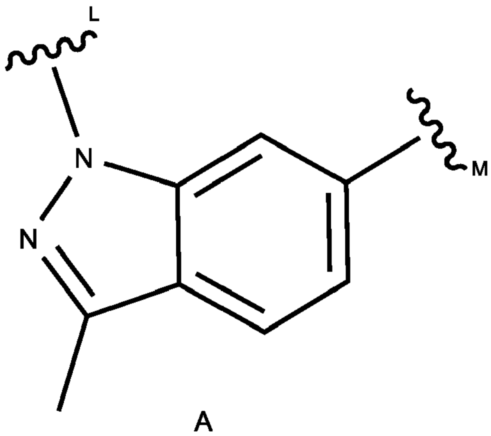

A

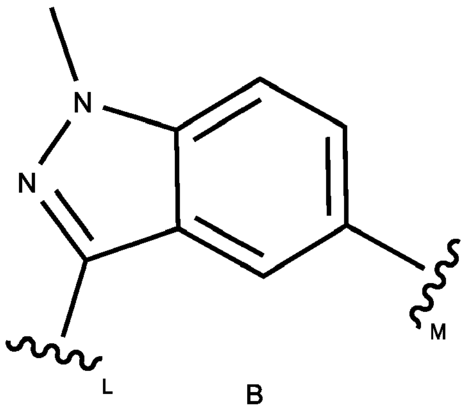

B

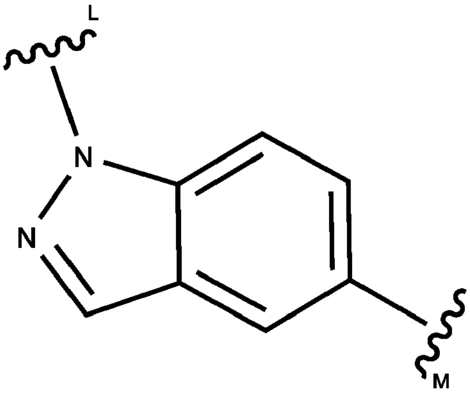

C

wherein:

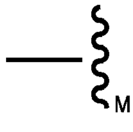

represents the point of attachment of $R^8$ to the rest of moiety M;

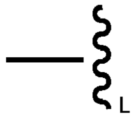

represents the point of attachment of $R^8$ to LINK; and the group $R^8$ is further unsubstituted or substituted by one or two substituents independently selected from fluorine and methyl.

In this embodiment, L2 is preferably selected from $-(CH_2)_m-$, $-(CH_2CH_2O)_m-$, - $(OCH_2CH_2)_m-$, $-(CH_2CH_2S)_m-$, $-(SCH_2CH_2)_m-$, and a divalent 4- to 7-membered heterocyclyl ring.

In this embodiment, preferably L1 and L3 are each independently selected from a single bond, branched or straight-chain -($C_{1-6}$ alkylene)-, -N(R')-, -C(O)N(R')-, -N(R')C(O)-, - O-, ethynyl and a divalent 4- to 7-membered heterocyclyl ring. Preferably in this embodiment, R' is H and the divalent 4- to 7-membered heterocyclyl ring is piperidine or diazinane.

In this embodiment, U is preferably (i) of formula (U1) wherein preferably Q is selected from $-CH_2-$ and -C(O)-, $R^{U6}$ is H, one of $R^{U2}$, $R^{U3}$, $R^{U4}$ and $R^{U5}$ is a single bond to LINK, and the rest are H, for instance wherein $R^{U2}$ and $R^{U3}$ are H, one of $R^{U4}$ and $R^{U5}$ is a single bond to LINK, and the other of $R^{U4}$ and $R^{U5}$ is H, or (ii) of formula (U3) wherein preferably V is S, W is N, $R^{U10}$ is methyl, $R^{U11}$ is H, and $R^{U8}$ is t-butyl or phenyl, more preferably t-butyl.

Particularly preferred compounds of the invention may be selected from:

1-(9-(2-(2,6-dioxopiperidin-3-yl)-1-oxoisoindolin-4-yl)non-8-yn-1-yl)-3-methyl-*N*-(2-((*R*)-1-methylpyrrolidin-2-yl)-1*H*-pyrrolo[3,2-c]pyridin-6-yl)-1*H*-indazole-6-carboxamide;
rac-1-(8-{[2-(2,6-dioxopiperidin-3-yl)-1,3-dioxoisoindol-4-yl]amino}octyl)-3-methyl-N-[2-(1-methylpyrrolidin-2-yl)-1H-pyrrolo[3,2-c]pyridin-6-yl]indazole-6-carboxamide;
1-(8-{[2-(2,6-dioxopiperidin-3-yl)-1,3-dioxoisoindol-4-yl]amino}octyl)-3-methyl-N-{2-[(2R)-1-methylpyrrolidin-2-yl]-1H-pyrrolo[3,2-c]pyridin-6-yl}indazole-6-carboxamide;
1-(8-{[2-(2,6-dioxopiperidin-3-yl)-1,3-dioxoisoindol-4-yl]amino}octyl)-3-methyl-N-{2-[(2S)-1-methylpyrrolidin-2-yl]-1H-pyrrolo[3,2-c]pyridin-6-yl}indazole-6-carboxamide;
2-(8-((2-(2,6-dioxopiperidin-3-yl)-1,3-dioxoisoindolin-4-yl)amino)octyl)-3-methyl-N-(2-((R)-1-methylpyrrolidin-2-yl)-1H-pyrrolo[3, 2-c]pyridin-6-yl)-2H-indazole-6-carboxamide;
3-[4-({4-[2-(2,6-dioxopiperidin-3-yl)-6-fluoro-1,3-dioxoisoindol-5-yl]piperazin-1-yl}methyl)piperidin-1-yl]-1-methyl-N-{2-[(2R)-1-methylpyrrolidin-2-yl]-1H-pyrrolo[3,2-c]pyridin-6-yl}indazole-5-carboxamide;
1-[1-(3-{[2-(2,6-dioxopiperidin-3-yl)-1,3-dioxoisoindol-4-yl]amino}propyl)piperidin-4-yl]-3-methyl-N-{2-[(2R)-1-methylpyrrolidin-2-yl]-1H-pyrrolo[3,2-c]pyridin-6-yl}indazole-6-carboxamide;
1-(8-{[2-(2,6-dioxopiperidin-3-yl)-1,3-dioxoisoindol-5-yl]oxy}octyl)-3-methyl-N-{2-[(2R)-1-methylpyrrolidin-2-yl]-1H-pyrrolo[3,2-c]pyridin-6-yl}indazole-6-carboxamide;
1-{8-[3-(2,4-dioxo-1,3-diazinan-1-yl)-2-methylphenoxy]octyl}-3-methyl-N-{2-[(2R)-1-methylpyrrolidin-2-yl]-1H-pyrrolo[3,2-c]pyridin-6-yl}indazole-6-carboxamide;
1-{6-[3-(2,4-dioxo-1,3-diazinan-1-yl)-2-methylphenoxy]hexyl}-3-methyl-N-{2-[(2R)-1-methylpyrrolidin-2-yl]-1H-pyrrolo[3,2-c]pyridin-6-yl}indazole-6-carboxamide;
1-{7-[1-(2,6-dioxopiperidin-3-yl)-3-methyl-2-oxo-1,3-benzodiazol-4-yl]hept-6-yn-1-yl}-3-methyl-N-{2-[(2R)-1-methylpyrrolidin-2-yl]-1H-pyrrolo[3,2-c]pyridin-6-yl}indazole-6-carboxamide;
1-(8-((2-(2,6-dioxopiperidin-3-yl)-1,3-dioxoisoindolin-4-yl)oxy)octyl)-3-methyl-N-(2-((R)-1-methylpyrrolidin-2-yl)-1H-pyrrolo[3,2-c]pyridin-6-yl)-1H-indazole-6-carboxamide;
1-(3-((2-(2,6-dioxopiperidin-3-yl)-1,3-dioxoisoindolin-4-yl)amino)propyl)-3-methyl-N-(2-((R)-1-methylpyrrolidin-2-yl)-1H-pyrrolo[3,2-c]pyridin-6-yl)-1H-indazole-6-carboxamide;
1-(8-{[2-(2,6-dioxopiperidin-3-yl)-1,3-dioxoisoindol-5-yl]amino}octyl)-3-methyl-N-{2-[(2R)-1-methylpyrrolidin-2-yl]-1H-pyrrolo[3,2-c]pyridin-6-yl}indazole-6-carboxamide;
1-(8-{[2-(2,6-dioxopiperidin-3-yl)-1,3-dioxoisoindol-5-yl]amino}octyl)-3-methyl-N-{2-[(2S)-1-methylpyrrolidin-2-yl]-1H-pyrrolo[3,2-c]pyridin-6-yl}indazole-6-carboxamide;
1-(8-{[2-(2,6-dioxopiperidin-3-yl)-1-oxo-3H-isoindol-4-yl]amino}octyl)-3-methyl-N-{2-[(2R)-1-methylpyrrolidin-2-yl]-1H-pyrrolo[3,2-c]pyridin-6-yl}indazole-6-carboxamide;
1-(8-{[2-(2,6-dioxopiperidin-3-yl)-1-oxo-3H-isoindol-4-yl]amino}octyl)-3-methyl-N-{2-[(2S)-1-methylpyrrolidin-2-yl]-1H-pyrrolo[3,2-c]pyridin-6-yl}indazole-6-carboxamide;
N-(3-chloro-2-((R)-1-methylpyrrolidin-2-yl)-1H-pyrrolo[3,2-c]pyridin-6-yl)-1-(8-((2-(2,6-dioxopiperidin-3-yl)-1,3-dioxoisoindolin-4-yl)amino)octyl)-3-methyl-1H-indazole-6-carboxamide;
1-(4-{4-[2-(2,6-dioxopiperidin-3-yl)-1,3-dioxoisoindol-5-yl]piperazin-1-yl}butyl)-3-methyl-N-{2-[(2R)-1-methylpyrrolidin-2-yl]-1H-pyrrolo[3,2-c]pyridin-6-yl}indazole-6-carboxamide;
1-(4-{4-[2-(2,6-dioxopiperidin-3-yl)-1,3-dioxoisoindol-5-yl]piperazin-1-yl}butyl)-3-methyl-N-{2-[(2S)-1-methylpyrrolidin-2-yl]-1H-pyrrolo[3,2-c]pyridin-6-yl}indazole-6-carboxamide;
1-(4-((2-(2,6-dioxopiperidin-3-yl)-1,3-dioxoisoindolin-4-yl)amino)butyl)-3-methyl-N-(2-((R)-1-methylpyrrolidin-2-yl)-1H-pyrrolo[3,2-c]pyridin-6-yl)-1H-indazole-6-carboxamide;
1-(6-{[2-(2,6-dioxopiperidin-3-yl)-1,3-dioxoisoindol-4-yl]amino}hexyl)-3-methyl-N-{2-[(2R)-1-methylpyrrolidin-2-yl]-1H-pyrrolo[3,2-c]pyridin-6-yl}indazole-6-carboxamide;

1-(5-(4-(2-(2,6-dioxopiperidin-3-yl)-6-fluoro-1,3-dioxoisoindolin-5-yl)piperazin-1-yl)pentyl)-3-methyl-N-(2-((R)-1-methylpyrrolidin-2-yl)-1H-pyrrolo[3,2-c]pyridin-6-yl)-1H-indazole-6-carboxamide;
1-(2-(2-(4-(2-(2,6-dioxopiperidin-3-yl)-6-fluoro-1,3-dioxoisoindolin-5-yl)piperazin-1-yl)ethoxy)ethyl)-3-methyl-N-(2-((*R*)-1-methylpyrrolidin-2-yl)-1*H*-pyrrolo[3,2-c]pyridin-6-yl)-1*H*-indazole-6-carboxamide;
1-(2-(2-(2-((2-(2,6-dioxopiperidin-3-yl)-1,3-dioxoisoindolin-4-yl)amino)ethoxy)ethoxy)ethyl)-3-methyl-*N*-(2-((R)-1-methylpyrrolidin-2-yl)-1*H*-pyrrolo[3,2-c]pyridin-6-yl)-1*H*-indazole-6-carboxamide;
1-(2-(2-((2-(2,6-dioxopiperidin-3-yl)-1,3-dioxoisoindolin-4yl)amino)ethoxy)ethyl)-3-methyl-*N*-(2-((*R*)-1-methylpyrrolidin-2-yl)-1*H*-pyrrolo[3,2-c]pyridin-6-yl)-1*H*-indazole-6-carboxamide;
1-(3-(1-(2-(2,6-dioxopiperidin-3-yl)-1,3-dioxoisoindolin-4-yl)piperidin-4-yl)propyl)-3-methyl-*N*-(2-((*R*)-1-methylpyrrolidin-2-yl)-1*H*-pyrrolo[3,2-*c*]pyridin-6-yl)-1*H*-indazole-6-carboxamide;
1-(3-(1-(4-((2,6-dioxopiperidin-3-yl)carbamoyl)-2-fluorophenyl)piperidin-4-yl)propyl)-3-methyl-N-(2-((*R*)-1-methylpyrrolidin-2-yl)-1H-pyrrolo[3,2-c]pyridin-6-yl)-1H-indazole-6-carboxamide;
1-(5-(1-(2-(2,6-dioxopiperidin-3-yl)-1,3-dioxoisoindolin-4-yl)piperidin-4-yl)pentyl)-3-methyl-*N*-(2-((*R*)-1-methylpyrrolidin-2-yl)-1*H*-pyrrolo[3,2-*c*]pyridin-6-yl)-1*H*-indazole-6-carboxamide;
1-(7-(2-(2, 6-dioxopiperidin-3-yl)-1-oxoisoindolin-4-yl) hept-6-yn-1-yl)-3-methyl-*N*-(2-((*R*)-1-methylpyrrolidin-2-yl)-1*H*-pyrrolo [3, 2-c] pyridin-6-yl)-1*H*-indazole-6-carboxamide;
1-(3-((3-(2-(2,6-dioxopiperidin-3-yl)-1-oxoisoindolin-4-yl)prop-2-yn-1-yl)oxy)propyl)-3-methyl-*N*-(2-((*R*)-1-methylpyrrolidin-2-yl)-1*H*-pyrrolo[3,2-c]pyridin-6-yl)-1*H*-indazole-6-carboxamide;
1-(5-(2-(2,6-dioxopiperidin-3-yl)-1-oxoisoindolin-4-yl)pent-4-yn-1-yl)-3-methyl-*N* (2-((*R*)-1-methylpyrrolidin-2-yl)-1*H*-pyrrolo[3,2-c]pyridin-6-yl)-1*H*-indazole-6-carboxamide;
1-(7-(2-(2,6-dioxopiperidin-3-yl)-1,3-dioxoisoindolin-4-yl)hept-6-yn-1-yl)-3-methyl-*N*-(2-((*R*)-1-methylpyrrolidin-2-yl)-1*H*-pyrrolo[3,2-c]pyridin-6-yl)-1*H*-indazole-6-carboxamide;
1-(7-(3-((2,6-dioxopiperidin-3-yl)carbamoyl)-2-fluorophenyl)hept-6-yn-1-yl)-3-methyl-*N*-(2-((*R*)-1-methylpyrrolidin-2-yl)-1*H*-pyrrolo[3,2-c]pyridin-6-yl)-1*H*-indazole-6-carboxamide;
1-(7-(3-((2,6-dioxopiperidin-3-yl)carbamoyl)-2-methylphenyl)hept-6-yn-1-yl)-3-methyl-*N*-(2-((*R*)-1-methylpyrrolidin-2-yl)-1*H*-pyrrolo[3,2-c]pyridin-6-yl)-1*H*-indazole-6-carboxamide;
1-(8-((2-(2,6-dioxopiperidin-3-yl)-1,3-dioxoisoindolin-4-yl)amino)octyl)-*N*-(2-((*R*)-1-methylpyrrolidin-2-yl)-1*H*-pyrrolo[3,2-c]pyridin-6-yl)-1*H*-indazole-5-carboxamide;
1-(8-((2-(2,6-dioxopiperidin-3-yl)-1,3-dioxoisoindolin-4-yl)amino)octyl)-N-(2-((S)-1-methylpyrrolidin-2-yl)-1H-pyrrolo[3,2-c]pyridin-6-yl)-1H-indazole-5-carboxamide;
1-(9-(2-(2,6-dioxopiperidin-3-yl)-1-oxoisoindolin-4-yl)non-8-yn-1-yl)-*N*-(2-((*R*)-1-methylpyrrolidin-2-yl)-1*H*-pyrrolo [3,2-*c*]pyridin-6-yl)-1*H*-indazole-5-carboxamide;
1-(9-(1-(2,6-dioxopiperidin-3-yl)-3-methyl-2-oxo-2,3-dihydro-1*H*-benzo[d]imidazol-5-yl)non-8-yn-1-yl)-3-methyl-*N*-(2-((R)-1-methylpyrrolidin-2-yl)-1*H*-pyrrolo[3,2-c]pyridin-6-yl)-1*H*-indazole-6-carboxamide;
1-((E)-9-chloro-9-(1-(2,6-dioxopiperidin-3-yl)-3-methyl-2-oxo-2,3-dihydro-1*H*-benzo[d]imidazol-5-yl)non-8-en-1-yl)-3-methyl-*N*-(2-((*R*)-1-methylpyrrolidin-2-yl)-1*H*-pyrrolo[3,2-c]pyridin-6-yl)-1H-indazole-6-carboxamide;
1-((Z)-9-chloro-9-(1-(2,6-dioxopiperidin-3-yl)-3-methyl-2-oxo-2,3-dihydro-1*H*-benzo[d]imidazol-5-yl)non-8-en-1-yl)-3-methyl-*N*-(2-((*R*)-1-methylpyrrolidin-2-yl)-1*H*-pyrrolo[3,2-c]pyridin-6-yl)-1*H*-indazole-6-carboxamide;
1-(9-chloro-9-(1-(2,6-dioxopiperidin-3-yl)-3-methyl-2-oxo-2,3-dihydro-1*H*-benzo[d]imidazol-5-yl)non-8-en-1-yl)-3-methyl-*N*-(2-((*R*)-1-methylpyrrolidin-2-yl)-1*H*-pyrrolo[3,2-*c*]pyridin-6-yl)-1*H*-indazole-6-carboxamide;
1-(9-(6-(((*S*)-2,6-dioxopiperidin-3-yl)carbamoyl)pyridin-3-yl)non-8-yn-1-yl)-3-methyl-N-(2-((*R)*-1-methylpyrrolidin-2-yl)-1H-pyrrolo[3,2-c]pyridin-6-yl)-1H-indazole-6-carboxamide;
1-(9-(6-(((*S*)-2,6-dioxopiperidin-3-yl)carbamoyl)pyridin-2-yl)non-5-yn-1-yl)-3-methyl-N-(2-((*R*)-1-methylpyrrolidin-2-yl)-1H-pyrrolo[3,2-c]pyridin-6-yl)-1H-indazole-6-carboxamide;
1-(9-(2-(((*S*)-2,6-dioxopiperidin-3-yl)carbamoyl)pyridin-4-yl)non-8-yn-1-yl)-3-methyl-N-(2-((*R*)-1-methylpyrrolidin-2-yl)-1H-pyrrolo[3,2-c]pyridin-6-yl)-1H-indazole-6-carboxamide;
1-(9-(2-(2,6-dioxopiperidin-3-yl)-1-oxoisoindolin-4-yl)non-8-yn-1-yl)-3-methyl-*N*-(2-((R)-1-(methyl-d3)pyrrolidin-2-yl)-1*H*-pyrrolo[3,2-c]pyridin-6-yl)-1*H*-indazole-6-carboxamide;
1-(8-(2-(2,6-dioxopiperidin-3-yl)-1-oxoisoindolin-4-yl)oct-7-yn-1-yl)-3-methyl-*N*-(2-((R)-1-methylpyrrolidin-2-yl)-1*H*-pyrrolo[3,2-c]pyridin-6-yl)-1*H*-indazole-6-carboxamide;
1-(6-(2-(2,6-dioxopiperidin-3-yl)-1-oxoisoindolin-4-yl)hex-5-yn-1-yl)-3-methyl-*N*-(2-((*R*)-1-methylpyrrolidin-2-yl)-1*H*-pyrrolo[3,2-c]pyridin-6-yl)-1*H*-indazole-6-carboxamide;
1-(5-(4-(2-(2,6-dioxopiperidin-3-yl)-1,3-dioxoisoindolin-4-yl)piperazin-1-yl)pentyl)-3-methyl-*N*-(2-((*R*)-1-methylpyrrolidin-2-yl)-1*H*-pyrrolo[3,2-*c*]pyridin-6-yl)-1*H*-indazole-6-carboxamide;
1-(9-(2-(2,6-dioxopiperidin-3-yl)-1-oxoisoindolin-4-yl)non-8-yn-1-yl)-N-(2-((R)-1 - ethylpyrrolidin-2-yl)-1H-pyrrolo [3,2-c]pyridin-6-yl)-3-methyl-1H-indazole-6-carboxamide;
1-(5-((3-(2-(2,6-dioxopiperidin-3-yl)-1-oxoisoindolin-4-yl)prop-2-yn-1-yl)oxy)pentyl)-3-methyl-N-(2-((R)-1-methylpyrrolidin-2-yl)-1*H*-pyrrolo[3,2-c]pyridin-6-yl)-1*H*-indazole-6-carboxamide;

1-(2-((6-(2-(2,6-dioxopiperidin-3-yl)-1-oxoisoindolin-4-yl)hex-5-yn-1-yl)oxy)ethyl)-3-methyl-*N*-(2-((*R*)-1-methylpyrrolidin-2-yl)-1*H*-pyrrolo[3,2-*c*]pyridin-6-yl)-1*H*-indazole-6-carboxamide;
1-(9-(3-(((S)-2,6-dioxopiperidin-3-yl)carbamoyl)-2-fluorophenyl)non-8-yn-1-yl)-3-methyl-*N*-(2-((R)-1-methylpyrrolidin-2-yl)-1*H*-pyrrolo[3,2-*c*]pyridin-6-yl)-1*H*-indazole-6-carboxamide;
1-(9-(2-(2,6-dioxopiperidin-3-yl)-7-fluoro-1-oxoisoindolin-4-yl)non-8-yn-1-yl)-3-methyl-*N*-(2-((*R*)-1-methylpyrrolidin-2-yl)-1*H*-pyrrolo[3,2-*c*]pyridin-6-yl)-1*H*-indazole-6-carboxamide;
*N*-(3-chloro-2-((R)-1-methylpyrrolidin-2-yl)-1*H*-pyrrolo[3,2-c]pyridin-6-yl)-1-(9-(2-(2,6-dioxopiperidin-3-yl)-1-oxoisoindolin-4-yl)non-8-yn-1-yl)-3-methyl-1*H*-indazole-6-carboxamide;
1-(10-(2-(2,6-dioxopiperidin-3-yl)-1-oxoisoindolin-4-yl) dec-9-yn-1-yl)-3-methyl-*N*-(2-((R)-1-methylpyrrolidin-2-yl)-1*H*-pyrrolo[3,2-c]pyridin-6-yl)-1*H*-indazole-6-carboxamide;
1-(9-(2-(2, 6-dioxopiperidin-3-yl)-1-oxoisoindolin-4-yl) non-8-yn-1-yl)-*N*-(2-((R)-1-methylpyrrolidin-2-yl)-1*H*-pyrrol [3,2-c]pyridin-6-yl)-1*H*-indazole-6-carboxamide;
1-(9-(2-(2,6-dioxopiperidin-3-yl)-1-oxoisoindolin-4-yl)non-8-yn-1-yl)-3-methyl-*N*-(2-((*R*)-5-methyl-5-azaspiro[2.4]heptan-6-yl)-1*H*-pyrrolo[3,2-c]pyridin-6-yl)-1*H*-indazole-6-carboxamide;
1-(9-(2-(2,6-dioxopiperidin-3-yl)-1-oxoisoindolin-4-yl)non-8-yn-1-yl)-3-methyl-*N*-(3-methyl-2-((*R*)-1-methylpyrrolidin-2-yl)-1*H*-pyrrolo[3,2-c]pyridin-6-yl)-1*H*-indazole-6-carboxamide;
1-(9-(2-(2,6-dioxopiperidin-3-yl)-1-oxoisoindolin-4-yl)non-8-yn-1-yl)-5-fluoro-3-methyl-*N*-(2-((*R*)-1-methylpyrrolidin-2-yl)-1*H*-pyrrolo[3,2-*c*]pyridin-6-yl)-1*H*-indazole-6-carboxamide;
3-cyclopropyl-1-(9-(2-(2, 6-dioxopiperidin-3-yl)-1-oxoisoindolin-4-yl) non-8-yn-1-yl)-*N*-(2-((*R*)-1-methylpyrrolidin-2-yl)-1*H*-pyrrolo [3, 2-*c*] pyridin-6-yl)-1*H*-indazole-6-carboxamide;
1-(9-(2-(2,6-dioxopiperidin-3-yl)-6-fluoro-1-oxoisoindolin-5-yl)non-8-yn-1-yl)-3-methyl-*N*-(2-((*R*)-1-methylpyrrolidin-2-yl)-1*H*-pyrrolo[3,2-*c*]pyridin-6-yl)-1*H*-indazole-6-carboxamide;
1-(9-(3-(2, 6-dioxopiperidin-3-yl)-2-fluorophenyl) non-8-yn-1-yl)-3-methyl-*N*-(2-((*R*)-1-methylpyrrolidin-2-yl)-1*H*-pyrrolo[3,2-*c*]pyridin-6-yl)-1*H*-indazole-6-carboxamide;
1-(9-(6-(2, 6-dioxopiperidin-3-yl) pyridin-2-yl) non-8-yn-1-yl)-3-methyl-*N*-(2-((*R*)-1-methylpyrrolidin-2-yl)-1*H*-pyrrolo [3,2-*c*]pyridin-6-yl)-1*H*-indazole-6-carboxamide; (*R*)-1-(9-(3-(2, 4-dioxotetrahydropyrimidin-1(2*H*)-yl)-2-fluorophenyl) non-8-yn-1-yl)-3-methyl-*N*-(2-(1-methylpyrrolidin-2-yl)-1H-pyrrolo [3, 2-c] pyridin-6-yl)-1*H*-indazole-6-carboxamide;
*(*R)-1-(9-(6-(2,4-dioxotetrahydropyrimidin-1(2*H*)-yl)pyridin-2-yl)non-8-yn-1-yl)-3-methyl-*N*-(2-(1-methylpyrrolidin-2-yl)-1*H*-pyrrolo[3,2-c]pyridin-6-yl)-1*H*-indazole-6-carboxamide;
(*R*)-1-(9-(3-(2,4-dioxotetrahydropyrimidin-1(2*H*)-yl)-5-fluoro-1-methyl-1*H*-indazol-6-yl)non-8-yn-1-yl)-3-methyl-*N*-(2-(1-methylpyrrolidin-2-yl)-1*H*-pyrrolo[3,2-c]pyridin-6-yl)-1*H*-indazole-6-carboxamide;
*N*-(3-cyano-2-((*R*)-1-methylpyrrolidin-2-yl)-1*H*-pyrrolo[3,2-c]pyridin-6-yl)-1-(9-(2-(2,6-dioxopiperidin-3-yl)-1-oxoisoindolin-4-yl)non-8-yn-1-yl)-3-methyl-1*H*-indazole-6-carboxamide;
*N*-(3-chloro-2-((*R*)-1-methylpyrrolidin-2-yl)-1*H*-pyrrolo[3,2-*c*]pyridin-6-yl)-1-(9-(2-(2,6-dioxopiperidin-3-yl)-7-fluoro-1-oxoisoindolin-4-yl)non-8-yn-1-yl)-3 -methyl-1*H*-indazole-6-carboxamide;
1-(1-(3-(2-(2,6-dioxopiperidin-3-yl)-1-oxoisoindolin-4-yl)prop-2-yn-1-yl)piperidin-4-yl)-3-methyl-*N*-(2-((*R*)-1-methylpyrrolidin-2-yl)-1*H*-pyrrolo[3,2-*c*]pyridin-6-yl)-1*H*-indazole-6-carboxamide;
1-(1-((1-(2-(2,6-dioxopiperidin-3-yl)-6-fluoro-1,3-dioxoisoindolin-5-yl)piperidin-4-yl)methyl)piperidin-4-yl)-3-methyl-*N*-(2-((*R*)-1-methylpyrrolidin-2-yl)-1*H*-pyrrolo[3,2-c]pyridin-6-yl)-1*H*-indazole-6-carboxamide;
1-(1-((1-(2-(2,6-dioxopiperidin-3-yl)-1,3-dioxoisoindolin-5-yl)piperidin-4-yl)methyl)piperidin-4-yl)-3-methyl-*N*-(2-((*R*)-1-methylpyrrolidin-2-yl)-1*H*-pyrrolo[3,2-*c*]pyridin-6-yl)-1*H*-indazole-6-carboxamide;
1-(1-((1-(2-(2,6-dioxopiperidin-3-yl)-1,3-dioxoisoindolin-4-yl)piperidin-4-yl)methyl)piperidin-4-yl)-3-methyl-*N*-(2-((*R*)-1-methylpyrrolidin-2-yl)-1*H*-pyrrolo[3,2-c]pyridin-6-yl)-1*H*-indazole-6-carboxamide;
1-(3-(2-(2,6-dioxopiperidin-3-yl)-1-oxoisoindolin-4-yl)prop-2-yn-1-yl)-3-methyl-*N*-(2-((*R*)-1-methylpyrrolidin-2-yl)-1*H*-pyrrolo[3,2-*c*]pyridin-6-yl)-1*H*-indazole-6-carboxamide;
1-(6-(1-(6-(((S)-2,6-dioxopiperidin-3-yl)carbamoyl)pyridin-2-yl)piperidin-4-yl)hexyl)-3-methyl-N-(2-((R)-1-methylpyrrolidin-2-yl)-1H-pyrrolo[3,2-c]pyridin-6-yl)-1H-indazole-6-carboxamide; and
1-(4-(5-(2-(2,6-dioxopiperidin-3-yl)-1-oxoisoindolin-4-yl) pent-4-yn-1-yl) cyclohexyl)-3-methyl-N-(2-((R)-1-methylpyrrolidin-2-yl)-1H-pyrrolo[3,2-c] pyridin-6-yl)-1H-indazole-6-carboxamide;

and the pharmaceutically acceptable salts thereof.

*MLLT1 and/or MLLT3 inhibitors*

In a separate aspect of the present invention, compounds of formula (I') are provided that are potent inhibitors of MLLT1 and/or MLLT3. Accordingly, the invention provides a compound which is a bicyclic compound of formula (I') or a pharmaceutically acceptable salt thereof:

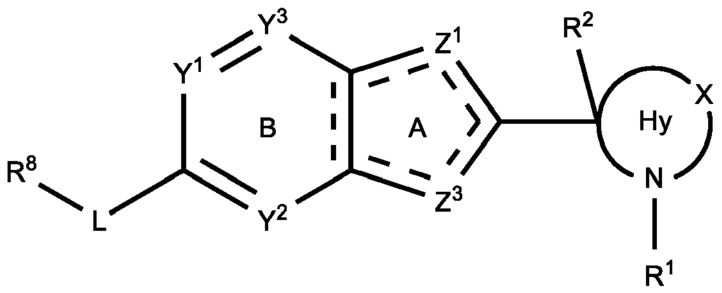

(I')

wherein:

one of $Z^1$ and $Z^3$ is -N(H)- and the other is N or -C($R^4$)-, $Y^1$ is N, $Y^2$ is N or -C($R^6$)-, and $Y^3$ is N or -C($R^5$)-;

Hy is a 4- to 7-membered heterocyclic ring containing X and at least one N atom, wherein: ring Hy is linked to ring A via a C atom within ring Hy, said C atom also being linked to $R^2$; a N atom within ring Hy is substituted by $R^1$; X is a bond, -N($R^{11}$)-, O, S, -S(O)$_2$-, -S(O)(N$R^{11}$)-, or -C($R^{11}$)$_2$-; and the rest of ring Hy is unsubstituted or substituted by one or two $R^3$;

L is -C(O)N(H)-, wherein the C atom of L is bonded to $R^8$, and the N atom of L is bonded to ring B;

$R^1$ is H, $C_{1-4}$ cycloalkyl, or $C_{1-4}$ alkyl which is itself unsubstituted or substituted with one $C_{1-4}$ alkoxy or one, two or three halo;

$R^2$ is H or methyl;

each $R^3$ is independently selected from $C_{1-4}$ alkyl, $C_{1-4}$ alkoxy, phenyl, a 5- to 6-membered heteroaryl ring and halo, or (i) two $R^3$ linked to adjacent C atoms in ring Hy form, together with the C atoms to which they are attached, a $C_{5-6}$ cycloalkyl ring, or (ii) two $R^3$ linked to the same C atom in ring Hy form, together with the C atom to which they are attached, a $C_{3-6}$ cycloalkyl ring or a $C_{3-6}$ heterocycloalkyl ring;

$R^4$ and $R^6$ are independently selected from H, halo, CN, $C_{1-4}$ alkoxy, and $C_{1-4}$ alkyl which is itself unsubstituted or substituted by one, two or three halo;

$R^5$ is selected from H, halo, $C_{1-4}$ alkoxy, $C_{3-5}$ cycloalkyl and $C_{1-4}$ alkyl which is itself unsubstituted or substituted by one, two or three halo;

at least one of $R^4$, $R^5$ and $R^6$ is not H;

$R^8$ is an 8- to 10-membered heteroaryl ring, the group $R^8$ being unsubstituted or substituted by one, two or three substituents independently selected from halo, $C_{1-4}$ alkoxy, $R^{10}$, -($C_{1-4}$ alkylene)-$R^{10}$, =O, -CN, and $C_{1-4}$ alkyl which is itself unsubstituted or substituted by one or two $R^9$;

each $R^9$ is independently selected from halo and $C_{1-4}$ alkoxy;

$R^{10}$ is a group selected from a 5- to 6-membered heteroaryl ring, a 3- to 6-membered cycloalkyl ring, a 4- to 6-membered heterocyclyl ring, and a phenyl ring, the group $R^{10}$ being unsubstituted or substituted by one or two substituents independently selected from $C_{1-4}$ alkyl, $C_{1-4}$ alkoxy, and halo;

each $R^{11}$ is independently selected from H, $C_{1-4}$ alkyl, and $C_{1-4}$ cycloalkyl.

Typically, in formula (I'), ring Hy is a 5- to 6- membered heterocyclyl ring containing X and at least one N atom, for instance one or two N atoms. X is typically a bond, -N($R^{11}$)-, O or -C($R^{11}$)$_2$-. Preferably, X is a bond, -N(Me)-, O or -$CH_2$-, most preferably X is a bond. In particular, when ring Hy is a 4- or 5-membered heterocyclyl ring, X is most preferably a bond. For example, ring Hy may be selected from a pyrrolidinyl, piperidinyl, morpholinyl or diazinanyl ring. Preferably, ring Hy is a 5-membered heterocyclyl ring containing X and at least one N atom, for instance one N atom. For example, ring Hy may be a pyrrolidinyl ring.

Ring Hy is linked to ring A via a C atom within ring Hy, said C atom also being linked to $R^2$. A N atom within ring Hy is substituted by $R^1$. Typically, the N atom of ring Hy which is substituted by $R^1$ is adjacent to the C atom of ring Hy that is bonded to ring A.

Typically, in formula (I'), $R^1$ is H, $C_{1-4}$ cycloalkyl, or $C_{1-4}$ alkyl which is itself unsubstituted or substituted with one $C_{1-4}$ alkoxy or one, two or three halo, preferably with one $C_{1-4}$ alkoxy, for example $R^1$ may be H or $C_{1-2}$ alkyl which is unsubstituted or substituted with one $C_{1-2}$ alkoxy or one, two or three halo, preferably with one $C_{1-2}$ alkoxy, or $R^1$ may be H or $C_{1-2}$ alkyl which is unsubstituted or substituted with one $C_{1-2}$ alkoxy. Preferably, $R^1$ is H, methyl, ethyl or methoxyethyl, more preferably methyl or ethyl. Most preferably, $R^1$ is methyl.

In formula (I'), $R^2$ is H or methyl. Preferably, $R^2$ is H.

Typically, in formula (I'), each $R^{11}$ is independently selected from H and methyl. When X is -N($R^{11}$)- or -S(O)

$(NR^{11})$-, $R^{11}$ is preferably methyl. When X is -$C(R^{11})_2$-, each $R^{11}$ is preferably H.

Aside from groups $R^1$ and $R^2$, ring Hy may further be unsubstituted or substituted by one or two $R^3$. Typically, each $R^3$ is independently selected from $C_{1-4}$ alkyl, $C_{1-4}$ alkoxy and halo, or (i) two $R^3$ linked to adjacent C atoms in ring Hy form, together with the C atoms to which they are attached, a $C_{5-6}$ cycloalkyl ring, or (ii) two $R^3$ linked to the same C atom in ring Hy form, together with the C atom to which they are attached, a $C_{3-6}$ cycloalkyl ring. More typically, each $R^3$ is independently selected from methyl, ethyl, t-butyl, methoxy and fluoro, or (i) two $R^3$ linked to adjacent C atoms in ring Hy form, together with the C atoms to which they are attached, a cyclohexyl ring, or (ii) two $R^3$ linked to the same C atom in ring Hy form, together with the C atom to which they are attached, a cyclopropyl or cyclopentyl ring. Preferably, ring Hy is substituted by no $R^3$ groups.

In another typical emobidment, each $R^3$ is independently selected from methyl, ethyl, t-butyl, methoxy and fluoro, or (i) two $R^3$ linked to adjacent C atoms in ring Hy form, together with the C atoms to which they are attached, a cyclohexyl ring, or (ii) two $R^3$ linked to the same C atom in ring Hy form, together with the C atom to which they are attached, a cyclopropyl ring or a cyclopentyl ring. Preferably, ring Hy is substituted by two $R^3$ groups which are both $C_{1-4}$ alkyl, for instance both being methyl, or one $R^3$ group which is $C_{1-4}$ alkyl, for instance methyl, ethyl or t-butyl.

Typically, therefore, in formula (I'), ring Hy is a 5- to 6- membered heterocyclyl ring containing X and at least one N atom, for example a pyrrolidinyl, piperidinyl, morpholinyl or diazinanyl ring; the N atom of ring Hy which is substituted by $R^1$ is adjacent to the C atom of ring Hy that is bonded to ring A; $R^1$ is H or $C_{1-2}$ alkyl which is unsubstituted or substituted with one $C_{1-2}$ alkoxy or one, two or three halo, preferably with one $C_{1-2}$ alkoxy; $R^2$ is H or methyl; X is a bond, -$N(R^{11})$-, O or -$C(R^{11})_2$-; $R^{11}$ is H or methyl; ring Hy is further unsubstituted or substituted by one or two $R^3$; and each $R^3$ is independently selected from $C_{1-4}$ alkyl, $C_{1-4}$ alkoxy and halo, or (i) two $R^3$ linked to adjacent C atoms in ring Hy form, together with the C atoms to which they are attached, a $C_{5-6}$ cycloalkyl ring, or (ii) two $R^3$ linked to the same C atom in ring Hy form, together with the C atom to which they are attached, a $C_{3-6}$ cycloalkyl ring.

Preferably, in formula (I'), ring Hy is a pyrrolidinyl ring; the N atom of ring Hy which is substituted by $R^1$ is adjacent to the C atom of ring Hy that is bonded to ring A; $R^1$ is methyl or ethyl, more preferably methyl; $R^2$ is H; and ring Hy is substituted by no $R^3$ groups, i.e. ring Hy is not further substituted and carries substituent $R^1$ only. Alternatively, in formula (I), ring Hy is a pyrrolidinyl ring; the N atom of ring Hy which is substituted by $R^1$ is adjacent to the C atom of ring Hy that is bonded to ring A; $R^1$ is H, methyl, ethyl or methoxyethyl, more preferably methyl; $R^2$ is H; and ring Hy is further substituted by two $R^3$ groups which are both $C_{1-4}$ alkyl, for instance both being methyl, or one $R^3$ group which is $C_{1-4}$ alkyl, for instance methyl, ethyl or t-butyl.

Typically, in formula (I'), no more than two of $Y^1$, $Y^2$ and $Y^3$ are N. Preferably, no more than one of $Y^1$, $Y^2$ and $Y^3$ are N. Most preferably, $Y^1$ is N, $Y^2$ is -$C(R^6)$-, and $Y^3$ is -$C(R^5)$-.

Typically, $Z^1$ is -$C(R^4)$-, $Z^3$ is -N(H)-, and $Y^1$ is N. Thus, the bicyclic structure formed by rings A and B has the structure:

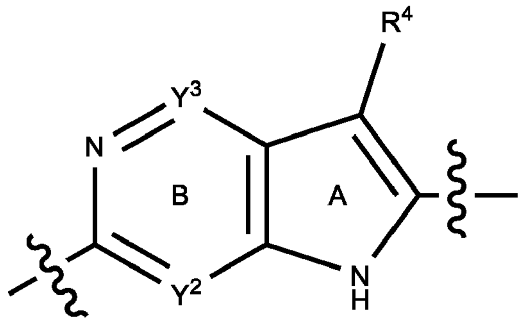

In one preferred embodiment, $Z^1$ is -$C(R^4)$-, $Z^3$ is -N(H)-, $Y^1$ is N, $Y^2$ is -$C(R^6)$-, and $Y^3$ is -$C(R^5)$-, such that the bicyclic structure formed by rings A and B has the structure:

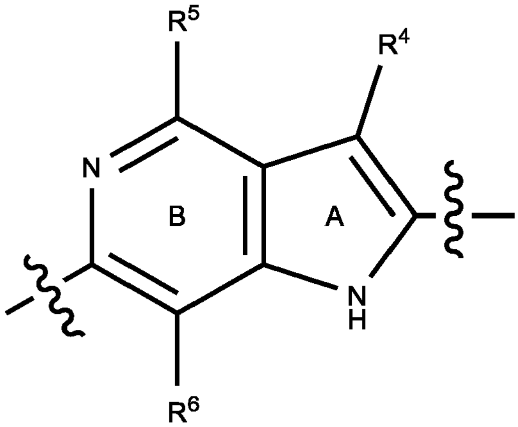

Typically, in formula (I'), one of $R^4$, $R^5$ and $R^6$ is selected from halo, CN, $C_{1-4}$ alkoxy, and $C_{1-4}$ alkyl which is itself unsubstituted or substituted by one, two or three halo, and the rest are H, with the proviso that $R^5$ and $R^6$ arer not CN. Preferably, one of $R^4$, $R^5$ and $R^6$ is selected from halo (e.g. fluoro or chloro), CN, methoxy, methyl and trifluoromethyl, and the rest are H, with the proviso that $R^5$ and $R^6$ are not CN.

In one preferred embodiment, $R^5$ and $R^6$ are as defined herein and $R^4$ is selected from halo, CN, $C_{1-4}$ alkoxy, and $C_{1-4}$ alkyl which is itself unsubstituted or substituted by one, two or three halo. More preferably, $R^5$ and $R^6$ are as defined herein and $R^4$ is selected from halo, CN, $C_{1-4}$ alkyl and $CF_3$. More preferably, $R^5$ and $R^6$ are as defined herein and $R^4$ is selected from halo, and $CF_3$. Most preferably, $R^5$ and $R^6$ are as defined herein and $R^4$ is selected from F, Cl, and $CF_3$.

In one preferred embodiment, $R^5$ is selected from H, cyclopropyl, $C_{1-4}$ alkoxy, and $C_{1-4}$ alkyl, for example from H, $C_{1-4}$ alkoxy, and $C_{1-4}$ alkyl. More preferably, $R^5$ is selected from H, cyclopropyl, methoxy and methyl, for example H, methoxy and methyl. In one particularly preferred embodiment, $R^5$ is selected from cyclopropyl and $C_{1-4}$ alkyl, for example from $C_{1-4}$ alkyl. More preferably, $R^5$ is selected from cyclopropyl and methyl, for example methyl.

In one preferred embodiment, $R^6$ is H.

Typically, therefore, in formula (I'):

- $R^4$ is selected from halo, CN, $C_{1-4}$ alkoxy, and $C_{1-4}$ alkyl which is itself unsubstituted or substituted by one, two or three halo, and $R^5$ and $R^6$ when present are H; or
- $R^5$ is selected from cyclopropyl, $C_{1-4}$ alkoxy, and $C_{1-4}$ alkyl, typically from $C_{1-4}$ alkyl, and $R^4$ and $R^6$ when present are H.

Preferably, in formula (I'):

- $R^4$ is selected from F, Cl, and $CF_3$, and $R^5$ and $R^6$ when present are H;
- $R^5$ is selected from cyclopropyl and methyl, typically from methyl, and $R^4$ and $R^6$ when present are H.

In formula (I'), L is -C(O)N(H)-, wherein the C atom of L is bonded to $R^8$, and the N atom of L is bonded to ring B.

Typically, in formula (I'), $R^8$ may be selected from indazolyl, thieno[2,3-c]pyrazolyl, imidazo[4,5-b]pyridinyl, pyrazolo[3,4-b]pyridinyl, furo[2,3-c]pyridinyl, indolyl, benzoxazolyl, benzothiazolyl, [1,2,4]triazolo[4,3-a]pyridinyl, thieno [2,3-d]pyrimidinyl, 1,2,3-benzotriazolyl, imidazo[1,5-a]pyridinyl, imidazo[1,2-a]pyrazinyl, oxazolo[5,4-b]pyridinyl, quinolinyl, naphthyridinyl, isoquinolinyl, quinazolinyl, and quinoxalinyl. Preferably, $R^8$ is an 8- to 10-membered heteroaryl ring preferably containing one, two or three N atoms. More preferably, $R^8$ is an indazolyl group.

In some preferred embodiments, $R^8$ is a group selected from the following structures. For the avoidance of doubt, the following structures may be unsubstituted or substituted by one, two or three substituents as defined herein. [see below]

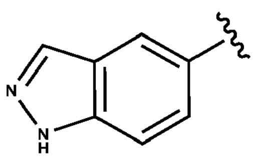
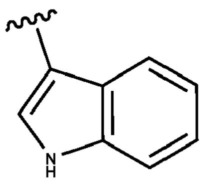
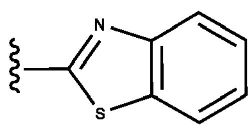

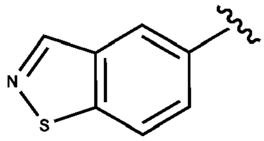 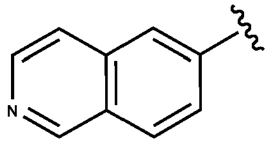 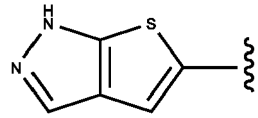

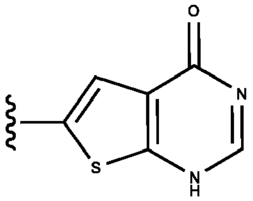 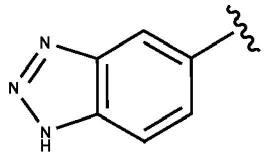 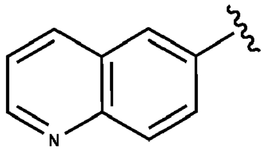

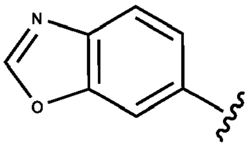 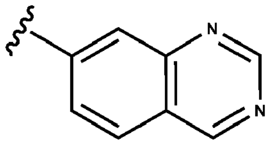 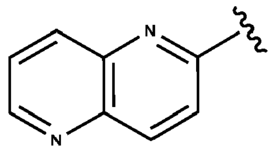

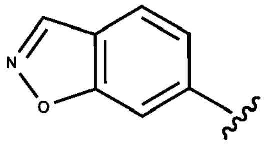 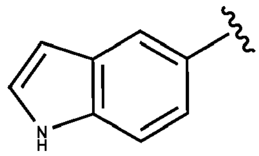 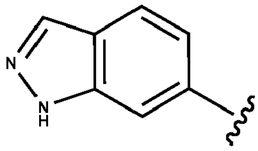 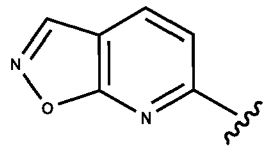

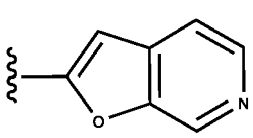 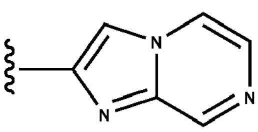 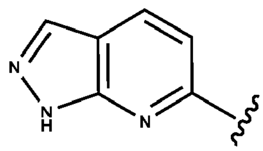

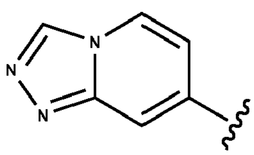 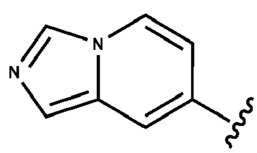 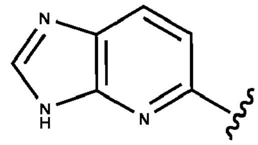

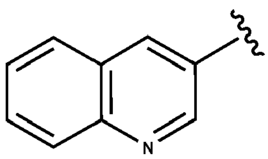 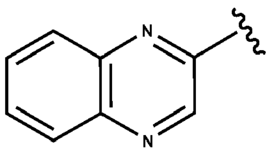 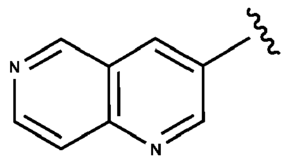

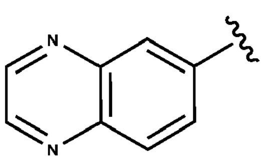 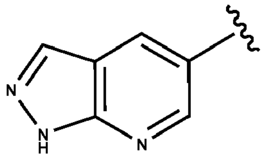

In formula (I'), $R^8$ is unsubstituted or substituted by one, two or three substituents, for instance one or two substituents. Typically, each substituent is independently selected from halo, $C_{1-4}$ alkoxy, $R^{10}$, $-(CH_2)-R^{10}$, =O, -CN, and $C_{1-4}$ alkyl which is itself unsubstituted or substituted by one or two groups selected from selected from halo and $C_{1-2}$ alkoxy. More typically, each substituent is independently selected from fluoro, chloro, methoxy, $R^{10}$, $-(CH_2)-R^{10}$, =O, -CN, and $C_{1-2}$ alkyl which is itself unsubstituted or substituted by one or two groups selected from selected from halo (e.g. fluoro) and methoxy. Preferably, each substituent is independently selected from chloro, fluoro, $R^{10}$ and methyl. Most preferably, each substituent is independently selected from fluoro and methyl, for instance methyl. $R^{10}$ is as defined herein.

Typically, in formula (I'), $R^{10}$ is a group selected from a 5- to 6-membered heteroaryl ring, a 3- to 6-membered

cycloalkyl ring, a 4- to 5-membered heterocyclyl ring, and a phenyl ring. The cycloalkyl, heteroaryl and heterocyclyl rings are as defined herein. Preferably, $R^{10}$ is selected from cyclopropyl, cyclobutyl, cyclopentyl, cyclohexyl, imidazolyl, oxadiazolyl, oxetanyl, phenyl, pyrazinyl, pyrazolyl, pyridazinyl, pyridinyl, pyrimidinyl, pyrrolidinyl, thiazinyl, thiazolyl and triazolyl, more preferably cyclopropyl and pyrazolyl.

Typically, in formula (I'), $R^{10}$ is unsubstituted or substituted by one or two substituents independently selected from fluoro, methyl and methoxy. Preferably, $R^{10}$ is unsubstituted.

Typically, therefore, in formula (I'), $R^8$ is an 8- to 10-membered heteroaryl ring; the group $R^8$ is unsubstituted or substituted by one, two or three substituents, for instance one or two substituents, each independently selected from halo, $C_{1-4}$ alkoxy, $R^{10}$, - $(CH_2)$-$R^{10}$, =O, -CN, and $C_{1-4}$ alkyl which is itself unsubstituted or substituted by one or two groups selected from selected from halo and $C_{1-2}$ alkoxy; wherein $R^{10}$ is a group selected from a 5- to 6-membered heteroaryl ring, a 3- to 6-membered cycloalkyl ring, a 4- to 5-membered heterocyclyl ring, and a phenyl ring; and $R^{10}$ is unsubstituted or substituted by one or two substituents independently selected from fluoro, methyl and methoxy.

Preferably, in formula (I'), $R^8$ is an 8- to 10-membered heteroaryl ring containing one, two or three N atoms, for example an indazolyl group; the group $R^8$ is unsubstituted or substituted by one or two substituents, each independently selected from fluoro, $R^{10}$ and methyl; $R^{10}$ is selected from cyclopropyl, cyclobutyl, cyclopentyl, cyclohexyl, imidazolyl, oxadiazolyl, oxetanyl, phenyl, pyrazinyl, pyrazolyl, pyridazinyl, pyridinyl, pyrimidinyl, pyrrolidinyl, thiazinyl, thiazolyl and triazolyl; and $R^{10}$ is unsubstituted.

In a typical embodiment of formula (I'), the bicyclic structure formed by rings A and B has the structure:

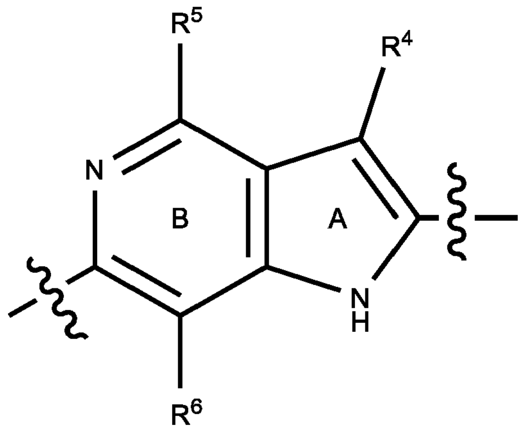

and:

- ring Hy is a 5- to 6- membered heterocyclyl ring containing X and at least one N atom, for example a pyrrolidinyl, piperidinyl, morpholinyl or diazinanyl ring; wherein the N atom of ring Hy which is substituted by $R^1$ is adjacent to the C atom of ring Hy that is bonded to ring A;
- $R^1$ is H or $C_{1-2}$ alkyl which is unsubstituted or substituted with one $C_{1-2}$ alkoxy or one, two or three halo, preferably with one $C_{1-2}$ alkoxy;
- $R^2$ is H or methyl;
- X is a bond, -N(Me)-, O or -$CH_2$-;
- ring Hy is further unsubstituted or substituted by one or two $R^3$; wherein each $R^3$ is independently selected from $C_{1-4}$ alkyl, $C_{1-4}$ alkoxy and halo, or (i) two $R^3$ linked to adjacent C atoms in ring Hy form, together with the C atoms to which they are attached, a $C_{5-6}$ cycloalkyl ring, or (ii) two $R^3$ linked to the same C atom in ring Hy form, together with the C atom to which they are attached, a $C_{3-6}$ cycloalkyl ring;
- $R^4$ is selected from CN, halo, $C_{1-4}$ alkoxy, and $C_{1-4}$ alkyl which is itself unsubstituted or substituted by one, two or three halo, and $R^5$ and $R^6$ when present are H;
- the C atom of L is bonded to $R^8$, and the N atom of L is bonded to ring B;
- $R^8$ is an 8- to 10-membered heteroaryl ring; wherein the group $R^8$ is unsubstituted or substituted by one, two or three substituents, for instance one or two substituents, each independently selected from halo, $C_{1-4}$ alkoxy, $R^{10}$, -$(CH_2)$-$R^{10}$, =O, -CN, and $C_{1-4}$ alkyl which is itself unsubstituted or substituted by one or two groups selected from halo and $C_{1-2}$ alkoxy; and
- $R^{10}$ is a group selected from a 5- to 6-membered heteroaryl ring, a 3- to 6-membered cycloalkyl ring, a 4- to 5-membered heterocyclyl ring, and a phenyl ring; wherein $R^{10}$ is unsubstituted or substituted by one or two substituents independently selected from fluoro, methyl and methoxy.

In a preferred embodiment of formula (I'), the bicyclic structure formed by rings A and B has the structure:

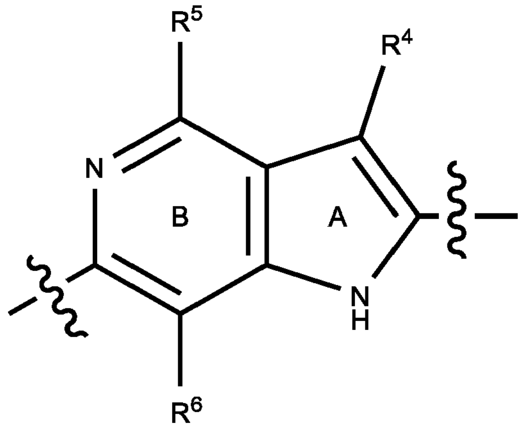

and:

ring Hy is a pyrrolidinyl ring; wherein the N atom of ring Hy which is substituted by $R^1$ is adjacent to the C atom of ring Hy that is bonded to ring A; $R^1$ is H, methyl, ethyl or
methoxyethyl, more preferably methyl; $R^2$ is H; and ring Hy is substituted by no $R^3$ groups;
$R^4$ is selected from CN, F, Cl, Me and $CF_3$, and $R^5$ and $R^6$ when present are H;
the C atom of L is bonded to $R^8$, and the N atom of L is bonded to ring B;
$R^8$ is an 8- to 10-membered heteroaryl ring containing one, two or three N atoms, for example an indazolyl group; the group $R^8$ is unsubstituted or substituted by one or two substituents, each independently selected from fluoro, $R^{10}$ and methyl; $R^{10}$ is selected from cyclopropyl, cyclobutyl, cyclopentyl, cyclohexyl, imidazolyl, oxadiazolyl, oxetanyl, phenyl, pyrazinyl, pyrazolyl, pyridazinyl, pyridinyl, pyrimidinyl, pyrrolidinyl, thiazinyl, thiazolyl and triazolyl; and $R^{10}$ is unsubstituted.

In a particular embodiment, the compound of the invention is a bicyclic compound of formula (II') or a pharmaceutically acceptable salt thereof:

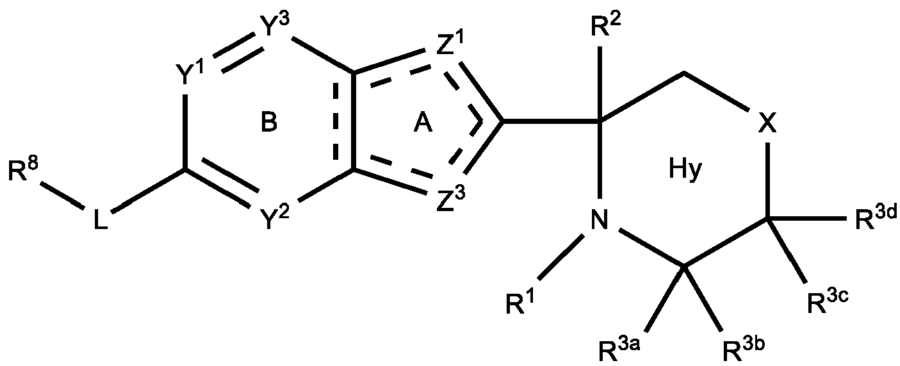

(II')

wherein:

$Z^1$, $Z^3$, $Y^1$, $Y^2$, $Y^3$, $R^1$, $R^2$, $R^8$, X and L are as defined in any one of the preceding claims;
$R^{3a}$ and $R^{3b}$ are independently selected from H, $C_{1-4}$ alkyl, phenyl, a 5- to 6-membered heteroaryl ring and $C_{1-4}$ alkoxy, or $R^{3a}$ and $R^{3b}$ form, together with the C atom to which they are attached, a $C_{3-6}$ cycloalkyl ring or a $C_{3-6}$ heterocycloalkyl ring,
$R^{3c}$ and $R^{3d}$ are independently selected from H, $C_{1-4}$ alkyl, halo, phenyl, a 5- to 6-membered heteroaryl ring and $C_{1-4}$ alkoxy, or $R^{3c}$ and $R^{3d}$ form, together with the C atom to which they are attached, a $C_{3-6}$ cycloalkyl ring or a $C_{3-6}$ heterocycloalkyl ring, or
$R^{3a}$ and $R^{3c}$ are H, and $R^{3b}$ and $R^{3d}$ form, together with the C atoms to which they are attached, a $C_{5-6}$ cycloalkyl ring, with the proviso that when X is -N($R^{11}$)-, O, S, -S(O)$_2$-or -S(O)(N$R^{11}$)-, then neither $R^{3c}$ nor $R^{3d}$ are halo;

wherein at least two of $R^{3a}$, $R^{3b}$, $R^{3c}$ and $R^{3d}$ are H.

Typically, in formula (II'): $R^{3a}$ and $R^{3b}$ are independently selected from H and methyl; $R^{3c}$ and $R^{3d}$ are independently selected from H, $C_{1-4}$ alkyl, halo and $C_{1-4}$ alkoxy, in particular from H, methyl, ethyl, fluoro, methoxy and t-butyl, or $R^{3c}$ and $R^{3d}$ form, together with the C atom to which they are attached, a $C_{5-6}$ cycloalkyl ring (in particular a cyclopentyl ring), with the proviso that when X is -N($R^{11}$)-, O, S, -S(O)$_2$- or - S(O)(NR$^{11}$)-, then neither $R^{3c}$ nor $R^{3d}$ are fluoro; or $R^{3a}$ and $R^{3c}$ are H, and $R^{3b}$ and $R^{3d}$ form, together with the C atoms to which they are attached, a $C_{5-6}$ cycloalkyl ring (in particular a cyclohexyl ring); wherein at least two of $R^{3a}$, $R^{3b}$, $R^{3c}$ and $R^{3d}$ are H.

Preferably, in formula (II'), $R^{3a}$, $R^{3b}$, $R^{3c}$ and $R^{3d}$ are all H.

In a typical embodiment of formula (II'), the bicyclic structure formed by rings A and B has the structure:

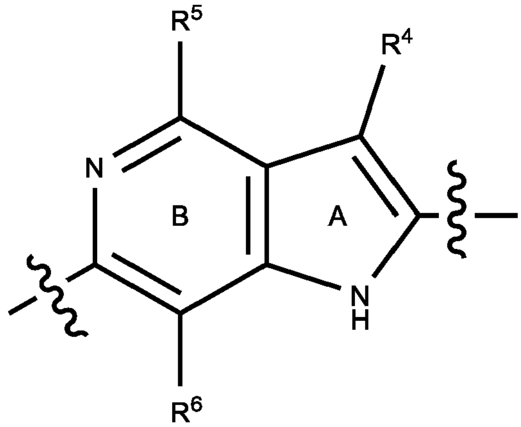

and:

$R^1$ is H or $C_{1-2}$ alkyl which is unsubstituted or substituted with one $C_{1-2}$ alkoxy or one, two or three halo, preferably with one $C_{1-2}$ alkoxy;

$R^2$ is H or methyl;

X is a bond, -N(Me)-, O or -CH$_2$-;

$R^{3a}$ and $R^{3b}$ are independently selected from H and methyl; $R^{3c}$ and $R^{3d}$ are independently selected from H, $C_{1-4}$ alkyl, halo and $C_{1-4}$ alkoxy, in particular from H, methyl, ethyl, fluoro, methoxy and t-butyl, or $R^{3c}$ and $R^{3d}$ form, together with the C atom to which they are attached, a $C_{5-6}$ cycloalkyl ring (in particular a cyclopentyl ring), with the proviso that when X is NR$^{11}$ or O, then neither $R^{3c}$ nor $R^{3d}$ are fluoro; or $R^{3a}$ and $R^{3c}$ are H, and $R^{3b}$ and $R^{3d}$ form, together with the C atoms to which they are attached, a $C_{5-6}$ cycloalkyl ring (in particular a cyclohexyl ring); wherein at least two of $R^{3a}$, $R^{3b}$, $R^{3c}$ and $R^{3d}$ are H;

$R^4$ is selected from CN, halo, $C_{1-4}$ alkoxy, and $C_{1-4}$ alkyl which is itself unsubstituted or substituted by one, two or three halo, and $R^5$ and $R^6$ when present are H;

the C atom of L is bonded to $R^8$, and the N atom of L is bonded to ring B;

$R^8$ is an 8- to 10-membered heteroaryl ring; wherein the group $R^8$ is unsubstituted or substituted by one, two or three substituents, for instance one or two substituents, each independently selected from halo, $C_{1-4}$ alkoxy, $R^{10}$, -(CH$_2$)-$R^{10}$, =O, -CN, and $C_{1-4}$ alkyl which is itself unsubstituted or substituted by one or two groups selected from selected from halo and $C_{1-2}$ alkoxy; and

$R^{10}$ is a group selected from a 5- to 6-membered heteroaryl ring, a 3- to 6-membered cycloalkyl ring, a 4- to 5-membered heterocyclyl ring, and a phenyl ring; wherein $R^{10}$ is unsubstituted or substituted by one or two substituents independently selected from fluoro, methyl and methoxy.

In a preferred embodiment of formula (II'), the bicyclic structure formed by rings A and B has the structure:

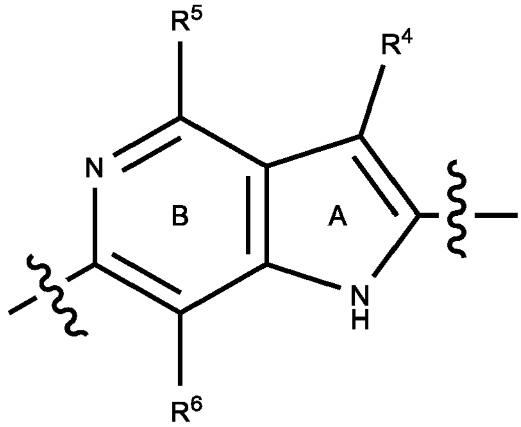

and:

X is absent;
$R^1$ is H, methyl, ethyl or methoxyethyl, more preferably methyl;
$R^2$ is H;
$R^{3a}$, $R^{3b}$ $R^{3c}$ and $R^{3d}$ are all H;
$R^4$ is selected from CN, F, Cl, Me and $CF_3$, and $R^5$ and $R^6$ when present are H;
the C atom of L is bonded to $R^8$, and the N atom of L is bonded to ring B;
$R^8$ is an 8- to 10-membered heteroaryl ring containing one, two or three N atoms, for example an indazolyl group; the group $R^8$ is unsubstituted or substituted by one or two substituents, each independently selected from fluoro, $R^{10}$ and methyl; $R^{10}$ is selected from cyclopropyl, cyclobutyl, cyclopentyl, cyclohexyl, imidazolyl, oxadiazolyl, oxetanyl, phenyl, pyrazinyl, pyrazolyl, pyridazinyl, pyridinyl, pyrimidinyl, pyrrolidinyl, thiazinyl, thiazolyl and triazolyl; and $R^{10}$ is unsubstituted.

In a particularly preferred embodiment, the compound of the invention is a bicyclic compound of formula (III') or a pharmaceutically acceptable salt thereof:

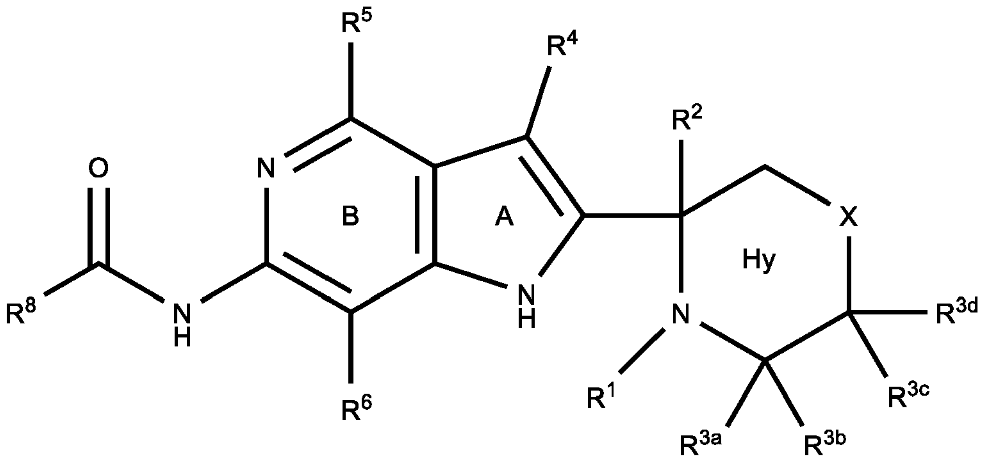

(III')

wherein $R^1$, $R^2$, $R^{3a}$, $R^{3b}$, $R^{3c}$, $R^{3d}$, X, $R^4$, $R^5$, $R^6$ and $R^8$ are as defined herein.

Typically, in the compounds of formula (III'):

$R^1$ is H or $C_{1-4}$ alkyl which is itself unsubstituted or substituted with $C_{1-4}$ alkoxy;
$R^2$ is H or methyl;
$R^{3a}$ and $R^{3b}$ are independently selected from H, $C_{1-4}$ alkyl, and $C_{1-4}$ alkoxy, or $R^{3a}$ and $R^{3b}$ form, together with the C atom to which they are attached, a $C_{3-6}$ cycloalkyl ring,
$R^{3c}$ and $R^{3d}$ are independently selected from H, $C_{1-4}$ alkyl, halo and $C_{1-4}$ alkoxy, or $R^{3c}$ and $R^{3d}$ form, together with the C atom to which they are attached, a $C_{3-6}$ cycloalkyl ring, or

$R^{3a}$ and $R^{3c}$ are H, and $R^{3b}$ and $R^{3d}$ form, together with the C atoms to which they are attached, a $C_{5-6}$ cycloalkyl ring, with the proviso that when X is O or -N(Me)-, then neither $R^{3c}$ nor $R^{3d}$ are halo;
wherein at least two of $R^{3a}$, $R^{3b}$, $R^{3c}$ and $R^{3d}$ are H;
X is a bond, -N(Me)-, O or $-CH_2-$;
$R^4$ is selected from CN, halo $C_{1-4}$ alkoxy, and $C_{1-4}$ alkyl, and $R^5$ and $R^6$ are H;
$R^8$ is an 8- to 10-membered heteroaryl ring, the group $R^8$ being unsubstituted or substituted by one, two or three substituents independently selected from halo, $C_{1-4}$ alkoxy, $R^{10}$, -($C_{1-4}$ alkylene)-$R^{10}$, =O, -CN, and $C_{1-4}$ alkyl which is itself unsubstituted or substituted by one or two $R^9$;
each $R^9$ is independently selected from halo and $C_{1-4}$ alkoxy; and
$R^{10}$ is a group selected from a 5- to 6-membered heteroaryl ring, a 3- to 6-membered cycloalkyl ring, a 4- to 6-membered heterocyclyl ring, and a phenyl ring, the group $R^{10}$ being unsubstituted or substituted by one or two substituents independently selected from $C_{1-4}$ alkyl, $C_{1-4}$ alkoxy, and halo.

Typically, in formula (III'), $R^1$ is H, or $C_{1-4}$ alkyl which is itself unsubstituted or substituted with one $C_{1-4}$ alkoxy or one, two or three halo, preferably with one $C_{1-4}$ alkoxy, for example $R^1$ may be H or $C_{1-2}$ alkyl which is unsubstituted or substituted with one $C_{1-2}$ alkoxy or one, two or three halo, preferably with one $C_{1-2}$ alkoxy. Preferably, $R^1$ is H, methyl, ethyl or methoxyethyl. Most preferably, $R^1$ is methyl.

In formula (III'), $R^2$ is H or methyl. Preferably, $R^2$ is H.

Typically, in formula (III'), X is a bond, -N($R^{11}$)-, O or -C($R^{11}$)$_2$-. Preferably, X is a bond, -N(Me)-, O or $-CH_2-$, most preferably X is a bond.

Typically, in formula (III'), each $R^{11}$ is independently selected from H and methyl. When X is -N($R^{11}$)- or -S(O)(N$R^{11}$)-, $R^{11}$ is preferably methyl. When X is -C($R^{11}$)$_2$-, each $R^{11}$ is preferably H.

Typically, in formula (III'), $R^{3a}$ and $R^{3b}$ are independently selected from H, $C_{1-4}$ alkyl, halo and $C_{1-4}$ alkoxy, in particular from H and methyl; $R^{3c}$ and $R^{3d}$ are independently selected from H, methyl, ethyl, fluoro, methoxy and t-butyl, or $R^{3c}$ and $R^{3d}$ form, together with the C atom to which they are attached, a $C_{5-6}$ cycloalkyl ring (in particular a cyclopentyl ring), with the proviso that when X is $NR^{11}$, O, or S, then neither $R^{3c}$ nor $R^{3d}$ are fluoro; or $R^{3a}$ and $R^{3c}$ are H, and $R^{3b}$ and $R^{3d}$ form, together with the C atoms to which they are attached, a $C_{5-6}$ cycloalkyl ring (in particular a cyclohexyl ring); wherein at least two of $R^{3a}$, $R^{3b}$, $R^{3c}$ and $R^{3d}$ are H.

Preferably, in formula (III'), $R^{3a}$, $R^{3b}$, $R^{3c}$ and $R^{3d}$ are all H.

Typically, in formula (III'), $R^4$ is selected from halo, CN, $C_{1-4}$ alkoxy, and $C_{1-4}$ alkyl which is itself unsubstituted or substituted by one, two or three halo. Preferably, $R^4$ is selected from CN, F, Cl, Me and $CF_3$.

Typically, in formula (III'), $R^5$ is selected from Hand $C_{1-4}$ alkyl. Preferably, $R^5$ is selected from H and methyl, more preferably H.

Typically, in formula (III'), $R^6$ is H.

Typically, therefore, in formula (III'):

- $R^4$ is selected from halo, CN, $C_{1-4}$ alkoxy, and $C_{1-4}$ alkyl which is itself unsubstituted or substituted by one, two or three halo, and $R^5$ and $R^6$ are H; or
- $R^5$ is selected from $C_{1-4}$ alkyl, and $R^4$ and $R^6$ when present are H.

Preferably, in formula (III'):

- $R^4$ is selected from selected from F, Cl, Me and $CF_3$, and $R^5$ and $R^6$ are H; or
- $R^5$ is selected from methyl, and $R^4$ and $R^6$ when present are H.

In one preferred embodiment of formula (III'), $R^8$ is selected from indazolyl, thieno[2,3-c]pyrazolyl, imidazo[4,5-b]pyridinyl, pyrazolo[3,4-b]pyridinyl, furo[2,3-c]pyridinyl, indolyl, benzoxazolyl, benzothiazolyl, [1,2,4]triazolo[4,3-a]pyridinyl, thieno[2,3-d]pyrimidinyl, 1,2,3-benzotriazolyl, imidazo[1,5-a]pyridinyl, imidazo[1,2-a]pyrazinyl, oxazolo[5,4-b]pyridinyl, quinolinyl, naphthyridinyl, isoquinolinyl, quinazolinyl, and quinoxalinyl. Preferably, $R^8$ is an 8- to 10-membered heteroaryl ring containing one, two or three N atoms. Most preferably, $R^8$ is an indazolyl group.

Typically, in formula (III'), $R^8$ is a group selected from the following structures. For the avoidance of doubt, the following structures may be unsubstituted or substituted by one, two or three substituents as defined herein.
[see below]

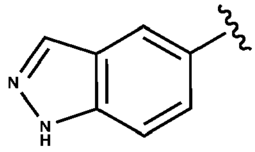
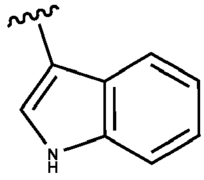
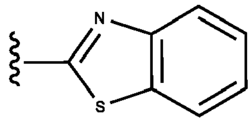

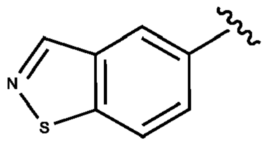
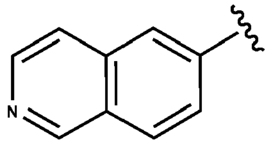
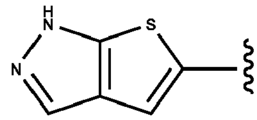

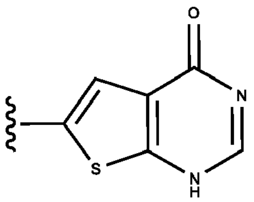
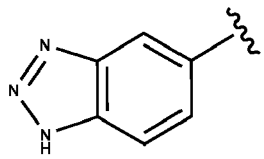
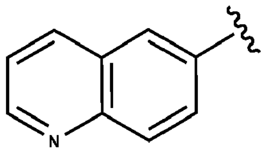

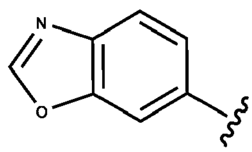
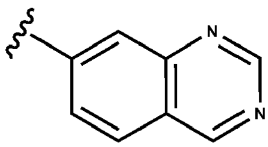
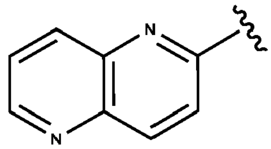

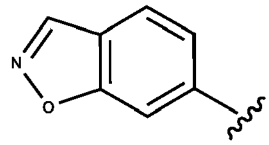
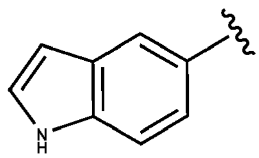
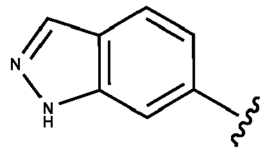
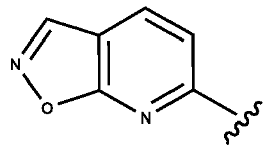

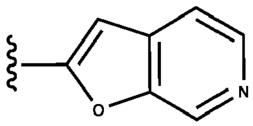
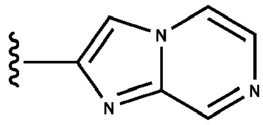
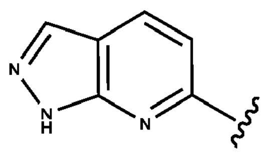

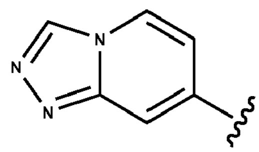
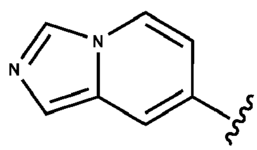
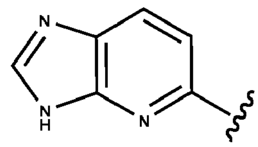

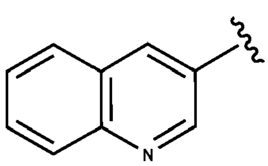
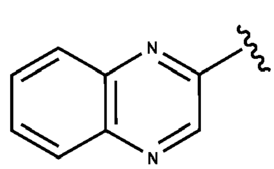
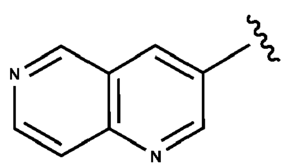

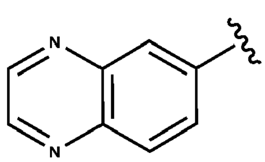
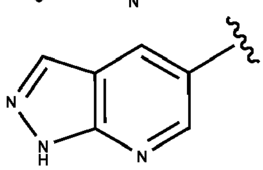

Preferably, $R^8$ is an indazolyl group.

Typically, in formula (III'), $R^8$ is unsubstituted or substituted by one, two or three substituents, for instance one or two substituents, independently selected from halo, $C_{1-4}$ alkoxy, $R^{10}$, $-(CH_2)-R^{10}$, =O, -CN, and $C_{1-4}$ alkyl which is itself unsubstituted or substituted by one or two groups selected from selected from halo and $C_{1-2}$ alkoxy. More typically, each substituent is independently selected from fluoro, chloro, methoxy, $R^{10}$, $-(CH_2)-R^{10}$, =O, -CN, and $C_{1-2}$ alkyl which is itself unsubstituted or substituted by one or two groups selected from selected from halo (e.g. fluoro) and methoxy.

Preferably, each substituent is independently selected from fluoro, $R^{10}$ and methyl. Most preferably, each substituent is independently selected from fluoro and methyl. $R^{10}$ is as defined herein.

Typically, in formula (III'), $R^{10}$ is a group selected from a 5- to 6-membered heteroaryl ring, a 3- to 6-membered cycloalkyl ring, a 4- to 5-membered heterocyclyl ring, and a phenyl ring. The aryl, heteroaryl and heterocyclyl rings are as defined herein. Preferably, $R^{10}$ is selected from cyclopropyl, cyclobutyl, cyclopentyl, cyclohexyl, imidazolyl, oxadiazolyl, oxetanyl, phenyl, pyrazinyl, pyrazolyl, pyridazinyl, pyridinyl, pyrimidinyl, pyrrolidinyl, thiazinyl, thiazolyl and triazolyl, more preferably pyrazolyl.

Typically, in in formula (III'), $R^{10}$ is unsubstituted or substituted by one or two substituents independently selected from fluoro, methyl and methoxy. Preferably, $R^{10}$ is unsubstituted.

In typical embodiments of formula (III'):

$R^1$ is H or $C_{1-2}$ alkyl which is unsubstituted or substituted with one $C_{1-2}$ alkoxy or one, two or three halo, preferably with one $C_{1-2}$ alkoxy;

$R^2$ is H or methyl;

X is a bond, -N(Me)- O or $-C(H)_2-$;

$R^{3a}$ and $R^{3b}$ are independently selected from H and methyl; $R^{3c}$ and $R^{3d}$ are independently selected from H, $C_{1-4}$ alkyl, halo and $C_{1-4}$ alkoxy, in particular from H, methyl, ethyl, fluoro, methoxy and t-butyl, or $R^{3c}$ and $R^{3d}$ form, together with the C atom to which they are attached, a $C_{5-6}$ cycloalkyl ring (in particular a cyclopentyl ring), with the proviso that when X is $NR^{11}$ or O, then neither $R^{3c}$ nor $R^{3d}$ are fluoro; or $R^{3a}$ and $R^{3c}$ are H, and $R^{3b}$ and $R^{3d}$ form, together with the C atoms to which they are attached, a $C_{5-6}$ cycloalkyl ring (in particular a cyclohexyl ring); wherein at least two of $R^{3a}$, $R^{3b}$, $R^{3c}$ and $R^{3d}$ are H;

one of the following options applies: $R^4$ is selected from halo, CN, $C_{1-4}$ alkoxy, and $C_{1-4}$ alkyl which is itself unsubstituted or substituted by one, two or three halo, and $R^5$ and $R^6$ are H; or $R^5$ is selected from $C_{1-4}$ alkyl, and $R^4$ and $R^6$ when present are H;

the group $R^8$ is unsubstituted or substituted by one, two or three substituents, for instance one or two substituents, each independently selected from halo, $C_{1-4}$ alkoxy, $R^{10}$, $-(CH_2)-R^{10}$, =O, -CN, and $C_{1-4}$ alkyl which is itself unsubstituted or substituted by one or two groups selected from selected from halo and $C_{1-2}$ alkoxy; and $R^{10}$ is a group selected from a 5- to 6-membered heteroaryl ring, a 3- to 6-membered cycloalkyl ring, a 4- to 5-membered heterocyclyl ring, and a phenyl ring; wherein $R^{10}$ is unsubstituted or substituted by one or two substituents independently selected from fluoro, methyl and methoxy; and $R^8$ is a group selected from the following structures:

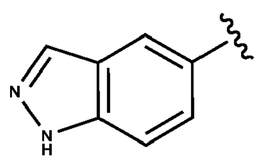
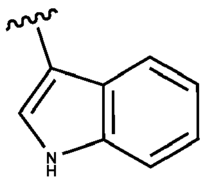
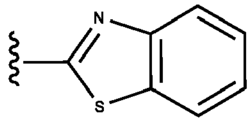

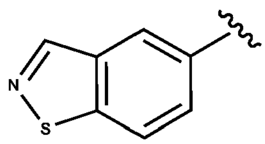
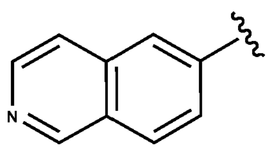
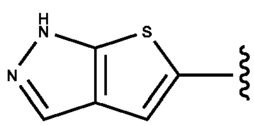

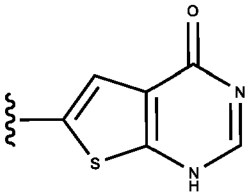
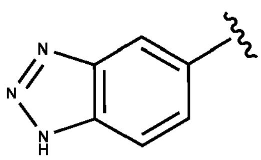
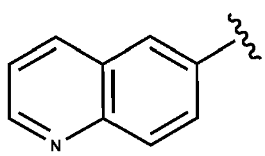

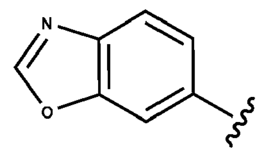
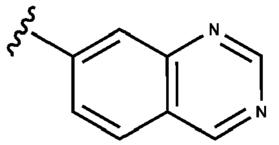
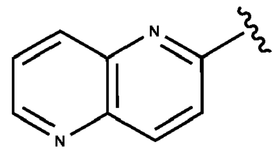

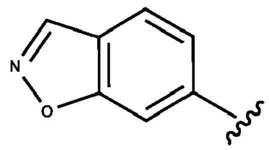
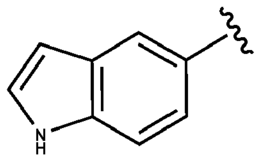
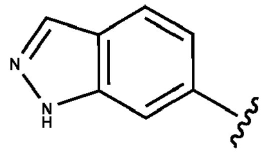
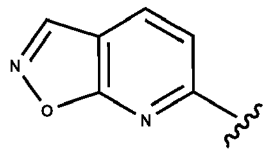

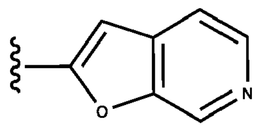
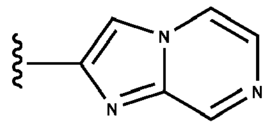
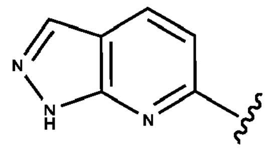

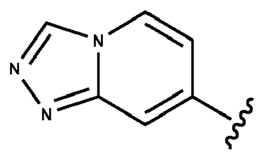
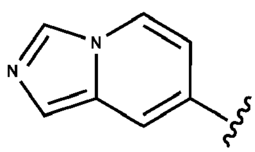
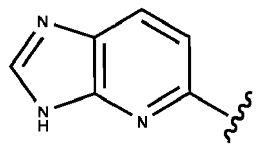

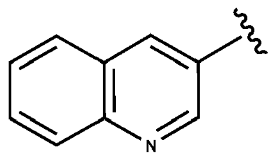
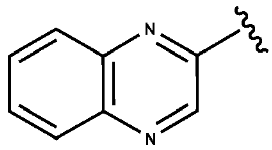
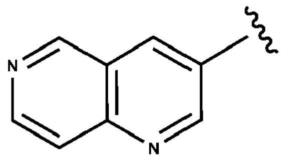

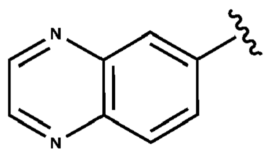
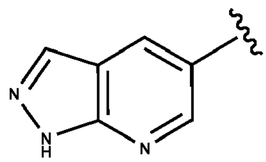

In preferred embodiments of formula (III'),

X is absent;
$R^1$ is H, methyl, ethyl or methoxyethyl, more preferably methyl;
$R^2$ is H;
$R^{3a}$, $R^{3b}$, $R^{3c}$ and $R^{3d}$ are all H;
$R^4$ is selected from F, Cl, Me and $CF_3$;
$R^8$ is an 8- to 10-membered heteroaryl ring containing one, two or three N atoms, for example an indazolyl group; the group $R^8$ is unsubstituted or substituted by one or two substituents, each independently selected from fluoro, $R^{10}$ and methyl;
$R^{10}$ is selected from cyclopropyl, cyclobutyl, cyclopentyl, cyclohexyl, imidazolyl, oxadiazolyl, oxetanyl, phenyl, pyrazinyl, pyrazolyl, pyridazinyl, pyridinyl, pyrimidinyl, pyrrolidinyl, thiazinyl, thiazolyl and triazolyl, preferably pyrazolyl; and $R^{10}$ is unsubstituted.

In a most preferred embodiment, the compound of the invention is a bicyclic compound of formula (IV') or a pharmaceutically acceptable salt thereof:

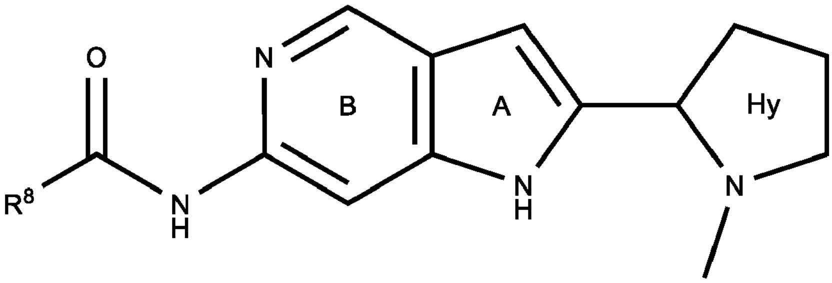

(IV')

wherein $R^8$ is an 8- to 10-membered heteroaryl ring, the group $R^8$ being unsubstituted or substituted by one, two or three substituents independently selected from halo and $C_{1-4}$ alkyl.

Typically, in formula (IV'), $R^8$ may be selected from thiindazolyl, thieno[2,3-c]pyrazolyl, imidazo[4,5-b]pyridinyl, pyrazolo[3,4-b]pyridinyl, furo[2,3-c]pyridinyl, indolyl, benzoxazolyl, benzothiazolyl, [1,2,4]triazolo[4,3-a]pyridinyl, thieno[2,3-d]pyrimidinyl, 1,2,3-benzotriazolyl, imidazo[1,5-a]pyridinyl, imidazo[1,2-a]pyrazinyl, oxazolo[5,4-b]pyridinyl, quinolinyl, naphthyridinyl, isoquinolinyl, quinazolinyl, and quinoxalinyl.

More typically, in formula (IV'), $R^8$ is a group selected from the following structures. For the avoidance of doubt, the following structures may be unsubstituted or substituted by one, two or three substituents as defined herein. [see below]

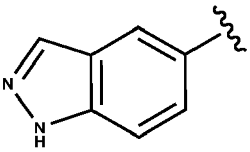
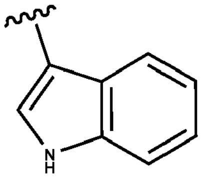
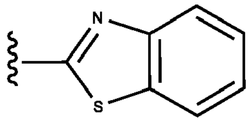

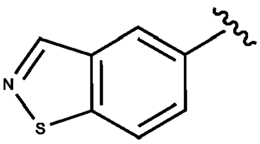
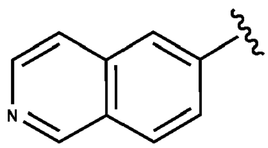
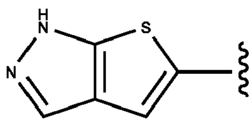

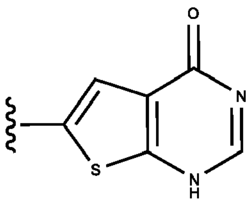
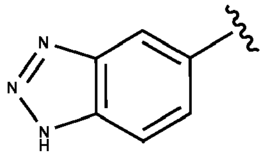
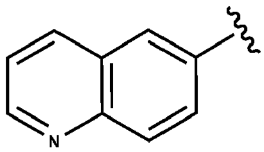

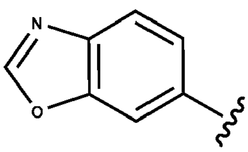
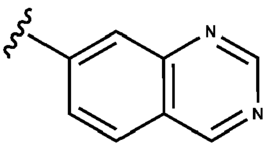
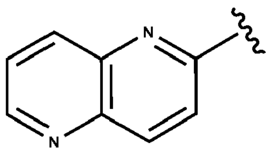

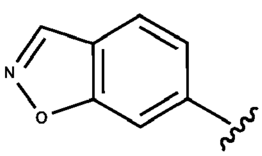
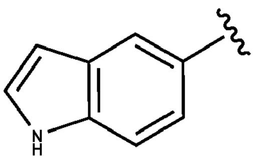
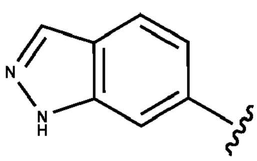
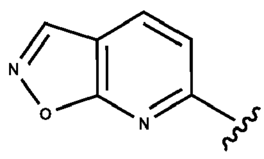

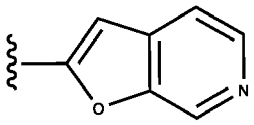
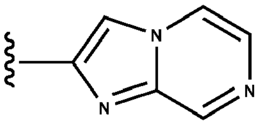
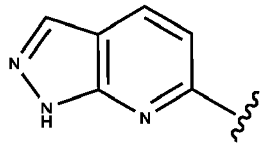
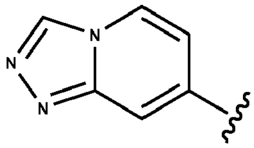
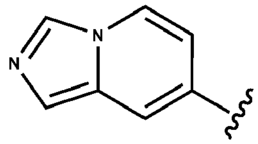
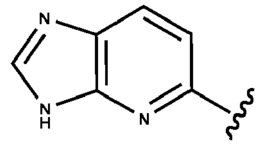
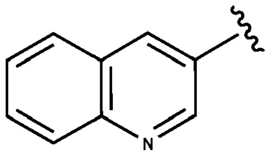
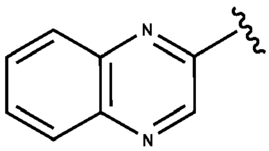
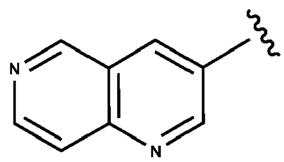
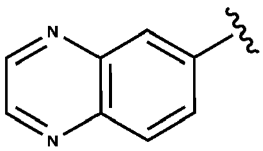
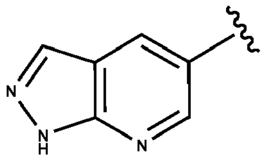

Preferably, $R^8$ is an indazolyl group.

Typically, in formula (IV'), $R^8$ is unsubstituted or substituted by one, two or three substituents, for instance one or two substituents, independently selected from halo and

$C_{1-4}$ alkyl. Preferably, each substituent is independently selected from fluoro and methyl.

In a particularly preferred embodiment, in formula (IV'), $R^8$ is selected from one of the following structures:

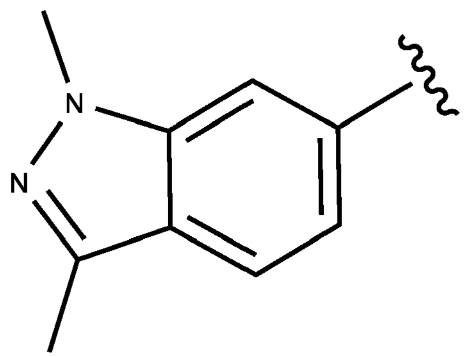
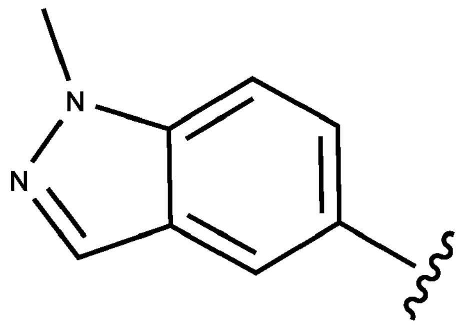

For the avoidance of doubt, $R^8$ may be selected from one of the two structures above in any of formulae (I'), (II') and (III').

In one aspect, the following MLLT1 and/or MLLT3 inhibitors are provided:

N-{3-chloro-2-[(2R)-1-methylpyrrolidin-2-yl]-1H-pyrrolo[3,2-c]pyridin-6-yl}-1,3-dimethylindazole-6-carboxamide;
N-{3-chloro-2-[(2S)-1-methylpyrrolidin-2-yl]-1H-pyrrolo[3,2-c]pyridin-6-yl}-1,3-dimethylindazole-6-carboxamide;
(R)-1,3-dimethyl-N-(2-(1-methylpyrrolidin-2-yl)-3-(trifluoromethyl)-1H-pyrrolo[3,2-c]pyridin-6-yl)-1H-indazole-6-carboxamide;
(S)-1,3-dimethyl-N-(2-(1-methylpyrrolidin-2-yl)-3-(trifluoromethyl)-1H-pyrrolo[3,2-c]pyridin-6-yl)-1H-indazole-6-carboxamide;
N-{3-fluoro-2-[(rel-2S)-1-methylpyrrolidin-2-yl]-1H-pyrrolo[3,2-c]pyridin-6-yl}-1-methylindazole-5-carboxamide;
N-{3-fluoro-2-[(rel-2R)-1-methylpyrrolidin-2-yl]-1H-pyrrolo[3,2-c]pyridin-6-yl}-1-methylindazole-5-carboxamide;
N-{3-fluoro-2-[(rel-2R)-1-methylpyrrolidin-2-yl]-1H-pyrrolo[3,2-c]pyridin-6-yl}-1,3-dimethylindazole-6-carboxamide;
N-{3-fluoro-2-[(rel-2S)-1-methylpyrrolidin-2-yl]-1H-pyrrolo[3,2-c]pyridin-6-yl}-1,3-dimethylindazole-6-carboxamide;
1,3-dimethyl-N- f 3-methyl-2-[(rel-2R)-1-methylpyrrolidin-2-yl]-1H-pyrrolo[3,2-c]pyridin-6-yl}indazole-6-carboxamide;

1,3-dimethyl-N-{3-methyl-2-[(rel-2S)-1-methylpyrrolidin-2-yl]-1H-pyrrolo[3,2-c]pyridin-6-yl} indazole-6-carboxamide;

1,3-dimethyl-N- f 4-methyl-2-[(rel-2R)-1-methylpyrrolidin-2-yl]-1H-pyrrolo[3,2-c]pyridin-6-yl}indazole-6-carboxamide;

1,3-dimethyl-N- f 4-methyl-2-[(rel-2S)-1-methylpyrrolidin-2-yl]-1H-pyrrolo[3,2-c]pyridin-6-yl}indazole-6-carboxamide; and

(R)-N-(3-cyano-2-(1-methylpyrrolidin-2-yl)-1H-pyrrolo[3,2-c]pyridin-6-yl)-1,3-dimethyl-1H-indazole-6-carboxamide; and the pharmaceutically acceptable salts thereof.

*Therapeutic Efficacy*

The compounds of the present invention are therapeutically useful. The present invention therefore provides compounds as described herein, for use in medicine. The present invention provides compounds as described herein, for use in treating the human or animal body. For the avoidance of doubt, the agent may comprise a compound of the invention in the form of a solvate. Also provided is a pharmaceutical composition comprising a compound of the invention together with a pharmaceutically acceptable carrier or diluent. Typically, the composition contains up to 85 wt% of a compound of the invention. More typically, it contains up to 50 wt% of a compound of the invention. Preferred pharmaceutical compositions are sterile and pyrogen free. Further, when the pharmaceutical compositions provided by the invention contain a compound of the invention which is optically active, the compound of the invention is typically a substantially pure optical isomer.

The composition of the invention may be provided as a kit comprising instructions to enable the kit to be used in the methods described herein or details regarding which subjects the method may be used for.

As explained above, the compounds of the invention are useful in treating or preventing various disorders. Disorders for treatment using the compounds of the invention may include cancer. In particular, the compounds of the invention are useful for inducing selective degradation of MLLT1 and/or MLLT3, or inhibiting MLLT1 and/or MLLT3.

Cancer, e.g. acute lymphoblastic leukemia, acute myeloid leukemia, adrenocortical carcinoma, aids-related lymphoma, primary CNS lymphoma, anal cancer, astrocytomas, brain cancer, basal cell carcinoma, bile duct cancer, bladder cancer, bone cancer (e.g. ewing sarcoma, osteosarcoma and malignant fibrous histiocytoma), breast cancer, bronchial tumors, medulloblastoma and other CNS embryonal tumors, cervical cancer, chronic lymphocytic leukemia, chronic myelogenous leukemia, chronic myeloproliferative neoplasms, colorectal cancer, craniopharyngioma, endometrial cancer, ependymoma, esophageal cancer, esthesioneuroblastoma, ewing sarcoma, extragonadal germ cell tumor, intraocular melanoma, retinoblastoma, fallopian tube cancer, gallbladder cancer, gastric cancer, gastrointestinal carcinoid tumor, gastrointestinal stromal tumors (gist), germ cell tumors, extragonadal germ cell tumors, ovarian germ cell tumors, testicular cancer, gestational trophoblastic disease, hairy cell leukemia, hepatocellular cancer, histiocytosis, langerhans cell, hodgkin lymphoma, hypopharyngeal cancer, intraocular melanoma, islet cell tumors, pancreatic neuroendocrine tumors, kaposi sarcoma, kidney (renal cell) cancer, langerhans cell histiocytosis, laryngeal cancer, leukemia, liver cancer, lung cancer (non-small cell, small cell, pleuropulmonary blastoma, and tracheobronchial tumor), lymphoma, malignant fibrous histiocytoma of bone and osteosarcoma, merkel cell carcinoma, mesothelioma, mouth cancer, multiple endocrine neoplasia syndromes, multiple myeloma/plasma cell neoplasms, mycosis fungoides, myelodysplastic syndromes, myelodysplastic/ myeloproliferative neoplasms, myelogenous leukemia, neuroblastoma, non-hodgkin lymphoma, oropharyngeal cancer, osteosarcoma and undifferentiated pleomorphic sarcoma, pancreatic cancer, pancreatic neuroendocrine tumors (islet cell tumors), papillomatosis, paraganglioma, parathyroid cancer, penile cancer, pharyngeal cancer, pheochromocytoma, pituitary tumor, prostate cancer, rectal cancer, retinoblastoma, rhabdomyosarcoma, t-cell lymphoma, testicular cancer, throat cancer, thymoma and thymic carcinoma, thyroid cancer, tracheobronchial tumors and the like is particularly suitable to being treated with the compounds of the invention provided herein.

Typically, the compounds of the invention are for use in treating cancers which are transcriptionally addicted cancers, such as breast cancer, acute leukemia, prostate cancer, bladder cancer, cholangiocarcinoma, colon adenocarcinoma, esophageal carcinoma, head and neck squamous cell carcinoma, kidney chromophobe, kidney renal clear cell carcinoma, kidney renal papillary cell carcinoma, liver hepatocellular carcinoma, lung adenocarcinoma, pheochromocytoma and paraganglioma, rectum adenocarcinoma, stomach adenocarcinoma, uterine corpus endometrial carcinoma, cervical squamous cell carcinoma and endocervical adenocarcinoma, lung squamous cell carcinoma and sarcoma (Cell Rep, 2021, 16, 1087).

Preferably, the compounds of the invention are for use in treating acute myeloid leukaemia (AML) or acute lymphoblastic leukaemia (ALL), such as mixed-lineage leukaemia (MLL) rearranged acute leukemia (AML and ALL) and NPM1 mutant leukemia.

The compounds of the invention may be used as standalone therapeutic agents. Alternatively, they may be used in combination with other active agents such as chemotherapeutic agents, targeted precision medicines or immune oncology agents. For example, they may be used in combination with a Bcl2 targeted drug (for instance venetoclax), FLT3

inhibitor (for instance sorafenib, quizartinib or gilteritinib), EGFR inhibitor (for instance erlotinib, gefitinib, lapatinib or osimertinib), CDK4/6 inhibitor (for instance abemaciclib, palbociclib or ribociclib), a chemotherapy (for instance cytarabine, doxorubicin, gemcitabine, cisplatin or paclitaxel) or an immune checkpoint inhibitor (for instance pembrolizumab, nivolumab, durvalumab or cemiplimab).

When used to treat a cancer, the compounds of the invention may be used in alleviating, ameliorating or preventing aggravation of the symptoms of the cancer. Typically, treating a cancer may comprise reducing progression of the cancer, e.g. increasing progression free survival (PFS) and/or increasing survival e.g. increasing overall survival (OS). Treating a cancer may comprise preventing or inhibiting growth of a tumour associated with the cancer. Treating a cancer may comprise preventing metastasis of the cancer. Preferably, treating a cancer may comprise reducing the size of a tumour associated with the cancer. As such, the treatment may cause tumour regression in the cancer. Treating a cancer may comprise reducing the number of tumours or lesions present in the patient. When the treatment reduces the size of a tumour associated with the cancer, the size of the tumour is typically reduced from base line by at least 10%. Base line is the size of the tumour at the date treatment with the compound is first started. The size of the tumour is typically as measured in accordance with version 1.1 of the RECIST criteria (for instance as described in Eisenhauer et al, European Journal of Cancer 45 (2009) 228-247).

The response to the treatment with the compound may be complete response, partial response or stable disease, in accordance with version 1.1 of the RECIST criteria. Preferably, the response is partial response or complete response. The treatment may achieve progression free survival for at least 60 days, at least 120 days or at least 180 days.

The reduction in tumour size may be greater 20%, greater than 30% or greater than 50% reduction relative to base line. The reduction in tumour size may be observed after 30 days of treatment or after 60 days of treatment.

In haematological cancers, treating a cancer preferentially may lead to complete remission (CR) of the cancer or it may lead to complete remission with incomplete haematological recovery (CRh) or complete remission but with incomplete platelet recovery (CRp). Additionally, treatment may lead to an increase in duration of remission (DoR), an increase in minimal residual disease (MRD), or an increase in relapse free survival (RFS) (Blood 2007 109 1815 and Leukemia Res 2018, 68, 32).

As explained here, the compounds of the invention are useful in treating or preventing various disorders. The present invention therefore provides a compound of the invention for use in medicine. The invention also provides the use of a compound of the invention in the manufacture of a medicament. The invention also provides compositions and products comprising the compounds of the invention. Such compositions and products are also useful in treating or preventing disorders. The present invention therefore provides a composition or product as defined herein for use in medicine. The invention also provides the use of a composition or product of the invention in the manufacture of a medicament. Also provided is a method of treating a subject in need of such treatment, said method comprising administering to the subject a compound of the invention. In some embodiments the subject suffers from or is at risk of suffering from one of the disorders disclosed herein.

In one aspect, the subject is a mammal, in particular a human. However, it may be non-human. Preferred non-human animals include, but are not limited to, primates, such as marmosets or monkeys, commercially farmed animals, such as horses, cows, sheep or pigs, and pets, such as dogs, cats, mice, rats, guinea pigs, ferrets, gerbils or hamsters. The subject can be any animal that is capable of being infected by a bacterium.

A subject is typically a human patient. The patient may be male or female. The age of the patient is typically at least 18 years, for instance from 30 to 70 years or from 40 to 60 years. Alternatively, the patient may be a child, for instance the patient may be under 18 years of age, or under 12 years of age. In one embodiment, the patient is a child under ten years of age or an infant under two years of age. In one embodiment the invention provides a compound as defined herein for use in treating cancer in paediatric patients.

A compound or composition of the invention can be administered to the subject in order to treat one or more symptoms of the disorder. In this embodiment, the subject is typically symptomatic. A therapeutically effective amount of the agent or formulation is administered to such a subject. A therapeutically effective amount is an amount effective to ameliorate one or more symptoms of the disorder.

The compound or composition of the invention may be administered in a variety of dosage forms. Thus, it can be administered orally, for example as tablets, troches, lozenges, aqueous or oily suspensions, dispersible powders or granules. The compound or composition of the invention may also be administered parenterally, whether subcutaneously, intravenously, intramuscularly, intrasternally, transdermally or by infusion techniques. The compound or composition may also be administered as a suppository. Preferably, the compound, composition or combination may be administered orally.

The compound or composition of the invention is typically formulated for administration with a pharmaceutically acceptable carrier or diluent. For example, solid oral forms may contain, together with the active compound, diluents, e.g. lactose, dextrose, saccharose, cellulose, corn starch or potato starch; lubricants, e.g. silica, talc, stearic acid, magnesium or calcium stearate, and/or polyethylene glycols; binding agents; e.g. starches, arabic gums, gelatin, methylcellulose, carboxymethylcellulose or polyvinyl pyrrolidone; disaggregating agents, e.g. starch, alginic acid, alginates or sodium starch glycolate; effervescing mixtures; dyestuffs; sweeteners; wetting agents, such as lecithin, polysorbates, laurylsulphates; and, in general, non toxic and pharmacologically inactive substances used in pharmaceutical formulations. Such pharmaceutical preparations may be manufactured in known manner, for example, by means of mixing, granulating, tableting, sugar coating, or film coating processes.

Liquid dispersions for oral administration may be syrups, emulsions and suspensions. The syrups may contain as carriers, for example, saccharose or saccharose with glycerine and/or mannitol and/or sorbitol.

Suspensions and emulsions may contain as carrier, for example a natural gum, agar, sodium alginate, pectin, methylcellulose, carboxymethylcellulose, or polyvinyl alcohol. The suspension or solutions for intramuscular injections or inhalation may contain, together with the active compound, a pharmaceutically acceptable carrier, e.g. sterile water, olive oil, ethyl oleate, glycols, e.g. propylene glycol, and if desired, a suitable amount of lidocaine hydrochloride.

Solutions for inhalation, injection or infusion may contain as carrier, for example, sterile water or preferably they may be in the form of sterile, aqueous, isotonic saline solutions. Pharmaceutical compositions suitable for delivery by needleless injection, for example, transdermally, may also be used.

A therapeutically effective amount of the compound or composition of the invention is administered to a subject. The dose may be determined according to various parameters, especially according to the compound used; the age, weight and condition of the subject to be treated; the route of administration; and the required regimen. Again, a physician will be able to determine the required route of administration and dosage for any particular subject. A typical daily dose is from about 0.01 to 100 mg per kg, preferably from about 0.1 mg/kg to 50 mg/kg, e.g. from about 1 to 10 mg/kg of body weight, according to the activity of the specific compound, the age, weight and conditions of the subject to be treated, the type and severity of the disease and the frequency and route of administration. Preferably, daily dosage levels are from 5 mg to 2 g.

When the compound or composition of the invention is administered to a subject in combination with another active agent, the dose of the other active agent can be determined as described above. The dose may be determined according to various parameters, especially according to the agent used; the age, weight and condition of the subject to be treated; the route of administration; and the required regimen. Again, a physician will be able to determine the required route of administration and dosage for any particular subject. A typical daily dose is from about 0.01 to 100 mg per kg, preferably from about 0.1 mg/kg to 50 mg/kg, e.g. from about 1 to 10 mg/kg of body weight, according to the activity of the specific agent, the age, weight and conditions of the subject to be treated, the type and severity of the disease and the frequency and route of administration. Preferably, daily dosage levels are from 5 mg to 2 g.

*Synthesis*

Compounds of the invention can be prepared by the synthetic methods described in the Examples that follow, or by analogy with such methods using appropriate starting materials and methodologies familiar to the skilled chemist.

The following examples illustrate the invention. They do not however limit the invention in any way. In this regard, it is important to understand that the particular assay used in the Examples section is designed only to provide an indication of biological activity. There are many assays available to determine biological activity, and a negative result in any one particular assay is therefore not determinative. The invention is defined according to the claims.

## Examples

In the examples which follow below, compounds may be produced in racemic form. Compounds in racemic form are indicated by use of the term rac- in advance of the IUPAC name. In some instances, as indicated, enantiomers and/or diastereomers are separated. In general, the enantiomers and/or diastereomers of the invention can be separated using chiral chromatography. The separated enantiomers and/or diastereomers may be identified in the order in which they elute, for example the first, second, third or fourth eluting isomer respectively, as obtained by separation using chiral chromatography. The isomers may alternatively or additionally be identified using IUPAC naming conventions. Thus, where enantiomers and/or diastereomers have been assigned an absolute stereochemistry, this is indicated as R or S stereochemistry, as appropriate, and by use of a wedge-shaped bond (e.g. 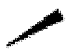)in the chemical formula.

Where enantiomers have been separated but have undefined absolute stereochemistry the term "rel-R" or "rel-S" has been used. In these examples it is understood that the absolute configuration is unknown and the "rel-R" and "rel-S" labels are used merely to distinguish the two enantiomers form one another. The absolute stereochemistry of an enantiomer labelled as "rel-R" may be either R or S. In either case, the corresponding "rel-S" enantiomer has the opposite configuration to the "rel-R" enantiomer. In these examples the use of a rectangular bond (e.g. 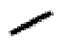 ) has been used to define the "rel" configuration.

Alternatively, where diastereomers have been separated and the absolute configuration is unknown they may be assigned a relative stereochemistry, which denotes the stereochemistry of a first chiral centre with respect to a second

chiral centre. Where a relative stereochemistry has been assigned, this is indicated by the term "rel" in the IUPAC name, and by use of a rectangular-shaped bond (e.g. 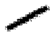 ). Where diastereomers have been separated and the relative and absolute configuration is unknown, the compounds have been labelled by the order in which they elute, for example the first, second, third or fourth eluting isomer respectively, as obtained by separation using chiral chromatography.

The moieties U of formulae (U1), (U4) when Q' is CH and (US) typically contain at least one chiral centre at the carbon atoms marked with an asterisk as depicted below:

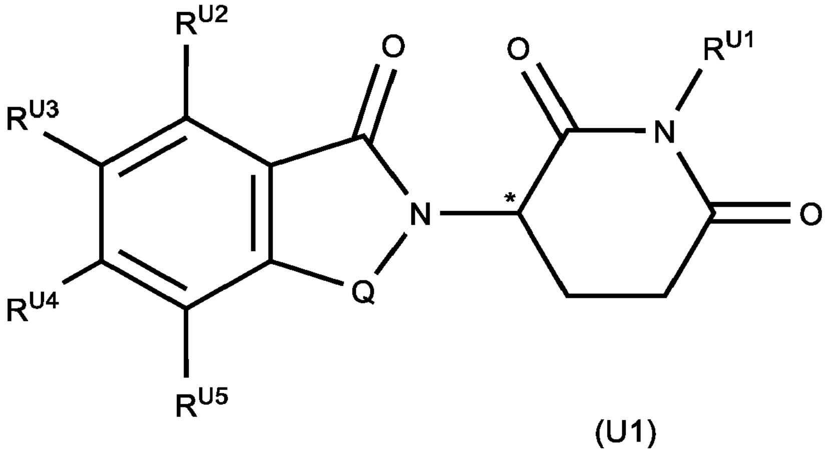

(U1) ;

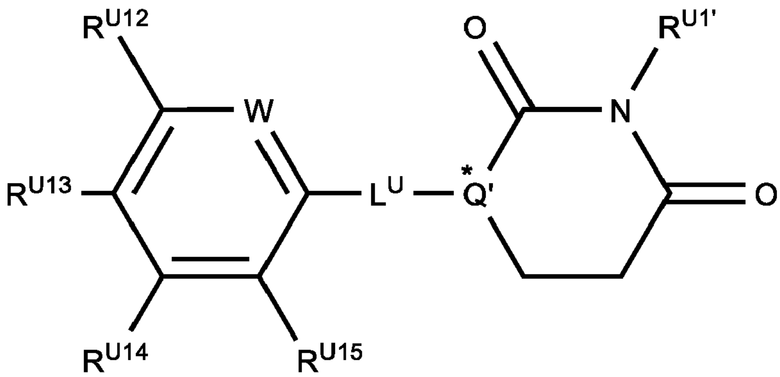

(U4) ;

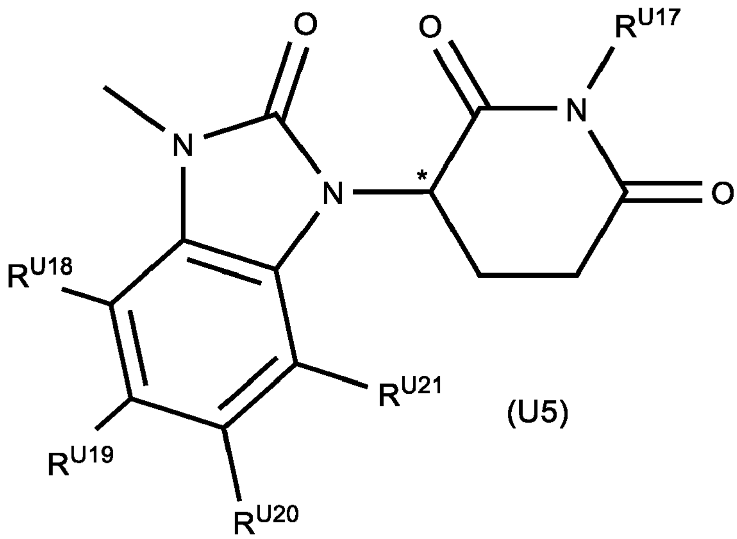

(U5) .

Specifically in relation to the examples that follow, it is understood that the moiety U is typically in the form of a 1:1 mixture of the R- and S-enantiomers at said carbon atom, indicated specifically in the examples that follow by the use of a simple line-shaped bond (e.g. 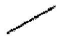 ).

Temperatures are given in degrees Celsius (°C). The reactants used in the examples below may be obtained from commercial sources or they may be prepared from commercially available starting materials as described herein or by methods known in the art. All the compounds of the invention are synthesized according to the Examples described herein. The progress of the reactions described herein were followed as appropriate by e.g. LC, GC or TLC, and as the skilled person will readily realize, reaction times and temperatures may be adjusted accordingly.

**NMR spectroscopy:**

Solids/oils were solubilized in DMSO-$d_6$ or MeOH-$d_4$, vortexed vigorously until the solution was clear and transferred to an NMR tube for data acquisition.

Liquid-state NMR experiments were recorded using:

- 600 MHz (14.1 Tesla) Bruker Avance III NMR spectrometer (600 MHz for $^1$H, 151 MHz for $^{13}$C) using a triple-resonance $^1$H,$^{15}$N,$^{13}$C CP-TCI 5 mm cryoprobe (Bruker Biospin, Germany)
- 500 MHz (11.75 Tesla) Bruker Avance I NMR spectrometer (500 MHz for $^1$H, 125 MHz for $^{13}$C) using a Dual Resonance BBI 5 mm probe (Bruker Biospin, Germany)
- 400 MHz (9.4 Tesla) Bruker Avance NEO NMR spectrometer (400 MHz for $^1$H, 100 MHz for $^{13}$C) using a SEI 5 mm probe (Bruker Biospin, Germany)
- 400 MHz (9.4 Tesla) Bruker Avance NEO NMR spectrometer using a PI HR-BB0400S1-BBF/H/D-5.0-Z SP probe (Bruker Biospin, Germany)
- 300 MHz Bruker AVANCE III HD NMR spectrometer using a PA BBO 300S1 BBF-H-D-05 Z probe (Bruker Biospin, Germany)
- 300 MHz Bruker Avance NEO NMR spectrometer using a PA BBO BBF-H-D-05 Z (Bruker Biospin, Germany)

All the experiments used for the resonance assignment procedure and the elucidation of the products structure (1D $^1$H, 2D $^1$H-$^1$H-COSY, 2D $^1$H-$^1$H-ROESY, 2D $^1$H-$^{13}$C-HSQC, 2D $^1$H-$^{13}$C-HMBC) were recorded at 300 K. $^1$H chemical shifts are reported in $\delta$ ppm as s (singlet), d (doublet), t (triplet), q (quartet), dd (double doublet), m (multiplet) or br s (broad singlet).

**UPLC-MS chromatography:**

UPLC-MS chromatography analysis were recorded using the following apparatus using:

- Waters UPLC: Acquity, UV: Acquity PDA, MS: Qda, ELSD
- Waters UPLC: Acquity, UV: Acquity TUV, MS: Qda
- Waters UPLC: Acquity, UV: Acquity PDA, MS: Qda

The apparatus was tested using a CSH C18 Waters column (50 $\times$ 2.1 mm), 1.7 $\mu$m. It used a combination of the following eluents: $H_2O$ + 0.05% TFA (v/v) (solvent A) and MeCN + 0.035% TFA (v/v) (solvent B) and a positive electrospray ES+ as ionization mode. The UV detection was set at 220 and 254 nm. The methods used were the following:

- Polar method (1.7 min run): gradient t=0 2% B, t=0.5 min 2% B, t=1.5 min 98% B, t=1.52 min 2% B, t=1.7 min 2% B
- Polar method (3.5 min run): gradient t=0 2% B, t=1.0 min 2% B, t=2.4 min 98% B, t=3.00 min 98% B, t=3.03 min 2% B, t=3.5 min 2% B
- Normal method (1.7 min run): gradient t=0 2% B, t=1.0 min 98% B, t=1.5 min 98% B, t=1.52 min 2% B, t=1.7 min 2% B
- Normal method (3.5 min run): gradient t=0 2% B, t=2.4 min 98% B, t=3.0 min 98% B, t=3.03 min 2% B, t=3.5 min 2% B

Where it is observed mass spectra analysis is reported as $[M+H]^+$ for the molecular ion; Someone skilled in the art will also appreciate that isotope patterns may be reported where evident, for example $[M+H+2]^+$ represents the isotopic peak of Br if it is present in the molecule; Additionally, some fragmentation ions may be included in the analysis, for example [M+H-*t*Bu]$^+$, [M+H-Boc]$^+$ and [M-Cl+MeOH]$^+$.

**Preparative HPLC**

Where Example purification has been performed by preparative HPLC the conditions are included in the reaction Step where the purification was performed or one of the general Methods below was used:

**Method A:**

Gilson system used for preparative HPLC purification purposes. The system is equipped with a 333 pump A and a 334 pump B, Gilson Verity 1741 detector and GX-271 liquid handler fraction collector. The reverse phase purification was carried out by using a preparative column X-BRIDGE C18 (250 × 30 mm), 5 μM. Gradient elution was done using 10 mmol ammonium bicarbonate in water (as Phase A) and 100% Acetonitrile (as Phase B) with a gradient Program (B%): 10% B at 0 min hold until 3 min, 35% B at 10.0 min, 65% B at 35 min, 99% B at 35.1 min hold till 40.0 min, 10% B at 40.1 min hold until 45 min. Flow rate: 25 mL/min.

**Method B:**

Gilson system used for preparative HPLC purification purposes. The system is equipped with a 333 pump A and a 334 pump B, Gilson Verity 1741 detector and GX-271 liquid handler Fraction collector. The reverse phase purification is carried out by using preparative column YMC-ACTUS C18 (250*20mm) 5 μM. Gradient elution is done with 0.1% Formic acid in water (as Phase A) and 100% Acetonitrile (Phase B) with a gradient Program (B%): 5% B at 0 min hold till 5min,15% B at 200.0 min, 35% B at 35 min, 99% B at 35.1min hold till 40.0 min, 5% B at 40.1min hold till 45 min. Flow rate: 18 mL/min.

**Abbreviations:**

In addition to the definitions above, the following abbreviations are used in the Example experimental procedures below. If an abbreviation used herein is not defined, it has its generally accepted meaning:

| | |
|---|---|
| Ac | Acetyl |
| AcOH | Acetic acid |
| AIBN | 2,2'-Azobis(2-methylpropionitrile) |
| Boc | *tert*-butyloxycarbonyl |
| $Boc_2O$ | Di-*tert*butyl decarbonate |
| BrettPhos | 2-(Dicyclohexylphosphino)3,6-dimethoxy-2',4',6'-triisopropyl-1,1'-biphenyl |
| BrettPhos Pd G3 | [(2-Di-cyclohexylphosphino-3,6-dimethoxy-2',4',6'- triisopropyl-1,1'-biphenyl)-2-(2'-amino-1,1'-biphenyl)]palladium(II) methanesulfonate methanesulfonate |
| $CBr_4$ | tetrabromomethane |
| $Cs_2CO_3$ | Cesium carbonate |
| CuI | Copper (I) iodide |
| DBU | 1,8-Diazabicyclo[5.4.0]undec-7-ene |
| DCM | Dichloromethane |
| DHP | 3,4-Dihydro-2H-pyran |
| DIAD | Diisopropyl azodicarboxylate |
| DIPEA | *N*,*N*-Diisopropylethylamine |
| Dioxane | 1,4-dioxane |
| DMAP | 4-Dimethylaminopyridine |
| DMEDA | *N*,*N'*-dimethylethylenediamine |
| DMF | Dimethylformamide |
| DMSO | Dimethylsulfoxide |
| DMSO-$d_6$ | Hexadeuterodimethylsulfoxide |
| ee | Enantiomeric excess |
| Et | Ethyl |
| EtOAc | Ethyl acetate |
| $Et_3N$ | Triethylamine |
| EtOH | Ethanol |
| $Et_2O$ | Diethylether |
| EPhos Pd G4 | Methanesulfonato{Dicyclohexyl[3-(1-methylethoxy)-2',4',6'-tris(1-methylethyl)[1,1'-biphenyl]-2-yl]phosphine}(2'-methylamino-1,1'-biphenyl-2-yl)palladium(II) |
| $FeCl_3$ | Iron(III) chloride |

| | |
|---|---|
| GC | Gas chromatography |
| GPhos | (3-(tert-Butoxy)-2',6'-diisopropyl-6-methoxy-[1,1'-biphenyl]-2-yl)dicyclohexylphosphane |
| Gphos Pd $G_6$ TES | GPhos OAC precatalyst TES |
| h | hour |
| $H_2O$ | water |
| HATU | 1-[Bis(dimethylamino)methylene]-1H-1,2,3-triazolo[4,5-b]pyridinium 3-oxid hexafluorophosphate |
| HCl | Hydrochloric acid |
| Het | Heteroaromatic |
| HPLC | High performance liquid chromatography |
| IPA | Isopropyl amine |
| $K_2CO_3$ | Potassium carbonate |
| KI | Potassium iodide |
| LC | Liquid chromatography |
| LDA | Lithium diisopropylamide |
| $LiBH_4$ | Lithium borohydride |
| LiHMDS | Lithium bis(trimethylsilyl)amide |
| LiOH | Lithium hydroxide |
| min | Minutes |
| Me | Methyl |
| MeCN | Acetonitrile |
| MeOH | Methanol |
| MeTHF | 2-Methyltetrahydrofuran |
| $MgSO_4$ | Magnesium sulfate |
| MS | Mass spectrometry |
| MsCl | Methanesulfonyl chloride |
| MsOH | Methanesulfonic acid |
| MTBE | Methyl tert-butyl ether |
| MW | Microwave |
| $N_2$ | Nitrogen |
| NaH | Sodium hydride |
| NaOCN | Sodium cyanate |
| NaOH | Sodium hydroxide |
| $Na_2SO_4$ | Sodium sulfate |
| $NaBH_3CN$ | Sodium cyanoborohydride |
| NBS | *N*-bromosuccinimide |
| NCS | N-chlorosuccinimide |
| $NH_4Cl$ | Ammonium chloride |
| NIS | N-iodosuccinimide |
| NMP | N-methyl-2-pyrrolidone |
| Pd/C | palladium on carbon |
| $Pd(dppf)Cl_2$ | [1,1'-Bis(diphenylphosphino)ferrocene]dichloropalladium(II) |
| $Pd(PPh_3)_2Cl_2$ | Bis(triphenylphosphine)palladium(II) dichloride |
| PE | Petroleum ether |
| $PPh_3$ | Triphenylphosphine |
| PMB-Cl | 4-methoxybenzyl chloride |
| rt | room temperature (18 to 22 °C) |
| Selectfluor | 1-(Chloromethyl)-4-fluoro-1,4-diazabicyclo[2.2.2]octane-1,4-diium ditetrafluoroborate |
| SEM-Cl | 2-(Trimethylsilyl)ethoxymethyl chloride |
| TBS-Cl | tert-Butyldimethylsilyl chloride |
| tBuOK | Potassium tert-butoxide |
| TFA | Trifluoroacetic acid |
| TfOH | Trifluoromethanesulfonic acid |
| TLC | Thin layer chromatography |
| THF | Tetrahydrofuran |
| TsCl | p-Toluenesulfonyl chloride |
| TsOH | p-Toluenesulfonic acid monohydrate |
| UPLC | Ultra Performance Liquid Chromatography |
| Xantphos | 4,5-Bis(diphenylphosphino)-9,9-dimethylxanthene |

**Example 1 was synthesised following Scheme 1**

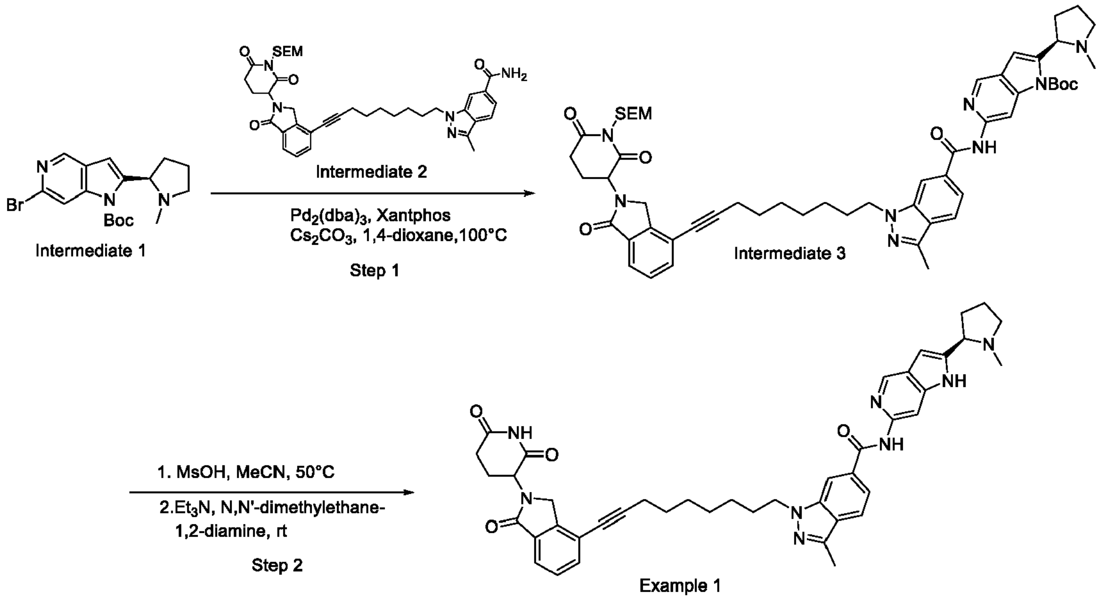


## Scheme 1

**Step 1**

**Intermediate 3: *tert*-butyl-6-(1-(9-(2-(2,6-dioxo-1-((2-(trimethylsilyl)ethoxy)methyl)piperidin-3-yl)-1-oxoisoindolin-4-yl)non-8-yn-1-yl)-3-methyl-1*H*-indazole-6-carboxamido)-2-((*R*)-1-methylpyrrolidin-2-yl)-1*H*-pyrrolo[3,2-c] pyridine-1-carboxylate**

To a solution of 1-(9-(2-(2,6-dioxo-1-((2-(trimethylsilyl)ethoxy)methyl)piperidin-3-yl)-1-oxoisoindolin-4-yl)non-8-yn-1-yl)-3-methyl-1*H*-indazole-6-carboxamide **(Intermediate 2,** 0.8 g, 1 mmol) in 1,4-dioxane (20 mL) was added *tert*-butyl-(*R*)-6-bromo-2-(1-methylpyrrolidin-2-yl)-1*H*-pyrrolo[3,2-c]pyridine-1-carboxylate **(Intermediate 1,** 1.00 g, 2.63 mmol) followed by cesium carbonate (2.5 g, 7.7 mmol). The reaction mixture was purged with argon for 15 min, then $Pd_2(dba)_3$ (0.15 g, 0.16 mmol) and Xantphos (0.3 g, 0.5 mmol) were added. The reaction mixture was further purged with argon for 10 min and then heated at 100°C for 2h. The reaction mixture was filtered through celite and washed with ethyl acetate (50 mL). The filtrate was concentrated *in vacuo* and the resultant crude material was purified by combi-flash column chromatography, by eluting with 100% ethyl acetate in heptane, to afford *tert*-butyl 6-(1-(9-(2-(2,6-dioxo-1-((2-(trimethylsilyl)ethoxy)methyl)piperidin-3-yl)-1-oxoisoindolin-4-yl)non-8-yn-1-yl)-3-methyl-1*H*-indazole-6-carboxamido)-2-((R)-1-methylpyrrolidin-2-yl)-1*H*-pyrrolo[3,2-c]pyridine-1-carboxylate **(Intermediate 3,** 1.00 g, 39%) as a yellow solid. $^1H$ NMR (400 MHz, DMSO-$d_6$) δ -0.05 (s, 9H), 0.76-0.84 (m, 2H), 1.25-1.45 (m, 7H), 1.59-1.66 (m, 2H), 1.70 (s, 9H), 1.73-1.80 (m, 2H), 1.82-1.92 (m, 3H), 2.04 (s, 3H), 2.31-2.34 (m, 1H), 2.36 (s, 3H), 2.42-2.47 (m, 3H), 2.72-2.81 (m, 2H), 2.98-3.17 (m, 3H), 3.46-3.55 (m, 1H), 3.88-3.96 (m, 1H), 4.21-4.26 (m, 1H), 4.35-4.47 (m, 3H), 4.99-5.10 (m, 2H), 5.24-5.28 (m, 1H), 6.76 (s, 1H), 7.47-7.52 (m, 1H), 7.58-7.62 (m, 1H), 7.68-7.73 (m, 2H), 7.76-7.81 (m, 1H), 8.42 (s, 1H), 8.60 (s, 1H), 8.97 (s, 1H), 10.74 (s, 1H). Mass spec: m/z: 969.2 $[M+H]^+$.

**Step 2**

**Example 1: 1-(9-(2-(2,6-dioxopiperidin-3-yl)-1-oxoisoindolin-4-yl)non-8-yn-1-yl)-3-methyl-*N*-(2-((*R*)-1-methylpyrrolidin-2-yl)-1*H*-pyrrolo[3,2-c]pyridin-6-yl)-1*H*-indazole-6-carboxamide**

To a solution of *tert*-butyl-6-(1-(9-(2-(2,6-dioxo-1-((2-(trimethylsilyl)ethoxy)methyl)piperidin-3-yl)-1-oxoisoindolin-4-yl)non-8-yn-1-yl)-3-methyl-1*H*-indazole-6-carboxamido)-2-((*R*)-1-methylpyrrolidin-2-yl)-1*H*-pyrrolo[3,2-c]pyridine-1-carboxylate **(Intermediate 3,** 5.00 g, 5.16 mmol) in acetonitrile (50 mL) was added methanesulfonic acid (3.35 mL, 51.59 mmol) and the reaction mixture was then heated at 50°C for 2h. After cooling to room temperature, *N,N'*-dimethylethylenediamine (2.78 mL, 25.79 mmol) followed by triethylamine (14.4 mL, 103.2 mmol) were added and

the reaction was stirred at room temperature for 3h. The reaction mixture was quenched with water (20 mL), the resultant precipitate was collected by filtration and dried *in vacuo* to afford the crude compound. The crude material was purified by preparative HPLC (Method A) to afford 1-(9-(2-(2,6-dioxopiperidin-3-yl)-1-oxoisoindolin-4-yl)non-8-yn-1-yl)-3-methyl-*N*-(2-((*R*)-1-methylpyrrolidin-2-yl)-1*H*-pyrrolo[3,2-c]pyridin-6-yl)-1*H*-indazole-6-carboxamide **(Example 1,** 2.00 g , 52%) as an off-white solid. $^1$H NMR (400 MHz, DMSO-$d_6$) δ 1.22-1.46 (m, 7H), 1.50-1.59 (m, 2H), 1.76-1.94 (m, 5H), 1.96-2.03 (m, 1H), 2.12-2.15 (m, 1H), 2.17 (s, 3H), 2.24-2.30 (m, 1H), 2.41-2.44 (m, 1H), 2.45 (s, 3H), 2.53-2.62 (m, 2H), 2.85-2.95 (m, 1H), 3.13-3.17 (m, 1H), 3.34-3.36 (m, 1H), 4.24-4.33 (m, 1H), 4.37-4.47 (m, 3H), 5.10-5.15 (m, 1H), 6.40 (s, 1H), 7.45-7.52 (m, 1H), 7.58-7.60 (m, 1H), 7.67-7.80 (m, 3H), 8.25 (s, 1H), 8.43 (s, 1H), 8.52 (s, 1H), 10.52 (s, 1H), 11.00 (s, 1H), 11.38 (s, 1H). Mass spec: m/z: 739.3 [M+H]$^+$.

**Intermediate 1 was synthesised following Scheme 2**

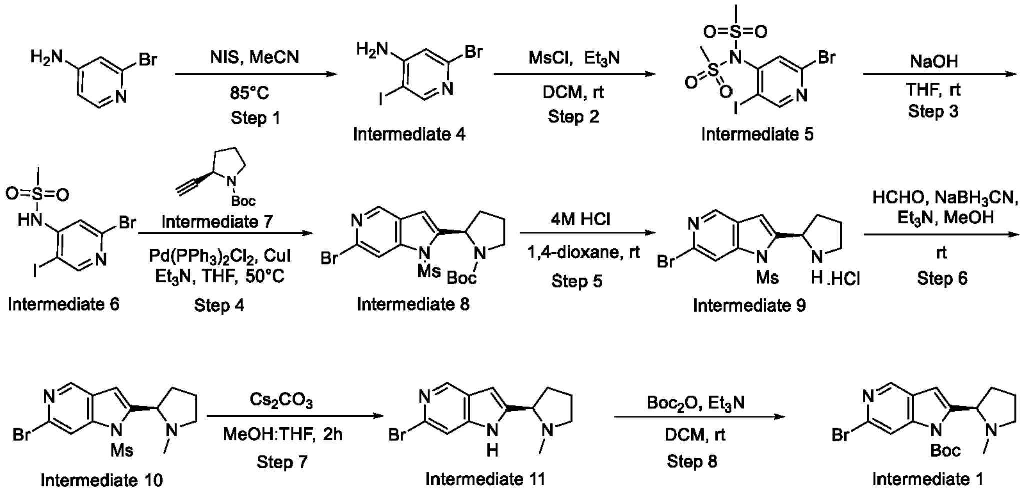


**Scheme 2**

**Step 1**

**Intermediate 4: 2-bromo-5-iodopyridin-4-amine**

To solution of 2-bromopyridin-4-amine (CAS No: 7598-35-8, 200 g, 1156.0 mmol) in acetonitrile (1 L) was added *N*-iodosuccinimide (297.23 g, 1294.7 mmol) and the reaction mixture was heated at 80°C for 16h. The reaction mixture was then cooled to room temperature and concentrated *in vacuo.* The obtained residue was diluted with aqueous saturated sodium thiosulphate solution (2 L) and extracted with ethyl acetate (3 × 2 L). The combined organic layers were dried over anhydrous $Na_2SO_4$ and concentrated *in vacuo* to obtain the crude compound. The crude material was purified by combi-flash chromatography, by eluting with 5% ethyl acetate in heptane, to afford 2-bromo-5-iodo-pyridin-4-amine **(Intermediate 4,** 115 g, 33%) as a yellow solid. $^1$H NMR (400 MHz, DMSO-$d_6$) δ ppm 6.48 (br s, 2H), 6.77 (s, 1H), 8.16 (s, 1H). Mass spec: m/z: 300.8 [M+H]$^+$.

**Step 2**

**Intermediate 5: N-(2-bromo-5-iodopyridin-4-yl)-N-(methylsulfonyl)methanesulfonamide**

To a cooled (0°C) solution of 2-bromo-5-iodo-pyridin-4-amine **(Intermediate 4,** 100 g, 334.55 mmol) in dichloromethane (2.50 g, 29 mmol) was added triethylamine (234 mL, 1673 mmol) followed by methanesulfonyl chloride (106 mL, 1338.2 mmol) in dichloromethane (300 mL). After the addition was complete, the reaction mixture was stirred at room temperature for 3h. The reaction mixture was quenched with aqueous saturated $NaHCO_3$ solution (2 L) and extracted with dichloromethane (2 × 2 L). The combined organic layers were dried over anhydrous $Na_2SO_4$ and concentrated *in vacuo* to afford *N*-(2-bromo-5-iodopyridin-4-yl)-*N*-(methylsulfonyl)methanesulfonamide **(Intermediate 5,** 150 g, 98%) as a yellow liquid, which was used for next step without further purification. $^1$H NMR (400 MHz, DMSO-$d_6$) δ 3.65 (s, 6H), 8.10 (s, 1H), 8.92 (s, 1H). Mass spec: m/z: 456 [M+H]$^+$.

**Step 3**

**Intermediate 6: *N*-(2-bromo-5-iodopyridin-4-yl)methanesulfonamide**

To a solution of *N*-(2-bromo-5-iodopyridin-4-yl)-*N*-(methylsulfonyl)methanesulfonamide **(Intermediate 5,** 150 g, 329.6 mmol) in tetrahydrofuran (2 L) and water (2 L) was added sodium hydroxide (79.9 g, 1978 mmol) and the reaction mixture was stirred at room temperature for 3h. The reaction mixture was poured into water (500 mL), extracted with ethyl acetate (3 × 1000 mL) and acidified with citric acid solution (1 L) to pH ~ 4. The resultant precipitate was collected by filtration, washed with water (100 mL) and dried in *vacuo* to afford *N*-(2-bromo-5-iodopyridin-4-yl) methanesulfonamide **(Intermediate 6,** 75 g, 60%) as a yellow solid, which was used for next step without further purification. $^1H$ NMR (400 MHz, DMSO-$d_6$) δ 3.28(s, 3H), 7.54 (s, 1H), 8.64 (s, 1H), 9.51 (s, 1H). Mass spec: m/z: 378.7 $[M+H]^+$.

**Step 4**

**Intermediate 8: tert-butyl (*R*)-2-(6-bromo-1-(methylsulfonyl)-1*H*-pyrrolo[3,2-c]pyridin-2-yl)pyrrolidine-1-carboxylate**

To a solution of *N*-(2-bromo-5-iodopyridin-4-yl)methanesulfonamide **(Intermediate 6,** 50.0 g, 132.63 mmol) and *tert*-butyl (*R*)-2-ethynylpyrrolidine-1-carboxylate **(Intermediate 7,** 25.89 g, 132.63 mmol) in THF (500 mL) was added DIPEA (138 g, 1061.0 mmol). The reaction mixture was purged with argon gas for 15 min then $PdCl_2(PPh_3)_2$ (9.60 g, 13.263 mmol) and copper(I) iodide (2.55 g, 13.26 mmol) were added. The reaction mixture was further purged with argon gas for 10 min and then heated at 60°C for 3h. The reaction mixture was diluted with water (500 mL) and extracted with ethyl acetate (3 × 300 mL). The combined organic layers were dried over anhydrous $Na_2SO_4$ and concentrated *in vacuo* to obtain crude compound. The crude material was purified by combi-flash chromatography, by eluting with 30% ethyl acetate in heptane, to afford *tert*-butyl (*R*)-2-(6-bromo-1-(methylsulfonyl)-1*H*-pyrrolo[3,2-c]pyridin-2-yl)pyrrolidine-1-carboxylate **(Intermediate 8, 38.0** g, 62%) as a yellow solid. $^1H$ NMR (400 MHz, DMSO-$d_6$) δ 1.40 (s, 9H), 1.85-1.91 (m, 4H), 3.18-3.21 (m, 1H), 3.37-3.41 (m, 1H), 3.62 (s, 3H), 5.23-5.31 (m, 1H), 6.72 (s, 1H), 7.97 (s, 1H), 8.69 (s, 1H). Mass spec: m/z: 445.9 $[M+H]^+$.

**Step 5**

**Intermediate 9: (*R*)-6-bromo-1-(methylsulfonyl)-2-(pyrrolidin-2-yl)-1*H*-pyrrolo[3,2-c]pyridine hydrochloride**

To a cooled (0°C) solution of *tert*-butyl-(*R*)-2-(6-bromo-1-(methylsulfonyl)-1*H*-pyrrolo[3,2-c]pyridin-2-yl)pyrrolidine-1-carboxylate **(Intermediate 8,** 200 g, 450.1 mmol) in 1,4-dioxane (1 L) was added 4M HCl in 1,4-dioxane (2 L) and the reaction mixture was stirred at room temperature for 6h. The reaction mixture was concentrated *in vacuo* to afford (*R*)-6-bromo-1-(methylsulfonyl)-2-(pyrrolidin-2-yl)-1*H*-pyrrolo[3,2-c]pyridine hydrochloride **(Intermediate 9,** 160 g) as a yellow solid, which was used for the next step without further purification. $^1H$ NMR (400 MHz, DMSO-$d_6$) δ 1.87-2.20 (m, 2H), 2.33-2.46 (m, 2H), 3.15-3.34 (m, 2H), 3.75 (s, 3H), 5.12-5.17 (m, 1H), 7.38 (s, 1H), 7.98 (s, 1H), 8.78 (s, 1H), 9.45 (s, 2H). Mass spec: m/z: 345.8 $[M+H]^+$.

**Step 6**

**Intermediate 10: (*R*)-6-bromo-2-(1-methylpyrrolidin-2-yl)-1-(methylsulfonyl)-1*H*-pyrrolo [3,2-c]pyridine**

To a cooled (0°C) solution of (*R*)-6-bromo-1-(methylsulfonyl)-2-(pyrrolidin-2-yl)-1*H*-pyrrolo[3,2-c]pyridine hydrochloride **(Intermediate 9,** 90.0 g, 236.4 mmol) in methanol (900 mL) was added triethylamine (192 g, 1891 mmol) followed by formaldehyde (60.83 g, 709.2 mmol). The reaction mixture was stirred for 5 min and then sodium cyanoborohydride (34.96 g, 472.8 mmol) was added at 0°C. The reaction mixture was then heated at 60°C for 16h. The reaction mixture was diluted with water (500 mL) and extracted with dichloromethane (3 × 500 mL). The combined organic layers were dried over anhydrous $Na_2SO_4$ and concentrated *in vacuo* to the obtain crude compound. The crude material was purified by combi-flash chromatography by eluting with 60% ethyl acetate in heptane, to afford (R)-6-bromo-2-(1-methylpyrrolidin-2-yl)-1-(methylsulfonyl)-1*H*-pyrrolo[3,2-c]pyridine **(Intermediate 10,** 60.0 g, 71%) as a yellow solid. $^1H$ NMR (400 MHz, DMSO-$d_6$) δ 1.70-1.78 (m, 3H), 2.33 (s, 3H), 2.36-2.40 (m, 2H), 3.01-3.22 (m, 1H), 3.56 (s, 3H), 3.76-3.81 (m, 1H), 6.89 (s, 1H), 7.97 (s, 1H), 8.68 (s, 1H). Mass spec: m/z: 359.8 $[M+H]^+$.

**Step 7**

**Intermediate 11: (*R*)-6-bromo-2-(1-methylpyrrolidin-2-yl)-1*H*-pyrrolo[3,2-c]pyridine**

To a solution of (*R*)-6-bromo-2-(1-methylpyrrolidin-2-yl)-1-(methylsulfonyl)-1*H*-pyrrolo[3,2-c]pyridine **(Intermediate 10,** 110 g, 307.1 mmol) in methanol (600 mL) was added tetrahydrofuran (600 mL) and the mixture cooled to 0°C. Cesium carbonate (200 g, 614.2 mmol) was added and the reaction mixture was stirred at room temperature for 4h. The reaction mixture was quenched with water (2 L) and extracted with ethyl acetate (2 × 1 L). The combined organic layers were dried over anhydrous $Na_2SO_4$ and concentrated *in vacuo* to afford (*R*)-6-bromo-2-(1-methylpyrrolidin-2-yl)-1*H*-pyrrolo[3,2-c]pyridine **(Intermediate 11,** 60.0 g, 70%) as a yellow solid, which was used for the next step without further purification. $^1$H NMR (400 MHz, DMSO-$d_6$) δ 1.75-1.91 (m, 3H), 2.14 (s, 3H), 2.21-2.29 (m, 1H), 3.10-3.15 (m, 1H), 3.33-3.36 (m, 2H), 6.45 (s, 1H), 7.43 (s, 1H), 8.48 (s, 1H), 11.57 (br s, 1H). Mass spec: m/z: 279.9 $[M+H]^+$.

**Step 8:**

**Intermediate 1: *tert*-butyl (*R*)-6-bromo-2-(1-methylpyrrolidin-2-yl)-1*H*-pyrrolo[3,2-c]pyridine-1-carboxylate**

To a solution of (*R*)-6-bromo-2-(1-methylpyrrolidin-2-yl)-1*H*-pyrrolo[3,2-c]pyridine **(Intermediate 11,** 60.0 g, 214.16 mmol) in dichloromethane (600 mL) was added triethylamine (65.3 g, 642.49 mmol) followed by di-*tert*-butyldicarbonate (94.42 g, 428.33 mmol) and 4-(dimethylamino)pyridine (5.28 g, 42.833 mmol) and the reaction mixture was stirred at room temperature for 3h. The reaction mixture was diluted with water (500 mL) and extracted with diethyl ether (3 × 500 mL). The combined organic layers were dried over anhydrous $Na_2SO_4$ and concentrated *in vacuo* to obtain the crude compound. The crude material was purified by combi-flash chromatography, by eluting with 45% ethyl acetate in heptane, followed by SFC purification (Waters SFC Prep 150 Mgm equipped with 2489 PDA detector; Column: Chiralpak IC (30 × 250 mm, 5 μM); Solvent A) $CO_2$ 70 g/min; B) 0.1% Isopropyl amine in IPA and MeCN (50:50) 25 mL/min) to afford *tert*-butyl-(*R*)-6-bromo-2-(1-methylpyrrolidin-2-yl)-1*H*-pyrrolo[3,2-c]pyridine-1-carboxylate **(Intermediate 1,** 43 g, 53%) as an off white solid. Enantiomeric excess (ee) 98.4%. $^1$H NMR (400 MHz, DMSO-$d_6$) δ ppm 1.65 (s, 9H), 1.71-1.75 (m, 3H), 2.30-2.33 (m, 1H), 2.34 (s, 3H), 2.37-2.40 (m, 1H), 3.11-3.14 (m, 1H), 3.87-3.91 (m, 1H), 6.80 (s, 1H), 8.05 (s, 1H), 8.61 (s, 1H). Mass spec: m/z: 379.9 $[M+H]^+$.

**Intermediate 1 can also be synthesized following Scheme 3**

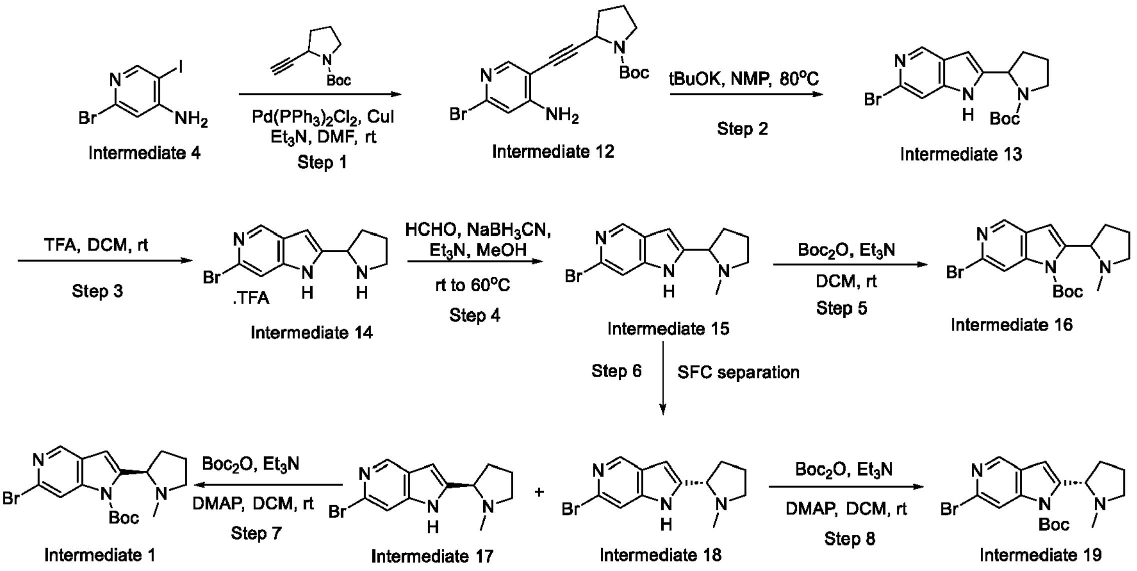


**Scheme 3**

**Step 1**

**Intermediate 12: rac-tert-butyl 2-[2-(4-amino-6-bromopyridin-3-yl)ethynyl]pyrrolidine-1-carboxylate**

A solution of 2-bromo-5-iodopyridin-4-amine **(Intermediate 4:** 17 g, 54.2 mmol), rac-tert-butyl 2-ethynylpyrrolidine-1-carboxylate (CAS: 316141-37-4, 13.4 g, 65.04 mmol), $Pd(PPh_3)_2Cl_2$ (3.81 g, 5.42 mmol), CuI (1.03 g, 5.42 mmol) and $Et_3N$ (16.4 g, 162.6 mmol) in DMF (400 mL) was stirred for 2h at rt under nitrogen atmosphere. The reaction mixture was quenched with water and the resulting solution was extracted three times with EtOAc. The organic layers were combined, washed with brine, dried over anhydrous $Na_2SO_4$, filtered, and concentrated under reduced pressure. The crude material was purified by flash chromatography on silica gel using petroleum ether/EtOAc (7/3) as eluent to afford rac-tert-butyl 2-[2-(4-amino-6-bromopyridin-3-yl)ethynyl]pyrrolidine-1-carboxylate **(Intermediate 12:** 18 g, 83%) as a yellow oil. Mass spec: m/z 366 $[M+H]^+$.

**Step 2**

**Intermediate 13: rac-tert-butyl 2-{6-bromo-1H-pyrrolo[3,2-c]pyridin-2-yl}pyrrolidine-1-carboxylate**

To a solution of rac-tert-butyl 2-[2-(4-amino-6-bromopyridin-3-yl)ethynyl]pyrrolidine-1-carboxylate **(Intermediate 12:** 8 g, 20.8 mmol) in NMP (40 mL) was added a solution of tBuOK (65.53 mL, 65.53 mmol, 1M in THF). The reaction mixture was stirred for 4h at 80°C. The reaction mixture was quenched with water and the resulting solution was extracted three times with EtOAc. The organic layers were combined, washed with brine, dried over anhydrous $Na_2SO_4$, filtered, and concentrated under reduced pressure. The crude material was purified by flash chromatography on silica gel using DCM/MeOH (95/5) as eluent to afford rac-tert-butyl 2-{6-bromo-1H-pyrrolo[3,2-c]pyridin-2-yl}pyrrolidine-1-carboxylate **(Intermediate 13:** 6 g, 71%) as a yellow oil. Mass spec: m/z 366 $[M+H]^+$.

**Step 3**

**Intermediate 14: rac-2-{6-bromo-1H-pyrrolo[3,2-c]pyridin-2-yl}pyrrolidine trifluoroacetate**

To a solution of rac-tert-butyl 2-{6-bromo-1H-pyrrolo[3,2-c]pyridin-2-yl}pyrrolidine-1-carboxylate **(Intermediate 13:** 16 g, 41.5 mmol) in DCM (300 mL) was added TFA (30 mL). The reaction mixture was stirred for 2h at rt. The solution was concentrated under reduced pressure to afford rac-2-{6-bromo-1H-pyrrolo[3,2-c]pyridin-2-yl}pyrrolidine trifluoroacetate **(Intermediate 14:** 10 g, 77%) as a yellow oil which was used in Step 5 without further purification. Mass spec: m/z 266 $[M+H]^+$.

**Step 4**

**Intermediate 15: rac-6-bromo-2-(1-methylpyrrolidin-2-yl)-1H-pyrrolo[3,2-c]pyridine**

To a solution of rac-6-bromo-2-(pyrrolidin-2-yl)-1H-pyrrolo[3,2-c]pyridine trifluoroacetate **(Intermediate 14:** 10 g, 35.8 mmol) in MeOH (300 mL) were added $Et_3N$ (10.9 g, 108 mmol) and paraformaldehyde (5.37 g, 179 mmol). The reaction mixture was stirred for 1h at rt. $NaBH_3CN$ (6.77 g, 107.4 mmol) was added and the reaction mixture was stirred overnight at 60°C. The reaction mixture was quenched with water and the resulting solution was extracted three times with EtOAc. The organic layers were combined, washed with brine, dried over anhydrous $Na_2SO_4$, filtered, and concentrated under reduced pressure. The residue was chromatographed by reverse-phase flash chromatography (C18 aq) using $MeCN/H_2O$ (45/55) as eluent to afford rac-6-bromo-2-(1-methylpyrrolidin-2-yl)-1H-pyrrolo[3,2-c]pyridine **(Intermediate 15:** 8 g, 69%) as a yellow oil. Mass spec: m/z 280 $[M+H]^+$.

**Step 5**

**Intermediate 16: rac-tert-butyl 6-bromo-2-(1-methylpyrrolidin-2-yl)-1H-pyrrolo[3,2-c]pyridine-1-carboxylate**

To a solution of rac-6-bromo-2-(1-methylpyrrolidin-2-yl)-1H-pyrrolo[3,2-c]pyridine **(Intermediate 15:** 8 8 g, 25.8 mmol) in DCM (200 mL) were added $Boc_2O$ (8.44 g, 38.7 mmol), $Et_3N$ (7.82 g, 77.4 mmol) and DMAP (310 mg, 2.58 mmol). The reaction mixture was stirred overnight at rt and then quenched with water. The resulting solution was extracted three times with EtOAc and the organic layers were combined, washed with brine, dried over anhydrous $Na_2SO_4$, filtered, and concentrated under reduced pressure. The crude material was purified by flash chromatography on silica gel using EtOAc/petroleum ether (3/2) as eluent to afford rac-tert-butyl 6-bromo-2-(1-methylpyrrolidin-2-yl)-1H-pyrrolo[3,2-c]pyridine-1-carboxylate **(Intermediate 16:** 8.1 g, 71%) as a yellow oil. Mass spec: m/z 380 $[M+H]^+$.

**Step 6**

**Intermediate 17: (2R)-2-{6-bromo-1H-pyrrolo[3,2-c]pyridin-2-yl}-1-methylpyrrolidine and Intermediate 18: (2S)-2-{6-bromo-1H-pyrrolo[3,2-c]pyridin-2-yl}-1-methylpyrrolidine**

rac-6-bromo-2-(1-methylpyrrolidin-2-yl)-1H-pyrrolo[3,2-c]pyridine **(Intermediate 15,** 9.00 g, 30.5 mmol) was separated by SFC (Column: CHIRALPAK IH 3*25 cm, 5 μm; Mobile Phase A: $CO_2$, Mobile Phase B: MeOH (+1%-2M $NH_3$ in MeOH); Flow rate: 85 mL/min; Gradient: isocratic 20% B) to afford (2R)-2-{6-bromo-1H-pyrrolo[3,2-c]pyridin-2-yl}-1-methylpyrrolidine **(Intermediate 17,** 1st eluting peak, 3.5 g, 37%) as a white solid and (2S)-2-{6-bromo-1H-pyrrolo[3,2-c]pyridin-2-yl}-1-methylpyrrolidine **(Intermediate 18,** 2nd eluting peak, 4.0 g, 40%) as a white solid.

**Intermediate 17: (2R)-2-{6-bromo-1H-pyrrolo[3,2-c]pyridin-2-yl}-1-methylpyrrolidine**

$1^{st}$ eluting peak from SFC purification. Retention time: 4.35 mins.

Enantiomeric excess (ee) 99.8%.

$^1H$ NMR (400 MHz, DMSO-d6) δ ppm 11.56 (s, 1H), 8.49 (s, 1H), 7.43 (s, 1H), 6.45 (s, 1H), 3.34 (t, J = 9.0 Hz, 1H), 3.13 (t, J = 7.8 Hz, 1H), 2.24 - 2.30 (m, 1H), 2.15 (s, 4H), 1.87 - 1.90 (m, 1H), 1.75 - 1.84 (m, 2H).

**Intermediate 18: (2S)-2-{6-bromo-1H-pyrrolo[3,2-c]pyridin-2-yl}-1-methylpyrrolidine**

2nd eluting peak from SFC purification. Retention time: 4.87 mins.

Enantiomeric excess (ee) 99.8%.$^1H$ NMR (400 MHz, DMSO-d6) δ ppm 11.56 (s, 1H), 8.49 (s, 1H), 7.43 (s, 1H), 6.45 (s, 1H), 3.35 (t, J = 11.4 Hz, 1H), 3.14 (t, J = 8.0 Hz, 1H), 2.26 (t, J = 8.6 Hz, 1H), 2.15 (s, 4H), 1.88 (d, J = 8.0 Hz, 1H), 1.80 (d, J = 8.0 Hz, 2H).

**Step 7**

**Intermediate 1: *tert*-butyl (*R*)-6-bromo-2-(1-methylpyrrolidin-2-yl)-1*H*-pyrrolo[3,2-c]pyridine-1-carboxylate**

A 500 mL round-bottom flask was charged with (2R)-2-{6-bromo-1H-pyrrolo[3,2-c]pyridin-2-yl}-1-methylpyrrolidine **(Intermediate** 17, 500 mg, 1.68 mmol), DCM (10 mL), $Et_3N$ (541 mg, 5.35 mmol), DMAP (21.8 mg, 0.178 mmol), $(Boc)_2O$ (778 mg, 3.57 mmol) and the reaction was stirred overnight at room temperature. The reaction was quenched with water (200 mL). The resulting solution was extracted with ethyl acetate (3 × 200 mL) and the organic layers were combined, washed with brine (2 × 300 mL), dried over anhydrous sodium sulfate, filtered, and concentrated under reduced pressure. The residue was chromatographed on a silica gel column, eluting with EtOAc/Petroleum ether (35/65) to afford tert-butyl (R)-6-bromo-2-(1-methylpyrrolidin-2-yl)-1H-pyrrolo[3,2-c]pyridine-1-carboxylate **(Intermediate 1,** 600 mg, 92%) as an off-white solid. $^1H$ NMR (400 MHz, DMSO-d6) δ ppm 1.65 (s, 9H), 1.71-1.75 (m, 3H), 2.30-2.33 (m, 1H), 2.34 (s, 3H), 2.37-2.40 (m, 1H), 3.11-3.14 (m, 1H), 3.87-3.91 (m, 1H), 6.80 (s, 1H), 8.05 (s, 1H), 8.61 (s, 1H). Mass spec: m/z: 379.9 $[M+H]^+$. Mass spec: m/z: 380.0 $[M+H]^+$.

**Step 8**

**Intermediate 19: tert-butyl (S)-6-bromo-2-(1-methylpyrrolidin-2-yl)-1H-pyrrolo[3,2-c]pyridine-1-carboxylate**

**Intermediate 19** was prepared in an analogous manner to **Intermediate 1** using (2S)-2-{6-bromo-1H-pyrrolo[3,2-c]pyridin-2-yl}-1-methylpyrrolidine **(Intermediate 18)** and exhibited the following data: $^1H$ NMR (400 MHz, DMSO-d6) δ ppm 1.65 (s, 9H), 1.71-1.75 (m, 3H), 2.30-2.33 (m, 1H), 2.34 (s, 3H), 2.37-2.40 (m, 1H), 3.11-3.14 (m, 1H), 3.87-3.91 (m, 1H), 6.80 (s, 1H), 8.05 (s, 1H), 8.61 (s, 1H). Mass spec: m/z: 379.9 $[M+H]^+$. Mass spec: m/z: 380.0 $[M+H]^+$.

**Intermediate 7: *tert*-butyl (R)-2-ethynylpyrrolidine-1-carboxylate**

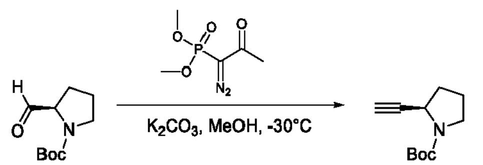


Intermediate 7

To a cooled (-30°C) solution of *tert*-butyl-(*R*)-2-formylpyrrolidine-1-carboxylate (CAS No: 73365-02-3, 50.0 g, 250.94 mmol) in methanol (500 mL) was added potassium carbonate (51.94 g, 376.41 mmol). Dimethyl(1-diazo-2-oxopropyl)phosphonate (CAS No: 90965-06-3, 57.85 g, 301.13 mmol) was then added dropwise over 20 min and the reaction mixture was stirred at -30°C for 3h. The reaction mixture was diluted with water (1000 mL) and extracted with diethyl ether (3 × 500 mL). The combined organic layers were dried over anhydrous $Na_2SO_4$ and concentrated *in vacuo* to obtain the crude compound. The crude material was purified by combi-flash chromatography, by eluting with 10% ethyl acetate in heptane, to afford *tert*-butyl (R)-2-ethynylpyrrolidine-1-carboxylate **(Intermediate 7,** 35.0 g, 71%) as a yellow liquid. $^1H$ NMR (400 MHz, DMSO-$d_6$) δ ppm 1.40 (s, 9H), 1.81-1.89 (m, 3H), 1.99-2.12 (m, 1H), 3.08-3.25 (m, 2H), 3.26-3.35 (m, 1H), 4.32-4.38 (m, 1H).

**Intermediate 2 was synthesized following Scheme 4**

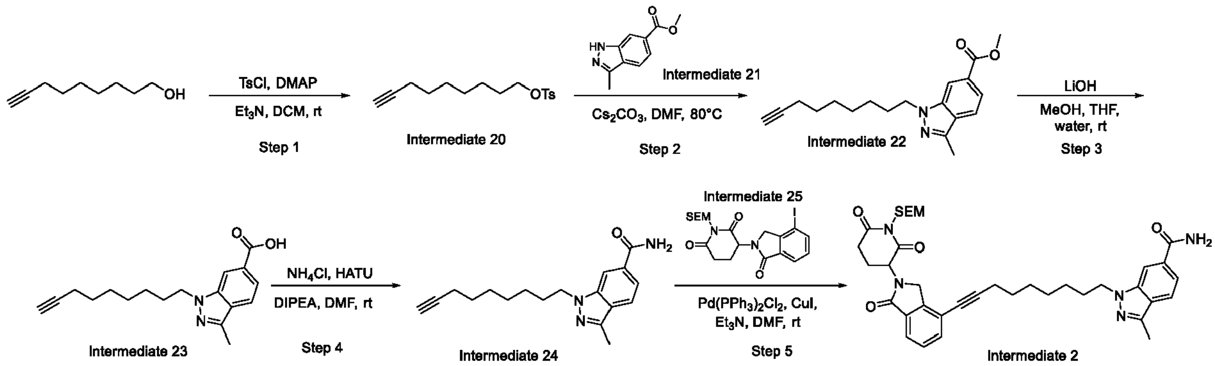


**Scheme 4**

**Step 1**

**Intermediate 20: non-8-yn-1-yl 4-methylbenzenesulfonate**

To a cooled (0°C) solution of non-8-yn-1-ol (CAS No: 10160-28-8, 25.0 g, 178.29 mmol) in dichloromethane (250 mL) was added trimethylamine (74.6 mL, 534.87 mmol) followed by 4-dimethylaminopyridine (2.30 g, 17.83 mmol), then p-toluene sulfonyl chloride (1.50 g, 7.9 mmol) and the reaction mixture was stirred at room temperature for 16h. The reaction mixture was then diluted with water (250 mL) and extracted with dichloromethane (3 × 250 mL). The combined organic layers were dried over anhydrous $Na_2SO_4$, and concentrated *in vacuo.* The crude material was purified by combi-flash chromatography, by eluting with 55% ethyl acetate in heptane, to afford non-8-yn-1-yl 4-methylbenzenesulfonate **(Intermediate 20, 34** g, 65%) as a colorless oil. $^1H$ NMR (400 MHz, DMSO-$d_6$) δ ppm 1.12-1.30 (m, 6H), 1.32-1.41 (m, 2H), 1.50-1.57 (m, 2H), 2.08-2.13 (m, 2H), 2.42 (s, 3H), 2.71 (s, 1H), 3.98-4.02 (m, 2H), 7.48 (d, *J*=7.88 Hz, 2H), 7.78 (d, *J*=8.29 Hz, 2H).

**Step 2**

**Intermediate 22: methyl 3-methyl-1-(non-8-yn-1-yl)-1*H*-indazole-6-carboxylate**

To a solution of non-8-yn-1-yl 4-methylbenzenesulfonate **(Intermediate 20,** 17.03 g, 57.83 mmol) in dimethylformamide (100 mL) was added cesium carbonate (51.4 g, 157.73 mmol) and methyl 3-methyl-1*H*-indazole-6-carboxylate **(Intermediate 21,** 10.0 g, 52.57 mmol) at room temperature. The reaction mixture was heated at 80°C for 2h then diluted with water (500 mL) and extracted with ethyl acetate (3 × 200 mL). The combined organic layers were washed with brine solution (2 × 200 mL), dried over $Na_2SO_4$, and concentrated *in vacuo.* The crude material was purified by combi-flash chromatography, by eluting with 30% ethyl acetate in heptane, to afford methyl 3-methyl-1-(non-8-yn-1-yl)-1*H*-indazole-6-carboxylate **(Intermediate 22,** 12 g, 67%) as an off-white solid. $^1H$ NMR (400 MHz, DMSO-$d_6$) δ ppm 1.18-1.32 (m, 6H), 1.34-1.43 (m, 2H), 1.76-1.83 (m, 2H), 2.09-2.13 (m, 2H), 2.51 (s, 3H), 2.70 (s, 1H), 3.90 (s, 3H), 4.40 (t, *J*=7.02 Hz, 2H), 7.65 (d, *J*=7.89 Hz, 1H), 7.81 (d, J=8.33 Hz, 1H), 8.23 (s, 1H). Mass spec: m/z 313.0 $[M+H]^+$.

**Step 3**

**Intermediate 23: 3-methyl-1-(non-8-yn-1-yl)-1*H*-indazole-6-carboxylic acid**

To a solution of methyl 3-methyl-1-(non-8-yn-1-yl)-1*H*-indazole-6-carboxylate **(Intermediate 22, 21** g, 50.3 mmol) in methanol (100 mL) and tetrahydrofuran (100 mL) and was added lithium hydroxide (4 g, 200 mmol) in water (50 mL) and the reaction mixture was stirred at room temperature for 16h. The reaction mixture was then concentrated *in vacuo.* The crude residue was taken up in water (30 mL) and acidified with 0.5 N HCl solution to pH~4. The resultant precipitate was collected by filtration and dried *in vacuo* to afford 3-methyl-1-(non-8-yn-1-yl)-1*H*-indazole-6-carboxylic acid **(Intermediate 23,** 19.0 g, 94%) as a white solid, which was used for the next step without further purification. $^1$H NMR (400 MHz, DMSO-$d_6$) δ ppm 1.26-1.38 (m, 8H), 1.75-1.78 (m, 2H), 2.08-2.11 (m, 2H), 2.52 (s, 3H), 2.70 (s, 1H), 4.33-4.37 (m, 2H), 7.63-7.72 (m, 2H), 8.13 (s, 1H). Mass spec m/z 299 $[M+H]^+$.

**Step 4**

**Intermediate 24: 3-methyl-1-(non-8-yn-1-yl)-1*H*-indazole-6-carboxamide**

To a solution of 3-methyl-1-(non-8-yn-1-yl)-1*H*-indazole-6-carboxylic acid **(Intermediate 23,** 18.0 g, 60.32 mmol) in dimethylformamide (180 mL) was added HATU (35.47 g, 90.48 mmol) and DIPEA (31.6 mL, 181.0 mmol) at room temperature. After stirring for 10 min, ammonium chloride (16.2 g, 301.6 mmol) was added and the reaction mixture was stirred at room temperature for a further 16h. The reaction mixture was then poured into ice cold water (200 mL), the resultant precipitate was collected by filtration and dried *in vacuo* to afford 3-methyl-1-(non-8-yn-1-yl)-1*H*-indazole-6-carboxamide **(Intermediate 24,** 17.0 g, 95%) as a white solid, which was used for the next step without further purification. $^1$H NMR (400 MHz, DMSO-$d_6$) δ ppm 1.19-1.33 (m, 6H), 1.35-1.44 (m, 2H), 1.75-1.87 (m, 2H), 2.09-2.13 (m, 2H), 2.51 (s, 3H), 2.71 (s, 1H), 4.31-4.35 (m, 2H), 7.42 (br s, 1H), 7.59 (d, *J*=8.29 Hz, 1H), 7.73 (d, *J*=8.29 Hz, 1H), 8.04 (br s, 1H), 8.12 (s, 1H). Mass spec m/z 296.0 $[M-H]^-$.

**Step 5**

**Intermediate 2: 1-(9-(2-(2, 6-dioxo-1-((2-(trimethylsilyl) ethoxy) methyl) piperidin-3-yl)-1-oxoisoindolin-4-yl) non-8-yn-1-yl)-3-methyl-1*H*-indazole-6-carboxamide**

To solution of 3-methyl-1-(non-8-yn-1-yl)-1*H*-indazole-6-carboxamide **(Intermediate 24,** 10.0 g, 33.62 mmol) and 3-(4-iodo-1-oxoisoindolin-2-yl)-1-((2-(trimethylsilyl)ethoxy)methyl)piperidine-2,6-dione **(Intermediate 25,** 16.83 g, 33.62 mmol) in dimethylformamide (100 mL) was added triethylamine (170 mL, 1210 mmol). The reaction mixture was purged with argon gas for 15 min then $PdCl_2(PPh_3)_2$ (2.38 g, 3.36 mmol) and copper (I) iodide (0.65 g, 3.36 mmol) were added. The reaction mixture was purged with argon gas for another 10 min and then stirred at room temperature for 2h. The reaction mixture was concentrated *in vacuo* to obtain the crude compound which was purified by combi-flash chromatography, by eluting with 80% ethyl acetate in heptane, to afford 1-(9-(2-(2,6-dioxo-1-((2-(trimethylsilyl)ethoxy)methyl) piperidin-3-yl)-1-oxoisoindolin-4-yl)non-8-yn-1-yl)-3-methyl-1*H*-indazole-6-carboxamide **(Intermediate 2, 16.0** g, 71%) as a yellow solid. $^1$H NMR (400 MHz, DMSO-$d_6$) δ ppm -0.04 (s, 9H), 0.79-0.86 (m, 2H), 1.24-1.41 (m, 6H), 1.49-1.56 (m, 2H), 1.78-1.85 (m, 2H), 2.01-2.09 (m, 1H), 2.42-2.45 (m, 3H), 2.50 (s, 3H), 2.73-2.76 (m, 2H), 3.01-3.11 (m, 1H), 3.48-3.55 (m, 2H), 4.23-4.27 (m, 1H), 4.30-4.37 (m, 2H), 4.43-4.47 (m, 1H), 5.00-5.08 (m, 2H), 7.41 (br s, 1H), 7.49-7.54 (m, 1H), 7.58-7.63 (m, 2H), 7.69-7.75 (m, 2H), 8.02 (br s, 1H), 8.12 (s, 1H). Mass spec m/z 670.1 $[M+H]^+$.

**Intermediate 25 was synthesised following Scheme 5**

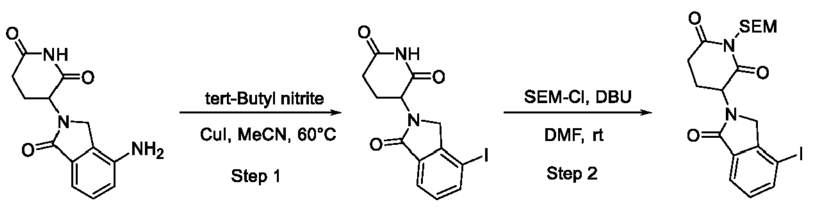


Intermediate 26

Intermediate 25

**Scheme 5**

**Step 1**

**Intermediate 26: 3-(4-iodo-1-oxoisoindolin-2-yl)piperidine-2,6-dione**

To a solution of 3-(4-amino-1-oxoisoindolin-2-yl)piperidine-2,6-dione (CAS No 191732-72-6, 25.0 g, 96.41 mmol) in acetonitrile (300 mL) was added copper(I) iodide (28.10 g, 144.6 mmol). The reaction mixture was purged with argon gas for 20 min then *tert*-butyl nitrite (16.57 g, 144.6 mmol) was added and the reaction mixture was heated at 60°C for 12h. The reaction mixture was poured then into water (300 mL), a solid precipitate formed, which was filtered and dried under vacuum to afford 3-(4-iodo-1-oxoisoindolin-2-yl) piperidine-2, 6-dione **(Intermediate 26, 45.2** g) as an off white solid, which was used for the next step without further purification. $^1$H NMR (400 MHz, DMSO-d6) δ ppm 1.98-2.04 (m, 2H), 2.82-2.98 (m, 2H), 4.11-4.32 (m, 2H), 5.13 (m, 1H), 7.31-7.42 (m, 1H), 7.71-7.80 (m, 1H), 7.98-8.06 (m, 1H), 10.99 (br s, 1H). Mass spec: m/z $[M+H]^+$371.0.

**Step 2**

**Intermediate 25: 3-(4-iodo-1-oxoisoindolin-2-yl)-1-((2-(trimethylsilyl) ethoxy)methyl) piperidine-2,6-dione**

To solution of 3-(4-iodo-1-oxoisoindolin-2-yl)piperidine-2,6-dione **(Intermediate 24,** 30.0 g, 81.06 mmol) in dimethylformamide (250 mL) was added DBU (44.07 g, 283.7 mmol) at 0°C, then 2-(trimethylsilyl)ethoxymethyl chloride (52 mL, 283.7 mmol) was added and the reaction mixture was stirred at room temperature for 16h under a nitrogen atmosphere. The reaction mixture was quenched with water (700 mL), diluted with ethyl acetate (500 mL) and filtered through a celite bed. The organic layer was separated, washed with water (500 mL), dried over anhydrous $Na_2SO_4$ and concentrated in vacuo. The crude material was purified by combi-flash chromatography, eluting with 50% ethyl acetate in heptane to afford 3-(4-iodo-1-oxoisoindolin-2-yl)-1-((2-(trimethylsilyl) ethoxy) methyl) piperidine-2,6-dione **(Intermediate 25,** 8.56 g, 21%) as an off-white solid. $^1$H NMR (400 MHz, DMSO-d6) δ ppm -0.02 (s, 9H), 0.78-0.90 (m, 2H), 2.03-2.11 (m, 1H), 2.76-2.82 (m, 1H), 3.02-3.12 (m, 1H), 3.48-3.60 (m, 3H), 4.08-4.13 (m, 1H), 4.28-4.33 (m, 1H), 5.02-5.10 (m, 2H), 5.26-5.31 (m, 1H), 7.31-7.37 (m, 1H), 7.79 (d, J=7.46 Hz, 1H), 8.05 (d, J=7.46 Hz, 1H). Mass spec: m/z $[M-H]^-$ 498.8.

**Intermediate 21 was synthesized following Scheme 6**

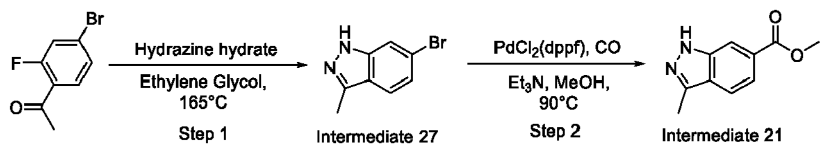


**Scheme 6**

**Step** 1

**Intermediate 27: 6-bromo-3-methyl-1*H*-indazole**

To a solution of 1-(4-bromo-2-fluorophenyl)ethan-1-one (CAS No: 625446-22-2, 25.0 g, 115.19 mmol) in ethylene glycol (160.0 mL, 2850 mmol) was added hydrazine hydrate solution (9.18 mL, 287.97 mmol) at room temperature and the reaction mixture was heated at 165°C for 16h. The reaction mixture was quenched with water (250 mL) resulting in a precipitate which was filtered, washed with water (3 × 150 mL) and pentane (3 × 60 mL) and dried under vacuum to afford 6-bromo-3-methyl-1H-indazole **(Intermediate 27,** 22 g, 90%) as an off-white solid, which was used for the next step without further purification. $^1$H NMR (400 MHz, DMSO-$d_6$) δ ppm 2.47 (s, 3H), 7.19 (d, *J*=8.80 Hz, 1H), 7.62-7.68 (m, 2H), 12.73 (br s, 1H). Mass spec: m/z: 213.0 $[M+2]^+$.

**Step 2**

**Intermediate 21: methyl 3-methyl-1*H*-indazole-6-carboxylate**

To a solution of 6-bromo-3-methyl-1*H*-indazole **(Intermediate 27,** 15.00 g, 71.07 mmol) in methanol (200 mL) was added triethylamine (29.9 mL, 213.2 mmol) followed by [1,1'-bis(diphenylphosphino)ferrocene] dichloropalladium(II)

(5.25 g, 7.11 mmol) at room temperature and the reaction mixture was heated at 90°C for 16h under a CO atmosphere (100 psi). The reaction mixture was filtered through celite and concentrated *in vacuo* to obtain crude compound. The crude material was purified by combi-flash column chromatography by eluting with 25% EtOAc in heptane to afford methyl 3-methyl-1*H*-indazole-6-carboxylate **(Intermediate 21,** 10.5 g, 78%) as an off white solid. $^1$H NMR (400 MHz, DMSO-$d_6$) δ ppm 2.52 (s, 3H), 3.88 (s, 3H), 7.64 (d, J=8.32 Hz, 1H), 7.81 (d, J=8.32 Hz, 1H), 8.08 (s, 1H), 13.00 (br s, 1H). Mass spec: m/z: 190.8 $[M+H]^+$.

**Example 2 was synthesized following Scheme 7**

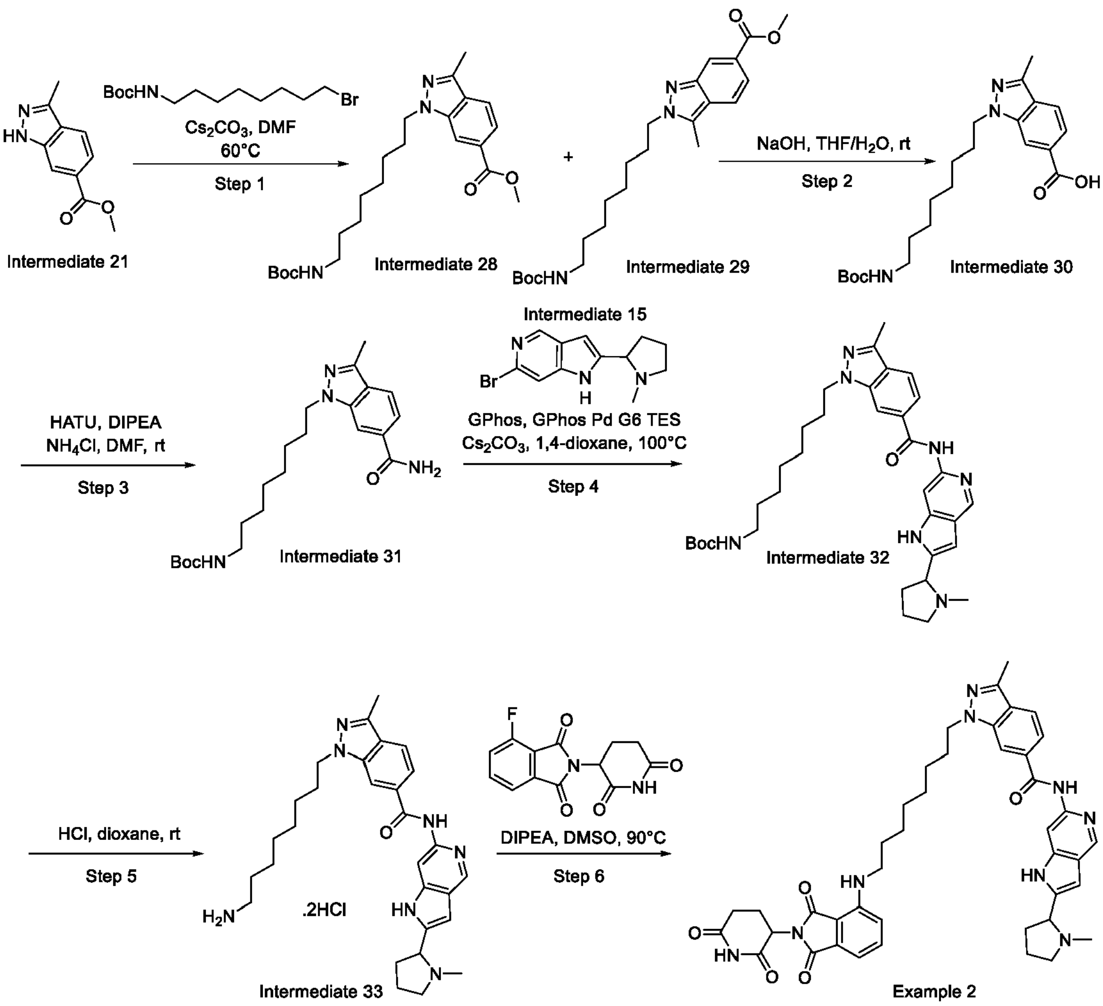


Scheme 7

**Step 1**

**Intermediate 28: methyl 1-{8-[(tert-butoxycarbonyl)amino]octyl}-3-methylindazole-6-carboxylate and Intermediate 29: methyl 2-(8-((tert-butoxycarbonyl)amino)octyl)-3-methyl-2H-indazole-6-carboxylate**

To a solution of methyl 3-methyl-1H-indazole-6-carboxylate (Intermediate 21, 12.0 g, 56.8 mmol) in DMF (150 mL) was added tert-butyl (8-bromooctyl)carbamate (21.0 g, 68.2 mmol) and $Cs_2CO_3$ (55.5 g, 170 mmol). and the mixture was stirred at 60°C overnight. Water (200 mL) was added and the resulting solution was extracted with ethyl acetate (3 × 400 mL) and the organic layers were combined, washed with brine (1000 mL), dried over anhydrous sodium sulfate, filtered and concentrated under reduced pressure. The residue was chromatographed on a silica gel column, eluting with ethyl acetate/petroleum ether (65/35), to afford methyl 1-(8-((tert-butoxycarbonyl)amino)octyl)-3-methyl-1H-indazole-6-carboxylate **(Intermediate 28,** 20.0 g, 76%) as a yellow oil and methyl 2-(8-((tert-butoxycarbonyl)amino)octyl)-3-methyl-2H-indazole-6-carboxylate **(Intermediate 29,** 7.0 g, 27%) as a yellow oil.

**Intermediate 28** exhibited: $^1$H NMR (400 MHz, DMSO-d6) δ ppm 8.20 (s, 1H), 7.78 (d, *J* = 8.4 Hz, 1H), 7.64 (d, *J* = 8.4 Hz, 1H), 6.72 (br, 1H), 4.37 (t, *J* = 6.8 Hz, 2H), 3.90 (s, 3H), 2.85 - 2.87 (m, 2H), 1.78 -1.79 (m, 2H), 1.30 - 1.35 (m, 13H), 1.17 - 1.22 (m, 9H). Mass spec: m/z: 418.0 $[M+H]^+$.

**Intermediate 29** exhibited: $^1$H NMR (400 MHz, DMSO-d6) δ ppm 8.21 (s, 1H), 7.78 (d, *J* = 8.8 Hz, 1H), 7.49 (d, *J* = 8.8 Hz, 1H), 6.74 (t, *J* = 5.2 Hz, 1H), 4.38 (t, *J* = 7.2 Hz, 2H), 3.87 (s, 3H), 2.87 - 2.88 (m, 2H), 2.65 (s, 3H), 1.86 (br, 2H), 1.32 - 1.36 (m, 10H), 1.22 - 1.28 (m, 9H). Mass spec: m/z: 418.0 $[M+H]^+$.

**Step 2**

**Intermediate 30: 1-18-[(tert-butoxycarbonyl)amino]octyll-3-methylindazole-6-carboxylic acid**

To a solution of methyl 1-{8-[(tert-butoxycarbonyl)amino]octyl}-3-methylindazole-6-carboxylate **(Intermediate 28:** 500 mg, 1.08 mmol) in $H_2O$ (30 mL)/THF (30 mL) was added NaOH (3.00 g, 75.0 mmol). The reaction mixture was stirred at rt for 2h, followed by addition of water. The solution was adjusted to pH = 6 with a solution of HCl (12N in $H_2O$). The mixture was extracted three times with EtOAc and the combined organic layers were washed with brine, dried over anhydrous $Na_2SO_4$, filtered, and concentrated under reduced pressure to afford 1-{8-[(tert-butoxycarbonyl)amino] octyl}-3-methylindazole-6-carboxylic acid **(Intermediate 30:** 451 mg, 91%) which was used in Step 3 without further purification. Mass spec: m/z: 404 $[M+H]^+$.

Step 3

**Intermediate 31: tert-butyl N-[8-(6-carbamoyl-3-methylindazol-1-yl)octyl]carbamate**

To a solution of 1-{8-[(tert-butoxycarbonyl)amino]octyl}-3-methylindazole-6-carboxylic acid **(Intermediate 30**: 451 mg, 1.13 mmol) in DMF (15 mL) were added $NH_4Cl$ (265 mg, 5.00 mmol), DIPEA (961 mg, 7.43 mmol) and HATU (612 mg, 1.61mmol). The reaction mixture was stirred at rt overnight and quenched with water. The mixture was extracted three times with EtOAc and the combined organic layers were washed with brine, dried over anhydrous $Na_2SO_4$, filtered, and concentrated under reduced pressure to afford tert-butyl N-[8-(6-carbamoyl-3-methylindazol-1-yl)octyl]carbamate (**Intermediate 31**: 265 mg, 65%) as a yellow solid which was used in Step 4 without further purification. Mass spec: m/z: 403 $[M+H]^+$.

**Step 4**

**Intermediate 32: rac-tert-butyl N-[8-(3-methyl-6-{[2-(1-methylpyrrolidin-2-yl)-1H-pyrrolo[3,2-c]pyridin-6-yl]carbamoyl}indazol-1-yl)octyl]carbamate**

To a solution of tert-butyl N-[8-(6-carbamoyl-3-methylindazol-1-yl)octyl]carbamate (**Intermediate 31**: 230 mg, 0.496 mmol) in 1,4-dioxane (8 mL) were added rac-2-{6-bromo-1H-pyrrolo[3,2-c]pyridin-2-yl}-1-methylpyrrolidine **(Intermediate 15**: 160 mg, 0.513 mmol), $Cs_2CO_3$ (558 mg, 1.71 mmol), GPhos Pd G6 TES (54.0 mg, 0.0570 mmol) and GPhos (30.7 mg, 0.0570 mmol). The reaction mixture was stirred at 100°C for 3h under nitrogen atmosphere and then quenched with water. The mixture was extracted three times with EtOAc, the combined organic layers were washed with brine, dried over anhydrous $Na_2SO_4$, filtered, and concentrated under reduced pressure. The crude material was purified by flash chromatography on silica gel using DCM/MeOH (85/15) as eluent to afford rac-tert-butyl N-[8-(3-methyl-6-{ [2-(1-methylpyrrolidin-2-yl)-1H-pyrrolo[3,2-c]pyridin-6-yl]carbamoyl}indazol-1-yl)octyl]carbamate (**Intermediate 32**: 230 mg, 77%) as a yellow solid. Mass spec: m/z: 602 $[M+H]^+$.

**Step 5**

**Intermediate 33: rac-1-(8-aminooctyl)-3-methyl-N-[2-(1-methylpyrrolidin-2-yl)-1H-pyrrolo[3,2-c]pyridin-6-yl]indazole-6-carboxamide dihydrochloride**

To a solution of rac-tert-butyl N-[8-(3-methyl-6-{ [2-(1-methylpyrrolidin-2-yl)-1H-pyrrolo[3,2-c]pyridin-6-yl]carbamoyl}indazol-1-yl)octyl]carbamate (**Intermediate 32**: 160 mg, 0.256 mmol) in 1,4-dioxane (2 mL) was added a solution of HCl (1 mL, 12N in $H_2O$). The reaction mixture was stirred at rt for 2h and concentrated under reduced pressure to afford rac-1-(8-aminooctyl)-3-methyl-N-[2-(1-methylpyrrolidin-2-yl)-1H-pyrrolo[3,2-c]pyridin-6-yl]indazole-6-carboxamide dihydrochloride (**Intermediate 33**: 130 mg, 96%) as a yellow solid. Mass spec: m/z: 502 $[M+H]^+$.

**Step 6**

**Example 2: rac-1-(8-{[2-(2,6-dioxopiperidin-3-yl)-1,3-dioxoisoindol-4-yl]amino}octyl)-3-methyl-N-[2-(1-methylpyrrolidin-2-yl)-1H-pyrrolo[3,2-c]pyridin-6-yl]indazole-6-carboxamide**

To a solution of rac-1-(8-aminooctyl)-3-methyl-N-[2-(1-methylpyrrolidin-2-yl)-1H-pyrrolo[3,2-c]pyridin-6-yl]indazole-6-carboxamide hydrochloride (**Intermediate 33**: 130 mg, 0.247 mmol) in DMF (5 mL) were added 2-(2,6-dioxopiperidin-3-yl)-4-fluoroisoindole-1,3-dione (CAS: 835616-60-9, 71.6 mg, 0.260 mmol) and $K_2CO_3$ (143 mg, 1.04 mmol). The reaction mixture was stirred at 90°C overnight and then quenched with water. The mixture was extracted three times with EtOAc, the combined organic layers were washed with brine, dried over anhydrous $Na_2SO_4$, filtered, and concentrated under reduced pressure. The crude material was purified by flash chromatography on silica gel using DCM/MeOH (95/5) as eluent, followed by a second purification using preparative HPLC (column: Kinetex EVO C18 21.2 × 250 mm, 5.0 um; Eluent: $H_2O$ (10 mmol/L $NH_4HCO_3$)/MeCN from 49/51 to 19/81; Flow rate: 25 mL/min) to afford rac-1-(8-{[2-(2,6-dioxopiperidin-3-yl)-1,3-dioxoisoindol-4-yl]amino}octyl)-3-methyl-N-[2-(1-methylpyrrolidin-2-yl)-1H-pyrrolo[3,2-c]pyridin-6-yl]indazole-6-carboxamide (**Example 2:** 9.6 mg, 5%) as a yellow solid. Mass spec: m/z: 758 $[M+H]^+$; $^1H$ NMR (300 MHz, MeOH-$d_4$) δ ppm 8.52 (s, 1H), 8.20 (d, J = 6.6 Hz, 2H), 7.81 - 7.84 (m, 1H), 7.70 - 7.74 (m, 1H), 7.44 - 7.49 (m, 1H), 6.93 - 6.98 (m, 2H), 6.51 (s, 1H), 4.40 - 4.44 (m, 1H), 3.20 - 3.64 (m, 5H), 2.70 - 2.85 (m, 3H), 2.58 (s, 3H), 2.35 - 2.38 (m, 1H), 2.29 (s, 3H), 1.89 - 2.09 (m, 6H), 1.55 - 1.57 (m, 2H), 1.28 - 1.32 (m, 9H).

**Example 3 was synthesised following Scheme 8**

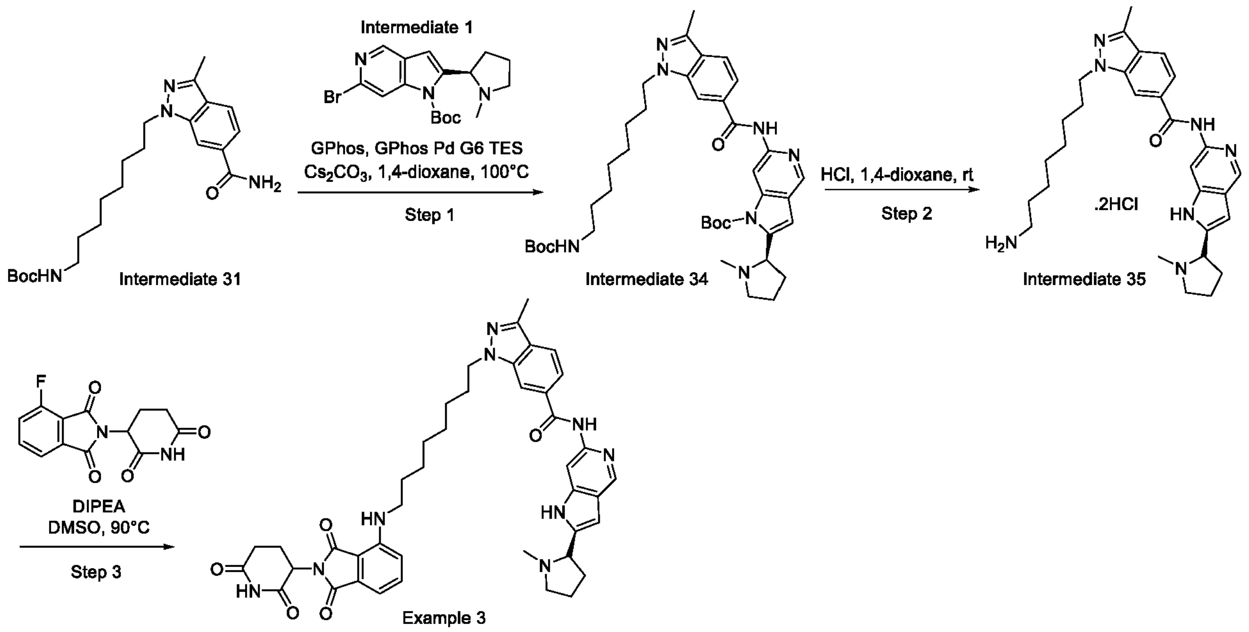


Scheme 8

**Step 1**

**Intermediate 34: tert-butyl 6-(1-{8-[(tert-butoxycarbonyl)amino]octyl}-3-methylindazole-6-amido)-2-[(2R)-1-methylpyrrolidin-2-yl]pyrrolo[3,2-c]pyridine-1-carboxylate**

To a solution of tert-butyl N-[8-(6-carbamoyl-3-methylindazol-1-yl)octyl]carbamate (**Intermediate 31:** 200 mg, 0.621 mmol) in 1,4-dioxane (6.00 ml) were added tert-butyl 6-bromo-2-[(2R)-1-methylpyrrolidin-2-yl]pyrrolo[3,2-c]pyridine-1-carboxylate (**Intermediate 1:** 212 mg, 0.559 mmol), $Cs_2CO_3$ (1.01 g, 3.11 mmol) followed by Gphos (31.9 mg, 0.106 mmol) and Gphos Pd G6 TES (43.3 mg, 0.154 mmol). The reaction mixture was stirred at 100°C for 3h under nitrogen atmosphere and quenched with water. The mixture was extracted three times with EtOAc, the combined organic layers were washed with brine, dried over anhydrous $Na_2SO_4$, filtered, and concentrated under reduced pressure. The crude material was purified by flash chromatography on silica gel using DCM/MeOH (95/5) as eluent to afford tert-butyl

6-(1-{8-[(tert-butoxycarbonyl)amino]octyl}-3-methylindazole-6-amido)-2-[(2R)-1-methylpyrrolidin-2-yl]pyrrolo[3,2-c]pyridine-1-carboxylate (**Intermediate 34:** 250 mg, 75%) as an off-white solid. Mass spec: m/z: 602 $[M+H-Boc]^+$.

**Step 2**

**Intermediate 35: 1-(8-aminooctyl)-3-methyl-N-{2-[(2R)-1-methylpyrrolidin-2-yl]-1H-pyrrolo[3,2-c]pyridin-6-yl}indazole-6-carboxamide dihydrochloride**

To a solution of tert-butyl 6-(1-{8-[(tert-butoxycarbonyl)amino]octyl}-3-methylindazole-6-amido)-2-[(2R)-1-methylpyrrolidin-2-yl]pyrrolo[3,2-c]pyridine-1-carboxylate (**Intermediate 34**: 250 mg, 0.299 mmol) in 1,4-dioxane (3 mL) was added a solution of HCl (1.5 mL, 12N in $H_2O$). The reaction mixture was stirred at rt for 2h and concentrated under reduced pressure to afford 1-(8-aminooctyl)-3-methyl-N-{2-[(2R)-1-methylpyrrolidin-2-yl]-1H-pyrrolo[3,2-c]pyridin-6-yl}indazole-6-carboxamide dihydrochloride (**Intermediate 35**: 170 mg, 95%) as an off-white solid. Mass spec: m/z: 502 $[M+H]^+$.

**Step 4**

**Example 3: 1-(8-{[2-(2,6-dioxopiperidin-3-yl)-1,3-dioxoisoindol-4-yl]amino}octyl)-3-methyl-N-{2-[(2R)-1-methylpyrrolidin-2-yl]-1H-pyrrolo[3,2-c]pyridin-6-yl}indazole-6-carboxamide**

To a solution of 1-(8-aminooctyl)-3-methyl-N-{2-[(2R)-1-methylpyrrolidin-2-yl]-1H-pyrrolo[3,2-c]pyridin-6-yl}indazole-6-carboxamide dihydrochloride (**Intermediate 35**: 150 mg, 0.299 mmol) in DMSO (1 mL) were added 2-(2,6-dioxopiperidin-3-yl)-4-fluoroisoindole-1,3-dione (CAS: 835616-60-9, 165 mg, 0.598 mmol) and DIPEA (231 mg, 1.79 mmol). The reaction mixture was stirred at 90°C overnight and concentrated under reduced pressure. The crude material was purified by preparative HPLC (Column: XBridge OBD, 30 × 150 mm, 5μm; Eluent: $H_2O$ (10 mmol/L $NH_4HCO_3$)/MeCN from 1/1 to 20/80; Flow rate: 60 mL/min) to afford 1-(8-{[2-(2,6-dioxopiperidin-3-yl)-1,3-dioxoisoindol-4-yl]amino}octyl)-3-methyl-N-{2-[(2R)-1-methylpyrrolidin-2-yl]-1H-pyrrolo[3,2-c]pyridin-6-yl}indazole-6-carboxamide (**Example 3**: 45.1 mg, 20%) as a yellow solid. $^1H$ NMR (400 MHz, DMSO-$d_6$) δ ppm 11.43 (s, 1H), 11.01 - 11.09 (m, 1H), 10.61 (s, 1H), 8.51 (s, 1H), 8.47 (s, 1H), 8.28 (s, 1H), 7.73 - 7.82 (m, 2H), 7.53 - 7.59 (m, 1H), 6.92 - 7.06 (m, 2H), 6.53 - 6.59 (m, 1H), 6.41 (s, 1H), 5.02 - 5.09 (m, 1H), 4.36 - 4.58 (m, 2H), 3.32 (s, 3H), 3.13-3.17 (m, 1H), 2.84 - 2.88 (m, 1H), 2.67 - 2.72 (m, 4H), 2.24 - 2.28 (m, 1H), 2.14 -2.17 (m, 4H), 1.99 - 2.03 (m, 1H), 1.74 - 1.90 (m, 5H), 1.53 - 1.62 (m, 2H), 1.21 - 1.36 (m, 9H). Mass spec: m/z: 758 $[M+H]^+$.

**Example 4: 1-(8-{[2-(2,6-dioxopiperidin-3-yl)-1,3-dioxoisoindol-4-yl]amino}octyl)-3-methyl-N-{2-[(2S)-1-methylpyrrolidin-2-yl]-1H-pyrrolo [3,2-c] pyridin-6-yl}indazole-6-carboxamide**

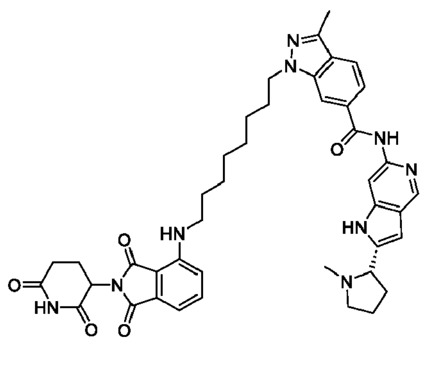

Following an analogous procedure to **Scheme 8**, **Step 3**, using 1-(8-aminooctyl)-3-methyl-N-{2-[(2S)-1-methylpyrrolidin-2-yl]-1H-pyrrolo[3,2-c]pyridin-6-yl}indazole-6-carboxamide dihydrochloride (**Intermediate 36**: 50 mg, 0.119 mmol), after purification by reverse-phase flash chromatography (C18) using MeCN/$H_2O$ (80/20) as eluent, afforded 1-(8-{[2-(2,6-dioxopiperidin-3-yl)-1,3-dioxoisoindol-4-yl]amino}octyl)-3-methyl-N-{2-[(2S)-1-methylpyrrolidin-2-yl]-1H-pyrrolo[3,2-c]pyridin-6-yl}indazole-6-carboxamide (**Example 4**: 26.4 mg, 35%) as a yellow solid. Mass spec: m/z: 758 $[M+H]^+$, $^1H$ NMR (400 MHz, DMSO-$d_6$) δ ppm 11.41 (s, 1H), 11.09 (s, 1H), 10.56 (s, 1H), 8.53 (s, 1H), 8.44 (s, 1H), 8.26 (s, 1H), 7.72 - 7.79 (m, 2H), 7.54 -7.58 (m, 1H), 6.99 - 7.05 (m, 2H), 6.43 - 6.53 (m, 2H), 5.02 - 5.06 (m, 1H), 4.38 - 4.41 (m, 2H), 3.17 (s, 3H), 2.84 - 2.87 (m, 1H), 2.51 -2.60 (m, 5H), 2.01 - 2.31 (m, 6H), 1.89 - 2.02 (m, 5H), 1.57 - 1.61 (m, 2H), 1.87 - 1.91

(m, 9H).

**Intermediate 36: 1-(8-aminooctyl)-3-methyl-N-{2-[(2S)-1-methylpyrrolidin-2-yl]-1H-pyrrolo[3,2-c]pyridin-6-yl}indazole-6-carboxamide dihydrochloride**

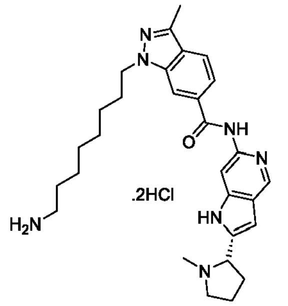

Following an analogous procedure to **Scheme 8**, **Step 2**, using tert-butyl 6-(1-{8-[(tert-butoxycarbonyl)amino]octyl}-3-methylindazole-6-amido)-2-[(2S)-1-methylpyrrolidin-2-yl]pyrrolo[3,2-c]pyridine-1-carboxylate (**Intermediate 37**, 120 mg, 0.141 mmol), afforded 1-(8-aminooctyl)-3-methyl-N-{2-[(2S)-1-methylpyrrolidin-2-yl]-1H-pyrrolo[3,2-c]pyridin-6-yl}indazole-6-carboxamide dihydrochloride (**Intermediate 36**: 50 mg, 90%) as an off-white solid. Mass spec: m/z: 502 $[M+H]^+$.

**Intermediate 37: tert-butyl 6-(1-{8-[(tert-butoxycarbonyl)amino]octyl}-3-methylindazole-6-amido)-2-[(2S)-1-methylpyrrolidin-2-yl]pyrrolo[3,2-c]pyridine-1-carboxylate**

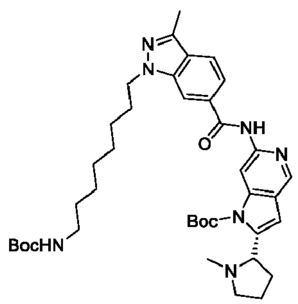

Following an analogous procedure to **Scheme 8**, **Step 1**, using tert-butyl N-[8-(6-carbamoyl-3-methylindazol-1-yl)octyl]carbamate (**Intermediate 31**: 190 mg, 0.472 mmol) and tert-butyl 6-bromo-2-[(2S)-1-methylpyrrolidin-2-yl]pyrrolo[3,2-c]pyridine-1-carboxylate (**Intermediate 19**: 162 mg, 0.424 mmol), afforded tert-butyl 6-(1-{8-[(tert-butoxycarbonyl)amino]octyl}-3-methylindazole-6-amido)-2-[(2S)-1-methylpyrrolidin-2-yl]pyrrolo[3,2-c]pyridine-1-carboxylate (**Intermediate 37**: 120 mg, 25%) as an off-white solid. Mass spec: m/z: 602 $[M+H-Boc]^+$.

**Example 5 was synthesised following Scheme 9**

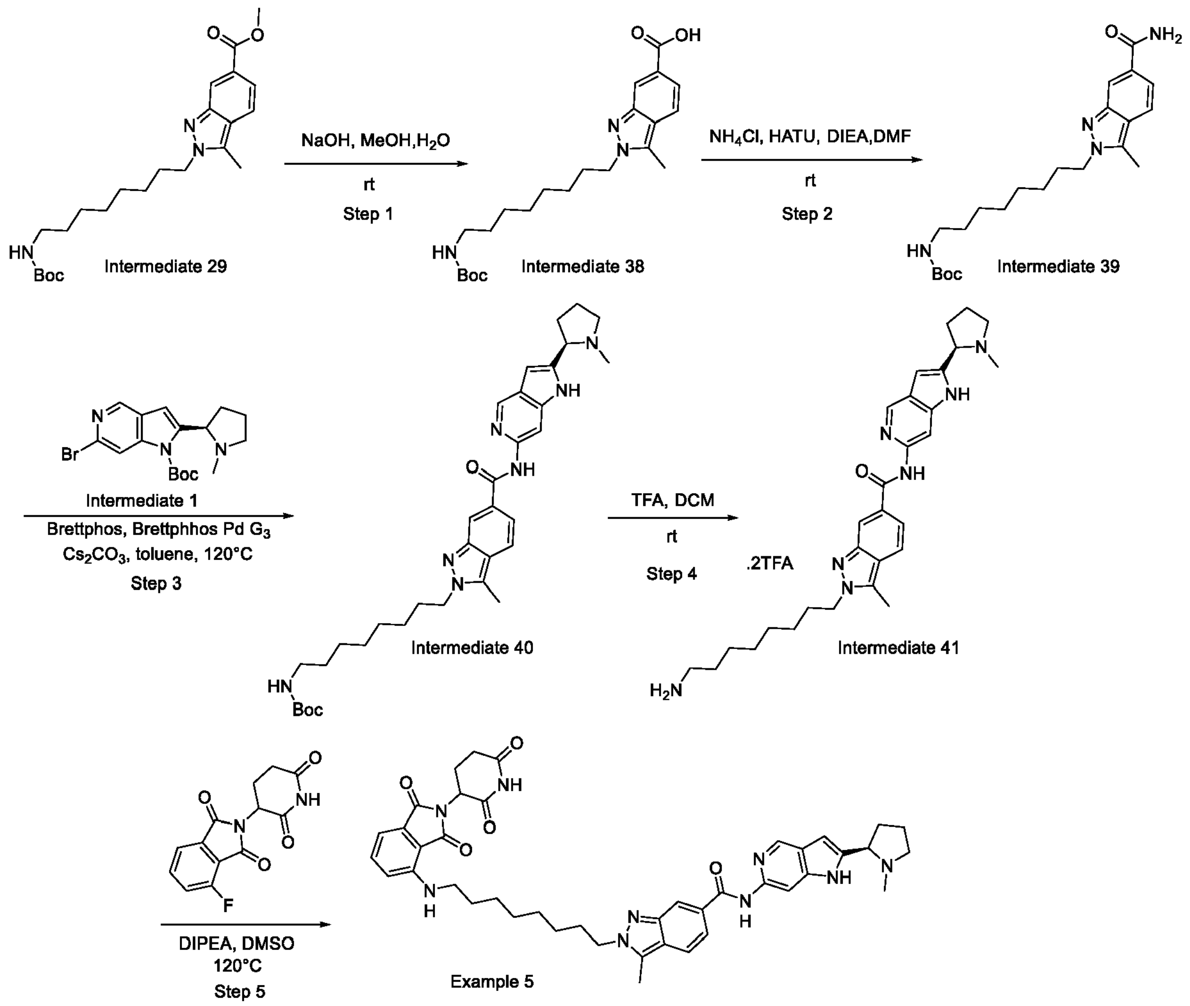


**Scheme 9**

**Step 1**

**Intermediate 38: 2-(8-((tert-butoxycarbonyl)amino)octyl)-3-methyl-2H-indazole-6-carboxylic acid**

To a solution of methyl 2-(8-((tert-butoxycarbonyl)amino)octyl)-3-methyl-2H-indazole-6-carboxylate (**Intermediate 29**, 500 mg, 1.14 mmol, 95%) in MeOH (10 mL) was added NaOH (800 mg, 21.1 mmol) in $H_2O$ (10 mL) and the mixture was stirred at room temperature for 3h. The pH of the mixture was adjusted to 6 by the addition of HCl (1M). The resulting solution was extracted with ethyl acetate (3 × 50 mL) and the organic layers were combined, washed with brine (200 mL), dried over anhydrous sodium sulfate, filtered and concentrated under reduced pressure to afford 2-(8-((tert-butoxycarbonyl)amino)octyl)-3-methyl-2H-indazole-6-carboxylic acid (**Intermediate 38**, 480 mg, 99%) as a yellow solid, which was used in the next step without further purification. Mass spec: m/z 404.3 $[M+H]^+$.

**Step 2**

**Intermediate 39: tert-butyl (8-(6-carbamoyl-3-methyl-2H-indazol-2-yl)octyl)carbamate**

To a solution of 2-(8-((tert-butoxycarbonyl)amino)octyl)-3-methyl-2H-indazole-6-carboxylic acid (**Intermediate 38,** 250 mg, 0.589 mmol) in DMF (5 mL) was added $NH_4Cl$ (126 mg, 2.36 mmol), HATU (336 mg, 0.883 mmol) and DIPEA (456 mg, 3.53 mmol) and the mixture was stirred at room temperature for 3h. Water (10 mL) was added and the resulting solution was extracted with ethyl acetate (3 × 20 mL). The organic layers were combined, washed with brine (100 mL), dried over anhydrous sodium sulfate, filtered and concentrated under reduced pressure. The residue was chromatographed on a silica gel column, eluting with methanol/dichloromethane (5/95), to afford tert-butyl (8-(6-carbamoyl-3-methyl-2H-indazol-2-yl)octyl)carbamate (**Intermediate 39,** 240 mg, 98%) as yellow oil. Mass spec: m/z 403.2 $[M+H]^+$.

**Step 3**

**Intermediate 40: tert-butyl (R)-(8-(3-methyl-6-((2-(1-methylpyrrolidin-2-yl)-1H-pyrrolo[3,2-c]pyridin-6-yl)carbamoyl)-2H-indazol-2-yl)octyl)carbamate**

To a solution of tert-butyl (8-(6-carbamoyl-3-methyl-2H-indazol-2-yl)octyl)carbamate (**Intermediate 39**, 200 mg, 0.482 mmol) in toluene (10 mL) was added tert-butyl 6-bromo-2-[(2R)-1-methylpyrrolidin-2-yl]pyrrolo[3,2-c]pyridine-1-carboxylate (**Intermediate 1**, 183 mg, 0.482 mmol), Brettphos (155 mg, 0.289 mmol), Brettphos Pd $G_3$ (131 mg, 0.145 mmol) and $Cs_2CO_3$ (314 mg, 0.964 mmol) and the mixture was stirred at 120°C for overnight under nitrogen. The reaction was then concentrated under reduced pressure and the resulting residue was chromatographed on a silica gel column, eluting with MeOH/DCM (4/96), to afford tert-butyl (R)-(8-(3-methyl-6-((2-(1-methylpyrrolidin-2-yl)-1H-pyrrolo[3,2-c]pyridin-6-yl)carbamoyl)-2H-indazol-2-yl)octyl)carbamate (**Intermediate 40**, 150 mg, 41%) as yellow oil. Mass spec: m/z: 702.4 $[M+H]^+$.

**Step 4**

**Intermediate 41: (R)-2-(8-aminooctyl)-3-methyl-N-(2-(1-methylpyrrolidin-2-yl)-1H-pyrrolo[3,2-c]pyridin-6-yl)-2H-indazole-6-carboxamide ditrifluoroacetate**

To a solution of tert-butyl (R)-(8-(3-methyl-6-((2-(1-methylpyrrolidin-2-yl)-1H-pyrrolo[3,2-c]pyridin-6-yl)carbamoyl)-2H-indazol-2-yl)octyl)carbamate (**Intermediate 40**, 150 mg, 0.197 mmol) in DCM (2 mL) was added TFA (1 mL) and the mixture was stirred at room temperature for 1h. The volatiles were removed under reduced pressure to afford (R)-2-(8-aminooctyl)-3-methyl-N-(2-(1-methylpyrrolidin-2-yl)-1H-pyrrolo[3,2-c]pyridin-6-yl)-2H-indazole-6-carboxamide ditrifluoroacetate (**Intermediate 41**, 100 mg, 97%) as yellow oil, which was used in the next step without further purification. Mass spec: m/z: 502.3 $[M+H]^+$.

**Step 5**

**Example 5: 2-(8-((2-(2,6-dioxopiperidin-3-yl)-1,3-dioxoisoindolin-4-yl)amino)octyl)-3-methyl-N-(2-((R)-1-methylpyrrolidin-2-yl)-1H-pyrrolo[3,2-c]pyridin-6-yl)-2H-indazole-6-carboxamide**

To a solution of (R)-2-(8-aminooctyl)-3-methyl-N-(2-(1-methylpyrrolidin-2-yl)-1H-pyrrolo[3,2-c]pyridin-6-yl)-2H-indazole-6-carboxamide ditrifluoroacetate (**Intermediate 41**, 100 mg, 0.191 mmol) in DMSO (4 mL) was added 2-(2,6-dioxopiperidin-3-yl)-4-fluoroisoindole-1,3-dione (CAS No: 835616-60-9, 79.3 mg, 0.286 mmol) and DIPEA (148 mg, 1.15 mmol) and the reaction mixture was stirred at 120°C for overnight. Water (5 mL) was added the resulting crude was purified by preparative HPLC (Column: WelFlash C18, Regular C18 20-40 μm, 120g; Mobile Phase A: Water (with 10 mmol/L $NH_4HCO_3$ + 0.05% $NH_3.H_2O$), Mobile Phase B: MeCN; Flow rate: 60 mL/min; Gradient: 35% B to 65% B in 30 min) to afford 2-(8-((2-(2,6-dioxopiperidin-3-yl)-1,3-dioxoisoindolin-4-yl)amino)octyl)-3-methyl-N-(2-((R)-1-methylpyrrolidin-2-yl)-1H-pyrrolo[3,2-c]pyridin-6-yl)-2H-indazole-6-carboxamide (**Example 5**, 15.2 mg, 10%) as a yellow solid. $^1H$ NMR (400 MH, DMSO-d6) δ ppm 11.37 (s, 1H), 11.10 (s, 1H), 10.43 (s, 1H), 8.51 (s, 1H), 8.29 (s, 1H), 8.20 (s, 1H), 7.75 (d, *J* = 8.8Hz, 1H), 7.58 (t, *J* = 8.0Hz, 2H), 7.09 (d, *J* = 8.8Hz, 1H), 7.02 (d, *J* = 6.8Hz, 1H), 6.52 (t, *J* = 5.2Hz, 1H), 6.40 (s, 1H), 5.03 - 5.07 (m, 1H), 4.39 (t, *J* = 7.2Hz, 2H), 3.24 - 3.32 (m, 2H), 3.15 (t, *J* = 7.6Hz, 1H), 2.87 - 2.93 (m, 1H), 2.83 - 2.84 (m, 3H), 2.57 - 2.65 (m, 1H), 2.26 - 2.34 (m, 1H), 2.12 - 2.17 (m, 4H), 1.99 - 2.11 (m, 2H), 1.78 - 1.97 (m, 6H), 1.51 - 1.59 (m, 2H), 1.29 - 1.39 (m, 8H). Mass spec: m/z: 758.5 $[M+H]^+$.

**Example 6 were synthesized following Scheme 10**

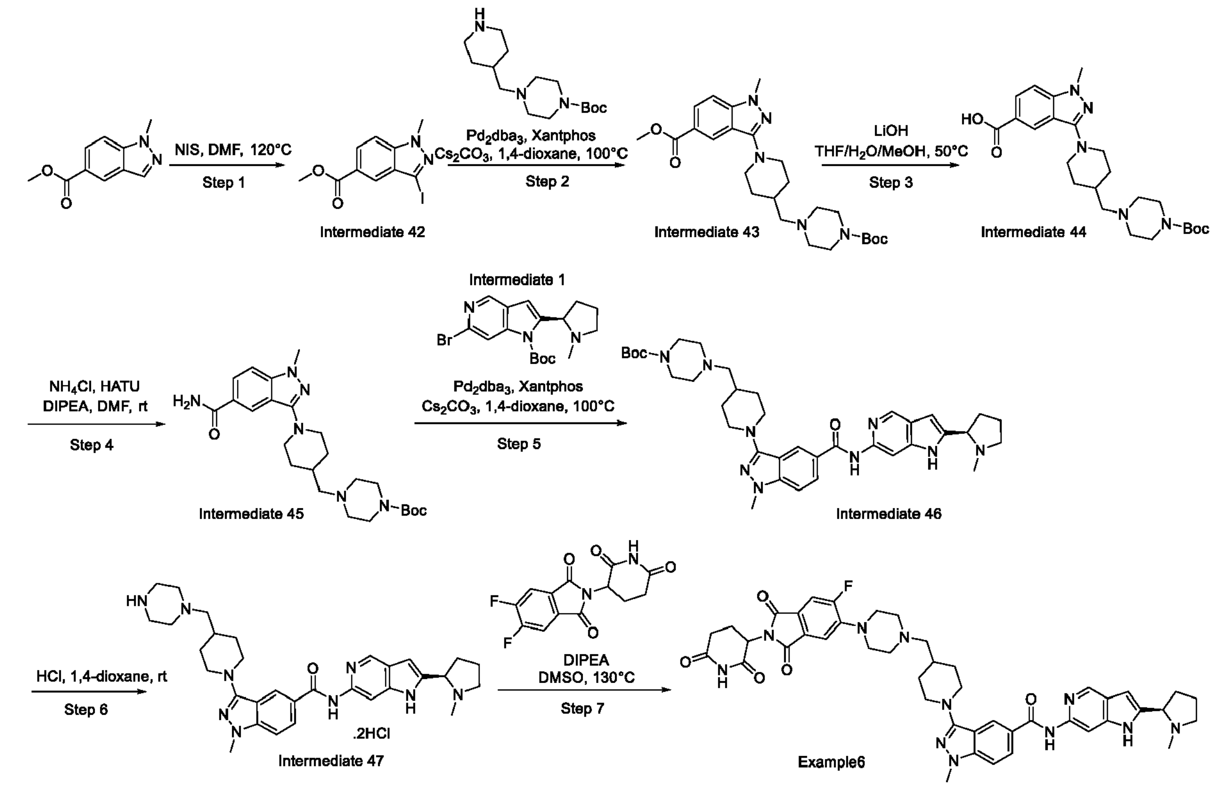


**Scheme 10**

**Step** 1

**Intermediate 42: methyl 3-iodo-1-methylindazole-5-carboxylate**

To a solution of methyl 1-methylindazole-5-carboxylate (CAS: 1092351-82-0, 5.00 g, 25.5 mmol) in DMF (80 mL) was added NIS (13.2 g, 58.6 mmol). The reaction mixture was stirred at 120°C overnight and then quenched with water. The mixture was extracted three times with EtOAc, the combined organic layers were washed with brine, dried over anhydrous $Na_2SO_4$, filtered, and concentrated under reduced pressure. The crude material was purified by flash chromatography on silica gel using petroleum ether/EtOAc (8/2) as eluent to afford methyl 3-iodo-1-methylindazole-5-carboxylate (**Intermediate 42**, 5 g, 25%) as a yellow solid. Mass spec: m/z: 317 $[M+H]^+$.

**Step 2**

**Intermediate 43: methyl 3-(4-{[4-(tert-butoxycarbonyl)piperazin-1-yl]methyl}piperidin-1-yl)-1-methylindazole-5-carboxylate**

To a solution of methyl 3-iodo-1-methylindazole-5-carboxylate (**Intermediate 42**, 5.00g, 6.33 mmol) and *tert*-butyl 4-(piperidin-4-ylmethyl)piperazine-1-carboxylate (CAS: 1211568-27-2, 1.80 g, 6.33 mmol) in 1,4-dioxane (80 mL) were added Xantphos (0.730 g, 1.26 mmol), $Cs_2CO_3$ (6.18 g, 19.0 mmol) and $Pd_2dba_3$ (0.580 g, 0.633 mmol). The reaction mixture was stirred at 100°C overnight under nitrogen atmosphere. The reaction mixture was quenched with water and extracted three times with EtOAc. The combined organic extracts were washed with brine, dried over anhydrous $Na_2SO_4$, filtered, and concentrated under vacuum. The crude material was purified by flash chromatography on silica gel using petroleum ether/EtOAc (1/2) as eluent to afford methyl 3-(4-{[4-(tert-butoxycarbonyl)piperazin-1-yl]methyl}piperidin-1-yl)-1-methylindazole-5-carboxylate (**Intermediate 43**, 1.70 g, 44%) as a yellow solid. Mass spec: m/z: 472 $[M+H]^+$.

**Step 3**

**Intermediate 44: 3-(4-1[4-(tert-butoxycarbonyl)piperazin-1-yl]methyllpiperidin-1-yl)-1-methylindazole-5-carboxylic acid**

To a solution of methyl 3-(4-{[4-(tert-butoxycarbonyl)piperazin-1-yl]methyl}piperidin-1-yl)-1-methylindazole-5-carboxylate (**Intermediate 43**, 1.70 g, 2.81 mmol) in THF (30 mL)/MeOH (10 mL)/$H_2O$ (8 mL) was added LiOH (0.67 g, 28.1 mmol). The reaction mixture was stirred at 50°C for 3h. The reaction mixture was acidified to pH = 5 with an aqueous solution HCl 4M, then concentrated under reduced pressure. The crude material was chromatographed by reverse-phase flash chromatography (C18) using MeCN/$H_2O$ (72/28) as eluent to afford 3-(4-{[4-(tert-butoxycarbonyl)piperazin-1-yl]methyl}piperidin-1-yl)-1-methylindazole-5-carboxylic acid (**Intermediate 44**, 1.10 g, 77%) as a yellow solid. Mass spec: m/z: 458 $[M+H]^+$.

**Step 4**

**Intermediate 45: tert-butyl 4-{[1-(5-carbamoyl-1-methylindazol-3-yl)piperidin-4-yl] methyl} piperazine-1 -carboxylate**

A solution of 3-(4-{[4-(tert-butoxycarbonyl)piperazin-1-yl]methyl}piperidin-1-yl)-1-methylindazole-5-carboxylic acid (**Intermediate 44**, 1.00 g, 1.97 mmol), DIPEA (1.27 g, 9.83 mmol) and HATU (0.820 g, 2.16 mmol) in DMF (15 mL) was stirred at rt for 1h. Then a solution of $NH_4Cl$ (0.320 g, 5.90 mmol) in $H_2O$ (1 mL) was added dropwise and the mixture was stirred for another 1h. The reaction mixture was quenched with water and concentrated under reduced pressure. The crude material was chromatographed by reverse-phase flash chromatography (C18) using MeCN/$H_2O$ (75/25) as eluent to afford tert-butyl 4-{[1-(5-carbamoyl-1-methylindazol-3-yl)piperidin-4-yl]methyl}piperazine-1-carboxylate (**Intermediate 45**, 900 mg, 98%) as a yellow solid. Mass spec: m/z: 457 $[M+H]^+$.

**Step 5**

**Intermediate 46: tert-butyl 4-({1-[1-methyl-5-({2-[(2R)-1-methylpyrrolidin-2-yl]-1H-pyrrolo[3,2-c]pyridin-6-yl}carbamoyl)indazol-3-yl]piperidin-4-yl}methyl)piperazine-1-carboxylate**

To a solution of tert-butyl 4-{[1-(5-carbamoyl-1-methylindazol-3-yl)piperidin-4-yl]methyl}piperazine-1-carboxylate (**Intermediate 45,** 200 mg, 0.430 mmol) and tert-butyl 6-bromo-2-[(2R)-1-methylpyrrolidin-2-yl]pyrrolo[3,2-c]pyridine-1-carboxylate (**Intermediate 1**, 212 mg, 0.558 mmol) in 1,4-dioxane (6 mL) were added $Cs_2CO_3$ (420 mg, 1.29 mmol), Xantphos (99.3 mg, 0.172 mmol) and $Pd_2dba_3$ (39.3 mg, 0.043 mmol). The reaction mixture was stirred at 100°C overnight under nitrogen atmosphere, then concentrated under reduced pressure. The residue was chromatographed by reverse-phase flash chromatography (C18) using MeCN/$H_2O$ (28/72) as eluent to afford tert-butyl 4-({1-[1-methyl-5-({2-[(2R)-1-methylpyrrolidin-2-yl]-1H-pyrrolo[3,2-c]pyridin-6-yl}carbamoyl)indazol-3-yl]piperidin-4-yl}methyl) piperazine-1-carboxylate (**Intermediate 46:** 180 mg, 56%) as a yellow solid. Mass spec: m/z: 656 $[M+H]^+$.

**Step 6**

**Intermediate 47: 1-methyl-N-{2-[(2R)-1-methylpyrrolidin-2-yl]-1H-pyrrolo[3,2-c]pyridin-6-yl}-3-[4-(piperazin-1-ylmethyl)piperidin-1-yl]indazole-5-carboxamide dihydrochloride**

To a stirred solution of tert-butyl 4-{[1-(5-{[1-(tert-butoxycarbonyl)-2-[(2R)-1-methylpyrrolidin-2-yl]pyrrolo[3,2-c]pyridin-6-yl]carbamoyl}-1-methylindazol-3-yl)piperidin-4-yl]methyl}piperazine-1-carboxylate (**Intermediate 46**, 180 mg, 0.207 mmol) in 1,4-dioxane (5 mL) was added a solution of HCl (2 mL, 12N in $H_2O$). The reaction mixture was stirred at rt for 2h, then concentrated under reduced pressure to afford 1-methyl-N-{2-[(2R)-1-methylpyrrolidin-2-yl]-1H-pyrrolo[3,2-c]pyridin-6-yl}-3-[4-(piperazin-1-ylmethyl)piperidin-1-yl]indazole-5-carboxamide dihydrochloride (120 mg, 95%) as a yellow solid. Mass spec: m/z: 556 $[M+H]^+$.

**Step 7**

**Example 6: 3-[4-({4-[2-(2,6-dioxopiperidin-3-yl)-6-fluoro-1,3-dioxoisoindol-5-yl]piperazin-1-yl}methyl)piperidin-1-yl]-1-methyl-N-{2-[(2R)-1-methylpyrrolidin-2-yl]-1H-pyrrolo[3,2-c]pyridin-6-yl}indazole-5-carboxamide**

A solution of 1-methyl-N-{2-[(2R)-1-methylpyrrolidin-2-yl]-1H-pyrrolo[3,2-c]pyridin-6-yl} -3 -[4-(piperazin-1 -ylmethyl)piperidin-1 -yl]indazole-5-carboxamide dihydrochloride (**Intermediate 47**, 80.0 mg, 0.131 mmol) in DMSO (3 mL) were added 2-(2,6-dioxopiperidin-3-yl)-5,6-difluoroisoindole-1,3-dione (CAS: 1496997-41-1, 46.2 mg, 0.157 mmol) and

DIPEA (169 mg, 1.31 mmol). The reaction mixture was stirred at 130°C for 2h, quenched with water and concentrated under reduced pressure. The residue was purified by preparative HPLC (Column: XBridge OBD, 30 × 150 mm, 5μm; Eluent: Water (10mmol/L $NH_4HCO_3$ + 0.05% $NH_3H_2O$)/MeCN from 72/28 to 42/58; Flow rate: 60 mL/min) to afford 3-[4-({4-[2-(2,6-dioxopiperidin-3-yl)-6-fluoro-1,3-dioxoisoindol-5-yl]piperazin-1-yl}methyl)piperidin-1-yl]-1-methyl-N-{2-[(2R)-1-methylpyrrolidin-2-yl]-1H-pyrrolo[3,2-c]pyridin-6-yl}indazole-5-carboxamide (**Example 6**, 20.5 mg, 18%) as a yellow solid. Mass spec: m/z: 830 $[M+H]^+$, $^1H$ NMR (400 MHz, DMSO-$d_6$) δ ppm 11.36 (s, 1H), 11.10 (s, 1H), 10.59 (s, 1H), 8.52 (d, J = 6.0 Hz, 2H), 8.20 (s, 1H), 8.02 (d, J = 9.6 Hz, 1H), 7.72 - 7.75 (m, 1H), 7.45 - 7.52 (m, 2H), 6.39 (s, 1H), 5.08 - 5.13 (m, 1H), 4.00 - 4.03 (m, 2H), 3.87 (s, 3H), 3.27 - 3.31 (m, 5H), 3.14 - 3.16 (m, 1H), 2.84 - 2.87 (m, 3H), 2.67 (s, 1H), 2.50 - 2.57 (m, 5H), 2.30 - 2.32 (m, 3H), 2.14 - 2.28 (m, 4H), 2.02 - 2.04 (m, 1H), 1.90 - 2.04 (m, 4H), 1.81 - 1.88 (m, 2H), 1.32 - 1.46 (m, 2H).

**Example 7 was synthesized following Scheme 11**

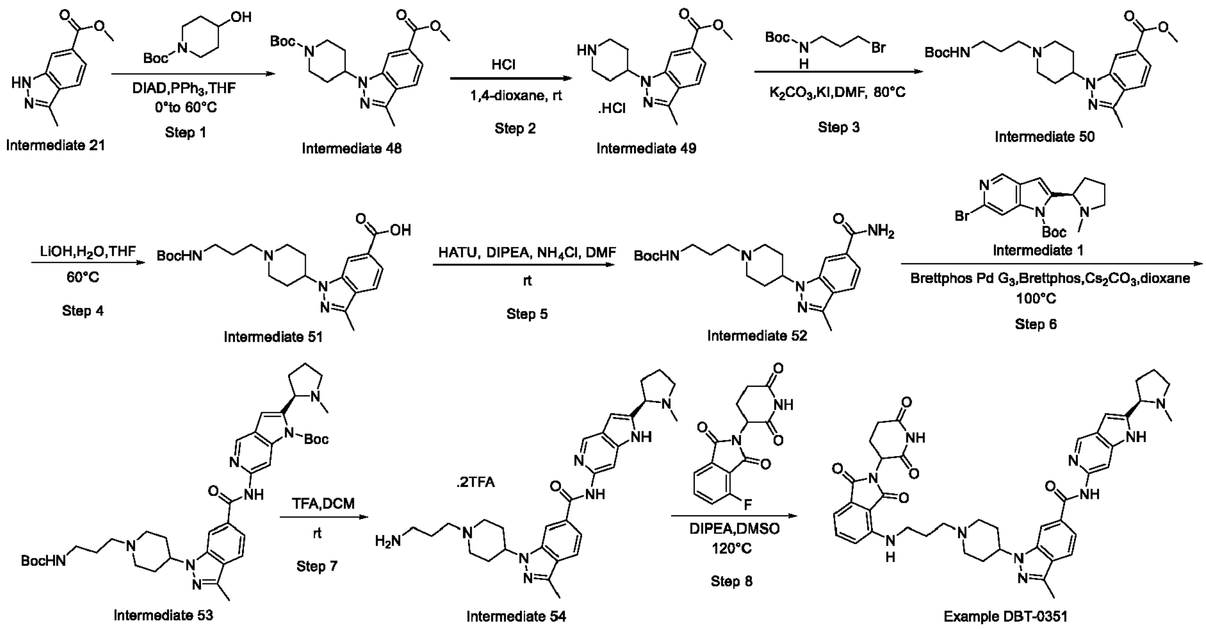


**Scheme 11**

**Step 1**

**Intermediate 48: methyl-1-(1-(*tert*-butoxycarbonyl)piperidin-4-yl)-3-methyl-1H-indazole-6-carboxylate**

To a cooled (0°C) solution of methyl 3-methyl-1H-indazole-6-carboxylate (**Intermediate 21**, 400 mg, 2.10 mmol), $PPh_3$ (1.10 g, 4.21 mmol) and *tert*-butyl 4-hydroxypiperidine-1-carboxylate (CAS No: 109384-19-2, 846 mg, 4.21 mmol) in tetrahydrofuran (10 mL) was added DIAD (850 mg, 4.21 mmol) dropwise and the resulting mixture was stirred overnight at 60°C. Water (300 mL) was then added and the mixture extracted with ethyl acetate (3 × 300 mL). The combined organic layers were washed with brine, dried over anhydrous $Na_2SO_4$, filtered and concentrated *in vacuo.* The crude material was purified by combi-flash column chromatography, by eluting with 14% ethyl acetate in heptane, then further purified by reverse-phase C18 combi-flash column chromatography, by eluting with 78% acetonitrile in water, to afford methyl 1-(1-(tert-butoxycarbonyl)piperidin-4-yl)-3-methyl-1H-indazole-6-carboxylate (**Intermediate 48**, 290 mg, 35%) as a colorless oil. Mass spec: m/z: 374.0 $[M+H]^+$.

**Step 2**

**Intermediate 49: methyl-3-methyl-1-(piperidin-4-yl)-1H-indazole-6-carboxylate hydrochloride**

A solution of methyl-1-[1-(*tert*-butoxycarbonyl)piperidin-4-yl]-3-methylindazole-6-carboxylate (**Intermediate 48**, 290 mg, 0.787 mmol) in HCl (4M in 1,4-dioxane) was stirred at room temperature for 2h. The resulting mixture

was concentrated *in vacuo* to afford methyl 3-methyl-1-(piperidin-4-yl)-1H-indazole-6-carboxylate hydrochloride (**Intermediate 49**, 200 mg) as a colorless oil, which was used in the next step without further purification. Mass spec: m/z: 274.0 $[M+H]^+$.

**Step 3**

**Intermediate 50: methyl-1-(1-{3-[(*tert*-butoxycarbonyl)amino]propyl}piperidin-4-yl)-3-methylindazole-6-carboxylate**

To a solution of methyl-3-methyl-1-(piperidin-4-yl)indazole-6-carboxylate hydrochloride (**Intermediate 49**, 200 mg, 0.732 mmol) and *tert*-butyl N-(3-bromopropyl)carbamate (CAS No: 83948-53-2, 261 mg, 1.10 mmol) in dimethylformamide (6 mL) was added potassium carbonate (303 mg, 2.19 mmol) and potassium iodide (24.3 mg, 0.146 mmol) and the reaction mixture was stirred at 80 °C for 2h. Water (200 mL) was added and the aqueous phase extracted with ethyl acetate (3 × 200 mL). The combined organic layers were washed with brine, dried over anhydrous $Na_2SO_4$, filtered, and concentrated *in vacuo.* The crude material was purified by combi-flash column chromatography, by eluting with 4% methanol in dichloromethane, to afford methyl-1-(1-{3-[(*tert*-butoxycarbonyl)amino]propyl}piperidin-4-yl)-3-methylindazole-6-carboxylate (**Intermediate 50**, 170 mg, 48%) as a colorless oil. Mass spec: m/z: 431.0 $[M+H]^+$.

**Step 4**

**Intermediate 51: 1-(1-(3-((tert-butoxycarbonyl)amino)propyl)piperidin-4-yl)-3-methyl-1H-indazole-6-carboxylic acid**

To a solution of methyl-1-(1-{3-[(*tert*-butoxycarbonyl)amino]propyl}piperidin-4-yl)-3-methylindazole-6-carboxylate (**Intermediate 50**, 170 mg, 0.395 mmol) in THF (4 mL) and $H_2O$ (1 mL) was added LiOH (47.4 mg, 1.97 mmol) and the reaction mixture was stirred at 60°C for 2h. The pH of the solution was adjusted to pH 5 by the dropwise addition of dilute aqueous HCl (1M) and the resulting mixture was extracted with $CH_3Cl$/i-PrOH (3:1, 3 × 200 mL). The combined organic layers were washed with brine, dried over anhydrous $Na_2SO_4$, filtered and concentrated *in vacuo* to afford 1-(1-(3-((*tert*-butoxycarbonyl)amino)propyl)piperidin-4-yl)-3-methyl-1H-indazole-6-carboxylic acid (**Intermediate 51**, 150 mg, 88%) as a brown solid, which was used in the next step without further purification. Mass spec: m/z: 417.0 $[M+H]^+$.

**Step 5**

**Intermediate 52: *tert*-butyl-(3-(4-(6-carbamoyl-3-methyl-1H-indazol-1-yl)piperidin-1-yl)propyl)carbamate**

To a solution of 1-(1-{3-[(tert-butoxycarbonyl)amino]propyl}piperidin-4-yl)-3-methylindazole-6-carboxylic acid (**Intermediate 51**, 150 mg, 0.360 mmol) in DMF (4 mL) was added $NH_4Cl$ (57.8 mg, 1.08 mmol), HATU (205 mg, 0.540 mmol) and DIPEA (233 mg, 1.80 mmol) and the reaction mixture was stirred at room temperature for 2h. The reaction mixture was purified directly by reverse-phase C18 combi-flash column chromatography, by eluting with 70% acetonitrile in water, to afford *tert*-butyl-(3-(4-(6-carbamoyl-3-methyl-1H-indazol-1-yl)piperidin-1-yl)propyl)carbamate (**Intermediate 52**, 80 mg, 52%) as a brown oil. Mass spec: m/z: 416.0 $[M+H]^+$.

**Step 6**

**Intermediate 53: *tert*-butyl-(*R*)-6-(1-(1-(3-((tert-butoxycarbonyl)amino)propyl)piperidin-4-yl)-3-methyl-1H-indazole-6-carboxamido)-2-(1-methylpyrrolidin-2-yl)-1H-pyrrolo[3,2-c]pyridine-1-carboxylate**

To a solution of *tert*-butyl-N-{3-[4-(6-carbamoyl-3-methylindazol-1-yl)piperidin-1-yl]propyl}carbamate (**Intermediate 52**, 80 mg, 0.193 mmol) and *tert*-butyl-6-bromo-2-[(2R)-1-methylpyrrolidin-2-yl]pyrrolo[3,2-c]pyridine-1-carboxylate (**Intermediate 1**, 73.2 mg, 0.193 mmol) in 1,4-dioxane (3 mL) was added BrettPhos Pd G3 (52.4 mg, 0.058 mmol), BrettPhos (62.0 mg, 0.116 mmol) and $Cs_2CO_3$ (125 mg, 0.386 mmol) and the reaction mixture was stirred at 100°C for 4h. The reaction mixture was concentrated *in vacuo* and the resulting residue was purified by preparative TLC, by eluting with 10% methanol in dichloromethane, to afford *tert*-butyl-(*R*)-6-(1-(1-(3-((tert-butoxycarbonyl)amino)propyl)piperidin-4-yl)-3-methyl-1H-indazole-6-carboxamido)-2-(1-methylpyrrolidin-2-yl)-1H-pyrrolo[3,2-c]pyridine-1-carboxylate (**Intermediate 53**, 80 mg, 57%) as a yellow oil. Mass spec: m/z: 715.0 $[M+H]^+$.

**Step** 7

**Intermediate 54: 1-[1-(3-aminopropyl)piperidin-4-yl]-3-methyl-N-{2-[(2R)-1-methylpyrrolidin-2-yl]-1H-pyrrolo [3,2-c]pyridin-6-yl}indazole-6-carboxamide ditrifluoroacetate**

To a solution of *tert*-butyl 6-[1-(1-{3-[(tert-butoxycarbonyl)amino]propyl}piperidin-4-yl)-3-methylindazole-6-amido]-2-[(2R)-1-methylpyrrolidin-2-yl]pyrrolo[3,2-c]pyridine-1-carboxylate (**Intermediate 53**, 75 mg, 0.105 mmol) in dichloromethane (5 mL) was added TFA (1 mL) and the reaction mixture was stirred at room temperature for 2h. The resulting mixture was concentrated *in vacuo* to afford 1-[1-(3-aminopropyl)piperidin-4-yl]-3-methyl-N-{2-[(2R)-1-methylpyrrolidin-2-yl]-1H-pyrrolo[3,2-c]pyridin-6-yl}indazole-6-carboxamide ditrifluoroacetate **(Intermediate 54**, 55 mg, 67%) as a brown oil, which was used in the next step without further purification. Mass spec: m/z: 515.0 $[M+H]^+$.

**Step 8**

**Example 7: 1-[1-(3-{[2-(2,6-dioxopiperidin-3-yl)-1,3-dioxoisoindol-4-yl]amino}propyl)piperidin-4-yl]-3-methyl-N-{2-[(2R)-1-methylpyrrolidin-2-yl]-1H-pyrrolo[3,2-c]pyridin-6-yl}indazole-6-carboxamide**

To a solution of 1-[1-(3-aminopropyl)piperidin-4-yl]-3-methyl-N-{2-[(2R)-1-methylpyrrolidin-2-yl]-1H-pyrrolo[3,2-c]pyridin-6-yl}indazole-6-carboxamide ditrifluoroacetate (**Intermediate 54**, 55 mg, 0.107 mmol) and 2-(2,6-dioxopiperidin-3-yl)-4-fluoroisoindole-1,3-dione (CAS No: 835616-60-9, 44.3 mg, 0.161 mmol) in DMSO (3 mL) was added DIPEA (82.9 mg, 0.642 mmol) and the reaction mixture was stirred at 120°C for 2h. The reaction mixture was then purified directly by preparative HPLC (Column: XBridge Prep OBD C18 Column, 30*150 mm, 5μm; Mobile Phase A: Water (10 mmol/L $NH_4HCO_3$), Mobile Phase B: MeCN; Flow rate: 60 mL/min; Gradient: 35% B to 65 % B in 10 min), to afford 1-[1-(3-{[2-(2,6-dioxopiperidin-3-yl)-1,3-dioxoisoindol-4-yl]amino}propyl)piperidin-4-yl]-3-methyl-N-{2-[(2R)-1-methylpyrrolidin-2-yl]-1H-pyrrolo[3,2-c]pyridin-6-yl}indazole-6-carboxamide (**Example 7**, 11.7 mg, 14%) as a yellow solid. $^1$H NMR (400 MHz, MeOH-d4) δ ppm 8.54 (s, 1H), 8.24 - 8.28 (m, 2H), 7.84 (d, *J* = 8.4 Hz, 1H), 7.74 (d, *J* = 8.4 Hz, 1H), 7.59 (t, *J* = 8.4 Hz, 1H), 7.14 (d, *J* = 8.8 Hz, 1H), 7.06 (d, *J* = 7.2 Hz, 1H), 6.54 (s, 1H), 5.05 - 5.09 (m, 1H), 4.60 - 4.79 (m, 4H), 3.47 - 3.49 (m, 3H), 3.17 - 3.25 (m, 3H), 2.85 - 2.87 (m, 1H), 2.76 - 2.77 (m, 2H), 2.74 - 2.75 (m, 1H), 2.68 - 2.72 (m, 5H), 2.32 - 2.60 (m, 9H), 2.05 - 2.21 (m, 5H), 1.89 - 1.99 (m, 3H). Mass spec: m/z: 771.5 $[M+H]^+$.

**Example 8 was synthesized following Scheme 12**

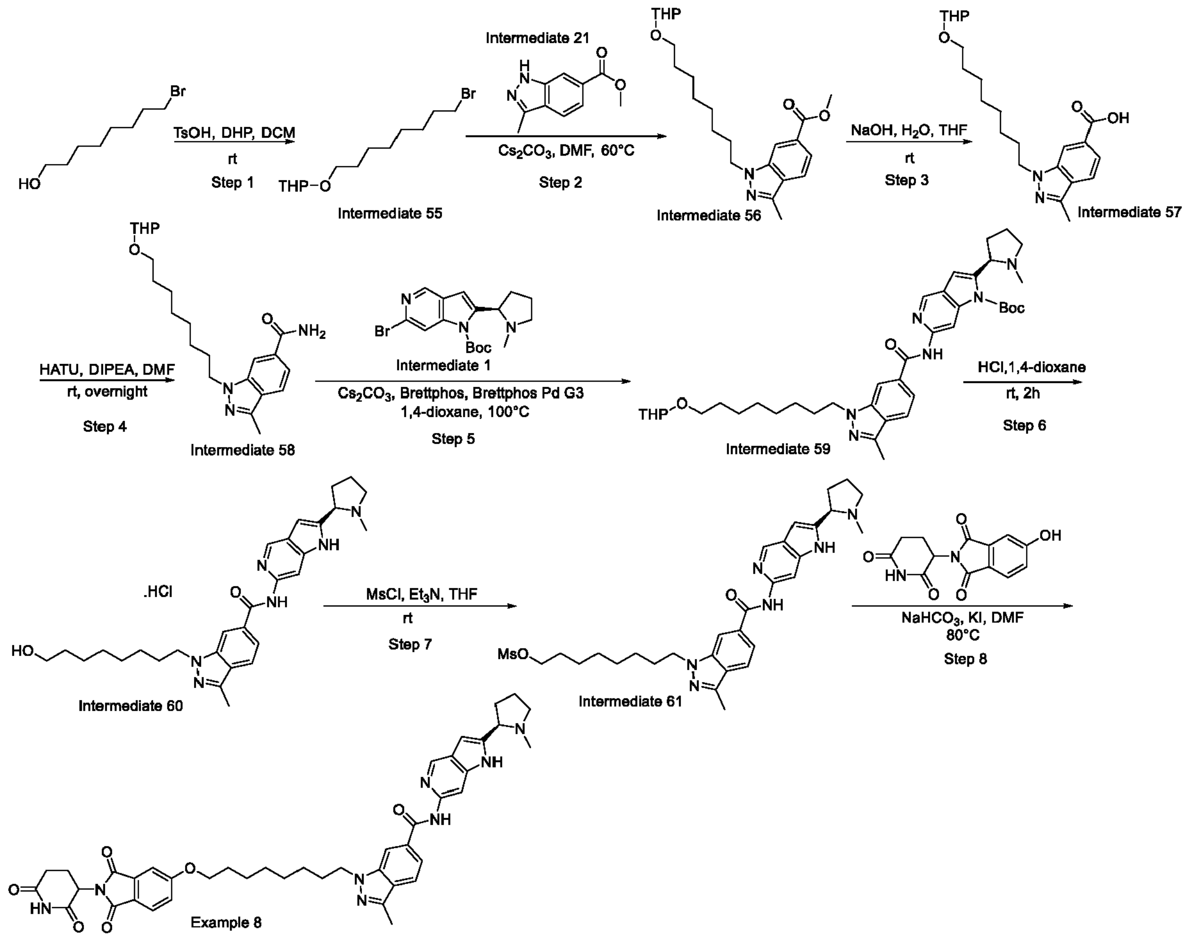


**Scheme 12**

**Step 1**

**Intermediate 55: 2-[(8-bromooctyl)oxy]oxane**

To a solution of 8-bromooctan-1-ol (CAS No: 50816-19-8, 4.00 g, 19.1 mmol) and DHP (4.83 g, 57.3 mmol) in DCM (30.0 mL) was added TsOH (99.6 mg, 1.92 mmol) at 0°C. The reaction was stirred at room temperature overnight and quenched with water (300 ml). The resulting solution was extracted with EtOAc (3 × 100 mL) and the organic layers were combined, washed with brine (3 × 50 mL),dried over anhydrous sodium sulfate, filtered and concentrated under reduced pressure to afford 2-[(8-bromooctyl)oxy]oxane (**Intermediate 55**, 4.70 g, 80%) as a yellow oil. $^1$H NMR (400 MHz, DMSO-d6) δ ppm 4.53 - 4.54 (m, 1H), 3.70 - 3.76 (m, 1H), 3.58 - 3.63 (m, 1H), 3.51 - 3.54 (m, 2H), 3.40 - 3.44 (m, 1H), 3.24 - 3.30 (m, 1H), 1.76 - 1.81 (m, 2H), 1.70 - 1.73 (m, 1H), 1.57 - 1.68 (m, 3H), 1.32 - 1.47 (m, 7H), 1.29 (br, 5H).

**Step 2**

**Intermediate 56: methyl 3-methyl-1-[8-(oxan-2-yloxy)octyl]indazole-6-carboxylate**

To a solution of methyl 3-methyl-1H-indazole-6-carboxylate **(Intermediate 21,** 1.00 g, 5.26 mmol) in DCM (10.0 mL) was added 2-[(8-bromooctyl)oxy]oxane (**Intermediate 55**, 4.63 g, 15.8 mmol) and $Cs_2CO_3$ (3.43 g, 10.5 mmol) and the reaction stirred at 60°C overnight. Water (200 mL) was added and the resulting solution was extracted with EtOAc (3 × 300 mL) and the organic layers were combined, washed with brine (3 × 300 mL), dried over anhydrous sodium sulfate, filtered and concentrated under reduced pressure. The residue was chromatographed on a silica gel column, eluting with EtOAc/petroleum ether (11/89) to afford methyl 3-methyl-1-[8-(oxan-2-yloxy)octyl]indazole-6-carboxylate (**Intermediate 56**, 1.80 g, 54.4%) as an oil. Mass spec: m/z: 403.0 [M+H]$^+$.

**Step 3**

**Intermediate 57: 3-methyl-1-[8-(oxan-2-yloxy)octyl]indazole-6-carboxylic acid**

To a solution of methyl 3-methyl-1-[8-(oxan-2-yloxy)octyl]indazole-6-carboxylate (**Intermediate 56**, 800 mg, 1.99 mmol) in THF (10.0 mL) was added 2M NaOH solution (5 mL, 10 mmol) and the reaction was stirred at room temperature for 2h. The pH was adjusted to 6 by the addition of aqueous HCl (12M). The resulting mixture was extracted with EtOAc (3 × 100 mL) and the organic layers were combined, washed with brine (100 mL), dried over anhydrous sodium sulfate, filtered and concentrated under reduced pressure to afford 3-methyl-1-[8-(oxan-2-yloxy)octyl]indazole-6-carboxylic acid (**Intermediate 57**, 400 mg, 81.2%) as a yellow oil, which was used in the next step without further purification. Mass spec: m/z: 389.0 $[M+H]^+$.

**Step 4**

**Intermediate 58: 3-methyl-1-[8-(oxan-2-yloxy)octyl]indazole-6-carboxamide**

To a solution of 3-methyl-1-[8-(oxan-2-yloxy)octyl]indazole-6-carboxylic acid (**Intermediate 57**, 400 mg, 1.03 mmol) in DMF (10.0mL) was added DIPEA (798 mg, 6.18 mmol), HATU (391 mg, 1.03 mmol) and $NH_4Cl$ (220 mg, 4.12 mmol) and the reaction was stirred overnight at room temperature. Water (10 mL) was added and the mixture was extracted with EtOAc (3 × 100 mL) and the organic layers were combined, washed with brine (100 mL), dried over anhydrous sodium sulfate, filtered and concentrated under reduced pressure. The residue was chromatographed on a silica gel column, eluting with ethyl acetate/petroleum ether (60/40), to afford 3-methyl-1-[8-(oxan-2-yloxy)octyl]indazole-6-carboxamide (**Intermediate 58**, 340 mg, 85%) as a white solid. m/z: 388.0 $[M+H]^+$.

**Step 5**

**Intermediate 59: tert-butyl-6-(3-methyl-1-(8-((tetrahydro-2H-pyran-2-yl)oxy)octyl)-1H-indazole-6-carboxamido)-2-((R)-1-methylpyrrolidin-2-yl)-1H-pyrrolo[3,2-c]pyridine-1-carboxylate**

To a solution of 3-methyl-1-[8-(oxan-2-yloxy)octyl]indazole-6-carboxamide (**Intermediate 58**, 200 mg, 0.516 mmol) and tert-butyl-6-bromo-2-[(2R)-1-methylpyrrolidin-2-yl]pyrrolo[3,2-c]pyridine-1-carboxylate (**Intermediate 1**, 177 mg, 0.464 mmol) in 1,4-dioxane (10.0 mL) was added Brettphos (166 mg, 0.310 mmol), Brettphos Pd G3 (173 mg, 0.323 mmol) and $Cs_2CO_3$ (336 mg, 1.03 mmol) and the reaction was stirred overnight at 100°C under nitrogen. Water (50 mL) was added, the mixture was extracted with EtOAc (3 × 100 mL) and the organic layers were combined, washed with brine (3 × 50 mL), dried over anhydrous sodium sulfate, filtered and concentrated under reduced pressure. The residue was chromatographed on a silica gel column, eluting with EtOAc/petroleum ether (30/70), to afford tert-butyl 6-(3-methyl-1-(8-((tetrahydro-2H-pyran-2-yl)oxy)octyl)-1H-indazole-6-carboxamido)-2-((R)-1-methylpyrrolidin-2-yl)-1H-pyrrolo[3,2-c]pyridine-1-carboxylate (**Intermediate 59**, 270 mg, 49%) as an off-white solid. Mass spec: m/z: 687.0 $[M+H]^+$.

**Step 6**

**Intermediate 60: 1-(8-hydroxyoctyl)-3-methyl-N-{2-[(2R)-1-methylpyrrolidin-2-yl]-1H-pyrrolo[3,2-c]pyridin-6-yl} indazole-6-carboxamide hydrochloride**

To a solution of tert-butyl 6-{3-methyl-1-[8-(oxan-2-yloxy)octyl]indazole-6-amido}-2-[(2R)-1-methylpyrrolidin-2-yl]pyrrolo[3,2-c]pyridine-1-carboxylate (**Intermediate 59**, 270 mg, 0.393 mmol) in 1,4-dioxane (10.0 mL) was added conc. aqueous HCl (12M, 2.00 mL) and the reaction was stirred at room temperature for 2h. The reaction was concentrated under reduced pressure to afford 1-(8-hydroxyoctyl)-3-methyl-N-{2-[(2R)-1-methylpyrrolidin-2-yl]-1H-pyrrolo[3,2-c]pyridin-6-yl}indazole-6-carboxamide hydrochloride (**Intermediate 60**, 120 mg) as an off-white solid, which was used in the next step without further purification. Mass spec: m/z: 503.0 $[M+H]^+$.

**Step 7**

**Intermediate 61: 8-[3-methyl-6-({2-[(2R)-1-methylpyrrolidin-2-yl]-1H-pyrrolo[3,2-c]pyridin-6-yl}carbamoyl)indazol-1-yl]octyl methanesulfonate**

To a solution of 1-(8-hydroxyoctyl)-3-methyl-N-{2-[(2R)-1-methylpyrrolidin-2-yl]-1H-pyrrolo[3,2-c]pyridin-6-yl} indazole-6-carboxamide hydrochloride (**Intermediate 60**, 120 mg, 0.398 mmol) and $Et_3N$ (313 mg, 1.91 mmol) in THF (10.0 mL) was added MsCl (82 mg, 0.716 mmol) at 0°C and the reaction was stirred at room temperature for 2h. Water (20 mL) was added and the resulting mixture was extracted with EtOAc (3 × 100 mL) and the organic layers were combined,

washed with brine (3 × 100 mL), dried over anhydrous sodium sulfate, filtered and concentrated under reduced pressure to afford 8-[3-methyl-6-({2-[(2R)-1-methylpyrrolidin-2-yl]-1H-pyrrolo[3,2-c]pyridin-6-yl}carbamoyl)indazol-1-yl]octyl methanesulfonate (**Intermediate 61**, 110 mg, 53%) as an off-white solid, which was used in the next step without further purification. Mass spec: m/z: 581.0 $[M+H]^+$.

**Step 8**

**Example 8: 1-(8-{[2-(2,6-dioxopiperidin-3-yl)-1,3-dioxoisoindol-5-yl]oxy}octyl)-3-methyl-N-{2-[(2R)-1-methylpyrrolidin-2-yl]-1H-pyrrolo[3,2-c]pyridin-6-yl}indazole-6-carboxamide**

To a solution of 8-[3-methyl-6-({2-[(2R)-1-methylpyrrolidin-2-yl]-1H-pyrrolo[3,2-c]pyridin-6-yl}carbamoyl)indazol-1-yl]octyl methanesulfonate (**Intermediate 61**, 60.0 mg, 0.103 mmol) in DMF (3 mL) was added $NaHCO_3$ (17.4 mg, 0.206 mmol), KI (17.2 mg, 0.103 mmol) and 2-(2,6-dioxopiperidin-3-yl)-5-hydroxyisoindole-1,3-dione (CAS No: 64567-60-8, 28.3 mg, 0.103 mmol) and the reaction was stirred at 80°C overnight. The crude reaction was then purified by preparative HPLC (Column: XBridge Prep OBD C18 Column, 30 * 150 mm, 5 μm; Mobile Phase A: Water (with 10 mmol/L $NH_4HCO_3$), Mobile Phase B: MeCN; Flow rate: 60 mL/min; Gradient: 46% B to76 % B in 10 min) to afford 1-(8-{[2-(2,6-dioxopiperidin-3-yl)-1,3-dioxoisoindol-5-yl]oxy}octyl)-3-methyl-N-{2-[(2R)-1-methylpyrrolidin-2-yl]-1H-pyrrolo[3,2-c]pyridin-6-yl}indazole-6-carboxamide (**Example 8**, 4.3 mg, 8.5%) as a white solid. $^1H$ NMR (400 MHz, DMSO-d6) δ ppm 11.40 (s, 1H), 11.10 (s, 1H), 10.56 (s, 1H), 8.52 (s, 1H), 8.45 (s, 1H), 8.25 (s, 1H), 7.73 - 7.80 (m, 3H), 7.39 (s, 1H), 7.30 (*d*, J= 8.4 Hz, 1H), 6.41 (s, 1H), 5.11 (t, J=12.8 Hz, 1H), 4.41 (s, 2H), 4.12 (s, 2H), 3.15 - 3.35 (m, 2H), 2.86 - 2.89 (m, 2H), 2.51 - 2.62 (m, 2H), 2.26 - 2.28 (m, 1H), 2.17 (s, 4H), 2.06 - 2.09 (m, 1H), 1.72 - 1.87 (m, 8H), 1.18 - 1.49 (m, 9H). Mass spec: m/z: 759.5 $[M+H]^+$.

**Example 9 was synthesized following Scheme 13**

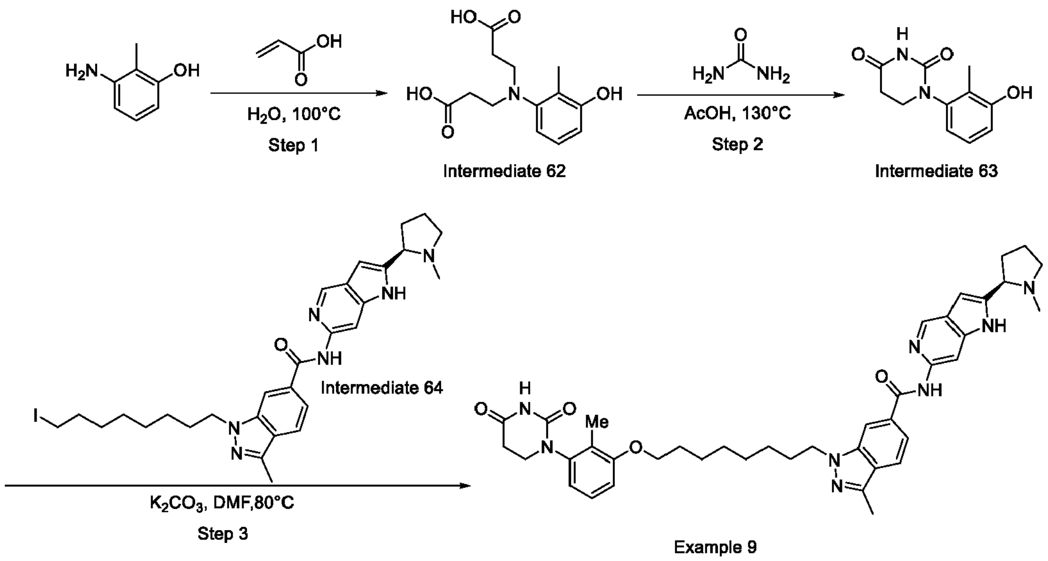


**Scheme 13**

**Step 1**

**Intermediate 62: 3-[(2-carboxyethyl)(3-hydroxy-2-methylphenyl)amino]propanoic acid**

To a solution of 3-amino-2-methylphenol (CAS No: 53222-92-7, 2.00 g, 16.2 mmol) in $H_2O$ (20.0 mL) was added acrylic acid (3.51 g, 48.7 mmol) and the reaction was stirred overnight at 100°C. The mixture was then chromatographed directly on a C18 silica column, eluting with water, to afford 3-[(2-carboxyethyl)(3-hydroxy-2-methylphenyl)amino]propanoic acid (**Intermediate 62**, 3.00 g, 58%) as a yellow oil. Mass spec: m/z: 268.0 $[M+H]^+$.

**Step 2**

**Intermediate 63: 1-(3-hydroxy-2-methylphenyl)-1,3-diazinane-2,4-dione**

To a solution of 3-[(2-carboxyethyl)(3-hydroxy-2-methylphenyl)amino]propanoic acid (**Intermediate 62**, 1.70 g, 6.36 mmol) in acetic acid (12.8 mL) was added urea (573 mg, 9.54 mmol) and the reaction was stirred overnight at 130 °C. The mixture was allowed to cool to room temperature, water (50 mL) was added, resulting in a formation of a precipitate. The precipitate was filtered, washed with 0.1% aqueous HCl (3 × 100 mL) and dried under vacuum to afford 1-(3-hydroxy-2-methylphenyl)-1,3-diazinane-2,4-dione (**Intermediate 63**, 500 mg, 42%) as an off-white solid. Mass spec: m/z: 221.0 $[M+H]^+$

**Step 3**

**Example 9: 1-{8-[3-(2,4-dioxo-1,3-diazinan-1-yl)-2-methylphenoxy]octyl}-3-methyl-N-{2-[(2R)-1-methylpyrrolidin-2-yl]-1H-pyrrolo[3,2-c]pyridin-6-yl}indazole-6-carboxamide**

To a solution of 1-(3-hydroxy-2-methylphenyl)-1,3-diazinane-2,4-dione (**Intermediate 63**, 27.7 mg, 0.125 mmol) and 1-(8-iodooctyl)-3-methyl-N-{2-[(2R)-1-methylpyrrolidin-2-yl]-1H-pyrrolo[3,2-c]pyridin-6-yl}indazole-6-carboxamide (**Intermediate 64**, 70.0 mg, 0.114 mmol) in DMF (3.50 mL) was added $K_2CO_3$ (63.2 mg, 0.456 mmol) and the reaction was stirred at 80°C for 2h. The resulting solution was purified directly by preparative HPLC (Column: XBridge Prep Phenyl OBD C18 Column, 30*150 mm, 5μm; Mobile Phase A: Water (10 mmol/L $NH_4HCO_3$), Mobile Phase B: MeCN; Flow rate: 60 mL/min; Gradient: 40% B to70 % B in 10 min) to afford 1-{8-[3-(2,4-dioxo-1,3-diazinan-1-yl)-2-methylphenoxy]octyl}-3-methyl-N-{2-[(2R)-1-methylpyrrolidin-2-yl]-1H-pyrrolo[3,2-c]pyridin-6-yl}indazole-6-carboxamide (**Example 9**, 1.7 mg, 2%) as a white solid. $^1$H NMR δ ppm 11.42 (s, 1H), 10.54 (s, 1H), 10.32 (s, 1H), 8.52 (s, 1H), 8.44 (s, 1H), 8.25 (s, 1H), 7.72 - 7.80 (m, 2H), 7.14 (t, *J* = 8.8 Hz, 1H), 6.82 - 6.87 (m, 2H), 6.41 (s, 1H), 4.41 - 4.45 (m, 3H), 3.93 (t, *J* = 6.4 Hz, 2H), 3.70 - 3.76 (m, 2H), 3.15 - 3.16 (m, 2H), 2.18 - 2.33 (m, 4H), 1.96 (s, 4H), 1.82 - 1.88 (m, 5H), 1.71 - 1.72 (m, 3H), 1.32 - 1.36 (m, 3H), 1.24 - 1.26 (m, 7H), 1.19 - 1.21 (m, 1H). Mass spec: m/z: 705.5 $[M+H]^+$.

**Example 10 was synthesized following Scheme 14**

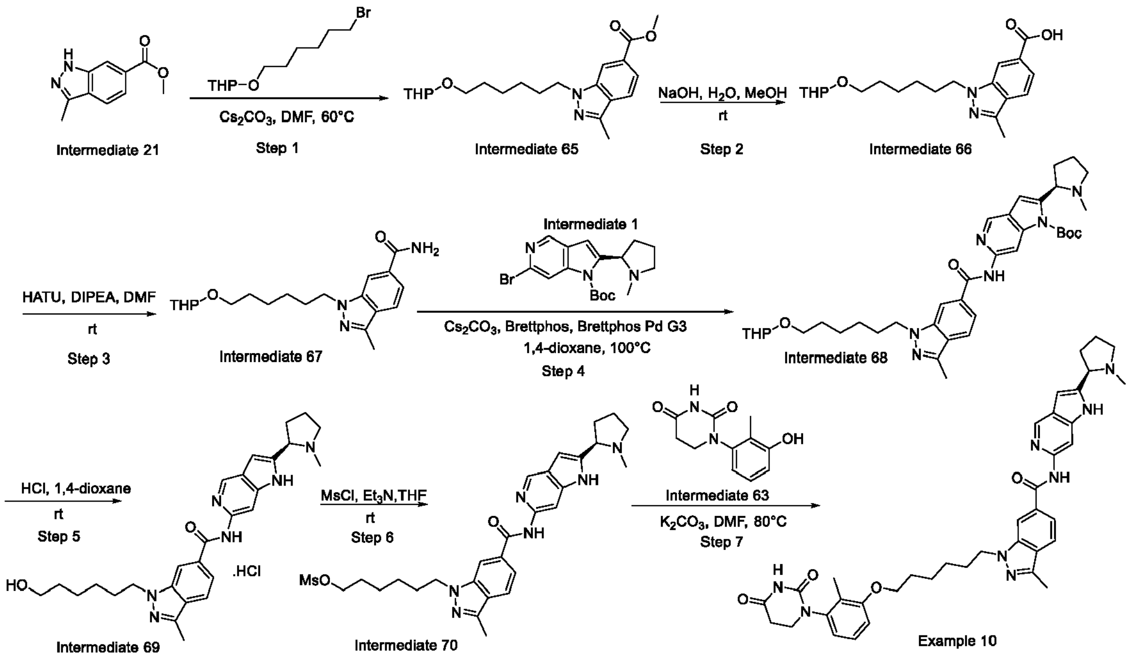


Scheme 14

**Step 1**

**Intermediate 65: methyl 3-methyl-1-[6-(oxan-2-yloxy)hexyl]indazole-6-carboxylate**

To a solution of methyl 3-methyl-1H-indazole-6-carboxylate (**Intermediate 21**, 1.00 g, 5.26 mmol) in DMF (20.0 mL) was added 2-(6-Bromohexyloxy)tetrahydro-2H-pyran (CAS No: 53963-10-3, 2.09 g, 7.89 mmol) and $Cs_2CO_3$ (3.43 g, 10.5 mmol) and the reaction was stirred at 60 °C for 2h. Water (50 mL) was added and the aqueous phase was extracted with EtOAc (3 × 200 mL). The combined organic phases were washed with brine (3 × 200 mL), dried over anhydrous sodium sulfate, filtered and concentrated under reduced pressure. The resulting residue was chromatographed on a silica gel column, eluting with EtOAc/petroleum ether (20/80), to afford methyl 3-methyl-1-[6-(oxan-2-yloxy)hexyl]indazole-6-carboxylate (**Intermediate 65**, 1.30 g, 61%) as a yellow oil. Mass spec: m/z: 375.0 $[M+H]^+$.

**Step 2**

**Intermediate 66: 3-methyl-1-[6-(oxan-2-yloxy)hexyl]indazole-6-carboxylic acid**

To a solution of methyl 3-methyl-1-[6-(oxan-2-yloxy)hexyl]indazole-6-carboxylate (**Intermediate 65**, 1.30 g, 3.47 mmol) in MeOH (25 mL) was added NaOH (4M in $H_2O$, 5 mL) and the reaction was stirred at room temperature for 2h. Water (50 mL) was added and the pH the solution was adjusted to pH 6 by dropwise addition of concentrated aqueous HCl. The mixture was then extracted with EtOAc (3 × 200 mL), washed with brine (3 × 200 mL), dried over anhydrous sodium sulfate and concentrated under reduced pressure to afford 3-methyl-1-[6-(oxan-2-yloxy)hexyl]indazole-6-carboxylic acid (**Intermediate 66**, 1.10 g, 87%) as a colourless oil. Mass spec: m/z: 361.0 $[M+H]^+$.

**Step 3**

**Intermediate 67: 3-methyl-1-[6-(oxan-2-yloxy)hexyl]indazole-6-carboxamide**

To a solution of 3-methyl-1-[6-(oxan-2-yloxy)hexyl]indazole-6-carboxylic acid (**Intermediate 66**, 800 mg, 2.22 mmol) in DMF (10 mL) was added DIPEA (1.72 g, 13.3 mmol), HATU (844 mg, 2.22 mmol) and $NH_4Cl$ (475 mg, 8.88 mmol) and the reaction was stirred at room temperature for 4h. Water (20 mL) was added and the aqueous phase extracted with EtOAc (3 × 100 mL). The combined organic phases were washed with brine (3 × 100 mL), dried over anhydrous sodium sulfate, filtered and concentrated under reduced pressure. The resulting residue was chromatographed on a silica gel column, eluting with EtOAc/petroleum ether (65/35) to afford 3-methyl-1-[6-(oxan-2-yloxy)hexyl]indazole-6-carboxamide (**Intermediate 67**, 700 mg, 99%) as a colorless oil. Mass spec: m/z: 360.0 $[M+H]^+$.

**Step 4**

**Intermediate 68: tert-butyl 6-{3-methyl-1-[6-(oxan-2-yloxy)hexyl]indazole-6-amido}-2-[(2R)-1-methylpyrrolidin-2-yl]pyrrolo[3,2-c]pyridine-1-carboxylate**

To a solution of 3-methyl-1-[6-(oxan-2-yloxy)hexyl]indazole-6-carboxamide (**Intermediate 67**, 700 mg, 1.95 mmol) in 1,4-dioxane (10.0 ml) was added tert-butyl 6-bromo-2-[(2R)-1-methylpyrrolidin-2-yl]pyrrolo[3,2-c]pyridine-1-carboxylate (**Intermediate 1**, 0.665 g, 1.75 mmol), Brettphos (0.630 g, 1.168 mmol), Brettphos Pd G3 (0.532 g, 0.585 mmol) and $Cs_2CO_3$ (1.27 g, 3.89 mmol) and the reaction mixture was stirred at 100 °C for 4h , under $N_2$. The volatiles were then removed under reduced pressure and the resulting residue was purified on a C18 silica column, eluting with MeCN, to afford tert-butyl 6-{3-methyl-1-[6-(oxan-2-yloxy)hexyl]indazole-6-amido}-2-[(2R)-1-methylpyrrolidin-2-yl]pyrrolo[3,2-c]pyridine-1-carboxylate (**Intermediate 68**, 500 mg, 37%) as a yellow solid. Mass spec: m/z: 659.0 $[M+H]^+$.

**Step 5**

**Intermediate 69: 1-(6-hydroxyhexyl)-3-methyl-N-{2-[(2R)-1-methylpyrrolidin-2-yl]-1H-pyrrolo[3,2-c]pyridin-6-yl}indazole-6-carboxamide hydrochloride**

To a solution of tert-butyl 6-{3-methyl-1-[6-(oxan-2-yloxy)hexyl]indazole-6-amido}-2-[(2R)-1-methylpyrrolidin-2-yl]pyrrolo[3,2-c]pyridine-1-carboxylate (**Intermediate 68**, 500 mg, 0.759 mmol) in 1,4-dioxane (16.6 mL) was added concentrated aqueous HCl (8.3 mL) and the reaction was stirred at room temperature for 2h. The volatiles were then removed under reduced pressure to afford 1-(6-hydroxyhexyl)-3-methyl-N-{2-[(2R)-1-methylpyrrolidin-2-yl]-1H-pyrrolo[3,2-c]pyridin-6-yl}indazole-6-carboxamide hydrochloride (**Intermediate 69**, 330 mg, 97%) as a yellow oil. Mass spec: m/z: 475.0 $[M+H]^+$.

**Step 6**

**Intermediate 70: 6-[3-methyl-6-({2-[(2R)-1-methylpyrrolidin-2-yl]-1H-pyrrolo[3,2-c]pyridin-6-yl}carbamoyl)indazol-1-yl]hexyl methanesulfonate**

To a solution of 1-(6-hydroxyhexyl)-3-methyl-N-{2-[(2R)-1-methylpyrrolidin-2-yl]-1H-pyrrolo[3,2-c]pyridin-6-yl} indazole-6-carboxamide hydrochloride (**Intermediate 69**, 150 mg, 0.316 mmol) and $Et_3N$ (320 mg, 3.16 mmol) in THF (4.50 mL) was added MsCl (109 mg, 0.948 mmol), at 0°C, and the reaction was stirred at room temperature for 2h. Water (10 mL) was added and the resulting aqueous phase was extracted with EtOAc (3 × 50 mL). The organic layers were combined, washed with brine (3 × 50 mL), dried over anhydrous sodium sulfate, filtered and concentrated under reduced pressure to afford 6-[3-methyl-6-({2-[(2R)-1-methylpyrrolidin-2-yl]-1H-pyrrolo[3,2-c]pyridin-6-yl}carbamoyl)indazol-1-yl] hexyl methanesulfonate (**Intermediate 70**, 170 mg, 81%) as an off-white solid, which was used in the next step without further purification. Mass spec: m/z: 553.0 $[M+H]^+$.

**Step 7**

**Example 10: 1-16-[3-(2,4-dioxo-1,3-diazinan-1-yl)-2-methylphenoxy]hexyll-3-methyl-N-{2-[(2R)-1-methylpyrrolidin-2-yl]-1H-pyrrolo [3,2-c] pyridin-6-yl}indazole-6-carboxamide**

To a solution of 1-(6-hydroxyhexyl)-3-methyl-N-{2-[(2R)-1-methylpyrrolidin-2-yl]-1H-pyrrolo[3,2-c]pyridin-6-yl} indazole-6-carboxamide (**Intermediate 70**, 100 mg, 0.211 mmol) in DMF (5.00 mL) was added 1-(3-hydroxy-2-methylphenyl)-1,3-diazinane-2,4-dione (**Intermediate 63**, 51.0 mg, 0.232 mmol) and $K_2CO_3$ (116 mg, 0.844 mmol) and the reaction was stirred at 80 °C for 2h. The resulting mixture was purified directly by preparative HPLC (Column: Xselect CSH Prep C18 Column, 30*150 mm, 5μm; Mobile Phase A: Water (with 0.1% formic acid), Mobile Phase B: MeCN; Flow rate: 60 mL/min; Gradient: 5% B to35 % B in 10 min) to afford 1-{6-[3-(2,4-dioxo-1,3-diazinan-1-yl)-2-methylphenoxy]hexyl}-3-methyl-N-{2-[(2R)-1-methylpyrrolidin-2-yl]-1H-pyrrolo[3,2-c]pyridin-6-yl}indazole-6-carboxamide (**Example DBT-0356,** 4.5 mg, 4.3%) as a white solid. $^1H$ NMR (400 MHz, DMSO-d6) δ ppm 11.43 (s, 1H), 10.54 (s, 1H), 10.31 (s, 1H), 8.53 (s, 1H), 8.45 (s, 1H), 8.25 (s, 1H), 7.73 - 7.80 (m, 2H), 7.15 (t, *J* = 8.0 Hz, 1H), 6.83 - 6.89 (m, 2H), 6.41(s, 1H), 4.43 (t, *J* = 6.8 Hz, 2H), 3.95 (t, *J* = 6.0 Hz, 2H), 3.68 - 3.71 (m, 3H), 3.13 - 3.16 (m, 2H), 2.71 -2.75 (m, 3H), 2.27 - 2.34 (m, 2H), 2.15 - 2.18 (m, 3H), 1.93 (s, 3H), 1.72 - 1.91 (m, 7H), 1.49 - 1.52 (m, 2H), 1.37 - 1.41 (m, 2H), 0.94 - 0.96 (m, 1H). Mass spec: m/z: 677.50 $[M+H]^+$.

**Example 11 was synthesized following Scheme 15**

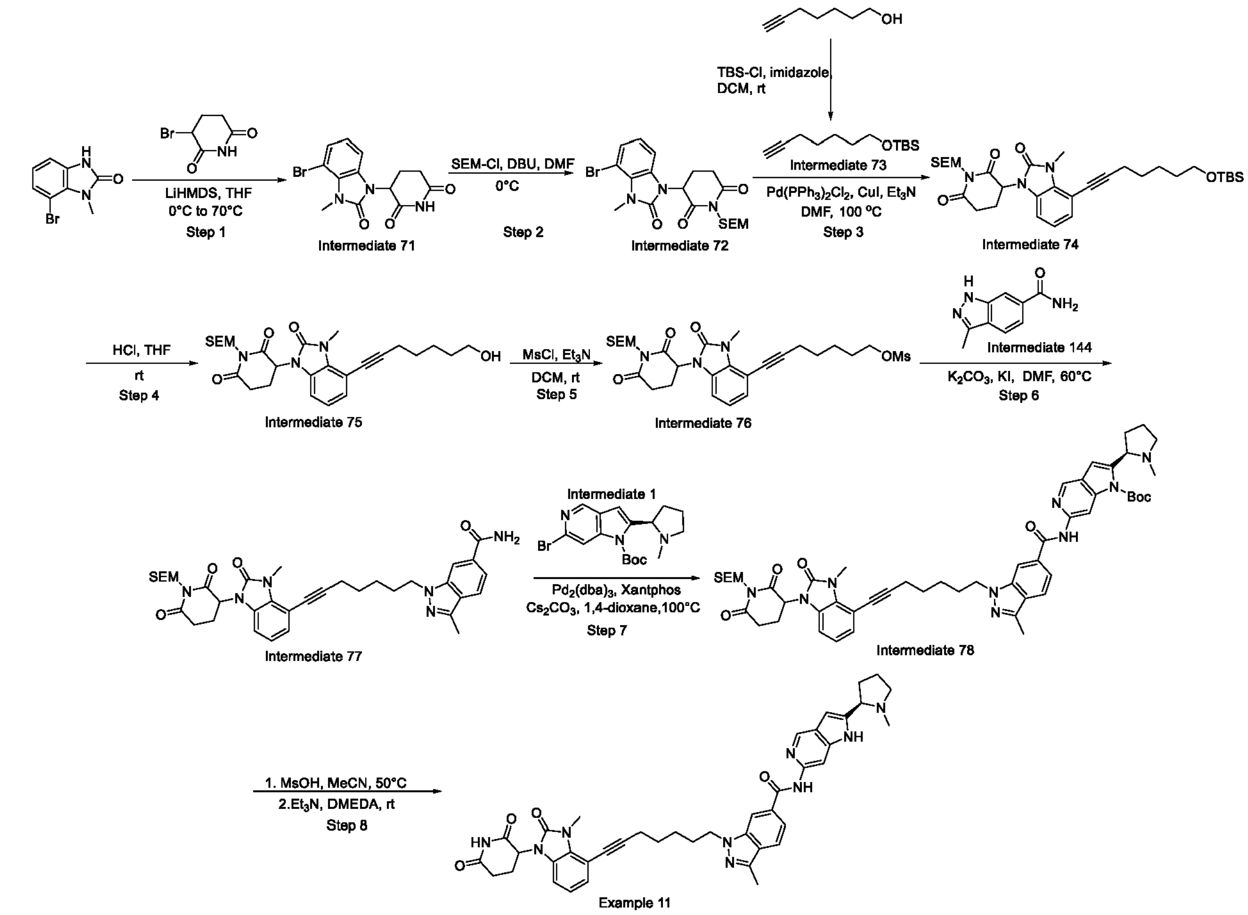


## Scheme 15

**Step 1**

**Intermediate 71: 3-(4-bromo-3-methyl-2-oxo-1,3-benzodiazol-1-yl)piperidine-2,6-dione**

To a solution of 7-bromo-1-methyl-3H-1,3-benzodiazol-2-one (CAS No: 913297-44-6, 2.00 g, 8.72 mmol) in THF (20 mL) was added LiHMDS (26.2 mL, 26.2 mmol, 1M in THF) dropwise at 0 °C under $N_2$. After 15 min, 3-bromopiperidine-2,6-dione (CAS No: 62595-74-8, 2.51 g, 13.1 mmol) in THF (5 mL) was added dropwise at 0 °C and the reaction was stirred at 70 °C for 4h under $N_2$. The reaction mixture was then cooled to 0 °C and pH of the mixture was adjusted to pH 2-3 with dilute aqueous HCl (1M) resulting in a precipitate. The precipitate was collected by filtration to afford 3-(4-bromo-3-methyl-2-oxo-1,3-benzodiazol-1-yl)piperidine-2,6-dione (**Intermediate 71**, 2.00 g, 34%) as an off white solid. Mass spec: m/z: 338.0 $[M+H]^+$.

**Step 2**

**Intermediate 72: 3-(4-bromo-3-methyl-2-oxo-1,3-benzodiazol-1-yl)-1-{[2-(trimethylsilyl)ethoxy]methyl}piperidine-2,6-dione**

To a stirred solution of 3-(4-bromo-3-methyl-2-oxo-1,3-benzodiazol-1-yl)piperidine-2,6-dione (**Intermediate 71**, 1.9 g, 2.81 mmol) and DBU (0.86 g, 5.62 mmol) in DMF (40 mL) was added SEM-Cl (0.94 g, 5.62 mmol) at 0° C. The resulting mixture was allowed to warm to room temperature and stirred overnight. The mixture was quenched with water (100 mL) and extracted with EtOAc (3 × 200 mL). The combined organic layers were washed with brine, dried over anhydrous $Na_2SO$, filtered and concentrated under reduced pressure. The residue was chromatographed on a silica gel column, eluting with EtOAc/petroleum ether (35/65), to afford 3-(4-bromo-3-methyl-2-oxo-1,3-benzodiazol-1-yl)-1-{[2-(trimethylsilyl)ethoxy]methyl}piperidine-2,6-dione (**Intermediate 72**, 1.5 g, 91%) as a an oil. Mass spec: m/z :468.1 $[M+H]^+$.

**Step 3**

**Intermediate 74: 3-(4-{7-[(tert-butyldimethylsilyl)oxy]hept-1-yn-1-yl}-3-methyl-2-oxo-1,3-benzodiazol-1-yl)-1-{[2-(trimethylsilyl)ethoxy] methyllpiperidine-2,6-dione**

To a stirred solution of hept-6-yn-1-ol (CAS No: 63478-76-2, 500 mg, 4.413 mmol) in DCM (10 mL) was added imidazole (360 mg, 5.296 mmol) and TBS-Cl (798 mg, 5.296 mmol) and the reaction mixture was stirred overnight at room temperature. The resulting mixture was filtered and concentrated under reduced pressure to afford tert-butyl(hept-6-yn-1-yloxy)dimethylsilane (**Intermediate 73**, 1.00 g, 91%) as a colorless liquid, which was used in the stage without further purification. A 50 mL round-bottom flask was charged with 3-(4-bromo-3-methyl-2-oxo-1,3-benzodiazol-1-yl)-1-{[2-(trimethylsilyl)ethoxy]methyl}piperidine-2,6-dione (**Intermediate 72**, 700 mg, 1.196 mmol), tert-butyl(hept-6-yn-1-yloxy) dimethylsilane (541 mg, 2.39 mmol), $Pd(PPh_3)_2Cl_2$ (134 mg, 0.191 mmol), CuI (45.5 mg, 0.239 mmol), triethylamine (3 mL, 21.58 mmol) in DMF (10 mL) under $N_2$ and the reaction was stirred overnight at 100°C. The mixture was quenched with water (100 mL) and extracted with EtOAc (3 × 100 mL). The combined organic layers were washed with brine, dried over anhydrous $Na_2SO_4$, filtered, and concentrated under reduced pressure. The residue was chromatographed on a silica gel column, eluting with petroleum ether/EtOAc (38/72) to afford 3-(4-{7-[(tertbutyldimethylsilyl)oxy]hept-1-yn-1-yl}-3-methyl-2-oxo-1,3-benzodiazol-1-yl)-1{[2-(trimethylsilyl)ethoxy]methyl}piperidine-2,6-dione (**Intermediate 74**, 900 mg, 86%) as a yellow oil. Mass spec: m/z :614.0 $[M+H]^+$.

**Step 4**

**Intermediate 75: 3-[4-(7-hydroxyhept-1-yn-1-yl)-3-methyl-2-oxo-1,3-benzodiazol-1-yl]-1-{[2-(trimethylsilyl) ethoxy]methyl}piperidine-2,6-dione**

To a stirred solution of 3-(4-{7-[(tert-butyldimethylsilyl)oxy]hept-1-yn-1-yl}-3-methyl-2-oxo-1,3-benzodiazol-1-yl)-1-{[2-(trimethylsilyl)ethoxy]methyl}piperidine-2,6-dione (**Intermediate 74**, 870 mg, 0.992 mmol) in THF (15 mL) was added HCl (4M in 1,4-dioxane, 3 mL) at room temperature and the reaction stirred for 0.5h. The mixture was quenched with water (100 mL) and extracted with EtOAc (3 × 100 mL). The combined organic layers were washed with brine, dried over anhydrous $Na_2SO_4$, filtered and concentrated under reduced pressure. The residue was chromatographed on a silica gel column, eluting with DCM/MeOH (96/4), to afford 3-[4-(7-hydroxyhept-1-yn-1-yl)-3-methyl-2-oxo-1,3-benzodiazol-1-yl]-1-{[2-(trimethylsilyl)ethoxy]methyl}piperidine-2,6-dione (**Intermediate 75**, 450 mg, 73%) as a yellow oil. Mass spec: m/z: 500.0 $[M+H]^+$.

**Step 5**

**Intermediate 76: 7-[1-(2,6-dioxo-1-{[2-(trimethylsilyl)ethoxy]methyl}piperidin-3-yl)-3-methyl-2-oxo-1,3-benzodiazol-4-yl]hept-6-yn-1-yl methanesulfonate**

To a solution of 3-[4-(7-hydroxyhept-1-yn-1-yl)-3-methyl-2-oxo-1,3-benzodiazol-1-yl]-1-{[2-(trimethylsilyl) ethoxy]methyl}piperidine-2,6-dione (**Intermediate 75**, 450 mg, 0.720 mmol) in DCM (6 mL) was added triethylamine (365 mg, 3.60 mmol) and MsCl (165 mg, 1.44 mmol) and the reaction mixture was stirred at room temperature for 1h. MeOH (10 mL) was added and the volatiles were removed under reduced pressure. The residue was then chromatographed on a silica gel column, eluting with EtOAc/petroleum ether (60/40), to afford 7-[1-(2,6-dioxo-1-{[2-(trimethylsilyl) ethoxy]methyl}piperidin-3-yl)-3-methyl-2-oxo-1,3-benzodiazol-4-yl]hept-6-yn-1-yl methanesulfonate (**Intermediate 76**, 390 mg, 84%) as a yellow oil. Mass spec: m/z :578.0 $[M+H]^+$.

**Step 6**

**Intermediate 77: 1-{7-[1-(2,6-dioxo-1-{[2-(trimethylsilyl)ethoxy]methyl}piperidin-3-yl)-3-methyl-2-oxo-1,3-benzodiazol-4-yl]hept-6-yn-1-yl}-3-methylindazole-6-carboxamide**

A 10 mL round-bottom flask was charged with 7-[1-(2,6-dioxo-1-{[2-(trimethylsilyl)ethoxy]methyl}piperidin-3-yl)-3-methyl-2-oxo-1,3-benzodiazol-4-yl]hept-6-yn-1-yl methanesulfonate (**Intermediate 76**, 370 mg, 0.576 mmol), 3-methyl-1H-indazole-6-carboxamide (**Intermediate 144**, 121 mg, 0.691 mmol,), $K_2CO_3$ (159 mg, 1.152 mmol), KI (95.6 mg, 0.576 mmol) in DMF (4 mL) and the reaction mixture was stirred at 60°C for 4h, under $N_2$. The crude reaction mixture was purified on a C18 silica column, eluting with MeCN/$H_2O$ (1-2 drops formic acid) (45/55), to afford 1-{7-[1-(2,6-dioxo-1-{[2-(trimethylsilyl)ethoxy]methyl}piperidin-3-yl)-3-methyl-2-oxo-1,3-benzodiazol-4-yl]hept-6-yn-1-yl}-3-methylindazole-6-carboxamide (**Intermediate 77**, 80 mg, 17%d) as a yellow solid. Mass spec: m/z 657.0 $[M+H]^+$.

**Step 7**

**Intermediate 78: tert-butyl 6-(1-{7-[1-(2,6-dioxo-1-{[2-(trimethylsilyl)ethoxy]methyllpiperidin-3-yl)-3-methyl-2-oxo-1,3-benzodiazol-4-yl]hept-6-yn-1-yl}-3-methylindazole-6-amido)-2-[(2R)-1-methylpyrrolidin-2-yl]pyrrolo [3,2-c]pyridine-1-carboxylate**

A 5 mL round-bottom flask was charged with 1-{7-[1-(2,6-dioxo-1-{[2-(trimethylsilyl)ethoxy]methyl}piperidin-3-yl)-3-methyl-2-oxo-1,3-benzodiazol-4-yl]hept-6-yn-1-yl}-3-methylindazole-6-carboxamide (**Intermediate 77**, 80.0 mg, 0.097 mmol), tert-butyl 6-bromo-2-[(2R)-1-methylpyrrolidin-2-yl]pyrrolo[3,2-c]pyridine-1-carboxylate (**Intermediate 1**, 40.7 mg, 0.107 mmol), $Pd_2(dba)_3$ (8.92 mg, 0.010 mmol), Xantphos (11.3 mg, 0.019 mmol), $Cs_2CO_3$ (63.5 mg, 0.194 mmol) in 1,4-dioxane (3 mL), under $N_2$ and the mixture was stirred at 60°C for 3h. The volatiles were removed under reduced pressure and the residue was purified by preparative TLC, eluting with DCM/MeOH (95/5), to afford tert-butyl 6-(1-{7-[1-(2,6-dioxo-1-{[2-(trimethylsilyl)ethoxy]methyl}piperidin-3-yl)-3-methyl-2-oxo-1,3-benzodiazol-4-yl]hept-6-yn-1-yl}-3-methylindazole-6-amido)-2-[(2R)-1-methylpyrrolidin-2-yl]pyrrolo[3,2-c]pyridine-1-carboxylate (**Intermediate 78**, 38 mg, 34%) as a yellow solid. Mass spec: m/z 956.0 $[M+H]^+$.

**Step 8**

**Example 11: 1-{7-[1-(2,6-dioxopiperidin-3-yl)-3-methyl-2-oxo-1,3-benzodiazol-4-yl]hept-6-yn-1-yl}-3-methyl-N-{2-[(2R)-1-methylpyrrolidin-2-yl]-1H-pyrrolo[3,2-c]pyridin-6-yl}indazole-6-carboxamide**

To a solution of tert-butyl 6-(1-{7-[1-(2,6-dioxo-1-{[2-(trimethylsilyl)ethoxy]methyl}piperidin-3-yl)-3-methyl-2-oxo-1,3-benzodiazol-4-yl]hept-6-yn-1-yl}-3-methylindazole-6-amido)-2-[(2R)-1-methylpyrrolidin-2-yl]pyrrolo[3,2-c]pyridine-1-carboxylate (**Intermediate 78**, 38 mg, 0.033 mmol) in DCM (1 mL) was added MsOH (41.7 mg, 0.429 mmol) and the reaction mixture was stirred at room temperature for 2h. Triethylamine (39.5 mg, 0.390 mmol) and *N,N'*-dimethylethylenediamine (5.89 mg, 0.066 mmol) was then added and the reaction stirred for a further 2h at room temperature. The reaction mixture was then purified by preparative TLC, eluting with DCM/MeOH (90/10), to afford crude product. The crude product was further purified by preparative HPLC (Column: XBridge Prep Shield RP C18 Column, 19*250 mm, 5μm; Mobile Phase A: Water (0.1%FA), Mobile Phase B: MeOH; Flow rate: 25 mL/min ; Gradient: 25% B to 55% B in 10 min), to afford 1-{7-[1-(2,6-dioxopiperidin-3-yl)-3-methyl-2-oxo-1,3-benzodiazol-4-yl]hept-6-yn-1-yl}-3-methyl-N-{2-[(2R)-1-methylpyrrolidin-2-yl]-1H-pyrrolo[3,2-c]pyridin-6-yl}indazole-6-carboxamide (**Example 11**, 2.1 mg, 8.2%) as a white solid. $^1H$ NMR (400 MHz, MeOH-d4) δ ppm 8.63 (s, 1H), 8.28 (s, 1H), 8.19 (s, 1H), 7.68 (d, *J* = 8.0 Hz, 1H), 7.57 (d, *J* = 8.4 Hz, 1H), 7.04 (d, *J* = 7.6 Hz, 1H), 6.92 - 6.94 (m, 2H), 6.75 (s, 1H), 5.36 - 5.40 (m, 1H), 4.47 - 4.87 (m, 2H), 4.09 - 4.12 (m, 1H), 3.50 - 3.56 (m, 1H), 3.35 - 3.36 (m, 3H), 3.31 - 3.32 (m, 3H), 2.91 - 2.94 (m, 2H), 2.79 - 2.82 (m, 2H), 2.62 (s, 3H). 2.48 - 2.52 (m, 1H), 2.43- 2.46 (m, 2H), 2.29 - 2.35 (m, 1H), 2.16 - 2.19 (m, 3H), 2.00 - 2.02 (m, 2H), 1.61 - 1.66 (m, 2H), 1.42 - 1.48 (m, 2H). Mass spec: m/z 726.4 $[M+H]^+$.

**Example 12 was synthesis following Scheme 16**

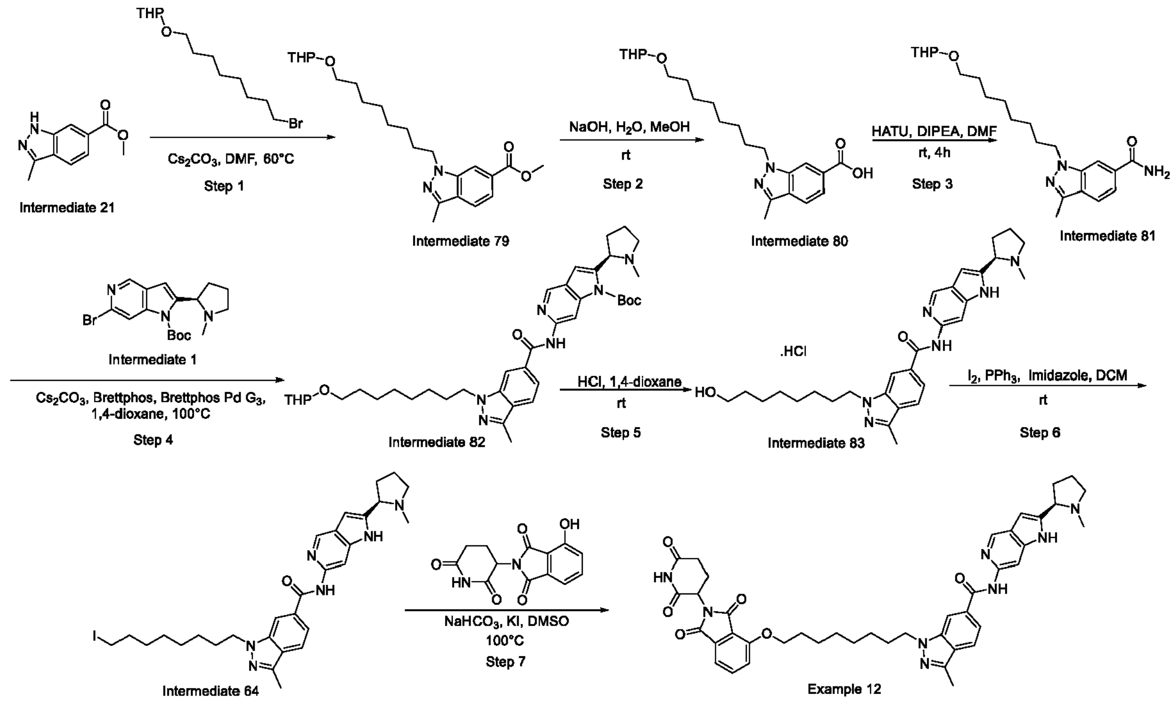


Scheme 16

**Step 1**

**Intermediate 79: methyl 3-methyl-1-(8-((tetrahydro-2H-pyran-2-yl)oxy)octyl)-1H-indazole-6-carboxylate**

To a solution of methyl 3-methyl-1H-indazole-6-carboxylate (**Intermediate 21**, 2.00 g, 9.46 mmol) in DMF (20 mL) was added 2-((8-bromooctyl)oxy)tetrahydro-2H-pyran (CAS No: 50816-20-1, 8.33 g, 28.4 mmol) and $Cs_2CO_3$ (6.17 g, 18.9 mmol) and the mixture was stirred at 60 °C for 2h. Water (20 mL) was added and the aqueous phase was extracted with EtOAc (3 × 50 mL). The organic layers were combined, washed with brine (150 mL), dried over anhydrous sodium sulfate, filtered and concentrated under reduced pressure. The residue was chromatographed on a silica gel column, eluting with EtOAc/petroleum ether (1/9), to afford methyl 3-methyl-1-(8-((tetrahydro-2H-pyran-2-yl)oxy)octyl)-1H-indazole-6-carboxylate (**Intermediate 79**, 3.50 g 83%) as a yellow oil. Mass spec: m/z 403.0 $[M+H]^+$.

**Step 2**

**Intermediate 80: 3-methyl-1-(8-((tetrahydro-2H-pyran-2-yl)oxy)octyl)-1H-indazole-6-carboxylic acid**

To a solution of methyl 3-methyl-1-(8-((tetrahydro-2H-pyran-2-yl)oxy)octyl)-1H-indazole-6-carboxylate (**Intermediate 79**, 3.50 g, 7.83 mmol) in MeOH (40 mL) and $H_2O$ (27 mL), was added NaOH (12.5 g, 313 mmol) and the mixture was stirred at room temperature for 2h. The pH value of the mixture was adjusted to pH 6 by the dropwise addition of dilute aqueous HCl (1M). The mixture was then extracted with $CHCl_3$/iPrOH (3/1, 3 × 100 mL) and the organic layers were combined, washed with brine (300 mL), dried over anhydrous sodium sulfate, filtered and concentrated under reduced pressure to afford 3-methyl-1-(8-((tetrahydro-2H-pyran-2-yl)oxy)octyl)-1H-indazole-6-carboxylic acid (**Intermediate 80**, 3.00 g 84%) as a yellow oil, which was used in the next step without further purification. Mass spec: m/z 389.0 $[M+H]^+$.

**Step 3**

**Intermediate 81: 3-methyl-1-(8-((tetrahydro-2H-pyran-2-yl)oxy)octyl)-1H-indazole-6-carboxamide**

To a solution of 3-methyl-1-(8-((tetrahydro-2H-pyran-2-yl)oxy)octyl)-1H-indazole-6-carboxylic acid (**Intermediate 80**, 3.00 g, 6.56 mmol) in DMF (40 mL) was added HATU (3.74 g, 9.85 mmol), DIPEA (5.09 g, 39.4 mmol) and $NH_4Cl$ (1.40 g, 26.3 mmol) and the reaction mixture was stirred at room temperature for 4h. Water (50 mL) was added and the mixture was extracted with EtOAc (3 × 100 mL) and the organic layers were combined, washed with brine (300 mL), dried over anhydrous sodium sulfate, filtered and concentrated under reduced pressure. The residue was chromatographed on

a silica gel column, eluting with MeOH/DCM (5/95) to afford 3-methyl-1-(8-((tetrahydro-2H-pyran-2-yl)oxy)octyl)-1H-indazole-6-carboxamide (**Intermediate 81**, 3.00 g 94%) as a yellow oil. Mass spec: m/z 388.0 $[M+H]^+$.

**Step 4**

**Intermediate 82: tert-butyl 6-(3-methyl-1-(8-((tetrahydro-2H-pyran-2-yl)oxy)octyl)-1H-indazole-6-carboxamido)-2-((R)-1-methylpyrrolidin-2-yl)-1H-pyrrolo[3,2-c]pyridine-1-carboxylate**

To a solution of 3-methyl-1-(8-((tetrahydro-2H-pyran-2-yl)oxy)octyl)-1H-indazole-6-carboxamide (**Intermediate 81**, 1.00 g, 2.32 mmol) in 1,4-dioxane (50 mL) was added tert-butyl (R)-6-bromo-2-(1-methylpyrrolidin-2-yl)-1H-pyrrolo [3,2-c]pyridine-1-carboxylate (**Intermediate 1**, 1.28 g, 3.02 mmol), Brettphos (748 mg, 1.39 mmol), Brettphos Pd $G_3$ (632 mg, 0.697 mmol) and $Cs_2CO_3$ (1.51 g, 4.64 mmol) an the reaction was stirred at 100 °C for 2 days. The resulting crude was then chromatographed on a silica gel column, eluting with MeOH/DCM (5/95), to afford tert-butyl 6-(3-methyl-1-(8-((tetrahydro-2H-pyran-2-yl)oxy)octyl)-1H-indazole-6-carboxamido)-2-((R)-1-methylpyrrolidin-2-yl)-1H-pyrrolo[3,2-c]pyridine-1-carboxylate (**Intermediate 82**, 900 mg 50.77%) as a yellow oil. Mass spec: m/z 687.0 $[M+H]^+$.

**Step 5**

**Intermediate 83: (R)-1-(8-hydroxyoctyl)-3-methyl-N-(2-(1-methylpyrrolidin-2-yl)-1H-pyrrolo[3,2-c]pyridin-6-yl)-1H-indazole-6-carboxamide hydrochloride**

To a solution of tert-butyl 6-(3-methyl-1-(8-((tetrahydro-2H-pyran-2-yl)oxy)octyl)-1H-indazole-6-carboxamido)-2-((R)-1-methylpyrrolidin-2-yl)-1H-pyrrolo[3,2-c]pyridine-1-carboxylate (**Intermediate 82**, 850 mg, 1.11 mmol) in 1,4-dioxane (15 mL) was added concentrated HCl (2 mL) and the mixture was stirred at room temperature for 2h. The volatiles were then removed under reduced pressure to afford (R)-1-(8-hydroxyoctyl)-3-methyl-N-(2-(1-methylpyrrolidin-2-yl)-1H-pyrrolo[3,2-c]pyridin-6-yl)-1H-indazole-6-carboxamide hydrochloride (**Intermediate 83**, 550 mg) as a yellow solid, which was used in the next step without further purification. Mass spec: m/z: 503.0 $[M+H]^+$.

**Step 6**

**Intermediate 64: (R)-1-(8-iodooctyl)-3-methyl-N-(2-(1-methylpyrrolidin-2-yl)-1H-pyrrolo[3,2-c]pyridin-6-yl)-1H-indazole-6-carboxamide**

To a solution of (R)-1-(8-hydroxyoctyl)-3-methyl-N-(2-(1-methylpyrrolidin-2-yl)-1H-pyrrolo[3,2-c]pyridin-6-yl)-1H-indazole-6-carboxamide hydrochloride (**Intermediate 83**, 450 mg, 0.806 mmol) in DCM (10 mL) was added $PPh_3$ (634 mg, 2.42 mmol) and imidazole (494 mg, 7.25 mmol), under $N_2$. $I_2$ (675 mg, 2.66 mmol) was then added and the mixture was stirred at room temperature for 3h. Water (10 mL) was added and the mixture was extracted with EtOAc (3 × 20 mL). The organic layers were combined, washed with brine (50 mL), dried over anhydrous sodium sulfate, filtered and concentrated under reduced pressure. The residue was chromatographed on a silica gel column, eluting with EtOAc/petroleum ether (3/7), to afford (R)-1-(8-iodooctyl)-3-methyl-N-(2-(1-methylpyrrolidin-2-yl)-1H-pyrrolo[3,2-c]pyridin-6-yl)-1H-indazole-6-carboxamide (**Intermediate 64**, 280 mg 51.06%) as a yellow solid. Mass spec: m/z 613.0 $[M+H]^+$.

**Step 7**

**Example 12: 1-(8-((2-(2,6-dioxopiperidin-3-yl)-1,3-dioxoisoindolin-4-yl)oxy)octyl)-3-methyl-N-(2-((R)-1-methylpyrrolidin-2-yl)-1H-pyrrolo[3,2-c]pyridin-6-yl)-1H-indazole-6-carboxamide**

To a solution of (R)-1-(8-iodooctyl)-3-methyl-N-(2-(1-methylpyrrolidin-2-yl)-1H-pyrrolo[3,2-c]pyridin-6-yl)-1H-indazole-6-carboxamide (**Intermediate 64**, 80.0 mg, 0.118 mmol) in DMSO (2 mL) was added 2-(2,6-dioxopiperidin-3-yl)-4-hydroxyisoindoline-1,3-dione (CAS No: 5054-59-1, 32.2 mg, 0.118 mmol), $NaHCC_3$ (29.6 mg, 0.354 mmol) and KI (19.5 mg, 0.118 mmol) and the reaction was stirred overnight at 100°C. The resulting crude was chromatographed on a C18 silica column, eluting with water/MeCN (5/5) to afford crude product (40 mg). This was further purified by preparative HPLC (Column: YMC-Actus Triart C18 ExRs, 30*150 mm, 5μm; Mobile Phase A: Water (10 mmol/L $NH_4HCO_3$), Mobile Phase B: MeCN; Flow rate: 60 mL/min; Gradient: 38% B to 68% B in 7 min, 68% B to 81% B in 10 min) to afford 1-(8-((2-(2,6-dioxopiperidin-3-yl)-1,3-dioxoisoindolin-4-yl)oxy)octyl)-3-methyl-N-(2-((R)-1-methylpyrrolidin-2-yl)-1H-pyrrolo[3,2-c]pyridin-6-yl)-1H-indazole-6-carboxamide (**Example 12**, 7.00 mg 7.8%) as a white solid. $^1H$ NMR (400 MHz, DMSO-d6) δ ppm as 11.38 (s, 1H), 11.10 (s, 1H), 10.54 (s, 1H), 8.52 (s, 1H), 8.44 (s, 1H), 8.25 (s, 1H), 7.72 - 7.80 (m, 3H), 7.41 - 7.48 (m, 2H), 6.41 - 6.53 (m, 1H), 5.05 - 5.09 (m, 1H), 4.41 (t, *J* = 6.8Hz, 2H), 4.16 (t, *J* = 6.4Hz, 2H),

3.16 - 3.17 (m, 1H), 2.85 - 2.95 (m, 1H), 2.55 - 2.65 (m, 2H), 2.45 - 2.55 (m, 2H), 2.20 - 2.25 (m, 1H), 2.15 - 2.20 (m, 4H), 1.65 - 2.01(m, 10H) 1.40 - 1.55 (m, 2H), 1.25 - 1.30 (m, 6H). Mass spec: m/z 759.5 $[M+H]^+$.

**Example 13 was synthesised following Scheme 17**

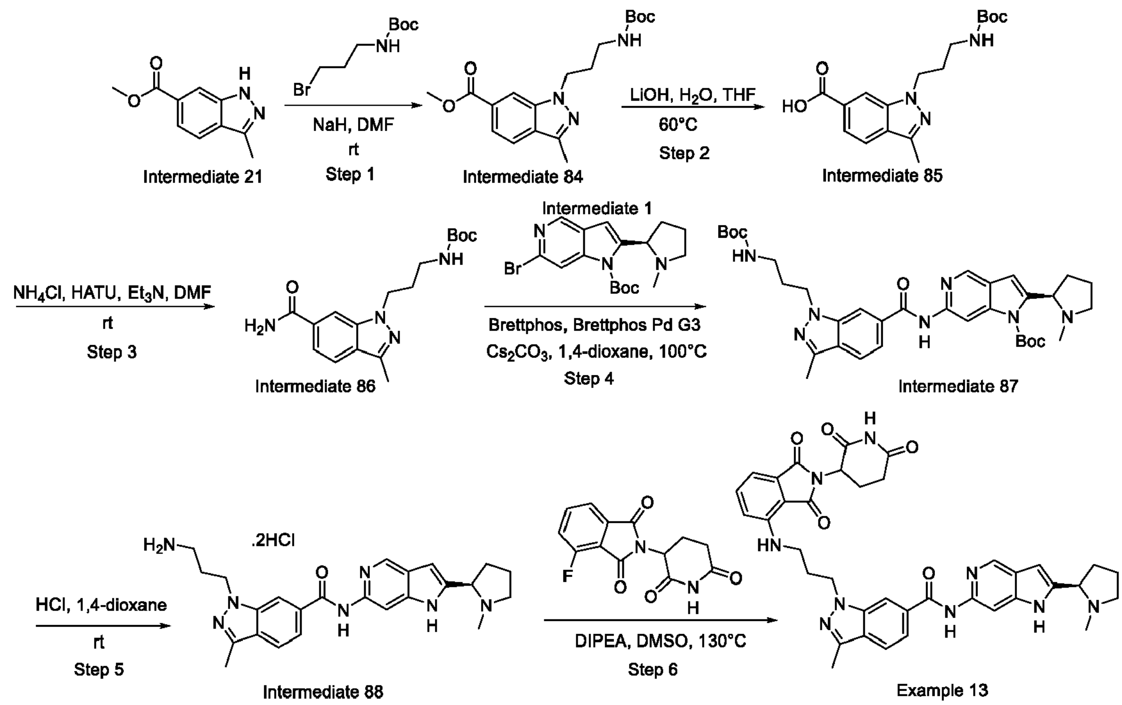


**Scheme 17**

**Step 1**

**Intermediate 84: methyl 1-(3-((tert-butoxycarbonyl)amino)propyl)-3-methyl-1H-indazole-6-carboxylate**

To a solution of methyl 3-methyl-1H-indazole-6-carboxylate (**Intermediate 21**, 500 mg, 2.50 mmol) in DMF (8 mL) was added NaH (60% in mineral oil, 150 mg, 3.75 mmol) at 0°C. The reaction was then stirred for 30 min at 0 °C. tert-Butyl (3-bromopropyl)carbamate (CAS No: 83948-53-2, 751 mg, 3.00 mmol) was then added and the reaction was stirred at room temperature for 2h. Water (20 mL) was added and the aqueous phase was extracted with EtOAc (2 × 50 mL). The organic layers were combined, washed with brine, dried over anhydrous sodium sulfate, filtered and concentrated under reduced pressure. The residue was then chromatographed on a silica gel column, eluting with MeOH/DCM (2/98), to afford methyl 1-(3-((tert-butoxycarbonyl)amino)propyl)-3-methyl-1H-indazole-6-carboxylate (**Intermediate 84**, 270 mg, 28%) as a colorless oil. Mass spec: m/z: 348.2 $[M+H]^+$.

**Step 2**

**Intermediate 85: 1-(3-((tert-butoxycarbonyl)amino)propyl)-3-methyl-1H-indazole-6-carboxylic acid**

To a stirred solution of methyl 1-(3-((tert-butoxycarbonyl)amino)propyl)-3-methyl-1H-indazole-6-carboxylate (**Intermediate 84**, 454 mg, 1.18 mmol) in THF (8 mL) and water (2 mL) was added LiOH (141 mg, 5.88 mmol) and the resulting mixture was stirred at 60°C for 2h. The pH value of the solution was adjusted to pH ~5 by dropwise addition of dilute aqueous HCl (1M) and the resulting mixture was extracted with EtOAc (2 × 50 mL). The combined organic layers were washed with brine and dried over anhydrous $Na_2SO_4$ and then concentrated under reduced pressure to afford 1-(3-((tert-butoxycarbonyl)amino)propyl)-3-methyl-1H-indazole-6-carboxylic acid (**Intermediate 85**, 230 mg, 52%) as a white solid. Mass spec: m/z: 334.1 $[M+H]^+$.

**Step 3**

**Intermediate 86: tert-butyl (3-(6-carbamoyl-3-methyl-1H-indazol-1-yl)propyl)carbamate**

A 100 mL round-bottom flask was charged with 1-(3-((tert-butoxycarbonyl)amino)propyl)-3-methyl-1H-indazole-6-carboxylic acid (**Intermediate 85**, 332 mg, 0.896 mmol), $NH_4Cl$ (240 mg, 4.48 mmol), DMF (6 mL), HATU (511 mg, 1.34 mmol), DIPEA (348 mg, 2.69 mmol) and the reaction was stirred overnight at room temperature. The mixture was chromatographed on a C18 silica column, eluting with MeCN/water (with 0.5% $NH_4HCO_3$) (3/7) to afford tert-butyl (3-(6-carbamoyl-3-methyl-1H-indazol-1-yl)propyl)carbamate (**Intermediate 86**, 280 mg, 89%) as a white solid. Mass spec: m/z: 333.2[M+H]$^+$.

**Step 4**

**Intermediate 87: tert-butyl (R)-6-(1-(3-((tert-butoxycarbonyl)amino)propyl)-3-methyl-1H-indazole-6-carboxamido)-2-(1-methylpyrrolidin-2-yl)-1H-pyrrolo[3,2-c]pyridine-1-carboxylate**

A 100 mL round-bottom flask was charged with tert-butyl (3-(6-carbamoyl-3-methyl-1H-indazol-1-yl)propyl) carbamate (**Intermediate 86**, 120 mg, 0.343 mmol), tert-butyl (R)-6-bromo-2-(1-methylpyrrolidin-2-yl)-1H-pyrrolo[3,2-c] pyridine-1-carboxylate (**Intermediate 1**, 159 mg, 0.377 mmol), Brettphos (55.2 mg, 0.103 mmol), Brettphos Pd G3 (187 mg, 0.206 mmol), $Cs_2CO_3$ (559 mg, 1.72 mmol) in 1,4-dioxane (8 mL) and the reaction was stirred at 100°C overnight, under $N_2$. Water (10 mL)was added and the aqueous phase was extracted with EtOAc (2 × 50 mL). The combined organic layers were washed with brine, dried over anhydrous $Na_2SO_4$, and concentrated under reduced pressure. The resulting residue was chromatographed on a silica gel column, eluting with MeOH/DCM (1/9), to afford tert-butyl (R)-6-(1-(3-((tert-butoxycarbonyl)amino)propyl)-3-methyl-1H-indazole-6-carboxamido)-2-(1-methylpyrrolidin-2-yl)-1H-pyrrolo[3,2-c]pyridine-1-carboxylate (**Intermediate 87**, 70 mg, 29.1%) as a light yellow oil. Mass spec: m/z: 632.4 [M+H]$^+$.

**Step 5**

**Intermediate 88: (R)-1-(3-aminopropyl)-3-methyl-N-(2-(1-methylpyrrolidin-2-yl)-1H-pyrrolo[3,2-c]pyridin-6-yl)-1H-indazole-6-carboxamide dihydrochloride**

To a solution of tert-butyl (R)-6-(1-(3-((tert-butoxycarbonyl)amino)propyl)-3-methyl-1H-indazole-6-carboxamido)-2-(1-methylpyrrolidin-2-yl)-1H-pyrrolo[3,2-c]pyridine-1-carboxylate (**Intermediate 87**, 70 mg, 0.125 mmol) in 1,4-dioxane (5 mL) was added concentrated aqueous HCl (1 mL) and the reaction was stirred for 1 h at room temperature. The volatiles were then removed under reduced pressure to afford (R)-1-(3-aminopropyl)-3-methyl-N-(2-(1-methylpyrrolidin-2-yl)-1H-pyrrolo[3,2-c]pyridin-6-yl)-1H-indazole-6-carboxamide dihydrochloride (**Intermediate 88**, 70 mg, 97%) as an off-white solid, which was used in the next step without further purification. Mass spec: m/z: 433.2 [M+H]$^+$.

**Step 6**

**Example 13: 1-(3-((2-(2,6-dioxopiperidin-3-yl)-1,3-dioxoisoindolin-4-yl)amino)propyl)-3-methyl-N-(2-((R)-1-methylpyrrolidin-2-yl)-1H-pyrrolo[3,2-c]pyridin-6-yl)-1H-indazole-6-carboxamide**

To a solution of (R)-1-(3-aminopropyl)-3-methyl-N-(2-(1-methylpyrrolidin-2-yl)-1H-pyrrolo[3,2-c]pyridin-6-yl)-1H-indazole-6-carboxamide dihydrochloride (**Intermediate 88**, 70 mg, 0.122 mmol), 2-(2,6-dioxopiperidin-3-yl)-4-fluoroisoindoline-1,3-dione (CAS No: 835616-60-9, 67.2 mg, 0.244 mmol) in DMSO (4 mL) was added DIPEA (94.3 mg, 0.732 mmol) and the reaction was stirred at 130°C for 1h. The reaction mixture was then purified by a C18 silica column, eluting with MeCN/water (3/2), to afford a crude product. This crude product was further purified by preparative HPLC (Column: XBridge Prep OBD C18 Column, 30* 150 mm, 5μm; Mobile Phase A: Water (with 10 mmol/L $NH_4HCO_3$), Mobile Phase B: MeCN; Flow rate: 60 mL/min; Gradient: 29% B to59 % B in 10 min) to afford 1-(3-((2-(2,6-dioxopiperidin-3-yl)-1,3-dioxoisoindolin-4-yl)amino)propyl)-3-methyl-N-(2-((R)-1-methylpyrrolidin-2-yl)-1H-pyrrolo[3,2-c]pyridin-6-yl)-1H-indazole-6-carboxamide (**Example 13**, 5.2 mg, 6.1%) as a yellow solid. $^1$H NMR (400 MHz, MeOH-d4) δ ppm 8.54 (s, 1H), 8.20 (s, 1H), 8.14 (s, 1H), 7.82 (d, *J* = 8.4 Hz, 1H), 7.67 (d, *J* = 8.4 Hz, 1H), 7.41 (t, *J* = 7.2 Hz, 1H), 6.92 (d, *J* = 7.2 Hz, 1H), 6.82 (d, *J* = 8.8 Hz, 1H), 6.55 (s, 1H), 4.60 - 4.63 (m, 2H), 3.37 - 3.50 (m, 1H), 3.28 - 3.33 (m, 4H), 2.59 - 2.64 (m, 6H), 2.33 - 2.46 (m, 7H), 2.05 - 2.08 (m, 2H), 1.97 - 2.01 (m, 2H). Mass spec: m/z: 688.6 [M+H]$^+$.

**Example 14: 1-(8-{[2-(2,6-dioxopiperidin-3-yl)-1,3-dioxoisoindol-5-yl]amino}octyl)-3-methyl-N-{2-[(2R)-1-methylpyrrolidin-2-yl]-1H-pyrrolo [3,2-c] pyridin-6-yl}indazole-6-carboxamide**

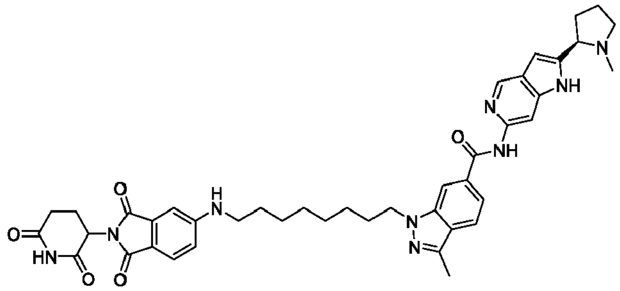

To a solution of 1-(8-aminooctyl)-3-methyl-N-{2-[(2R)-1-methylpyrrolidin-2-yl]-1H-pyrrolo[3,2-c]pyridin-6-yl}indazole-6-carboxamide dihydrochloride (**Intermediate 35**, 100 mg, 0.179 mmol) and 2-(2,6-dioxopiperidin-3-yl)-5-fluoroisoindole-1,3-dione (CAS No: 835616-61-0, 71.6 mg, 0.259 mmol) in DMSO (3 mL) was added DIPEA (258 mg, 1.99 mmol) and the reaction was stirred at 120°C for 4h. the reaction mixture was then purified by preparative HPLC (Column: WelFlash C18, Regular C18 20-40μm, 120g; Mobile Phase A: Water (with 10 mmol/L $NH_4HCO_3$ + 0.05% $NH_3.H_2O$), Mobile Phase B: MeCN; Flow rate: 60 mL/min mL/min; Gradient: 41% B to 71% B in 30 min) to afford 1-(8-{[2-(2,6-dioxopiperidin-3-yl)-1,3-dioxoisoindol-5-yl]amino}octyl)-3-methyl-N-{2-[(2R)-1-methylpyrrolidin-2-yl]-1H-pyrrolo[3,2-c]pyridin-6-yl}indazole-6-carboxamide (**Example 14**, 56.6 mg, 41%) as a yellow solid. (400 MHz, DMSO-d6) δ ppm 11.40 (s, 1H), 11.05 (br, 1H), 10.55 (s, 1H), 8.53 (s, 1H), 8.44 (s, 1H), 8.26 (s, 1H), 7.73 - 7.80 (m, 2H), 7.54 (d, *J* = 8.4 Hz, 1H), 7.05 - 7.08 (m, 1H), 6.92 (s, 1H), 6.80 - 6.82 (m, 1H), 6.41 (s, 1H), 5.00 - 5.05 (m, 1H), 4.39 - 4.42 (m, 2H), 3.08 - 3.17 (m, 3H), 2.80 - 2.95 (m, 1H), 2.50 - 2.60 (m, 5H), 2.26 - 2.28 (m, 1H), 2.08 - 2.18 (m, 4H), 1.83 - 2.01 (m, 6H), 1.52 - 1.55 (m, 2H), 1.20 - 1.40 (m, 9H). Mass spec: m/z: 758.5 $[M+H]^+$.

**Example 15: 1-(8-{[2-(2,6-dioxopiperidin-3-yl)-1,3-dioxoisoindol-5-yl]amino}octyl)-3-methyl-N-{2-[(2S)-1-methylpyrrolidin-2-yl]-1H-pyrrolo[3,2-c]pyridin-6-yl}indazole-6-carboxamide**

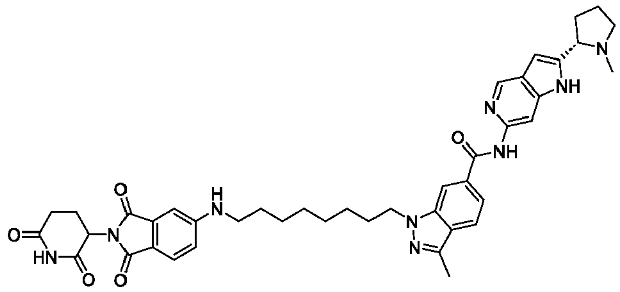

**Example 15** was made in an analogous manner to **Example 14** using 1-(8-aminooctyl)-3-methyl-N-{2-[(2S)-1-methylpyrrolidin-2-yl]-1H-pyrrolo[3,2-c]pyridin-6-yl}indazole-6-carboxamide dihydrochloride (**Intermediate 35**) and 2-(2,6-dioxopiperidin-3-yl)-5-fluoroisoindole-1,3-dione (CAS No: 835616-61-0), to afford 1-(8-{[2-(2,6-dioxopiperidin-3-yl)-1,3-dioxoisoindol-5-yl]amino}octyl)-3-methyl-N-{2-[(2S)-1-methylpyrrolidin-2-yl]-1H-pyrrolo[3,2-c]pyridin-6-yl}indazole-6-carboxamide (**Example 15**). $^1H$ NMR (400 MHz, MeOH-d4) δ ppm 8.57 (s, 1H), 8.21- 8.25 (m, 2H), 7.86 (d, *J* = 8.4 Hz, 1H), 7.75 (d, *J* = 8.4 Hz, 1H), 7.51 (d, *J* = 8.8 Hz, 1H), 6.93 (s, 1H), 6.75 - 6.78 (m, 1H), 6.59 (s, 1H), 5.02-5.07 (m, 1H), 4.44 - 4.48 (m, 2H), 3.62 - 3.66 (m, 1H), 3.12 - 3.15 (m, 2H), 2.82 - 2.86 (m, 1H), 2.67 - 2.73 (m, 2H), 2.61 (s, 3H), 2.55 - 2.57 (m, 1H), 2.36 - 2.43 (m, 4H), 2.11 - 2.16 (m, 3H), 1.94 - 2.01 (m, 3H), 1.61 - 1.68 (m, 2H), 1.42 -1.53 (m, 9H). Mass spec: m/z: 758.65 $[M+H]^+$.

**Example 16: 1-(8-{[2-(2,6-dioxopiperidin-3-yl)-1-oxo-3H-isoindol-4-yl]amino}octyl)-3-methyl-N-{2-[(2R)-1-methylpyrrolidin-2-yl]-1H-pyrrolo[3,2-c]pyridin-6-yl}indazole-6-carboxamide**

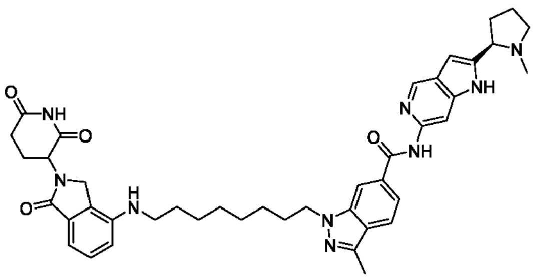

To a solution of 1-(8-aminooctyl)-3-methyl-N-{2-[(2R)-1-methylpyrrolidin-2-yl]-1H-pyrrolo[3,2-c]pyridin-6-yl}indazole-6-carboxamide (**Intermediate 35**, 80.0 mg, 0.143 mmol) and 3-(4-bromo-1-oxo-3H-isoindol-2-yl)piperidine-2,6-dione (CAS No: 2093387-36-9, 67 mg, 0.21 mmol) in 1,4-dioxane (2 mL) was added $Cs_2CO_3$ (156 mg, 0.477 mmol), Pd-PEPPSI-IHept-Cl (CAS No: 1814936-54-3, 15.5 mg, 0.016 mmol) and the reaction was stirred overnight at 100 °C, under $N_2$. The reaction mixture was then purified using preparative HPLC (Column: XselectCSH Prep OBD C18 Column, 30*150 mm, 5μm; Mobile Phase A: Water (with 0.1% formic acid), Mobile Phase B: MeCN; Flow rate: 60 mL/min mL/min; Gradient: 14% B to 34% B in 8 min) to afford get 1-(8-{[2-(2,6-dioxopiperidin-3-yl)-1-oxo-3H-isoindol-4-yl]amino}octyl)-3-methyl-N-{2-[(2R)-1-methylpyrrolidin-2-yl]-1H-pyrrolo[3,2-c]pyridin-6-yl}indazole-6-carboxamide (**Example 16**, 3.6 mg, 3.2%) as a white solid. $^1$H-NMR (400 MHz, MeOH-d4) δ ppm 8.60 (s, 1H), 8.21 - 8.25 (m, 2H), 7.83 (d, J = 8.4 Hz, 1H), 7.74 (d, J = 8.4 Hz, 1H), 7.28 (t, J = 8.0 Hz, 1H), 7.05 (d, J = 7.2 Hz, 1H), 6.77 (d, J = 8.0 Hz, 1H), 6.59 (s, 1H), 5.14 - 5.18 (m, 1H), 4.43 (t, J = 6.8 Hz, 2H), 4.26 (s, 2H), 3.60 - 3.70 (m, 1H), 3.15 (t, J = 7.0 Hz, 2H), 2.85 - 2.95 (m, 1H), 2.70 - 2.80 (m, 1H), 2.55 - 2.60 (m, 4H), 2.35 - 2.44 (m, 5H), 1.91 - 2.18 (m, 7H), 1.55 - 1.65 (m, 2H), 1.20 - 1.40 (m, 9H). Mass spec: m/z: 744.5 $[M+H]^+$.

**Example 17: 1-(8-{[2-(2,6-dioxopiperidin-3-yl)-1-oxo-3H-isoindol-4-yl]amino}octyl)-3-methyl-N-{2-[(2S)-1-methylpyrrolidin-2-yl]-1H-pyrrolo[3,2-c]pyridin-6-yl}indazole-6-carboxamide**

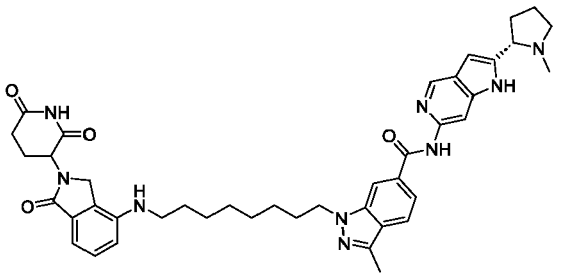

**Example 17** was synthesised in an analogous manner to **Example 16** using 1-(8-aminooctyl)-3-methyl-N-{2-[(2S)-1-methylpyrrolidin-2-yl]-1H-pyrrolo[3,2-c]pyridin-6-yl}indazole-6-carboxamide (**Intermediate 35**) as the amine and 3-(4-bromo-1-oxo-3H-isoindol-2-yl)piperidine-2,6-dione (CAS no: 2093387-36-9) as the aryl bromide, to afford 1-(8-{[2-(2,6-dioxopiperidin-3-yl)-1-oxo-3H-isoindol-4-yl]amino}octyl)-3-methyl-N-{2-[(2S)-1-methylpyrrolidin-2-yl]-1H-pyrrolo[3,2-c]pyridin-6-yl}indazole-6-carboxamide (**Example 17**, 4.6 mg, 6.1%) as a white solid. $^1$H NMR (400 MHz, $CD_3OD$) δ ppm 8.55 (s, 1H), 8.23 (s, 1H), 8.19 (s, 1H), 7.80 (d, *J* = 8.4 Hz, 1H),7.73 (d, *J* = 7.6 Hz, 1H), 7.28 (t, *J* = 8.0 Hz, 1H), 7.05 (d, *J* = 7.2 Hz, 1H), 6.75 (d, *J* = 8.4 Hz, 1H), 6.52 (s, 1H), 5.12 - 5.17 (m, 1H), 4.40 (t, *J* = 7.2 Hz, 2H), 4.26 (s, 2H), 3.45 - 3.47 (m, 1H), 3.25 - 3.30 (m, 1H), 3.12 - 3.15 (m, 2H), 2.81 - 2.89 (m, 1H), 2.77 - 2.78 (m, 1H), 2.58 (s, 3H), 2.40 - 2.44 (m, 2H), 2.25 - 2.31 (m, 4H), 2.11 - 2.16 (m, 1H), 1.92 - 2.05 (m, 2H), 1.88 - 1.90 (m, 2H), 1.56 - 1.60 (m, 2H), 1.29 - 1.31 (m, 9H). Mass spec: m/z: 744.5 $[M+H]^+$.

**Example 18: N-(3-chloro-2-((R)-1-methylpyrrolidin-2-yl)-1H-pyrrolo[3,2-c]pyridin-6-yl)-1-(8-((2-(2,6-dioxopiperidin-3-yl)-1,3-dioxoisoindolin-4-yl)amino)octyl)-3-methyl-1H-indazole-6-carboxamide**

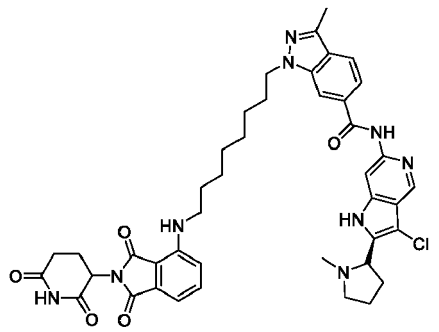

To a solution of 1-(8-((2-(2,6-dioxopiperidin-3-yl)-1,3-dioxoisoindolin-4-yl)amino)octyl)-3-methyl-N-(2-((R)-1-methylpyrrolidin-2-yl)-1H-pyrrolo[3,2-c]pyridin-6-yl)-1H-indazole-6-carboxamide (**Example 3**, 100 mg, 0.125 mmol) in DMF (2 mL) was added NCS (21.8 mg, 0.163 mmol) and the mixture was stirred at room temperature overnight under a nitrogen atmosphere. The resulting crude was purified by preparative HPLC (Column: XBridge Prep RP OBD C18 Column, 30*150 mm, 5μm; Mobile Phase A: Water (10 mmol/L $NH_4HCO_3$), Mobile Phase B: MeCN; Flow rate: 60 mL/min; Gradient: 60% B to 90% B in 7 min) to afford N-(3-chloro-2-((R)-1-methylpyrrolidin-2-yl)-1H-pyrrolo[3,2-c]pyridin-6-yl)-1-(8-((2-(2,6-dioxopiperidin-3-yl)-1,3-dioxoisoindolin-4-yl)amino)octyl)-3-methyl-1H-indazole-6-carboxamide (**Example 18**, 23.5 mg, 23%) as a yellow solid. $^1$H NMR (400 MHz, DMSO-d6) δ ppm 11.72 (s, 1H), 11.09 (s, 1H), 10.70 (s, 1H), 8.53 (s, 1H), 8.45 (s, 1H), 8.32 (s, 1H), 7.73 - 7.80 (m, 2H), 7.54 (t, J = 7.6Hz, 1H), 6.99 - 7.04 (m, 2H), 6.49 (t, J = 5.2Hz, 1H), 5.02 - 5.07 (m, 1H), 4.40 (t, J = 6.4Hz, 2H), 3.50 - 3.60 (m, 1H), 3.20 - 3.32 (m, 3H), 2.80 - 2.93 (m, 1H), 2.60 - 2.65 (m, 1H), 2.50 - 2.55 (m, 3H), 2.25 - 2.35 (m, 1H), 2.15 - 2.22 (m, 4H), 1.80 - 2.05 (m, 6H), 1.40 - 1.45 (m, 2H), 1.30 (s, 9H). Mass spec: m/z 792.4 [M+H]+.

**Example 19 was synthesized following Scheme 18**

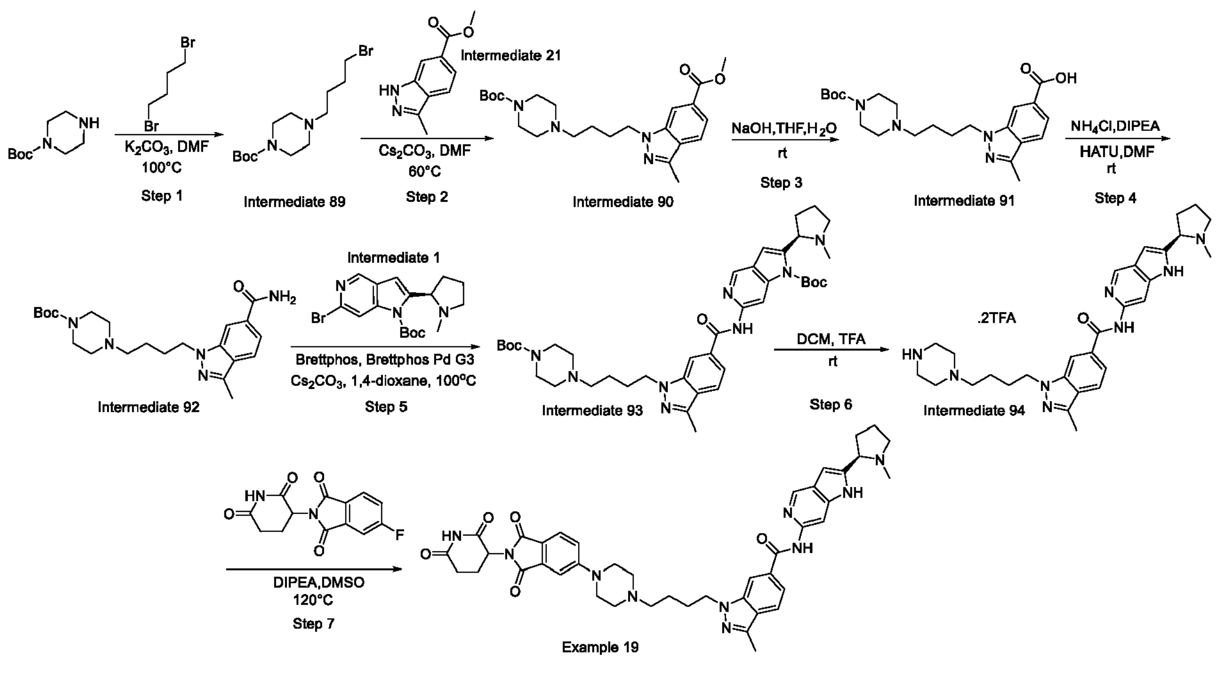


## Scheme 18

**Step 1**

**Intermediate 89: tert-butyl 4-(4-bromobutyl)piperazine-1-carboxylate**

To a solution of tert-butyl piperazine-1-carboxylate (CAS No: 57260-71-6, 1.00 g, 5.10 mmol) in dimethyformamide (10 mL) was added 1,4-dibromobutane (CAS No: 110-52-1, 1.10 g, 5.10 mmol) and potassium carbonate (1.06 g, 7.65 mmol) then the reaction was stirred at 100°C for 5h. The mixture was quenched with water (100 mL) and extracted with ethyl acetate (3 × 100 mL). The combined organic layers were washed with brine, dried over anhydrous $Na_2SO_4$, filtered, and concentrated *in vacuo* to afford tert-butyl 4-(4-bromobutyl)piperazine-1-carboxylate (**Intermediate 89**, 1.50 g, 91%) as a white solid. Mass spec: m/z: 321 $[M+H]^+$.

**Step 2**

**Intermediate 90: methyl 1-{4-[4-(tert-butoxycarbonyl)piperazin-1-yl]butyl}-3-methylindazole-6-carboxylate**

To a solution of tert-butyl 4-(4-bromobutyl)piperazine-1-carboxylate (**Intermediate 89**, 500 mg, 1.55 mmol) in dimethylformamide (15 mL) was added methyl 3-methyl-1H-indazole-6-carboxylate (**Intermediate 21**, 296 mg, 1.55 mmol) and cesium carbonate (1.52 g, 4.66 mmol) and the reaction was stirred at 60°C for 16h. The mixture was quenched with water (100 mL) and extracted with ethyl acetate (3 × 100 mL). The combined organic layers were washed with brine, dried over anhydrous $Na_2SO_4$, filtered and concentrated *in vacuo.* The crude material was purified by combi-flash column chromatography, by eluting with 2% MeOH in DCM, to afford methyl 1-{4-[4-(tert-butoxycarbonyl)piperazin-1-yl]butyl} -3-methylindazole-6-carboxylate (**Intermediate 90**, 200 mg, 30%) as a white solid. Mass spec: m/z: 431 $[M+H]^+$.

**Step 3**

**Intermediate 91: 1-{4-[4-(tert-butoxycarbonyl)piperazin-1-yl]butyl}-3-methylindazole-6-carboxylic acid**

To a solution of methyl-1-{4-[4-(tert-butoxycarbonyl)piperazin-1-yl]butyl}-3-methylindazole-6-carboxylate (**Intermediate 90**, 200 mg, 0.460 mmol) in tetrahydrofuran (4 mL) was added water (4 mL) and sodium hydroxide (81.3 mg, 2.03 mmol) and the reaction was stirred at room temperature for 16h. The pH of the reaction mixture was adjusted to pH 5 by the dropwise addition of concentrated aqueous HCl and then extracted with ethyl acetate (3 × 100 mL). The combined organic phases were then dried over anhydrous sodium sulfate and concentrated *in vacuo* to afford 1-{4-[4-(tert-butoxycarbonyl)piperazin-1-yl]butyl} -3-methylindazole-6-carboxylic acid (**Intermediate 91**, 180 mg, 89%) as a white solid, which was used in the next step without further purification. Mass spec: m/z: 417 $[M+H]^+$.

**Step 4**

**Intermediate 92: tert-butyl 4-[4-(6-carbamoyl-3-methylindazol-1-yl)butyl]piperazine-1-carboxylate**

To a solution of 1-{4-[4-(tert-butoxycarbonyl)piperazin-1-yl]butyl}-3-methylindazole-6-carboxylic acid (**Intermediate 91**, 180 mg, 0.427 mmol) in dimethylformamide (5 mL) was added DIPEA (331 mg, 2.56 mmol), HATU (243 mg, 0.640 mmol) and $NH_4Cl$ (91.4 mg, 1.70 mmol) and the reaction was stirred at room temperature for 16h. The mixture was quenched with water (100 mL) and the aqueous phase extracted with ethyl acetate (3 × 100 mL). The combined organic layers were washed with brine, dried over anhydrous $Na_2SO_4$, filtered and concentrated *in vacuo.* The crude material was purified by combi-flash column chromatography, by eluting with 9% MeOH in DCM, to afford tert-butyl 4-[4-(6-carbamoyl-3-methylindazol-1-yl)butyl]piperazine-1-carboxylate (**Intermediate 92**, 180 mg, 98%) as a yellow solid. Mass spec: m/z: 416 $[M+H]^+$.

**Step 5**

**Intermediate 93: tert-butyl 4-[4-(6-{[1-(tert-butoxycarbonyl)-2-[(2R)-1-methylpyrrolidin-2-yl]pyrrolo[3,2-c]pyridin-6-yl]carbamoyl}-3-methylindazol-1-yl)butyl]piperazine-1-carboxylate**

To a solution of tert-butyl 4-[4-(6-carbamoyl-3-methylindazol-1-yl)butyl]piperazine-1-carboxylate (**Intermediate 92**, 140 mg, 0.337 mmol) in 1,4-dioxane (5 mL) was added tert-butyl 6-bromo-2-[(2R)-1-methylpyrrolidin-2-yl]pyrrolo[3,2-c]pyridine-1-carboxylate (**Intermediate 1**, 128 mg, 0.337 mmol), Brettphos (108 mg, 0.202 mmol), Brettphos Pd G3 (91.6 mg, 0.101 mmol) and cesium carbonate (219 mg, 0.674 mmol). The resulting mixture was stirred at 100°C for 16h. The crude material was purified by combi-flash column chromatography, by eluting with 12% MeOH in DCM, to afford tert-butyl 4-[4-(6-{ [1-(tert-butoxycarbonyl)-2-[(2R)-1-methylpyrrolidin-2-yl]pyrrolo[3,2-c]pyridin-6-yl]carbamoyl}-3-methylindazol-1-yl)butyl]piperazine-1-carboxylate (**Intermediate 92**, 150 mg, 59%) as a light yellow solid. Mass spec: m/z: 715 $[M+H]^+$.

**Step 6**

**Intermediate 94: 3-methyl-N-{2-[(2R)-1-methylpyrrolidin-2-yl]-1H-pyrrolo[3,2-c]pyridin-6-yl}-1-[4-(piperazin-1-yl)butyl]indazole-6-carboxamide ditrifluoroacetate**

To a solution of tert-butyl 4-[4-(6-{[1-(tert-butoxycarbonyl)-2-[(2R)-1-methylpyrrolidin-2-yl]pyrrolo[3,2-c]pyridin-6-yl]carbamoyl}-3-methylindazol-1-yl)butyl]piperazine-1-carboxylate (130 mg, 0.182 mmol) in dichloromethane (1 mL) was added TFA (0.5 mL). The reaction mixture was stirred at room temperature for 1h and concentrated *in vacuo* to afford 3-methyl-N-{2-[(2R)-1-methylpyrrolidin-2-yl]-1H-pyrrolo[3,2-c]pyridin-6-yl}-1-[4-(piperazin-1-yl)butyl]indazole-6-carboxamide ditrifluoroacetate (**Intermediate 94**, 93.0 mg, 94%) as a brown oil, which was used for the next step without further purification. Mass spec: m/z: 515 $[M+H]^+$.

**Step 7**

**Example 19: 1-(4-{4-[2-(2,6-dioxopiperidin-3-yl)-1,3-dioxoisoindol-5-yl]piperazin-1-yl}butyl)-3-methyl-N-{2-[(2R)-1-methylpyrrolidin-2-yl]-1H-pyrrolo[3,2-c]pyridin-6-yl}indazole-6-carboxamide**

To a solution of 3-methyl-N-{2-[(2R)-1-methylpyrrolidin-2-yl]-1H-pyrrolo[3,2-c]pyridin-6-yl}-1-[4-(piperazin-1-yl)butyl]indazole-6-carboxamide ditrifluoroacetate (**Intermediate 94**, 93.0 mg, 0.181 mmol) in DMSO (2 mL) was added 2-(2,6-dioxopiperidin-3-yl)-5-fluoroisoindole-1,3-dione (CAS No: 835616-61-0, 49.9 mg, 0.181 mmol) and DIPEA (140 mg, 1.086 mmol) and the resulting mixture was stirred at 120°C for 16h. The crude product was then purified by preparative HPLC (Column: XBridge Prep OBD C18 Column, 30*150 mm, 5μm; Mobile Phase A: Water (+10 mmol/L $NH_4HCO_3$), Mobile Phase B: MeCN; Flow rate: 60 mL/min mL/min; Gradient: 30% B to 60% B in 7 min) to afford 1-(4-{4-[2-(2,6-dioxopiperidin-3-yl)-1,3-dioxoisoindol-5-yl]piperazin-1-yl}butyl)-3-methyl-N-{2-[(2R)-1-methylpyrrolidin-2-yl]-1H-pyrrolo[3,2-c]pyridin-6-yl}indazole-6-carboxamide (**Example 19**, 22 mg, 15%) as a yellow solid. $^1H$ NMR (400 MHz, MeOH-d4) δ ppm 8.55 (s, 1H), 8.22 (d, *J* = 5.6 Hz, 2H), 7.87 (d, *J* = 8.4 Hz, 1H), 7.75 (d, *J* = 8.8 Hz, 1H), 7.60 (d, *J* = 8.4 Hz 1H), 7.30 (s, 1H), 7.14 (d, *J* = 8.8 Hz 1H), 6.53 (s, 1H), 5.05 - 5.09 (m, 1H), 4.52 (t, J = 7.2 Hz, 2H), 3.40 - 3.47 (m, 5H), 3.32 - 3.33 (m, 1H), 2.82 - 2.87 (m, 3H), 2.70 - 2.77 (m, 3H), 2.57 - 2.62 (m, 4H), 2.32 (s, 3H), 2.11 - 2.12 (m, 4H), 2.01 - 2.09 (m, 6H), 1.57-1.59 (m, 2H). Mass spec: m/z: 771 $[M+H]^+$.

**Example 20 was synthesized following Scheme 19**

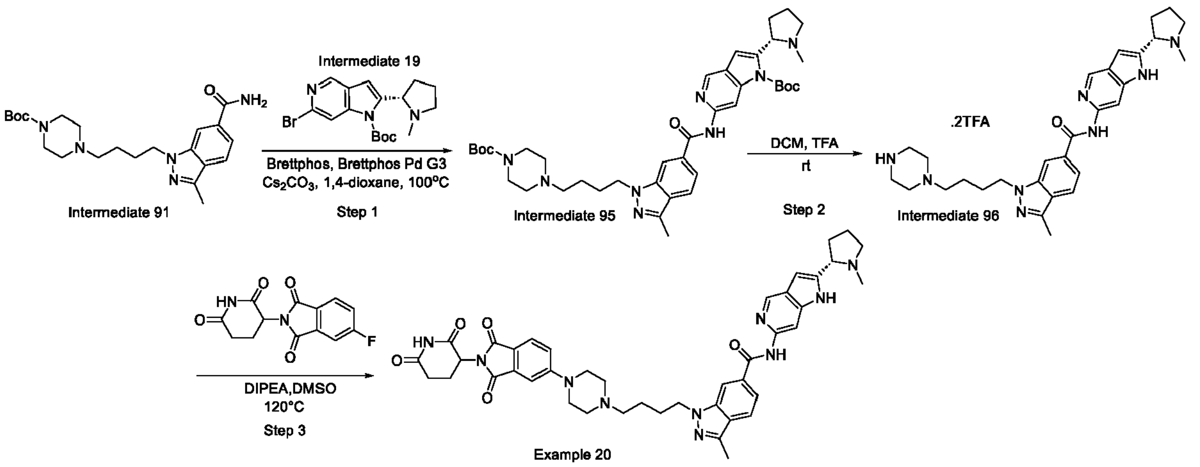


Scheme 19

**Step 1**

**Intermediate 95: tert-butyl 4-[4-(6-{[1-(tert-butoxycarbonyl)-2-[(2S)-1-methylpyrrolidin-2-yl]pyrrolo[3,2-c]pyridin-6-yl]carbamoyl}-3-methylindazol-1-yl)butyl]piperazine-1-carboxylate**

**Intermediate 95** was synthesised in an analogous manner to **Intermediate 93**, shown in **Step 5** of **Scheme 18**, using tert-butyl 4-[4-(6-carbamoyl-3-methylindazol-1-yl)butyl]piperazine-1-carboxylate (**Intermediate 91**) as the amide and *tert*-butyl 6-bromo-2-[(2S)-1-methylpyrrolidin-2-yl]pyrrolo[3,2-c]pyridine-1-carboxylate as the aryl bromide (**Inter-**

**mediate 19**) to afford tert-butyl 4-[4-(6-{[1-(tert-butoxycarbonyl)-2-[(2S)-1-methylpyrrolidin-2-yl]pyrrolo[3,2-c]pyridin-6-yl]carbamoyl}-3-methylindazol-1-yl)butyl]piperazine-1-carboxylate (**Intermediate 95**). Mass spec: m/z: 715.4 [M+H]$^+$.

**Step 2**

**Intermediate 96: 3-methyl-N-{2-[(2S)-1-methylpyrrolidin-2-yl]-1H-pyrrolo[3,2-c]pyridin-6-yl}-1-[4-(piperazin-1-yl)butyl]indazole-6-carboxamide ditrifluoroacetate**

**Intermediate 96** was synthesised in an analogous manner to **Intermediate 94**, shown in **Step 6** of **Scheme 18**, using TFA as the acid to afford 3-methyl-N-{2-[(2S)-1-methylpyrrolidin-2-yl]-1H-pyrrolo[3,2-c]pyridin-6-yl}-1-[4-(piperazin-1-yl)butyl]indazole-6-carboxamide ditrifluoroacetate (**Intermediate 96**). Mass spec: m/z: 515.4 [M+H]$^+$.

**Step 3**

**Example 20: 1-(4-{4-[2-(2,6-dioxopiperidin-3-yl)-1,3-dioxoisoindol-5-yl]piperazin-1-yl}butyl)-3-methyl-N-{2-[(2S)-1-methylpyrrolidin-2-yl]-1H-pyrrolo[3,2-c]pyridin-6-yl}indazole-6-carboxamide**

**Example 20** was synthesised in an analogous manner to **Example 19**, shown in **Step 7** of **Scheme 18**, using 3-methyl-N-{2-[(2S)-1-methylpyrrolidin-2-yl]-1H-pyrrolo[3,2-c]pyridin-6-yl}-1-[4-(piperazin-1-yl)butyl]indazole-6-carboxamide ditrifluoroacetate (**Intermediate 96**) as the amine and 2-(2,6-dioxopiperidin-3-yl)-5-fluoroisoindole-1,3-dione (CAS No: 835616-61-0) as the aryl fluoride, to afford 1-(4-{4-[2-(2,6-dioxopiperidin-3-yl)-1,3-dioxoisoindol-5-yl]piperazin-1-yl}butyl)-3-methyl-N-{2-[(2S)-1-methylpyrrolidin-2-yl]-1H-pyrrolo[3,2-c]pyridin-6-yl}indazole-6-carboxamide (**Example 20**). $^1$H NMR (400 MHz, $CD_3OD$) δ ppm 8.54 (s, 1H), 8.24 (d, $J$ = 7.6 Hz, 2H), 7.88 (d, $J$ = 9.2 Hz, 1H), 7.75 (d, $J$ = 7.2 Hz, 1H), 7.60 (d, $J$ = 8.8 Hz, 1H), 7.32 (s, 1H), 7.12 - 7.15 (m, 1H), 6.53 (s, 1H), 4.51 - 4.54 (m, 2H), 3.63 - 3.65 (m, 1H), 3.49 - 3.51 (m, 1H), 3.40 - 3.46 (m, 6H), 3.25 - 3.27 (m, 1H), 3.14 - 3.15 (m, 1H), 2.65 - 2.71 (m, 3H), 2.56 - 2.61 (m, 4H), 2.40 - 2.48 (m, 3H), 2.25 - 2.28 (m, 4H), 2.02 - 2.11 (m, 6H), 1.57 - 1.59 (m, 2H). Mass spec: m/z: 771.7 [M+H]$^+$.

**Example 21 was synthesised following Scheme 20**

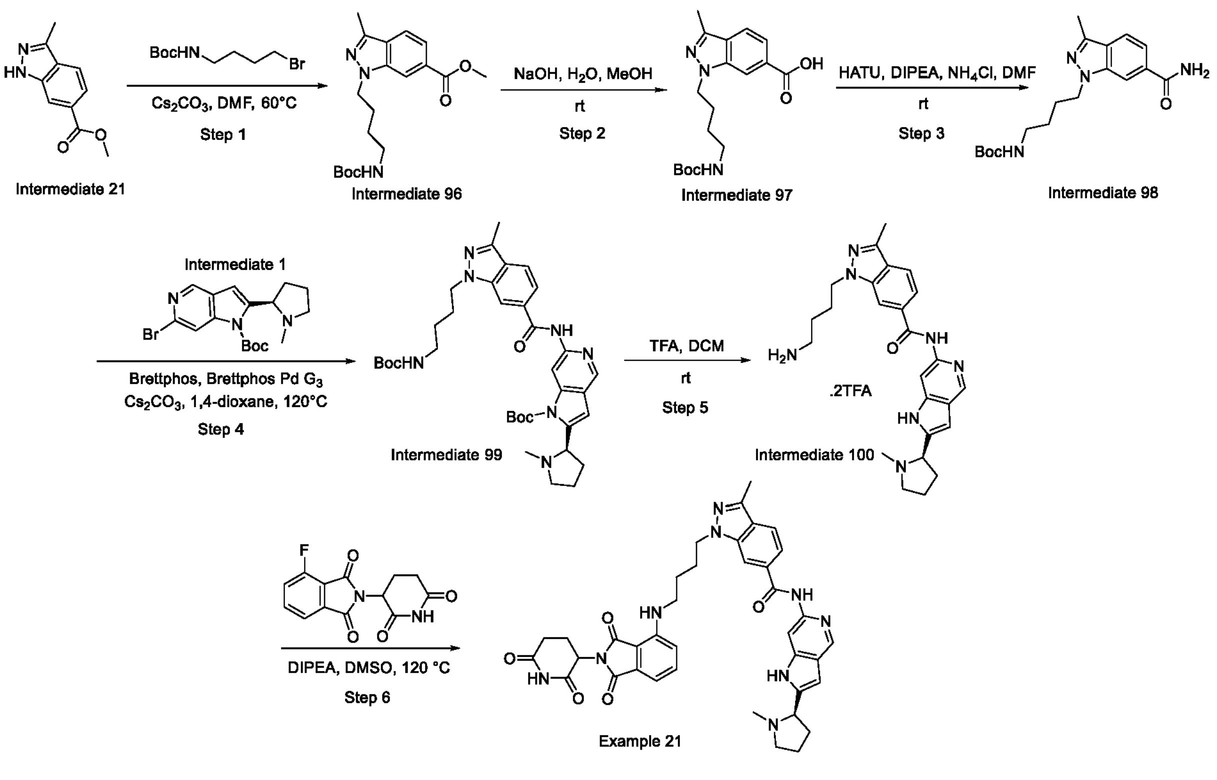


**Scheme 20**

**Step 1**

**Intermediate 96: methyl 1-(4-((tert-butoxycarbonyl)amino)butyl)-3-methyl-1H-indazole-6-carboxylate**

To a solution of methyl 3-methyl-1H-indazole-6-carboxylate (**Intermediate 21**, 500 mg, 2.37 mmol) in DMF (10 mL) was added tert-butyl N-(4-bromobutyl)carbamate (CAS No: 164365-88-2, 776 mg, 3.08 mmol) and $Cs_2CO_3$ (2.31 g, 7.10 mmol) and the reaction was stirred at 60°C for 3h. Water (30 mL) was added and the aqueous phase was extracted with EtOAc (3 × 50 mL). The organic layers were combined, washed with brine (200 mL), dried over anhydrous sodium sulfate, filtered and concentrated under reduced pressure. The residue was chromatographed on a silica gel column, eluting with EtOAc/petroleum ether (20/80) to afford methyl 1-(4-((tert-butoxycarbonyl)amino)butyl)-3-methyl-1H-indazole-6-carboxylate (**Intermediate 96**, 550 mg, 63%) as yellow oil. Mass spec: m/z 362.2 $[M+H]^+$.

**Step 2**

**Intermediate 97: 1-(4-((tert-butoxycarbonyl)amino)butyl)-3-methyl-1H-indazole-6-carboxylic acid**

To a solution of methyl 1-(4-((tert-butoxycarbonyl)amino)butyl)-3-methyl-1H-indazole-6-carboxylate (**Intermediate 96**, 550 mg, 1.49 mmol) in MeOH (10 mL) was added NaOH (800 mg, 20.0 mmol) in $H_2O$ (10 mL) and the reaction was stirred at room temperature for 3h. The pH of the mixture was adjusted to pH ~6 by the dropwise addition of diluted aqueous HCl (1M). The mixture was then extracted with EtOAc (3 × 50 mL) and the organic layers were combined, washed with brine (200 mL), dried over anhydrous sodium sulfate, filtered and concentrated under reduced pressure to afford 1-(4-((tert-butoxycarbonyl)amino)butyl)-3-methyl-1H-indazole-6-carboxylic acid (**Intermediate 97**, 520 mg, 98%) as a yellow solid, which was used in the next step without further purification. Mass spec: m/z 348.1 $[M+H]^+$.

**Step 3**

**Intermediate 98: tert-butyl (4-(6-carbamoyl-3-methyl-1H-indazol-1-yl)butyl)carbamate**

To a solution of 1-(4-((tert-butoxycarbonyl)amino)butyl)-3-methyl-1H-indazole-6-carboxylic acid (**Intermediate 97**, 250 mg, 0.705 mmol, 98%) in DMF (5 mL) was added $NH_4Cl$ (151 mg, 2.82 mmol), HATU (402 mg, 1.06 mmol) and DIPEA (547 mg, 4.23 mmol) and the reaction was stirred overnight at room temperature. Water (10 mL) was then added and he resulting solution was extracted with EtOAc (3 × 20 mL). The organic layers were combined, washed with brine (100 mL), dried over anhydrous sodium sulfate, filtered and concentrated under reduced pressure. The residue was then chromatographed on a silica gel column, eluting with MeOH/DCM (3/97) to afford tert-butyl (4-(6-carbamoyl-3-methyl-1H-indazol-1-yl)butyl)carbamate (**Intermediate 98**, 240 mg, 97%) as yellow oil. Mass spec: m/z 347.2 $[M+H]^+$.

**Step 4**

**Intermediate 99: tert-butyl (R)-6-(1-(4-((tert-butoxycarbonyl)amino)butyl)-3-methyl-1H-indazole-6-carboxamido)-2-(1-methylpyrrolidin-2-yl)-1H-pyrrolo [3,2-c] pyridine- 1-carboxylate**

To a solution of tert-butyl (4-(6-carbamoyl-3-methyl-1H-indazol-1-yl)butyl)carbamate (**Intermediate 98**, 200 mg, 0.57 mmol) in 1,4-dioxane (4 mL) was added tert-butyl (R)-6-bromo-2-(1-methylpyrrolidin-2-yl)-1H-pyrrolo[3,2-c]pyridine-1-carboxylate (**Intermediate 1**, 217 mg, 0.572 mmol), Brettphos (184 mg, 0.343 mmol), Brettphos Pd $G_3$ (155 mg, 0.172 mmol) and $Cs_2CO_3$ (372 mg, 1.14 mmol) and the mixture was stirred overnight at 120 °C under nitrogen. The volatiles were then concentrated under reduced pressure and the resulting residue was chromatographed on a silica gel column, eluting with MeOH/DCM (4/96), to afford tert-butyl (R)-6-(1-(4-((tert-butoxycarbonyl)amino)butyl)-3-methyl-1H-indazole-6-carboxamido)-2-(1-methylpyrrolidin-2-yl)-1H-pyrrolo[3,2-c]pyridine-1-carboxylate (**Intermediate 99**, 160 mg, 37%) as yellow oil. Mass spec: m/z 646.3 $[M+H]^+$.

**Step 5**

**Intermediate 100: (R)-1-(4-aminobutyl)-3-methyl-N-(2-(1-methylpyrrolidin-2-yl)-1H-pyrrolo[3,2-c]pyridin-6-yl)-1H-indazole-6-carboxamide ditrifluoroacetate**

To a solution of tert-butyl (R)-6-(1-(4-((tert-butoxycarbonyl)amino)butyl)-3-methyl-1H-indazole-6-carboxamido)-2-(1-methylpyrrolidin-2-yl)-1H-pyrrolo[3,2-c]pyridine-1-carboxylate (**Intermediate 99**, 160 mg, 0.21 mmol) in DCM (2 mL) was added TFA (1 mL) and the reaction was stirred at room temperature for 2h. The volatiles were then removed

under reduced pressure to afford (R)-1-(4-aminobutyl)-3-methyl-N-(2-(1-methylpyrrolidin-2-yl)-1H-pyrrolo[3,2-c]pyridin-6-yl)-1H-indazole-6-carboxamide ditrifluoroacetate **(Intermediate 100,** 110 mg, 93%) as yellow oil, which was used in the next step without further purification. Mass spec: m/z 446.2 $[M+H]^+$.

**Step 6**

**Example 21: 1-(4-((2-(2,6-dioxopiperidin-3-yl)-1,3-dioxoisoindolin-4-yl)amino)butyl)-3-methyl-N-(2-((R)-1-methylpyrrolidin-2-yl)-1H-pyrrolo[3,2-c]pyridin-6-yl)-1H-indazole-6-carboxamide**

To a solution of (R)-1-(4-aminobutyl)-3-methyl-N-(2-(1-methylpyrrolidin-2-yl)-1H-pyrrolo[3,2-c]pyridin-6-yl)-1H-indazole-6-carboxamide ditrifluoroacetate **(Intermediate 100,** 110 mg, 0.197 mmol) in DMSO (4 mL) was added 2-(2,6-dioxopiperidin-3-yl)-4-fluoroisoindoline-1,3-dione (CAS No: 835616-60-9, 65.5 mg, 0.236 mmol) and DIPEA (76.6 mg, 0.591 mmol) and the reaction mixture was stirred overnight at 120 °C. Water (10 mL) was added and resulting mixture was purified by preparative SFC (Column: DAICEL DCpak P4VP 3*25 cm, 5 um; Mobile Phase A: $CO_2$, Mobile Phase B: MeOH (1% 2M $NH_3$ in MeOH); Flow rate: 65 mL/min; Gradient: isocratic 46% B) to afford 1-(4-((2-(2,6-dioxopiperidin-3-yl)-1,3-dioxoisoindolin-4-yl)amino)butyl)-3-methyl-N-(2-((R)-1-methylpyrrolidin-2-yl)-1H-pyrrolo[3,2-c]pyridin-6-yl)-1H-indazole-6-carboxamide **(Example 21,** 16.8 mg, 12%) as a yellow solid. $^1H$ NMR (400 MHz, DMSO-d6) δ ppm 11.41 (s, 1H), 11.08 (s, 1H), 10.54 (s, 1H), 8.48 (s, 1H), 8.53 (s, 1H), 8.26 (s, 1H), 7.73 - 7.81 (m, 2H), 7.54 (t, *J* = 7.2Hz, 1H), 7.08 (d, *J* = 8.4Hz, 1H), 7.00 (d, *J* = 6.8Hz, 1H), 6.61 (t, *J* = 5.6Hz, 1H), 6.41 (s, 1H), 5.01 - 5.05 (m, 1H), 4.47 (t, *J* = 6.8Hz, 2H), 3.12 - 3.20 (m, 2H), 2.83-2.87 (m, 1H), 2.50 - 2.59 (m, 5H), 2.24 - 2.29 (m, 1H), 2.15 - 2.19 (m, 4H), 1.79 - 2.02 (m, 7H), 1.56 - 1.60 (m, 3H). Mass spec: m/z 702.4 $[M+H]^+$.

**Example 22 was synthesised following Scheme 21**

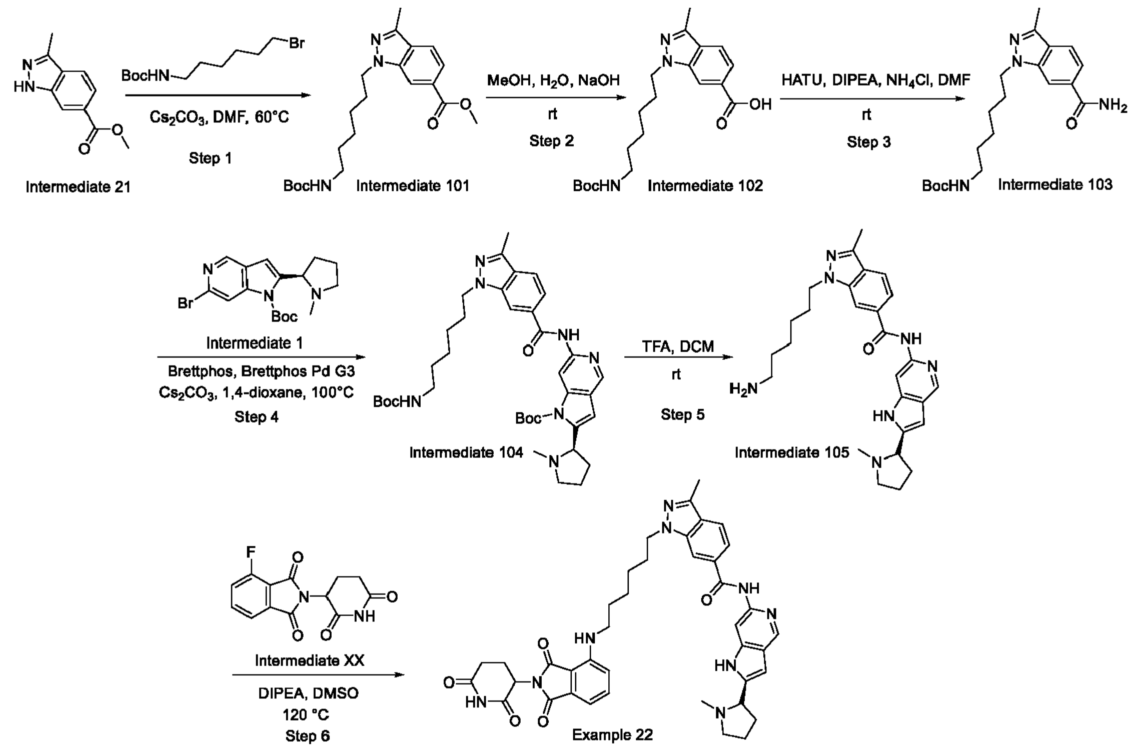


**Scheme 21**

**Step 1**

**Intermediate 101: methyl 1-{6-[(tert-butoxycarbonyl)amino]hexyl}-3-methylindazole-6-carboxylate**

To a solution of methyl 3-methyl-1H-indazole-6-carboxylate **(Intermediate 21,** 500 mg, 2.62 mmol) in DMF (20

mL) was added tert-butyl N-(6-bromohexyl)carbamate (CAS No: 142356-33-0, 810 mg, 2.89 mmol) and $Cs_2CO_3$ (2.6 g, 7.88 mmol) and the reaction was stirred overnight at 60 °C for 3 h. The reaction was quenched with water (300 mL) and extracted with EtOAc (3 × 300 mL). The combined organic layers were washed with brine, dried over anhydrous $Na_2SO_4$ and concentrated under reduced pressure. The residue was chromatographed on a silica gel column with EtOAc/petroleum ether (30/70) to afford methyl 1-{6-[(tert-butoxycarbonyl)amino]hexyl}-3-methylindazole-6-carboxylate **(Intermediate 101,** 800 mg, 75%) as a yellow solid. Mass spec: m/z: 390.0 $[M+H]^+$.

**Step 2**

**Intermediate 102: 1-{6-[(tert-butoxycarbonyl)amino]hexyl}-3-methylindazole-6-carboxylic acid**

To a solution of methyl 1-{6-[(tert-butoxycarbonyl)amino]hexyl}-3-methylindazole-6-carboxylate **(Intermediate 101,** 300 mg, 0.77 mmol) in MeOH (4 mL) was added $H_2O$ (4 mL) and NaOH (184 mg, 4.62 mmol) and the reaction was stirred at rt for 3h. The pH of the reaction mixture was then adjusted to pH ~5 by the drop-wise addition concentrated aqueous HCl. The resulting mixture was extracted with EtOAc (3 x 50 mL), the organic phases were dried over anhydrous sodium sulfate and concentrated under reduced pressure to afford 1-{6-[(tert-butoxycarbonyl)amino]hexyl}-3-methylindazole-6-carboxylic acid **(Intermediate 102,** 270 mg, 92%) as a yellow solid, which was used in the next step without further purification. Mass spec: m/z: 376.2 $[M+H]^+$.

**Step 3**

**Intermediate 103: tert-butyl N-[6-(6-carbamoyl-3-methylindazol-1-yl)hexyl]carbamate**

To a solution of 1-{6-[(tert-butoxycarbonyl)amino]hexyl}-3-methylindazole-6-carboxylic acid **(Intermediate 102,** 270 mg, 0.719 mmol) in DMF (8 mL) was added $NH_4Cl$ (153 mg, 2.87 mmol) and DIPEA (557 mg, 4.31 mmol), HATU (410 mg, 1.07 mmol) and the reaction was stirred overnight at rt. The mixture was then quenched with water (200 mL) and the aqueous phase extracted with EtOAc (3 × 200 mL). The combined organic layers were washed with brine, dried over anhydrous $Na_2SO_4$, and the volatiles were removed under reduced pressure. The crude was then chromatographed on a silica gel column with MeOH / DCM (3/97) to afford tert-butyl N-[6-(6-carbamoyl-3-methylindazol-1-yl)hexyl]carbamate **(Intermediate 103,** 240 mg, 88%) as a yellow solid. Mass spec: m/z: 375.2 $[M+H]^+$.

**Step 4**

**Intermediate 104: tert-butyl 6-(1-{6-[(tert-butoxycarbonyl)amino]hexyl}-3-methylindazole-6-amido)-2-[(2R)-1-methylpyrrolidin-2-yl]pyrrolo[3,2-c]pyridine-1-carboxylate**

To a solution of tert-butyl N-[6-(6-carbamoyl-3-methylindazol-1-yl)hexyl]carbamate **(Intermediate 103,** 200 mg, 0.53 mmol) in 1,4-dioxane (7 mL) was added tert-butyl 6-bromo-2-[(2R)-1-methylpyrrolidin-2-yl]pyrrolo[3,2-c]pyridine-1-carboxylate **(Intermediate 1,** 203 mg, 0.53 mmol), Brettphos (172 mg, 0.320 mmol), Brettphos Pd G3 (145 mg, 0.160 mmol) and $Cs_2CO_3$ (348 mg, 1.06 mmol) and the resulting mixture was stirred overnight at 100 °C under $N_2$. The reaction mixture was then purified on a silica gel column, eluting with MeOH / DCM (6/94), to afford tert-butyl 6-(1-{6-[(tert-butoxycarbonyl)amino]hexyl}-3-methylindazole-6-amido)-2-[(2R)-1-methylpyrrolidin-2-yl]pyrrolo[3,2-c]pyridine-1-carboxylate **(Intermediate 104,** 178 mg, 47%) as a yellow solid. Mass spec: m/z: 674.3 $[M+H]^+$.

**Step 5**

**Intermediate 105: 1-(6-aminohexyl)-3-methyl-N-{2-[(2R)-1-methylpyrrolidin-2-yl]-1H-pyrrolo[3,2-c]pyridin-6-yl} indazole-6-carboxamide ditrifluoroacetate**

To a solution of tert-butyl 6-(1-{6-[(tert-butoxycarbonyl)amino]hexyl}-3-methylindazole-6-amido)-2-[(2R)-1-methylpyrrolidin-2-yl]pyrrolo[3,2-c]pyridine-1-carboxylate **(Intermediate 104,** 178 mg, 0.251 mmol) in DCM (6 mL) was added TFA (3 mL) and the reaction mixture was stirred at rt for 2h. The volatiles were then removed under reduced pressure to afford 1-(6-aminohexyl)-3-methyl-N-{2-[(2R)-1-methylpyrrolidin-2-yl]-1H-pyrrolo[3,2-c]pyridin-6-yl}indazole-6-carboxamide ditrifluoroacetate **(Intermediate 105,** 125 mg, 94%) as a yellow oil, which was used in the next step without further purification. Mass spec m/z: 474.3 $[M+H]^+$.

**Step 6**

**Example 22: 1-(6-{[2-(2,6-dioxopiperidin-3-yl)-1,3-dioxoisoindol-4-yl]amino}hexyl)-3-methyl-N-{2-[(2R)-1-methylpyrrolidin-2-yl]-1H-pyrrolo[3,2-c]pyridin-6-yl}indazole-6-carboxamide**

To a solution of 1-(6-aminohexyl)-3-methyl-N-{2-[(2R)-1-methylpyrrolidin-2-yl]-1H-pyrrolo[3,2-c]pyridin-6-yl}indazole-6-carboxamide **(Intermediate 105,** 125 mg, 0.264 mmol) in DMSO (2 mL) was added 2-(2,6-dioxopiperidin-3-yl)-4-fluoroisoindole-1,3-dione (CAS No: 835616-60-9, 72.9 mg, 0.264 mmol) and DIPEA (102 mg, 0.792 mmol) and the reaction stirred overnight at 120 °C. The crude product was then purified by preparative HPLC (Column: XBridge Prep OBD C18 Column, 30*150 mm, 5μm; Mobile Phase A: Water (10 mmol/L $NH_4HCO_3$), Mobile Phase B: MeCN; Flow rate: 60 mL/min mL/min; Gradient: 38% B to 68 % B in 10 min) to afford 1-(6-{[2-(2,6-dioxopiperidin-3-yl)-1,3-dioxoisoindol-4-yl]amino}hexyl)-3-methyl-N-{2-[(2R)-1-methylpyrrolidin-2-yl]-1H-pyrrolo[3,2-c]pyridin-6-yl}indazole-6-carboxamide **(Example 22,** 5.7 mg, 3%) as a yellow solid. $^1$H NMR (400 MHz, DMSO-d6) δ ppm 8.55 (s, 1H), 8.22 (d, *J* = 9.2 Hz 2H), 7.84 (d, *J* = 8.4 Hz, 1H), 7.74 (d, *J* = 7.6 Hz 1H), 7.49 (t, *J* = 7.2 Hz 1H), 6.95 - 7.00 (m, 2H), 6.53 (s, 1H), 5.01 - 5.04 (m, 1H), 4.46 (t, *J* = 6.8 Hz, 2H), 3.44 - 3.45 (m, 1H), 3.32 - 3.33 (m, 3H), 2.70 - 2.74 (m, 3H), 2.59 (s, 3H), 2.40 - 2.43 (m, 1H), 2.32 (s, 4H), 2.04 - 2.09 (m, 3H), 1.94 - 1.99 (m, 3H), 1.61-1.65 (m, 2H), 1.45 - 1.49 (m, 2H), 1.30 - 1.38(m, 2H). Mass spec: m/z: 730.5 $[M+H]^+$.

**Example 23 was synthesized following Scheme 22**

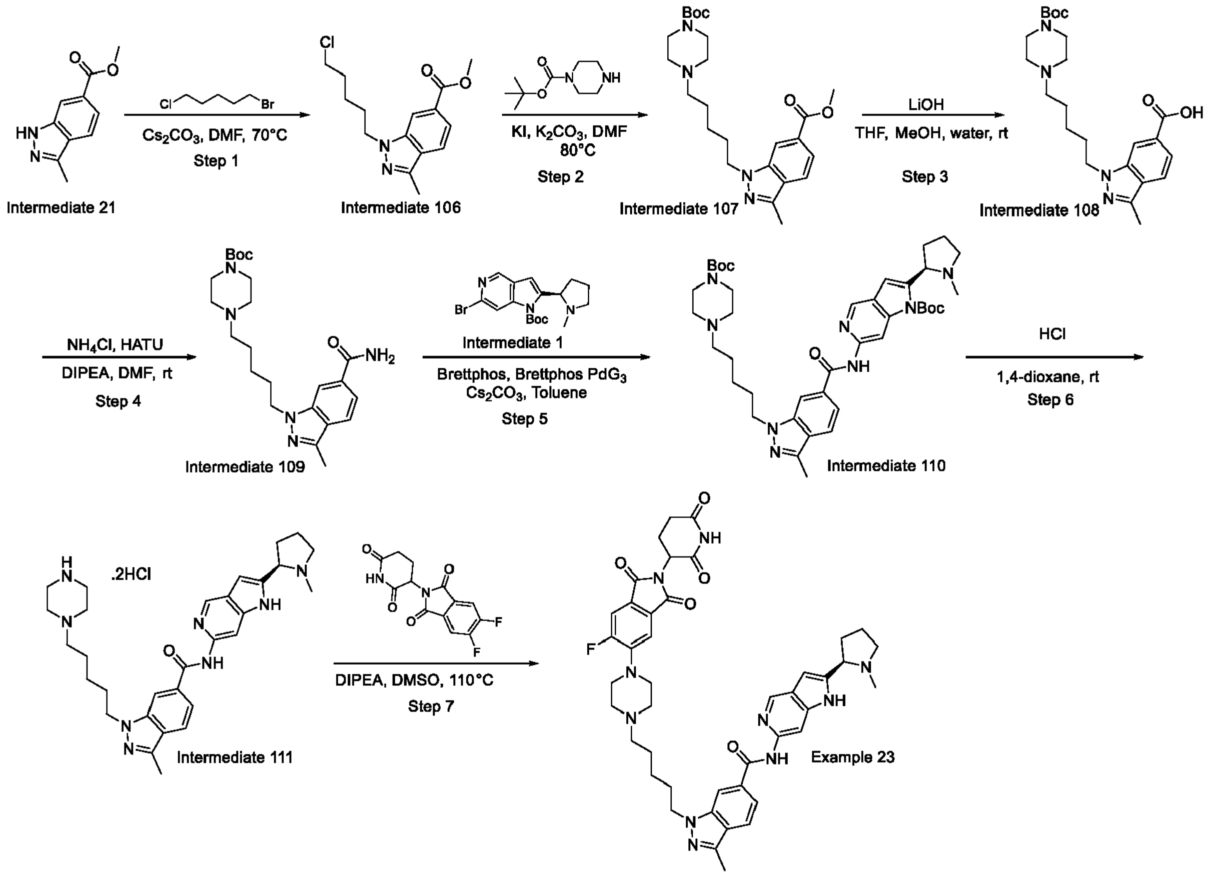


# Scheme 22

**Step 1**

**Intermediate 106: Methyl 1-(5-chloropentyl)-3-methyl-1H-indazole-6-carboxylate**

To a solution of methyl 3-methyl-1H-indazole-6-carboxylate **(Intermediate 21,** 3.0 g, 15.8 mmol) in DMF (30 mL) was added cesium carbonate (12.9 g, 39.4 mmol) followed by 1-bromo-5-chloropentane (CAS No: 54512-75-3, 5.97 g, 31.6 mmol). The reaction mixture was heated to 70 °C for 16h. The reaction mixture was cooled to room temperature, water

added (100 mL) and the mixture extracted with ethyl acetate (3 × 60 mL). The combined organic layers were dried, washed with brine solution (2 × 50 mL), dried over $Na_2SO_4$ and concentrated *in vacuo.* The obtained residue was purified by combi-flash column chromatography, by eluting with 10% ethyl acetate in heptane, to afford methyl 1-(5-chloropentyl)-3-methyl-1H-indazole-6-carboxylate **(Intermediate 106,** 3.2 g, 69%) as a yellow liquid. 1H NMR (400 MHz, DMSO-d6) δ ppm 1.32-1.41 (m, 2H), 1.69-1.87 (m, 4H), 2.51 (s, 3H), 3.59 (t, J = 6.47 Hz, 2H), 3.90 (s, 3H), 4.42 (t, J = 6.94 Hz, 2H), 7.65 (d, J = 8.32 Hz, 1H), 7.81 (d, J = 8.32 Hz, 1H), 8.25 (s, 1H). Mass spec: m/z 294.8 [M+H]+.

**Step 2**

**Intermediate 107: Methyl 1-(5-(4-(*tert*-butoxycarbonyl)piperazin-1-yl)pentyl)-3-methyl-1H-indazole-6-carboxylate**

To a solution of methyl 1-(5-chloropentyl)-3-methyl-1H-indazole-6-carboxylate **(Intermediate 106,** 3.3 g, 11 mmol) in dimethylformamide (35 mL) was added potassium carbonate (3.1 g, 22 mmol), potassium iodide (1.9 g, 11 mmol) and *tert*-butyl piperazine-1-carboxylate (CAS No: 57260-71-6, 2.6 g, 13 mmol). The reaction mixture was heated to 80°C for 16h. The reaction mixture cooled to room temperature, treated with water (100 mL) and extracted with EtOAc (3 × 100 mL). The organic layers were washed with brine solution (3 × 50 mL), dried over $Na_2SO_4$, and concentrated *in vacuo* to afford methyl 1-(5-(4-(*tert*-butoxycarbonyl)piperazin-1-yl)pentyl)-3-methyl-1H-indazole-6-carboxylate **(Intermediate 107,** 3.85 g, 77%) a yellow liquid. $^1$H NMR (400 MHz, DMSO-d6) δ ppm 1.20-1.22 (m, 2H), 1.36 (s, 9H) 1.37-1.45 (m, 4H), 1.76-1.86 (m, 2H), 2.15-2.27 (m, 2H), 2.51 (s, 3H), 2.58-2.63 (m, 1H), 3.23-3.25 (m, 5H), 3.90 (s, 3H), 4.40 (t, J = 6.80 Hz, 2H), 7.65 (d, J = 8.33 Hz, 1H), 7.81 (d, J = 8.33 Hz, 1H) 8.23 (s, 1H). Mass spec: m/z 445.47 $[M+H]^+$.

**Step 3**

**Intermediate 108: 1-(5-(4-(*tert*-butoxycarbonyl)piperazin-1-yl)pentyl)-3-methyl-1H-indazole-6-carboxylic acid**

To a solution of methyl 1-(5-(4-(*tert*-butoxycarbonyl)piperazin-1-yl)pentyl)-3-methyl-1H-indazole-6-carboxylate **(Intermediate 107,** 3.4 g, 7.6 mmol) in THF (12 mL) and MeOH (12 mL) was added LiOH (0.47 g, 19 mmol) in water (12 mL). The reaction mixture was stirred at room temperature for 16h then concentrated *in vacuo.* The aqueous layer pH was adjusted to 3-4 with 0.5 N HCl and extracted with ethyl acetate (2 × 50 mL). The combined organic layers were dried over $Na_2SO_4$ and concentrated *in vacuo* to afford 1-(5-(4-(*tert*-butoxycarbonyl)piperazin-1-yl)pentyl)-3-methyl-1H-indazole-6-carboxylic acid **(Intermediate 108,** 2.55 g, 77%) as an off-white solid which was used for the next step without further purification. $^1$H NMR (400 MHz, DMSO-d6) δ ppm 1.20.1.22 (m, 4H), 1.32 (s, 9H) 1.36-1.44 (m, 4H), 1.78-1.79 (m, 2H), 2.23 -2.25 (m, 6H) 2.50 (s, 3H) 4.37-4.38 (m, 2H), 7.62 (d, J = 8.32 Hz, 1H), 7.72-7.80 (m, 1H), 8.18 (s, 1H). Mass spec: m/z 431.0 $[M+H]^+$.

**Step 4**

**Intermediate 109: *tert*-Butyl 4-(5-(6-carbamoyl-3-methyl-1H-indazol-1-yl)pentyl)piperazine-1-carboxylate:**

To a solution of 1-(5-(4-(*tert*-butoxycarbonyl)piperazin-1-yl)pentyl)-3-methyl-1H-indazole-6-carboxylic acid **(Intermediate 108,** 1.5 g, 3.5 mmol) in dimethylformamide (15 mL) was added HATU (2.7 g, 7.0 mmol). The reaction mixture was stirred at room temperature for 15 min. Ammonium chloride (1.3 g, 24 mmol), followed by DIPEA (2.1 mL, 12 mmol) were then added and the mixture stirred at room temperature for 16h. The reaction mixture was diluted with water (100 mL) and extracted with ethyl acetate (3 × 50 mL). The combined organic layers were washed sequentially with $NaHCO_3$ (2 × 40 mL) and brine solution (2 × 30 mL), then dried over $Na_2SO_4$ and concentrated *in vacuo.* The crude solid was washed with DCM and n-pentane to afford *tert*-butyl 4-(5-(6-carbamoyl-3-methyl-1H-indazol-1-yl)pentyl)piperazine-1-carboxylate **(Intermediate 109,** 0.94 g, 2.188 mmol, 63%) as an off-white solid which was used for the next step without further purification. Mass spec: m/z 430.5 $[M+H]^+$.

**Step 5**

**Intermediate 110: tert-Butyl (R)-6-(1-(5-(4-(tert-butoxycarbonyl)piperazin-1-yl)pentyl)-3-methyl-1H-indazole-6-carboxamido)-2-(1-methylpyrrolidin-2-yl)-1H-pyrrolo[3,2-c]pyridine-1-carboxylate**

To a solution of *tert*-butyl 4-(5-(6-carbamoyl-3-methyl-1H-indazol-1-yl)pentyl)piperazine-1-carboxylate **(Intermediate 109,** 0.38 g, 0.89 mmol) in toluene (5 mL) was added tert-butyl 6-bromo-2-[(2R)-1-methylpyrrolidin-2-yl]pyrrolo [3,2-c]pyridine-1-carboxylate **(Intermediate 1,** 0.40 g, 1.06 mmol) followed by $Cs_2CO_3$ (0.865 g, 2.65 mmol). The reaction

mixture was purged with argon for 10 min, then BrettPhos (0.073 g, 0.13 mmol) and BrettPhos Pd G3 (0.12 g, 0.13 mmol) were added. The reaction was purged with argon for 10 min and heated to 100°C for 2h. The reaction mixture was then cooled to room temperature, treated with water (50 mL) and extracted with ethyl acetate (3 × 50 mL). The combined organic layers were dried over $Na_2SO_4$ and concentrated *in vacuo.* The resulting residue was purified by combi-flash column chromatography, by eluting with 1.5% MeOH in DCM, to afford *tert*-butyl (R)-6-(1-(5-(4-(*tert*-butoxycarbonyl) piperazin-1-yl)pentyl)-3-methyl-1H-indazole-6-carboxamido)-2-(1-methylpyrrolidin-2-yl)-1H-pyrrolo[3,2-c]pyridine-1-carboxylate **(Intermediate 110,** 0.32 g, 50%) as an off-white solid. $^1$H NMR (400 MHz, DMSO-d6) δ ppm 1.19-1.29 (m, 3H), 1.35 (s, 9H), 1.42-1.49 (m, 2H), 1.60-1.63 (m, 1H), 1.71 (s, 9H), 1.86-1.88 (m, 3H), 2.16-2.27 (m, 5H), 2.39 (s, 3H) 2.40-2.41 (m, 3H), 2.52 (s, 3H), 3.10-3.17 (m, 1H), 3.20-3.22 (m, 4H), 3.89-3.97 (m, 1H), 4.41 (t, J = 6.80 Hz, 2H), 6.77 (s, 1H), 7.71-7.82 (m, 2H), 8.45 (s, 1H), 8.62 (s, 1H), 8.99 (s, 1H), 10.77 (s, 1H). Mass spec: m/z 729.4 $[M+H]^+$.

**Step 6**

**Intermediate 111: (R)-3-methyl-N-(2-(1-methylpyrrolidin-2-yl)-1H-pyrrolo[3,2-c]pyridin-6-yl)-1-(5-(piperazin-1-yl) pentyl)-1H-indazole-6-carboxamide dihydrochloride**

To a cooled (0°C) solution of *tert*-butyl (R)-6-(1-(5-(4-(tert-butoxycarbonyl)piperazin-1-yl)pentyl)-3-methyl-1H-indazole-6-carboxamido)-2-(1-methylpyrrolidin-2-yl)-1H-pyrrolo[3,2-c]pyridine-1-carboxylate **(Intermediate 110,** 0.3 g, 0.41 mmol) in 1,4-dioxane (2 mL) was added 4M HCl in 1,4-dioxane (5 mL) and the reaction mixture was stirred at room temperature for 16h then concentrated *in vacuo.* The resultant precipitate was collected by filtration, washed with dichloromethane (2 × 20 mL) and dried *in vacuo* to afford (R)-3-methyl-N-(2-(1-methylpyrrolidin-2-yl)-1H-pyrrolo[3,2-c]pyridin-6-yl)-1-(5-(piperazin-1-yl)pentyl)-1H-indazole-6-carboxamide dihydrochloride **(Intermediate 111,** 0.215 g, 99%) as a white solid, which was used for next step without further purification. $^1$H NMR (400 MHz, DMSO-d6) δ ppm 1.28-1.39 (m, 2H), 1.75-1.77 (m, 2H), 1.88-1.97 (m, 2H), 2.16-2.24 (m, 2H), 2.55 (s, 3H), 2.82 (s, 3H), 3.06-3.16 (m, 2H), 3.33-3.54 (m, 8H), 3.58-3.81 (m, 5H), 4.48 (t, J = 6.36 Hz, 2H), 7.29 (s, 1H), 7.82 (d, J = 8.31 Hz, 1H), 7.88-7.93 (m, 1H), 8.45 (s, 1H), 8.87 (s, 1H), 9.12 (s, 1H), 9.71 (s, 1H), 9.70 (s, 1H), 11.70 (s, 2H). Mass spec: m/z 529.3 $[M+H]^+$.

**Step 7**

**Example 23: 1-(5-(4-(2-(2,6-dioxopiperidin-3-yl)-6-fluoro-1,3-dioxoisoindolin-5-yl)piperazin-1-yl)pentyl)-3-methyl-N-(2-((R)-1-methylpyrrolidin-2-yl)-1H-pyrrolo[3,2-c]pyridin-6-yl)-1H-indazole-6-carboxamide**

To a solution of (R)-3-methyl-N-(2-(1-methylpyrrolidin-2-yl)-1H-pyrrolo[3,2-c]pyridin-6-yl)-1-(5-(piperazin-1-yl) pentyl)-1H-indazole-6-carboxamide **(Intermediate 111,** 0.215 g, 0.4067 mmol) in DMSO (3 mL) was added 2-(2,6-dioxopiperidin-3-yl)-5,6-difluoroisoindoline-1,3-dione (CAS No: 1496997-41-1: 0.13 g, 0.45 mmol) followed by DIPEA (0.21 mL, 1.22 mmol) and the reaction mixture was heated at 110°C for 6h. The reaction mixture was then cooled to room temperature and diluted with water (50 mL). The resultant precipitate was collected by filtration, washed with DCM/Pentane (2 × 20 mL) and then dried *in vacuo.* The crude compound was purified by preparative HPLC (Method A) to afford 1-(5-(4-(2-(2,6-dioxopiperidin-3-yl)-6-fluoro-1,3-dioxoisoindolin-5-yl)piperazin-1-yl)pentyl)-3-methyl-N-(2-((R)-1-methylpyrrolidin-2-yl)-1H-pyrrolo[3,2-c]pyridin-6-yl)-1H-indazole-6-carboxamide **(Example 23**, 0.085 g, 26%) as a yellow solid. $^1$H NMR (400 MHz, DMSO-d6) δ ppm 1.24-1.33 (m, 2H), 1.46-1.55 (m, 2H), 1.77-1.94 (m, 5H), 2.00-2.08 (m, 2H), 2.16 (s, 3H), 2.23-2.32 (m, 3H), 2.44-2.48 (m, 4H), 2.52 (s, 3H), 2.54-2.59 (m, 1H), 2.60-2.69 (m, 1H), 2.86-2.90 (m, 1H), 3.16-3.17 (s, 4H), 3.26-3.29 (m, 2H), 4.43-4.46 (m, 2H), 5.10-5.13 (m 1H), 6.39-6-41 (m, 1H), 7.39 (d, J = 7.38 Hz, 1H), 7.67 (d, J = 11.38 Hz, 1H), 7.72-7.75 (m, 1H), 7.78-7.81 (m, 1H), 8.24 (s, 1H), 8.46 (s, 1H), 8.51 (s, 1H), 10.53 (s, 1H), 11.00-11.17 (m, 1H), 11.36 (s, 1H). Mass spec: m/z 803.3 $[M+H]^+$.

**Example 24 was synthesized following Scheme 23**

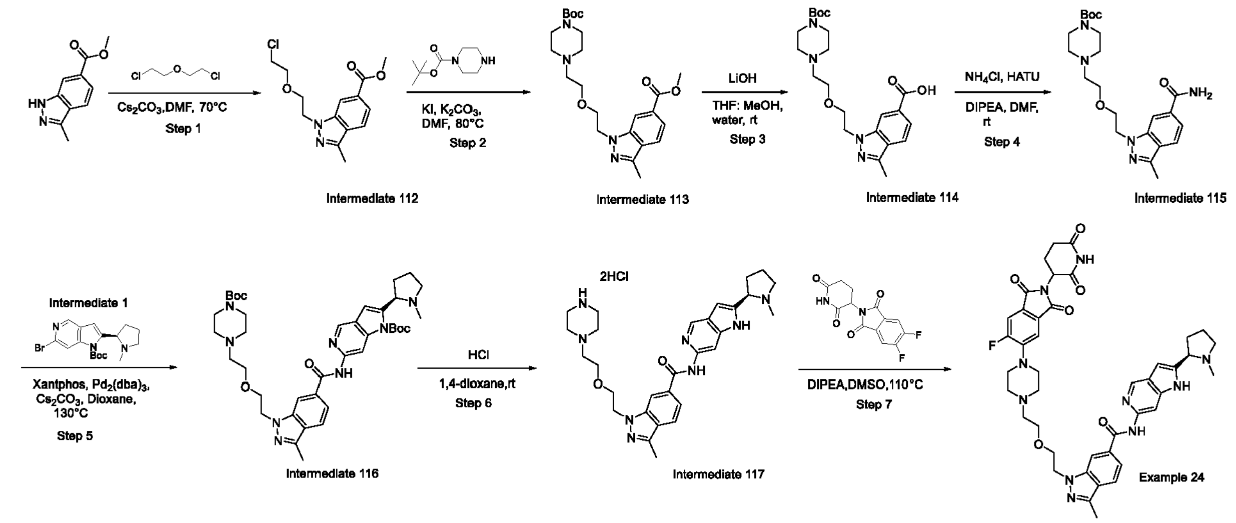


- **Scheme 23**

**Step** 1

**Intermediate 112: Methyl 1-(2-(2-chloroethoxy) ethyl)-3-methyl-1*H*-indazole-6-carboxylate**

To a solution of methyl 3-methyl-1*H*-indazole-6-carboxylate **(Intermediate 21,** 3.0 g, 15.73 mmol) in DMF (20 mL) was added cesium carbonate (12.9 g, 39.43 mmol) followed by 1-chloro-2-(2-chloroethoxy)ethane (CAS No: 111-44-4, 4.55 g, 31.54 mmol) and the reaction mixture was heated at 70°C for 16h. The reaction mixture was diluted with water (200 mL) and extracted with ethyl acetate (3 × 200 mL). The combined organic layers were separated, dried over anhydrous $Na_2SO_4$ and concentrated *in vacuo* to obtain crude compound as a mixture of regioisomers. The regioisomers were separated by combi-flash column chromatography, by eluting with 30% EtOAc in heptane, to afford desired regioisomer methyl 1-(2-(2-chloroethoxy) ethyl)-3-methyl-1*H*-indazole-6-carboxylate **(Intermediate 112,** 2.6 g, 56%) as an off-white solid. $^1$H NMR (400 MHz, DMSO-$d_6$) δ ppm 2.51 (s, 3H), 3.57-3.61 (m, 4H), 3.84-3.87 (t, *J*=5.04 Hz, 2H), 3.89 (s, 3H), 4.58 (t, *J*=5.26 Hz, 2H), 7.64 (d, *J*=8.33 Hz, 1H), 7.79 (d, *J*=8.33 Hz, 1H), 8.27 (s, 1H).

**Step 2**

**Intermediate 113: methyl 1-(2-(2-(4-(*tert*-butoxycarbonyl) piperazin-1-yl)ethoxy)ethyl)-3-methyl-1*H*-indazole-6-carboxylate**

To a solution of methyl 1-[2-(2-chloroethoxy)ethyl]-3-methyl-indazole-6-carboxylate **(Intermediate 112,** 2.4 g, 8.1 mmol) in dimethylformamide (25 mL) was added potassium carbonate (1.7 g, 12 mmol) followed by potassium iodide (1.3 g, 8.1 mmol) and tert-butyl piperazine-1-carboxylate (CAS No: 57260-71-6, 2.0 g, 9.7 mmol). The reaction mixture was heated at 80°C for 12h then diluted with water (200 mL) and extracted with ethyl acetate (3 × 200 mL). The combined organic layers were separated, dried over anhydrous $Na_2SO_4$ and concentrated *in vacuo.* The crude material was purified by combi-flash column chromatography, by eluting with 10% ethyl acetate in heptane, to afford methyl 1-(2-(2-(4-(*tert*-butoxycarbonyl) piperazin-1-yl)ethoxy)ethyl)-3-methyl-1*H*-indazole-6-carboxylate **(Intermediate 113,** 2.2 g, 61%) as a white solid. $^1$H NMR (400 MHz, DMSO-$d_6$) δ ppm 1.38 (s, 9H), 2.02-2.12 (m, 4H), 2.30 (t, *J*=5.14 Hz, 2H), 2.51 (s, 3H), 3.04-3.11 (m, 4H), 3.42 (t, *J*=5.14 Hz, 2H), 3.75 (t, *J*=4.65 Hz, 2H), 3.90 (s, 3H), 4.57 (t, *J*=4.65 Hz, 2H), 7.65 (d, *J*=8.31 Hz, 1H), 7.79 (d, *J*=8.31 Hz, 1H), 8.25 (s, 1H). Mass spec: m/z 447.8 $[M+2]^+$.

**Step 3**

**Intermediate 114: 1-(2-(2-(4-(*tert*-butoxycarbonyl)piperazin-1-yl)ethoxy)ethyl)-3-methyl-1*H*-indazole-6-carboxylic acid**

To a cooled (0°C) solution of methyl 1-(2-(2-(4-(*tert*-butoxycarbonyl)piperazin-1-yl)ethoxy)ethyl)-3-methyl-1*H*-indazole-6-carboxylate **(Intermediate 113,** 2.2 g, 4.5 mmol) in tetrahydrofuran (10 mL) and methanol (5 mL) was added lithium hydroxide (0.33 g, 14 mmol) in water (2 mL). The reaction mixture was stirred at room temperature for 24h then

concentrated *in vacuo.* The residue was diluted with water (50 mL) and extracted with ethyl acetate (3 × 50 mL). The combined organic layers were separated, dried over anhydrous $Na_2SO_4$ and concentrated *in vacuo.* The crude material was purified by combi-flash column chromatography, by eluting with 50% ethyl acetate in heptane, to afford 1-(2-(2-(4-(*tert*-butoxycarbonyl) piperazin-1-yl) ethoxy) ethyl)-3-methyl-1*H*-indazole-6-carboxylic acid **(Intermediate 114,** 1.7 g, 85%) as a yellow liquid. Mass spec: m/z 433.8 $[M+H]^+$.

**Step 4**

**Intermediate 115: *tert*-butyl 4-(2-(2-(6-carbamoyl-3-methyl-1*H*-indazol-1-yl) ethoxy) ethyl) piperazine-1-carboxylate**

To a solution of 1-[2-[2-(4-*tert*-butoxycarbonylpiperazin-1-yl) ethoxy]ethyl]-3-methylindazole-6-carboxylic acid **(Intermediate 114,** 1.00 g, 2.31 mmol) in dimethylformamide (10 mL) was added HATU (1.36 g, 3.468 mmol) and DIPEA (1.21 mL, 6.936 mmol). The reaction mixture was stirred at room temperature for 15 min and then ammonium chloride (0.62 g, 11.56 mmol) was added, and the reaction stirred at room temperature for 16h. The reaction mixture was quenched with water (50 mL) and extracted with ethyl acetate (3 × 50 mL). The combined organic layers were separated, dried over anhydrous $Na_2SO_4$ and concentrated *in vacuo.* The crude material was purified by combi-flash column chromatography, by eluting with 10% ethyl acetate in heptane, to afford *tert*-butyl 4-(2-(2-(6-carbamoyl-3-methyl-1*H*-indazol-1-yl)ethoxy) ethyl)piperazine-1-carboxylate **(Intermediate 115,** 0.80 g, 80%) as a yellow solid. $^1H$ NMR (400 MHz, DMSO-$d_6$) δ ppm 1.37 (s, 9H), 2.02-2.12 (m, 4H), 2.33 (t, *J*=5.37 Hz, 2H), 2.87 (s, 3H), 3.07-3.11 (m, 4H), 3.43 (t, *J*=5.48 Hz, 2H), 3.77 (t, *J*=5.26 Hz, 2H), 4.48 (t, *J*=5.26 Hz, 2H), 7.58 (d, *J*=8.44 Hz, 1H), 7.70 (d, *J*=8.33 Hz, 1H), 7.93 (s, 1H), 7.98 (br s, 1H), 8.13 (s, 1H).

**Step 5**

**Intermediate 116: *tert*-butyl (R)-6-(1-(2-(2-(4-(tert-butoxycarbonyl) piperazin-1-yl)ethoxy)ethyl)-3-methyl-1*H*-indazole-6-carboxamido)-2-(1-methylpyrrolidin-2-yl)-1*H*-pyrrolo[3,2-c]pyridine-1-carboxylate**

To a stirred solution of *tert*-butyl 6-bromo-2-[(2R)-1-methylpyrrolidin-2-yl]pyrrolo[3,2-c]pyridine-1-carboxylate **(Intermediate 1,** 0.40 g, 1.05 mmol) in toluene (10 mL) was added *tert*-butyl 4-[2-[2-(6-carbamoyl-3-methyl-indazol-1-yl) ethoxy]ethyl]piperazine-1-carboxylate **(Intermediate 115,** 0.36 g, 0.84 mmol) followed by cesium carbonate (1.02 g, 3.15 mmol). The reaction mixture was purged with argon for 15 min, then BrettPhos (0.05 g, 0.10 mmol) followed by BrettPhos Pd G3 (0.09 g, 0.10 mmol) were added and the reaction mixture was purged with argon for 10 min then heated at 130°C for 3h. The reaction mixture was diluted with water (50 mL) and extracted with EtOAc (3 × 60 mL). The combined organic layers were separated, dried over anhydrous $Na_2SO_4$ and concentrated *in vacuo.* The crude material was purified by combi-flash column chromatography, by eluting with 80% EtOAc in heptane, to afford *tert*-butyl (R)-6-(1-(2-(2-(4-(*tert*-butoxycarbonyl)piperazin-1-yl)ethoxy)ethyl)-3-methyl-1*H*-indazole-6-carboxamido)-2-(1-methylpyrrolidin-2-yl)-1*H*-pyrrolo[3,2-c]pyridine-1-carboxylate **(Intermediate 116,** 0.30 g, 39%) as an off white solid. Mass spec: m/z 731.3 $[M+2]^+$.

**Step 6**

**Intermediate 117: (*R*)-3-methyl-*N*-(2-(1-methylpyrrolidin-2-yl)-1*H*-pyrrolo[3,2-c]pyridin-6-yl)-1-(2-(2-(piperazin-1-yl)ethoxy)ethyl)-1*H*-indazole-6-carboxamide dihydrochloride**

To a cooled (0°C) solution of *tert*-butyl 6-[[1-[2-[2-(4-*tert*-butoxycarbonylpiperazin-1-yl)ethoxy]ethyl]-3-methylindazole-6-carbonyl]amino]-2-[(2R)-1-methylpyrrolidin-2-yl]pyrrolo[3,2-c]pyridine-1-carboxylate **(Intermediate 116,** 0.29 g, 0.39 mmol) in 1,4-dioxane (7 mL) was added 4M HCl in 1,4-dioxane (7 mL) and the reaction mixture was stirred at 0°C for 15 min then at room temperature for 16h. The reaction mixture was concentrated *in vacuo* to afford (*R*)-3-methyl-*N*-(2-(1-methylpyrrolidin-2-yl)-1*H*-pyrrolo [3, 2-c] pyridin-6-yl)-1-(2-(2-(piperazin-1-yl) ethoxy) ethyl)-1*H*-indazole-6-carboxamide dihydrochloride **(Intermediate 117,** 0.25 g crude) as a yellow solid which was used for the next step without further purification. $^1H$ NMR (400 MHz, DMSO-$d_6$) δ ppm 2.12-2.28 (m, 2H), 2.33-2.44 (m, 2H), 2.53 (s, 3H), 2.54-2.57 (m, 1H), 2.82 (s, 3H), 3.17-3.19 (m, 1H), 3.22-3.34 (m, 5H), 3.35-3.45 (m, 5H), 3.82-3.84 (m, 2H), 3.94 (t, *J*=5.15 Hz, 2H), 4.63-4.79 (m, 3H), 7.29 (s, 1H), 7.82-7.87 (m, 1H), 7.89-7.94 (m, 1H), 8.42 (s, 1H), 8.89 (s, 1H), 9.13 (s, 1H),9.63 (br s, 2H), 11.34 (br s, 1H), 12.27 (br s, 1H), 13.25 (br s, 1H). Mass spec: m/z 531.0 $[M+H]^+$.

**Step 7**

**Example 24: 1-(2-(2-(4-(2-(2,6-dioxopiperidin-3-yl)-6-fluoro-1,3-dioxoisoindolin-5-yl)piperazin-1-yl)ethoxy) ethyl)-3-methyl-*N*-(2-((*R*)-1-methylpyrrolidin-2-yl)-1*H*-pyrrolo[3,2-c]pyridin-6-yl)-1*H*-indazole-6-carboxamide**

To a solution of 3-methyl-*N*-[2-[(2*R*)-1-methylpyrrolidin-2-yl]-1*H*-pyrrolo[3,2-c]pyridin-6-yl]-1-[2-(2-piperazin-1-ylethoxy)ethyl]indazole-6-carboxamide dihydrochloride **(Intermediate 117,** 0.25 g, 0.47 mmol) and 2-(2,6-dioxo-3-piperidyl)-5,6-difluoro-isoindoline-1,3-dione (CAS No: 1496997-41-1, 0.14 g, 0.47 mmol) in dimethyl sulfoxide (4 mL) was added DIPEA (0.41 mL, 2.35 mmol) and the reaction mixture was heated at 110°C for 8h. The reaction mixture was quenched with ice cold water (15 mL), the resultant yellow precipitate was collected by filtration and dried *in vacuo.* The crude material was purified by preparative HPLC (Method A) afford 1-(2-(2-(4-(2-(2,6-dioxopiperidin-3-yl)-6-fluoro-1,3-dioxoisoindolin-5-yl)piperazin-1-yl)ethoxy)ethyl)-3-methyl-*N*-(2-((*R*)-1-methylpyrrolidin-2-yl)-1*H*-pyrrolo[3,2-c]pyridin-6-yl)-1*H*-indazole-6-carboxamide **(Example 24,** 0.06 g, 16%) as a yellow solid. $^{1}H$ NMR (400 MHz, DMSO-$d_6$) δ ppm 1.80-1.88 (m, 3H), 2.02-2.05 (m, 1H), 2.12-2.14 (m, 1H), 2.15 (s, 3H), 2.24-2.27 (m, 1H), 2.38-2.43 (m, 6H), 2.52 (s, 3H), 2.55-2.62 (m, 1H), 2.85-2.89 (m, 1H), 2.98-3.05 (m, 4H), 3.11-3.17 (m, 1H), 3.25-3.29 (m, 2H), 3.52 (t, *J*=5.32 Hz, 2H), 3.84 (t, *J*=5.19 Hz, 2H), 4.60 (t, *J*=5.25 Hz, 2H), 5.08-5.13 (m, 1H), 6.36-6.37 (m, 1H), 7.29 (d, *J*=7.25 Hz, 1H), 7.59-7.60 (m, 1H), 7.71-7.80 (m, 2H), 8.23 (s, 1H), 8.48-8.49 (m, 2H), 10.47 (s, 1H), 11.10 (br s, 1H), 11.32 (br s, 1H). Mass spec: m/z 805.3 $[M+H]^+$.

**Example 25 was synthesised following the Scheme 24**

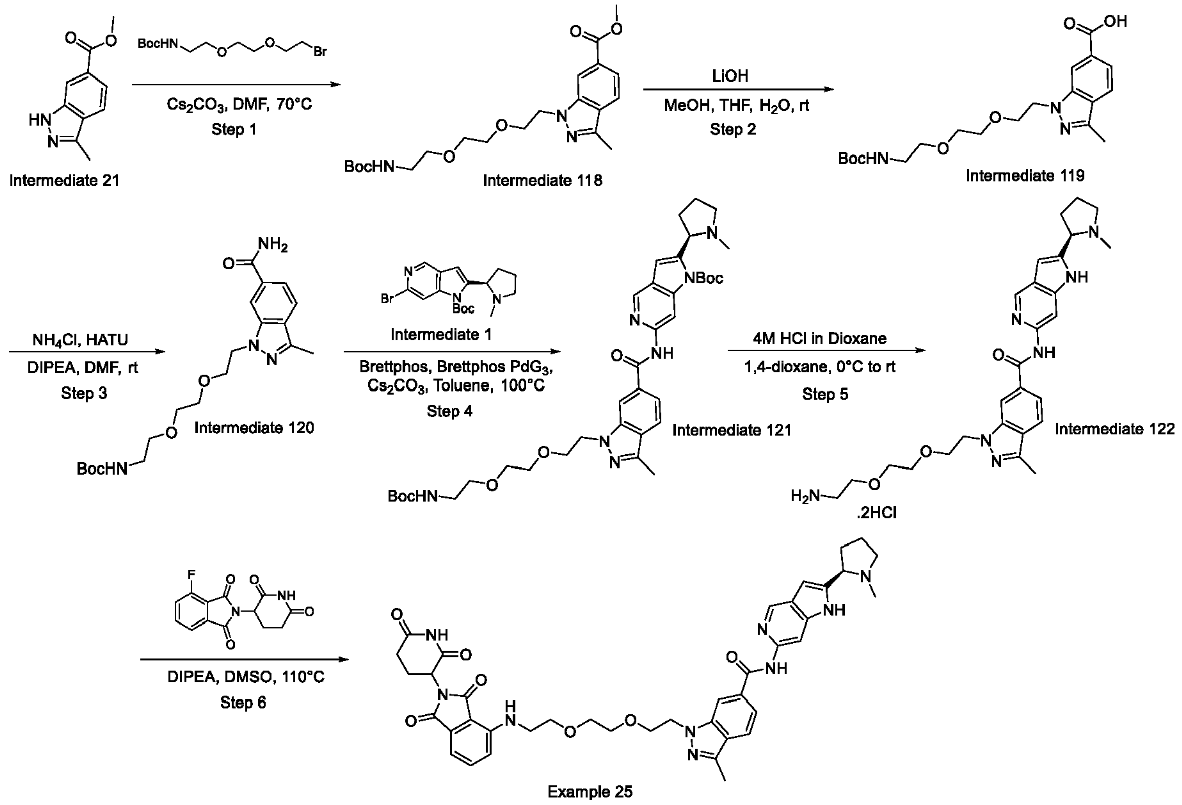


Scheme 24

**Step 1**

**Intermediate 118: Methyl 1-(2,2-dimethyl-4-oxo-3,8,11-trioxa-5-azatridecan-13-yl)-3-methyl-1*H*-indazole-6-carboxylate**

To a solution of methyl 3-methyl-1*H*-indazole-6-carboxylate **(Intermediate 21,** 1 g, 5.26 mmol) in DMF (10 mL) was added cesium carbonate (4.29 g, 13.14 mmol) followed by *tert*-butyl (2-(2-(2-bromoethoxy)ethoxy)ethyl)carbamate (CAS No: 1076199-21-7, 2.51 g, 7.89 mmol) and the reaction mixture was stirred at 70°C for 16h. The mixture was then

cooled to room temperature, treated with water (50 mL) and extracted with ethyl acetate (3 × 50 mL). The combined organic layers were washed with brine solution (2 × 50 mL), dried over $Na_2SO_4$ and concentrated *in vacuo.* The resulting residue was purified by combi-flash column chromatography, by eluting with 45% ethyl acetate in heptane, to afford methyl 1-(2,2-dimethyl-4-oxo-3,8,11-trioxa-5-azatridecan-13-yl)-3-methyl-1*H*-indazole-6-carboxylate **(Intermediate 118,** 1.2 g, 54%) as a colourless liquid. $^1$H NMR (400 MHz, DMSO-$d_6$) δ ppm 1.35 (s, 9H), 2.51 (s, 3H), 2.96 (q, *J* = 5.46 Hz, 2H), 3.25 (t, *J* = 6.18 Hz, 2H), 3.35-3.40 (m, 2H), 3.42-3.46 (m, 2H), 3.80 (t, *J* = 5.24 Hz, 2H), 3.90 (s, 3H), 4.56 (t, *J* = 5.24 Hz, 2H), 6.66 (s, 1H), 7.65 (d, *J* = 8.33 Hz, 1H), 7.80 (d, *J* = 8.60 Hz, 1H), 8.26 (s, 1H). Mass spec: m/z 421.9 $[M+H]^+$.

**Step 2**

**Intermediate 119: 1-(2,2-dimethyl-4-oxo-3,8,11-trioxa-5-azatridecan-13-yl)-3-methyl-1H-indazole-6-carboxylic acid**

To a solution of methyl 1-(2,2-dimethyl-4-oxo-3,8,11-trioxa-5-azatridecan-13-yl)-3-methyl-1*H*-indazole-6-carboxylate **(Intermediate 118,** 1.2 g, 2.85 mmol) in THF (4 mL) and MeOH (4 mL) was added lithium hydroxide (0.174 g, 7.12 mmol) in water (4 mL) and the reaction mixture was stirred at room temperature for 16h. Additional water (10 mL) was then added, and the pH of the solution was adjusted to 3-4 with 0.5 N HCl and extracted with EtOAc (2 × 50 mL). The combined organic layers were dried over $Na_2SO_4$ and concentrated *in vacuo.* The resultant solid was washed with n-pentanes (2 × 30 mL) to afford 1-(2,2-dimethyl-4-oxo-3,8,11-trioxa-5-azatridecan-13-yl)-3-methyl-1*H*-indazole-6-carboxylic acid **(Intermediate 119,** 0.95 g, 82%) as an off-white solid which was used for the next step without further purification. $^1$H NMR (400 MHz, DMSO-$d_6$) δ ppm 1.35 (s, 9H), 2.97 (s, 3H), 3.26-3.30 (m, 4H), 3.38 (d, *J* = 4.62 Hz, 2H), 3.42-3.47 (m, 2H), 3.80 (t, *J* = 5.09 Hz, 2H), 4.55 (t, *J* = 5.09 Hz, 2H), 6.66 (s, 1H), 7.64 (d, *J* = 8.32 Hz, 1H), 7.77 (d, *J* = 8.32 Hz, 1H), 8.22 (s, 1H), 12.97 (s, 1H). Mass spec: m/z 407.9 $[M+H]^+$.

**Step 3**

**Intermediate 120: *tert*-butyl (2-(2-(2-(6-carbamoyl-3-methyl-1*H*-indazol-1-yl)ethoxy)ethoxy)ethyl)carbamate**

To a solution of 1-(2,2-dimethyl-4-oxo-3,8,11-trioxa-5-azatridecan-13-yl)-3-methyl-1*H*-indazole-6-carboxylic acid **(Intermediate 119,** 0.93 g, 2.28 mmol) in dimethylformamide (10 mL) was added HATU (1.79 g, 4.56 mmol) and the reaction mixture was stirred at room temperature for 15 min. Ammonium chloride (0.856 g, 15.98 mmol) followed by DIPEA (1.043 g, 7.988 mmol) were then added and the reaction mixture stirred at room temperature for 16h. The reaction mixture was then treated with water (100 mL) and extracted with ethyl acetate (3 × 50 mL). The combined organic layers were washed sequentially with saturated aqueous $NaHCO_3$ solution (2 × 40 mL), brine solution (2 × 30 mL), dried over anhydrous $Na_2SO_4$ and concentrated *in vacuo.* The resultant crude solid was washed with DCM (10 mL) and n-pentane (10 mL), then dried *in vacuo* to afford *tert*-butyl (2-(2-(2-(6-carbamoyl-3-methyl-1*H*-indazol-1-yl)ethoxy)ethoxy)ethyl) carbamate **(Intermediate 120,** 0.82 g, 88%) as an off-white solid which was used for the next step without further purification. $^1$H NMR (400 MHz, DMSO-$d_6$) 1.35 (s, 9H), 2.98 (d, *J* = 6.01 Hz, 2H), 3.28 (t, *J* = 6.01 Hz, 2H), 3.30 (s, 3H) 3.40 (d, *J* = 5.09 Hz, 2H), 3.44-3.49 (m, 2H), 3.83 (t, *J* = 5.55 Hz, 2H), 4.50 (t, *J* = 5.55 Hz, 2H), 6.68 (s, 1H), 7.40 (s, 1H), 7.60 (d, *J* = 8.32 Hz, 1H), 7.72 (d, *J* = 8.32 Hz, 1H), 8.00 (s, 1H), 8.15 (s, 1H). Mass spec: m/z 406.9 $[M+H]^+$.

**Step 4**

**Intermediate 121: *tert*-butyl (R)-6-(1-(2,2-dimethyl-4-oxo-3,8,11-trioxa-5-azatridecan-13-yl)-3-methyl-1*H*-indazole-6-carboxamido)-2-(1-methylpyrrolidin-2-yl)-1*H*-pyrrolo[3,2-c]pyridine-1-carboxylate**

To a solution of *tert*-butyl (2-(2-(2-(6-carbamoyl-3-methyl-1*H*-indazol-1-yl)ethoxy)ethoxy)ethyl)carbamate **(Intermediate 120,** 0.32 g, 0.79 mmol) in toluene (10 mL) was added *tert*-butyl (R)-6-bromo-2-(1-methylpyrrolidin-2-yl)-1*H*-pyrrolo[3,2-c]pyridine-1-carboxylate **(Intermediate 1,** 0.15 g, 0.39 mmol) followed by cesium carbonate (0.39 g, 1.18 mmol). The reaction mixture was purged with argon for 10 min then BrettPhos (0.044 g, 0.079 mmol) and BrettPhos Pd G3 (0.073 g, 0.079 mmol) were added. The mixture was further purged with argon gas 10 min and then heated to 100°C for 2h. The reaction mixture was then cooled to room temperature, diluted with water (50 mL) and extracted with ethyl acetate (3 × 50 mL). The combined organic layers were dried over $Na_2SO_4$ and concentrated *in vacuo.* The resulting residue was purified by combi-flash column chromatography, by eluting with 1.5% MeOH in DCM) to afford *tert*-butyl (*R*)-6-(1-(2,2-dimethyl-4-oxo-3,8,11-trioxa-5-azatridecan-13-yl)-3-methyl-1H-indazole-6-carboxamido)-2-(1-methylpyrrolidin-2-yl)-1*H*-pyrrolo[3,2-c]pyridine-1-carboxylate **(Intermediate 121,** 0.30 g, 54%) as an off-white solid. $^1$H NMR (400 MHz, DMSO-$d_6$) δ ppm 1.30-1.34 (m, 2H), 1.37 (s, 9H), 1.69-1.70 (m, 2H), 1.71 (s, 9H), 2.31-2.40 (m, 2H), 2.52 (s, 3H), 2.93-3.01 (m, 2H), 3.27 (s, 3H), 3.37-3.43 (m, 2H), 3.44-3.52 (m, 2H), 3.80-3.97 (m, 3H), 4.50 (t, *J* = 5.59 Hz, 1H), 4.57 (t, *J* = 5.48 Hz,

1H), 6.76 (s, 1H), 7.77-7.81 (m, 1H), 8.01 (s, 1H), 8.15 (s, 1H), 8.46 (s, 1H), 8.61 (s, 1H), 8.98 (s, 1H), 10.73 (s, 1H). Mass spec: m/z 706 [M+H]+.

**Step 5**

**Intermediate 122: (*R*)-1-(2-(2-(2-aminoethoxy)ethoxy)ethyl)-3-methyl-*N*-(2-(1-methylpyrrolidin-2-yl)-1*H*-pyrrolo [3,2-c]pyridin-6-yl)-1*H*-indazole-6-carboxamide dihydrochloride**

To a cooled (0°C) solution of *tert*-butyl (*R*)-6-(1-(2,2-dimethyl-4-oxo-3,8,1 1-trioxa-5-azatridecan-13-yl)-3-methyl-1*H*-indazole-6-carboxamido)-2-(1-methylpyrrolidin-2-yl)-1*H*-pyrrolo[3,2-c]pyridine-1-carboxylate **(Intermediate 121,** 0.3 g, 0.43 mmol) in 1,4-dioxane (3 mL) was added 4M HCl in 1,4-dioxane (5 mL) and the reaction mixture was allowed to warm slowly to room temperature and then stirred for 16h. The reaction mixture then was concentrated *in vacuo,* washed with DCM (2 × 20 mL). The resultant solid was washed with diethyl ether (2 × 20 mL) then dried *in vacuo* to afford (R)-1-(2-(2-(2-aminoethoxy)ethoxy)ethyl)-3-methyl-N (2-(1-methylpyrrolidin-2-yl)-1*H*-pyrrolo[3,2-c]pyridin-6-yl)-1*H*-indazole-6-carboxamide dihydrochloride **(Intermediate 122,** 0.24 g) as a white solid, which was used for next step without further purification. $^1$H NMR (400 MHz, DMSO-$d_6$) δ ppm 2.19-2.20 (m, 2H), 2.42 (d, *J* = 9.65 Hz, 2H), 2.55 (s, 3H), 2.80-2.89 (m, 5H), 3.48 (s, 3H), 3.70-3.73 (m, 4H), 3.90-3.91 (m, 4H), 4.60-4.63 (m, 2H), 4.76 (d, *J* = 6.80 Hz, 1H), 7.30 (s, 1H), 7.82-8.03 (m, 4H), 8.44 (s, 1H), 8.80 (s, 1H), 9.13 (s, 1H), 11.55 (s, 1H), 12.33 (s, 1H).

**Step 6**

**Example 25: 1-(2-(2-(2-((2-(2,6-dioxopiperidin-3-yl)-1,3-dioxoisoindolin-4-yl)amino)ethoxy)ethoxy)ethyl)-3-methyl-*N*-(2-((R)-1-methylpyrrolidin-2-yl)-1*H*-pyrrolo[3,2-c]pyridin-6-yl)-1*H*-indazole-6-carboxamide**

To a solution of (R)-1-(2-(2-(2-aminoethoxy)ethoxy)ethyl)-3-methyl-N-(2-(1-methylpyrrolidin-2-yl)-1*H*-pyrrolo [3,2-c]pyridin-6-yl)-1*H*-indazole-6-carboxamide dihydrochloride **(Intermediate 122, 0.28** g, 0.55 mmol) in DMSO (5 mL) was added 2-(2,6-dioxopiperidin-3-yl)-4-fluoroisoindoline-1,3-dione (CAS No: 835616-60-9, 0.15 g, 0.55 mmol) followed by DIPEA (0.29 mL, 1.66 mmol) and the reaction mixture was stirred at 110°C for 6h. The reaction mixture was then cooled to room temperature and diluted with water (50 mL). The resultant precipitate was collected by filtration, washed with DCM/pentane (1:1, 2 × 20 mL) and then dried *in vacuo.* The crude compound was purified by preparative HPLC (Method A) to afford 1-(2-(2-(2-((2-(2,6-dioxopiperidin-3-yl)-1,3-dioxoisoindolin-4-yl)amino)ethoxy)ethoxy) ethyl)-3-methyl-*N*-(2-((R)-1-methylpyrrolidin-2-yl)-1*H*-pyrrolo[3,2-c]pyridin-6-yl)-1*H*-indazole-6-carboxamide **(Example 25, 60** mg, 14%) as yellow solid. $^1$H NMR (400 MHz, DMSO-$d_6$) δ ppm 1.75-2.04 (m, 4H), 2.17 (s, 3H), 2.22-2.35 (m, 1H), 2.42-2.48 (m, 1H), 2.52 (s, 3H), 2.83-2.86 (m, 2H), 3.15 (t, *J* = 8.07 Hz, 1H), 3.27-3.31 (m, 4H), 3.45-3.54 (m, 6H), 3.86 (t, *J* = 5.50 Hz, 2H), 4.56 (t, *J* = 5.44 Hz, 2H), 5.03 (dd, *J* = 12.88, 5.38 Hz, 1H), 6.40 (s, 1H), 6.51 (t, *J* = 6.13 Hz, 1H), 6.96-6.98 (m, 2H), 7.47 (dd, *J* = 8.38, 7.25 Hz, 1H), 7.67-7.81 (m, 2H), 8.25 (s, 1H), 8.43-8.54 (m, 2H), 10.49 (s, 1H), 11.09 (s, 1H), 11.39 (s, 1H). Mass spec: m/z 762.2 [M+H]+.

**Example 26 was synthesised following the Scheme 25**

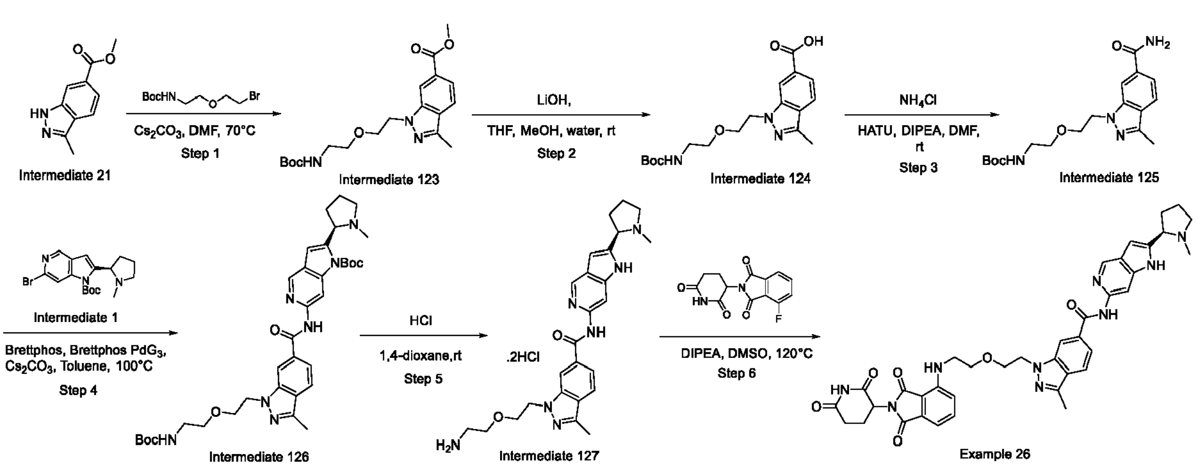


Scheme 25

**Step 1:**

**Intermediate 123: Methyl 1-(2-(2-((*tert*-butoxycarbonyl)amino)ethoxy)ethyl)-3-methyl-1*H*-indazole-6-carboxylate**

To a solution of methyl 3-methyl-1*H*-indazole-6-carboxylate **(Intermediate 21,** 1.5 g, 7.89 mmol) in DMF (15 mL) was added *tert*-butyl (2-(2-bromoethoxy)ethyl)carbamate (CAS No: 164332-88-1, 3.17 g, 11.8 mmol) followed by cesium carbonate (6.43 g,19.7 mmol) and the reaction mixture was heated to 70 °C for 16h. The reaction mixture was diluted with water (100 mL) and extracted with ethyl acetate (2 × 100 mL). The combined organic layers were separated, dried over $Na_2SO_4$ and concentrated *in vacuo.* The crude material was purified by combi-flash column chromatography, by eluting with 35% ethyl acetate in heptane, to afford methyl 1-(2-(2-((*tert*-butoxycarbonyl)amino)ethoxy)ethyl)-3-methyl-1*H*-indazole-6-carboxylate **(Intermediate 123,** 2.0 g, 67%) as a yellow sticky liquid. $^1$H NMR (400 MHz, DMSO-$d_6$) δ ppm 1.34 (s, 9H), 2.51 (s, 3H), 2.97-2.99 (m, 2H), 3.27- 3.35 (m, 2H), 3.78 (t, *J*=4.86 Hz, 2H), 3.90 (s, 3H),4.56 (t, *J*=5.09 Hz, 2H), 6.54 (br s, 1H), 7.65 (d, *J*=8.32 Hz, 1H), 7.80 (d, *J*=8.32 Hz, 1H), 8.26 (s, 1H). Mass spec: m/z 378.2 [M+H] $^+$.

**Step 2**

**Intermediate 124: 1-(2-(2-((*tert*-butoxycarbonyl)amino)ethoxy)ethyl)-3-methyl-1H indazole-6-carboxylic acid**

To a solution of methyl 1-(2-(2-((*tert*-butoxycarbonyl)amino)ethoxy)ethyl)-3-methyl-1*H*-indazole-6-carboxylate **(Intermediate 123,** 0.65 g, 1.722 mmol) in tetrahydrofuran (2.5 mL) and methanol (2.5 mL) was added lithium hydroxide (0.25 g, 10 mmol) in water (2.5 mL). The reaction mixture was stirred at room temperature for 16h then concentrated *in vacuo* to one-third volume. The pH of the aqueous layer was adjusted to 3-4 with 0.5 N HCl and extracted with EtOAc (2 × 50 mL). The combined organic layers were dried over $Na_2SO_4$ and concentrated *in vacuo* to afford 1-(2-(2-((*tert*-butoxycarbonyl)amino)ethoxy)ethyl)-3-methyl-1*H*-indazole-6-carboxylic acid **(Intermediate 124,** 0.53 g, 85%) as an off-white solid which was used for the next step without further purification. $^1$H NMR (400 MHz, DMSO-$d_6$) δ ppm 1.34 (s, 9H), 2.52 (s, 3H), 2.94-2.99 (m, 2H), 3.30-3.34 (m, 2H), 3.78 (t, *J*=5.32 Hz, 2 H), 4.54 (t, *J*=5.32 Hz, 2H), 6.55 (br s, 1H), 7.64-7.66 (m, 1H), 7.77-7.78 (m, 1H), 8.22 (s, 1H), 12.98 (br s, 1H). Mass spec: m/z 364.3 [M+H] $^+$.

**Step 3:**

**Intermediate 125: *tert*-Butyl (2-(2-(6-carbamoyl-3-methyl-1*H*-indazol-1-yl)ethoxy)ethyl)carbamate**

To a solution of 1-(2-(2-((*tert*-butoxycarbonyl)amino)ethoxy)ethyl)-3-methyl-1*H*-indazole-6-carboxylic acid **(Intermediate 124,** 0.70 g, 1.926 mmol) in dimethylformamide (8 mL) was added ammonium chloride (0.72 g, 13.48 mmol) and HATU (1.88 g, 4.81 mmol) followed by DIPEA (1.27 g, 9.63 mmol) and reaction mixture was stirred at room temperature for 16h. The reaction mixture was diluted with water (100 mL) and extracted with EtOAc (2 × 70 mL). The combined organic layers were dried over $Na_2SO_4$ and concentrated *in vacuo* to afford *tert*-butyl (2-(2-(6-carbamoyl-3-methyl-1*H*-indazol-1-yl) ethoxy) ethyl) carbamate **(Intermediate 125,** 0.60 g, 86%) as an off white solid which was used for the next step without further purification. $^1$H NMR (400 MHz, DMSO-$d_6$) δ ppm 1.34 (s, 9H), 2.50 (s, 3H), 2.92-3.03 (m, 2H), 3.30-3.37 (m, 2H), 3.76-3.82 (m, 2H), 4.49 (t, *J*=5.22 Hz, 2H), 6.64 (br s, 1H), 7.60 (d, *J*=8.31 Hz, 1H), 7.73 (d, *J*=8.31 Hz, 1H), 8.01 (br s, 1H), 8.15 (s, 1H). Mass spec: m/z 362.8 [M+H]$^+$.

**Step 4:**

**Intermediate 126: *tert-Butyl* (*R*)-6-(1-(2-(2-((*tert*-butoxycarbonyl)amino)ethoxy)ethyl)-3-methyl-1*H*-indazole-6-carboxamido)-2-(1-methylpyrrolidin-2-yl)-1*H*-pyrrolo[3,2-c] pyridine-1-carboxylate:**

To a solution of *tert*-butyl (2-(2-(6-carbamoyl-3-methyl-1*H*-indazol-1-yl) ethoxy) ethyl) carbamate **(Intermediate 125,** 0.25 g, 0.68 mmol) in toluene (5 mL) was added *tert*-butyl (*R*)-6-bromo-2-(1-methylpyrrolidin-2-yl)-1*H*-pyrrolo[3,2-c] pyridine-1-carboxylate **(Intermediate 1,** 0.20 g, 0.55 mmol) and cesium carbonate (0.67 g, 2.070 mmol). The reaction mixture was purged with argon gas for 10 min then BrettPhos (0.057 mg, 0.10 mmol) and BrettPhos Pd G3 (0.095 g, 0.10 mmol) were added and the mixture was purged again with argon gas for 10 min, then heated to 100°C for 1h. The reaction mixture was cooled to room temperature, treated with water (50 mL), and extracted with ethyl acetate (3 × 50 mL). The combined organic layers were dried over $Na_2SO_4$ and concentrated *in vacuo.* The crude material was purified by combi-flash column chromatography, by eluting with 80% ethyl acetate in heptane, to afford tert-butyl *(R)*-6-(1-(2-(2-((*tert*-butoxycarbonyl)amino)ethoxy)ethyl)-3-methyl-1*H*-indazole-6-carboxamido)-2-(1-methylpyrrolidin-2-yl)-1*H*-pyrrolo [3,2-*c*]pyridine-1-carboxylate **(Intermediate 126,** 0.20 g, 44%) as a yellow solid. Mass spec: m/z 661.9 [M+H] $^+$.

**Step 5:**

**Intermediate 127: (*R*)-1-(2-(2-aminoethoxy) ethyl)-3-methyl-*N*-(2-(1-methylpyrrolidin-2-yl)-1*H*-pyrrolo [3,2-*c*] pyridin-6-yl)-1*H*-indazole-6-carboxamide dihydrochloride**

To a cooled (0°C) solution of *tert*-butyl (R)-6-(1-(2-(2-((*tert*-butoxycarbonyl)amino)ethoxy)ethyl)-3-methyl-1*H*-indazole-6-carboxamido)-2-(1-methylpyrrolidin-2-yl)-1*H*-pyrrolo[3,2-*c*]pyridine-1-carboxylate **(Intermediate 126,** 0.20 g, 0.30 mmol) in 1,4-dioxane (5 mL) was added 4 M HCl in 1,4dioxane (5 mL). The reaction mixture was allowed to stir to room temperature for 16h. The reaction mixture was concentrated *in vacuo,* the resultant precipitate was collected by filtration, washed with diethyl ether (2 × 20 mL) and dried *in vacuo* to to afford crude (*R*)-1-(2-(2-aminoethoxy) ethyl)-3-methyl-*N*-(2-(1-methylpyrrolidin-2-yl)-1*H*-pyrrolo [3, 2-*c*] pyridin-6-yl)-1*H*-indazole-6-carboxamide dihydrochloride **(Intermediate 127,** 0.16 g) as a yellow solid which was directly used in the next step without purification. $^1$H NMR (400 MHz, DMSO-$d_6$) δ ppm 1.23-1.25 (m, 2H), 1.58-1.61 (m, 1H), 2.20-2.22 (m, 2H), 2.56 (s, 3H), 2.82 (s, 3H), 2.90-2.92 (m, 3H), 3.70-3.85 (m, 4H), 3.94-3.96 (m, 3H), 4.66-4.68 (br s, 3H), 7.24 (br s, 1H), 7.84 (br s, 1H), 7.89 (br s, 1H), 8.42 (s, 1H), 8.84 (s, 1H), 9.08 (s, 1H), 11.27 (br s, 1H), 12.2 (s, 1H) 13.09 (br s, 1H). Mass spec: m/z 461.8 $[M+H]^+$.

**Step 6:**

**Example 26: 1-(2-(2-((2-(2,6-dioxopiperidin-3-yl)-1,3-dioxoisoindolin-4yl)amino)ethoxy)ethyl)-3-methyl-*N*-(2-((*R*)-1-methylpyrrolidin-2-yl)-1*H*-pyrrolo[3,2-c]pyridin-6-yl)-1*H*-indazole-6-carboxamide**

To a solution of (R)-1-(2-(2-aminoethoxy) ethyl)-3-methyl-*N*-(2-(1-methylpyrrolidin-2-yl)-1*H*-pyrrolo [3, 2-*c*] pyridin-6-yl)-1*H*-indazole-6-carboxamide dihydrochloride **(Intermediate 127,** 0.157 g, 0.34 mmol,) in DMSO (5.00 mL) was added 2-(2,6-dioxopiperidin-3-yl)-4-fluoroisoindoline-1,3-dione (CAS No: 835616-60-9, 0.093 g, 0.3401 mmol) and DIPEA (0.135 g, 1.020 mmol). The reaction mixture was heated at 110°C for 5h. The reaction mixture was cooled to room temperature, treated with cold water (50 mL) and the resultant yellow precipitate was collected by filtration and dried *in vacuo.* The crude compound was purified by preparative HPLC (Method A) to afford 1-(2-(2-((2-(2,6-dioxopiperidin-3-yl)-1,3-dioxoisoindolin-4-yl)amino)ethoxy)ethyl)-3-methyl-*N*-(2-((*R*)-1-methylpyrrolidin-2-yl)-1*H*-pyrrolo[3,2-*c*]pyridin-6-yl)-1*H*-indazole-6-carboxamide **(Example 26,** 0.035 g, 14%) as a yellow solid. $^1$H NMR (400 MHz, DMSO-$d_6$) δ ppm 1.77-1.94 (m, 3H), 1.97-2.03 (m, 1H), 2.15-2.16 (m, 1H), 2.17 (s, 3H), 2.27-2.28 (m, 1H), 2.38-2.48 (m, 1H), 2.57 (s, 3H), 2.80-2.90 (m, 1H), 3.08-3.21 (m, 1H), 3.28- 3.31 (m, 1H), 3.38-3.40 (m, 3H), 3.60 (t, J=5.44 Hz, 2H), 3.93 (t, J=5.57 Hz, 2H), 4.58 (t, *J*=5.69 Hz, 2H), 5.01-5.03 (m, 1H), 6.40 (s, 1H), 6.50 (t, *J*=5.88 Hz, 1H), 6.97-6.99 (m, 2H), 7.41-7.48 (m, 1H), 7.70-7.77 (m, 2H), 8.24 (s, 1H), 8.47 (s, 1H), 8.51 (s, 1H), 10.48 (s, 1H), 11.09 (s, 1H), 11.39 (s, 1H). Mass spec: m/z 717.2 $[M+H]^+$.

**Example 27 was made following Scheme 26**

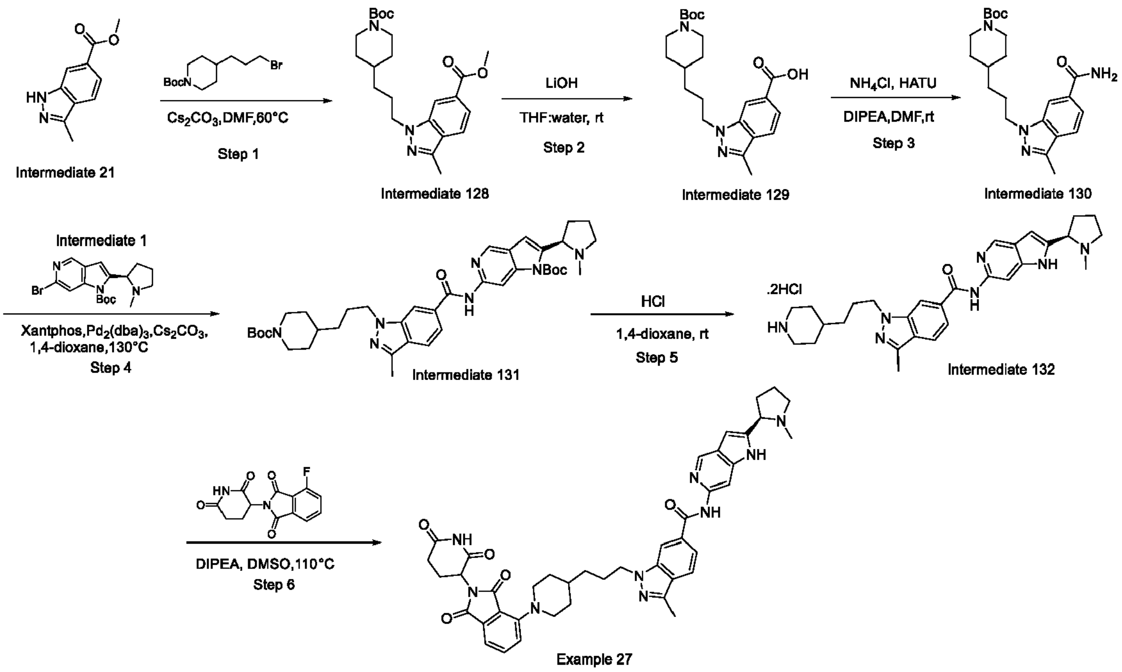


**Scheme 26**

Step 1

**Intermediate 128: methyl 1-(3-(1-(*tert*-butoxycarbonyl)piperidin-4-yl) propyl)-3-methyl-1*H*-indazole-6-carboxylate:**

To a cooled (0°C) solution of methyl 3-methyl-1*H*-indazole-6-carboxylate **(Intermediate 21,** 5.00 g, 26.28 mmol) in DMF (50 mL) was added cesium carbonate (17.1 g, 52.57 mmol) followed by tert-butyl 4-(3-bromopropyl)piperidine-1-carboxylate (CAS No: 164149-27-3, 8.04 g, 26.28 mmol) and the reaction mixture was heated to 80°C for 16h. The reaction mixture was then cooled to room temperature, treated with water (60 mL) and extracted with ethyl acetate (2 × 100 mL). The combined organic layers were dried over anhydrous $Na_2SO_4$ and concentrated *in vacuo.* The crude material was purified by combi-flash column chromatography, by eluting with 15% ethyl acetate in heptane, to afford methyl 1-(3-(1-(tert-butoxycarbonyl) piperidin-4-yl)propyl)-3-methyl-1H-indazole-6-carboxylate **(Intermediate 128,** 6.0 g, 55%) as an off white solid. $^1$H NMR (400 MHz, DMSO-$d_6$) δ ppm 0.86-0.97 (m, 3H), 1.11-1.23 (m, 2H), 1.37 (s, 9H), 1.54-1.57 (m, 2H), 1.77-1.83 (m, 2H), 2.51 (s, 3H), 2.63-2.65 (m, 2H), 3.82-3.87 (m, 2H), 3.90 (s, 3H), 4.39 (t, *J*=7.02 Hz, 2H), 7.65 (d, *J*=8.33, 1H), 7.81 (d, *J*=8.33 Hz, 1H), 8.23 (s, 1H). Mass spec: m/z 416.3 [M+H]$^+$.

**Step 2:**

**Intermediate 129: 1-(3-(1-(tert-butoxycarbonyl) piperidin-4-yl) propyl)-3-methyl-1H-indazole-6-carboxylic acid**

To a solution of methyl 1-(3-(1-(*tert*-butoxycarbonyl) piperidin-4-yl)propyl)-3-methyl-1*H*-indazole-6-carboxylate **(Intermediate 128,** 6.00 g, 14.44 mmol) in THF (30 mL) and methanol (30 mL) was added lithium hydroxide (3.03 g, 72.20 mmol) in water (15 mL). The reaction mixture was stirred at room temperature for 4h and then concentrated *in vacuo.* Water (30 mL) was added, the mixture acidified to pH~3 using aqueous saturated citric acid solution and the resultant precipitate was collected by filtration and dried *in vacuo* to afford to afford *tert*-butyl 4-(3-(6-carbamoyl-3-methyl-1H-indazol-1-yl) propyl) piperidine-1-carboxylate **(Intermediate 129,** 5.0 g, 86%) as an off-white solid which was used for the next step without further purification. $^1$H NMR (400 MHz, DMSO-$d_6$) δ ppm 0.84-0.93 (m, 2H), 1.14-1.20 (m, 3H), 1.37 (s, 9H), 1.52-1.58 (m, 2H), 1.78-1.85 (m, 2H), 2.50 (s, 3H), 2.62-2.73 (m, 2H), 3.86-3.89 (m, 2H), 4.37 (t, J=6.91 Hz, 2H), 7.64 (d, *J*=8.33 Hz, 1H), 7.78 (d, *J*=8.33 Hz, 1H), 8.19 (s, 1H), 13.02 (br s, 1H). Mass spec: m/z 402.3 [M+H]$^+$.

**Step** 3

**Intermediate 130: tert-butyl 4-(3-(6-carbamoyl-3-methyl-1H-indazol-1-yl) propyl)piperidine-1-carboxylate**

To a solution of 1-(3-(1-(*tert*-butoxycarbonyl)piperidin-4-yl)propyl)-3-methyl-1H-indazole-6-carboxylic acid **(Intermediate 129,** 5.00 g, 12.45 mmol) in dimethylformamide (50 mL) was added HATU (5.85 g, 14.94 mmol) and DIPEA (6.53 mL, 37.36 mmol). The reaction mixture was stirred at room temperature for 15 min. Ammonium chloride (0.7 g, 62.27 mmol) was then added and the reaction mixture was stirred at room temperature for 16h. The reaction mixture was then treated with water (200 mL) and extracted with EtOAc (3 × 150 mL). The combined organic layers were washed with $NaHCO_3$ (2 × 60 mL) and brine solution (2 × 60 mL), dried over $Na_2SO_4$ and concentrated *in vacuo.* The crude material was purified by combi-flash column chromatography, by eluting with 70% ethyl acetate in heptane, to afford *tert*-butyl 4-(3-(6-carbamoyl-3-methyl-1*H*-indazol-1-yl)propyl) piperidine-1-carboxylate **(Intermediate 130,** 2.7 g, 54%) as an off-white solid. Mass spec: m/z 401.3 $[M+H]^+$.

**Step 4**

**Intermediate 131: *tert*-butyl-(*R*)-6-(1-(3-(1-(tert-butoxycarbonyl)piperidin-4-yl)propyl)-3-methyl-1*H*-indazole-6-carboxamido)-2-(1-methylpyrrolidin-2-yl)-1H-pyrrolo [3, 2-c] pyridine-1-carboxylate**

To a solution of *tert*-butyl 4-(3-(6-carbamoyl-3-methyl-1*H*-indazol-1-yl)propyl)piperidine-1-carboxylate **(Intermediate 130,** 0.42 g, 1.05 mmol) in 1,4-dioxane (10 mL) was added *tert*-butyl-(R)-6-bromo-2-(1-methylpyrrolidin-2-yl)-1H-pyrrolo[3,2-c]pyridine-1-carboxylate **(Intermediate 1,** 0.5 g, 1.31 mmol) followed by cesium carbonate (1.28 g, 3.94mmol). The reaction mixture was purged with argon for 10 min then $Pd_2(dba)_3$(0.18 g, 0.19 mmol) and 4,5-bis(diphenylphosphino)-9,9-dimethylxanthene (0.22 g, 0.39 mmol) was added and the reaction mixture was heated at 130°C for 2h. The reaction mixture was then diluted with water (100 mL) and extracted with ethyl acetate (2 × 100 mL). The combined organic layers were separated, dried over anhydrous $Na_2SO_4$ and concentrated *in vacuo.* The crude material was purified by combi-flash chromatography, by eluting with 70% ethyl acetate in heptane, to afford *tert*-butyl-(*R*)-6-(1-(3-(1-(*tert*-butoxycarbonyl)piperidin-4-yl)propyl)-3-methyl-1*H*-indazole-6-carboxamido)-2-(1-methylpyrrolidin-2-yl)-1H-pyrrolo[3,2-c]pyridine-1-carboxylate **(Intermediate 131,** 0.3 g, 33%) as a yellow solid. Mass spec: m/z 700 $[M+H]^+$.

**Step 5**

**Intermediate 132: (*R*)-3-methyl-*N*-(2-(1-methylpyrrolidin-2-yl)-1*H*-pyrrolo[3,2-c]pyridin-6-yl)-1-(3-(piperidin-4-yl)propyl)-1*H*-indazole-6-carboxamide dihydrochloride**

To a 0°C solution of (*R*)-6-(1-(3-(1-(tert-butoxycarbonyl)piperidin-4-yl)propyl)-3-methyl-1*H*-indazole-6-carboxamido)-2-(1-methylpyrrolidin-2-yl)-1H-pyrrolo[3,2-c]pyridine-1-carboxylate **(Intermediate 131, 0.6** g, 0.85 mmol) in 1,4-dioxane (8 mL) was added 4M HCl in 1,4-dioxane (8 mL). The reaction mixture was stirred at room temperature for 16h and then concentrated *in vacuo* to afford (R)-3-methyl-*N*-(2-(1-methylpyrrolidin-2-yl)-1*H*-pyrrolo[3,2-c]pyridin-6-yl)-1-(3-(piperidin-4-yl)propyl)-1*H*-indazole-6-carboxamide dihydrochloride **(Intermediate 132,** 0.46 g) as a yellow solid, which was used in next step without further purification. $^1H$ NMR (400 MHz, DMSO-$d_6$) δ ppm 1.29-1.35 (m, 4H), 1.58-1.60 (m, 1H), 1.74-1.77 (m, 2H), 1.82-1.98 (m, 2H), 2.18 (s, 3H), 2.19-2.22 (m, 2H), 2.55 (s, 3H), 2.74-2.87 (m, 4H), 3.15-3.31 (m, 4H), 4.46 (t, *J*=6.51 Hz, 2H), 4.72-4.75 (m, 1H), 7.27 (s, 1H), 7.81-7.83 (m, 1H), 7.79-7.91 (m, 1H), 8.43 (br s, 2H), 8.80 (br s, 2H), 9.10 (s, 1H), 11.32 (br s, 1H), 12.25 (br s, 1H), 13.17 (br s, 1H). Mass spec: m/z 500.1 $[M+H]^+$.

**Step 6**

**Example 27: 1-(3-(1-(2-(2,6-dioxopiperidin-3-yl)-1,3-dioxoisoindolin-4-yl)piperidin-4-yl)propyl)-3-methyl-*N*-(2-((*R*)-1-methylpyrrolidin-2-yl)-1*H*-pyrrolo[3,2-c]pyridin-6-yl)-1*H*-indazole-6-carboxamide**

To a solution of (*R*)-3-methyl-*N*-(2-(1-methylpyrrolidin-2-yl)-1*H*-pyrrolo [3,2-c]pyridin-6-yl)-1-(3-(piperidin-4-yl)propyl)-1*H*-indazole-6-carboxamide dihydrochloride **(Intermediate 132,** 0.30 g, 0.60 mmol) in dimethyl sulfoxide (4 mL) was added 2-(2,6-dioxopiperidin-3-yl)-4-fluoroisoindoline-1,3-dione (CAS No: 835616-60-9, 0.16 g, 0.60 mmol) followed by DIPEA (0.29 mL, 3.00 mmol) and the reaction mixture was stirred at 110°C for 8h. The reaction mixture was then quenched with ice cold water, a yellow precipitate was obtained which was filtered and dried *in vacuo.* The crude material was then purified by preparative HPLC (Method A) to afford 1-(3-(1-(2-(2,6-dioxopiperidin-3-yl)-1,3-dioxoisoindolin-4-yl)piperidin-4-yl)propyl)-3-methyl-*N*-(2-((R)-1-methylpyrrolidin-2-yl)-1*H*-pyrrolo[3,2-c]pyridin-6-yl)-1*H*-indazole-6-carboxamide **(Example DBT-SAI-88,** 0.07 g, 15%) as a yellow solid. $^1H$ NMR (400 MHz, DMSO-$d_6$) δ ppm 1.29-1.35 (m, 4H), 1.46-1.48 (m, 1H), 1.72-1.85 (m, 3H), 1.89-2.02 (m, 5H), 2.18 (s, 3H), 2.24-2.30 (m, 1H), 2.53 (s, 3H), 2.55-2.59 (m, 2H),

2.80-2.86 (m, 3H), 3.13-3.17 (m, 1H), 3.32-3.34 (m, 2H), 3.65-3.68 (m, 2H), 4.43 (t, *J*=6.88 Hz, 2H), 5.05-5.09 (m, 1H), 6.40 (s, 1H), 7.28-7.30 (m, 2H), 7.62-7.66 (m, 1H), 7.72-7.76 (m, 1H), 7.80 (d, *J*=8.50 Hz, 1H), 8.25 (s, 1H), 8.45 (s, 1H), 8.52 (s, 1H), 10.53 (s, 1H), 11.39 (s, 1H). Mass spec: m/z 756.2 $[M+H]^+$.

**Example 28 was made following Scheme 27**

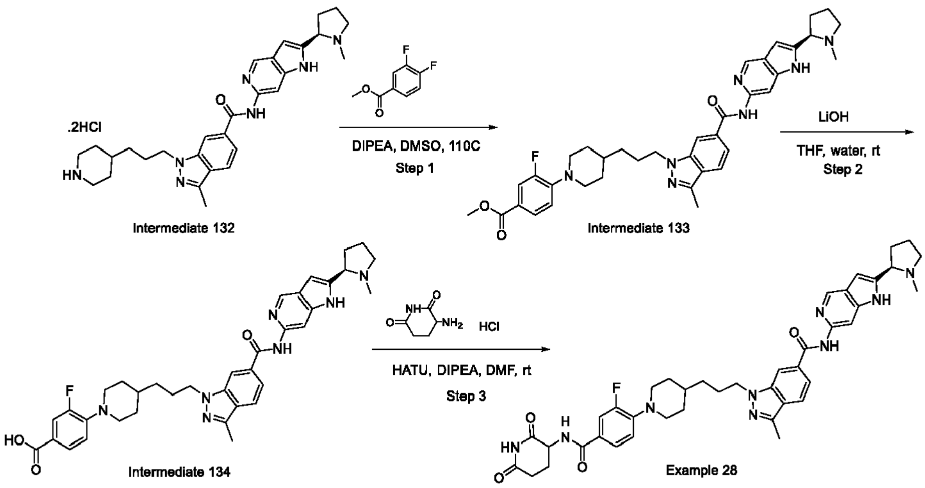


**Scheme 27**

**Step** 1

**Intermediate 133: methyl (*R*)-3-fluoro-4-(4-(3-(3-methyl-6-((2-(1-methylpyrrolidin-2-yl)-1H-pyrrolo[3,2-c]pyridin-6-yl)carbamoyl)-1H-indazol-1-yl)propyl)piperidin-1-yl)benzoate**

To a suspension of (*R*)-3-methyl-*N*-(2-(1-methylpyrrolidin-2-yl)-1*H*-pyrrolo [3,2-c]pyridin-6-yl)-1-(3-(piperidin-4-yl)propyl)-1*H*-indazole-6-carboxamide dihydrochloride **(Intermediate 132,** 300 mg, 0.60 mmol) and methyl 3,4-difluorobenzoate (CAS No: 369-25-5, 0.124 g) in DMSO (3.0 mL) was added DIPEA (1.80 g, 1.801 mmol) and the reaction mixture was heated at 80°C for 16h. Ice cold water (5 mL) was added and the resultant precipitate was collected by filtration and dried *in vacuo* to afford methyl (*R*)-3-fluoro-4-(4-(3-(3-methyl-6-((2-(1-methylpyrrolidin-2-yl)-1H-pyrrolo[3,2-c]pyridin-6-yl)carbamoyl)-1H-indazol-1-yl)propyl)piperidin-1-yl)benzoate **(Intermediate 133,** 0.17 g, 43%) as a pale yellow solid which was used for the next step without further purification. $^1$H NMR (400 MHz, DMSO-$d_6$) δ ppm 1.17-1.32 (m, 4H), 1.69-1.98 (m, 4H), 2.13-2.19 (m, 4H), 2.22-2.33 (m, 1H), 2.54 (s, 3H), 2.68-2.78 (m, 2H) 3.15-3.17 (m, 1H) 3.37 (s, 3H), 3.49-3.50 (m, 2H), 3.79 (s, 3H), 4.11-4.13 (m, 2H), 4.41-4.42 (m, 2H), 6.41 (s, 1H), 7.04 (t, *J*=8.82 Hz, 1H), 7.53-7.57 (m, 2H), 7.72-7.75 (m, 1H), 7.77-7.81 (m, 1H), 8.24 (s, 1H), 8.43 (s, 1H), 8.52 (s, 1H) 10.51 (s, 1H), 11.38 (br s, 1H). Mass spec: m/z 652.3 $[M+H]^+$.

**Step 2**

**Intermediate 134: (*R*)-3-fluoro-4-(4-(3-(3-methyl-6-((2-(1-methylpyrrolidin-2-yl)-1*H*-pyrrolo[3,2-c]pyridin-6-yl) carbamoyl)-1*H*-indazol-1-yl)propyl)piperidin-1-yl)benzoic acid**

A solution of (R)-3-fluoro-4-(4-(3-(3-methyl-6-((2-(1-methylpyrrolidin-2-yl)-1H-pyrrolo[3,2-c]pyridin-6-yl)carbamoyl)-1H-indazol-1-yl)propyl)piperidin-1-yl)benzoate **(Intermediate 133,** 0.17 g, 0.26 mmol) and LiOH (0.021 g, 0.52 mmol) in THF (3 mL), methanol (2 mL) and water (2 mL) was stirred at room temperature for 4h. The reaction mixture was concentrated *in vacuo* to one-third volume and water (10 mL) was added. The pH was adjusted to pH~2 by addition of aqueous citric acid solution and the resultant white precipitate was filtered and dried *in vacuo* to afford (R)-3-fluoro-4-(4-(3-(3-methyl-6-((2-(1-methylpyrrolidin-2-yl)-1H-pyrrolo[3,2-c]pyridin-6-yl)carbamoyl)-1H-indazol-1-yl)propyl)piperidin-1-yl)benzoic acid **(Intermediate 134,** 0.13 g, 78%) as off white solid which was used for the next step without further purification. $^1$H NMR (400 MHz, DMSO-$d_6$) δ ppm 1.15-1.32 (m, 7H), 1.35-1.47 (m, 2H), 2.16-2.18 (m, 3H), 2.19 (s, 3H), 2.22-2.31 (m, 2H), 2.54 (s, 3H), 2.60-2.72 (m, 3H), 3.11-3.17 (m, 2H), 4.00-4.06 (m, 1H), 4.42-4.44 (m, 2H), 6.39-6.47

(m, 2H), 7.43 (s, 1H), 7.72-7.81 (m, 2H), 8.25 (s, 1H), 8.43-8.55 (m, 3H), 10.54 (s, 1H), 11.40 (s, 1H), 11.60 (br s, 1H). Mass spec: m/z 638.36 $[M+H]^+$.

**Step 3**

**Example 28: 1-(3-(1-(4-((2,6-dioxopiperidin-3-yl)carbamoyl)-2-fluorophenyl)piperidin-4-yl)propyl)-3-methyl-N-(2-((*R*)-1-methylpyrrolidin-2-yl)-1H-pyrrolo[3,2-c]pyridin-6-yl)-1H-indazole-6-carboxamide**

To a solution of (*R*)-3-fluoro-4-(4-(3-(3-methyl-6-((2-(1-methylpyrrolidin-2-yl)-1H-pyrrolo[3,2-c]pyridin-6-yl)carbamoyl)-1H-indazol-1-yl)propyl)piperidin-1-yl)benzoic acid **(Intermediate 134,** 0.15 g, 0.23 mmol) in DMF (3.00 mL) was added HATU (0.11 g 0.28 mmol) and DIPEA (0.093 g, 0.70 mmol) and the reaction mixture was stirred at room temperature for 15 min. 3-Aminopiperidine-2,6-dione hydrochloride (CAS No: 24666-56-6, 0.042 g, 0.25 mmol) was added and the reaction mixture was stirred at room temperature for 16h. The reaction mixture was then diluted with water (100 mL) and extracted with EtOAc (3 × 50 mL). The combined organic layers were washed with $NaHCO_3$ (2 × 40 mL) and brine (2 × 30 mL), then dried over anhydrous $Na_2SO_4$ and concentrated *in vacuo.* The crude material was purified by preparative HPLC (Method A) to afford (*R*)-3-fluoro-4-(4-(3-(3-methyl-6-((2-(1-methylpyrrolidin-2-yl)-1H-pyrrolo[3,2-c]pyridin-6-yl)carbamoyl)-1H-indazol-1-yl)propyl)piperidin-1-yl)benzoic acid **(Example 28,** 0.015 g, 9%) as off-white solid. $^1H$ NMR (400 MHz, DMSO-$d_6$) δ ppm 1.23-1.31 (m, 4H), 1.42-1.44 (m, 1H), 1.71-1.83 (m, 3H), 1.81-1.96 (m, 5H), 2.07-2.14 (m, 2H), 2.17 (s, 3H), 2.23-2.30 (m, 1H), 2.53 (s, 3H), 2.66-2.69 (m, 2H), 2.73-2.81 (m, 1H), 3.12-3.17 (m, 1H), 3.24-3.31 (m, 1H), 3.33-3.35 (m, 1H), 3.44-3.47 (m, 2H), 4.42 (t, *J*=6.94 Hz, 2H), 4.71-4.78 (m, 1H), 6.41 (s, 1H), 7.05-7.10 (m, 1H), 7.57-7.63 (m, 2H), 7.74 (d, *J*=8.51, 1H), 7.80 (d, *J*=8.50 Hz, 1H), 8.25 (s, 1H), 8.45 (s, 1H), 8.52 (s, 1H), 8.63-8.64 (m, 1H), 10.54 (s, 1H), 10.84 (s, 1H), 11.40 (s, 1H). Mass spec: m/z: 748.4 $[M+H]^+$.

**Example 29 was synthesised following Scheme 28**

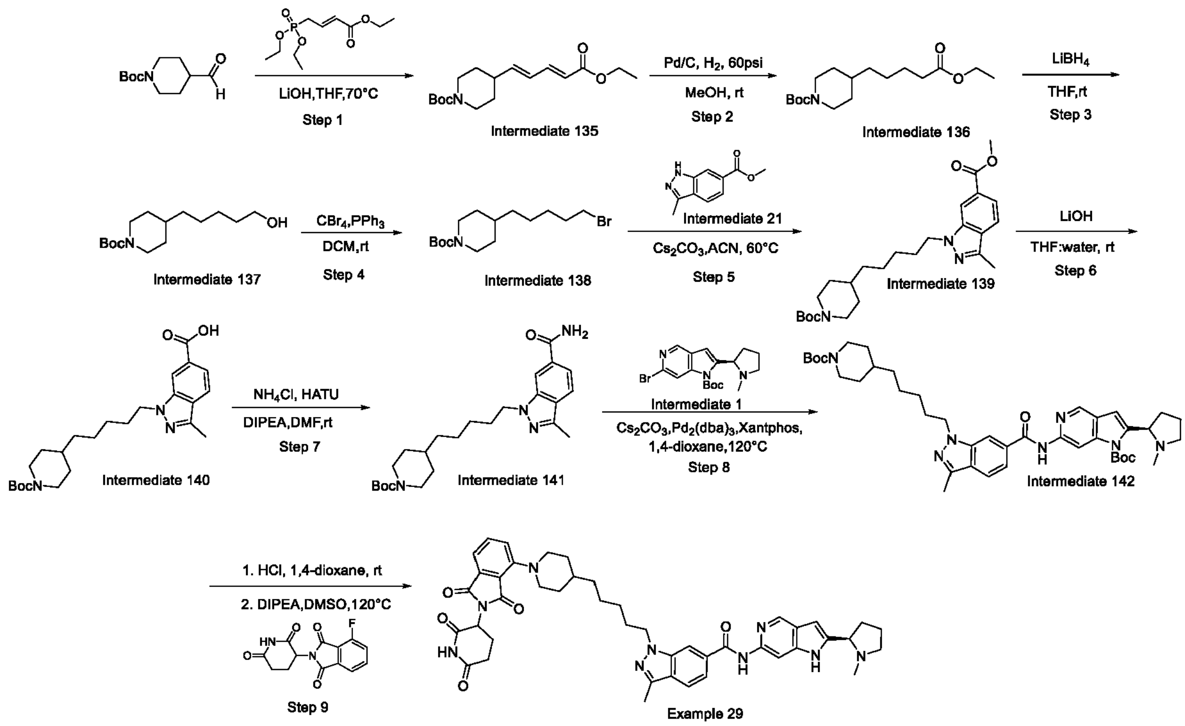


**Scheme 28**

**Step 1**

**Intermediate 135: tert-butyl 4-((1E, 3E)-5-ethoxy-5-oxopenta-1, 3-dien-1-yl) piperidine-1-carboxylate**

To a solution of tert-butyl 4-formylpiperidine-1-carboxylate (CAS No: 137076-22-3, 10.0 g, 46.9 mmol) and ethyl

(E)-4-(diethoxyphosphoryl)but-2-enoate (CAS No: 42516-28-9, 11.7 g, 46.9 mmol) in tetrahydrofuran (100 mL) was added lithium hydroxide (1.35 g, 56.3 mmol) and the reaction mixture was heated at 80°C for 2h. The reaction mixture was diluted with water (50 mL), resulting in a precipitate, which was filtered and dried to obtain crude compound. The crude material was purified by combi-flash column chromatography by eluting with 30% EtOAc in heptane to afford tert-butyl 4-((1E, 3E)-5-ethoxy-5-oxopenta-1, 3-dien-1-yl) piperidine-1-carboxylate **(Intermediate 135,** 10.0 g, 70%) as a yellow liquid. Mass spec: m/z: 209.9 $[M+H-100]^+$.

**Step 2**

**Intermediate 136: tert-butyl 4-(5-ethoxy-5-oxopentyl) piperidine-1-carboxylate**

To a solution of tert-butyl 4-((1E,3E)-5-ethoxy-5-oxopenta-1,3-dien-1-yl)piperidine-1-carboxylate **(Intermediate 135,** 12.00 g, 40.62 mmol) in methanol (120 mL) was added 10% Pd/C (3.2 g, 30 mmol) at room temperature and the reaction mixture was stirred for 6h under $H_2$ atmosphere at 60 psi. The reaction mixture was filtered through celite and concentrated in *vacuo.* The crude material was purified by combi-flash column chromatography by eluting with 30% EtOAc in heptane to afford tert-butyl 4-(5-ethoxy-5-oxopentyl) piperidine-1-carboxylate **(Intermediate 136,** 9.6 g, 79%) as a colorless liquid. $^1H$ NMR (400 MHz, DMSO-$d_6$) δ ppm 0.87-0.90 (m, 2H), 1.15-1.20 (m, 6H), 1.23-1.31 (m, 1H), 1.38 (s, 9H), 1.46-1.53 (m, 2H), 1.58-1.61 (m, 2H), 2.27 (t, *J*=7.23 Hz, 2H), 2.62-2.65 (m, 2H), 3.89-3.91 (m, 2H), 4.01-4.06 (m, 2H). Mass spec: m/z: 214.5 $[M+H-100]^+$.

**Step 3**

**Intermediate 137: tert-butyl 4-(5-hydroxypentyl)piperidine-1-carboxylate**

To a solution of tert-butyl 4-(5-methoxy-5-oxopentyl) piperidine-1-carboxylate **(Intermediate 136,** 3.80 g, 13 mmol) in tetrahydrofuran (70 mL) was added lithium borohydride (2.50 g, 100 mmol) slowly at 0°C and the reaction stirred for 30 min. The reaction mixture was then stirred at room temperature for 2h. The reaction mixture was quenched with cold water (50 mL) and extracted with ethyl acetate (2 × 100 mL). The combined organic phases were washed with water (200 mL) and brine solution (100 mL), dried over $Na_2SO_4$ and concentrated *in vacuo.* The obtained crude was purified by combi-flash column chromatography by eluting with 75% ethyl acetate in heptane to afford tert-butyl 4-(5-hydroxypentyl) piperidine-1-carboxylate **(Intermediate 137,** 3.1 g, 90%) as a liquid. $^1H$ NMR (400 MHz, DMSO-$d_6$) δ ppm 0.86-0.99 (m, 4H), 1.15-1.19 (m, 1H), 1.38 (s, 9H), 1.58-1.62 (m, 4H), 1.90-1.98 (m, 1H), 2.24-2.28 (m, 1H), 3.37 (t, *J*=6.36 Hz, 2H), 3.66-3.70 (m, 2H), 3.88-3.92 (m, 4H), 4.01-4.06 (m, 1H). Mass spec: m/z: 272.2 $[M+H]^+$.

**Step 4**

**Intermediate 138: tert-butyl 4-(5-bromopentyl)piperidine-1-carboxylate**

To a solution of tert-butyl 4-(5-hydroxypentyl)piperidine-1-carboxylate **(Intermediate 137,** 2.50 g, 9.2 mmol) in dichloromethane (25 mL) was added triphenylphosphine (3.0 g, 11 mmol) followed by carbon tetrabromide (5.0 g, 15 mmol) at 0°C and the reaction mixture was stirred at room temperature for 2h. The reaction mixture was diluted with water (50 mL) and extracted with DCM (2 × 100 mL). The combined organic phases were washed with water (2 × 60 mL) and brine solution (2 × 60 mL), dried over $Na_2SO_4$ and concentrated *in vacuo.* The crude material was purified by combi-flash column chromatography by eluting with 35% EtOAc in heptane to afford tert-butyl 4-(5-bromopentyl) piperidine-1-carboxylate **(Intermediate 138,** 1.80 g, 58%) as a liquid. Mass spec: m/z: 278.08 $[M-56]^+$.

**Step 5**

**Intermediate 139: methyl 1-(5-(1-(tert-butoxycarbonyl)piperidin-4-yl)pentyl)-3-methyl-1*H*-indazole-6-carboxylate**

To a solution of methyl 3-methyl-1*H*-indazole-6-carboxylate **(Intermediate 21,** 1.0 g, 5.0 mmol) in acetonitrile (25 mL) was added cesium carbonate (4.0 g, 10.0 mmol) followed by tert-butyl 4-(5-bromopentyl)piperidine-1-carboxylate **(Intermediate 138,** 2.0 g, 6.0 mmol) and the reaction mixture was heated at 60°C for 16h. The reaction mixture was quenched with cold water (50 mL) and extracted with ethyl acetate (2 × 100 mL). The combined organic layers were washed with water (200 mL) and brine solution (100 mL), dried over $Na_2SO_4$ and concentrated *in vacuo* to obtain crude compound. Purification by combi-flash chromatography by eluting with 30% ethyl acetate in heptane to afforded methyl 1-(5-(1-(tert-butoxycarbonyl)piperidin-4-yl)pentyl)-3-methyl-1*H*-indazole-6-carboxylate **(Intermediate 139,** 1.6 g, 70%)

as a yellow liquid. $^1$H NMR (400 MHz, DMSO-$d_6$) δ ppm 0.83-0.93 (m, 2H), 1.08-1.17 (m, 2H), 1.17-1.27 (m, 5H), 1.37 (s, 9H), 1.53-1.56 (m, 2H), 1.76-1.83 (m, 2H), 2.51 (s, 3H), 2.56-2.62 (m, 2H), 3.83-3.86 (m, 2H), 3.90 (s, 3H), 4.40-4.42 (m, 2H), 7.65 (d, *J*=8.31 Hz, 1H), 7.81 (d, *J*=8.31 Hz, 1H), 8.22 (s, 1H). Mass spec: m/z: 444.2 $[M+H]^+$.

**Step 6**

**Intermediate 140: 1-(5-(1-(tert-butoxycarbonyl) piperidin-4-yl) pentyl)-3-methyl-1*H*-indazole-6-carboxylic acid**

To a solution of methyl 1-(5-(1-(tert-butoxycarbonyl)piperidin-4-yl) pentyl)-3-methyl-1*H*-indazole-6-carboxylate **(Intermediate 139,** 1.5 g, 3.4 mmol) in tetrahydrofuran (10 mL) and methanol (5 mL) was added lithium hydroxide (0.25 g, 10.0 mmol) in water (5 mL) at 0°C and the reaction mixture was stirred at room temperature for 24h. The reaction mixture was concentrated *in vacuo.* The reaction mixture was then diluted with water (50 mL), adjusted to pH ~4 with 1N HCl, resulting in a precipitate, which was filtered and dried to afford 1-(5-(1-(tert-butoxycarbonyl)piperidin-4-yl) pentyl)-3-methyl-1*H*-indazole-6-carboxylic acid **(Intermediate 140,** 1.3 g, 89%) as a yellow liquid, which was used in the next step without further purification. $^1$H NMR (400 MHz, DMSO-$d_6$) δ ppm 1.39 (s, 9H), 1.89-1.92 (m, 5H), 2.30-2.33 (m, 2H), 2.51 (s, 3H), 2.56-2.60 (m, 3H), 2.63-2.70 (m, 2H), 3.12-3.16 (m, 2H), 3.49-3.51 (m, 2H), 3.77-3.80 (m, 2H), 4.57 (t, *J*=4.80 Hz, 2H), 7.64 (d, *J*=8.52 Hz, 1H), 7.77 (d, *J*=8.40 Hz, 1H), 8.22 (s, 1H).

**Step 7**

**Intermediate 141: tert-butyl 4-(5-(6-carbamoyl-3-methyl-1*H*-indazol-1-yl) pentyl) piperidine-1-carboxylate**

To a solution of 1-(5-(1-(tert-butoxycarbonyl)piperidin-4-yl)pentyl)-3-methyl-1*H*-indazole-6-carboxylic acid **(Intermediate 140,** 1.3 g, 3.0 mmol) in dimethylformamide (10 mL) was added HATU (1.8 g, 4.5 mmol) and the reaction mixture was stirred at room temperature for 15 min. Ammonium chloride (0.81 g, 15.0 mmol) and DIPEA (2.6 mL, 15.0 mmol) were added and the reaction and stirred at room temperature for 12h. The reaction mixture was diluted with water (50 mL) and extracted with EtOAc (3 × 50 mL). The combined organic phases were dried over $Na_2SO_4$ and concentrated *in vacuo.* The crude material was purified by combi-flash column chromatography by eluting with 50% EtOAc in heptane to afford tert-butyl 4-(5-(6-carbamoyl-3-methyl-1*H*-indazol-1-yl) pentyl) piperidine-1-carboxylate **(Intermediate 141,** 1.1 g, 85%) as a yellow solid. $^1$H NMR (400 MHz, DMSO-$d_6$) δ ppm 0.84-0.92 (m, 2H), 1.11-1.13 (m, 2H), 1.12-1.26 (m, 6H), 1.37 (s, 9H), 1.53-1.57 (m, 2H), 1.79-1.82 (m, 2H), 2.50 (s, 3H), 2.60-2.68 (m, 2H), 3.85-3.89 (m, 2H), 4.31-4.35 (m, 2H), 7.42 (br s, 1H), 7.59 (d, *J*=8.40 Hz, 1H), 7.73 (d, *J*=8.40 Hz, 1H), 8.03 (br s, 1H), 8.12 (s, 1H). Mass spec: m/z: 427.2 $[M-H]^-$.

**Step 8**

**Intermediate 142: tert-butyl-(R)-6-(1-(5-(1-(tert-butoxycarbonyl)piperidin-4-yl)pentyl)-3-methyl-1H-indazole-6-carboxamido)-2-(1-methylpyrrolidin-2-yl)-1H-pyrrolo[3,2-c]pyridine-1-carboxylate**

To a solution of tert-butyl 4-(5-(6-carbamoyl-3-methyl-1*H*-indazol-1-yl)pentyl)piperidine-1-carboxylate **(Intermediate 141,** 0.4 g, 0.93 mmol) n 1,4-dioxane (5 mL) was added tert-butyl (R)-6-bromo-2-(1-methylpyrrolidin-2-yl)-1*H*-pyrrolo[3,2-c]pyridine-1-carboxylate **(Intermediate 1,** 0.35 g, 0.93 mmol) followed by cesium carbonate (1.0 g, 3.08 mmol) and the reaction mixture was purged with argon for 10 min. $Pd_2(dba_3$ (1.32 g, 1.40 mmol) and Xantphos (0.13 g, 0.14 mmol) were added and the reaction mixture was further purged with argon for 10 min. and the reaction mixture heated at 120°C for 16h. The reaction mixture was diluted with water (20 mL) and extracted with EtOAc (3 × 25 mL). The combined organic phases were dried over $Na_2SO_4$ and concentrated *in vacuo.* The crude material was purified by combi-flash column chromatography by eluting with 80% EtOAc in heptane to afford tert-butyl (R)-6-(1-(5-(1-(tert-butoxycarbonyl)piperidin-4-yl)pentyl)-3-methyl-1H-indazole-6-carboxamido)-2-(1-methylpyrrolidin-2-yl)-1H-pyrrolo[3,2-c]pyridine-1-carboxylate **(Intermediate 142,** 0.4 g, 66%) as a yellow solid. Mass spec: m/z: 626.3 $[M+H-100]^+$.

**Step 9**

**Example 29: 1-(5-(1-(2-(2,6-dioxopiperidin-3-yl)-1,3-dioxoisoindolin-4-yl)piperidin-4-yl)pentyl)-3-methyl-*N*-(2-((R)-1-methylpyrrolidin-2-yl)-1*H*-pyrrolo[3,2-c]pyridin-6-yl)-1*H*-indazole-6-carboxamide**

To a solution of tert-butyl (R)-6-(1-(5-(1-(tert-butoxycarbonyl)piperidin-4-yl)pentyl)-3-methyl-1H-indazole-6-carboxamido)-2-(1-methylpyrrolidin-2-yl)-1H-pyrrolo[3,2-c]pyridine-1-carboxylate **(Intermediate 142,** 0.33 g, 0.45 mmol) in 1,4-dioxane (10 mL), 4M HCl in 1,4-dioxane (5 mL) was added at 0°C and the reaction mixture was stirred at room temperature for 4h. The reaction mixture was concentrated *in vacuo* to afford as (R)-3-methyl-*N*-(2-(1-methylpyrrolidin-2-yl)-1*H*-pyrrolo[3,2-c]pyridin-6-yl)-1-(5-(piperidin-4-yl)pentyl)-1*H*-indazole-6-carboxamide dihydrochloride (1.35 g) as a white solid which was used for the next step without further purification. To (R)-3-methyl-*N*-(2-(1-methylpyrrolidin-2-yl)-1*H*-pyrrolo[3,2-c]pyridin-6-yl)-1-(5-(piperidin-4-yl)pentyl)-1*H*-indazole-6-carboxamide dihydrochloride (0.3 g, 0.56 mmol) in dimethyl sulfoxide (5 mL) was added 2-(2,6-dioxopiperidin-3-yl)-4-fluoroisoindoline-1,3-dione (CAS No: 835616-60-9, 0.16 g, 0.56 mmol) followed by *N,N*- DIPEA (0.22 g, 1.70 mmol) and the reaction mixture was heated at 120°C for 3h. The reaction mixture was diluted with water (50 mL), resulting in a precipitate, which was filtered and dried. The crude material was purified by reverse phase preparative HPLC (Method A) method to afford 1-(5-(1-(2-(2,6-dioxopiperidin-3-yl)-1,3-dioxoisoindolin-4-yl)piperidin-4-yl)pentyl)-3-methyl-*N*-(2-((*R*)-1-methylpyrrolidin-2-yl)-1H-pyrrolo[3,2-c]pyridin-6-yl)-1*H*-indazole-6-carboxamide **(Example 29,** 0.12 g, 30%) as a yellow solid. $^1$H NMR (400 MHz, DMSO-$d_6$) δ ppm 1.19-1.37(m, 9H), 1.70-1.74 (m, 2H), 1.78-1.94 (m, 5H), 2.00-2.02 (m, 1H), 2.11-2.15 (m, 1H), 2.17 (s, 3H), 2.25-2.28 (m, 1H), 2.52 (s, 3H), 2.56-2.60 (m, 2H), 2.74-2.79 (m, 2H), 2.85-2.92 (m, 1H), 3.12-3.17 (m, 1H), 3.33-3.35 (m, 1H), 3.60-3.63 (m, 2H), 4.42 (t, J=7.00 Hz, 2H), 5.04-5.09 (m, 1H), 6.40 (s, 1H), 7.22 (d, *J*=8.80 Hz, 1H), 7.29 (d, *J*=6.80 Hz, 1H), 7.59-7.63 (m, 1H), 7.72-7.76 (m, 1H), 7.77-7.81 (m, 1H), 8.26 (s, 1H), 8.46 (s, 1H), 8.52 (s, 1H), 10.54 (s, 1H), 11.06 (s, 1H), 11.38 (s, 1H). Mass spec: m/z: 784.4 [M+H]$^+$.

**Example 30 was synthesized following Scheme 29**

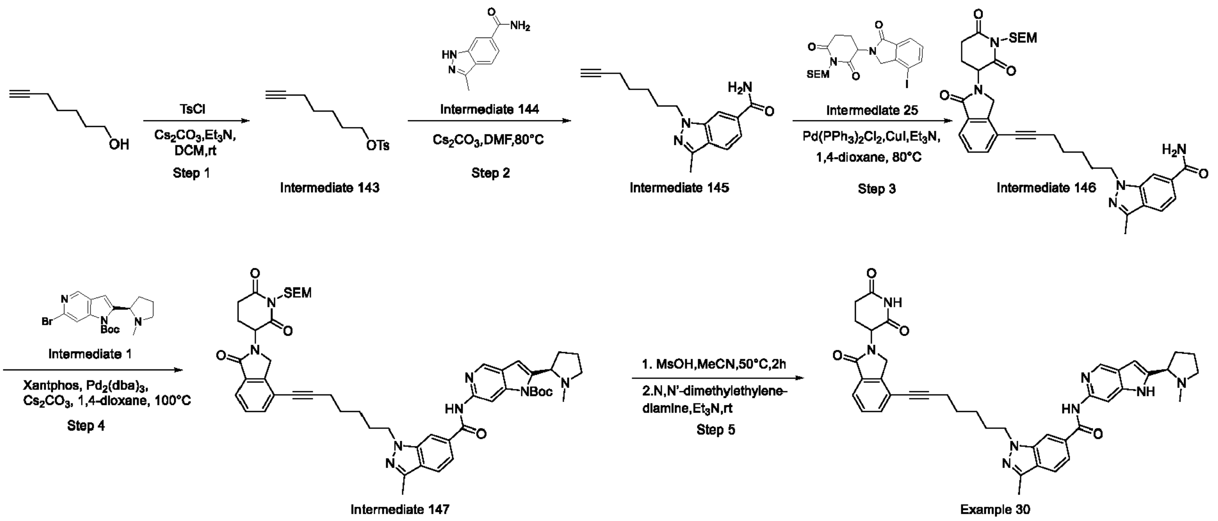


**Scheme 29**

**Step** 1

**Intermediate 143: hept-6-yn-1-yl 4-methylbenzenesulfonate**

To cooled (0°C) solution of hept-6-yn-1-ol (CAS No. 63478-76-2, 25 g, 222.88 mmol) in dichloromethane (250 mL) was added triethylamine (93.7 mL, 668.63 mmol) followed by cesium carbonate (218 g, 668.63 mmol) and p-toluenesulfonyl chloride (65.0 g, 334.31 mmol) and the reaction mixture was stirred at room temperature for 16h. The reaction mixture was diluted with water (200 mL) and extracted with dichloromethane 2 × 200 mL). The combined organic layers were dried over anhydrous $Na_2SO_4$ and concentrated *in vacuo.* The crude material was purified by combi-flash chromatography, by eluting with 30% ethyl acetate in heptane, to afford hept-6-yn-1-yl 4-methylbenzenesulfonate **(Intermediate 143,** 35 g, 59%) as a colourless liquid.$^1$H NMR (400 MHz, DMSO-$d_6$) δ ppm 1.29-1.33 (m, 4H), 1.51-1.59 (m, 2H), 2.06-2.09 (m, 2H), 2.42 (s, 3H), 2.74 (s, 1H), 4.00 (t, *J*=6.01 Hz, 2H), 7.48 (d, *J*=7.88 Hz, 2H), 7.78 (d, *J*=7.88 Hz, 2H). Mass spec: m/z 266.97 [M+H]$^+$.

**Step 2**

**Intermediate 145: 1-(hept-6-yn-1-yl)-3-methyl-1H indazole-6-carboxamide**

To a solution of hept-6-yn-1-yl 4-methylbenzenesulfonate **(Intermediate 143,** 7.00 g, 26.28 mmol) in DMF (70

mL) was added cesium carbonate (25.7 g, 78.83 mmol) followed by 3-methyl-1*H*-indazole-6-carboxamide **(Intermediate 144,** 4.60 g, 26.28 mmol) and the reaction mixture was heated at 80°C for 16h. The reaction mixture was diluted with water (100 mL) and extracted with ethyl acetate (3 × 100 mL). The combined organic layers were dried over anhydrous $Na_2SO_4$ and concentrated *in vacuo.* The crude material was purified by combi-flash chromatography, by eluting with 50% ethyl acetate in heptane, to afford 1-(hept-6-yn-1-yl)-3-methyl-1*H*-indazole-6-carboxamide **(Intermediate 145,** 2.5 g, 35%) as an off white solid. $^1H$ NMR (400 MHz, DMSO-$d_6$) δ ppm 1.31-1.35 (m, 2H), 1.45-1.49 (m, 2H), 1.80-1.84 (m, 2H), 2.11-2.17 (m, 2H), 2.51 (s, 3H), 2.71 (s, 1H), 4.34 (t, *J*=6.63 Hz, 2H), 7.41 (br s, 1H), 7.59 (d, *J*=8.71 Hz, 1H), 7.73 (d, *J*=8.29 Hz, 1H), 8.02 (br s, 1H), 8.12 (s, 1H). Mass spec: m/z 270.1 $[M+H]^+$.

**Step 3**

**Intermediate 146: 1-(7-(2-(2, 6-dioxo-1-((2-(trimethylsilyl) ethoxy) methyl) piperidin-3-yl)-1-oxoisoindolin-4-yl) hept-6-yn-1-yl)-3-methyl-1H-indazole-6-carboxamide**

To a solution of 1-(hept-6-yn-1-yl)-3-methyl-1*H*-indazole-6-carboxamide **(Intermediate 145, 2.00** g, 7.43 mmol) in 1,4-dioxane (20 mL) was added copper iodide (0.14 g, 0.74 mmol) and dichlorobis(triphenylphosphine)palladium(II) (2.69 g, 3.71 mmol) followed by 3-(4-iodo-1-oxoisoindolin-2-yl)-1-((2-(trimethylsilyl)ethoxy)methyl)piperidine-2,6-dione **(Intermediate 25, 3.72** g, 7.43 mmol) and triethylamine (5.20 mL, 37.13 mmol). The reaction mixture was stirred at room temperature for 2h then diluted with water (100 mL) and extracted with ethyl acetate (3 × 100mL). The combined organic layers were dried over anhydrous $Na_2SO_4$ and concentrated *in vacuo.* The crude material was purified by combi-flash chromatography, by eluting with 60% ethyl acetate in heptane, to afford 1-(7-(2-(2, 6-dioxo-1-((2-(trimethylsilyl) ethoxy) methyl) piperidin-3-yl)-1-oxoisoindolin-4-yl) hept-6-yn-1-yl)-3-methyl-1*H*-indazole-6-carboxamide **(Intermediate 146,** 2.50 g, 51%) as a yellow solid. $^1H$ NMR (400 MHz, DMSO-$d_6$) δ ppm -0.05 (s, 9H), 0.79-0.83 (m, 2H), 1.15-1.19 (m, 1H), 1.41-1.45 (m, 2H), 1.55-1.63 (m, 2H), 1.83-1.89 (m, 2H), 2.03-2.06 (m, 1H), 2.41-2.44 (m, 3H), 2.46 (s, 3H), 2.72-2.78 (m, 1H), 3.02-3.09 (m, 2H), 3.47-3.53 (m, 1H), 4.22-4.27 (m, 1H), 4.34-4.45 (m, 2H), 4.98-5.06 (m, 2H), 5.23-5.27 (m, 1H), 7.41 (br s, 1H), 7.46-7.61 (m, 3H), 7.71-7.73 (m, 2H), 8.02 (br s, 1H), 8.13 (s, 1H). Mass spec: m/z 640.2 $[M+H]^+$.

**Step 4**

**Intermediate 147: *tert*-butyl-6-(1-(7-(2-(2,6-dioxo-1-((2-(trimethylsilyl)ethoxy)methyl)piperidin-3-yl)-1-oxoisoindolin-4-yl)hept-6-yn-1-yl)-3-methyl-1*H*-indazole-6-carboxamido)-2-((R)-1-methylpyrrolidin-2-yl)-1*H*-pyrrolo[3,2-c]pyridine-1-carboxylate**

To a solution of 1-(7-(2-(2,6-dioxo-1-((2-(trimethylsilyl)ethoxy)methyl) piperidin-3-yl)-1-oxoisoindolin-4-yl) hept-6-yn-1-yl)-3-methyl-1*H*-indazole-6-carboxamide **(Intermediate 146,** 0.80 g, 1.24 mmol) in 1,4-dioxane (10 mL) was added cesium carbonate (1.22 g, 3.74 mmol) followed by *tert*-butyl-(*R*)-6-bromo-2-(1-methylpyrrolidin-2-yl)-1*H*-pyrrolo[3,2-c]pyridine-1-carboxylate **(Intermediate 1,** 0.47 g, 1.25 mmol) and the reaction mixture was purged with argon gas for 5 minutes. Dichloro[1,3-bis(2,6-di-3-pentylphenyl)imidazol-2-ylidene](3-chloropyridyl)palladium(II) (0.13 g, 0.16 mmol) and Xantphos (0.07 g, 0.12 mmol) were then added and the reaction mixture was again purged with argon gas for 5 minutes then heated at 100°C for 2h. The reaction mixture was concentrated *in vacuo* to obtain crude compound which was purified by combi-flash chromatography, by eluting with 80% ethyl acetate in heptane, to afford *tert*-butyl 6-(1-(7-(2-(2,6-dioxo-1-((2-(trimethylsilyl)ethoxy)methyl)piperidin-3-yl)-1-oxoisoindolin-4-yl)hept-6-yn-1-yl)-3-methyl-1*H*-indazole-6-carboxamido)-2-((R)-1-methylpyrrolidin-2-yl)-1*H*-pyrrolo[3,2-c]pyridine-1-carboxylate **(Intermediate 147,** 0.80 g, 62%) as an off white solid. $^1H$ NMR (400 MHz, DMSO-$d_6$) δ ppm -0.06 (s, 9H), 0.78-0.84 (m, 2H), 1.45-1.48 (m, 2H), 1.62-1.66 (m, 3H), 1.70 (s, 9H), 1.73-1.84 (m, 3H), 1.94-2.05 (m, 4H), 2.36 (s, 3H), 2.47-2.49 (s, 5H), 2.72-2.77 (m, 2H), 3.39-3.51 (m, 4H), 3.91-4.04 (m, 1H), 4.21-4.26 (m, 1H), 4.39-4.43 (m, 3H), 4.96-5.05 (m, 2H), 5.23-5.26 (m, 1H), 6.76 (s, 1H), 7.47-7.51 (m, 1H), 7.53 (d, J=7.60 Hz, 1H), 7.69-7.73(m, 2H), 7.77 (d, J=8.40 Hz, 1H), 8.45 (s, 1H), 8.60 (s, 1H), 8.97 (s, 1H), 10.73 (s, 1H).

**Step 5**

**Example 30: 1-(7-(2-(2, 6-dioxopiperidin-3-yl)-1-oxoisoindolin-4-yl) hept-6-yn-1-yl)-3-methyl-*N*-(2-((*R*)-1-methylpyrrolidin-2-yl)-1*H*-pyrrolo [3, 2-c] pyridin-6-yl)-1*H*-indazole-6-carboxamide**

To a solution of *tert*-butyl-6-(1-(7-(2-(2,6-dioxo-1-((2-(trimethylsilyl)ethoxy)methyl)piperidin-3-yl)-1-oxoisoindolin-4-yl)hept-6-yn-1-yl)-3-methyl-1*H*-indazole-6-carboxamido)-2-((*R*)-1-methylpyrrolidin-2-yl)-1*H*-pyrrolo[3,2-c]pyridine-1-carboxylate **(Intermediate 147,** 0.50 g, 0.53 mmol) in acetonitrile (15.0 mL) was added methanesulfonic acid (0.78 g, 7.96 mmol) and the reaction mixture was heated at 50°C for 2h. The reaction mixture was cooled to room temperature

then *N,N'*-dimethylethylenediamine (0.31 g, 3.18 mmol) followed by triethylamine (1.08 g, 10.6 mmol) were added and the reaction mixture was stirred at room temperature for 3h. The reaction mixture was concentrated *in vacuo* to obtain crude compound which was purified by preparative HPLC (Method A) to afford 1-(7-(2-(2, 6-dioxopiperidin-3-yl)-1-oxoisoindolin-4-yl) hept-6-yn-1-yl)-3-methyl-*N*-(2-((*R*)-1-methylpyrrolidin-2-yl)-1H-pyrrolo [3, 2-c] pyridin-6-yl)-1*H*-indazole-6-carboxamide **(Example 30,** 0.040 g, 11%) as an off white solid. $^{1}H$ NMR (400 MHz, DMSO-$d_6$) δ ppm 1.43-1.49 (m, 2H), 1.59-1.66 (m, 2H), 1.79-2.01 (m, 6H), 2.13-2.16 (m, 1H), 2.18 (s, 3H), 2.24-2.30 (m, 1H), 2.40-2.45 (m, 2H), 2.48 (s, 3H), 2.56-2.58 (m, 2H), 2.84-2.93 (m, 1H), 3.13-3.17 (m, 1H), 3.32-3.34 (m, 1H), 4.26-4.31 (m, 1H), 4.39-4.45 (m, 3H), 5.09-5.14 (m, 1H), 6.40 (s, 1H), 7.48-7.49 (m, 1H), 7.54-7.56 (m, 1H), 7.67-7.78 (m, 3H), 8.24 (s, 1H), 8.45 (s, 1H), 8.51 (s, 1H), 10.51 (s, 1H), 10.98 (s, 1H), 11.38 (s, 1H). Mass spec: m/z 711.2 $[M+H]^+$.

**Intermediate 144 was synthesised following Scheme 30**

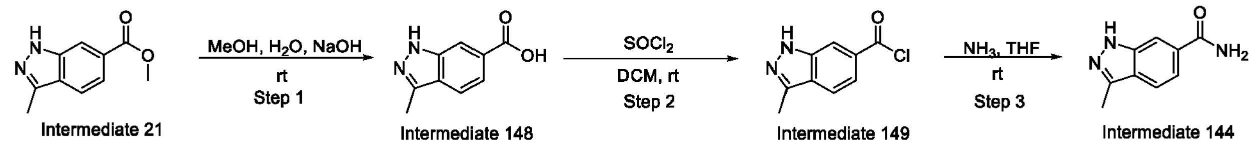


**Scheme 30**

**Step 1**

**Intermediate 148: 3-methyl-1H-indazole-6-carboxylic acid**

To a solution of methyl 3-methyl-1H-indazole-6-carboxylate **(Intermediate 21,** 2.00 g, 10.5 mmol) in MeOH (40.0 mL) was added $H_2O$ (40.0 mL) and NaOH (3.20 g, 80.0 mmol). The reaction was stirred at room temperature for 3h. The pH of the mixture was adjusted to 4-6 by the addition of HCl (1M) and the mixture was extracted with EtOAc (3 × 200 mL). The combined organic layers were washed with brin and dried over anhydrous $Na_2SO_4$, filtered and concentrated under reduced pressure to provide 3-methyl-1H-indazole-6-carboxylic acid **(Intermediate 148,** 1.90 g, 92.4%) as a yellow solid, which was used in the next step without further purificaiton. Mass spec: m/z: 177.0 $[M+H]^+$.

**Step 2**

**Intermediate 149: 3-methyl-1H-indazole-6-carbonyl chloride**

To a solution of 3-methyl-1H-indazole-6-carboxylic acid **(Intermediate 148,** 1.90 g, 10.8 mmol) in DCM (30.0 mL) was added thionyl chloride (30.0 mL). The reaction was stirred at room temperature for overnight and concentrated under reduced pressure to provide 3-methyl-1H-indazole-6-carbonyl chloride **(Intermediate 149,** 2.00 g, 76.4% ) as a white solid, which was used in the next step without further purification. Mass spec: m/z: 195.0 $[M+H]^+$.

**Step 3**

**Intermediate 144: 3-methyl-1H-indazole-6-carboxamide**

To a solution of ammonia (0.5M in THF, 60 mL) was added 3-methyl-1H-indazole-6-carbonyl chloride **(Intermediate 149,** 2.00 g, 10.3 mmol) at 0 °C and the reaction was stirred at room temperature for 5h and concentrated under reduced pressure to afford 3-methyl-1H-indazole-6-carboxamide **(Intermediate 144,** 900 mg, 62.6%) as a white solid. Mass spec: m/z: 176.0 $[M+H]^+$.

**Example 31 was synthesized following Scheme 31**

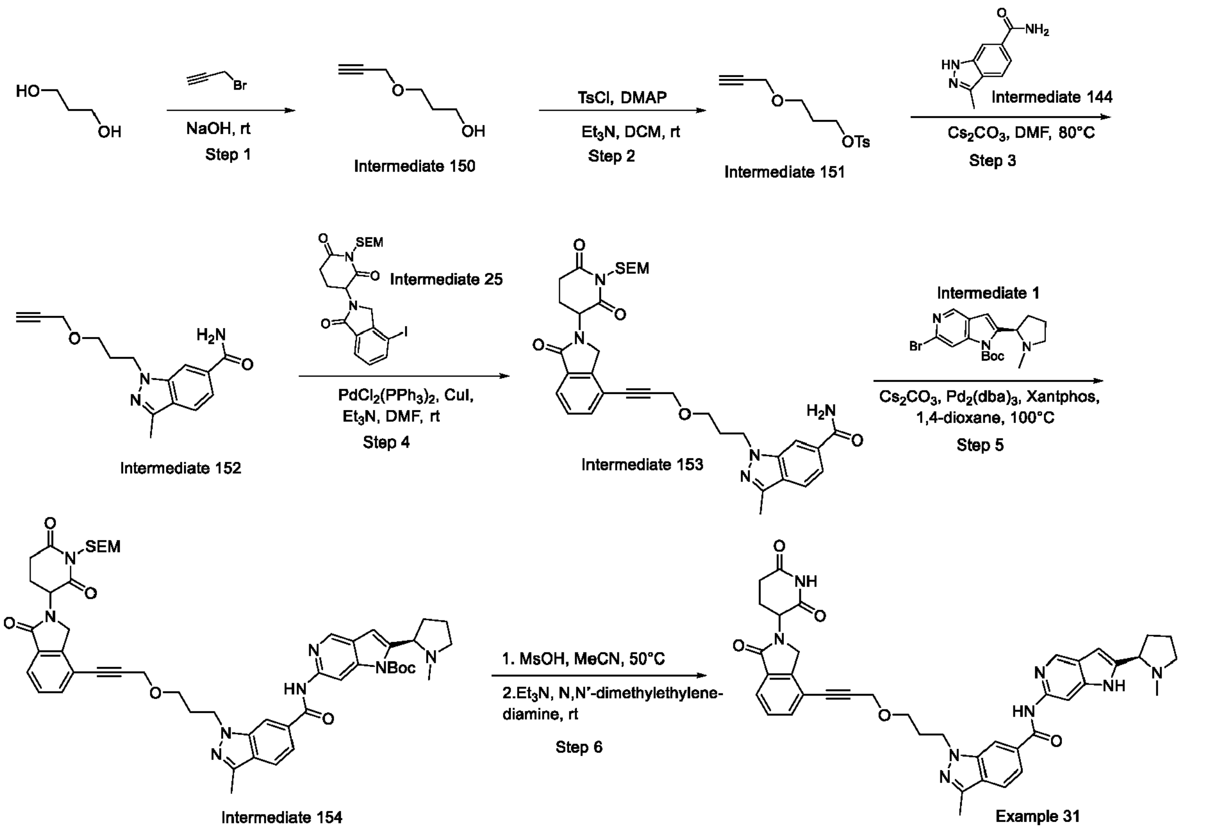


## Scheme 31

Step 1

**Intermediate 150: 3-(prop-2-yn-1-yloxy) propan-1-ol**

To a mixture of propane-1, 3-diol (CAS No: 504-63-2, 102.3 g, 1345.0 mmol) and 3-bromoprop-1-yne (CAS No: 106-96-7, 63.7 mL, 672.49 mmol) was added sodium hydroxide (32.60 g, 806.99 mmol) at 0°C. The reaction mixture was stirred at room temperature for 2h. After completion, the reaction mixture was filtered, washed with dichloromethane (2 × 100 mL) and the filtrate was concentrated *in vacuo.* The crude material was purified by combi-flash chromatography, by eluting with 50% ethyl acetate in heptane, to afford 3-(prop-2-yn-1-yloxy) propan-1-ol **(Intermediate 150,** 30.0 g, 39%) as a pale yellow oil. $^1$H NMR (400 MHz, $CDCl_3$) δ ppm 1.84-1.90 (m, 2H), 2.44 (t, *J*=2.38 Hz, 1H), 3.71 (t, *J*=5.54 Hz, 2H), 3.78 (t, *J*=5.55 Hz, 2H), 4.16 (d, *J*=2.38 Hz, 2H).

**Step 2**

**Intermediate 151: 3-(prop-2-yn-1-yloxy) propyl 4-methylbenzenesulfonate**

To a solution of 3-(prop-2-yn-1-yloxy) propan-1-ol **(Intermediate 150,** 25.0 g, 219.0 mmol) in dichloromethane (250 mL) were added triethylamine (66.49 g, 657.09 mmol) and 4-dimethylaminopyridine (2.82 g, 21.90 mmol) at 0°C. To the reaction mixture p-toluene sulfonyl chloride (62.63 g, 328.54 mmol) was added at 0°C and stirred at room temperature for 16h. The reaction mixture was diluted with water (500 mL) and extracted with dichloromethane (3 × 250 mL). The organic layer was separated, dried over anhydrous $Na_2SO_4$, and filtered and concentrated *in vacuo* to afford 3-(prop-2-yn-1-yloxy) propyl 4-methylbenzenesulfonate **(Intermediate 151,** 37.0 g, 63%) as a yellow liquid, which was used for the next step without further purification. $^1$H NMR (400 MHz, DMSO-$d_6$) δ ppm 1.80-1.82 (m, 2H), 2.42 (s, 3H), 3.35-3.46 (m, 3H), 3.98-4.02 (m, 2H), 4.04-4.06 (m, 2H), 7.48 (d, *J*=7.20 Hz, 2H), 7.79 (d, *J*=7.20 Hz, 2H).

**Step 3**

**Intermediate 152: 3-methyl-1-(3-(prop-2-yn-1-yloxy)propyl)-1*H*-indazole-6-carboxamide**

To a solution of 3-(prop-2-yn-1-yloxy) propyl 4-methylbenzenesulfonate **(Intermediate 151,** 4.00 g, 14.91 mmol) in dimethylformamide (50 mL) was added 3-methyl-1*H*-indazole-6-carboxamide **(Intermediate 144,** 2.61 g, 14.91 mmol) followed by cesium carbonate (15.34 g, 44.73 mmol) at room temperature. The reaction mixture was heated at 80°C for 2h. The reaction mixture was diluted with water (500 mL) and extracted with dichloromethane (3 × 500 mL). The organic layer was separated, dried over anhydrous $Na_2SO_4$, filtered, and concentrated *in vacuo* to obtain the crude compound as a mixture of regioisomers. The regioisomers were separated by combi-flash chromatography, by eluting with 30% ethyl acetate in heptane, to afford the desired regioisomer 3-methyl-1-(3-(prop-2-yn-1-yloxy) propyl)-1*H*-indazole-6-carboxamide **(Intermediate 152,** 2.40 g, 59%) as a white solid. $^1$H NMR (400 MHz, $CDCl_3$) δ ppm 2.12-2.24 (m, 2H), 2.34-2.38 (m, 1H), 2.53 (s, 3H), 3.38-3.46 (m, 2H), 4.05-4.12 (m, 2H), 4.42-4.48 (m, 2H), 7.48 (d, J=7.20 Hz, 1H), 7.68 (d, J=7.20 Hz, 1H), 7.96 (s, 1H). Mass spec m/z 272.0 $[M+H]^+$.

Step 4

**Intermediate 153: 1-(3-((3-(2-(2, 6-dioxo-1-((2-(trimethylsilyl) ethoxy) methyl) piperidin-3-yl)-1-oxoisoindolin-4-yl) prop-2-yn-1-yl) oxy) propyl)-3-methyl-1*H*-indazole-6-carboxamide**

To a solution of 3-methyl-1-(3-(prop-2-yn-1-yloxy) propyl)-1*H*-indazole-6-carboxamide **(Intermediate 152,** 2.40 g, 8.8 mmol) and 3-(4-iodo-1-oxoisoindolin-2-yl)-1-((2-(trimethylsilyl)ethoxy)methyl)piperidine-2,6-dione **(Intermediate 25,** 4.4 g, 8.8 mmol) in dimethylformamide (30 mL) was added triethylamine (34.0 g, 320.0 mmol). The reaction mixture was purged with argon gas for 15 min then $PdCl_2(PPh_3)_2$ (0.62 g, 0.88 mmol) and copper (I) iodide (0.17 g, 0.88 mmol) were added. The reaction mixture was purged with argon gas for 10 min and stirred at room temperature for 2h. The reaction mixture was diluted with water (1000 mL) and extracted with dichloromethane (3 × 500 mL). The organic layer was separated, dried over anhydrous $Na_2SO_4$, filtered, and concentrated *in vacuo.* The crude material was purified by combi-flash chromatography, by eluting with 90% ethyl acetate in heptane, to afford 1-(3-((3-(2-(2, 6-dioxo-1-((2-(trimethylsilyl) ethoxy) methyl) piperidin-3-yl)-1-oxoisoindolin-4-yl) prop-2-yn-1-yl) oxy) propyl)-3-methyl-1*H*-indazole-6-carboxamide **(Intermediate 153,** 2.00 g, 35%) as a yellow solid. $^1$H NMR (400 MHz, DMSO-$d_6$) δ ppm -0.05 (s, 9H), 0.79-0.84 (m, 2H), 1.98-2.17 (m, 4H), 2.46 (s, 3H), 2.54-2.58 (m, 1H), 2.72-2.78 (m, 1H), 2.99-3.14 (m, 2H), 3.45-3.57 (m, 3H), 4.26-4.30 (m, 1H), 4.38-4.46 (m, 4H), 4.97-5.07 (m, 2H), 5.23-5.28 (m, 1H), 7.39 (br s, 1H), 7.53-7.61 (m, 2H), 7.64 (d, *J*=7.60 Hz, 1H), 7.68-7.72 (m, 1H), 7.77 (d, *J*=7.60 Hz, 1H), 8.02 (br s, 1H), 8.12 (s, 1H). Mass spec m/z 644.1 $[M+H]^+$.

**Step 5**

**Intermediate 154: *tert*-butyl 6-(1-(3-((3-(2-(2,6-dioxo-1-((2-(trimethylsilyl)ethoxy)methyl)piperidin-3-yl)-1-oxoisoindolin-4-yl)prop-2-yn-1-yl)oxy)propyl)-3-methyl-1*H*-indazole-6-carboxamido)-2-((*R*)-1-methylpyrrolidin-2-yl)-1*H*-pyrrolo[3,2-c]pyridine-1-carboxylate**

To a solution of 1-(3-((3-(2-(2, 6-dioxo-1-((2-(trimethylsilyl) ethoxy) methyl) piperidin-3-yl)-1-oxoisoindolin-4-yl) prop-2-yn-1-yl) oxy) propyl)-3-methyl-1*H*-indazole-6-carboxamide **(Intermediate 153,** 0.67 g, 1.05 mmol) in 1,4-dioxane (15 mL) was added *tert*-butyl(R)-6-bromo-2-(1-methylpyrrolidin-2-yl)-1*H*-pyrrolo[3,2-c]pyridine-1-carboxylate **(Intermediate 1,** 0.50 g, 1.31 mmol) followed by cesium carbonate (1.29 g, 3.94 mmol) at room temperature. The reaction mixture was purged with argon for 15 min followed by $Pd_2(dba)_3$ (0.18 g, 0.19 mmol) and Xantphos (0.23 g, 0.39 mmol). The reaction mixture was purged with argon for 10 min and heated at 100°C for 2h. The reaction mixture was filtered through celite bed and washed with ethyl acetate (100 mL). The filtrate was concentrated *in vacuo.* The crude material was purified by combi-flash column chromatography by eluting with 90% ethyl acetate in heptane to afford tert-butyl 6-(1-(3-((3-(2-(2,6-dioxo-1-((2-(trimethylsilyl)ethoxy)methyl)piperidin-3-yl)-1-oxoisoindolin-4-yl)prop-2-yn-1-yl)oxy)propyl)-3-methyl-1*H*-indazole-6-carboxamido)-2-((*R*)-1-methylpyrrolidin-2-yl)-1*H*-pyrrolo[3,2-c]pyridine-1-carboxylate **(Intermediate 154,** 0.60 g, 48%) as a yellow solid. $^1$H NMR (400 MHz, $CDCl_3$) δ ppm -0.04 (s, 9H), 0.85-0.95 (m, 2H), 1.61-1.71 (m, 2H), 1.76 (s, 9H), 2.05-2.19 (m, 2H), 2.21-2.32 (m, 2H), 2.34-2.40 (m, 2H), 2.43 (s, 3H), 2.57 (s, 3H), 2.79-2.98 (m, 2H), 3.18-3.22 (m, 1H), 3.46-3.50 (m, 2H), 3.55-3.63 (m, 2H), 3.97-4.01 (m, 1H), 4.23-4.41 (m, 4H), 4.50-4.54 (m, 2H), 5.12-5.20 (m, 2H), 5.28 (s, 2H), 6.73 (s, 1H), 7.34-7.38 (m, 1H), 7.46-7.48 (m, 1H), 7.56-7.62 (m, 1H), 7.66-7.70 (m, 1H), 7.75-7.81 (m, 1H), 8.07 (s, 1H), 8.78 (s, 1H), 9.02 (s, 1H). Mass spec m/z 943.2 $[M+H]^+$.

**Step 6**

**Example 31: 1-(3-((3-(2-(2,6-dioxopiperidin-3-yl)-1-oxoisoindolin-4-yl)prop-2-yn-1-yl)oxy)propyl)-3-methyl-*N*-(2-((*R*)-1-methylpyrrolidin-2-yl)-1*H*-pyrrolo[3,2-c]pyridin-6-yl)-1*H*-indazole-6-carboxamide**

To a solution of *tert*-butyl 6-(1-(3-((3-(2-(2,6-dioxo-1-((2-(trimethylsilyl)ethoxy)methyl)piperidin-3-yl)-1-oxoisoindolin-4-yl)prop-2-yn-1-yl)oxy)propyl)-3-methyl-1*H*-indazole-6-carboxamido)-2-((*R*)-1-methylpyrrolidin-2-yl)-1*H*-pyrrolo[3,2-c]pyridine-1-carboxylate **(Intermediate 154,** 0.60 g, 0.63 mmol) in acetonitrile (15 mL) was added methanesulfonic acid (0.42 mL, 6.36 mmol) at room temperature and the reaction was heated at 50°C for 2h. The reaction mixture was cooled to room temperature and *N*, *N*'-dimethylethylenediamine (0.38 mL, 3.18 mmol) and triethylamine (1.78 mL, 12.72 mmol) were added and the mixture stirred at room temperature for 3h. The reaction mixture was concentrated *in vacuo.* The crude residue was quenched with water (50 mL) to form a solid precipitate which was filtered and dried *in vacuo.* The crude material was purified by preparative HPLC (Method A) to afford 1-(3-((3-(2-(2,6-dioxopiperidin-3-yl)-1-oxoisoindolin-4-yl)prop-2-yn-1-yl)oxy)propyl)-3-methyl-*N*-(2-((*R*)-1-methylpyrrolidin-2-yl)-1*H*-pyrrolo[3,2-c]pyridin-6-yl)-1*H*-indazole-6-carboxamide **(Example 31,** 0.12 g, 26%) as an off white solid. $^1$H NMR (400 MHz, DMSO-$d_6$) δ ppm 1.76-2.02 (m, 4H), 2.13-2.16 (m, 2H), 2.18 (s, 3H), 2.19-2.22 (m, 1H), 2.23-2.31 (m, 1H), 2.38-2.42 (m, 2H), 2.52 (s, 3H), 2.81-2.93 (m, 1H), 3.13-3.18 (m, 1H), 3.32-3.37 (m, 1H), 3.54-3.57 (m, 2H), 4.26-4.34 (m, 1H), 4.38-4.40 (m, 1H), 4.43 (s, 2H), 4.44-4.53 (m, 2H), 5.08-5.13 (m, 1H), 6.40 (s, 1H), 7.46-7.54 (m, 1H), 7.60-7.64 (m, 1H), 7.68-7.78 (m, 3H), 8.24 (s, 1H), 8.42 (s, 1H), 8.51 (s, 1H), 10.54 (br s, 1H), 10.97 (br s, 1H), 11.37 (br s, 1H). Mass spec m/z 713.0 $[M+H]^+$.

**Example 32 was synthesized following Scheme 32**

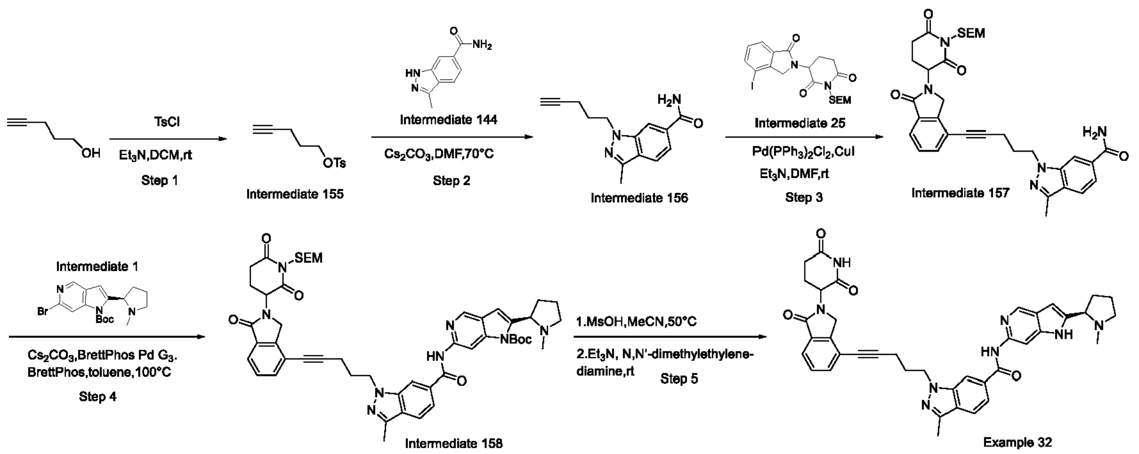


**Scheme 32**

**Step 1**

**Intermediate 155: pent-4-yn-1-yl-4-methylbenzenesulfonate**

To a cooled (0°C) solution of pent-4-yn-1-ol (CAS No: 5390-04-5, 5.00 g, 59.4 mmol) in dichloromethane (10 mL) was added triethylamine (16.7 mL, 119.0 mmol), 4-dimethylaminopyridine (0.67 mL, 29.7 mmol) and toluenesulfonyl chloride (17.3 g, 89.2 mmol) and the reaction mixture was stirred at room temperature for 16h. The reaction mixture was diluted with water (100 mL) and extracted with dichloromethane (3 × 100 mL). The combined organic layers were washed with $NaHCO_3$ (2 × 60 mL) and brine solution (80 mL), dried over anhydrous $Na_2SO_4$ and concentrated *in vacuo.* The crude material was purified by combi-flash column chromatography, by eluting with 20% ethyl acetate in heptane, to afford pent-4-yn-1-yl-4-methylbenzenesulfonate **(Intermediate 155,** 7.5 g, 53%) as a colorless oil. $^1$H NMR (400 MHz, DMSO-$d_6$) δ ppm 1.72-1.75 (m, 2H), 2.15-2.17 (m, 2H), 2.42 (s, 3H), 2.75 (s, 1H), 4.08 (t, *J*=5.60 Hz, 2H), 7.49 (d, *J*=7.05 Hz, 2H), 7.79 (d, *J*=7.46 Hz, 2H).

**Step 2**

**Intermediate 156: 3-methyl-1-(pent-4-yn-1-yl)-1*H*-indazole-6-carboxamide**

To a solution of pent-4-yn-1-yl-4-methylbenzenesulfonate **(Intermediate 155,** 3.00 g, 12.6 mmol) and 3-methyl-1*H*-indazole-6-carboxamide **(Intermediate 144,** 2.21 g, 12.6 mmol) in dimethylformamide (50 mL) was added cesium carbonate (12.3 g, 37.8 mmol) and the reaction mixture was heated at 70°C for 16h. The reaction mixture was diluted with water (100 mL) and extracted with ethyl acetate (2 × 100 mL). The combined organic layers were dried over anhydrous $Na_2SO_4$ and concentrated *in vacuo* to obtain crude compound as a mixture of regioisomers. The regioisomer were separated by combi-flash chromatography, by eluting with 50-55% ethyl acetate in heptane, to afford desired regioisomer 3-methyl-1-(pent-4-yn-1-yl)-1*H*-indazole-6-carboxamide **(Intermediate 156, 1.6** g, 53%) as a white solid. $^1$H NMR (400 MHz, DMSO-$d_6$) δ ppm 1.98-2.02 (m, 2H), 2.14-2.18 (m, 2H), 2.50 (s, 3H), 2.83 (s, 1H), 4.41 (t, *J*=6.84 Hz, 2H), 7.41 (br s, 1H), 7.60 (d, *J*=8.29 Hz, 1H), 7.74 (d, *J*=8.71 Hz, 1H), 8.03 (br s, 1H), 8.12 (s, 1H). Mass spec: m/z 242.0 [M+H]$^+$.

**Step 3**

**Intermediate 157: 1-(5-(2-(2, 6-dioxo-1-((2-(trimethylsilyl) ethoxy)methyl)piperidin-3-yl)-1-oxoisoindolin-4-yl) pent-4-yn-1-yl)-3-methyl-1H-indazole-6-carboxamide**

To a solution of 3-(4-iodo-1-oxoisoindolin-2-yl)-1-((2-(trimethylsilyl)ethoxy)methyl)piperidine-2,6-dione **(Intermediate 25,** 1.0 g, 4.14 mmol) in dimethylformamide (10 mL) was added copper(I) iodide (0.08 g, 0.41 mmol), $PdCl_2(PPh_3)_2$ (0.29 g, 0.41 mmol) and 3-methyl-1-(pent-4-yn-1-yl)-1*H*-indazole-6-carboxamide **(Intermediate 156,** 2.39 g, 4.14 mmol) followed by triethylamine (20.3 mL, 145.0 mmol). The reaction mixture was purged with argon for 5 min then stirred at room temperature for 2h. The reaction mixture was diluted with water (60 mL) and extracted with ethyl acetate (3 × 80 mL). The combined organic layers were dried over anhydrous $Na_2SO_4$ and concentrated *in vacuo.* The crude material was purified by combi-flash column chromatography, by eluting with 90% ethyl acetate in heptane, to afford 1-(5-(2-(2, 6-dioxo-1-((2-(trimethylsilyl) ethoxy)methyl)piperidin-3-yl)-1-oxoisoindolin-4-yl)pent-4-yn-1-yl)-3-methyl-1H-indazole-6-carboxamide **(Intermediate 157,** 1.30 g, 51%) as a white solid. $^1$H NMR (400 MHz, DMSO-$d_6$) δ ppm -0.07 (s, 9H), 0.79-0.81 (m, 2H), 2.07-2.15 (m, 3H), 2.38-2.42 (m, 3H), 2.47 (s, 3H), 2.70-2.91 (m, 1H), 2.99-3.08 (m, 1H), 3.47-3.52 (m, 2H), 4.23-4.27 (m, 1H), 4.42 (s, 2H), 4.47-4.51 (m, 1H), 4.99-5.07 (m, 2H), 5.23-5.28 (m, 1H), 7.41 (br s, 1H), 7.50-7.55 (m, 1H), 7.57-7.67 (m, 2H), 7.72-7.74 (m, 2H), 8.02 (br s, 1H), 8.17 (s, 1H). Mass spec: m/z 612.4 [M+H]$^+$.

**Step 4**

**Intermediate 158: *tert*-butyl-6-(1-(5-(2-(2,6-dioxo-1-((2-(trimethylsilyl)ethoxy)methyl)piperidin-3-yl)-1-oxoisoindolin-4-yl)pent-4-yn-1-yl)-3-methyl-1*H*-indazole-6-carboxamido)-2-((*R*)-1-methylpyrrolidin-2-yl)-1*H*-pyrrolo[3,2-c]pyridine-1-carboxylate**

To a solution of 1-(5-(2-(2,6-dioxo-1-((2-(trimethylsilyl)ethoxymethyl)piperidin-3-yl)-1-oxoisoindolin-4-yl)pent-4-yn-1-yl)-3-methyl-1*H*-indazole-6-carboxamide **(Intermediate 157,** 0.6 g, 0.97 mmol) and *tert*-butyl-(*R*)-6-bromo-2-(1-methylpyrrolidin-2-yl)-1*H*-pyrrolo[3,2-c]pyridine-1-carboxylate **(Intermediate 1,** 0.44 g, 1.17 mmol) in toluene (12 mL) was added cesium carbonate (0.95 g, 2.93 mmol). The reaction mixture was purged with argon for 10 min, then BrettPhos (0.08, 0.1466 mmol) and BrettPhos Pd G3 (0.13 g, 0.1466 mmol) were added and the reaction mixture was purged with argon for a further 10 min. The reaction mixture was heated at 100°C for 3h then diluted with water (80 mL) and extracted with ethyl acetate (2 × 80 mL). The combined organic layers were dried over anhydrous $Na_2SO_4$ and concentrated *in vacuo.* The crude material was purified by combi-flash column chromatography, by eluting with 2% MeOH in dichloromethane, to afford *tert*-butyl 6-(1-(5-(2-(2,6-dioxo-1-((2-(trimethylsilyl)ethoxy)methyl)piperidin-3-yl)-1-oxoisoindolin-4-yl)pent-4-yn-1-yl)-3-methyl-1*H*-indazole-6-carboxamido)-2-((*R*)-1-methylpyrrolidin-2-yl)-1*H*-pyrrolo[3,2-c]pyridine-1-carboxylate **(Intermediate 158,** 0.41 g, 46%) as an off white solid. $^1$H NMR (400 MHz, DMSO-$d_6$) δ ppm -0.08 (s, 9H), 0.71-0.79 (m, 2H), 0.81-0.85 (m, 1H), 1.15-1.19 (m, 1H), 1.21-1.23 (m, 1H), 1.62-1.64 (m, 2H), 1.69 (s, 9H), 1.74-1.77 (m, 2H), 1.98-2.03 (m, 2H), 2.15-2.21 (m, 2H), 2.31 (s, 3H), 2.36 (s, 3H), 2.69-2.73 (m, 1H), 2.97-3.06 (m, 1H), 3.11-3.15 (m, 1H), 3.45-3.50 (m, 2H), 3.91-3.94 (m, 1H), 4.20-4.25 (m, 1H), 4.38-4.42 (m, 1H), 4.56 (t, J=6.22 Hz, 2H), 4.95-5.03 (m, 2H), 5.21-5.23 (m, 1H), 6.77 (s, 1H), 7.42-7.49 (m, 1H), 7.60-7.80 (m, 4H), 8.44 (s, 1H), 8.60 (s, 1H), 8.97 (s, 1H), 10.79 (s, 1H). Mass spec: m/z 913.1 [M+H]$^+$.

**Step 5**

**Example 32: 1-(5-(2-(2,6-dioxopiperidin-3-yl)-1-oxoisoindolin-4-yl)pent-4-yn-1-yl)-3-methyl-*N*-(2-((*R*)-1-methylpyrrolidin-2-yl)-1*H*-pyrrolo[3,2-c]pyridin-6-yl)-1*H*-indazole-6-carboxamide**

To a solution of *tert*-butyl 6-(1-(5-(2-(2,6-dioxo-1-((2-(trimethylsilyl)ethoxy)methyl)piperidin-3-yl)-1-oxoisoindolin-4-yl)pent-4-yn-1-yl)-3-methyl-1*H*-indazole-6-carboxamido)-2-((*R*)-1-methylpyrrolidin-2-yl)-1H-pyrrolo[3,2-c]pyridine-1-carboxylate **(Intermediate 158,** 0.38 g, 0.41 mmol) in acetonitrile (10 mL) was added methanesulfonic acid (0.41

mL, 6.24 mmol) and the reaction mixture was heated at 50°C for 2h. The reaction mixture was cooled to room temperature and triethylamine (0.87 mL, 6.24 mmol) and *N*, *N'*-dimethylethylenediamine (0.29 mL, 2.49 mmol) added, then the mixture stirred at room temperature for 2h. The reaction mixture was concentrated *in vacuo* and the crude material was purified by preparative HPLC (Method A) to afford 1-(5-(2-(2,6-dioxopiperidin-3-yl)-1-oxoisoindolin-4-yl)pent-4-yn-1-yl)-3-methyl-*N*-(2-((R)-1-methylpyrrolidin-2-yl)-1*H*-pyrrolo[3,2-c]pyridin-6-yl)-1*H*-indazole-6-carboxamide **(Example 32,** 0.11 g, 39%) as an off white solid. $^1$H NMR (400 MHz, DMSO-$d_6$) δ ppm 1.79-1.99 (m, 4H), 2.17-2.28 (m, 7H), 2.49 (s, 3H), 2.53 (s, 3H), 2.55-2.57 (m, 1H), 2.82-2.91 (m, 1H), 3.15-3.17 (m, 1H), 3.31-3.34 (m, 1H), 4.25-4.29 (m, 1H), 4.37-4.42 (m, 1H), 4.57 (t, *J*=6.60 Hz, 2H), 5.08-5.13 (m, 1H), 6.41 (s, 1H), 7.45 (t, *J*=7.64 Hz, 1H), 7.61 (d, *J*=7.58, 1H), 7.66 (d, *J*=7.28 Hz, 1H), 7.70-7.77 (m, 2H), 8.24 (s, 1H), 8.44 (s, 1H), 8.51 (s, 1H), 10.57 (s, 1H), 10.98 (br s, 1H), 11.39 (s, 1H). Mass spec: m/z 683.2 [M+H]$^+$.

**Example 33 was synthesized following Scheme 33**

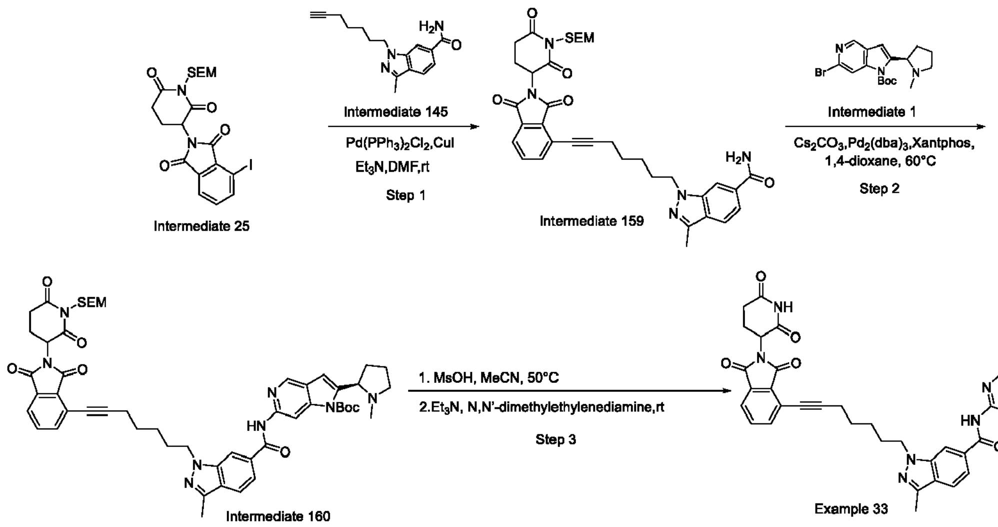


**Scheme 33**

**Step 1**

**Intermediate 159: 1-(7-(2-(2,6-dioxo-1-((2-(trimethylsilyl)ethoxy)methyl)piperidin-3-yl)-1,3-dioxoisoindolin-4-yl) hept-6-yn-1-yl)-3-methyl-1*H*-indazole-6-carboxamide**

To a solution of 2-(2,6-dioxo-1-((2-(trimethylsilyl)ethoxymethyl)piperidin-3-yl)-4-iodoisoindoline-1,3-dione **(Intermediate 25,** 1.34 g, 2.59 mmol) in dimethylformamide (3 mL) was added 1-(hept-6-yn-1-yl)-3-methyl-1*H*-indazole-6-carboxamide **(Intermediate 145,** 0.70 g, 2.59 mmol) followed by triethylamine (9.29 g, 90.98 mmol) and the reaction mixture was purged with argon gas for 10 min. $PdCl_2(PPh_3)_2$ (0.18 g, 0.26 mmol) and copper (I) iodide (0.05 g, 0.25 mmol) were added and the mixture was stirred at room temperature for 2h. The reaction mixture was filtered through celite bed, the filtrate was diluted with water (30 mL) and extracted with ethyl acetate (3 × 40 mL). The combined organic layers were washed with brine solution (2 × 50 mL), dried over anhydrous $Na_2SO_4$ and concentrated *in vacuo.* The crude material was purified by combi-flash chromatography, by eluting with 65% ethyl acetate in heptane, to obtain 1-(7-(2-(2,6-dioxo-1-((2-(trimethylsilyl)ethoxy)methyl) piperidin-3-yl)-1,3-dioxoisoindolin-4-yl)hept-6-yn-1-yl)-3-methyl-1*H*-indazole-6-carboxamide **(Intermediate 159,** 0.75 g, 45%) as a white solid. $^1$H NMR (400 MHz, DMSO-$d_6$) δ ppm -0.04 (s, 9H), 0.80-0.84 (m, 2H), 1.44-1.46 (m, 2H), 1.60-1.62 (m, 2H), 1.86-1.88 (m, 2H), 2.03-2.13 (m, 2H), 2.48 (s, 3H), 2.73-2.89 (m, 2H), 2.95-3.08 (m, 2H), 3.44-3.54 (m, 2H), 4.35-4.37 (m, 2H), 5.06 (s, 2H), 5.25-5.27 (m, 1H), 7.41 (br s, 1H), 7.52-7.66 (m, 2H), 7.72 (d, J=7.89 Hz, 1H), 7.79-7.89 (m, 2H), 8.03 (br s, 1H), 8.14 (s, 1H). Mass spec: m/z 656.4 [M+H]$^+$.

**Step 2**

**Intermediate 160: *tert*-butyl-6-(1-(7-(2-(2,6-dioxo-1-((2-(trimethylsilyl)ethoxy)methyl)piperidin-3-yl)-1,3-dioxoisoindolin-4-yl)hept-6-yn-1-yl)-3-methyl-1*H*-indazole-6-carboxamido)-2-((*R*)-1-methylpyrrolidin-2-yl)-1*H*-pyrrolo[3,2-c]pyridine-1-carboxylate**

To a solution of 1-(7-(2-(2, 6-dioxo-1-((2-(trimethylsilyl) ethoxy)methyl)piperidin-3-yl)-1,3-dioxoisoindolin-4-yl)hept-6-yn-1-yl)-3-methyl-1*H*-indazole-6-carboxamide **(Intermediate 159, 0.55** g, 0.84 mmol) and tert-butyl-(*R*)-6-bromo-2-(1-methylpyrrolidin-2-yl)-1*H*-pyrrolo[3,2-c]pyridine-1-carboxylate **(Intermediate 1,** 0.40 g, 1.05 mmol) in 1,4-dioxane (12 mL) was added cesium carbonate (0.68 g, 2.10 mmol) and the reaction mixture was purged with argon gas for 10 min. $Pd_2(dba)_3$ (0.15 g, 0.15 mmol) and Xantphos (0.18 g, 0.31 mmol) were then added and reaction mixture was purged again with argon gas for 5 min then heated at 60°C for 2h. The reaction mixture was diluted with water (70 mL) and extracted with ethyl acetate (3 × 70 mL). The combined organic layers were washed with brine solution (2 × 60 mL), dried over anhydrous $Na_2SO_4$ and concentrated *in vacuo.* The crude material was purified by combi-flash chromatography, by eluting with 95% ethyl acetate in heptane, to afford *tert*-butyl-6-(1-(7-(2-(2,6-dioxo-1-((2-(trimethylsilyl)ethoxy)methyl)piperidin-3-yl)-1,3-dioxoisoindolin-4-yl)hept-6-yn-1-yl)-3-methyl-1*H*-indazole-6-carboxamido)-2-((R)-1-methylpyrrolidin-2-yl)-1*H*-pyrrolo[3,2-c]pyridine-1-carboxylate **(Intermediate 160,** 0.55 g, 55%) as a liquid. Mass spec: m/z 955.2 $[M+H]^+$.

**Step 3**

**Example 33: 1-(7-(2-(2,6-dioxopiperidin-3-yl)-1,3-dioxoisoindolin-4-yl)hept-6-yn-1-yl)-3-methyl-N-(2-((R)-1-methylpyrrolidin-2-yl)-1*H*-pyrrolo[3,2-c]pyridin-6-yl)-1*H*-indazole-6-carboxamide**

To a solution of *tert*-butyl-6-(1-(7-(2-(2,6-dioxo-1-((2-(trimethylsilyl)ethoxy)methyl)piperidin-3-yl)-1,3-dioxoisoindolin-4-yl)hept-6-yn-1-yl)-3-methyl-1*H*-indazole-6-carboxamido)-2-((R)-1-methylpyrrolidin-2-yl)-1*H*-pyrrolo[3,2-c]pyridine-1-carboxylate **(Intermediate 160,** 0.55 g, 0.57 mmol) in acetonitrile (5 mL) was added methanesulfonic acid (0.28 g, 2.87 mmol) and the reaction was heated at 50°C for 2h. The reaction mixture was cooled to room temperature then *N, N'*-dimethylethylenediamine (0.31 g, 3.45 mmol) followed by triethylamine (5.00 g, 49 mmol) were added and the reaction mixture stirred at room temperature for 3h. The reaction mixture was concentrated *in vacuo* and the crude material was purified by preparative HPLC (Method A) to afford 1-(7-(2-(2,6-dioxopiperidin-3-yl)-1,3-dioxoisoindolin-4-yl)hept-6-yn-1-yl)-3-methyl-*N*-(2-((*R*)-1-methylpyrrolidin-2-yl)-1*H*-pyrrolo[3,2-c]pyridin-6-yl)-1*H*-indazole-6-carboxamide **(Example 33,** 0.10 g, 24%) as an off white solid. $^1H$ NMR (400 MHz, DMSO-$d_6$) δ ppm 1.48-1.54 (m, 2H), 1.62-1.69 (m, 2H), 1.79-1.97 (m, 6H), 2.02-2.06 (m, 1H), 2.13-2.16 (m, 1H), 2.18 (s, 3H), 2.24-2.30 (m, 1H), 2.52 (s, 3H), 2.53-2.55 (m, 2H), 2.82-2.91 (m, 1H), 3.13-3.17 (m, 1H), 3.32-3.36 (m, 2H), 4.44 (t, *J*=6.94 Hz, 2H), 5.09-5.14 (m, 1H), 6.40 (s, 1H), 7.70-7.73 (m, 2H), 7.76-7.80 (m, 2H), 7.82-7.85 (m, 1H), 8.24 (s, 1H), 8.45 (s, 1H), 8.51 (s, 1H), 10.51 (s, 1H), 11.11 (s, 1H), 11.38 (s, 1H). Mass spec: m/z 725.2 $[M+H]^+$.

**Example 34 was synthesized following Scheme 34**

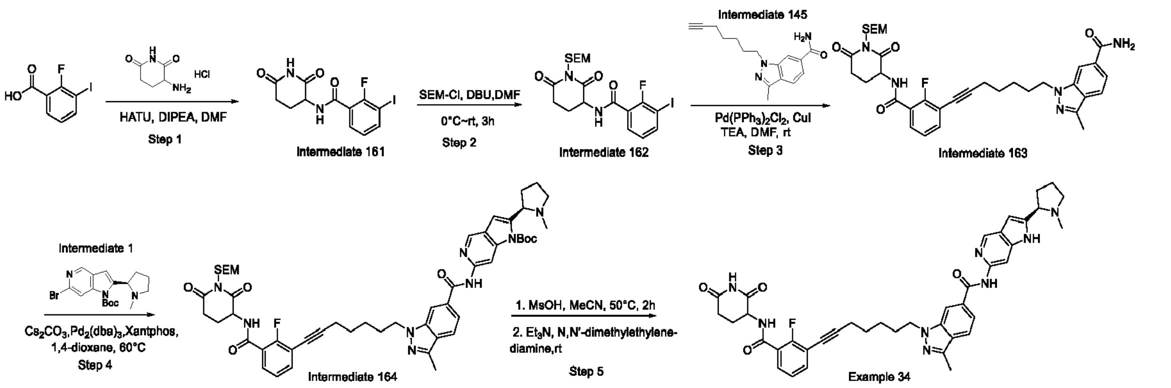


Scheme 34

**Step 1**

**Intermediate 161: *N*-(2,6-dioxopiperidin-3-yl)-2-fluoro-3-iodobenzamide**

To a solution of 2-fluoro-3-iodobenzoic acid (CAS No: 447464-03-1, 3.0 g, 11.27 mmol) in dimethylformamide (20 mL) were added DIPEA (5.94 g, 45.11 mmol) and HATU (6.77 g, 16.91 mmol). The reaction was stirred at room temperature for 15 min then 3-aminopiperidine-2,6-dione hydrochloride (CAS No: 24666-56-6, 2.22 g, 13.53 mmol) was added and reaction mixture was stirred at room temperature for a further 16 h. The reaction was quenched with water (500 mL) and extracted with EtOAc (3 × 250 mL). The combined organic layers were over anhydrous $Na_2SO_4$ and concentrated *in vacuo* to afford N-(2,6-dioxopiperidin-3-yl)-2-fluoro-3-iodobenzamide **(Intermediate 161,** 3.90 g) as a yellow solid which was used for the next step without further purification. $^1$H NMR (400 MHz, DMSO-$d_6$) δ ppm 1.97-2.13 (m, 2H), 2.50-2.52 (m, 1H), 2.71-2.88 (m, 1H), 4.70-4.83 (m, 1H), 7.11 (t, *J*=8.17 Hz, 1H), 7.61 (t, *J*=8.10 Hz, 1H), 7.98 (t, *J*=6.63 Hz, 1H), 8.72 (d, *J*=8.05 Hz, 1H), 10.86 (br s, 1H). Mass spec: m/z 376.8 $[M+H]^+$.

**Step 2**

**Intermediate 162: *N*-(2,6-dioxo-1-((2-(trimethylsilyl)ethoxy)methyl)piperidin-3-yl)-2-fluoro-3-iodobenzamide**

To a cooled (0°C) solution of *N*-(2,6-dioxopiperidin-3-yl)-2-fluoro-3-iodobenzamide **(Intermediate 161,** 1.50 g, 4 mmol) in DMF (10 mL) was added 1,8-diazabicyclo[5.4.0]undec-7-ene (1.21 g, 8 mmol) and the reaction mixture was stirred for 10 min, followed by the addition of 2-(trimethylsilyl)ethoxymethyl chloride (1.33 g, 8 mmol). The reaction mixture was allowed to warm to room temperature and stirred for 12h. The reaction was diluted with water (40 mL) and extracted with ethyl acetate (3 × 50 mL). The combined organic layers were washed with brine (2 × 50 mL), dried over anhydrous $Na_2SO_4$, filtered and concentrated *in vacuo.* The crude material was purified by combi-flash column chromatography, by eluting with 65% ethyl acetate in heptane, to afford *N*-(2,6-dioxo-1-((2-(trimethylsilyl)ethoxy)methyl)piperidin-3-yl)-2-fluoro-3-iodobenzamide **(Intermediate 162,** 1.5 g, 74%) as a viscous liquid. $^1$H NMR (400 MHz, DMSO-$d_6$) δ ppm -0.02 (s, 9 H), 0.84-0.86 (m, 2H), 2.02-2.12 (m, 2H), 2.70-2.74 (m, 1H), 2.87-3.10 (m, 1H), 3.52-3.54 (m, 2H), 4.87-4.89 (m, 1H), 4.99-5.15 (m, 2H), 7.11-7.12 (m, 1H), 7.61 (s, 1H), 7.92-8.03 (m, 1H), 8.84 (d, *J*=7.45 Hz, 1H).

**Step 3**

**Intermediate 163: 1-(7-(3-((2,6-dioxo-1-((2-(trimethylsilyl)ethoxy)methyl)piperidin-3-yl)carbamoyl)-2-fluorophenyl)hept-6-yn-1-yl)-3-methyl-1*H*-indazole-6-carboxamide**

To a solution of *N*-(2,6-dioxo-1-((2-(trimethylsilyl)ethoxy)methyl)piperidin-3-yl)-2-fluoro-3-iodobenzamide **(Intermediate 162,** 1.5 g, 3.71 mmol) and 1-(hept-6-yn-1-yl)-3-methyl-1*H*-indazole-6-carboxamide **Intermediate 145** (0.99 g, 3.71 mmol) in dimethylformamide (10 mL) was added triethylamine (13.28 g, 130.10 mmol), $PdCl_2(PPh_3)_2$ (0.26 g, 0.27 mmol) and copper (I) iodide (0.07 g, 0.37 mmol). The reaction mixture was stirred at room temperature for 2h, then diluted with water (100 mL) and extracted with ethyl acetate (3 × 100 mL). The combined organic layers were dried over anhydrous $Na_2SO_4$, filtered and concentrated *in vacuo.* The crude material was purified by combi-flash column chromatography, by eluting with 95% ethyl acetate in heptane, to afford 1-(7-(3-((2,6-dioxo-1-((2-(trimethylsilyl) ethoxy)methyl)piperidin-3-yl)carbamoyl)-2-fluorophenyl)hept-6-yn-1-yl)-3-methyl-1H-indazole-6-carboxamide **(Intermediate 163,** 1.40 g, 59%) as a yellow sticky liquid. $^1$H NMR (400 MHz, DMSO-$d_6$) δ ppm -0.11-0.06 (m, 9H), 0.79-0.88 (m, 1H), 1.15-1.19 (m, 1H), 1.41-1.43 (m, 2H), 1.56-1.60 (m, 1H), 1.56-1.60 (m, 1H), 1.87-1.90 (m, 2H), 1.98-2.02 (m, 1H), 2.34 (s, 3H), 2.43-2.48 (m, 1H), 2.71-2.75 (m, 1H), 3.17-3.20 (m, 2H), 3.50-3.56 (m, 2H), 4.02-4.08 (m, 1H), 4.35-4.39 (m, 2H), 4.83-4.84 (m, 2H), 5.03-5.10 (m, 1H), 7.19-7.22 (m, 1H), 7.31 (m, 2 H), 7.40 (s, 1H), 7.59 (d, *J*=8.29 Hz, 1H), 7.72 (d, *J*=8.29 Hz, 1H), 8.02 (s, 1H), 8.14 (s, 1H), 8.71 (d, *J*=8.29 Hz, 1H).

**Step 4**

**Intermediate 164: tert-butyl-6-(1-(7-(3-((2,6-dioxo-1-((2-(trimethylsilyl)ethoxy)methyl)piperidin-3-yl)carbamoyl)-2-fluorophenyl)hept-6-yn-1-yl)-3-methyl-1H-indazole-6-carboxamido)-2-((R)-1-methylpyrrolidin-2-yl)-1H pyrrolo[3,2-c]pyridine-1-carboxylate**

To a solution of 1-(7-(3-((2,6-dioxo-1-((2-(trimethylsilyl)ethoxy)methyl)piperidin-3-yl)carbamoyl)-2-fluorophenyl)hept-6-yn-1-yl)-3-methyl-1H-indazole-6-carboxamide **(Intermediate 163,** 1.36 g, 2.10 mmol) and *tert*-butyl-(*R*)-6-bromo-2-(1-methylpyrrolidin-2-yl)-1H-pyrrolo[3,2-c]pyridine-1-carboxylate **(Intermediate 1,** 1.0 g, 2.63 mmol) in 1,4-dioxane (30 mL) was added cesium carbonate (1.72 g, 5.25 mmol). The mixture was purged with argon gas for 20 min, then Xantphos (0.47 g, 0.78 mmol) and $Pd_2(dba)_3$ (0.37 g, 0.39 mmol) were added and the reaction mixture was stirred at 60°C for 2h. The reaction mixture was diluted with water (70 mL) and extracted with ethyl acetate (3 × 70 mL). The combined

organic layers were washed with brine (2 × 60 mL), dried over anhydrous $Na_2SO_4$ and concentrated *in vacuo.* The crude material was purified by combi-flash column chromatography, by eluting with 95% ethyl acetate in heptane, to afford tert-butyl-6-(1-(7-(3-((2,6-dioxo-1-((2-(trimethylsilyl)ethoxy)methyl)piperidin-3-yl)carbamoyl)-2-fluorophenyl)hept-6-yn-1-yl)-3-methyl-1*H*-indazole-6-carboxamido)-2-((*R*)-1-methylpyrrolidin-2-yl)-1H-pyrrolo[3,2-c]pyridine-1-carboxylate (**Intermediate 164**, 0.55 g, 22%) as an off-white solid. $^1H$ NMR (400 MHz, DMSO-$d_6$) δ ppm -0.14-0.01 (m, 9H), 0.72-0.90 (m, 2H), 1.06-1.29 (m, 2H), 1.36-1.53 (m, 2H), 1.57-1.79 (m, 14H), 1.70 (s, 3H), 1.87-1.96 (m, 4H), 2.31-2.44 (m, 4H), 2.28 (s, 3H), 3.13-3.22 (m, 1H), 3.27-3.29 (m, 2H), 3.42-3.61 (m, 1H), 3.88-3.97 (m, 1H), 4.03 (m, 1 H), 4.34-4.55 (m, 2H), 4.97-5.12 (m, 1H), 6.76 (s, 1H), 7.13-7.45 (m, 1H), 7.16-7.30 (m, 1H), 7.30-7.41 (m, 1H), 7.44 (d, J=8.88 Hz, 1 H), 7.47-7.52 (m, 1H) 7.54-7.59 (m, 1H), 7.69-7.74 (m, 1H), 7.74-7.81 (m, 1H), 8.61 (s, 1H), 8.95 (br s, 1H).

**Step 5**

**Example 34: 1-(7-(3-((2,6-dioxopiperidin-3-yl)carbamoyl)-2-fluorophenyl)hept-6-yn-1-yl)-3-methyl-*N*-(2-((*R*)-1-methylpyrrolidin-2-yl)-1*H*-pyrrolo[3,2-*c*]pyridin-6-yl)-1*H*-indazole-6-carboxamide**

To a solution of *tert*-butyl-6-(1-(7-(3-((2,6-dioxo-1-((2-(trimethylsilyl)ethoxy)methyl)piperidin-3-yl)carbamoyl)-2-fluorophenyl)hept-6-yn-1-yl)-3-methyl-1H-indazole-6-carboxamido)-2-((*R*)-1-methylpyrrolidin-2-yl)-1H-pyrrolo[3,2-c] pyridine-1-carboxylate (**Intermediate 164**, 0.55 g, 0.58 mmol) in acetonitrile (10 mL) was added methanesulfonic acid (0.56 g, 5.80 mmol) and the mixture heated at 50°C for 2h. The reaction mixture was then cooled to room temperature, then N,N'-dimethylethylenediamine (0.28 g, 2.90 mmol) followed by triethylamine (1.18 g, 11.61 mmol) were added and the reaction was stirred at room temperature for 3h. The reaction was concentrated *in vacuo* and the crude compound was purified by preparative HPLC (Method A) to afford 1-(7-(3-((2,6-dioxopiperidin-3-yl)carbamoyl)-2-fluorophenyl)hept-6-yn-1-yl)-3-methyl-N-(2-((*R*)-1-methylpyrrolidin-2-yl)-1H-pyrrolo[3,2-c]pyridin-6-yl)-1H-indazole-6-carboxamide (**Example 34**, 0.095 g, 23%) as an off-white solid. 400 MHz, DMSO-$d_6$) δ ppm 1.41-1.52 (m, 2H), 1.62-1.67 (m, 2H), 1.78-1.96 (m, 5H), 1.98-2.10 (m, 2H), 2.12-2.16 (m, 1H), 2.18 (s, 3H), 2.22-2.32 (m, 1H), 2.45-2.48 (m, 3H), 2.49 (s, 3H), 2.68-2.87 (m, 2H), 3.15 (t, J=8.13 Hz, 1H), 4.43-4.45 (m, 2H), 4.72-4.80 (m, 1 H), 6.40 (s, 1H), 7.23 (t, J=7.69 Hz, 1H), 7.47-7.58 (m, 2H), 7.73 (d, J=8.17 Hz, 1H), 7.78 (d, J=8.65 Hz, 1 H), 8.25 (s, 1 H), 8.45 (s, 1H), 8.52 (s, 1H), 8.67 (d, J=7.88 Hz, 1H), 10.53 (s, 1 H), 10.86 (s, 1H), 11.38 (s, 1H). Mass spec: m/z 717.2 $[M+H]^+$.

**Example 35 was synthesized following Scheme 35**

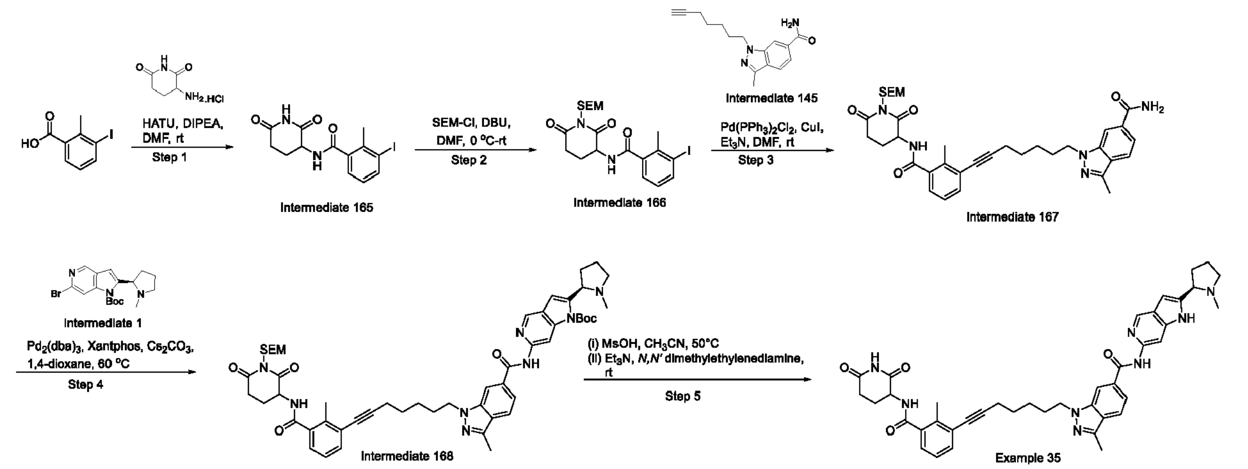


**Scheme 35**

**Step 1**

**Intermediate 165: *N*-(2,6-dioxopiperidin-3-yl)-3-iodo-2-methylbenzamide**

To a solution of 3-iodo-2-methyl-benzoic acid (CAS No. 133232-56-1, 5.0 g, 19.08 mmol) and 3-aminopiperidine-2,6-dione hydrochloride (CAS No: 24666-56-6, 3.14 g, 19.08 mmol) in dimethylformamide (50 mL) was added HATU (11.45 g, 28.62 mmol) and DIPEA (7.55 g, 57.24 mmol) and the reaction mixture was stirred at room temperature for 16h.

The reaction mixture was diluted with water (50 mL) and extracted with ethyl acetate (3 × 100 mL). The combined organic layers were washed with brine, dried over anhydrous $Na_2SO_4$ and concentrated *in vacuo* to afford *N*-(2,6-dioxopiperidin-3-yl)-3-iodo-2-methylbenzamide (**Intermediate 165**, 5.0 g, crude) as a white solid which was used for the next step without further purification. $^1$H NMR (400 MHz, DMSO-$d_6$) δ ppm 2.01-2.08 (m, 2H), 2.43 (s, 3H), 2.51-2.55 (m, 1H), 2.73-2.76 (m, 1H), 4.68-4.74 (m, 1H), 7.02 (t, *J*=7.2 Hz, 1H). 7.33 (d, *J*=7.2 Hz, 1H), 7.92 (d, *J*=7.60 Hz, 1H), 8.67 (d, *J*=8.4 Hz, 1H), 10.84 (s, 1H). Mass spec: m/z 372.8 $[M+H]^+$.

**Step 2**

**Intermediate 166: *N*-(2,6-dioxo-1-((2-(trimethylsilyl)ethoxy)methyl)piperidin-3-yl)-3-iodo-2-methylbenzamide**

To a cooled (0°C) solution of *N*-(2,6-dioxo-3-piperidyl)-3-iodo-5-methyl-benzamide (**Intermediate 165**, 2.5 g, 6.7 mmol) and 1,8-diazabicyclo[5.4.0]undec-7-ene (2.10 g, 13 mmol) in dimethylformamide (10 mL) was added 2-(trimethylsilyl)ethoxymethyl chloride (2.40 g, 13 mmol) and the reaction mixture was stirred at room temperature for 2h. The mixture was diluted with water (50 mL) and extracted with ethyl acetate (3 × 60 mL). The combined organic layers were washed with brine (2 × 60 mL), dried over anhydrous $Na_2SO_4$ and concentrated *in vacuo.* The crude material was purified by combi-flash column chromatography, by eluting with 60-65% ethyl acetate in hexane, to afford *N*-(2,6-dioxo-1-((2-(trimethylsilyl)ethoxy)methyl)piperidin-3-yl)-3-iodo-2-methylbenzamide (**Intermediate 166**, 2.70 g, 80%) as a light yellow sticky compound. $^1$H NMR (500 MHz, DMSO-$d_6$) δ ppm -0.18 (s, 9H), 0.84 (t, *J*=7.6 Hz, 2H), 1.99-2.11 (m, 2H), 2.45 (s, 3H), 2.71-2.75 (m, 1H), 2.89-2.95 (m, 1H), 3.52-3.55 (m, 2H), 4.82-4.87 (m,1H), 5.03-5.09 (m, 2H), 7.03 (t, *J*=8.0 Hz, 1H), 7.35 (d, *J*=7.6 Hz, 1H), 7.93 (d, *J*=8.0 Hz, 1H), 8.79 (d, *J*=8.4 Hz, 1H). Mass spec: m/z 500.9 $[M-H]^+$.

**Step 3**

**Intermediate 167: 1-(7-(3-((2,6-dioxo-1-((2-(trimethylsilyl)ethoxy)methyl)piperidin-3-yl)carbamoyl)-2-methylphenyl)hept-6-yn-1-yl)-3-methyl-1*H*-indazole-6-carboxamide**

To a solution of *N*-(2,6-dioxo-1-((2-(trimethylsilyl)ethoxy)methyl)piperidin-3-yl)-3-iodo-2-methylbenzamide (**Intermediate 166**, 1.87 g, 3.71 mmol) and 1-hept-6-ynyl-3-methyl-indazole-6-carboxamide (**Intermediate 145**, 1.0 g, 3.71 mmol) in dimethylformamide (10 mL) was added triethylamine (13.28 g,130.0 mmol) and the reaction mixture was purged with argon for 5 min. $Pd(PPh_3)Cl_2$ (266 mg, 0.37 mmol) and copper(I) iodide (72 mg, 0.37 mmol) were then added and the reaction mixture was purged with argon for a further 5 min, then stirred at room temperature for 2h. The, mixture was filtered through celite bed and the filtrate was diluted with water (100 mL) and extracted with ethyl acetate (3 × 100 mL). The combined organic layers were washed with brine (2 × 60 mL), dried over anhydrous $Na_2SO_4$ and concentrated *in vacuo.* The crude product was purified by combi-flash column chromatography, by eluting with 90% ethyl acetate in heptane, to afford 1-(7-(3-((2,6-dioxo-1-((2-(trimethylsilyl)ethoxy)methyl)piperidin-3-yl)carbamoyl)-2-methylphenyl)hept-6-yn-1-yl)-3-methyl-1*H*-indazole-6-carboxamide (**Intermediate 167**, 1.4 g, 59%) as a yellow sticky solid. $^1$H NMR (500 MHz, DMSO-$d_6$) δ ppm -0.24 (s, 9H), 0.83 (t, *J*=8.4 Hz, 2H), 1.18 (t, *J*=6.80 Hz, 1H), 1.41-1.43 (m, 2H), 1.56-160 (m, 2H), 1.88 (t, *J*=7.60 Hz, 2H), 2.00 (s, 3H), 2.34 (s, 3H), 2.43-2.47 (m, 1H), 2.71-2.76 (m, 1H), 2.91-2.95 (m, 1H), 3.06-3.17 (m, 1H), 3.51-3.56 (m, 2H), 4.02-4.09 (m, 1H), 4.37 (t, *J*=6.80 Hz, 2H), 4.60-4.83 (m, 1H), 5.03-5.07 (m, 2H), 7.21 (t, *J*=7.2 Hz, 1H), 7.28-7.33 (m, 2H), 7.40 (br s, 1H), 7.58 (d, *J*=8.0 Hz, 1H), 7.72 (d, *J*=8.0 Hz, 1H), 8.02 (br s, 1H), 8.14 (s, 1H), 8.70 (d, *J*=8.40 Hz, 1H).

**Step 4**

**Intermediate 168: *tert*-butyl-6-(1-(7-(3-((2,6-dioxo-1-((2-(trimethylsilyl)ethoxy)methyl)piperidin-3-yl)carbamoyl)-2-methylphenyl)hept-6-yn-1-yl)-3-methyl-1*H*-indazole-6-carboxamido)-2-((*R*)-1-methylpyrrolidin-2-yl)-*1*H-pyrrolo[3,2-c]pyridine-1-carboxylate**

To a solution of 1-(7-(3-((2,6-dioxo-1-((2-(trimethylsilyl)ethoxy)methyl)piperidin-3-yl)carbamoyl)-2-methylphenyl)hept-6-yn-1-yl)-3-methyl-1*H*-indazole-6-carboxamide **(Intermediate 167,** 1.35 g, 2.10 mmol) and *tert*-butyl-(*R*)-6-bromo-2-(1-methylpyrrolidin-2-yl)-1*H*-pyrrolo[3,2-c]pyridine-1-carboxylate (**Intermediate 1**, 1.0 g, 2.63 mmol) in 1,4-dioxane (40 mL) was added cesium carbonate (1.72 g, 5.29 mmol). The resulting mixture was degassed with argon for 20 min, then $Pd_2(dba)_3$ (370 mg, 0.39 mmol) and Xantphos (470 mg, 0.78 mmol) were added and the reaction mixture was degassed again with argon for 10 min, then heated at 60°C for 2h. The mixture was diluted with water (70 mL) and extracted with ethyl acetate (3 × 70 mL). The combined organic layers were washed with brine (2 × 60 mL), dried over anhydrous $Na_2SO_4$ and concentrated *in vacuo.* The crude product was purified by combi-flash column chromatography, by eluting with 90% ethyl acetate in hexane, to afford *tert*-butyl-6-(1-(7-(3-((2,6-dioxo-1-((2-(trimethylsilyl)ethoxy)methyl)piperidin-3-yl)carbamoyl)-2-methylphenyl)hept-6-yn-1-yl)-3-methyl-1*H*-indazole-6-carboxamido)-2-((*R*)-1-methylpyrrolidin-2-yl)-1*H*-pyrrolo[3,2-c]pyridine-1-carboxylate (**Intermediate 168**, 550 mg, 22%) as a white solid. $^1$H NMR (400 MHz, DMSO-$d_6$) δ ppm -0.37 (s, 9H), 0.82 (t, *J*=8.0 Hz, 2H), 1.08 (t, *J*=6.8 Hz, 1H), 1.36-1.46 (m, 2H), 1.61-1.65 (m, 4H), 1.70 (s, 9H), 1.71-1.75 (m, 1H), 2.01 (s, 3H), 2.09-2.14 (m, 2H), 2.24-2.27 (m, 1H), 2.28 (s, 3H), 2.34 (s, 3H), 2.67-2.75 (m, 2H), 2.91-2.94 (m, 2H), 3.13-3.25 (m, 2H), 3.37-3.39 (m, 1H), 3.53-3.55 (m, 2H), 3.93 (t, *J*=7.60 Hz, 1H), 4.43-4.49 (m, 2H), 4.70-4.83 (m, 2H), 5.03-5.09 (m, 1H), 6.76 (s, 1H), 7.19 (t, *J*=6.0 Hz, 1H), 7.27 (d, *J*=7.60 Hz, 1H), 7.33 (d, *J*=7.20 Hz, 1H), 7.72 (d, *J*=8.0 Hz, 1H), 7.78 (d, *J*=8.40 Hz, 1H), 8.45 (s, 1H), 8.61 (s, 1H), 8.69 (d, *J*=8.0 Hz, 1H), 8.97 (s, 1H), 10.74 (s, 1H). Mass spec: m/z 943.5 [M-H]$^+$.

**Step 5**

**Example 35: 1-(7-(3-((2,6-dioxopiperidin-3-yl)carbamoyl)-2-methylphenyl)hept-6-yn-1-yl)-3-methyl-*N*-(2-((*R*)-1-methylpyrrolidin-2-yl)-1*H*-pyrrolo[3,2-c]pyridin-6-yl)-1*H*-indazole-6-carboxamide**

To a solution of *tert*-butyl 6-(1-(7-(3-((2,6-dioxo-1-((2-(trimethylsilyl)ethoxy)methyl)piperidin-3-yl)carbamoyl)-2-methylphenyl)hept-6-yn-1-yl)-3-methyl-1*H*-indazole-6-carboxamido)-2-((*R*)-1-methylpyrrolidin-2-yl)-1*H*-pyrrolo[3,2-c] pyridine-1-carboxylate (**Intermediate 168**, 500 mg, 0.53 mmol) in acetonitrile (10 mL) was added methanesulfonic acid (519 mg, 5.30 mmol) and the reaction mixture was heated at 50°C for 1h. After cooling to room temperature, *N,N'*-dimethylethylenendiamine (259 mg, 2.65 mmol) followed by triethylamine (1.08 g, 10.60 mmol) were added and the reaction mixture was stirred at room temperature for 3h. The mixture was concentrated *in vacuo* to one third volume and then poured into ice-cold water (20 mL). The resultant precipitate was collected by filtration and dried *in vacuo.* The solid was purified by preparative HPLC (Method A) to afford 1-(7-(3-((2,6-dioxopiperidin-3-yl)carbamoyl)-2-methylphenyl) hept-6-yn-1-yl)-3-methyl-*N*-(2-((*R*)-1-methylpyrrolidin-2-yl)-1H-pyrrolo[3,2-c]pyridin-6-yl)-1*H*-indazole-6-carboxamide

(**Example 35**, 65 mg, 15%) as an off-white solid. $^1$H NMR (400 MHz, DMSO-$d_6$) δ ppm 1.45-1.59 (m, 2H), 1.61-1.78 (m, 2H), 1.86-1.91 (m, 9H), 2.17 (s, 3H), 2.28-2.31 (m, 1H), 2.46 (s, 3H), 2.52 (s, 3H), 2.53-2.56 (m, 1H), 2.74-2.78 (m, 1H), 3.15 (t, *J*=7.20 Hz, 1H), 3.31-3.35 (m, 1H), 3.36-3.45 (m, 1H), 4.40 (t, *J*=6.80 Hz, 2H), 4.69-4.74 (m, 1H), 6.40 (s, 1H), 7.18 (t, *J*=7.60 Hz, 1H), 7.26 (d, *J*=8.0 Hz, 1H), 7.31-7.34 (m, 1H), 7.75-7.79 (m, 2H), 8.25 (s, 1H), 8.45 (s, 1H), 8.52 (s, 1H), 8.58 (d, *J*=8.4 Hz, 1H), 10.50 (s, 1H), 10.85 (s, 1H), 11.4 (s, 1H). Mass spec: m/z 713.2 [M+H]$^+$.

**Example 36 was prepared following Scheme 36**

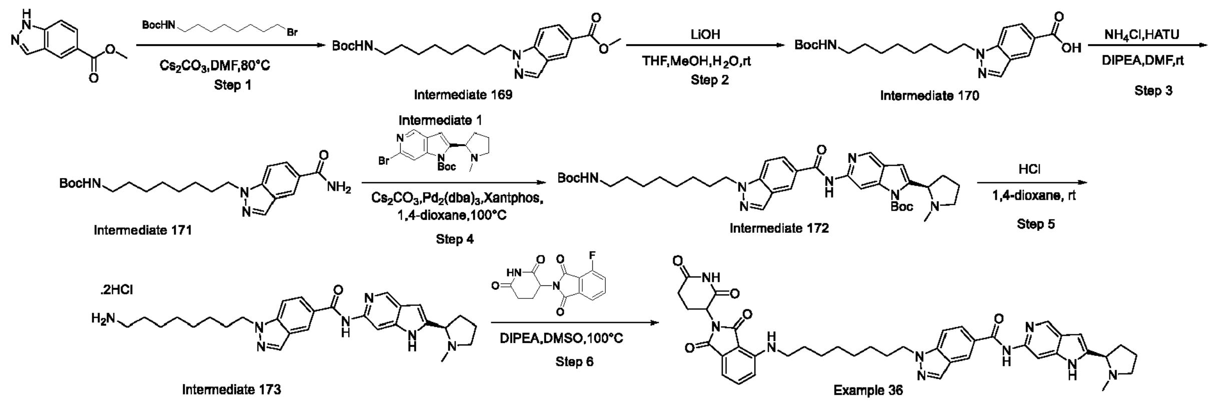


Scheme 36

**Step 1**

**Intermediate 169: methyl-1-(8-((*tert*-butoxycarbonyl)amino)octyl)-1*H*-indazole-5-carboxylate**

To solution of methyl 1*H*-indazole-5-carboxylate (CAS No: 473416-12-5, 2.00 g, 11.35 mmol) and *tert*-butyl *N*-(8-bromooctyl)carbamate (CAS No: 142356-35-2, 4.20 g, 13.62 mmol) in dimethylformamide (25 mL) was added cesium carbonate (11.1 g, 34.05 mmol) and the reaction mixture was heated at 80°C for 2h. The reaction mixture was then diluted with water (100 mL) and extracted with ethyl acetate (2 × 50 mL). The combined organic layers were dried over anhydrous $Na_2SO_4$ and concentrated *in vacuo.* The crude material was purified by combi-flash chromatography, by eluting with

20-25% ethyl acetate in heptane, to afford the desired regioisomer methyl-1-(8-((*tert*-butoxycarbonyl)amino)octyl)-1*H*-indazole-5-carboxylate (**Intermediate 169,** 2.5 g, 55%) as a brown solid. $^1$H NMR (400 MHz, DMSO-$d_6$) δ ppm 1.17-1.31 (m, 9H), 1.35 (s, 9H), 1.80-1.84 (m, 2H), 2.83-2.88 (m, 2H), 3.16-3.18 (m, 1H), 3.87 (s, 3H), 4.43 (t, *J*=6.63 Hz, 2H), 6.71 (br s, 1H), 7.76 (d, *J*=8.71 Hz, 1H), 7.94 (d, *J*=8.71 Hz, 1H), 8.25 (s, 1H), 8.47 (s, 1H). Mass spec: m/z 404.3 [M+H]$^+$.

**Step 2**

**Intermediate 170: 1-(8-((*tert*-butoxycarbonyl)amino)octyl)-1*H*-indazole-5-carboxylic acid**

To a solution of methyl-1-(8-((*tert*-butoxycarbonyl)amino)octyl)-1*H*-indazole-5-carboxylate (**Intermediate 69, 2.50** g, 6.20 mmol) in THF (20 mL), methanol (20 mL) and $H_2O$ (10 mL) was added LiOH (0.59 g, 25 mmol) and the reaction mixture was stirred at room temperature for 16h. The reaction mixture was concentrated in *vacuo,* water (10 mL) was added, and the pH of the solution was adjusted to pH~4 by the addition of saturated aqueous citric acid solution. The resultant precipitate was collected by filtration, washed with water (30 mL) and dried *in vacuo* to afford 1-(8-((*tert*-butoxycarbonyl)amino)octyl)-1*H*-indazole-5-carboxylic acid (**Intermediate 170,** 2.2 g, 91%) as a yellow solid. $^1$H NMR (400 MHz, DMSO-$d_6$) δ ppm 1.18-1.32 (m, 10H), 1.36 (s, 9H), 1.78-1.82 (m, 2H), 2.83-2.86 (m, 2H), 4.35 (t, *J*=6.63 Hz, 2H), 6.72 (br s, 1H), 7.47 (d, *J*=8.71 Hz, 1H), 7.96 (d, *J*=8.71 Hz, 1H), 8.04 (s, 1H), 8.25 (s, 1H). Mass spec: m/z 390.2 [M+H]$^+$.

**Step 3**

**Intermediate 171: *tert*-butyl-(8-(5-carbamoyl-1*H*-indazol-1-yl) octyl) carbamate**

To a solution of 1-[8-(*tert*-butoxycarbonylamino)octyl]indazole-5-carboxylic acid **(Intermediate 170, 2.20** g, 5.6 mmol) and ammonium chloride (1.5 g, 28 mmol) in dimethylformamide (5 mL) was added HATU (3.3 g, 8.5 mmol) followed by DIPEA (5 mL, 28 mmol) and the reaction mixture was stirred at room temperature for 16h. The reaction mixture was then poured into water (150 mL) and the resulting precipitate was collected by filtration, washed with water (50 mL) and dried *in vacuo* to afford *tert*-butyl-(8-(5-carbamoyl-1*H*-indazol-1-yl) octyl) carbamate (**Intermediate 171**, 1.70 g, 77%) as a yellow solid. $^1$H NMR (400 MHz, DMSO-$d_6$) δ ppm 1.19-1.31 (m, 10H), 1.35 (s, 9H), 1.80-1.83 (m, 2H), 2.83-2.86 (m, 2H), 4.41 (t, *J*=6.84 Hz, 2H), 6.72 (br s, 1H), 7.26 (br s, 1H), 7.69 (d, *J*=8.71 Hz, 1H), 7.89 (d, *J*=8.71 Hz, 1H), 7.96 (br s, 1H), 8.17 (s, 1H), 8.33 (s, 1H). Mass spec: m/z 389.2 [M+H]$^+$.

**Step 4**

**Intermediate 172: *tert*-butyl-(*R*)-6-(1-(8-((tert-butoxycarbonyl)amino)octyl)-1*H* indazole-5-carboxamido)-2-(1-methylpyrrolidin-2-yl)-1*H*-pyrrolo[3,2-c]pyridine-1-carboxylate**

A solution of *tert*-butyl-(8-(5-carbamoyl-1*H*-indazol-1-yl) octyl) carbamate **(Intermediate 171, 0.50** g, 1.28 mmol), *tert*-butyl-(R)-6-bromo-2-(1-methylpyrrolidin-2-yl)-1*H*-pyrrolo[3,2-c]pyridine-1-carboxylate (**Intermediate 1**, 0.58 g, 1.54 mmol) and cesium carbonate (1.27 g, 3.86 mmol) in 1,4-dioxane (10.0 mL) was purged with argon gas for 30 min. $Pd_2(dba)_3$ (0.18 g, 0.19 mmol) and Xantphos (0.23 g, 0.38 mmol) were added and the reaction was purged further with argon for 10 min and then heated at 100°C for 2h. The reaction mixture was then concentrated *in vacuo.* The crude material was purified by combi-flash column chromatography, by eluting with 90% ethyl acetate in heptane, to afford *tert*-butyl-(*R*)-6-(1-(8-((*tert*-butoxycarbonyl)amino)octyl)-1*H*-indazole-5-carboxamido)-2-(1-methylpyrrolidin-2-yl)-1*H*-pyrrolo[3,2-c]pyridine-1-carboxylate (**Intermediate 172**, 0.44 g, 50%) as a yellow solid. $^1$H NMR (400 MHz, DMSO-$d_6$) δ ppm 1.14-1.29 (m, 10H), 1.35 (s, 9H), 1.70 (s, 9H), 1.74-1.76 (m, 2H), 1.82-1.85 (m, 3H), 2.31-2.33 (m, 1H), 2.36 (s, 3H), 2.36-2.38 (m, 1H), 2.84-2.89 (m, 2H), 3.11-3.15 (m, 1H), 3.91-3.95 (m, 1H), 4.44 (t, *J*=6.85 Hz, 2H), 6.71 (br s, 1H), 6.75 (s, 1H), 7.76 (d, *J*=8.80 Hz, 1H), 8.06 (d, *J*=8.80 Hz, 1H), 8.22 (s, 1H), 8.56 (s, 1H), 8.60 (s, 1H), 8.95 (s, 1H), 10.67 (s, 1H). Mass spec: m/z 688.5 [M+H]$^+$.

**Step 5**

**Intermediate 173: (*R*)-1-(8-aminooctyl)-*N*-(2-(1-methylpyrrolidin-2-yl)-1*H* pyrrolo[3,2-*c*]pyridin-6-yl)-1H-indazole-5-carboxamide dihydrochloride**

To a cooled (0°C) solution of *tert*-butyl-(*R*)-6-(1-(8-((*tert*-butoxycarbonyl)amino)octyl)-1*H*-indazole-5-carboxamido)-2-(1-methylpyrrolidin-2-yl)-1*H*-pyrrolo[3,2-c]pyridine-1-carboxylate (**Intermediate 172,** 0.44 g, 0.63 mmol) in 1,4-dioxane (4 mL) was added 4M HCl in 1,4-dioxane (8 mL, 32 mmol) and the reaction mixture was stirred at room temperature for 16h. The reaction mixture was then concentrated *in vacuo* to afford (*R*)-1-(8-aminooctyl)-*N*-(2-(1-methylpyrrolidin-2-yl)-1*H*-pyrrolo[3,2-c]pyridin-6-yl)-1*H*-indazole-5-carboxamide dihydrochloride (**Intermediate 173**, 0.38 g, crude) as a brown solid which was used in the next step without further purification. $^1$H NMR (400 MHz, DMSO-$d_6$) $\delta$ ppm 1.19-1.29 (m, 10H), 1.48-1.52 (m, 2H), 1.85-1.92 (m, 2H), 2.18-2.21 (m, 2H), 2.72 (s, 3H), 2.80-2.82 (m, 2H), 3.65-3.73 (m, 2H), 4.46-4.49 (m, 1H), 4.70-4.73 (m, 2H), 7.24 (s, 1H), 7.81 (s, 3H), 7.87 (d, *J*=8.71 Hz, 1H), 8.17 (d, *J*=8.71 Hz, 1H), 8.25 (s, 1H), 8.32 (s, 1H), 8.73 (s, 1H), 9.05 (br s, 1H), 11.30 (br s, 1H), 11.89 (br s, 1H), 13.11 (br s, 1H).

**Step 6**

**Example 36: 1-(8-((2-(2,6-dioxopiperidin-3-yl)-1,3-dioxoisoindolin-4-yl)amino)octyl)-*N*-(2-((*R*)-1-methylpyrrolidin-2-yl)-1*H*-pyrrolo[3,2-c]pyridin-6-yl)-1*H*-indazole-5-carboxamide**

To a solution of (*R*)-1-(8-aminooctyl)-*N*-(2-(1-methylpyrrolidin-2-yl)-1*H*-pyrrolo[3,2-c]pyridin-6-yl)-1*H*-indazole-5-carboxamide dihydrochloride (**Intermediate 173,** 0.35 g, 0.66 mmol) in DMSO (3 mL) was added DIPEA (0.58 mL, 3.34 mmol) followed by 2-(2,6-dioxopiperidin-3-yl)-4-fluoroisoindoline-1,3-dione (CAS No: 835616-60-9, 0.18 g, 0.66 mmol) and the reaction mixture was heated at 100°C for 2h. The reaction mixture was then quenched with ice cold water (50 mL) and the resultant precipitate was collected by filtration and dried in *vacuo.* The solid was purified by preparative HPLC (Method A) to afford 1-(8-((2-(2,6-dioxopiperidin-3-yl)-1,3-dioxoisoindolin-4-yl)amino)octyl)-*N*-(2-((*R*)-1-methylpyrrolidin-2-yl)-1*H*-pyrrolo[3,2-c]pyridin-6-yl)-1*H*-indazole-5-carboxamide (**Example 36**, 0.056 g, 11%) as a yellow solid. $^1$H NMR (400 MHz, DMSO-$d_6$) $\delta$ ppm 1.23-1.29 (m, 9H), 1.52-1.54 (m, 2H), 1.78-1.93 (m, 5H), 1.98-2.03 (m, 1H), 2.09-2.15 (m, 1H), 2.17 (s, 3H), 2.26-2.28 (m, 1H), 2.51-2.60 (m, 2H), 2.84-2.87 (m, 2H), 3.12-3.16 (m, 2H), 4.42-4.46 (m, 2H), 5.01-5.06 (m, 1H), 6.39 (s, 1H), 6.49 (t, *J*=5.79 Hz, 1H), 7.01 (d, *J*=6.80 Hz, 1H), 7.07 (d, *J*=8.38 Hz, 1H), 7.54-7.59 (m, 1H), 7.75 (d, *J*=8.76 Hz, 1H), 8.04-8.07 (m, 1H), 8.20-8.22 (m, 2H), 8.51 (s, 1H), 8.55 (s, 1H), 10.40 (s, 1H), 11.07 (br s, 1H), 11.34 (s, 1H). Mass spec: m/z 744.2 [M+H]$^+$.

**Example 37 was synthesised following Scheme 37**

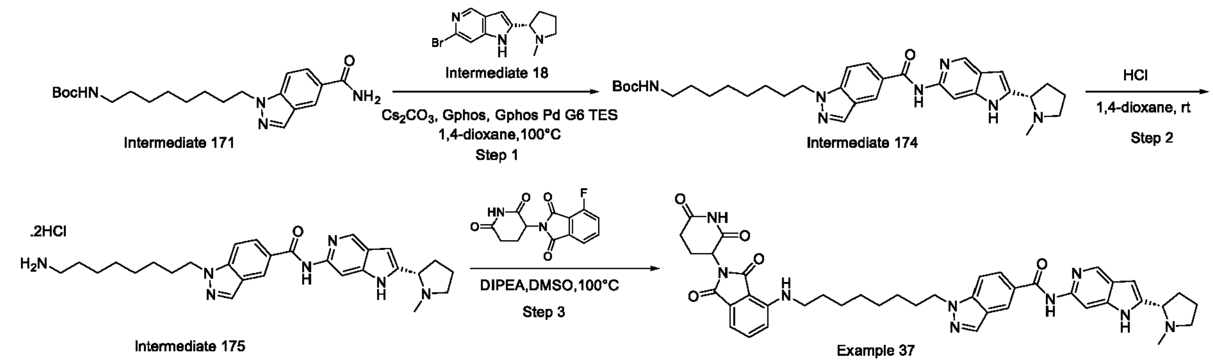


**Scheme 37**

**Step 1**

**Intermediate 174: tert-butyl N-{8-[5-({2-[(2S)-1-methylpyrrolidin-2-yl]-1H-pyrrolo [3,2-c]pyridin-6-yl}carbamoyl) indazol-1-yl]octyl}carbamate**

A 50 mL round-bottom flask was charged with tert-butyl N-[8-(5-carbamoylindazol-1-yl)octyl]carbamate (**Intermediate 171**, 210 mg, 0.486 mmol), (2S)-2-{6-bromo-1H-pyrrolo[3,2-c]pyridin-2-yl}-1-methylpyrrolidine (**Intermediate 18**, 182 mg, 0.583 mmol), $Cs_2CO_3$ (793 mg, 2.43 mmol), Gphos (94.2 mg, 0.175 mmol), Gphos Pd G6 TES (96.0 mg, 0.102 mmol) and 1,4-dioxane (10 mL) and the reaction was stirred overnight at 100°C under nitrogen.. The volatiles were removed under reduced pressure and the residue was chromatographed on a silica gel column, eluting with MeOH/DCM (4/96), to afford tert-butyl N-{8-[5-({2-[(2S)-1-methylpyrrolidin-2-yl]-1H-pyrrolo[3,2-c]pyridin-6-yl}carbamoyl)indazol-1-yl] octyl}carbamate (**Intermediate 174**, 140 mg, 47%) as a yellow solid. Mass spec: m/z: 588.40[M+H]$^+$.

**Step 2**

**Intermediate 175: 1-(8-aminooctyl)-N-{2-[(2S)-1-methylpyrrolidin-2-yl]-1H-pyrrolo[3,2-c]pyridin-6-yl}indazole-5-carboxamide dihydrochloride**

**Intermediate 175** was synthesised in an analogous manner to **Intermediate 173**, shown in **Step 5** of **Scheme 36**, using tert-butyl N-{8-[5-({2-[(2*S*)-1-methylpyrrolidin-2-yl]-1H-pyrrolo[3,2-c]pyridin-6-yl}carbamoyl)indazol-1-yl]octyl} carbamate as the amide, to afford 1-(8-aminooctyl)-N-{2-[(2S)-1-methylpyrrolidin-2-yl]-1H-pyrrolo[3,2-c]pyridin-6-yl}indazole-5-carboxamide dihydrochloride (**Intermediate 175**). Mass spec: m/z: 488.40 $[M+H]^+$.

**Step 3**

**Example 37: 1-(8-((2-(2,6-dioxopiperidin-3-yl)-1,3-dioxoisoindolin-4-yl)amino)octyl)-N-(2-((S)-1-methylpyrrolidin-2-yl)-1H-pyrrolo[3,2-c]pyridin-6-yl)-1H-indazole-5-carboxamide**

**Example 37** was synthesised in an analogous manner to **Example 36**, shown in **Step 6** of **Scheme 36**, using 1-(8-aminooctyl)-N-{2-[(2S)-1-methylpyrrolidin-2-yl]-1H-pyrrolo[3,2-c]pyridin-6-yl}indazole-5-carboxamide dihydrochloride (**Intermediate 175**) as the amine and 2-(2,6-dioxopiperidin-3-yl)-4-fluoroisoindoline-1,3-dione (CAS No: 835616-60-9) as the aryl fluoride, to afford 1-(8-((2-(2,6-dioxopiperidin-3-yl)-1,3-dioxoisoindolin-4-yl)amino)octyl)-N-(2-((S)-1-methylpyrrolidin-2-yl)-1H-pyrrolo[3,2-c]pyridin-6-yl)-1H-indazole-5-carboxamide (**Example 37**). $^1$H NMR (400 MHz, MeOH-d4) δ ppm 8.51 - 8.60 (m, 2H), 8.18 - 8.23 (m, 2H), 8.06 (d, J = 8.8 Hz, 1H), 7.72 (d, J = 8.8 Hz, 1H), 7.53 (t, J = 7.6 Hz, 1H), 7.01 (d, J = 8.4 Hz, 2H), 6.62 (s, 1H), 5.05 - 5.09 (m, 1H), 4.47 - 4.51 (m, 2H), 3.70 - 3.80 (m, 1H), 3.45 - 3.49 (m, 1H), 3.33 - 3.37 (m, 1H), 2.82 - 2.86 (m, 1H), 2.72 - 2.77 (m, 2H), 2.61 - 2.63 (m, 1H), 2.40 - 2.44 (m, 3H), 2.36 - 2.37 (m, 1H), 2.11 - 2.18 (m, 3H), 1.94 - 2.03 (m, 3H), 1.59 - 1.61 (m, 2H), 1.20 - 1.21 (m, 9H). Mass spec: m/z: 744.60 $[M+H]^+$.

**Example 38 was synthesised following Scheme 38**

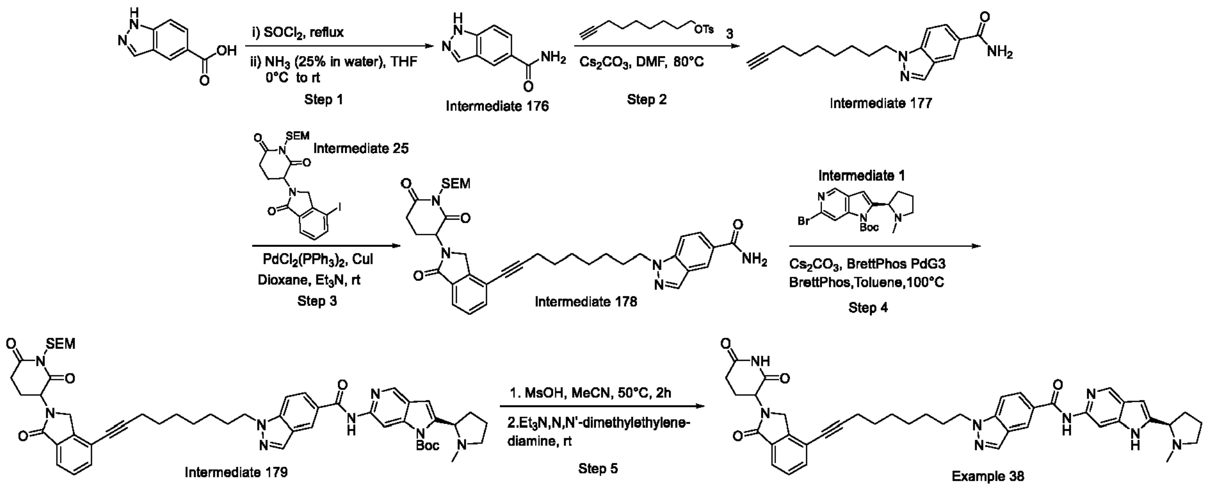


## Scheme 38

**Step** 1

**Intermediate 176: 1*H*-indazole-5-carboxamide**

To solution of 1*H*-indazole-5-carboxylic acid (CAS No: 61700-61-6, 3.0 g, 18.50 mmol) in thionyl chloride (30 mL) was stirred at reflux for 1h. The reaction mixture was cooled to room temperature and the solution was concentrated *in vacuo.* The residue was suspended in dry tetrahydrofuran (80 mL), cooled to 0°C, and 25% $NH_3$ solution (40 mL, 1100 mmol) was added. The reaction mixture was stirred at room temperature for 6h. The reaction mixture was diluted with water (100 mL) and the resulting precipitate was collected by filtration and dried to afford 1*H*-indazole-5-carboxamide **(Intermediate 176**, 1.5 g, 50%) as a yellow solid. $^1$H NMR (400 MHz, DMSO-$d_6$) δ 7.24 (br s, 1H), 7.55 (d, *J*=8.80 Hz, 1H), 7.87 (d,

*J*=8.80 Hz, 1H), 7.95 (br s, 1H), 8.18 (s, 1H), 8.35 (s, 1H), 13.23 (br s, 1H). Mass spec: m/z 162 $[M+H]^+$.

**Step 2**

**Intermediate 177: 1-(non-8-yn-1-yl)-1*H*-indazole-5-carboxamide**

To a solution of 1*H*-indazole-5-carboxamide (**Intermediate 176**, 1.80 g, 11.2 mmol) in dimethylformamide (30 mL) was added cesium carbonate (10.9 g, 33.5 mmol) followed by non-8-ynyl-4-methylbenzenesulfonate (CAS No: 87462-64-4, 3.95 g, 13.4 mmol) at room temperature and the reaction mixture was stirred at 80°C for 4h. The reaction mixture was then diluted with water (100 mL) and extracted with ethyl acetate (3 × 60 mL). The combined organic layers were dried over anhydrous $Na_2SO_4$, filtered and concentrated *in vacuo* to obtain the crude compound as a mixture of regioisomers. The regioisomers were separated by combi-flash chromatography, by eluting with 60% ethyl acetate in heptane, to afford desired regioisomer 1-(non-8-yn-1-yl)-1*H*-indazole-5-carboxamide (**Intermediate 177**, 1.5 g, 47%) as a white solid. $^1H$ NMR (400 MHz, DMSO-$d_6$) δ 1.20-1.38 (m, 8H), 1.80-1.84 (m, 2H), 2.08-2.12 (m, 2H), 2.70 (s, 1H), 4.41 (t, *J*=6.80 Hz, 2H), 7.26 (br s, 1H), 7.69 (d, *J*=8.60 Hz, 1H), 7.90 (d, *J*=8.60 Hz, 1H), 7.96 (br s, 1H), 8.17 (s, 1H), 8.33 (s, 1H). Mass spec: m/z: 284.3 $[M+H]^+$.

**Step 3**

**Intermediate 178: 1-(9-(2-(2,6-dioxo-1-((2-(trimethylsilyl)ethoxy)methyl)piperidin-3-yl)-1-oxoisoindolin-4-yl) non-8-yn-1-yl)-1H indazole-5-carboxamide**

To a solution of 1-(non-8-yn-1-yl)-1*H*-indazole-5-carboxamide **(Intermediate 177**, 0.80 g, 2.82 mmol) and 3-(4-iodo-1-oxoisoindolin-2-yl)-1-((2-(trimethylsilyl)ethoxy)methyl)piperidine-2,6-dione (**Intermediate 25**, 1.55 g, 3.10 mmol) in 1,4-dioxane (6.0 mL) was added triethylamine (13.8 mL, 98.80 mmol). The reaction mixture was purged with argon gas for 15 min then $PdCl_2(PPh_3)_2$ (0.20 g, 0.28 mmol) and copper (I) iodide (0.05 g, 0.28 mmol) were added and the reaction mixture was further purged with argon gas for 10 min, then stirred at room temperature for 2h. The reaction mixture was diluted with water (60 mL) and extracted with ethyl acetate (3 × 50 mL). The combined organic layers were washed with brine solution (2 × 50 mL), dried over anhydrous $Na_2SO_4$, filtered and concentrated *in vacuo.* The crude material was purified by combi-flash chromatography, by eluting with 2% MeOH in dichloromethane, to afford 1-(9-(2-(2,6-dioxo-1-((2-(trimethylsilyl) ethoxy)methyl)piperidin-3-yl)-1-oxoisoindolin-4-yl)non-8-yn-1-yl)-1*H*-indazole-5-carboxamide (**Intermediate 178**, 0.80 g, 43%) as a white solid. $^1H$ NMR (400 MHz, DMSO-$d_6$) δ -0.04 (s, 9H), 0.76-0.88 (m, 2H), 1.28-1.41 (m, 6H), 1.48-1.57 (m, 2H), 1.80-1.87 (m, 2H), 2.04-2.08 (m, 1H), 2.39-2.46 (m, 3H), 2.77-2.82 (m, 1H), 3.02-3.11 (m, 1H), 3.49-3.56 (m, 2H), 4.23-4.27 (m, 1H), 4.38-4.48 (m, 3H), 5.01-5.09 (m, 2H), 5.25-5.30 (m, 1H), 7.26 (br s, 1H), 7.51 (t, *J*=7.20 Hz, 1H), 7.61 (d, *J*=7.20 Hz, 1H), 7.68-7.72 (m, 2H), 7.96 (d, *J*=8.80 Hz, 1H), 7.96 (br s, 1H), 8.16 (s, 1H), 8.33 (s, 1H). Mass spec: m/z: 654.1 $[M-H]^-$.

**Step 4**

**Intermediate 179: *tert*-butyl 6-(1-(9-(2-(2,6-dioxo-1-((2-(trimethylsilyl)ethoxy)methyl)piperidin-3-yl)-1-oxoisoindolin-4-yl)non-8-yn-1-yl)-1*H*-indazole-5-carboxamido)-2-((*R*)-1-methylpyrrolidin-2-yl)-1*H*-pyrrolo[3,2-*c*]pyridine-1-carboxylate**

To a solution of 1-(9-(2-(2,6-dioxo-1-((2-(trimethylsilyl)ethoxy)methyl)piperidin-3-yl)-1-oxoisoindolin-4-yl) non-8-yn-1-yl)-1*H*-indazole-5-carboxamide (**Intermediate 178**, 0.60 g, 0.91 mmol) in toluene (10 mL) was added *tert*-butyl (*R*)-6-bromo-2-(1-methylpyrrolidin-2-yl)-1*H*-pyrrolo[3,2-c]pyridine-1-carboxylate (**Intermediate 1**, 0.41 g, 1.09 mmol) followed by cesium carbonate (0.89 g, 2.74 mmol). The reaction mixture was purged with argon gas for 15 min, then BrettPhos (0.075 g, 0.13 mmol) and BrettPhos Pd G3 (0.12 g, 0.13 mmol) were added. The reaction mixture was purged with argon gas for 15 min and then stirred at 100°C for 2h. The reaction mixture was diluted with water (60 mL) and extracted with ethyl acetate (3 × 50 mL). The combined organic layers were washed with brine solution (2 × 50 mL), separated, dried over anhydrous $Na_2SO_4$, filtered, and concentrated *in vacuo.* The crude material was purified by combi-flash chromatography, by eluting with 2% MeOH in dichloromethane, to afford *tert*-butyl 6-(1-(9-(2-(2,6-dioxo-1-((2-(trimethylsilyl)ethoxy)methyl)piperidin-3-yl)-1-oxoisoindolin-4-yl)non-8-yn-1-yl)-1*H*-indazole-5-carboxamido)-2-((*R*)-1-methylpyrrolidin-2-yl)-1*H*-pyrrolo[3,2-c]pyridine-1-carboxylate (**Intermediate 179**, 0.45 g, 51%) as a white solid. $^1H$ NMR (400 MHz, DMSO-$d_6$) δ ppm -0.05 (s, 9H), 0.79-0.83 (m, 2H), 1.18-1.38 (m, 8H), 1.48-1.55 (m, 2H), 1.61-1.65 (m, 1H), 1.70 (s, 9H), 1.80-1.85 (m, 2H), 2.01-2.07 (m, 1H), 2.26-2.30 (m, 2H), 2.36 (s, 3H), 2.41-2.46 (m, 3H), 2.76-2.80 (m, 1H), 3.03-3.15 (m, 2H), 3.48-3.54 (m, 2H), 3.91-3.94 (m, 1H), 4.23-4.27 (m, 1H), 4.42-4.48 (m, 3H), 5.00-5.08 (m, 2H), 5.25-5.29 (m, 1H), 6.75 (br s, 1H), 7.49-7.53 (m, 1H), 7.62 (d, *J*=8.00 Hz, 1H), 7.70-7.76 (m, 2H), 8.05 (d, *J*=8.00 Hz, 1H),

8.21 (br s, 1H), 8.55-8.62 (m, 2H), 8.95 (br s, 1H), 10.67 (br s, 1H). Mass spec: m/z: 955.2 [M+H]$^+$.

**Step 5**

**Example 38: 1-(9-(2-(2,6-dioxopiperidin-3-yl)-1-oxoisoindolin-4-yl)non-8-yn-1-yl)-*N*-(2-((*R*)-1-methylpyrrolidin-2-yl)-1*H*-pyrrolo[3,2-*c*]pyridin-6-yl)-1*H*-indazole-5-carboxamide**

To a solution of *tert*-butyl 6-(1-(9-(2-(2,6-dioxo-1-((2-(trimethylsilyl)ethoxy)methyl)piperidin-3-yl)-1-oxoisoindolin-4-yl)non-8-yn-1-yl)-1*H*-indazole-5-carboxamido)-2-((*R*)-1-methylpyrrolidin-2-yl)-1*H*-pyrrolo[3,2-c]pyridine-1-carboxylate (**Intermediate 179**, 0.42 g, 0.44 mmol) in acetonitrile (10 mL) was added methanesulfonic acid (0.43 mL, 6.59 mmol) and the reaction mixture was stirred at 50°C for 2h. The reaction mixture was cooled to room temperature, then *N,N'*-dimethylethylenediamine (0.32 mL, 2.63 mmol) followed by triethylamine (0.92 mL, 6.59 mmol) were added and the reaction stirred at room temperature for 2h. The reaction mixture was concentrated *in vacuo* and the resultant solid washed with ether (2 × 50 mL), then DCM/Pentanes (2 × 40 mL) and dried *in vacuo.* The crude compound was then purified by purified by preparative HPLC (Method A) to afford 1-(9-(2-(2,6-dioxopiperidin-3-yl)-1-oxoisoindolin-4-yl)non-8-yn-1-yl)-*N*-(2-((*R*)-1-methylpyrrolidin-2-yl)-1*H*-pyrrolo[3,2-*c*]pyridin-6-yl)-1*H*-indazole-5-carboxamide (**Example 38**, 0.09 g, 28%) as an off white solid. $^1$H NMR (400 MHz, $CDCl_3$-*d*) δ ppm 1.24-1.60 (m, 9H), 1.87-2.04 (m, 4H), 2.18-2.26 (m, 2H), 2.29 (s, 3H), 2.35-2.49 (m, 4H), 2.82-2.96 (m, 2H), 3.27-3.32 (m, 1H), 3.49-3.58 (m, 1H), 4.33-4.47 (m, 3H), 4.53-4.58 (m, 1H), 5.24-5.28 (m, 1H), 6.44 (s, 1H), 7.38-7.41 (m, 1H), 7.46-7.49 (m, 1H), 7.59-7.61 (m, 1H), 7.78-7.84 (m, 2H), 8.02-8.04 (m, 1H), 8.43 (s, 1H), 8.47 (s, 1H), 8.64 (s, 1H), 9.25 (br s, 1H) 9.31 (br s, 1H). Mass spec: m/z: 725.1 [M+H]$^+$.

**Example 39 was synthesized following Scheme 39**

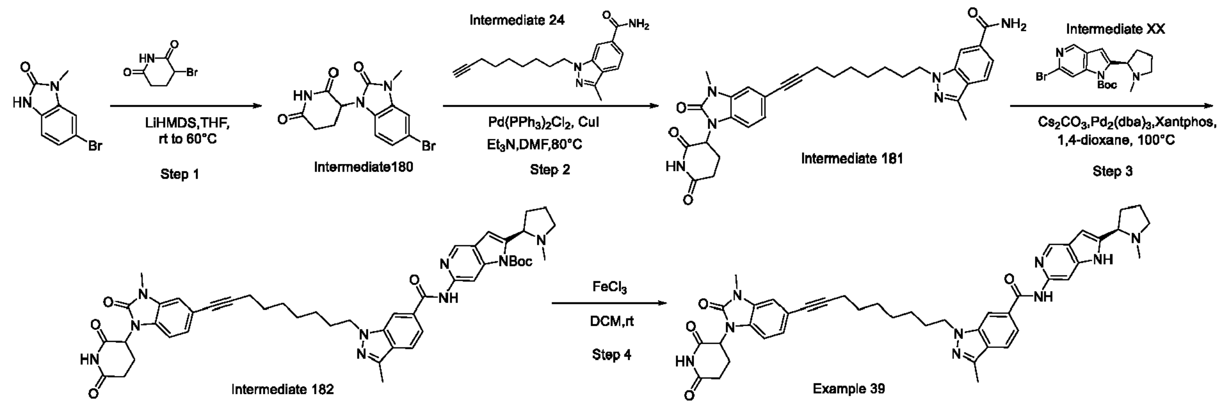


## Scheme 39

**Step 1**

**Intermediate 180: 3-(5-bromo-3-methyl-2-oxo-2, 3-dihydro-1*H*-benzo[*d*]imidazol-1-yl) piperidine-2, 6-dione**

To solution of 6-bromo-1-methyl-1, 3-dihydro-2*H*-benzo[d]imidazol-2-one (CAS No. 305790-48-1, 5.00 g, 22.0 mmol) in tetrahydrofuran (30 mL) was added LiHMDS in THF (55 mL, 55.1 mmol) dropwise over a period of 5 min and reaction mixture was stirred at room temperature for 30 min. To the reaction mixture was added to 3-bromopiperidine-2, 6-dione (CAS No. 62595-74-8, 8.46 g, 44.0 mmol) in tetrahydrofuran (30 mL) over a period of 5 min, then the reaction was heated at 60°C for 4h. The reaction mixture was cooled to room temperature and a cold saturated solution of $NH_4Cl$ (300 mL) was added. The mixture was stirred at room temperature for 20 min. The resultant precipitate was collected by filtration, washed with ethyl acetate (60 mL) and heptane (100 mL) and dried *in vacuo* to afford 3-(5-bromo-3-methyl-2-oxo-2, 3-dihydro-1*H*-benzo[d]imidazol-1-yl) piperidine-2, 6-dione **(Intermediate 180,** 3.5 g, 47%) as an off white solid which was used for the next step without further purification. $^1$H NMR (400 MHz, DMSO-$d_6$) δ ppm 1.98-2.06 (m, 1H), 2.60-2.75 (m, 2H), 2.82-2.96 (m, 1H), 3.34 (s, 3H), 5.35-5.39 (m, 1H), 7.10 (d, *J*=8.33 Hz, 1H), 7.21 (d, *J*=8.11 Hz, 1H), 7.46 (s, 1H). Mass spec m/z 337.7 [M+H]$^+$.

**Step 2**

**Intermediate 181: 1-(9-(1-(2, 6-dioxopiperidin-3-yl)-3-methyl-2-oxo-2, 3-dihydro-1*H*-benzo[d]imidazol-5-yl) non-8-yn-1-yl)-3-methyl-1*H*-indazole-6-carboxamide**

To a mixture of 3-(5-bromo-3-methyl-2-oxo-2, 3-dihydro-1*H*-benzo[d]imidazol-1-yl)piperidine-2,6-dione (**Intermediate 180,** 1.00 g, 2.95 mmol) and 3-methyl-1-(non-8-yn-1-yl)-1*H*-indazole-6-carboxamide (**Intermediate 24**, 1.05 g, 3.54 mmol) in dimethylformamide (10 mL) was added cesium carbonate (3.04 g, 8.87 mmol) and the reaction mixture was purged with argon gas for 10 min then Pd $(PPh_3)_2Cl_2$ (0.21 g, 0.29 mmol) and copper (I) iodide (0.057 g, 0.29 mmol) were added. The reaction mixture was purged with argon gas for 10 min and the heated at 80°C for 4h. The reaction mixture was diluted with water (50 mL) and extracted with ethyl acetate (2 × 100 mL). The combined organic layers were washed with brine solution (100 mL), dried over anhydrous $Na_2SO_4$ and concentrated *in vacuo.* The crude material was purified by combi-flash chromatography, by eluting with 2% MeOH in dichloromethane, to afford 1-(9-(1-(2,6-dioxopiperidin-3-yl)-3-methyl-2-oxo-2,3-dihydro-1*H*-benzo[d]imidazol-5-yl)non-8-yn-1-yl)-3-methyl-1*H*-indazole-6-carboxamide (**Intermediate 181**, 1.00 g, 61%) as a pale yellow solid. $^1H$ NMR (400 MHz, DMSO-$d_6$) δ ppm 1.24-1.42 (m, 7H), 1.46-1.48 (m, 2H), 1.79-1.82 (m, 2H), 1.96-2.01 (m, 1H), 2.34-2.37 (m, 1H), 2.57 (s, 3H), 2.82-2.87 (m, 1H), 3.15 (d, *J*=5.04 Hz, 1H), 3.31 (s, 3H), 3.98-4.08 (m, 1H), 4.31-4.34 (m, 2H), 5.33-5.37 (m, 1H), 7.04 (s, 2H), 7.20 (s, 1H), 7.41 (s, 1H), 7.57 (d, *J*=8.33 Hz, 1H), 7.71 (d, *J*=8.33 Hz, 1H), 7.98-8.16 (m, 2H), 11.10 (s, 1H). Mass spec m/z 555.1 $[M+H]^+$.

**Step 3**

**Intermediate 182: tert-butyl 6-(1-(9-(1-(2,6-dioxopiperidin-3-yl)-3-methyl-2-oxo-2,3-dihydro-1*H*-benzo[d]imidazol-5-yl)non-8-yn-1-yl)-3-methyl-1*H*-indazole-6-carboxamido)-2-((*R*)-1-methylpyrrolidin-2-yl)-1*H*-pyrrolo[3,2-c] pyridine-1-carboxylate**

To a solution of 1-(9-(1-(2,6-dioxopiperidin-3-yl)-3-methyl-2-oxo-2,3-dihydro-1*H*-benzo[d]imidazol-5-yl)non-8-yn-1-yl)-3-methyl-1*H*-indazole-6-carboxamide (**Intermediate 181**, 0.60 g, 1.08 mmol) and *tert*-butyl (R)-6-bromo-2-(1-methylpyrrolidin-2-yl)-1*H*-pyrrolo[3,2-c]pyridine-1-carboxylate (**Intermediate 1**, 0.49 g, 1.29 mmol) in 1,4-dioxane (14 mL) was added cesium carbonate (1.06 g, 3.24 mmol). The reaction mixture was purged with argon gas for 10 min, then Xantphos (0.19 g, 0.32 mmol) and $Pd_2(dba)_3$ (0.15 g, 0.16 mmol) were added. The reaction mixture was purged with argon gas for 10 min and then heated at 100°C for 2h. The reaction mixture was diluted with water (40 mL) and extracted with ethyl acetate (3 × 50 mL). The combined organic layers were washed with brine solution (2 × 50 mL), dried over anhydrous $Na_2SO_4$, and concentrated *in vacuo.* The crude material was purified by combi-flash chromatography, by eluting with 4% MeOH in dichloromethane, to afford *tert*-butyl 6-(1-(9-(1-(2,6-dioxopiperidin-3-yl)-3-methyl-2-oxo-2,3-dihydro-1*H*-benzo [d]imidazol-5-yl)non-8-yn-1-yl)-3-methyl-1*H*-indazole-6-carboxamido)-2-((R)-1-methylpyrrolidin-2-yl)-1*H*-pyrrolo[3,2-c] pyridine-1-carboxylate (**Intermediate 182**, 0.43 g, 47%) as a yellow solid. $^1H$ NMR (400 MHz, DMSO-$d_6$) δ ppm 1.18-1.40 (m, 8H), 1.48-1.53 (m, 2H), 1.60-1.66 (m, 2H), 1.70 (s, 9H), 1.73-1.80 (m, 2H), 1.86-1.90 (m, 2H), 1.98-2.03 (m, 1H), 2.14-2.20 (m, 1H), 2.36 (s, 3H), 2.37-2.42 (m, 5H), 2.60-2.74 (m, 2H), 2.84-2.94 (m, 1H), 3.11-3.18 (m, 2H), 3.91-3.94 (m, 1H), 4.38-4.42 (m, 2H), 5.34-5.40 (m, 1H), 6.76 (s, 1H), 7.04-7.06 (m, 2H), 7.20 (s, 1H), 7.71-7.80 (m, 2H), 8.43 (s, 1H), 8.61 (s, 1H), 8.98 (s, 1H), 10.75 (br s, 1H), 11.10 (br s, 1H). Mass spec m/z 854.2 $[M+H]^+$.

**Step 4**

**Example 39: 1-(9-(1-(2,6-dioxopiperidin-3-yl)-3-methyl-2-oxo-2,3-dihydro-1*H* benzo[d]imidazol-5-yl)non-8-yn-1-yl)-3-methyl-*N*-(2-((R)-1-methylpyrrolidin-2-yl)-1*H*-pyrrolo[3,2-*c*]pyridin-6-yl)-1*H*-indazole-6-carboxamide**

To a solution of *tert*-butyl 6-(1-(9-(1-(2,6-dioxopiperidin-3-yl)-3-methyl-2-oxo-2,3-dihydro-1H-benzo[d]imidazol-5-yl)non-8-yn-1-yl)-3-methyl-1*H*-indazole-6-carboxamido)-2-((R)-1-methylpyrrolidin-2-yl)-1*H*-pyrrolo[3,2-c]pyridine-1-carboxylate (**Intermediate 182**, 0.13 g, 0.15 mmol) in dichloromethane (8 mL) was added ferric chloride (0.074g, 0.45 mmol) and the reaction was stirred at room temperature for 48h. The reaction mixture was concentrated *in vacuo,* the resultant precipitate collected by filtration, washed with pentane (2 × 20 mL) and dried *in vacuo* to obtain the crude compound. The crude material was purified by preparative HPLC (Method A) to afford 1-(9-(1-(2,6-dioxopiperidin-3-yl)-3-methyl-2-oxo-2,3-dihydro-1H-benzo[d]imidazol-5-yl)non-8-yn-1-yl)-3-methyl-*N*-(2-((R)-1-methylpyrrolidin-2-yl)-1*H*-pyrrolo[3,2-c]pyridin-6-yl)-1*H*-indazole-6-carboxamide (**Example 39**, 0.025 g, 22%) as an off white solid. $^1H$ NMR (400 MHz, DMSO-$d_6$) δ ppm 1.31-1.40 (m, 7H), 1.48-1.56 (m, 2H), 1.78-2.05 (m, 7H), 2.13-2.15 (m, 2H), 2.17 (s, 3H), 2.24-2.30 (m, 1H), 2.37-2.40 (m, 2H), 2.51 (s, 3H), 2.59-2.70 (m, 2H), 2.84-2.94 (m, 1H), 3.12-3.16 (m, 1H), 3.34-3.36 (m, 1H), 4.41 (t, *J*=6.88 Hz, 2H), 5.34-5.38 (m, 1H), 6.40 (s, 1H), 7.05-7.08 (m, 2H), 7.20 (s, 1H), 7.71-7.79 (m, 2H), 8.25 (s, 1H), 8.44 (s, 1H), 8.51 (s, 1H), 10.52 (s, 1H), 11.10 (s, 1H), 11.38 (s, 1H). Mass spec m/z 754.3 $[M+H]^+$.

**Example 40 was synthesized following Scheme 40**

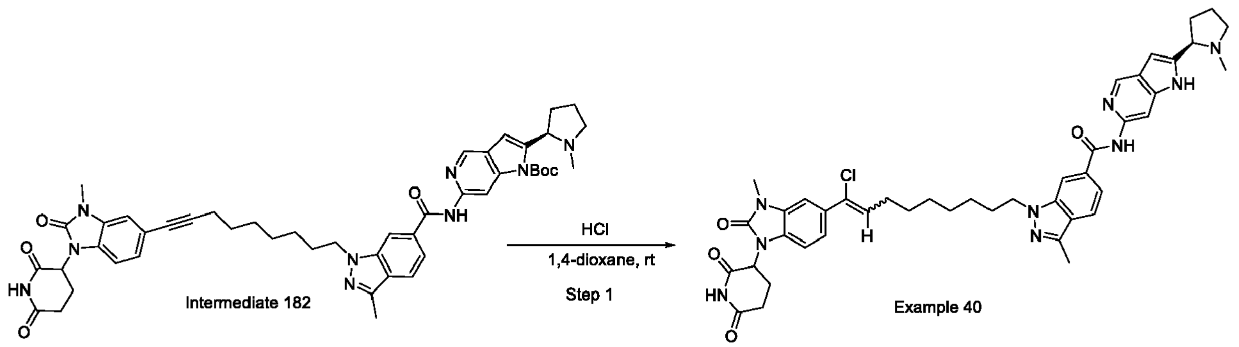


**Scheme 40**

**Step 1**

**Example 40: 1-((E)-9-chloro-9-(1-(2,6-dioxopiperidin-3-yl)-3-methyl-2-oxo-2,3-dihydro-1*H*-benzo[d]imidazol-5-yl)non-8-en-1-yl)-3-methyl-*N*-(2-((*R*)-1-methylpyrrolidin-2-yl)-1*H*-pyrrolo[3,2-c]pyridin-6-yl)-1*H*-indazole-6-carboxamide *and* 1-((Z)-9-chloro-9-(1-(2,6-dioxopiperidin-3-yl)-3-methyl-2-oxo-2,3-dihydro-1*H*-benzo[d]imidazol-5-yl)non-8-en-1-yl)-3-methyl-*N*-(2-((*R*)-1-methylpyrrolidin-2-yl)-1*H*-pyrrolo[3,2-*c*]pyridin-6-yl)-1*H*-indazole-6-carboxamide**

To a solution of *tert*-butyl 6-(1-(9-(1-(2,6-dioxopiperidin-3-yl)-3-methyl-2-oxo-2,3-dihydro-1*H*-benzo[d]imidazol-5-yl)non-8-yn-1-yl)-3-methyl-1*H*-indazole-6-carboxamido)-2-((*R*)-1-methylpyrrolidin-2-yl)-1*H*-pyrrolo[3,2-c]pyridine-1-carboxylate (**Intermediate 182**, 0.20 g, 0.23 mmol) in 1,4-dioxane (8.0 mL) was added 4M HCl in 1,4-dioxane (5.0 mL) and the mixture was stirred at room temperature for 4h then concentrated *in vacuo.* The resultant solid was washed with dichloromethane (2 $\times$ 20 mL) and dried *in vacuo* to obtain the crude compound which was purified by preparative HPLC (Method A) to afford as a 40:60 ratio of E and Z-alkene isomers of 1-((E)-9-chloro-9-(1-(2,6-dioxopiperidin-3-yl)-3-methyl-2-oxo-2,3-dihydro-1H-benzo[d]imidazol-5-yl)non-8-en-1-yl)-3-methyl-*N*-(2-((*R*)-1-methylpyrrolidin-2-yl)-1*H*-pyrrolo[3,2-c]pyridin-6-yl)-1*H*-indazole-6-carboxamide and 1-((Z)-9-chloro-9-(1-(2,6-dioxopiperidin-3-yl)-3-methyl-2-oxo-2,3-dihydro-1*H*-benzo[d]imidazol-5-yl)non-8-en-1-yl)-3-methyl-*N*-(2-((*R*)-1-methylpyrrolidin-2-yl)-1*H*-pyrrolo[3,2-c]pyridin-6-yl)-1*H*-indazole-6-carboxamide (**Example 40**, 0.045 g, 25%) as an off white solid. $^1$H NMR (400 MHz, DMSO-$d_6$) $\delta$ ppm 1.17-1.48 (m, 8H), 1.76-1.95 (m, 5H), 1.99-2.09 (m, 2H), 2.11-2.15 (m, 1H), 2.17 (s, 3H), 2.23-2.34 (m, 2H), 2.52 (s, 3H), 2.57-2.75 (m, 2H), 2.82-2.94 (m, 1H), 3.11-3.18 (m, 1H), 3.32-3.33 (m, 1H), 3.34 (s, 3H), 4.34-4.43 (m, 2H), 5.35-5.40 (m, 1H), 5.95 (t, *J*=7.4 Hz, *0.6H*), 6.31 (t, *J*=7.4 Hz, *0.4H*), 6.40 (s, 1H), 7.00-7.39 (m, 3H), 7.71-7.80 (m, 2H), 8.25 (s, 1H), 8.41-8.44 (m, 1H), 8.52 (s, 1H), 10.52 (s, 1H), 11.10 (br s, 1H), 11.38 (s, 1H). Mass spec m/z 790.1 [M+H]$^+$.

**Example 41 was synthesized following Scheme 41**

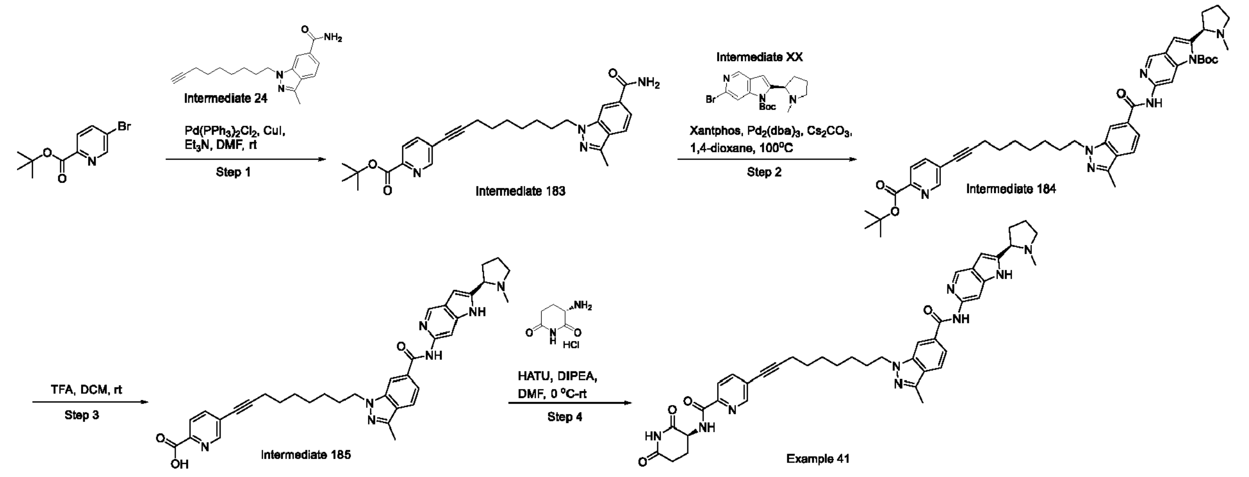


- **Scheme 41**

**Step 1**

**Intermediate 183: *tert*-butyl 5-(9-(6-carbamoyl-3-methyl-1H-indazol-1-yl)non-1-yn-1-yl)picolinate**

A solution of *tert*-butyl-5-bromopicolinate (CAS No: 845306-08-3, 1.5 g, 5.81 mmol), 3-methyl-1-(non-8-yn-1-yl)-1H-indazole-6-carboxamide (**Intermediate 24**, 1.73 g, 5.81 mmol) and triethylamine (21 g, 209 mmol) in dimethylformamide (10 mL) was degassed with argon gas for 10 min, then copper (I) iodide (0.11 g, 0.58 mmol) and $Pd(PPh_3)_2Cl_2$ (0.42 g, 0.58 mmol) were added. The resulting reaction mixture was stirred at room temperature for 3h then poured into water (60 mL), extracted with ethyl acetate (3 × 60 mL). The combined organic layers were dried over anhydrous $Na_2SO_4$, filtered, and concentrated *in vacuo* The crude compound was purified by combi-flash column chromatography, by eluting with 80% ethyl acetate in heptane, to afford *tert*-butyl 5-(9-(6-carbamoyl-3-methyl-1H-indazol-1-yl) non-1-yn-1-yl) picolinate (**Intermediate 183**, 1.60 g, 58%) as an off-white solid. $^1H$ NMR (400 MHz, DMSO-$d_6$) δ ppm 1.24-1.34 (m, 4H), 1.49-1.51 (m, 4H), 1.51 (s, 9H), 1.80-1.82 (m, 2H), 2.42-2.44 (m, 2H), 2.47 (s, 3H), 4.30-4.44 (m, 2H), 7.39-7.40 (m, 1H), 7.56-7.58 (m, 2H), 7.70 (d, *J*=8.40 Hz, 1H), 7.91 (br s, 2H), 8.10 (s, 1H), 8.63 (s, 1H). Mass spec: m/z 475.1 $[M+H]^+$.

**Step 2**

**Intermediate 184: *tert*-butyl-(*R*)-6-(1-(9-(6-(*tert*-butoxycarbonyl)pyridin-3-yl)non-8-yn-1-yl)-3-methyl-1H-indazole-6-carboxamido)-2-(1-methylpyrrolidin-2-yl)-1H-pyrrolo[3,2-c]pyridine-1-carboxylate**

To a solution of *tert*-butyl 5-(9-(6-carbamoyl-3-methyl-1H-indazol-1-yl) non-1-yn-1-yl) picolinate (**Intermediate 183**, 800 mg, 1.69 mmol), in 1,4-dioxane (20 mL) was added tert-butyl-(*R*)-6-bromo-2-(1-methylpyrrolidin-2-yl)-1*H*-pyrrolo [3,2-c]pyridine-1-carboxylate (**Intermediate 1**, 0.51 g, 1.35 mmol) and cesium carbonate (1.10 g, 3.38 mmol). The reaction mixture was degassed with argon gas for 10 min, then $Pd_2(dba)_3$ (297 mg, 0.31 mmol) followed by Xantphos (376 mg, 0.51 mmol) were added and reaction mixture was purged again with argon gas for 5 min, then heated at 100°C for 2h. The reaction mixture was concentrated *in vacuo* to obtain crude compound which was purified by combi-flash column chromatography, by eluting with 80% ethyl acetate in heptane, to obtain *tert*-butyl-(*R*)-6-(1-(9-(6-(tert-butoxycarbonyl) pyridin-3-yl)non-8-yn-1-yl)-3-methyl-1H-indazole-6-carboxamido)-2-(1-methylpyrrolidin-2-yl)-1H-pyrrolo[3,2-c]pyridine-1-carboxylate (**Intermediate 184**, 550 mg, 34%) as an off-white solid. $^1H$ NMR (500 MHz, DMSO-$d_6$) δ ppm 1.31-1.37 (m, 8H), 1.46 (s, 9H) 1.47-1.54 (br s, 5H), 1.69 (s, 9H), 1.70-1.80 (m, 3 H), 1.90-1.91 (m, 2H), 2.35 (s, 3H), 2.36-2.27 (m, 2H), 3.13-3.14 (m, 1H), 3.92-3.98 (m, 1H), 4.41-4.42 (m, 2H), 6.75 (s, 1H), 7.56-7.62 (m, 2H), 7.70-7.77 (m, 2H), 7.90 (s, 1H), 8.43 (s, 1H), 8.62-8.63 (m, 1H), 8.97 (s, 1H), 10.76 (s, 1H). Mass spec: m/z 774.2 $[M+H]^+$.

**Step 3**

**Intermediate 185: (*R*)-5-(9-(3-methyl-6-((2-(1-methylpyrrolidin-2-yl)-1H-pyrrolo[3,2-c]pyridin-6-yl)carbamoyl)-1H-indazol-1-yl)non-1-yn-1-yl)picolinic acid trifluoroacetate**

To a cooled (0°C) solution of *tert*-butyl-(R)-6-(1-(9-(6-(tert-butoxycarbonyl)pyridin-3-yl)non-8-yn-1-yl)-3-methyl-1H-indazole-6-carboxamido)-2-(1-methylpyrrolidin-2-yl)-1H-pyrrolo[3,2-c]pyridine-1-carboxylate (**Intermediate**

**184**, 0.52 g, 0.567 mmol) in dichloromethane (10 mL) was added TFA (4.0 mL) and the resulting reaction mixture was stirred at room temperature for 16h. The reaction mixture was concentrated *in vacuo* to obtain crude (*R*)-5-(9-(3-methyl-6-((2-(1-methylpyrrolidin-2-yl)-1H-pyrrolo[3,2-c]pyridin-6-yl)carbamoyl)-1H-indazol-1-yl)non-1-yn-1-yl)picolinic acid trifluoroacetate (**Intermediate 185**, 400 mg) which was used for next step without further purification. $^1$H NMR (500 MHz, DMSO-$d_6$) δ ppm 1.08-1.10 (m, 1H), 1.31-1.37 (m, 6H), 1.52-1.54 (m, 3H), 1.85-1.88 (m, 3H), 2.19 (s, 3H), 2.45-2.54 (m, 4H), 2.87 (s, 3H), 3.30-3.31 (m, 1H), 3.80-3.81 (m, 1H), 4.43-4.44 (m, 1H), 4.70-4.71 (m, 1H), 7.10 (s, 1H), 7.54-7.62 (m, 3H), 7.74 (d, *J*=8.0 Hz, 1H), 7.87-7.98 (m, 3H), 8.11 (s, 1H), 8.63 (s, 1H), 8.98 (s, 2H), 11.40 (br s, 1H).

**Step 4**

**Example 41: 1-(9-(6-(((*S*)-2,6-dioxopiperidin-3-yl)carbamoyl)pyridin-3-yl)non-8-yn-1-yl)-3-methyl-N-(2-((*R*)-1-methylpyrrolidin-2-yl)-1H-pyrrolo [3,2-c] pyridin-6-yl)-1*H*-indazole-6-carboxamide**

To a cooled (0°C) solution of (*R*)-5-(9-(3-methyl-6-((2-(1-methylpyrrolidin-2-yl)-1H-pyrrolo[3,2-c]pyridin-6-yl) carbamoyl)-1H-indazol-1-yl)non-1-yn-1-yl)picolinic acid trifluoroacetate (**Intermediate 185**, 400 mg, 0.64 mmol), (*S*)-3-aminopiperidine-2,6-dione hydrochloride (CAS No: 25181-50-4, 126 mg, 0.77 mmol) and HATU (388 mg, 0.97 mmol) in dimethylformamide (10.0 mL) was added DIPEA (440 mg, 3.23 mmol). The resulting reaction mixture was stirred at room temperature for 16h then poured into water (15 mL). The resultant precipitate was collected by filtration and dried *in vacuo* to obtain the crude compound as brown solid (400 mg) which was purified by preparative HPLC (Method A) to obtain 1-(9-(6-(((*S*)-2,6-dioxopiperidin-3-yl)carbamoyl)pyridin-3-yl)non-8-yn-1-yl)-3-methyl-N-(2-((*R*)-1-methylpyrrolidin-2-yl)-1H-pyrrolo[3,2-c]pyridin-6-yl)-1H-indazole-6-carboxamide (**Example 41**, 85 mg 18%) as an off-white solid. $^1$H NMR (400 MHz, DMSO-$d_6$) δ ppm 1.30-1.40 (m, 6H), 1.54-1.57 (m, 2H), 1.84-1.88 (m, 5H), 1.90-1.95 (m, 1H), 2.17 (s, 3H) 2.18-2.25 (m, 3H), 2.45-2.47 (m, 3H), 2.49 (s, 3H), 2.52-2.54 (m, 1H), 3.13-3.17 (m, 1H), 3.30-3.31 (m, 1H), 4.41 (t, *J*=7.2 Hz, 2H), 4.72-4.79 (m, 1H), 6.40 (s, 1H), 7.72-7.79 (m, 2H), 7.94-8.00 (m, 2H), 8.24 (s, 1H), 8.44 (s, 1H), 8.52 (s, 1H), 8.63-8.64 (m, 1H), 9.06 (d, *J*=8.4 Hz, 1H), 10.51 (s, 1H), 10.91 (s, 1H), 11.41 (s, 1H). Mass spec: m/z 728.1 [M+H]$^+$.

**Example 42 was synthesized following Scheme 42**

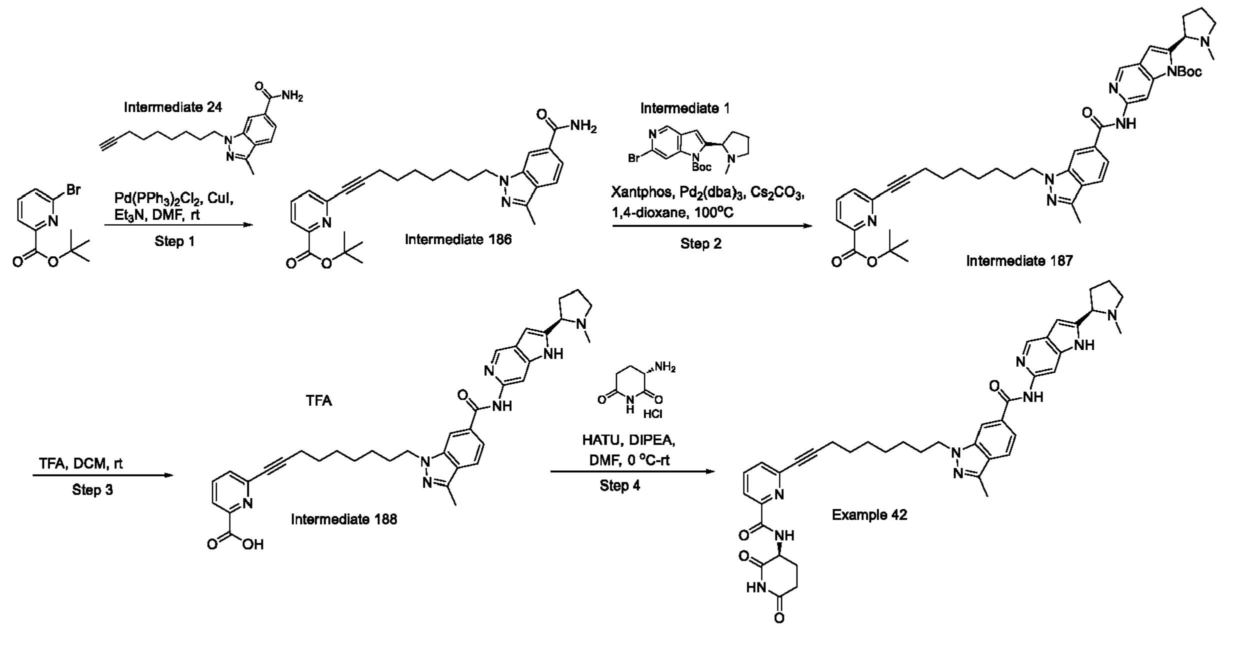


## Scheme 42

**Step 1**

**Intermediate 186: tert-butyl 6-(9-(6-carbamoyl-3-methyl-1H-indazol-1-yl) non-1-yn-1-yl) picolinate**

To a solution of *tert*-butyl 6-bromopicolinate (CAS No: 910044-07-4, 1.5 g, 5.81 mmol) in dimethylformamide (10

mL) was added 3-methyl-1-non-8-ynyl-indazole-6-carboxamide (**Intermediate 24**, 1.7 g, 5.81 mmol) and triethylamine (29 g, 209 mmol). The resulting solution was degassed with argon gas for 10 min, then copper (I) iodide (0.11 g, 0.58 mmol) and $PdCl_2(PPh_3)_2$ (0.42 g, 0.58 mmol) were added and the reaction mixture was again purged with argon gas for 10 min. The resulting reaction mixture was stirred at room temperature for 3h. The reaction mixture was poured into water (60 mL) and extracted with ethyl acetate (3 × 60 mL). The combined organic layers were dried over anhydrous $Na_2SO_4$, filtered, and concentrated *in vacuo.* The crude material was purified by combi-flash column chromatography, by eluting with 80% ethyl acetate in heptane, to afford *tert*-butyl 6-(9-(6-carbamoyl-3-methyl-1H-indazol-1-yl) non-1-yn-1-yl) picolinate (**Intermediate 186**, 1.90 g, 69%) as an off white solid. $^1$H NMR (400 MHz, DMSO-$d_6$) δ ppm 1.15-1.19 (m, 2H), 1.27-1.35 (m, 6H), 1.50-1.51 (m, 2H) 1.52 (s, 9H), 1.82-1.84 (m, 2H), 1.44 (s, 3H), 4.33-4.35 (m, 2H), 7.42 (s, 1H), 7.58-7.60 (m, 2H), 7.72 (d, *J*=7.6 Hz, 1H), 7.90 (br s, 2H), 8.04 (br s, 1H), 8.13 (br s, 1H). Mass spec m/z 475.10 [M+H]$^+$.

**Step 2**

**Intermediate 187: *tert*-butyl (*R*)-6-(1-(9-(6-(tert-butoxycarbonyl)pyridin-2-yl)non-8-yn-1-yl)-3-methyl-1H-indazole-6-carboxamido)-2-(1-methylpyrrolidin-2-yl)-1H-pyrrolo[3,2-c]pyridine-1-carboxylate**

To a solution of *tert*-butyl 6-(9-(6-carbamoyl-3-methyl-1H-indazol-1-yl)non-1-yn-1-yl)picolinate (**Intermediate 186**, 0.798 g, 1.68 mmol) in 1,4-dioxane (15 mL) was added *tert*-butyl (*R*)-6-bromo-2-(1-methylpyrrolidin-2-yl)-1H-pyrrolo [3,2-c]pyridine-1-carboxylate (**Intermediate 1**, 0.80 g, 2.10 mmol) and cesium carbonate (1.38 g, 4.20 mmol) and the solution was purged with argon gas for 10 min prior to the addition of $Pd_2(dba)_3$ (297 mg, 0.31 mmol) followed by Xantphos (376 mg, 0.63 mmol). The resulting reaction mixture was heated at 100°C for 2h. The mixture was concentrated *in vacuo* to obtain crude compound which was purified by combi-flash chromatography, by eluting with 80% ethyl acetate in heptane, to afford *tert*-butyl (*R*)-6-(1-(9-(6-(tert-butoxycarbonyl)pyridin-2-yl)non-8-yn-1-yl)-3-methyl-1H-indazole-6-carboxamido)-2-(1-methylpyrrolidin-2-yl)-1H-pyrrolo[3,2-c]pyridine-1-carboxylate (**Intermediate 187**, 510 mg, 31%) as a yellow solid. $^1$H NMR (500 MHz, DMSO-*d*6) δ ppm 1.3-1.40 (m, 8H), 1.5 (s, 9H), 1.56-159 (m, 4H), 1.71 (s, 9H), 1.75-1.77 (m, 1H), 1.86-1.91 (m, 2H), 2.33 (s, 3H), 2.43-2.46 (m, 2H), 2.52 (s, 3H), 3.12-3.15 (m, 1H), 3.91-3.94 (m, 1H), 4.34-4.43 (m, 2H), 6.76 (s, 1H), 7.59-7.60 (m, 1H), 7.71-7.74 (m, 2H), 7.88-7.90 (m, 2H), 8.44 (s, 1H), 8.60 (d, *J*=8 Hz, 1H), 8.98 (s, 1H), 10.77 (s, 1H). Mass spec m/z 774.4 [M+H]$^+$.

**Step 3**

**Intermediate 188: (*R*)-6-(9-(3-methyl-6-((2-(1-methylpyrrolidin-2-yl)-1H-pyrrolo[3,2-c]pyridin-6-yl)carbamoyl)-1H-indazol-1-yl)non-1-yn-1-yl)picolinic acid trifluoroacetate**

To a cooled (0°C solution of *tert*-butyl (*R*)-6-(1-(9-(6-(tert-butoxycarbonyl)pyridin-2-yl)non-8-yn-1-yl)-3-methyl-1H-indazole-6-carboxamido)-2-(1-methylpyrrolidin-2-yl)-1H-pyrrolo[3,2-c]pyridine-1-carboxylate (**Intermediate 187**, 500 mg, 0.65 mmol) in DCM (5 mL) was added TFA (2 mL). The resulting reaction mixture was stirred at room temperature for 16h. The reaction mixture was concentrated *in vacuo* to obtain crude (*R*)-6-(9-(3-methyl-6-((2-(1-methylpyrrolidin-2-yl)-1H-pyrrolo[3,2-c]pyridin-6-yl)carbamoyl)-1H-indazol-1-yl)non-1-yn-1-yl)picolinic acid trifluoroacetate (**Intermediate 88**, 390 mg) as a brown sticky solid which was used directly in next step without further purification. Mass spec m/z 618.42 [M+H]$^+$.

**Step 4**

**Example 42: 1-(9-(6-(((*S*)-2,6-dioxopiperidin-3-yl)carbamoyl)pyridin-2-yl)non-8-yn-1-yl)-3-methyl-N-(2-((*R*)-1-methylpyrrolidin-2-yl)-1H-pyrrolo [3,2-c] pyridin-6-yl)-1*H*-indazole-6-carboxamide**

To a cooled (0°C) solution of (*R*)-6-(9-(3-methyl-6-((2-(1-methylpyrrolidin-2-yl)-1H-pyrrolo[3,2-c]pyridin-6-yl) carbamoyl)-1H-indazol-1-yl)non-1-yn-1-yl)picolinic acid trifluoroacetate (**Intermediate 188**, 400 mg, 0.64 mmol), (*S*)-3-aminopiperidine-2,6-dione hydrochloride (CAS No: 25181-50-4, 127 mg, 0.77 mmol) and HATU (388 mg, 0.97 mmol) in dimethylformamide (10 mL) was added DIPEA (440 mg, 3.23 mmol). The resulting reaction mixture was stirred at room temperature for 16h. The mixture was poured into water (15 mL), the resultant brown precipitate collected by filtration and dried *in vacuo.* The crude solid was purified by preparative HPLC (Method A) to afford 1-(9-(6-(((*S*)-2,6-dioxopiperidin-3-yl)carbamoyl)pyridin-2-yl)non-8-yn-1-yl)-3-methyl-*N*-(2-((*R*)-1-methylpyrrolidin-2-yl)-1H-pyrrolo[3,2-c]pyridin-6-yl)-1H-indazole-6-carboxamide (**Example 42**, 75 mg, 16%) as an off white solid. $^1$H NMR (400 MHz, DMSO-$d_6$) δ ppm 1.30-1.38 (m, 7H), 1.53-1.57 (m, 2H), 1.85-1.97 (m, 7H),1.98-2.18 (m, 3H), 2.20 (s, 3H), 2.49 (s, 3H), 2.54-2.55 (m, 2H), 2.67-2.75 (m, 1H), 3.20-3.31 (m, 1H), 4.40 (t, *J*= 7.20 Hz, 2H), 4.77-4.81 (m, 1H), 6.40 (s, 1H), 7.62-7.64 (m, 1H), 7.77-7.80 (m, 2H), 7.92-8.00 (m, 2H), 8.25 (s, 1H), 18.43 (s, 1H), 8.52 (s, 1H), 8.96 (d, *J*=8.80 Hz, 1H), 10.56 (s, 1H), 11.50 (s, 1H). Mass spec

m/z 728.10 $[M+H]^+$.

**Example 43 was synthesized following Scheme 43**

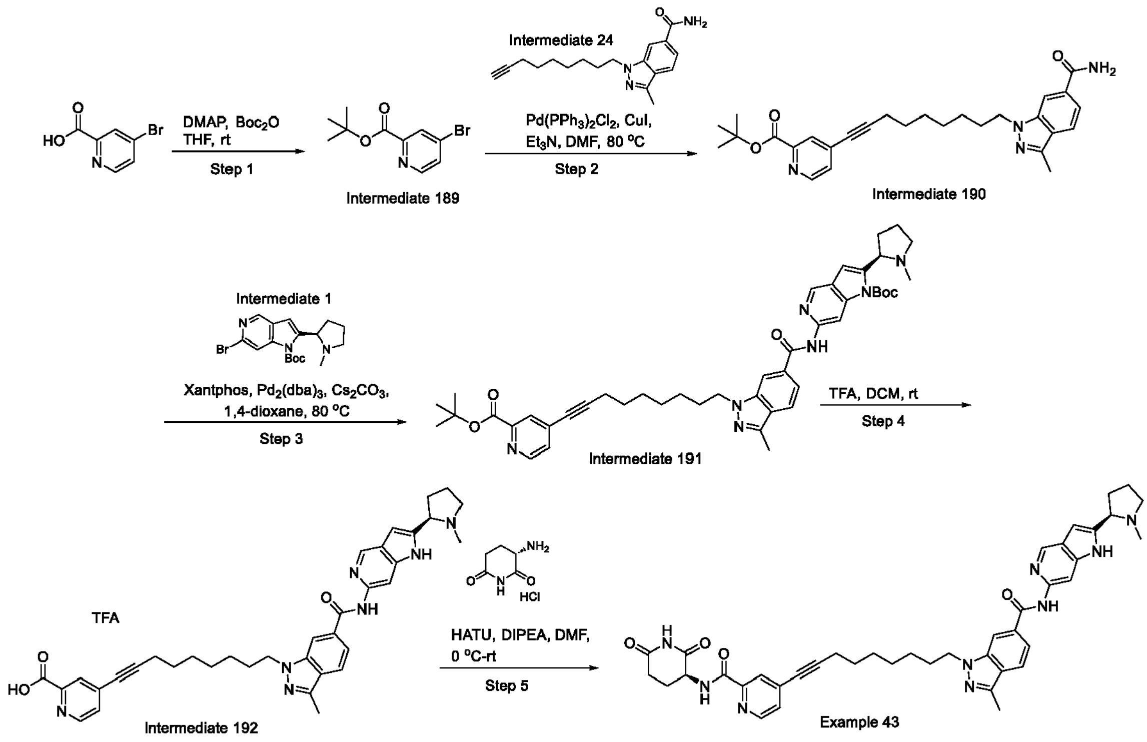


**Scheme 43**

**Step 1**

**Intermediate 189: *tert*-butyl 4-bromopicolinate**

To a solution of 4-bromopyridine-2-carboxylic acid (CAS No: 30766-03-1, 5.50 g, 27 mmol) and 4-dimethylaminopyridine (1.66 g, 14 mmol) in tetrahydrofuran (120 mL) was added di-*tert*-butyldicarbonate (8.80 g, 41 mmol) dropwise over 10 min and the reaction mixture was stirred at room temperature for 16h. Water (100 mL) was added and the mixture extracted with diethyl ether (2 × 100 mL). The combined organic layers were washed sequentially with water (100 mL), 5% $NaHCO_3$ (50 mL) and brine solution (50 mL). The combined organic layers were dried over anhydrous $Na_2SO_4$ and concentrated *in vacuo* to afford the crude product which was purified by combi-flash column chromatography, by eluting with 30% ethyl acetate in heptane, to afford *tert*-butyl 4-bromopicolinate (**Intermediate 189**, 6 g, 85%) as a colourless liquid. $^1H$ NMR (400 MHz, DMSO-$d_6$) δ ppm 1.55 (s, 9H), 7.90 (d, *J*=4.4 Hz, 1H), 8.13 (s, 1H), 8.57 (d, *J*=4.8 Hz, 1H). Mass spec m/z 259.90 $[M+H]^+$.

**Step 2**

**Intermediate 190: *tert*-butyl 4-(9-(6-carbamoyl-3-methyl-1H-indazol-1-yl)non-1-yn-1-yl)picolinate**

To a solution of 3-methyl-1-(non-8-yn-1-yl)-1H-indazole-6-carboxamide (**Intermediate 24**, 1.65 g, 5.54 mmol ) in acetonitrile (20 mL) was added *tert*-butyl 4-bromopyridine-2-carboxylate (**Intermediate 189**, 1.30 g, 5.04 mmol) followed by triethylamine (2.05 g, 20.16 mmol). The reaction mixture was purged with argon gas for 10 min, then $PdCl_2(PPh_3)_2$ (353 mg, 0.504 mmol) and copper (I) iodide (96 mg, 0.504 mmol) were added and the reaction was purged with argon gas for 10 min then stirred at 80°C for 2h. The reaction mixture was concentrated *in vacuo* and the crude residue was diluted with saturated sodium thiosulphate solution (200 mL) and extracted with ethyl acetate (3 × 200 mL). The combined organic layers were dried over anhydrous $Na_2SO_4$ and concentrated *in vacuo.* The crude product was purified by combi-flash

column chromatography, by eluting with 80% ethyl acetate in heptane, to afford *tert*-butyl 4-(9-(6-carbamoyl-3-methyl-1H-indazol-1-yl) non-1-yn-1-yl) picolinate (**Intermediate 190**, 1.8 g, 75%) as a yellow solid. $^1$H NMR (500 MHz, DMSO-$d_6$) δ ppm 1.26-1.35 (m, 8H), 1.54 (s, 9H), 1.83-1.85 (m, 2H), 2.44-2.46 (m, 2H), 2.49 (s, 3H), 4.33-4.34 (m, 2H), 7.45 (br s, 1H), 7.54-7.60 (m, 2H), 7.71-7.77 (m, 2H), 7.84 (s, 1H), 8.10 (br s, 1H), 8.12 (s, 1H). Mass spec m/z 475.1 [M+H]$^+$.

**Step 3**

**Intermediate 191: *tert*-butyl (*R*)-6-(1-(9-(2-(tert-butoxycarbonyl)pyridin-4-yl)non-8-yn-1-yl)-3-methyl-1H-indazole-6-carboxamido)-2-(1-methylpyrrolidin-2-yl)-1H-pyrrolo[3,2-c]pyridine-1-carboxylate**

To a solution of *tert*-butyl (*R*)-6-bromo-2-(1-methylpyrrolidin-2-yl)-1H-pyrrolo[3,2-c]pyridine-1-carboxylate (**Intermediate 1**, 1.0 g, 2.70 mmol) in 1,4-dioxane (15 mL) was added *tert*-butyl 4-(9-(6-carbamoyl-3-methyl-1H-indazol-1-yl) non-1-yn-1-yl)picolinate (**Intermediate 190**, 0.9 g, 1.89 mmol) and cesium carbonate (1.86 g, 5.70 mmol) and the solution was degassed with argon gas for 10 min. $Pd_2(dba)_3$ (260 mg, 0.31 mmol) was added, followed by Xantphos (328 mg, 0.57 mmol) and the mixture was purged again with argon gas for 10 min then heated at 80°C for 2h. The reaction mixture was concentrated *in vacuo* and the crude material was purified by combi-flash chromatography, by eluting with 80% ethyl acetate in heptane, to obtain *tert*-butyl (*R*)-6-(1-(9-(2-(tert-butoxycarbonyl)pyridin-4-yl)non-8-yn-1-yl)-3 -methyl- 1H-indazole-6-carboxamido)-2-(1-methylpyrrolidin-2-yl)-1H-pyrrolo[3,2-c]pyridine-1-carboxylate (**Intermediate 191**, 0.81 g, 50%) as a yellow solid. $^1$H NMR (400 MHz, DMSO-$d_6$) δ ppm 1.17-1.90 (m,1H), 1.29-1.36 (m, 6H), 1.53 (s, 9H) 1.54-1.65 (m, 3H), 1.68-1.74 (m, 3H), 1.75 (s, 9H), 1.87-1.89 (m, 2H), 2.35 (s, 3H), 2.40-2.50 (m, 2H), 2.51 (s, 3H), 3.12-3.13 (m, 1H), 3.92-3.93 (m, 1H), 4.39-4.40 (m, 2H), 6.75 (s, 1H), 7.50-7.52 (m, 1H), 7.71-7.81 (m, 3H), 8.43 (s, 1H), 8.61-8.62 (m, 2H), 8.97 (s, 1H), 10.75 (s, 1H). Mass spec m/z 774.3 [M+H]$^+$.

**Step 4**

**Intermediate 192: (*R*)-4-(9-(3-methyl-6-((2-(1-methylpyrrolidin-2-yl)-1H-pyrrolo[3,2-c] pyridin-6-yl) carbamoyl)-1H-indazol-1-yl) non-1-yn-1-yl)picolinic acid trifluoroacetate**

To a cooled (0°C) solution of *tert*-butyl (*R*)-6-(1-(9-(2-(tert-butoxycarbonyl)pyridin-4-yl)non-8-yn-1-yl)-3-methyl-1H-indazole-6-carboxamido)-2-(1-methylpyrrolidin-2-yl)-1H-pyrrolo[3,2-c]pyridine-1-carboxylate (**Intermediate 191**, 800 mg, 1.03 mmol) in DCM (3 mL) was added TFA (3 mL). The resulting reaction mixture was stirred at room temperature for 16h then concentrated *in vacuo* to obtain crude (*R*)-4-(9-(3-methyl-6-((2-(1-methylpyrrolidin-2-yl)-1H-pyrrolo[3,2-c]pyridin-6-yl)carbamoyl)-1H-indazol-1-yl)non-1-yn-1-yl)picolinic acid trifluoroacetate (**Intermediate 192**, 0.90 g) as a yellow solid which was used directly into next step without further purification. Mass spec m/z 618.34 [M+H]$^+$.

**Step 5**

**Example 43: 1-(9-(2-(((*S*)-2,6-dioxopiperidin-3-yl)carbamoyl)pyridin-4-yl)non-8-yn-1-yl)-3-methyl-N-(2-((R)-1-methylpyrrolidin-2-yl)-1H-pyrrolo [3,2-c] pyridin-6-yl)-1*H*-indazole-6-carboxamide**

To a cooled (0°C) solution of (*R*)-4-(9-(3-methyl-6-((2-(1-methylpyrrolidin-2-yl)-1H-pyrrolo[3,2-c]pyridin-6-yl) carbamoyl)-1H-indazol-1-yl)non-1-yn-1-yl)picolinic acid trifluoroacetate (**Intermediate 192**, 0.9 g, 1.45 mmol) and (*S*)-3-aminopiperidine-2,6-dione hydrochloride (CAS No: 25181-50-4, 0.24 g, 1.45 mmol) in dimethylformamide (8 mL) were added HATU (0.66 g, 1.74 mmol) and DIPEA (0.93 g, 7.25 mmol). The reaction mixture was stirred at room temperature for 16h. The mixture was poured into ice cold water (50 mL), the resultant precipitate collected by filtration and dried *in vacuo* to afford the crude product which was purified by preparative HPLC (Method A) to afford 1-(9-(2-(((*S*)-2,6-dioxopiperidin-3-yl) carbamoyl)pyridin-4-yl)non-8-yn-1-yl)-3-methyl-N-(2-((*R*)-1-methylpyrrolidin-2-yl)-1H-pyrrolo[3,2-c]pyridin-6-yl)-1H-indazole-6-carboxamide (**Example 43**, 0.10 g, 9%) as an off-white solid. $^1$H NMR (400 MHz, DMSO-$d_6$) δ ppm 1.30-1.39 (m, 6H), 1.53-1.57 (m, 2H), 1.81-1.87 (m, 5H), 1.89-1.98 (m, 1H), 2.20 (s, 3H), 2.46-2.49 (m, 3H), 2.51-2.53 (m, 2H), 2.54 (s, 3H), 2.55 (m, 1H), 2.58-2.59 (m, 1H), 3.14-3.17 (m, 1H), 3.30-3.34 (m, 1H), 4.41 (t, *J*=6.8 Hz, 2H), 4.77-4.81 (m, 1H), 6.40 (s, 1H), 7.54-7.56 (m, 1H), 7.70-7.72 (m, 2H), 7.89 (d, J=4.0 Hz, 1H), 8.24 (s, 1H), 8.44 (s, 1H), 8.51 (s, 1H), 8.61 (s, 1H), 9.08 (d, *J*=8.4 Hz, 1H), 10.53 (s, 1H), 10.87 (s, 1H), 11.38 (s, 1H). Mass spec m/z 728.10 [M+H]$^+$.

**Example 44 was synthesized following Scheme 44**

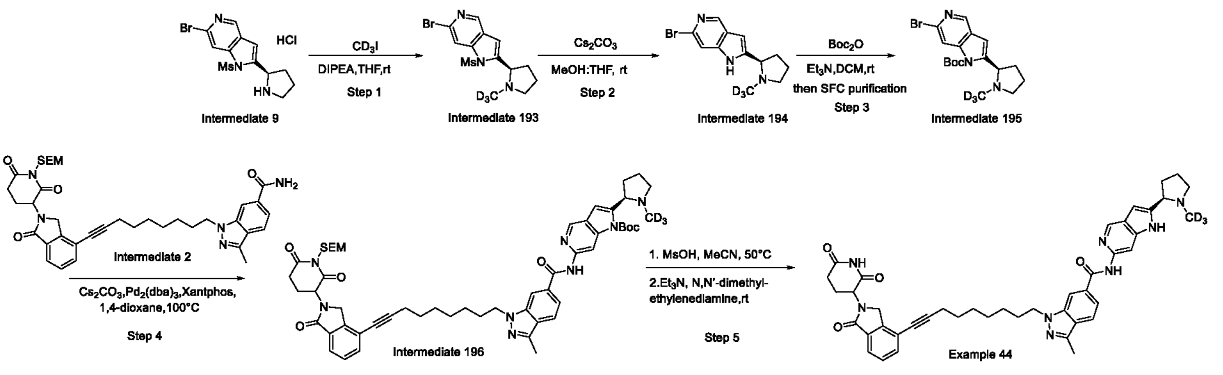


## Scheme 44

**Step** 1

**Intermediate 193: (R)-6-bromo-2-(1-(methyl-d3) pyrrolidin-2-yl)-1-(methylsulfonyl)-1*H*-pyrrolo [3, 2-c] pyridine**

To a cooled (0°C) solution of (R)-6-bromo-1-(methylsulfonyl)-2-(pyrrolidin-2-yl)-1*H*-pyrrolo[3,2-c]pyridine hydrochloride (**Intermediate 9**, 10.0 g, 29.1 mmol) in tetrahydrofuran (40 mL) was added DIPEA (15.8 g, 116 mmol) followed by $CD_3I$ (6.32 g, 43.6 mmol). The reaction mixture was stirred at room temperature for 16h. The reaction mixture was poured into water (100 mL) and extracted with ethyl acetate (3 × 150 mL). The organic layer was separated, dried over anhydrous $Na_2SO_4$, filtered, and concentrated *in vacuo.* The crude material was purified by combi-flash chromatography, by eluting with 80% ethyl acetate in heptane, to afford (R)-6-bromo-2-(1-(methyl-d3)pyrrolidin-2-yl)-1-(methylsulfonyl)-1*H*-pyrrolo [3,2-c]pyridine (**Intermediate 193**, 5.2 g, 50%) as a yellow solid. $^1$H NMR (400 MHz, DMSO-$d_6$) δ ppm 1.68-1.80 (m, 3H), 2.29-2.38 (m, 2H), 3.10-3.12 (m, 1H), 3.56 (s, 3H), 3.77-3.81 (m, 1H), 6.89 (s, 1H), 7.96 (s, 1H), 8.68 (s, 1H). Mass spec m/z 362.8 $[M+2H]^+$.

**Step 2**

**Intermediate 194: (R)-6-bromo-2-(1-(methyl-d3)pyrrolidin-2-yl)-1*H*-pyrrolo[3, 2-c]pyridine**

To a cooled (0°C) solution of (R)-6-bromo-2-(1-(methyl-d3) pyrrolidin-2-yl)-1-(methylsulfonyl)-1*H*-pyrrolo [3, 2-c] pyridine (**Intermediate 193**, 5.0 g, 14 mmol) in tetrahydrofuran (20 mL) and methanol (20 mL) was added cesium carbonate (9.0 g, 28 mmol), then the reaction mixture was stirred at room temperature for 2h. The reaction mixture was poured into ice cold water (100 mL) and extracted with dichloromethane (3 × 100 mL). The combined organic layers were dried over anhydrous $Na_2SO_4$, filtered, and concentrated *in vacuo* to afford (R)-6-bromo-2-(1-(methyl-d3) pyrrolidin-2-yl)-1*H*-pyrrolo[3, 2-c]pyridine (**Intermediate 194**, 3.2 g, 77%) as an off-white solid which was used for the next step without further purification. $^1$H NMR (400 MHz, DMSO-$d_6$) δ ppm 1.70-1.94 (m, 3H), 2.12-2.19 (m, 1H), 2.23-2.30 (m, 1H), 3.13-3.15 (m, 1H), 3.32-3.38 (m, 1H), 6.45 (s, 1H), 7.43 (s, 1H), 8.48 (s, 1H), 11.57 (br s, 1H).

**Step 3**

**Intermediate 195: *tert*-butyl (*R*)-6-bromo-2-(1-(methyl-d3) pyrrolidin-2-yl)-1*H*-pyrrolo [3, 2-c] pyridine-1-carboxylate**

To a solution of (R)-6-bromo-2-(1-(methyl-d3) pyrrolidin-2-yl)-1*H*-pyrrolo [3, 2-c] pyridine (**Intermediate 194**, 3.0 g, 11 mmol) in dichloromethane (30 mL) was added triethylamine (3.2 g, 32 mmol) followed by di-tert-butyl dicarbonate (3.5 g, 16 mmol) and 4-dimethylaminopyridine (0.13 g, 1.1 mmol) and the reaction mixture was stirred at room temperature for 16h. The reaction mixture was poured into water (100 mL) and extracted with dichloromethane (3 × 100 mL). The combined organic layers were dried over anhydrous $Na_2SO_4$, filtered, and concentrated *in vacuo* to obtain the crude compound. The crude material was purified by combi-flash chromatography, by eluting with 50% ethyl acetate in heptane, to afford *tert*-butyl (R)-6-bromo-2-(1-(methyl-d3)pyrrolidin-2-yl)-1*H*-pyrrolo[3,2-c]pyridine-1-carboxylate (2.5 g) as an off white solid. This material was further purified by SFC chiral purification (Column: Chiralpak IK (250 * 30 mm) 5 um. Isocratic elution with 25% Mobile Phase (A): $CO_2$; mobile Phase (B): MeCN + isopropylalcohol) to afford (R)-6-bromo-2-(1-(methyl-d3) pyrrolidin-2-yl)-1*H*-pyrrolo[3, 2-c]pyridine-1-carboxylate (**Intermediate 195**, 1.8 g, 44%) as an off white solid. $^1$H NMR (400 MHz, DMSO-$d_6$) δ ppm 1.65 (s, 9H), 1.69-1.81 (m, 3H), 2.31-2.36 (m, 2H), 3.07-3.14 (m, 1H), 3.85-3.62 (m, 1H), 6.79

(s, 1H), 8.04 (s, 1H), 8.60 (s, 1H). Mass spec m/z 384.9 [M+2H]$^+$.

**Step 4**

**Intermediate 196: *tert*-butyl 6-(1-(9-(2-(2,6-dioxo-1-((2-(trimethylsilyl)ethoxy)methyl)piperidin-3-yl)-1-oxoisoindolin-4-yl)non-8-yn-1-yl)-3-methyl-1*H*-indazole-6-carboxamido)-2-((R)-1-(methyl-d3)pyrrolidin-2-yl)-1*H*-pyrrolo [3,2-c]pyridine-1-carboxylate**

To a solution of 1-(9-(2-(2,6-dioxo-1-((2-(trimethylsilyl)ethoxy)methyl)piperidin-3-yl)-1-oxoisoindolin-4-yl)non-8-yn-1-yl)-3-methyl-1*H*-indazole-6-carboxamide (**Intermediate 2**, 0.56 g, 0.83 mmol) and *tert*-butyl (R)-6-bromo-2-(1-(methyl-d3)pyrrolidin-2-yl)-1*H*-pyrrolo[3,2-c]pyridine-1-carboxylate (**Intermediate 195**, 0.40 g, 1.04 mmol) in 1,4-dioxane (20 mL) was added cesium carbonate (0.68 g, 2.08 mmol). The reaction mixture was purged with argon gas for 20 min then Xantphos (0.18 g, 0.31 mmol) and $Pd_2(dba)_3$(0.15 g, 0.15 mmol) were added. The reaction mixture was purged with argon gas for 10 min and then heated at 100°C for 2h. The reaction mixture was concentrated *in vacuo* and the crude material was purified by combi-flash chromatography, by eluting with 80% ethyl acetate in heptane, to afford *tert*-butyl 6-(1-(9-(2-(2,6-dioxo-1-((2-(trimethylsilyl)ethoxy)methyl)piperidin-3-yl)-1-oxoisoindolin-4-yl)non-8-yn-1-yl)-3-methyl-1*H*-indazole-6-carboxamido)-2-((R)-1-(methyl-d3)pyrrolidin-2-yl)-1*H*-pyrrolo[3,2-c]pyridine-1-carboxylate (**Intermediate 196**, 0.39 g, 38%) as a yellow solid. $^1$H NMR (400 MHz, DMSO-$d_6$) δ ppm -0.06 (s, 9H), 0.79-0.89 (m, 2H), 1.27-1.38 (m, 8H), 1.51-1.54 (m, 2H), 1.70 (s, 9H), 1.74-1.80 (m, 2H), 1.86 (s, 3H), 2.33-2.45 (m, 4H), 2.67-2.79 (m, 2H), 3.01-3.13 (m, 2H), 3.38-3.40 (m, 2H), 3.48-3.50 (m, 2H), 3.87-3.92 (m, 1H), 4.21-4.24 (m, 1H), 4.40 (s, 2H), 4.42-4.56 (m, 2H), 4.99-5.07 (m, 1H), 5.24-5.28 (m, 1H), 6.76 (s, 1H), 7.49-7.59 (m, 2H), 7.71-7.80 (m, 3H), 8.42 (s, 1H), 8.60 (s, 1H), 8.97 (s, 1H), 10.75 (br s, 1H). Mass spec m/z 972.2 [M+H]$^+$.

**Step 5**

**Example 44: 1-(9-(2-(2,6-dioxopiperidin-3-yl)-1-oxoisoindolin-4-yl)non-8-yn-1-yl)-3-methyl-*N*-(2-((*R*)-1-(methyl-d3)pyrrolidin-2-yl)-1*H*-pyrrolo[3,2-c]pyridin-6-yl)-1*H*-indazole-6-carboxamide**

To a solution of *tert*-butyl 6-(1-(9-(2-(2,6-dioxo-1-((2-(trimethylsilyl)ethoxy)methyl)piperidin-3-yl)-1-oxoisoindolin-4-yl)non-8-yn-1-yl)-3-methyl-1*H*-indazole-6-carboxamido)-2-((R)-1-(methyl-d3)pyrrolidin-2-yl)-1*H*-pyrrolo[3,2-c]pyridine-1-carboxylate (**Intermediate 196**, 0.38 g, 0.39 mmol) in acetonitrile (10 mL) was added methanesulfonic acid (0.38 g, 3.90 mmol) and the reaction mixture was heated at 50°C for 2h. The reaction mixture was cooled to room temperature then *N*,*N*'-dimethylethylenediamine (0.19 g, 1.95 mmol) followed by triethylamine (0.79 g, 7.81 mmol) were added and the mixture stirred for 3h at room temperature. The reaction mixture was concentrated *in vacuo* and poured into water (20 mL). The resultant precipitate was collected by filtration and dried *in vacuo* to afford the crude compound. The crude material was purified by preparative HPLC (Method A) to afford to afford 1-(9-(2-(2,6-dioxopiperidin-3-yl)-1-oxoisoindolin-4-yl) non-8-yn-1-yl)-3-methyl-*N*-(2-((R)-1-(methyl-d3)pyrrolidin-2-yl)-1*H*-pyrrolo[3,2-c]pyridin-6-yl)-1*H*-indazole-6-carboxamide (**Example 44**, 0.025 g, 9%) as an off white solid. $^1$H NMR (400 MHz, DMSO-$d_6$) δ ppm 1.32-1.39 (m, 6H), 1.52-1.56 (m, 2H), 1.78-1.92 (m, 5H), 1.98-2.03 (m, 1H), 2.14-2.17 (m, 1H), 2.25-2.28 (m, 1H), 2.41-2.48 (m, 5H), 2.54-2.59 (m, 3H), 2.84-2.95 (m, 1H), 3.12-3.16 (m, 1H), 4.26-4.31 (m, 1H), 4.38-4.45 (m, 3H), 5.10-5.15 (m, 1H), 6.40 (s, 1H), 7.49 (t, *J*=7.57 Hz, 1H), 7.59 (d, *J*=6.88 Hz, 1H), 7.66-7.75 (m, 2H), 7.75-7.79 (m, 1H), 8.25 (s, 1H), 8.43 (s, 1H), 8.51 (s, 1H), 10.51 (s, 1H), 11.00 (br s, 1H), 11.38 (br s, 1H). Mass spec m/z 742.3 [M+H]$^+$.

**Example 45 was synthesized following Scheme 45**

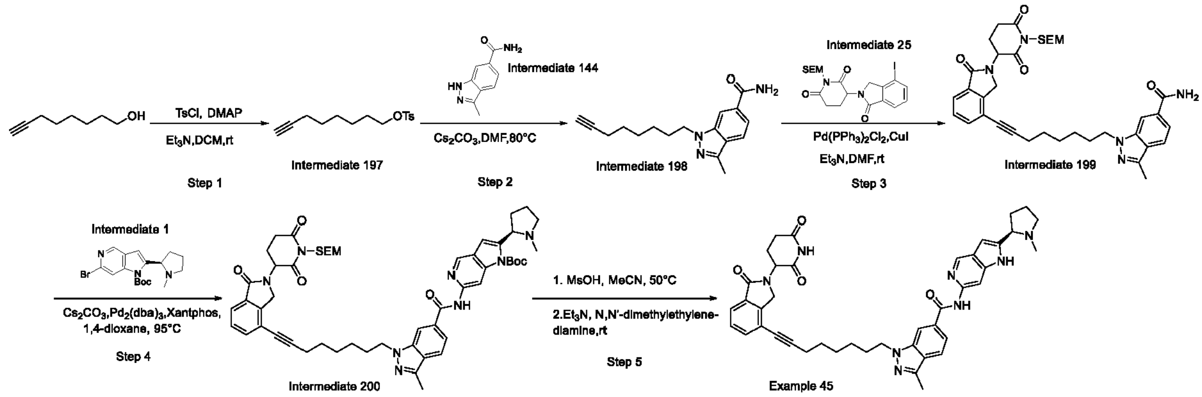


# Scheme 45

**Step 1**

**Intermediate 197: oct-7-yn-1-yl 4-methylbenzenesulfonate**

To a cooled (0°C) solution of oct-7-yn-1-ol (CAS No: 871-91-0, 5.0 g, 40 mmol) in dichloromethane (20 mL) was added triethylamine (13 g, 120 mmol) followed by p-toluenesulfonyl chloride (8.8 mL, 59 mmol) and 4-dimethylaminopyridine (0.51 g, 4.0 mmol). The reaction mixture was stirred at room temperature for 16h then poured into ice cold water (50 mL) and extracted with DCM (2 × 100 mL). The combined organic layers were dried over anhydrous $Na_2SO_4$, filtered, and concentrated *in vacuo*. The crude material was purified by combi-flash chromatography, by eluting with 50% ethyl acetate in heptane, to afford oct-7-yn-1-yl 4-methylbenzenesulfonate (**Intermediate 197**, 7.2 g, 65%) as a yellow liquid. $^1H$ NMR (400 MHz, $CDCl_3$) δ ppm 1.32-1.38 (m, 4H), 1.43-1.50 (m, 2H), 1.61-1.66 (m, 2H), 1.92 (s, 1H), 2.12-2.16 (m, 2H), 2.45 (s, 3H), 4.02 (t, *J*=6.47 Hz, 2H), 7.34 (d, *J*=7.89 Hz, 2H), 7.79 (d, *J*=8.40 Hz, 2H).

**Step 2**

**Intermediate 198: 3-methyl-1-(oct-7-yn-1-yl)-1*H*-indazole-6-carboxamide**

A mixture of oct-7-yn-1-yl 4-methylbenzenesulfonate (**Intermediate 197**, 5.76 g, 20.5 mmol), 3-methyl-1*H*-indazole-6-carboxamide (**Intermediate 144**, 3.00 g, 17.1 mmol) and cesium carbonate (17.6 g, 51.4 mmol) in dimethylformamide (60 mL) was heated at 80°C for 3h. The reaction mixture was poured into ice cold water (100 mL) and extracted with ethyl acetate (2 × 150 mL). The combined organic layers were dried over anhydrous $Na_2SO_4$, filtered, and concentrated *in vacuo* to obtain crude compound as a mixture of regioisomers. The regioisomer were separated by combi-flash chromatography, by eluting with 70% ethyl acetate in heptane, to afford desired regioisomer 3-methyl-1-(oct-7-yn-1-yl)-1*H*-indazole-6-carboxamide (**Intermediate 198**, 2.0 g, 41%) as an off white solid. $^1H$ NMR (400 MHz, DMSO-$d_6$) δ ppm 1.22-1.27 (m, 2H), 1.35-1.45 (m, 4H), 1.78-1.85 (m, 2H), 2.06-2.16 (m, 2H), 2.50 (s, 3H), 2.75 (s, 1H) 4.33 (t, *J*=7.05 Hz, 2H), 7.42 (br s, 1H), 7.59 (d, *J*=8.29 Hz, 1H), 7.73 (d, *J*=8.29 Hz, 1H). 8.03 (br s, 1H), 8.12 (s, 1H). Mass spec m/z 284.1 $[M+H]^+$.

**Step 3**

**Intermediate 199: 1-(8-(2-(2, 6-dioxo-1-((2-(trimethylsilyl) ethoxy) methyl) piperidin-3-yl)-1-oxoisoindolin-4-yl) oct-7-yn-1-yl)-3-methyl-1*H*-indazole-6-carboxamide**

To a solution of 3-methyl-1-(oct-7-yn-1-yl)-1*H*-indazole-6-carboxamide (**Intermediate 198**, 0.57 g, 2.0 mmol) and 3-(4-iodo-1-oxo-isoindolin-2-yl)-1-(2-trimethylsilylethoxymethyl)piperidine-2,6-dione (**Intermediate 25**, 1.0 g, 2.0 mmol) in dimethylformamide (15 mL) was added triethylamine (0.83 g, 7.8 mmol). The reaction mixture was purged with argon gas for 15 min then $Pd(PPh_3)_2Cl_2$ (0.15 g, 0.20 mmol) and copper (I) iodide (0.040 g, 0.20 mmol) were added. The reaction mixture was purged with argon gas for a further 10 min then stirred at room temperature for 1h. The reaction mixture was poured into water (90 mL) and extracted with ethyl acetate (2 × 80 mL). The combined organic layers were dried over anhydrous $Na_2SO_4$, filtered, and concentrated *in vacuo.* The crude material was purified by combi-flash chromatography, by eluting with 95% ethyl acetate in heptane, to afford 1-(8-(2-(2,6-dioxo-1-((2-**(trimethylsilyl)ethoxy) methyl)piperidin-3-yl)-1-oxoisoindolin-4-yl)oct-7-yn-1-yl)-3-**methyl-1*H*-indazole-6-carboxamide (**Intermediate 199**,

1.20 g, 92%) as a brown solid. $^1$H NMR (400 MHz, DMSO-$d_6$) δ ppm -0.05 (s, 9H), 0.79-0.85 (m, 2H), 1.27-1.29 (m, 2H), 1.45-1.56 (m, 4H), 1.79-1.83 (m, 2H), 1.99-2.07 (m, 2H), 2.37 (s, 3H), 2.67-2.77 (m, 2H), 2.87-2.89 (m, 2H), 3.02-3.07 (m, 1H), 3.46-3.52 (m, 2H), 4.23-4.48 (m, 4H), 4.98-5.06 (m, 2H), 5.24-5.27 (m, 1H), 7.50-7.53 (m, 1H), 7.56-7.63 (m, 2H), 7.71 (br s, 2H), 7.95-8.03 (m, 1H), 8.12 (s, 1H). Mass spec m/z 656.51 [M+H]$^+$.

**Step 4**

**Intermediate 200: *tert*-butyl 6-(1-(8-(2-(2,6-dioxo-1-((2-(trimethylsilyl)ethoxy)methyl)piperidin-3-yl)-1-oxoisoindolin-4-yl)oct-7-yn-1-yl)-3-methyl-1*H*-indazole-6-carboxamido)-2-((R)-1-methylpyrrolidin-2-yl)-1*H*-pyrrolo[3,2-c] pyridine-1-carboxylate**

To a solution of 1-(8-(2-(2,6-dioxo-1-((2-(trimethylsilyl)ethoxy)methyl)piperidin-3-yl)-1-oxoisoindolin-4-yl)oct-7-yn-1-yl)-3-methyl-1*H*-indazole-6-carboxamide (**Intermediate 199**, 1.10 g, 1.68 mmol) and *tert*-butyl (R)-6-bromo-2-(1-methylpyrrolidin-2-yl)-1*H*-pyrrolo[3,2-c]pyridine-1-carboxylate (**Intermediate 1**, 0.80 g, 2.10 mmol) in 1,4-dioxane (10 mL) was added cesium carbonate (1.44 g, 4.21 mmol). The reaction mixture was purged with argon gas for 15 min then Xantphos (0.38 g, 0.63 mmol) and $Pd_2(dba)_3$ (0.26 g, 0.31 mmol) were added. The reaction mixture was purged with argon gas for 10 min and then heated at 95°C for 1h. The reaction mixture was filtered through the celite bed, washed with dichloromethane (20 mL) and the filtrate was concentrated *in vacuo.* The crude material was purified by combi-flash chromatography, by eluting with 10% MeOH in DCM, to afford *tert*-butyl 6-(1-(8-(2-(2,6-dioxo-1-((2-(trimethylsilyl)ethoxy) methyl)piperidin-3-yl)-1-oxoisoindolin-4-yl)oct-7-yn-1-yl)-3-methyl-1*H*-indazole-6-carboxamido)-2-((R)-1-methylpyrrolidin-2-yl)-1*H*-pyrrolo[3,2-c]pyridine-1-carboxylate (**Intermediate 200**, 1.0 g, 50%) as a brown solid. $^1$H NMR (400 MHz, DMSO-$d_6$) δ ppm -0.06 (s, 9H), 0.76-0.83 (m, 2H), 1.27-1.38 (m, 2H), 1.47-1.57 (m, 5H), 1.70 (s, 9H), 1.74-1.77 (m, 2H), 1.83-1.91 (m, 3H), 1.99-2.06 (m, 2H), 2.36 (s, 3H), 2.43-2.46 (m, 1H), 2.47 (s, 3H), 2.67-2.75 (m, 3H), 2.87-2.89 (m, 1H), 2.99-3.08 (m, 2H), 3.12-3.16 (m, 1H), 3.46-3.51 (m, 2H), 3.90-3.94 (m, 1H), 4.22-4.26 (m, 1H), 4.39-4.46 (m, 2H), 4.95-5.06 (m, 1H), 5.22-5.26 (m, 1H), 6.76 (s, 1H), 7.48-7.52 (m, 1H), 7.59-7.62 (m, 1H), 7.69-7.74 (m, 2H), 7.75-7.81 (m, 1H), 8.42 (s, 1H), 8.60 (s, 1H), 8.97 (s, 1H), 10.75 (s, 1H). Mass spec m/z 955.1 [M+H]$^+$.

**Step 5**

**Example 45: 1-(8-(2-(2,6-dioxopiperidin-3-yl)-1-oxoisoindolin-4-yl)oct-7-yn-1-yl)-3-methyl-*N*-(2-((R)-1-methylpyrrolidin-2-yl)-1*H*-pyrrolo[3,2-c]pyridin-6-yl)-1*H*-indazole-6-carboxamide**

To a cooled (0°C) solution of *tert*-butyl 6-(1-(8-(2-(2,6-dioxo-1-((2-(trimethylsilyl)ethoxy)methyl)piperidin-3-yl)-1-oxoisoindolin-4-yl)oct-7-yn-1-yl)-3-methyl-1*H*-indazole-6-carboxamido)-2-((R)-1-methylpyrrolidin-2-yl)-1*H*-pyrrolo[3,2-c]pyridine-1-carboxylate (**Intermediate 200**, 0.50 g, 0.52 mmol) in acetonitrile (5 mL) was added methanesulfonic acid (0.53 g, 5.23 mmol) and the reaction mixture was heated at 50°C for 2h. The reaction mixture was cooled to room temperature then *N, N'*-dimethylethylenediamine (0.24 g, 2.62 mmol) followed by triethylamine (1.11 g, 10.47 mmol) were added and the reaction stirred at room temperature for 16h. The reaction mixture was concentrated *in vacuo* and the crude material was poured into water (60 mL). The resultant precipitate was collected by filtration and dried *in vacuo* to afford the crude product which was purified by preparative HPLC (Method A) to afford 1-(8-(2-(2,6-dioxopiperidin-3-yl)-1-oxoisoindolin-4-yl)oct-7-yn-1-yl)-3-methyl-N (2-((R)-1-methylpyrrolidin-2-yl)-1H-pyrrolo[3,2-c]pyridin-6-yl)-1*H*-indazole-6-carboxamide (**Example 45**, 0.10 g, 27%) as an off white solid. $^1$H NMR (400 MHz, DMSO-$d_6$) δ ppm 1.30-1.37 (m, 2H), 1.44-1.60 (m, 4H), 1.77-2.00 (m, 7H), 2.13-2.15 (m, 1H), 2.17 (s, 3H), 2.23-2.30 (m, 1H), 2.40-2.47 (m, 3H), 2.51 (s, 3H), 2.55-2.60 (m, 1H), 2.84-2.93 (m, 1H), 3.11-3.18 (m, 1H), 4.27-4.32 (m, 1H), 4.40-4.46 (m, 3H), 5.10-5.14 (m, 1H), 6.40 (s, 1H), 7.46-7.52 (m, 1H), 7.61 (m, 1H), 7.69 (m, 1H), 7.71-7.75 (m, 1H), 7.76-7.80 (m, 1H), 8.25 (s, 1H), 8.43 (s, 1H), 8.52 (s, 1H), 10.54 (s, 1H), 10.99 (s, 1H), 11.39 (s, 1H). Mass spec m/z 725.1 [M+H]$^+$.

**Example 46 was synthesized following Scheme 46**

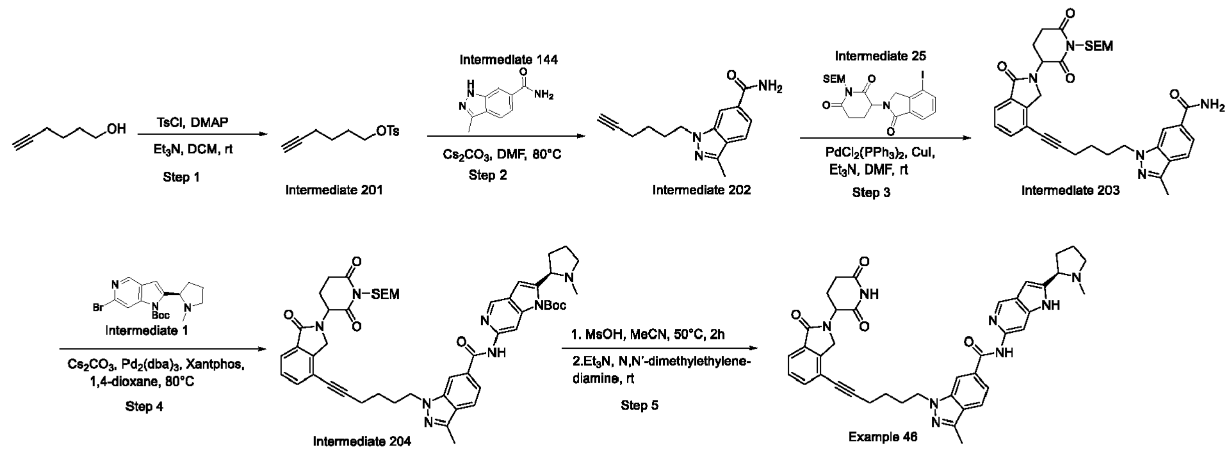


## Scheme 46

**Step** 1

**Intermediate 201: hex-5-yn-1-yl 4-methylbenzenesulfonate**

To solution of hex-5-yn-1-ol (CAS No: 928-90-5, 5.00 g, 51 mmol) in dichloromethane (50 mL) was added triethylamine (21 mL, 150 mmol) at 0°C followed by p-toluenesulfonyl chloride (15 g, 76 mmol) and 4-dimethylaminopyridine (0.66 g, 5.1 mmol). The reaction mixture was stirred at room temperature for 16h. The reaction mixture was diluted with water (25 mL) and extracted with dichloromethane (3 × 25 mL). The organic layer was separated, dried over anhydrous $Na_2SO_4$, filtered, and concentrated *in vacuo.* The crude material was purified by combi-flash chromatography, by eluting with 55% ethyl acetate in heptane, to afford hex-5-yn-1-yl 4-methylbenzenesulfonate (**Intermediate 201**, 7.00 g, 54%) as a yellow oil. $^1$H NMR (400 MHz, DMSO-$d_6$) δ ppm 1.37-1.44 (m, 2H), 1.61-1.68 (m, 2H), 2.08-2.13 (m, 2H), 2.42 (s, 3H), 2.74 (t, *J*=2.4 Hz, 1H), 4.04 (t, *J*=6.40 Hz, 2H), 7.48 (d, *J*=8.40 Hz, 2H), 7.78 (d, *J*=8.40 Hz, 2H).

**Step 2**

**Intermediate 202: 1-(hex-5-yn-1-yl)-3-methyl-1H-indazole-6-carboxamide**

To a solution of hex-5-yn-1-yl 4-methylbenzenesulfonate (**Intermediate 201**, 2.9 g, 11 mmol) in dimethylformamide (2.0 mL) was added 3-methyl-1*H*-indazole-6-carboxamide (**Intermediate 144**, 2.0 g, 11 mmol) followed by cesium carbonate (11 g, 34 mmol) at room temperature. The reaction mixture was heated at 80°C for 6h. The reaction mixture was diluted with water (100 mL) and extracted with ethyl acetate (3 × 100 mL). The organic layer was washed with brine solution (2 × 60 mL), separated, dried over anhydrous $Na_2SO_4$, filtered, and concentrated *in vacuo* to obtain the crude compound as a mixture of regioisomers which were separated by combi-flash chromatography, by eluting with 50% ethyl acetate in heptane, to afford the desired regioisomer 1-(hex-5-yn-1-yl)-3-methyl-1*H*-indazole-6-carboxamide (**Intermediate 202,** 1.50 g, 51%) as a white solid. $^1$H NMR (400 MHz, DMSO-$d_6$) δ ppm 1.42-1.49 (m, 2H), 1.92-1.99 (m, 2H), 2.18-2.22 (m, 2H), 2.63 (s, 3H), 2.77 (t, *J*=2.4 Hz, 1H), 4.39 (t, *J*=6.8 Hz, 2H), 7.28 (br s, 1H), 7.45 (d, *J*=8.80 Hz, 1H), 7.69 (d, J=8.71 Hz, 1H), 7.96 (br s, 1H), 8.12 (s, 1H). Mass spec m/z 256.0 $[M+H]^+$.

**Step 3**

**Intermediate 203: 1-(6-(2-(2,6-dioxo-1-((2-(trimethylsilyl)ethoxy)methyl)piperidin-3-yl)-1-oxoisoindolin-4-yl) hex-5-yn-1-yl)-3-methyl-1H-indazole-6-carboxamide**

To a solution of 1-(hex-5-yn-1-yl)-3-methyl-1*H*-indazole-6-carboxamide (**Intermediate 202,** 1.00 g, 3.9 mmol) in dimethylformamide (10 mL) was added 3-(4-iodo-1-oxoisoindolin-2-yl)-1-((2-(trimethylsilyl)ethoxy)methyl)piperidine-2,6-dione (**Intermediate 25**, 2.00 g, 3.9 mmol) followed by triethylamine (19 mL, 140 mmol) at room temperature. The reaction mixture was purged with argon gas for 15 min then Pd $Cl_2(PPh_3)_2$ (0.28 g, 0.39 mmol) and copper (I) iodide (0.076 g, 0.39 mmol) were added at room temperature. The reaction mixture was purged with argon gas for another 10 min and stirred at room temperature for 2h. The reaction mixture was diluted with water (100 mL) and extracted with ethyl acetate (3 × 200 mL). The organic layer was separated, dried over anhydrous $Na_2SO_4$, filtered, and concentrated *in vacuo.* The crude material was purified by combi-flash chromatography, by eluting with 100% ethyl acetate in heptane, to

afford 1-(6-(2-(2,6-dioxo-1-((2-(trimethylsilyl)ethoxy)methyl)piperidin-3-yl)-1-oxoisoindolin-4-yl)hex-5-yn-1-yl)-3-methyl-1*H*-indazole-6-carboxamide (**Intermediate 203**, 1.50 g, 61%) as a yellow solid. $^1$H NMR (400 MHz, DMSO-$d_6$) δ ppm -0.05 (s, 9H), 0.77-0.86 (m, 2H), 1.51-1.58 (m, 2H), 1.96-2.08 (m, 3H), 2.32-2.39 (m, 1H), 2.48 (s, 3H), 2.72-2.80 (m, 2H), 2.88-2.90 (m, 1H), 3.02-3.12 (m, 1H), 3.46-3.56 (m, 2H), 4.22-4.26 (m, 1H), 4.40-4.46 (m, 3H), 5.00-5.07 (m, 2H), 5.23-5.27 (m, 1H), 7.42 (br s, 1H), 7.49-7.54 (m, 1H), 7.58-7.62 (m, 2H), 7.70-7.74 (m, 2H), 8.03 (br s, 1H), 8.16 (s, 1H). Mass spec m/z 628.3 [M+H]$^+$.

**Step 4**

**Intermediate 204: *tert*-butyl 6-(1-(6-(2-(2,6-dioxo-1-((2-(trimethylsilyl)ethoxy)methyl)piperidin-3-yl)-1-oxoisoindolin-4-yl)hex-5-yn-1-yl)-3-methyl-1*H*-indazole-6-carboxamido)-2-((*R*)-1-methylpyrrolidin-2-yl)-1*H*-pyrrolo[3,2-c]pyridine-1-carboxylate**

To a solution of 1-(6-(2-(2,6-dioxo-1-((2-(trimethylsilyl)ethoxy)methyl)piperidin-3-yl)-1-oxoisoindolin-4-yl)hex-5-yn-1-yl)-3-methyl-1*H*-indazole-6-carboxamide (**Intermediate 203**, 0.90 g, 1.4 mmol) in 1,4-dioxane (15 mL) was added *tert*-butyl (*R*)-6-bromo-2-(1-methylpyrrolidin-2-yl)-1*H*-pyrrolo[3,2-c]pyridine-1-carboxylate (**Intermediate 1**, 0.65 g, 1.7 mmol) followed by cesium carbonate (1.40 g, 4.3 mmol). The reaction mixture was purged with argon gas for 15 min then Xantphos (0.26 g, 0.43 mmol) and $Pd_2$ (dba)$_3$ (0.20 g, 0.22 mmol) were added at room temperature. The reaction mixture was further purged with argon gas for 10 min and heated at 80°C for 2h. The reaction mixture was concentrated *in vacuo* to obtain crude compound. The crude material was purified by combi-flash chromatography, by eluting with 100% ethyl acetate in heptane ,to afford *tert*-butyl 6-(1-(6-(2-(2,6-dioxo-1-((2-(trimethylsilyl)ethoxy)methyl)piperidin-3-yl)-1-oxoisoindolin-4-yl)hex-5-yn-1-yl)-3-methyl- 1*H*-indazole-6-carboxamido)-2-((*R*)-1-methylpyrrolidin-2-yl)-1*H*-pyrrolo[3,2-c]pyridine-1-carboxylate (**Intermediate 204**, 0.50 g, 40%) as a yellow solid. $^1$H NMR (400 MHz, DMSO-$d_6$) δ ppm -0.06 (s, 9H), 0.76-0.83 (m, 2H), 1.19-1.23 (m, 2H), 1.47-1.57 (m, 6H), 1.70 (s, 9H), 2.04 (s, 3H), 2.36 (s, 3H), 2.43-2.47 (m, 4H), 2.66-2.78 (m, 2H), 3.04-3.14 (m, 2H), 3.46-3.51 (m, 2H), 3.84-4.10 (m, 2H), 4.22-4.26 (m, 2H), 4.39-4.46 (m, 2H), 4.95-5.06 (m, 1H), 5.22-5.26 (m, 1H), 6.76 (s, 1H), 7.11-7.54 (m, 2H), 7.56-7.86 (m, 3H), 8.46 (s, 1H), 8.60 (s, 1H), 8.97 (s, 1H), 10.74 (br s, 1H). Mass spec m/z 927.2 [M+H]$^+$.

**Step 5**

**Example 46: 1-(6-(2-(2,6-dioxopiperidin-3-yl)-1-oxoisoindolin-4-yl)hex-5-yn-1-yl)-3-methyl-*N*-(2-((*R*)-1-methylpyrrolidin-2-yl)-1*H*-pyrrolo[3.2-c]pyridin-6-yl)-1*H*-indazole-6-carboxamide**

To a solution of *tert*-butyl 6-(1-(6-(2-(2,6-dioxo-1-((2-(trimethylsilyl)ethoxy)methyl)piperidin-3-yl)-1-oxoisoindolin-4-yl)hex-5-yn-1-yl)-3-methyl-1*H*-indazole-6-carboxamido)-2-((*R*)-1-methylpyrrolidin-2-yl)-1*H*-pyrrolo[3,2-c]pyridine-1-carboxylate (**Intermediate 204**, 0.50 g, 0.5 mmol) in acetonitrile (8 mL) was added methanesulfonic acid (0.5 mL, 8 mmol) at room temperature and the reaction mixture was heated at 50°C for 2h. After cooling to room temperature, *N, N'*-dimethylethylenediamine (0.4 mL, 3 mmol) followed by triethylamine (2 mL, 10 mmol) were added and stirred for 3h at room temperature. The reaction mixture was concentrated *in vacuo* to obtain crude compound which was poured into ice cold water (10 mL), and the resultant solid precipitate collected by filtration and dried *in vacuo* to obtain crude compound. The crude material was purified by preparative HPLC (Method A) to afford 1-(6-(2-(2,6-dioxopiperidin-3-yl)-1-oxoisoindolin-4-yl)hex-5-yn-1-yl)-3-methyl-*N*-(2-((*R*)-1-methylpyrrolidin-2-yl)-1*H*-pyrrolo[3,2-c]pyridin-6-yl)-1*H*-indazole-6-carboxamide (**Example** 46, 0.062 g, 20%) as an off white solid. $^1$H NMR (400 MHz, DMSO-$d_6$) δ ppm 1.55-1.62 (m, 2H), 1.77-1.93 (m, 3H), 1.95-2.08 (m, 4H), 2.10-2.15 (m, 1H), 2.17 (s, 3H), 2.24-2.30 (m, 1H), 2.37-2.42 (m, 1H), 2.48 (s, 3H), 2.53-2.62 (m, 3H), 2.85-2.95 (m, 1H), 3.12-3.18 (m, 1H), 4.26-4.30 (m, 1H), 4.38-4.41 (m, 1H), 4.45-4.50 (m, 2H), 5.09-5.13 (m, 1H), 6.40 (s, 1H), 7.46-7.49 (m, 1H), 7.59-7.61 (m, 1H), 7.67-7.69 (m, 1H), 7.72-7.76 (m, 1H), 7.77-7.81 (m, 1H), 8.24 (s, 1H), 8.47 (s, 1H), 8.52 (s, 1H), 10.53 (s, 1H), 10.98 (br s, 1H), 11.39 (br s, 1H). Mass spec m/z 697.1 [M+H]$^+$.

**Example 47: 1-(5-(4-(2-(2,6-dioxopiperidin-3-yl)-1,3-dioxoisoindolin-4-yl)piperazin-1-yl)pentyl)-3-methyl-*N*-(2-((*R*)-1-methylpyrrolidin-2-yl)-1*H*-pyrrolo[3,2-c]pyridin-6-yl)-1*H*-indazole-6-carboxamide**

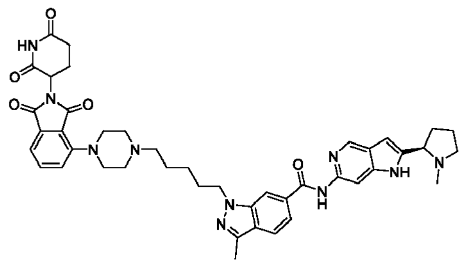

To a solution of (*R*)-3-methyl-N-(2-(1-methylpyrrolidin-2-yl)-1H-pyrrolo[3,2-c]pyridin-6-yl)-1-(5-(piperazin-1-yl) pentyl)-1H-indazole-6-carboxamide dihydrochloride (**Intermediate 111**, 0.40 g, 0.75 mmol) in DMSO (7 mL) was added DIPEA (0.41 g, 3.02 mmol) followed by 2-(2,6-dioxopiperidin-3-yl)-4-fluoroisoindoline-1,3-dione (CAS No: 835616-60-9, 0.20 g, 0.75 mmol) and the reaction mixture was heated at 100°C for 1h. The reaction mixture was poured into ice cold water and the resultant precipitate was collected by filtration and dried *in vacuo.* The crude compound was purified by preparative HPLC (Method A) to afford afford 1-(5-(4-(2-(2,6-dioxopiperidin-3-yl)-1,3-dioxoisoindolin-4-yl)piperazin-1-yl) pentyl)-3-methyl-N-(2-((R)-1-methylpyrrolidin-2-yl)-1H-pyrrolo[3,2-c]pyridin-6-yl)-1H-indazole-6-carboxamide (**Example 47**, 0.10 g, 17%) as an off-white solid. $^1$H NMR (400 MHz, DMSO-$d_6$) δ ppm 1.30-1.40 (m, 2H), 1.50-1.65 (m, 2H), 1.76-1.95 (m, 6H), 1.95-2.05 (m, 1H), 2.16-2.17 (m, 1H), 2.17 (s, 3H), 2.23-2.32 (m, 3H), 2.50-2.52 (m, 2H), 2.53 (s, 3H), 2.54-2.60 (m, 3H), 2.80-2.94 (m, 1H), 3.10-3.19 (m, 2H), 3.20-3.24 (m, 4H), 4.43-4.55 (m, 2 H), 5.07-5.40 (m, 1H), 6.40 (s, 1H), 7.23 (d, J=8.44 Hz, 1H), 7.32 (d, J=7.09 Hz, 1H), 7.63-7.70 (m, 1 H), 7.73 (d, J=8.41 Hz, 1H), 7.79 (d, J=7.87 Hz, 1H), 8.25 (s, 1H), 8.47 (s, 1H), 8.52 (s, 1H), 10.55 (s, 1H), 11.10 (br s, 1H), 11.40 (s, 1H). Mass spec: m/z 785.2 [M+H]$^+$.

**Example 48 was synthesized following Scheme 47**

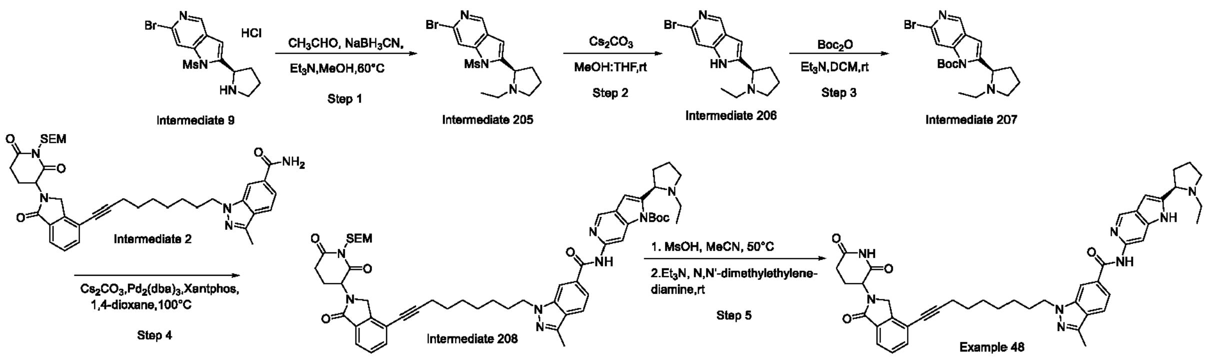


**Scheme 47**

**Step 1**

**Intermediate 205: (R)-6-bromo-2-(1-ethylpyrrolidin-2-yl)-1-(methylsulfonyl)-1*H*-pyrrolo [3, 2-c] pyridine**

To a cooled (0°C) solution of (R)-6-bromo-1-(methylsulfonyl)-2-(pyrrolidin-2-yl)-1*H*-pyrrolo[3,2-c]pyridine hydrochloride (**Intermediate 9**, 10.0 g, 26.27 mmol) in methanol (50 mL) was added triethylamine (29.4 mL, 210.1 mmol) and acetaldehyde (4.21 mL, 78.80 mmol) followed by sodium cyanoborohydride (3.88 g, 52.53 mmol). The reaction mixture was then heated at 60°C for 16h. The reaction mixture was diluted with water (200 mL) and extracted with ethyl acetate (3 × 200 mL). The combined organic layers were dried over anhydrous $Na_2SO_4$, filtered, and concentrated *in vacuo.* The crude material was purified by combi-flash chromatography, by eluting with 60% ethyl acetate in heptane, to afford (R)-6-bromo-2-(1-ethylpyrrolidin-2-yl)-1-(methylsulfonyl)-1*H*-pyrrolo[3,2-c]pyridine (**Intermediate 205**, 5.6 g, 57%) as a yellow solid. $^1$H NMR (400 MHz, DMSO-$d_6$) δ ppm 0.97-1.17 (m, 3H), 1.64-1.82 (m, 3H), 2.20-2.38 (m, 3H), 2.72-2.84 (m, 1H), 3.22-3.31 (m, 1H), 3.55 (s, 3H), 3.95-4.04 (m, 1H), 6.92 (s, 1H), 7.96 (s, 1H), 8.67 (s, 1H). Mass spec m/z 373.1 [M+2H]$^+$.

**Step 2**

**Intermediate 206: (*R*)-6-bromo-2-(1-ethylpyrrolidin-2-yl)-1*H*-pyrrolo [3,** 2-**c]pyridine**

To a cooled (0°C) solution of (*R*)-6-bromo-2-(1-ethylpyrrolidin-2-yl)-1-(methylsulfonyl)-1*H*-pyrrolo[3, 2-c] pyridine (**Intermediate 205**, 5.60 g, 15.04 mmol) in tetrahydrofuran (25 mL) and methanol (25 mL) was added cesium carbonate (9.81 g, 30.08 mmol). The reaction mixture was stirred at room temperature for 4h. The reaction mixture was diluted with water (200 mL) and extracted with ethyl acetate (3 × 200 mL). The combined organic layers were dried over anhydrous $Na_2SO_4$, filtered, and concentrated *in vacuo* to afford crude (R)-6-bromo-2-(1-ethylpyrrolidin-2-yl)-1*H*-pyrrolo [3,2-c]pyridine (**Intermediate 206**, 4.1 g, 93%) as an off-white solid which was used for the next step without further purification. $^1$H NMR (400 MHz, DMSO-$d_6$) δ ppm 0.91-0.97 (m, 3H), 1.69-1.88 (m, 3H), 2.06-2.20 (m, 3H), 2.49-2.55 (m, 1H), 3.17-3.21 (m, 1H), 3.44-3.48 (m, 1H), 6.40 (s, 1H), 7.40 (s, 1H), 8.44 (s, 1H), 11.48 (br s, 1H). Mass spec m/z 295.9 $[M+2H]^+$.

**Step 3**

**Intermediate 207: *tert*-butyl-(*R*)-6-bromo-2-(1-ethylpyrrolidin-2-yl)-1*H*-pyrrolo[3,2-c]pyridine-1-carboxylate**

To a solution of (*R*)-6-bromo-2-(1-ethylpyrrolidin-2-yl)-1*H*-pyrrolo[3,2-c]pyridine (**Intermediate 206**, 4.0 g, 14 mmol) in dichloromethane (40 mL) was added triethylamine (5.7 mL, 41 mmol) followed by di-*tert*-butyl dicarbonate (6.0 g, 27 mmol) and 4-dimethylaminopyridine (0.34 g, 2.7 mmol) and the reaction mixture was stirred at room temperature for 3h. The reaction mixture was diluted with water (500 mL) and extracted with DCM (3 × 500 mL). The combined organic layers were dried over anhydrous $Na_2SO_4$, filtered, and concentrated *in vacuo.* The crude material was purified by combi-flash chromatography, by eluting with 45% ethyl acetate in heptane, to afford *tert*-butyl (R)-6-bromo-2-(1-ethylpyrrolidin-2-yl)-1*H*-pyrrolo[3, 2-c]pyridine-1-carboxylate which was further purified by SFC chiral purification (Column: Chiralpak IK (250 * 30 mm) 5 um. Isocratic elution with 25% Mobile Phase (A): $CO_2$ and mobile Phase (B): MeCN + isopropyl alcohol with 0.1% isopropylamine) to afford *tert*-butyl-(*R*)-6-bromo-2-(1-ethylpyrrolidin-2-yl)-1*H*-pyrrolo[3,2-c]pyridine-1-carboxylate (**Intermediate 207**, 2.2 g, 41%) as an off white solid. $^1$H NMR (400 MHz, DMSO-$d_6$) δ ppm 1.06 (t, *J*=7.13 Hz, 3H), 1.65 (s, 9H), 1.70-1.80 (m, 2H), 2.24-2.38 (m, 4H), 2.72-2.82 (m, 1H), 3.22-3.25 (m, 1H), 4.06-4.10 (m, 1H), 6.83 (s, 1H), 8.05 (s, 1H), 8.61 (s, 1H). Mass spec m/z 395.8 $[M+H]^+$.

**Step 4**

**Intermediate 208: *tert*-butyl 6-(1-(9-(2-(2,6-dioxo-1-((2-(trimethylsilyl)ethoxy)methyl)piperidin-3-yl)-1-oxoisoindolin-4-yl)non-8-yn-1-yl)-3-methyl-1*H*-indazole-6-carboxamido)-2-((*R*)-1-ethylpyrrolidin-2-yl)-1*H*-pyrrolo[3,2-c] pyridine-1-carboxylate**

To a solution of 1-(9-(2-(2,6-dioxo-1-((2-(trimethylsilyl)ethoxy)methyl)piperidin-3-yl)-1-oxoisoindolin-4-yl)non-8-yn-1-yl)-3-methyl-1*H*-indazole-6-carboxamide (**Intermediate 2**, 0.7 g, 1 mmol) and *tert*-butyl (R)-6-bromo-2-(1-ethylpyrrolidin-2-yl)-1*H*-pyrrolo[3,2-c]pyridine-1-carboxylate (**Intermediate 207**, 0.50 g, 1 mmol) in 1,4-dioxane (10 mL) was added cesium carbonate (0.68 g, 4 mmol). The reaction mixture was purged with argon gas for 15 min then Xantphos (0.2 g, 0.4 mmol) and $Pd_2(dba)_3$ (0.2 g, 0.2 mmol) were added. The reaction mixture was purged with argon gas for a further10 min and then heated at 100°C for 2h. The reaction mixture was filtered through celite bed and the filter pad was washed with ethyl acetate (50 mL). The filtrate was concentrated *in vacuo* and the crude material purified by combi-flash chromatography, by eluting with 90% ethyl acetate in heptane, to afford *tert*-butyl 6-(1-(9-(2-(2,6-dioxo-1-((2-(trimethylsilyl)ethoxy) methyl)piperidin-3-yl)-1-oxoisoindolin-4-yl)non-8-yn-1-yl)-3-methyl-1*H*-indazole-6-carboxamido)-2-((R)-1-ethylpyrrolidin-2-yl)-1*H*-pyrrolo[3,2-c]pyridine-1-carboxylate (**Intermediate 208**, 0.42 g, 30%) as a yellow solid. $^1$H NMR (400 MHz, DMSO-$d_6$) δ ppm -0.06 (s, 9H), 0.74-0.88 (m, 2H), 1.08 (t, *J*=7.02 Hz, 3H), 1.25-1.44 (m, 6H), 1.52-1.66 (m, 5H), 1.69 (s, 9H), 1.73-1.75 (m, 2H), 1.85-1.92 (m, 2H), 2.02-2.08 (m, 1H), 2.20-2.36 (m, 2H), 2.38 (s, 3H), 2.41-2.45 (m, 2H), 2.74-2.84 (m, 2H), 3.00-3.09 (m, 1H), 3.23-3.26 (m, 1H), 3.45-3.55 (m, 2H), 4.09-4.13 (m, 1H), 4.21-4.26 (m, 1H), 4.38-4.45 (m, 3H), 4.99-5.07 (m, 2H), 5.24-5.28 (m, 1H), 6.79 (s, 1H), 7.47-7.51 (m, 1H), 7.59-7.62 (m, 1H), 7.69-7.74 (m, 2H), 7.77-7.81 (m, 1H), 8.42 (s, 1H), 8.60 (s, 1H), 8.97 (s, 1H), 10.73 (s, 1H). Mass spec m/z 983.2 $[M+H]^+$.

**Step 5**

**Example 48: 1-(9-(2-(2,6-dioxopiperidin-3-yl)-1-oxoisoindolin-4-yl)non-8-yn-1-yl)-*N*-(2-((R)-1-ethylpyrrolidin-2-yl)-1*H*-pyrrolo[3,2-c]pyridin-6-yl)-3-methyl-1*H*-indazole-6-carboxamide**

To a solution of *tert*-butyl 6-(1-(9-(2-(2,6-dioxo-1-((2-(trimethylsilyl)ethoxy)methyl)piperidin-3-yl)-1-oxoisoindolin-4-yl)non-8-yn-1-yl)-3-methyl-1*H*-indazole-6-carboxamido)-2-((R)-1-ethylpyrrolidin-2-yl)-1*H*-pyrrolo[3,2-c]pyridine-1-carboxylate (**Intermediate 208**, 0.42 g, 0.42 mmol) in acetonitrile (8 mL) was added methanesulfonic acid (0.42 mL, 6.41 mmol) and the reaction mixture was heated at 50°C for 2h. The reaction mixture was cooled to room temperature then *N,N'*-dimethylethylenediamine (0.31 mL, 2.56 mmol) followed by triethylamine (1.20 mL, 8.54 mmol) were added and stirred at room temperature for 3h. The reaction mixture was concentrated *in vacuo* and then water (20 mL) added. The resultant precipitate was collected by filtration and dried *in vacuo* to afford crude compound. The crude material was purified by preparative HPLC (Method A) to afford 1-(9-(2-(2,6-dioxopiperidin-3-yl)-1-oxoisoindolin-4-yl)non-8-yn-1-yl)-*N*-(2-((R)-1-ethylpyrrolidin-2-yl)-1*H*-pyrrolo[3,2-c]pyridin-6-yl)-3-methyl-1*H*-indazole-6-carboxamide (**Example 48**, 0.08 g, 27%) as an off white solid. $^1$H NMR (400 MHz, DMSO-$d_6$) δ ppm 0.98 (t, *J*=7.25 Hz, 3H), 1.26-1.45 (m, 6H), 1.51-1.58 (m, 2H), 1.79-1.92 (m, 5H), 1.97-2.04 (m, 1H), 2.11-2.24 (m, 3H), 2.40 (s, 3H), 2.42-2.48 (m, 2H), 2.56-2.68 (m, 3H), 2.85-2.94 (m, 1H), 3.22-3.27 (m, 1H), 3.46-3.50 (m, 1H), 4.27-4.31 (m, 1H), 4.38-4.44 (m, 3H), 5.10-5.15 (m, 1H), 6.39 (s, 1H), 7.47-7.52 (m, 1H), 7.58-7.60 (m, 1H), 7.68-7.70 (m, 1H), 7.72-7.75 (m, 1H), 7.76-7.80 (m, 1H), 8.25 (s, 1H), 8.43 (s, 1H), 8.51 (s, 1H), 10.51 (s, 1H), 11.00 (s, 1H), 11.34 (s, 1H). Mass spec m/z 753.2 $[M+H]^+$.

**Example 49 was synthesized following Scheme 48**

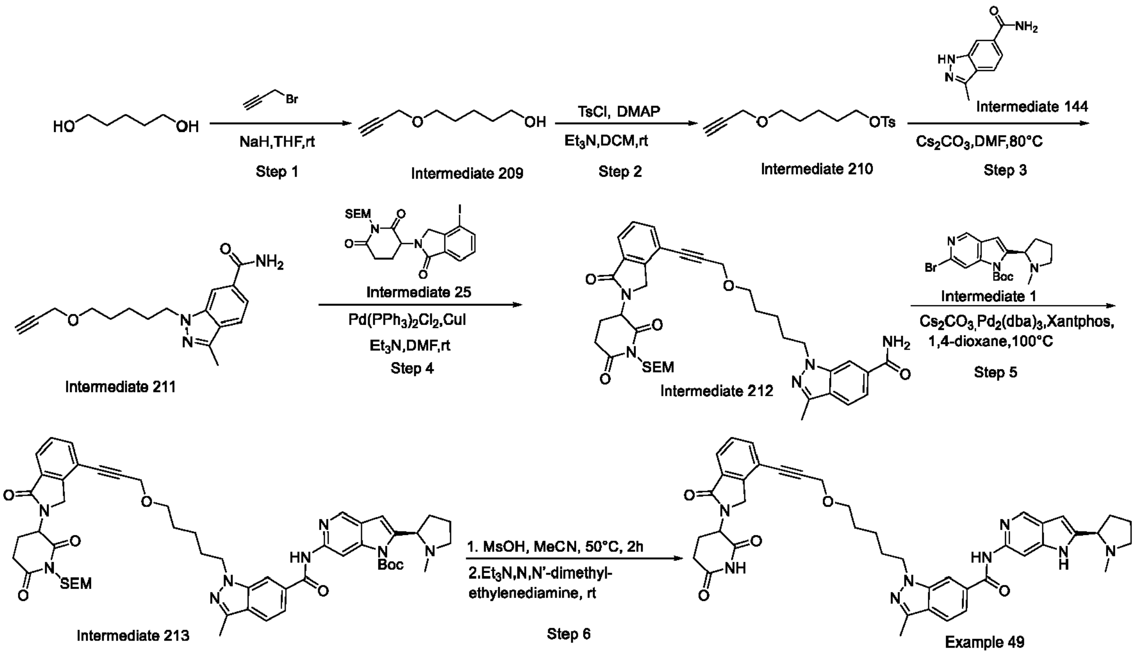


**Scheme 48**

**Step 1**

**Intermediate 209: 5-(prop-2-yn-1-yloxy) pentan-1-ol**

To a cooled (0°C) solution of sodium hydride (0.80 g, 33.62 mmol) in tetrahydrofuran (40 mL) was added pentane-1, 5-diol (CAS No: 111-29-5, 13.65 g, 131.13 mmol) and the reaction was stirred for 30 min at 0°C prior to the addition of 3-bromoprop-1-yne (CAS No: 106-96-7, 4.00 g, 33.6 mmol). The reaction mixture was stirred at room temperature for 16h. The reaction mixture was poured into ice cold water (150 mL) and extracted with DCM (2 × 200 mL). The combined organic layers were dried over anhydrous $Na_2SO_4$, filtered, and concentrated *in vacuo.* The crude material was purified by combi-flash chromatography, by eluting with 70% ethyl acetate in heptane, to afford 5-(prop-2-yn-1-yloxy) pentan-1-ol (**Intermediate 209**, 3.5 g, 74%) as a yellow liquid. $^1$H NMR (400 MHz, $CDCl_3$) δ ppm 1.38-1.46 (m, 3H), 1.54-1.66 (m, 4H), 2.39 (s, 1H), 3.51 (t, *J*=6.43 Hz, 2H), 3.63 (t, *J*=6.43 Hz, 2H), 4.11 (s, 2H).

**Step 2**

**Intermediate 210: 5-(prop-2-yn-1-yloxy) pentyl 4-methylbenzenesulfonate**

To a cooled (0°C) solution of 5-(prop-2-yn-1-yloxy)pentan-1-ol (**Intermediate 209**, 1.60 g, 11.3 mmol) in dichloromethane (80 mL) was added triethylamine (2.38 mL, 16.9 mmol) followed by 4-dimethylaminopyridine (0.14 g, 1.13 mmol) and the reaction was stirred at 0°C for 15 min prior to the addition of *p*-toluenesulfonyl chloride (2.63 g, 13.5 mmol). The reaction mixture was stirred at room temperature for 16h. The reaction mixture was diluted with 1N HCl (100 mL) and extracted with dichloromethane (2 × 100 mL). The combined organic layers were washed with $NaHCO_3$ (2 × 100 mL) and brine solution (100 mL), dried over anhydrous $Na_2SO_4$, and concentrated *in vacuo* to afford 5-(prop-2-yn-1-yloxy) pentyl 4-methylbenzenesulfonate (**Intermediate 210**, 3.25 g, 97%) as a yellow oil which was used for the next step without further purification. $^1H$ NMR (400 MHz, DMSO-$d_6$) δ ppm 1.21-1.29 (m, 2H), 1.38-1.45 (m, 2H), 1.53-1.60 (m, 2H), 2.42 (s, 3H), 3.33-3.39 (m, 2H), 3.39 (s, 1H), 4.01 (t, *J*=6.36 Hz, 2H), 4.07 (s, 2H), 7.48 (d, *J*=8.40 Hz, 2H), 7.78 (d, *J*=8.11 Hz, 2H).

**Step 3**

**Intermediate 211: 3-methyl-1-(5-(prop-2-yn-1-yloxy) pentyl)-1*H*-indazole-6-carboxamide**

To solution of 3-methyl-1*H*-indazole-6-carboxamide (**Intermediate 144**, 1.6 g, 9.1 mmol) in dimethylformamide (30 mL) was added cesium carbonate (8.9 g, 27 mmol) and 5-(prop-2-yn-1-yloxy)pentyl-4-methylbenzenesulfonate (**Intermediate 210**, 3.2 g, 11 mmol) and the reaction mixture was heated at 80°C for 4h. The reaction mixture was diluted with water (100 mL) and extracted with ethyl acetate (3 × 60 mL). The combined organic layers were washed with brine solution (2 × 50 mL), dried over anhydrous $Na_2SO_4$, and concentrated *in vacuo* to obtain crude compound as a mixture of regioisomers. The regioisomers were separated by combi-flash chromatography, by eluting with 60% ethyl acetate in heptane, to afford desired regioisomer 3-methyl-1-(5-(prop-2-yn-1-yloxy)pentyl)-1*H*-indazole-6-carboxamide (**Intermediate 211**, 1.40 g, 51%) as a white solid. $^1H$ NMR (400 MHz, DMSO-$d_6$) δ ppm 1.24-1.32 (m, 2H), 1.49-1.56 (m, 2H), 1.79-1.86 (m, 2H), 2.49 (s, 3H), 3.36-3.39 (m, 2H), 3.40 (s, 1H), 4.06 (s, 2H), 4.33 (t, *J*=7.02 Hz, 2H), 7.41 (br s, 1H), 7.59 (d, *J*=8.33 Hz, 1H), 7.73 (d, *J*=8.33 Hz, 1H), 8.02 (br s, 1H), 8.12 (s, 1H). Mass spec m/z 299.9 $[M+H]^+$.

**Step 4**

**Intermediate 212: 1-(5-((3-(2-(2,6-dioxo-1-((2-(trimethylsilyl)ethoxy)methyl)piperidin-3-yl)-1-oxoisoindolin-4-yl) prop-2-yn-1-yl) oxy) pentyl)-3-methyl-1*H*-indazole-6-carboxamide**

To solution of 3-methyl-1-(5-(prop-2-yn-l-yloxy)pentyl)-1*H*-indazole-6-carboxamide (**Intermediate 211**, 1.10 g, 3.67 mmol) and 3-(4-iodo-1-oxoisoindolin-2-yl)-1-((2-(trimethylsilyl)ethoxy)methyl)piperidine-2,6-dione (**Intermediate 25**, 2.02 g, 4.04 mmol) in dimethylformamide (8 mL) was added triethylamine (18 mL, 129 mmol). The reaction mixture was purged with argon gas for 10 min then $PdCl_2(PPh_3)_2$ (0.26 g, 0.36 mmol) and copper (I) iodide (0.07 g, 0.36 mmol) were added at room temperature. The reaction mixture was purged with argon gas for 10 min and stirred at room temperature for 2h. The reaction mixture was diluted with water (60 mL) and extracted with ethyl acetate (3 × 50 mL). The combined organic layers were washed with brine solution (2 × 50 mL), dried over anhydrous $Na_2SO_4$, and concentrated *in vacuo*. The crude material was purified by combi-flash chromatography, by eluting with 2% MeOH in DCM, to afford 1-(5-((3-(2-(2,6-dioxo-1-((2-(trimethylsilyl) ethoxy)methyl)piperidin-3-yl)-1-oxoisoindolin-4-yl)prop-2-yn-1-yl)oxy)pentyl)-3-methyl-1*H*-indazole-6-carboxamide (**Intermediate 212**, 1.30 g, 53%) as a white solid. $^1H$ NMR (400 MHz, DMSO-$d_6$) δ ppm -0.04 (s, 9H), 0.78-0.86 (m, 2H), 1.28-1.35 (m, 3H), 1.54-1.61 (m, 2H), 1.81-1.88 (m, 2H), 2.01-2.08 (m, 1H), 2.39-2.44 (m, 1H), 2.67-2.80 (m, 3H), 3.01-3.11 (m, 1H), 3.48-3.54 (m, 4H), 4.27-4.40 (m, 5H), 4.47-4.51 (m, 1H), 5.00-5.08 (m, 2H), 5.24-5.29 (m, 1H), 7.40 (br s, 1H), 7.54-7.60 (m, 2H), 7.69-7.73 (m, 2H), 7.77 (d, *J*=7.89 Hz, 1H), 8.01 (br s, 1H), 8.12 (s, 1H). Mass spec m/z 672.0 $[M+H]^+$.

**Step 5**

**Intermediate 213: *tert*-butyl 6-(1-(5-((3-(2-(2,6-dioxo-1-((2-(trimethylsilyl)ethoxy)methyl)piperidin-3-yl)-1-oxoisoindolin-4-yl)prop-2-yn-1-yl)oxy)pentyl)-3-methyl-1H indazole-6-carboxamido)-2-((R)-1-methylpyrrolidin-2-yl)-1*H*-pyrrolo[3,2-c]pyridine-1-carboxylate**

To a solution of 1-(5-((3-(2-(2, 6-dioxo-1-((2-(trimethylsilyl) ethoxy)methyl) piperidin-3-yl)-1-oxoisoindolin-4-yl) prop-2-yn-1-yl) oxy) pentyl)-3-methyl-1*H*-indazole-6-carboxamide (**Intermediate 212**, 0.60 g, 0.89 mmol) in 1,4-dioxane (10 mL) was added *tert*-butyl (R)-6-bromo-2-(1-methylpyrrolidin-2-yl)-1*H*-pyrrolo[3,2-c]pyridine-1-carboxylate (**Intermediate 1**, 0.40 g, 1.07 mmol) followed by cesium carbonate (0.87 g, 2.67 mmol). The reaction mixture was purged with argon gas for 15 min then $Pd_2(dba)_3$ (0.12 g, 0.14 mmol) and Xantphos (0.16 g, 0.26 mmol) were added and the

reaction mixture was purged with argon gas for 10 min. The reaction mixture was heated at 100°C for 2h then diluted with water (40 mL) and extracted with ethyl acetate (3 × 50 mL). The combined organic layers were washed with brine solution (2 × 50 mL), dried over anhydrous $Na_2SO_4$, and concentrated *in vacuo.* The crude material was purified by combi-flash chromatography, by eluting with 2% MeOH in DCM, to afford *tert*-butyl 6-(1-(5-((3-(2-(2,6-dioxo-1-((2-(trimethylsilyl) ethoxy)methyl)piperidin-3-yl)-1-oxoisoindolin-4-yl)prop-2-yn-1-yl)oxy)pentyl)-3-methyl-1*H*-indazole-6-carboxamido)-2-((*R*)-1-methylpyrrolidin-2-yl)-1*H*-pyrrolo[3,2-c]pyridine-1-carboxylate (**Intermediate 213**, 0.40 g, 46%) as a yellow solid. $^1$H NMR (400 MHz, DMSO-$d_6$) δ ppm -0.05 (s, 9H), 0.76-0.85 (m, 2H), 1.32-1.39 (m, 2H), 1.56-1.65 (m, 3H), 1.70 (s, 9H), 1.73-1.76 (m, 3H), 1.86-1.92 (m, 2H), 2.01-2.08 (m, 2H), 2.31-2.33 (m, 1H), 2.36 (s, 3H), 2.42 (s, 3H), 2.74-2.78 (m, 1H), 3.00-3.14 (m, 2H), 3.47-3.55 (m, 4H), 3.91-3.94 (m, 1H), 4.26-4.30 (m, 1H), 4.38-4.51 (m, 5H), 4.98-5.06 (m, 2H), 5.24-5.28 (m, 1H), 6.76 (s, 1H), 7.52-7.55 (m, 1H), 7.67-7.79 (m, 4H), 8.43 (s, 1H), 8.60 (s, 1H), 8.97 (s, 1H), 10.74 (s, 1H). Mass spec m/z 971.3 $[M+H]^+$.

**Step 6**

**Example 49: 1-(5-((3-(2-(2,6-dioxopiperidin-3-yl)-1-oxoisoindolin-4-yl)prop-2-yn-1-yl)oxy)pentyl)-3-methyl-*N*-(2-((R)-1-methylpyrrolidin-2-yl)-1*H*-pyrrolo[3,2-c]pyridin-6-yl)-1*H*-indazole-6-carboxamide**

To a solution of *tert*-butyl 6-(1-(5-((3-(2-(2,6-dioxo-1-((2-(trimethylsilyl)ethoxy)methyl)piperidin-3-yl)-1-oxoisoindolin-4-yl)prop-2-yn-1-yl)oxy)pentyl)-3-methyl-1*H*-indazole-6-carboxamido)-2-((R)-1-methylpyrrolidin-2-yl)-1*H*-pyrrolo [3,2-c]pyridine-1-carboxylate (**Intermediate 213**, 0.38 g, 0.39 mmol) in acetonitrile (6 mL) was added methanesulfonic acid (0.38 mL, 5.86 mmol) and the reaction mixture was heated to 50°C for 2h. The reaction mixture was cooled to room temperature, then *N*, *N*'-dimethylethylenediamine (0.28 mL, 2.34 mmol) followed by triethylamine (0.82 mL, 5.87 mmol) were added and the mixture stirred at room temperature for 2h. The reaction mixture was concentrated *in vacuo* and the resultant solid was washed with ether (2 × 50 mL) and DCM/Pentanes (2 × 40 mL) and dried *in vacuo.* The crude material was purified by preparative HPLC (Method A) to afford 1-(5-((3-(2-(2,6dioxopiperidin-3-yl)-1-oxoisoindolin-4-yl)prop-2-yn-1-yl)oxy)pentyl)-3-methyl-*N*-(2-((R)-1-methylpyrrolidin-2-yl)-1*H*-pyrrolo[3,2-c]pyridin-6-yl)-1*H*-indazole-6-carboxamide (**Example 49**, 0.067 g, 23%) as an off white solid. $^1$H NMR (400 MHz, DMSO-$d_6$) δ ppm 1.32-1.40 (m, 2H), 1.57-1.64 (m, 2H), 1.79-2.01 (m, 6H), 2.13-2.15 (m, 1H), 2.17 (s, 3H), 2.24-2.30 (m, 1H), 2.47 (s, 3H), 2.58-2.60 (m, 1H), 2.85-2.94 (m, 1H), 3.12-3.17 (m, 1H), 3.27-3.29 (m, 1H), 3.33-3.35 (m, 1H), 3.50-3.53 (m, 2H), 4.30-4.49 (m, 6H), 5.11-5.15 (m, 1H), 6.40 (s, 1H), 7.51-7.55 (m, 1H), 7.67-7.79 (m, 4H), 8.25 (s, 1H), 8.44 (s, 1H), 8.51 (s, 1H), 10.52 (s, 1H), 11.00 (br s, 1H), 11.39 (s, 1H). Mass spec: m/z: 741.3 $[M+H]^+$.

**Example 50 was synthesized following Scheme 49**

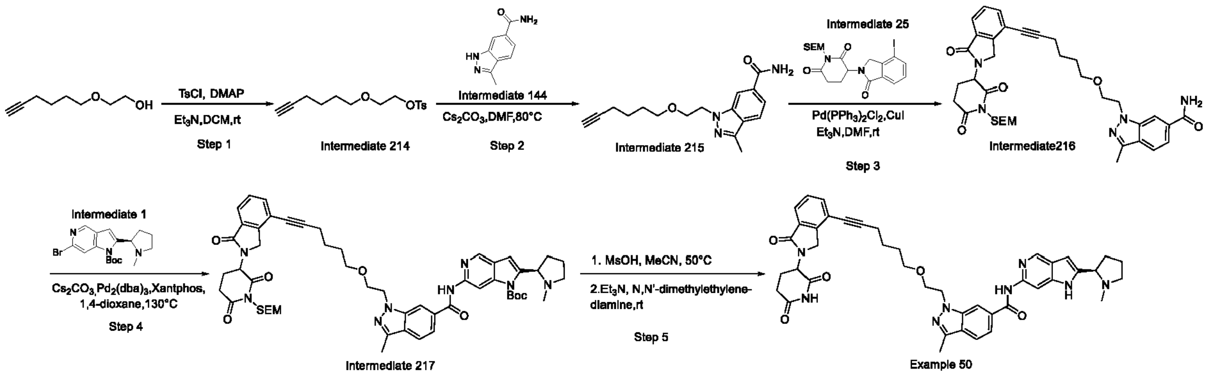


**Scheme 49**

**Step 1**

**Intermediate 214: 2-(hex-5-yn-1-yloxy) ethyl 4-methylbenzenesulfonate**

To a cooled (0°C) solution of 2-(hex-5-yn-1-yloxy)ethan-1-ol (CAS No: 252948-15-5, 3.00 g, 21.09 mmol) in dichloromethane (30 mL) was added sequentially triethylamine (4.43 mL, 31.64 mmol), 4-dimethylaminopyridine (0.52 g, 4.22 mmol) and *p*-toluenesulfonyl chloride (6.35 g, 31.64 mmol). The reaction mixture was stirred at room temperature for

16h then diluted with water (150 mL) and extracted with dichloromethane (3 × 150 mL). The combined organic layers were separated, dried over anhydrous $Na_2SO_4$ and concentrated *in vacuo.* The crude material was purified by combi-flash chromatography, by eluting with 40% ethyl acetate in heptane, to afford 2-(hex-5-yn-1-yloxy) ethyl 4-methylbenzenesulfonate (**Intermediate 214**, 3.4 g, 54%) as a colourless oil. $^1$H NMR (400 MHz, DMSO-$d_6$) δ ppm 1.36-1.43 (m, 2H), 1.45-1.52 (m, 2H), 2.11-2.16 (m, 2H), 2.42 (s, 3H), 2.73-2.75 (m, 1H), 3.29-3.34 (m, 2H), 3.49-3.54 (m, 2H), 4.10-4.14 (m, 2H), 7.48 (d, *J*=7.88 Hz, 2H), 7.78 (d, *J*=7.88 Hz, 2H).

**Step 2**

**Intermediate 215: 1-(2-(hex-5-yn-1-yloxy) ethyl)-3-methyl-1*H*-indazole-6-carboxamide**

To solution of 2-(hex-5-yn-1-yloxy) ethyl 4-methylbenzenesulfonate (**Intermediate 214, 2.03** g, 6.84 mmol) in dimethylformamide (10 mL) was added 3-methyl-1*H*-indazole-6-carboxamide (**Intermediate 144**, 1 g, 5.71 mmol) and cesium carbonate (5.57 g, 17.12 mmol). The reaction mixture was heated at 80°C for 16h. The reaction mixture was diluted with water (50 mL) and extracted with ethyl acetate (3 × 100 mL). The combined organic layers were separated, dried over anhydrous $Na_2SO_4$, concentrated *in vacuo* to obtain crude compound as a mixture of regioisomers. The regioisomers were separated by combi-flash chromatography, by eluting with 80% ethyl acetate in heptane, to afford desired regioisomer 1-(2-(hex-5-yn-1-yloxy)ethyl)-3-methyl-1*H*-indazole-6-carboxamide (**Intermediate 215**, 0.8 g, 47%) as a white solid. $^1$H NMR (400 MHz, DMSO-$d_6$) δ ppm 1.28-1.34 (m, 2H), 1.41-1.46 (m, 2H), 2.00-2.04 (m, 2H), 2.49 (s, 3H), 2.68 (s, 1H), 3.31-3.35 (m, 2H), 3.77 (t, *J*=5.48 Hz, 2H), 4.49 (t, *J*=5.48 Hz, 2H), 7.38 (br s, 1H), 7.59 (d, *J*=8.44 Hz, 1H), 7.72 (d, *J*=8.33 Hz, 1H), 8.00 (br s, 1H), 8.15 (s, 1H). Mass spec m/z 300.19 $[M+H]^+$.

**Step 3**

**Intermediate 216: 1-(2-((6-(2-(2, 6-dioxo-1-((2-(trimethylsilyl) ethoxy) methyl) piperidin-3-yl)-1-oxoisoindolin-4-yl) hex-5-yn-1-yl) oxy) ethyl)-3-methyl-1*H* indazole-6-carboxamide**

To solution of 1-(2-(hex-5-yn-1-yloxy)ethyl)-3-methyl-1*H*-indazole-6-carboxamide (**Intermediate 215**, 0.80 g, 2.67 mmol) and 3-(4-iodo-1-oxoisoindolin-2-yl)-1-((2-(trimethylsilyl)ethoxy)methyl)piperidine-2,6-dione (**Intermediate 25**, 1.34 g, 2.67 mmol) in dimethylformamide (10 mL) was added triethylamine (13.8 mL, 98.86 mmol). The reaction mixture was purged with argon gas for 15 min then $PdCl_2(PPh_3)_2$ (0.19 g, 0.26 mmol) and copper (I) iodide (0.050 g, 0.26 mmol) were added. The reaction mixture was purged with argon gas for 5 min and stirred at room temperature for 3h. The reaction mixture was poured into water (60 mL) and extracted with ethyl acetate (3 × 100 mL). The combined organic layers were separated, dried over anhydrous $Na_2SO_4$, and concentrated *in vacuo.* The crude material was purified by combi-flash chromatography, by eluting with 90% ethyl acetate in heptane, to afford 1-(2-((6-(2-(2,6-dioxo-1-((2-(trimethylsilyl)ethoxy)methyl)piperidin-3-yl)-1-oxoisoindolin-4-yl)hex-5-yn-1-yl)oxy)ethyl)-3-methyl-1*H*-indazole-6-carboxamide (**Intermediate 216**, 0.80 g, 45%) as an off white solid. Mass spec m/z 672.1 $[M+H]^+$.

**Step 4**

**Intermediate 217: *tert*-butyl 6-(1-(2-((6-(2-(2,6-dioxo-1-((2-(trimethylsilyl)ethoxy)methyl)piperidin-3-yl)-1-oxoisoindolin-4-yl)hex-5-yn-1-yl)oxy)ethyl)-3-methyl-1*H*-indazole-6-carboxamido)-2-((R)-1-methylpyrrolidin-2-yl)-1*H*-pyrrolo[3,2-c]pyridine-1-carboxylate**

To a solution of 1-(2-((6-(2-(2,6-dioxo-1-((2-(trimethylsilyl)ethoxy)methyl)piperidin-3-yl)-1-oxoisoindolin-4-yl) hex-5-yn-1-yl)oxy)ethyl)-3-methyl-1*H*-indazole-6-carboxamide (**Intermediate 216**, 0.71 g, 1.05 mmol) in 1,4-dioxane (20 mL) was added *tert*-butyl (R)-6-bromo-2-(1-methylpyrrolidin-2-yl)-1*H*-pyrrolo[3,2-c]pyridine-1-carboxylate (**Intermediate 1**, 0.50 g, 1.31 mmol) followed by cesium carbonate (1.08 g, 3.17 mmol). The reaction mixture was purged with argon gas for 15 min then $Pd_2(dba)_3$ (0.18 g, 0.16 mmol) and Xantphos (0.19 g, 0.32 mmol) were added and the reaction mixture was purged with argon gas for 5 min. The reaction mixture was heated at 130°C for 2h. The reaction mixture was concentrated *in vacuo* and the crude material was purified by combi-flash chromatography, by eluting with 90% ethyl acetate in heptane, to afford *tert*-butyl 6-(1-(2-((6-(2-(2,6-dioxo-1-((2-(trimethylsilyl)ethoxy)methyl)piperidin-3-yl)-1-oxoisoindolin-4-yl)hex-5-yn-1-yl)oxy)ethyl)-3-methyl-1*H*-indazole-6-carboxamido)-2-((R)-1-methylpyrrolidin-2-yl)-1*H*-pyrrolo[3,2-c]pyridine-1-carboxylate (**Intermediate 217**, 0.70 g, 70%) as an off white solid. $^1$H NMR (400 MHz, DMSO-$d_6$) δ ppm -0.06 (s, 9H), 0.77-0.86 (m, 2H), 1.43-1.55 (m, 5H), 1.61-1.64 (m, 2H), 1.69 (s, 9H), 1.74-1.76 (m, 2H), 1.98-2.06 (m, 2H), 2.31 (s, 3H), 2.36 (s, 3H), 2.36-2.41 (m, 2H), 2.74-2.79 (m, 1H), 3.00-3.13 (m, 2H), 3.39-3.42 (m, 2H), 3.48-3.52 (m, 2H), 3.80-3.83 (m, 2H), 3.90-3.96 (m, 1H), 4.18-4.23 (m, 1H), 4.36-4.40 (m, 1H), 4.58 (s, 2H), 4.98-5.07 (m, 2H), 5.22-5.27 (m, 1H), 6.75 (s, 1H), 7.44-7.48 (m, 1H), 7.54 (d, *J*=7.60 Hz, 1H), 7.67 (d, *J*=7.20 Hz, 1H), 7.71-7.77 (m, 2H),

8.47 (s, 1H), 8.59 (s, 1H), 8.96 (s, 1H), 10.68 (s, 1H). Mass spec m/z 971.3 [M+H]+.

**Step 5**

**Example 50: 1-(2-((6-(2-(2,6-dioxopiperidin-3-yl)-1-oxoisoindolin-4-yl)hex-5-yn-1-yl)oxy)ethyl)-3-methyl-*N*-(2-((*R*)-1-methylpyrrolidin-2-yl)-1*H*-pyrrolo[3,2-c]pyridin-6-yl)-1*H*-indazole-6-carboxamide**

To a solution of *tert*-butyl 6-(1-(2-((6-(2-(2,6-dioxo-1-((2-(trimethylsilyl)ethoxy)methyl)piperidin-3-yl)-1-oxoisoindolin-4-yl)hex-5-yn-1-yl)oxy)ethyl)-3-methyl-1*H*-indazole-6-carboxamido)-2-((R)-1-methylpyrrolidin-2-yl)-1*H*-pyrrolo[3,2-c]pyridine-1-carboxylate (**Intermediate 217**, 0.70 g, 0.72 mmol) in acetonitrile (15 mL) was added methanesulfonic acid (1.09 g, 10.81 mmol) and the reaction mixture was heated to 50°C for 2h. The reaction mixture was cooled to room temperature, then *N*,*N'*-dimethylethylenediamine (0.45 g, 4.32 mmol) followed by triethylamine (1.15 g, 10.81 mmol) were added and the mixture stirred at room temperature for 3h. The reaction mixture was concentrated *in vacuo* and poured into water (20 mL). The resultant precipitate was collected by filtration and dried *in vacuo.* The crude material was purified by preparative HPLC (Method A) to afford 1-(2-((6-(2-(2,6-dioxopiperidin-3-yl)-1-oxoisoindolin-4-yl)hex-5-yn-1-yl)oxy)ethyl)-3-methyl-N-(2-((R)-1-methylpyrrolidin-2-yl)-1*H*-pyrrolo[3,2-c]pyridin-6-yl)-1*H*-indazole-6-carboxamide (**Example 50**, 0.15 g, 28%) as an off white solid. $^1$H NMR (400 MHz, DMSO-$d_6$) δ ppm 1.41-1.50 (m, 2H), 1.50-1.59 (m, 2H), 1.76-2.02 (m, 4H), 2.13-2.15 (m, 1H), 2.17 (s, 3H), 2.23-2.34 (m, 3H), 2.47 (s, 3H), 2.55-2.61 (m, 2H), 2.84-2.95 (m, 1H), 3.12-3.18 (m, 1H), 3.22-3.29 (m, 1H), 3.41 (t, *J*=6.07 Hz, 2H), 3.83-3.85 (m, 2H), 4.23-4.30 (m, 1H), 4.36-4.42 (m, 1H), 4.58-4.60 (m, 2H), 5.09-5.14 (m, 1H), 6.40 (s, 1H), 7.45-7.49 (m, 1H), 7.54-7.58 (m, 1H), 7.67 (d, *J*=7.25 Hz, 1H), 7.71-7.77 (m, 2H), 8.24 (s, 1H), 8.47 (s, 1H), 8.51 (s, 1H), 10.47 (s, 1H), 11.00 (s, 1H), 11.37 (s, 1H). Mass spec m/z 741.2 [M+H]+.

**Example 51 was synthesised following Scheme 50**

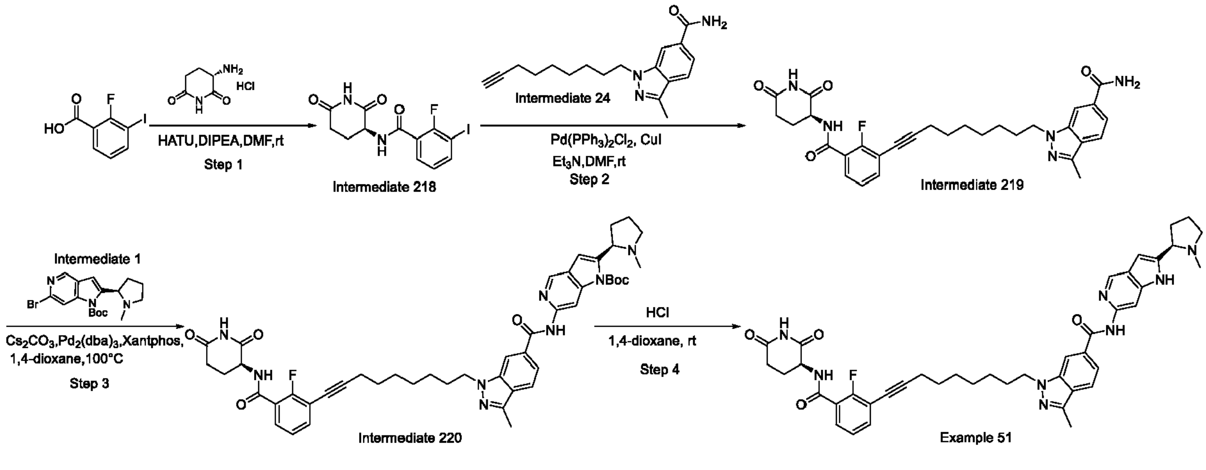


**Scheme 50**

**Step 1**

**Intermediate 218: (S)-*N*-(2, 6-dioxopiperidin-3-yl)-2-fluoro-3-iodobenzamide**

To a solution of 2-fluoro-3-iodobenzoic acid (CAS No: 447464-03-1, 3.55 g, 13.36 mmol) in dimethylformamide (50 mL) was added HATU (7.29 g, 18.22 mmol) and DIPEA (3.20 g, 24.30 mmol) and the mixture was stirred for 10 min prior to the addition of (S)-3-aminopiperidine-2,6-dione hydrochloride (CAS No: 25181-50-4, 2.00 g, 12.15 mmol). The reaction mixture was stirred for 16h then quenched by the addition of ice cold water (100 mL). The resultant precipitate was collected by filtration and dried *in vacuo* to afford (S)-N-(2, 6-dioxopiperidin-3-yl)-2-fluoro-3-iodobenzamide **(Intermediate 218,** 3.7 g, 81%) as a white solid which was used for the next step without further purification. $^1$H NMR (400 MHz, DMSO-$d_6$) δ ppm 2.01-2.14 (m, 2H), 2.52-2.58 (m, 1H), 2.74-2.85 (m, 1H), 4.73-4.80 (m, 1H), 7.11 (t, *J*=7.67 Hz, 1H), 7.61 (t, *J*=7.05 Hz, 1H), 7.98 (t, *J*=6.84 Hz, 1H), 8.72 (d, *J*=7.88 Hz, 1H), 10.86 (s, 1H). Mass spec m/z 376.9 [M+H]+.

**Step 2**

**Intermediate 219: (S)-1-(9-(3-((2, 6-dioxopiperidin-3-yl) carbamoyl)-2-fluorophenyl) non-8-yn-1-yl)-3-methyl-1*H*-indazole-6-carboxamide**

To a solution of (S)-*N*-(2,6-dioxopiperidin-3-yl)-2-fluoro-3-iodobenzamide (**Intermediate 218,** 1.00 g, 2.65 mmol) and 3-methyl-1-non-8-ynyl-indazole-6-carboxamide (**Intermediate 24**, 0.79 g, 2.65 mmol) in dimethylformamide (30 mL) was added triethylamine (10.19 g, 95.71 mmol). The reaction mixture was purged with argon gas for 15 min then $PdCl_2(PPh_3)_2$ (0.19 g, 0.26 mmol) and copper (I) iodide (0.05 g, 0.26 mmol) were added. The reaction mixture was purged with argon gas for 5 min and stirred at room temperature for 2h. The reaction mixture was diluted with water (100 mL) and extracted with ethyl acetate (3 × 150 mL). The combined organic layers were dried over anhydrous $Na_2SO_4$, filtered and concentrated *in vacuo.* The crude material was purified by combi-flash chromatography, by eluting with 90% ethyl acetate in heptane, to afford (S)-1-(9-(3-((2, 6-dioxopiperidin-3-yl)carbamoyl)-2-fluorophenyl)non-8-yn-1-yl)-3-methyl-1*H*-indazole-6-carboxamide (**Intermediate 219,** 0.95 g, 65%) as a yellow solid. $^1H$ NMR (400 MHz, DMSO-$d_6$) δ ppm 1.25-1.44 (m, 8H), 1.47-1.56 (m, 2H), 1.78-1.87 (m, 2H), 1.99-2.12 (m, 2H), 2.22-2.25 (m, 1H), 2.53 (s, 3H), 2.73-2.83 (m, 1H), 4.31-4.37 (m, 2H), 4.73-4.79 (m, 1H), 7.25 (t, *J*=7.67 Hz, 1H), 7.41 (br s, 1H), 7.55-7.62 (m, 3H), 7.72 (d, *J*=8.33 Hz, 1H), 8.02 (br s, 1H), 8.12 (s, 1H), 8.68 (d, *J*=8.11 Hz, 1H), 10.86 (s, 1H). Mass spec m/z 546.1 $[M+H]^+$.

**Step 3**

**Intermediate 220: *tert*-butyl 6-(1-(9-(3-(((S)-2,6-dioxopiperidin-3-yl)carbamoyl)-2-fluorophenyl)non-8-yn-1-yl)-3-methyl-1H indazole-6-carboxamido)-2-((R)-1-methylpyrrolidin-2-yl)-1*H*-pyrrolo[3,2-c]pyridine-1-carboxylate**

To a solution of (S)-1-(9-(3-((2, 6-dioxopiperidin-3-yl) carbamoyl)-2-fluorophenyl) non-8-yn-1-yl)-3-methyl-1*H*-indazole-6-carboxamide (**Intermediate 219,** 0.23 g, 0.42 mmol) and *tert*-butyl 6-bromo-2-[(2R)-1-methylpyrrolidin-2-yl] pyrrolo[3,2-c]pyridine-1-carboxylate **(Intermediate 1,** 0.20 g, 0.52 mmol) in 1,4-dioxane (6 mL) was added cesium carbonate (0.43 g, 1.31 mmol). The reaction mixture was purged with argon gas for 15 min then Xantphos (0.094 g, 0.15 mmol) and $Pd_2(dba)_3$ (0.074 g, 0.078 mmol) were added. The reaction mixture was purged with argon gas for 10 min and heated at 100°C for 2h. The reaction mixture was then filtered through celite bed and the filter cake was washed with ethyl acetate (50 mL). The filtrate was concentrated *in vacuo* and the crude residue was purified by combi-flash chromatography, by eluting with 90% ethyl acetate in heptane, to afford *tert*-butyl 6-(1-(9-(3-(((S)-2,6-dioxopiperidin-3-yl)carbamoyl)-2-fluorophenyl)non-8-yn-1-yl)-3-methyl-1*H*-indazole-6-carboxamido)-2-((R)-1-methylpyrrolidin-2-yl)-1*H*-pyrrolo [3,2-c]pyridine-1-carboxylate **(Intermediate 220, 0.15** g, 34%) as a yellow solid. Mass spec m/z 845.54 $[M+H]^+$.

**Step 4**

**Example 51: 1-(9-(3-(((S)-2,6-dioxopiperidin-3-yl)carbamoyl)-2-fluorophenyl)non-8-yn-1-yl)-3-methyl-*N*-(2-((R)-1-methylpyrrolidin-2-yl)-1*H*-pyrrolo[3,2-c]pyridin-6-yl)-1*H*-indazole-6-carboxamide**

To a cooled (0°C) solution of *tert*-butyl 6-(1-(9-(3-(((S)-2,6-dioxopiperidin-3-yl)carbamoyl)-2-fluorophenyl)non-8-yn-1-yl)-3-methyl-1*H*-indazole-6-carboxamido)-2-((R)-1-methylpyrrolidin-2-yl)-1*H*-pyrrolo[3,2-c]pyridine-1-carboxylate **(Intermediate 220, 0.45** g, 0.53 mmol) in 1,4-dioxane (5 mL) was added 4M HCl in 1,4-dioxane (5 mL). The reaction mixture was stirred at room temperature for 3h then was concentrated *in vacuo.* The crude material was purified by preparative HPLC (Method A) to afford 1-(9-(3-(((S)-2,6-dioxopiperidin-3-yl)carbamoyl)-2-fluorophenyl)non-8-yn-1-yl)-3-methyl-*N*-(2-((R)-1-methylpyrrolidin-2-yl)-1*H*-pyrrolo[3,2-c]pyridin-6-yl)-1*H*-indazole-6-carboxamide (**Example 51**, 0.040 g, 10%) as an off white solid. $^1H$ NMR (400 MHz, DMSO-$d_6$) δ ppm 1.30-1.39 (m, 6H), 1.49-1.55 (m, 2H), 1.83-1.92 (m, 5H), 1.96-2.11 (m, 3H), 2.18 (s, 3H), 2.25-2.31 (m, 2H), 2.43-2.46 (m, 3H), 2.54 (s, 3H), 2.73-2.78 (m, 1H), 3.13-3.18 (m, 1H), 4.41 (t, *J*=7.00 Hz, 2H), 4.73-4.77 (m, 1H), 6.41 (s, 1H), 7.24 (t, *J*=7.69 Hz, 1H), 7.53-7.60 (m, 2H), 7.71-7.75 (m, 1H), 7.76-7.80 (m, 1H), 8.25 (s, 1H), 8.44 (s, 1H), 8.53 (br s, 1H), 8.67-8.69 (m, 1H), 10.53 (br s, 1H), 10.86 (s, 1H), 11.39 (br s, 1H). Mass spec m/z 745.3 $[M+H]^+$.

**Example 52 was synthesized following Scheme 51**

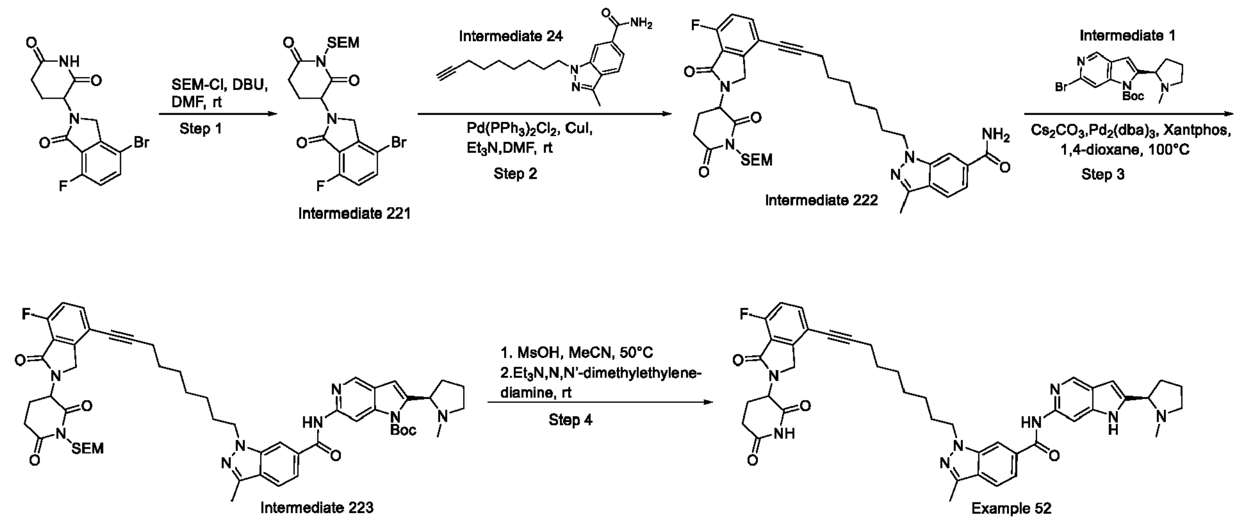


**Scheme 51**

**Step 1**

**Intermediate 221: 3-(4-bromo-7-fluoro-1-oxoisoindolin-2-yl)-1-((2-(trimethylsilyl) ethoxy) methyl) piperidine-2, 6-dione**

To a cooled (0°C) solution of 3-(4-bromo-7-fluoro-1-oxoisoindolin-2-yl)piperidine-2,6-dione (CAS No: 2438239-34-8, 1.00 g, 2.93 mmol) in dimethylformamide (10 mL) was added DBU (3.5 mL, 23 mmol) followed by 2-(trimethylsilyl)ethoxymethyl chloride (3.7 mL, 20 mmol) and the reaction mixture was stirred at room temperature for 16h. The reaction mixture was quenched with ice-cold water (50 mL) and extracted with ethyl acetate (2 × 50 mL). The combined organic layers were dried over anhydrous $Na_2SO_4$, filtered and concentrated *in vacuo.* The crude material was purified by combi-flash column chromatography, by eluting with 20% ethyl acetate in heptane, to afford 3-(4-bromo-7-fluoro-1-oxoisoindolin-2-yl)-1-((2-(trimethylsilyl) ethoxy) methyl) piperidine-2, 6-dione (**Intermediate 221,** 1.1 g, 80%) as a white solid. $^1H$ NMR (400 MHz, DMSO-$d_6$) δ ppm -0.02 (s, 9H), 0.78-0.88 (m, 2H), 2.05-2.08 (m, 1H), 2.39-2.43 (m, 1H), 2.76-2.82 (m, 1H), 3.02-3.10 (m, 1H), 3.47-3.58 (m, 2H), 4.20-4.25 (m, 1H), 4.42-4.46 (m, 1H), 5.02-5.09 (m, 2H), 5.22-5.26 (m, 1H), 7.35 (t, J=8.40 Hz, 1H), 7.89-7.92 (m, 1H). Mass spec m/z 471.0 $[M+H]^+$.

**Step 2**

**Intermediate 222: 1-(9-(2-(2,6-dioxo-1-((2-(trimethylsilyl) ethoxy) methyl) piperidin-3-yl)-7-fluoro-1-oxoisoindolin-4-yl) non-8-yn-1-yl)-3-methyl-1*H*-indazole-6-carboxamide**

To a solution of 3-(4-bromo-7-fluoro-1-oxoisoindolin-2-yl)-1-((2-(trimethylsilyl)ethoxy)methyl) piperidine-2,6-dione (**Intermediate 221**, 1.6 g, 3.4 mmol) and 3-methyl-1-(non-8-yn-1-yl)-1*H*-indazole-6-carboxamide (**Intermediate 24**, 1.00 g, 3.36 mmol) in dimethylformamide (15 mL) was added triethylamine (1.03 g, 10.1 mmol). The reaction mixture was purged with argon for 15 min then $PdCl_2(PPh_3)_2$ (0.48 g, 0.67 mmol) and copper (I) iodide (0.064 g, 0.34 mmol) were added. The reaction mixture was purged with argon for another 5 min and stirred at room temperature for 2h. The reaction mixture was diluted with water (200 mL) and extracted with ethyl acetate (3 × 100 mL). The combined organic layers were separated, dried over anhydrous $Na_2SO_4$, filtered and concentrated *in vacuo.* The crude material was purified by combi-flash chromatography, by eluting with 80% ethyl acetate in heptane, to afford 1-(9-(2-(2, 6-dioxo-1-((2-(trimethylsilyl) ethoxy) methyl) piperidin-3-yl)-7-fluoro-1-oxoisoindolin-4-yl) non-8-yn-1-yl)-3-methyl-1*H*-indazole-6-carboxamide (**Intermediate 222**, 0.90 g, 39%) as a yellow solid. $^1H$ NMR (400 MHz, DMSO-$d_6$) δ ppm -0.04 (s, 9H), 0.78-0.87 (m, 2H), 1.25-1.40 (m, 6H), 1.48-1.56 (m, 2H), 1.78-1.86 (m, 2H), 2.02-2.08 (m, 1H), 2.40-2.45 (m, 2H), 2.48 (s, 3H), 2.73-2.80 (m, 2H), 3.01-3.10 (m, 1H), 3.49-3.56 (m, 2H), 4.23-4.36 (m, 3H), 4.43-4.48 (m, 1H), 5.00-5.08 (m, 2H), 5.21-5.25 (m, 1H), 7.30 (t, *J*=8.80 Hz, 1H), 7.41 (br s, 1H), 7.58 (d, *J*=8.40 Hz, 1H), 7.63-7.67 (m, 1H), 7.72 (d, *J*=8.40 Hz, 1H), 8.02 (br s, 1H), 8.11 (s, 1H). Mass spec m/z 687.9 $[M+H]^+$.

**Step 3**

**Intermediate 223: *tert*-butyl 6-(1-(9-(2-(2,6-dioxo-1-((2-(trimethylsilyl)ethoxy)methyl)piperidin-3-yl)-7-fluoro-1-oxoisoindolin-4-yl)non-8-yn-1-yl)-3-methyl-1*H*-indazole-6-carboxamido)-2-((*R*)-1-methylpyrrolidin-2-yl)-1*H*-pyrrolo[3,2-c]pyridine-1-carboxylate**

To a solution of 1-(9-(2-(2,6-dioxo-1-((2-(trimethylsilyl)ethoxy)methyl)piperidin-3-yl)-7-fluoro-1-oxoisoindolin-4-yl) non-8-yn-1-yl)-3-methyl-1*H*-indazole-6-carboxamide (**Intermediate 222,** 0.57 g, 0.84 mmol) in 1, 4-dioxane (2 mL) was added *tert*-butyl (*R*)-6-bromo-2-(1-methylpyrrolidin-2-yl)-1*H*-pyrrolo[3,2-c]pyridine-1-carboxylate (**Intermediate 1,** 0.40 g, 1.05 mmol) followed by cesium carbonate (0.61 g, 3.15 mmol). The reaction mixture was purged with argon for 15 min. To the reaction mixture was added $Pd_2(dba)_3$ (0.15 g, 0.16 mmol) followed by Xantphos (0.18 g, 0.31 mmol) and the mixture was purged with argon gas for another 10 min then heated at 100°C for 2h under argon atmosphere. The reaction mixture was filtered through celite bed and the filter pad was washed with ethyl acetate (50 mL). The filtrate was concentrated *in vacuo* and the crude material purified by combi-flash column chromatography, by eluting with 90% ethyl acetate in heptane, to afford *tert*-butyl 6-(1-(9-(2-(2,6-dioxo-1-((2-(trimethylsilyl)ethoxy)methyl)piperidin-3-yl)-7-fluoro-1-oxoisoindolin-4-yl)non-8-yn-1-yl)-3-methyl-1*H*-indazole-6-carboxamido)-2-((*R*)-1-methylpyrrolidin-2-yl)-1*H*-pyrrolo[3,2-c]pyridine-1-carboxylate (**Intermediate 223**, 0.35 g, 34%) as a yellow solid. $^1$H NMR (400 MHz, DMSO-$d_6$) $\delta$ ppm -0.05 (s, 9H), 0.79-0.90 (m, 2H), 1.07-1.15 (m, 2H), 1.23-1.44 (m, 6H), 1.49-1.54 (m, 2H), 1.61-1.65 (m, 3H), 1.70 (s, 9H), 1.72-1.76 (m, 3H), 1.82-1.86 (m, 2H), 1.98-2.08 (m, 1H), 2.33 (s, 3H), 2.36-2.41 (m, 1H), 2.43 (s, 3H), 3.04-3.18 (m, 2H), 3.35-3.38 (m, 1H), 3.48-3.57 (m, 1H), 3.90-3.93 (m, 1H), 4.22-4.46 (m, 4H), 4.92-5.12 (m, 3H), 6.76 (s, 1H), 6.97-7.05 (m, 1H), 7.26-7.33 (m, 1H), 7.58-7.61 (m, 1H), 7.71-7.79 (m, 1H), 8.42 (s, 1H), 8.60 (s, 1H), 8.97 (s, 1H), 10.73 (br s, 1H). Mass spec m/z 987.0 $[M+H]^+$.

**Step 4**

**Example 52: 1-(9-(2-(2,6-dioxopiperidin-3-yl)-7-fluoro-1-oxoisoindolin-4-yl)non-8-yn-1-yl)-3-methyl-*N*-(2-((*R*)-1-methylpyrrolidin-2-yl)-1*H*-pyrrolo[3,2-c]pyridin-6-yl)-1*H*-indazole-6-carboxamide**

To a solution of *tert*-butyl 6-(1-(9-(2-(2,6-dioxo-1-((2-(trimethylsilyl)ethoxy)methyl)piperidin-3-yl)-7-fluoro-1-oxoisoindolin-4-yl)non-8-yn-1-yl)-3-methyl-1*H*-indazole-6-carboxamido)-2-((*R*)-1-methylpyrrolidin-2-yl)-1*H*-pyrrolo[3,2-c] pyridine-1-carboxylate (**Intermediate 223**, 0.30 g, 0.30 mmol) in acetonitrile (10 mL) was added methanesulfonic acid (0.20 mL, 3.04 mmol) and the reaction mixture was heated at 50°C for 2h. After cooling to room temperature, *N, N'*-dimethylethylenediamine (0.18 mL, 1.52 mmol) was added followed by triethylamine (0.85 mL, 6.07 mmol) and the reaction mixture was stirred at room temperature for 3h. The reaction mixture was quenched with water (20 mL) and the resultant precipitate was collected by filtration and dried *in vacuo.* The crude material was purified preparative HPLC (Method A) to afford 1-(9-(2-(2,6-dioxopiperidin-3-yl)-7-fluoro-1-oxoisoindolin-4-yl)non-8-yn-1-yl)-3-methyl-*N*-(2-((*R*)-1-methylpyrrolidin-2-yl)-1*H*-pyrrolo[3,2-c]pyridin-6-yl)-1H-indazole-6-carboxamide (**Example 52**, 0.045 g, 19%) as an off-white solid. $^1$H NMR (400 MHz, DMSO-$d_6$) $\delta$ ppm 1.29-1.42 (m, 6H), 1.50-1.57 (m, 2H), 1.84-2.00 (m, 6H), 2.12-2.16 (m, 1H), 2.17 (s, 3H), 2.24-2.30 (m, 1H), 2.39-2.41 (m, 1H), 2.43 (s, 3H), 2.45-2.47 (m, 1H), 2.54-2.60 (m, 1H), 2.84-2.94 (m, 1H), 3.13-3.18 (m, 1H), 3.32-3.36 (m, 2H), 4.27-4.32 (m, 1H), 4.38-4.45 (m, 3H), 5.07-5.11 (m, 1H), 6.40 (s, 1H), 7.27 (t, *J*=8.94 Hz, 1H), 7.60-7.64 (m, 1H), 7.71-7.75 (m, 1H), 7.76-7.80 (m, 1H), 8.25 (s, 1H), 8.43 (s, 1H), 8.52 (s, 1H), 10.51 (s, 1H), 11.01 (s, 1H), 11.38 (s, 1H). Mass spec m/z 757.3 $[M+H]^+$.

**Example 53: *N*-(3-chloro-2-((R)-1-methylpyrrolidin-2-yl)-1*H*-pyrrolo[3,2-c]pyridin-6-yl)-1-(9-(2-(2,6-dioxopiperidin-3-yl)-1-oxoisoindolin-4-yl)non-8-yn-1-yl)-3-methyl-1*H*-indazole-6-carboxamide**

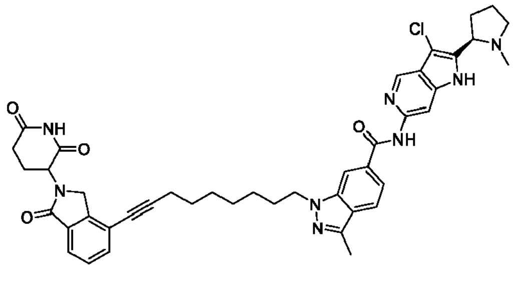

To a solution of 1-(9-(2-(2,6-dioxopiperidin-3-yl)-1-oxoisoindolin-4-yl)non-8-yn-1-yl)-3-methyl-*N*-(2-((R)-1-methylpyrrolidin-2-yl)-1*H*-pyrrolo[3,2-c]pyridin-6-yl)-1*H*-indazole-6-carboxamide (**Example 1,** 0.10 g, 0.13 mmol) in

dimethylformamide (3 mL) was added N-chlorosuccinimide (0.027 g, 0.20 mmol) and the reaction mixture was stirred for 30h. The reaction mixture was poured into ice cold water (50 mL), the resultant precipitate was collected by filtration and dried *in vacuo.* The crude material was purified by preparative HPLC (Method A) to afford *N*-(3-chloro-2-((R)-1-methylpyrrolidin-2-yl)-1*H*-pyrrolo[3,2-c]pyridin-6-yl)-1-(9-(2-(2,6-dioxopiperidin-3-yl)-1-oxoisoindolin-4-yl)non-8-yn-1-yl)-3-methyl-1*H*-indazole-6-carboxamide (**Example 53**, 0.016 g, 15%) as an off white solid. $^1$H NMR (400 MHz, DMSO-$d_6$) δ ppm 1.32-1.38 (m, 6H), 1.52-1.58 (m, 2H), 1.82-1.91 (m, 4H), 1.98-2.07 (m, 2H), 2.17 (s, 3H), 2.30-2.36 (m, 2H), 2.42-2.45 (m, 2H), 2.47 (s, 3H), 2.56-2.59 (m, 1H), 2.85-2.94 (m, 1H), 3.16-3.20 (m, 2H), 3.51-3.55 (m, 1H), 4.27-4.31 (m, 1H), 4.39-4.45 (m, 3H), 5.10-5.15 (m, 1H), 7.47-7.51 (m, 1H), 7.58-7.60 (m, 1H), 7.68-7.74 (m, 2H), 7.76-7.80 (m, 1H), 8.31 (s, 1H), 8.44 (s, 1H), 8.52 (s, 1H), 10.67 (s, 1H), 10.99 (s, 1H), 11.70 (br s, 1H). Mass spec m/z 773.2 [M+H]$^+$.

**Example 54 was synthesized following Scheme 52**

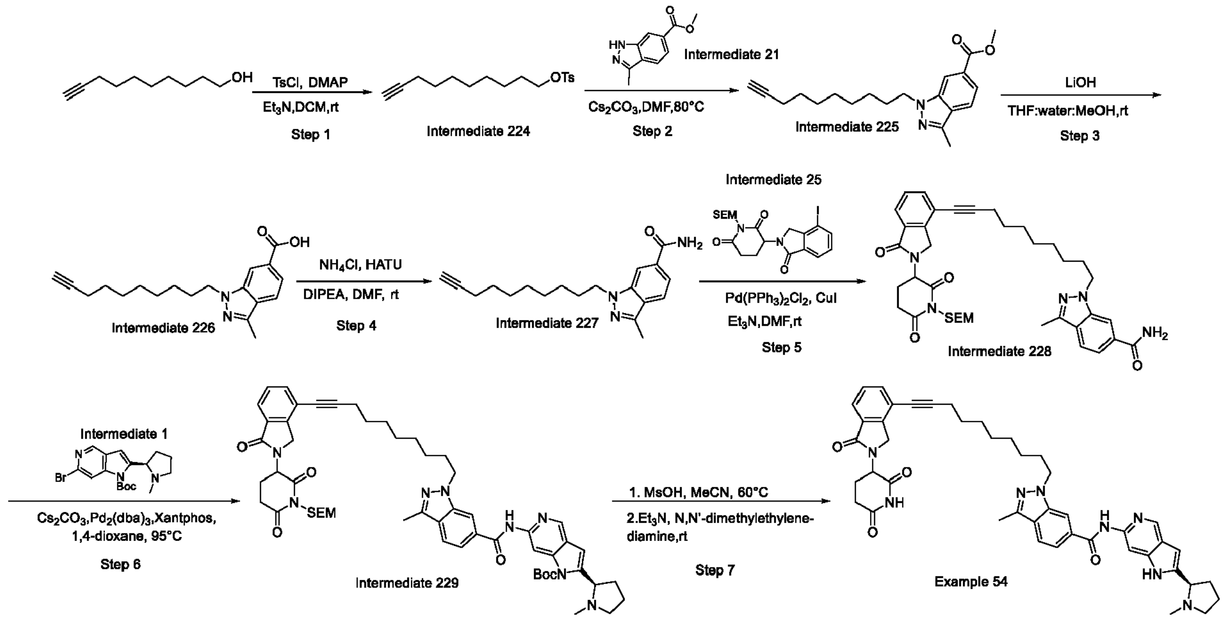


Scheme 52

**Step 1**

**Intermediate 224: dec-9-yn-1-yl 4-methylbenzenesulfonate**

To a cooled (0°C) solution of dec-9-yn-1-ol (CAS No: 13019-22-2, 4.0 g, 26 mmol) in dichloromethane (50 mL) was added *p*-toluenesulfonyl chloride (5.8 mL, 39 mmol) and triethylamine (8.3 g, 78 mmol) followed by 4-dimethylaminopyridine (0.33 g, 2.6 mmol). The reaction mixture was stirred at room temperature for 16h then poured into ice cold water (150 mL) and extracted with dichloromethane (2 × 100 mL). The combined organic layers were separated, dried over anhydrous $Na_2SO_4$, and concentrated *in vacuo.* The crude material was purified by combi-flash chromatography, by eluting with 20% ethyl acetate in heptane, to afford dec-9-yn-1-yl-4-methylbenzenesulfonate (**Intermediate 224,** 4.5 g, 56%) as a yellow liquid. $^1$H NMR (400 MHz, DMSO-$d_6$) δ ppm 1.11-1.30 (m, 8H), 1.36-1.44 (m, 2H), 1.49-1.58 (m, 2H), 2.09-2.16 (m, 2H), 2.42 (s, 3H), 2.72 (s, 1H), 4.00 (t, *J*=6.22 Hz, 2H), 7.48 (d, *J*=7.88 Hz, 2H), 7.78 (d, *J*=8.29 Hz, 2H).

**Step 2**

**Intermediate 225: methyl 1-(dec-9-yn-1-yl)-3-methyl-1*H*-indazole-6-carboxylate**

To a solution of dec-9-yn-1-yl-4-methylbenzenesulfonate (**Intermediate 224**, 2.9 g, 9.5 mmol) in dimethylformamide (15 mL) was added cesium carbonate (8.1 g, 24 mmol) and methyl 3-methyl-1*H*-indazole-6-carboxylate (**Intermediate 21**, 1.5 g, 7.9 mmol). The reaction mixture was heated at 80°C for 16h then poured into ice cold water (120 mL) and extracted with ethyl acetate (2 × 120 mL). The combined organic layers were separated, dried over $Na_2SO_4$,

and concentrated *in vacuo* to obtain a crude mixture of regioisomers. The regioisomers were separated by combi-flash chromatography, by eluting with 30% ethyl acetate in heptane, to afford desired regioisomer methyl-1-(dec-9-yn-1-yl)-3-methyl-1*H*-indazole-6-carboxylate (**Intermediate 225**, 1.60 g, 62%) as an off white solid. $^1$H NMR (400 MHz, DMSO-$d_6$) δ ppm 1.16-1.32 (m, 10H), 1.36-1.43 (m, 2H), 1.75-1.84 (m, 2H), 2.08-2.13 (m, 2H), 2.39-2.42 (m, 1H), 2.70 (s, 1H), 3.90 (s, 3H), 4.40 (t, *J*=6.91 Hz, 2H), 7.65 (d, *J*=8.33 Hz, 1H), 7.81 (d, *J*=8.33 Hz, 1H), 8.22 (s, 1H). Mass spec m/z 327.28 $[M+H]^+$.

**Step 3**

**Intermediate 226: 1-(dec-9-yn-1-yl)-3-methyl-1*H*-indazole-6-carboxylic acid**

To a solution of methyl-1-(dec-9-yn-1-yl)-3-methyl-1*H*-indazole-6-carboxylate (**Intermediate 225**, 1.9 g, 5.8 mmol) in tetrahydrofuran (6 mL), methanol (6 mL) and water (6 mL) was added lithium hydroxide (0.43 g, 17 mmol) and the reaction mixture was stirred for 16h. The reaction mixture was concentrated *in vacuo* and the crude residue was adjusted to pH~ 3 by the addition of 1N aqueous HCl. The resultant precipitate was collected by filtration, washed with heptane (30 mL) and dried *in vacuo* to afford 1-(dec-9-yn-1-yl)-3-methyl-1*H*-indazole-6-carboxylic acid (**Intermediate 226**, 1.75 g, 96%) as an off white solid. $^1$H NMR (400 MHz, DMSO-$d_6$) δ ppm 1.16-1.31 (m, 8H), 1.34-1.41 (m, 2H), 1.75-1.85 (m, 2H), 2.08-2.12 (m, 2H), 2.52 (s, 3H), 2.62 (t, *J*=2.63 Hz, 1H), 4.37 (t, *J*=6.82 Hz, 2H), 7.67 (d, *J*=8.44, 1H), 7.79 (d, *J*=8.38 Hz, 1H), 8.14 (s, 1H). Mass spec m/z 313.23 $[M+H]^+$.

**Step 4**

**Intermediate 227: 1-(dec-9-yn-1-yl)-3-methyl-1*H*-indazole-6-carboxamide**

To a cooled (0°C) solution of 1-(dec-9-yn-1-yl)-3-methyl-1*H*-indazole-6-carboxylic acid (**Intermediate 226**, 1.80 g, 5.76 mmol) in dimethylformamide (20 mL) was added HATU (5.65 g, 14.4 mmol) and DIPEA (5.03 mL, 28.8 mmol) and the mixture was stirred for 10 min. Ammonium chloride (1.01 mL, 28.8 mmol) was then added and the reaction mixture was stirred at room temperature for 6h. The reaction mixture was poured into ice cold water (200 mL), the resultant precipitate was collected by filtration, washed with heptane (50 mL) and dried *in vacuo* to afford 1-(dec-9-yn-1-yl)-3-methyl-1*H*-indazole-6-carboxamide (**Intermediate 227**, 1.70 g, 95%) as an off white solid which was used in the next step without further purification. $^1$H NMR (400 MHz, DMSO-$d_6$) δ ppm 1.09-1.27 (m, 7H), 1.29-1.38 (m, 2H), 1.72-1.80 (m, 2H), 2.02-2.08 (m, 2H), 2.46 (s, 3H), 2.56-2.58 (m, 1H), 4.30 (t, *J*=6.58 Hz, 2H), 7.55 (d, *J*=8.33 Hz, 1H), 7.73 (d, *J*=8.33 Hz, 1H), 8.05 (s, 1H). Mass spec m/z 312.27 $[M+H]^+$.

**Step 5**

**Intermediate 228: 1-(10-(2-(2, 6-dioxo-1-((2-(trimethylsilyl) ethoxy) methyl) piperidin-3-yl)-1-oxoisoindolin-4-yl) dec-9-yn-1-yl)-3-methyl-1*H*-indazole-6-carboxamide**

To a solution of 1-(dec-9-yn-1-yl)-3-methyl-1*H*-indazole-6-carboxamide (**Intermediate 227**, 1.0, 3.2 mmol) in dimethylformamide (1 mL) was added 3-(4-iodo-1-oxoisoindolin-2-yl)-1-((2-(trimethylsilyl)ethoxy)methyl)piperidine-2,6-dione (**Intermediate 25**, 1.6 g, 3.2 mmol) followed by triethylamine (20 mL, 120 mmol). The reaction mixture was purged with argon for 15 min followed by the addition of copper (I) iodide (0.06 g, 0.32 mmol) and $Pd(PPh_3)_2Cl_2$ (0.24 g, 0.32 mmol). The reaction mixture was purged with argon for 10 min and stirred at room temperature for 1h. The reaction mixture was poured into ice cold water (100 mL) and extracted with ethyl acetate (2 × 150 mL). The combined organic layers were separated, dried over anhydrous $Na_2SO_4$ and concentrated *in vacuo.* The crude material was purified by combi-flash column chromatography, by eluting with 7% MeOH in DCM, to afford 1-(10-(2-(2,6-dioxo-1-((2-(trimethylsilyl)ethoxy) methyl)piperidin-3-yl)-1-oxoisoindolin-4-yl)dec-9-yn-1-yl)-3-methyl-1*H*-indazole-6-carboxamide (**Intermediate 228**, 1.3 g, 59%) as a grey solid. $^1$H NMR (400 MHz, DMSO-$d_6$) δ ppm -0.04 (s, 9H), 0.78-0.86 (m, 2H), 1.16-1.46 (m, 9H), 1.48-1.56 (m, 2H), 1.75-1.83 (m, 2H), 2.03-2.10 (m, 1H), 2.36 (m, 1H), 2.39 (s, 3H), 2.73-2.80 (m, 2H), 3.02-3.10 (m, 1H), 3.48-3.57 (m, 2H), 4.21-4.34 (m, 3H), 4.43-4.47 (m, 1H), 4.99-5.10 (m, 2H), 5.25-5.29 (m, 1H), 7.41 (br s, 1H), 7.50-7.54 (m, 1H), 7.58-7.63 (m, 2H), 7.71-7.72 (m, 2H), 8.02 (br s, 1H), 8.11 (s, 1H). Mass spec m/z 684.51 $[M+H]^+$.

**Step 6**

**Intermediate 229: *tert*-butyl-6-(1-(10-(2-(2,6-dioxo-1-((2-(trimethylsilyl)ethoxy)methyl)piperidin-3-yl)-1-oxoisoindolin-4-yl)dec-9-yn-1-yl)-3-methyl-1*H*-indazole-6-carboxamido)-2-((R)-1-methylpyrrolidin-2-yl)-1*H*-pyrrolo[3,2-c]pyridine-1-carboxylate**

To a solution of 1-(10-(2-(2,6-dioxo-1-((2-(trimethylsilyl)ethoxy)methyl)piperidin-3-yl)-1-oxoisoindolin-4-yl)dec-9-yn-1-yl)-3-methyl-1*H*-indazole-6-carboxamide (**Intermediate 228, 0.52** g, 0.75 mmol) in 1,4-dioxane (10 mL) was added tert-butyl (R)-6-bromo-2-(1-methylpyrrolidin-2-yl)-1*H*-pyrrolo[3,2-c]pyridine-1-carboxylate (**Intermediate 1**, 0.36 g, 0.94 mmol) followed by cesium carbonate (0.48 g, 1.42 mmol). The reaction mixture was purged with argon for 15 min then Xantphos (0.086 g, 0.14 mmol) and $Pd_2(dba)_3$ (0.12 g, 0.14 mmol) were added. The reaction mixture was purged with argon gas for 10 min and then heated at 95°C for 1h. The reaction mixture was filtered through celite bed and the filter pad was washed with DCM (30 mL). The filtrate was concentrated *in vacuo* to afford tert-butyl 6-(1-(10-(2-(2,6-dioxo-1-((2-(trimethylsilyl)ethoxy)methyl)piperidin-3-yl)-1-oxoisoindolin-4-yl)dec-9-yn-1-yl)-3-methyl-1*H*-indazole-6-carboxamido)-2-((R)-1-methylpyrrolidin-2-yl)-1*H*-pyrrolo[3,2-c]pyridine-1-carboxylate (**Intermediate 229**, 0.7 g, 75%) as a liquid which was used for the next step without further purification. Mass spec m/z 983.65 $[M+H]^+$.

**Step 7**

**Example 54: 1-(10-(2-(2,6-dioxopiperidin-3-yl)-1-oxoisoindolin-4-yl) dec-9-yn-1-yl)-3-methyl-*N*-(2-((R)-1-methylpyrrolidin-2-yl)-1*H*-pyrrolo[3,2-c]pyridin-6-yl)-1*H*-indazole-6-carboxamide**

To a cooled (0°C) solution of *tert*-butyl-6-(1-(10-(2-(2,6-dioxo-1-((2-(trimethylsilyl)ethoxy)methyl)piperidin-3-yl)-1-oxoisoindolin-4-yl)dec-9-yn-1-yl)-3-methyl-1*H*-indazole-6-carboxamido)-2-((R)-1-methylpyrrolidin-2-yl)-1*H*-pyrrolo[3,2-c]pyridine-1-carboxylate (**Intermediate 229**, 0.50 g, 0.51 mmol) in acetonitrile (8 mL) was added methanesulfonic acid (0.51 g, 5.08 mmol) and the reaction mixture was then heated at 60°C for 2h. After cooling to room temperature, *N*, *N*'-dimethylethylenediamine (0.28 g, 3.05 mmol) followed by triethylamine (1.08 g, 10.17 mmol) were added the reaction mixture was stirred at room temperature for 16h. The reaction mixture was poured into ice cold water (120 mL), the resultant precipitate was collected by filtration and dried *in vacuo.* The crude material was purified by preparative HPLC (Method A) to afford 1-(10-(2-(2,6-dioxopiperidin-3-yl)-1-oxoisoindolin-4-yl)dec-9-yn-1-yl)-3-methyl-*N*-(2-((R)-1-methylpyrrolidin-2-yl)-1*H*-pyrrolo[3,2-c]pyridin-6-yl)-1*H*-indazole-6-carboxamide (**Example 54**, 0.02 g, 5%) as an off white solid. $^1H$ NMR (400 MHz, DMSO-$d_6$) δ ppm 1.24-1.43 (m, 9H), 1.51-1.58 (m, 2H), 1.77-1.93 (m, 5H), 1.96-2.02 (m, 1H), 2.13-2.15 (m, 1H), 2.17 (s, 3H), 2.24-2.31 (m, 1H), 2.41-2.46 (m, 4H), 2.52-2.62 (m, 2H), 2.85-2.95 (m, 1H), 3.13-3.16 (m, 1H), 3.32-3.36 (m, 1H), 4.26-4.32 (m, 1H), 4.38-4.44 (m, 3H), 5.11-5.15 (m, 1H), 6.40 (s, 1H), 7.48-7.53 (m, 1H), 7.58-7.62 (m, 1H), 7.68-7.79 (m, 3H), 8.25 (s, 1H), 8.42 (s, 1H), 8.52 (s, 1H), 10.52 (s, 1H), 11.00 (s, 1H), 11.38 (s, 1H). Mass spec m/z 753.3 $[M+H]^+$.

**Example 55 was synthesized following Scheme 53**

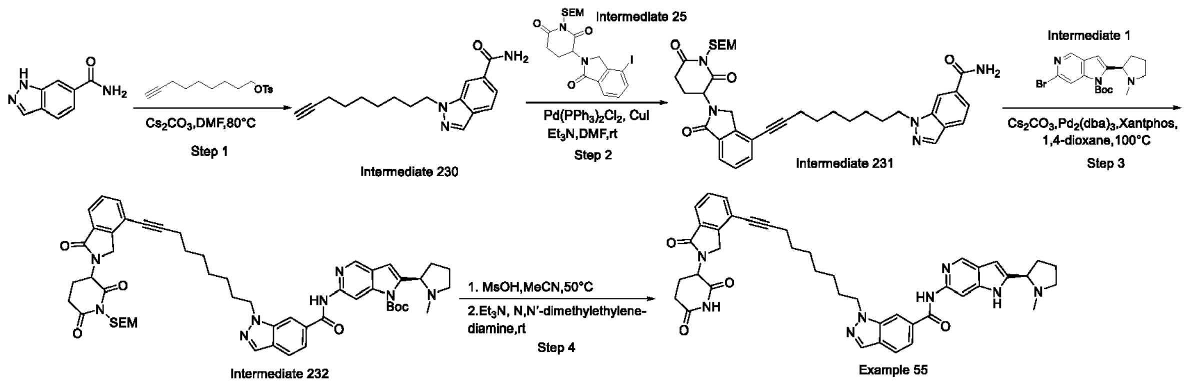


**Scheme 53**

**Step 1**

**Intermediate 230: 1-(non-8-yn-1-yl)-1*H*-indazole-6-carboxamide**

To solution of 1*H*-indazole-6-carboxamide (CAS No: 906000-44-0, 3.00 g, 18.61 mmol) in dimethylformamide (40 mL) was added cesium carbonate (18.3 g, 56.1 mmol) followed by non-8-ynyl-4-methylbenzenesulfonate (CAS No: 87462-64-4, 6.58 g, 22.4 mmol) and the reaction mixture was heated at 80°C for 2h. The reaction mixture was diluted with cold water (100 mL) and extracted with ethyl acetate (3 × 100 mL). The combined organic layers were washed with brine solution (100 mL), separated, dried over anhydrous $Na_2SO_4$, filtered and concentrated *in vacuo* to obtain crude compound as a mixture of regioisomers. The regioisomers were separated by combi-flash chromatography, by eluting with 55% ethyl acetate in heptane, to afford desired regioisomer 1-(non-8-yn-1-yl)-1*H*-indazole-6-carboxamide (**Intermediate 230**, 2.10 g, 40%) as an off white solid. $^1$H NMR (400 MHz, DMSO-$d_6$) δ ppm 1.20-1.42 (m, 8H), 1.80-1.90 (m, 2H), 2.08-2.12 (m, 2H), 2.70 (s, 1H), 4.43 (t, *J*=6.43 Hz, 2H), 7.44 (br s, 1H), 7.63 (d, *J*=8.29 Hz, 1H), 7.79 (d, *J*=8.29 Hz, 1H), 8.05 (br s, 1H), 8.11 (s, 1H), 8.21 (s, 1H). Mass spec m/z 284.0 [M+H]$^+$.

**Step 2**

**Intermediate 231: 1-(9-(2-(2, 6-dioxo-1-((2-(trimethylsilyl) ethoxy) methyl) piperidin-3-yl)-1-oxoisoindolin-4-yl) non-8-yn-1-yl)-1*H*-indazole-6-carboxamide**

To a solution of 1-(non-8-yn-1-yl)-1*H*-indazole-6-carboxamide (**Intermediate 230**, 1.00 g, 3.53 mmol) in dimethylformamide (8 mL) was added 3-(4-iodo-1-oxoisoindolin-2-yl)-1-((2-(trimethylsilyl)ethoxy)methyl)piperidine-2,6-dione (**Intermediate 25**, 1.95 g, 3.90 mmol) followed by triethylamine (12.7 g, 125 mmol) and the reaction mixture was purged with argon gas for 10 min. To the reaction mixture was added $PdCl_2(PPh_3)_2$(0.25 g, 0.36 mmol) followed by copper (I) iodide (0.070 g, 0.36 mmol). The reaction mixture was purged with argon for a further 5 min and then stirred at room temperature for 1h. The reaction mixture was diluted with water (60 mL) and extracted with ethyl acetate (3 × 50 mL). The combined organic layers were washed with brine solution (2 × 50 mL), separated, dried over anhydrous $Na_2SO_4$, filtered, and concentrated *in vacuo.* The crude material was purified by combi-flash column chromatography, by eluting with 2% MeOH in DCM, to afford 1-(9-(2-(2, 6-dioxo-1-((2-(trimethylsilyl) ethoxy) methyl) piperidin-3-yl)-1-oxoisoindolin-4-yl) non-8-yn-1-yl)-1*H*-indazole-6-carboxamide (**Intermediate 231**, 1.00 g, 43%) as a white solid. $^1$H NMR (400 MHz, DMSO-$d_6$) δ ppm -0.04 (s, 9H), 0.76-0.88 (m, 2H), 1.22-1.44 (m, 6H), 1.51-1.54 (m, 2H), 1.80-1.88 (m, 2H), 2.01-2.07 (m, 1H), 2.38-2.45 (m, 3H), 2.76-2.81 (m, 1H), 3.00-3.12 (m, 1H), 3.49-3.54 (m, 2H), 4.23-4.27 (m, 1H), 4.41-4.47 (m, 3H), 5.00-5.09 (m, 2H), 5.24-5.29 (m, 1H), 7.42 (br s, 1H), 7.49-7.53 (m, 1H), 7.61-7.65 (m, 2H), 7.71 (d, *J*=7.46 Hz, 1H), 7.78 (d, *J*=8.29 Hz, 1H), 8.03 (br s, 1H), 8.09 (s, 1H), 8.20 (s, 1H). Mass spec m/z 656.0 [M+H]$^+$.

**Step 3**

**Intermediate 232: *tert*-butyl-6-(1-(9-(2-(2,6-dioxo-1-((2-(trimethylsilyl)ethoxy)methyl)piperidin-3-yl)-1-oxoisoindolin-4-yl)non-8-yn-1-yl)-1*H*-indazole-6-carboxamido)-2-((R)-1-methylpyrrolidin-2-yl)-1*H*-pyrrolo[3,2-c]pyridine-1-carboxylate**

To a solution of 1-(9-(2-(2,6-dioxo-1-((2-(trimethylsilyl)ethoxy)methyl)piperidin-3-yl)-1-oxoisoindolin-4-yl) non-8-yn-1-yl)-1*H*-indazole-6-carboxamide (**Intermediate 231**, 0.70 g, 1.06 mmol) in 1,4-dioxane (15 mL) was added *tert*-butyl (R)-6-bromo-2-(1-methylpyrrolidin-2-yl)-1*H*-pyrrolo[3,2-c]pyridine-1-carboxylate (**Intermediate 1,** 0.49 g, 1.28 mmol) followed by cesium carbonate (1.08 g, 3.31 mmol). The reaction mixture was purged with argon for 5 min. To the reaction mixture was added $Pd_2(dba)_3$ (0.15 g, 0.16 mmol) followed by Xantphos (0.19 g, 0.32 mmol) and the mixture was purged with argon for a further 5 min then heated at 100°C for 2h. The reaction mixture was diluted with water (60 mL) and extracted with ethyl acetate (3 × 80 mL). The combined organic layers were washed with brine solution (2 × 50 mL), separated, dried over anhydrous $Na_2SO_4$, filtered, and concentrated *in vacuo.* The crude material was purified by combi-flash column chromatography, by eluting with 2% MeOH in DCM, to afford *tert*-butyl-6-(1-(9-(2-(2,6-dioxo-1-((2-(trimethylsilyl)ethoxy)methyl)piperidin-3-yl)-1-oxoisoindolin-4-yl)non-8-yn-1-yl)-1*H*-indazole-6-carboxamido)-2-((R)-1-methylpyrrolidin-2-yl)-1*H*-pyrrolo[3,2-c]pyridine-1-carboxylate (**Intermediate 232**, 0.46 g, 45%) as a yellow solid. $^1$H NMR (400 MHz, DMSO-$d_6$) δ ppm -0.06 (s, 9H), 0.76-0.86 (m, 2H), 1.22-1.44 (m, 7H), 1.48-1.57 (m, 2H), 1.60-1.64 (m, 1H), 1.70 (s, 9H), 1.73-1.80 (m, 2H), 1.86-1.93 (m, 2H), 2.00-2.08 (m, 1H), 2.31-2.33 (m, 2H), 2.36 (s, 3H), 2.41-2.47 (m, 3H), 2.73-2.80 (m, 1H), 3.01-3.16 (m, 2H), 3.46-3.53 (m, 2H), 4.21-4.26 (m, 1H), 4.41-4.51 (m, 3H), 4.99-5.07 (m, 2H), 5.25-5.29 (m, 1H), 6.76 (s, 1H), 7.47-7.51 (m, 1H), 7.60 (d, *J*=7.45 Hz, 1H), 7.70 (d, *J*=7.45 Hz, 1H), 7.75 (d, *J*=8.77 Hz, 1H), 7.81-7.87 (m, 1H), 8.14 (s, 1H), 8.52 (s, 1H), 8.61 (s, 1H), 8.98 (s, 1H), 10.80 (s, 1H). Mass spec m/z 955.1 [M+H]$^+$.

**Step 4**

**Example 55: 1-(9-(2-(2, 6-dioxopiperidin-3-yl)-1-oxoisoindolin-4-yl) non-8-yn-1-yl)-*N*-(2-((R)-1-methylpyrrolidin-2-yl)-1*H*-pyrrol[3,2-c]pyridin-6-yl)-1*H*-indazole-6-carboxamide**

To a solution of *tert*-butyl-6-(1-(9-(2-(2,6-dioxo-1-((2-(trimethylsilyl)ethoxy)methyl)piperidin-3-yl)-1-oxoisoindolin-4-yl)non-8-yn-1-yl)-1*H*-indazole-6-carboxamido)-2-((R)-1-methylpyrrolidin-2-yl)-1*H*-pyrrolo[3,2-c]pyridine-1-carboxylate (**Intermediate 232**, 0.45 g, 0.47 mmol) in acetonitrile (8 mL) was added methanesulfonic acid (0.31 mL, 4.71 mmol) at room temperature and the reaction mixture was heated at 50°C for 2h. The reaction mixture was cooled to room temperature, then *N, N'*-dimethylethylenediamine (0.34 mL, 2.82 mmol) followed by triethylamine (1.32 mL, 9.42 mmol) were added and the mixture stirred at room temperature for 2h. The reaction mixture was concentrated *in vacuo* and the obtained solid was washed with ether (2 $\times$ 50 mL) and DCM/Pentanes (2 $\times$ 40 mL) and then dried *in vacuo.* The crude material was purified by preparative HPLC (Method A) to afford 1-(9-(2-(2, 6-dioxopiperidin-3-yl)-1-oxoisoindolin-4-yl) non-8-yn-1-yl)-N-(2-((R)-1-methylpyrrolidin-2-yl)-1*H*-pyrrolo[3,2-c]pyridin-6-yl)-1*H*-indazole-6-carboxamide (**Example 55**, 0.08 g, 23%) as an off white solid. $^1$H NMR (400 MHz, DMSO-$d_6$) $\delta$ ppm 1.28-1.42 (m, 6H), 1.51-1.58 (m, 2H), 1.79-2.02 (m, 6H), 2.17 (s, 3H), 2.24-2.30 (m, 1H), 2.42-2.46 (m, 3H), 2.58-2.62 (m, 1H), 2.85-2.94 (m, 2H), 3.13-3.18 (m, 2H), 4.26-4.32 (m, 1H), 4.39-4.46 (m, 1H), 4.50 (t, *J*=6.91 Hz, 2H), 5.11-5.15 (m, 1H), 6.40 (s, 1H), 7.46-7.52 (m, 1H), 7.60 (d, *J*=7.58, 1H), 7.67-7.71 (m, 2H), 7.75-7.78 (m, 1H), 7.82-7.86 (m, 1H), 8.13 (s, 1H), 8.25 (s, 1H), 8.52 (s, 1H), 10.56 (s, 1H), 11.39 (s, 1H). Mass spec m/z 725.3 [M+H]$^+$.

**Example 56 was synthesized following Scheme 54**

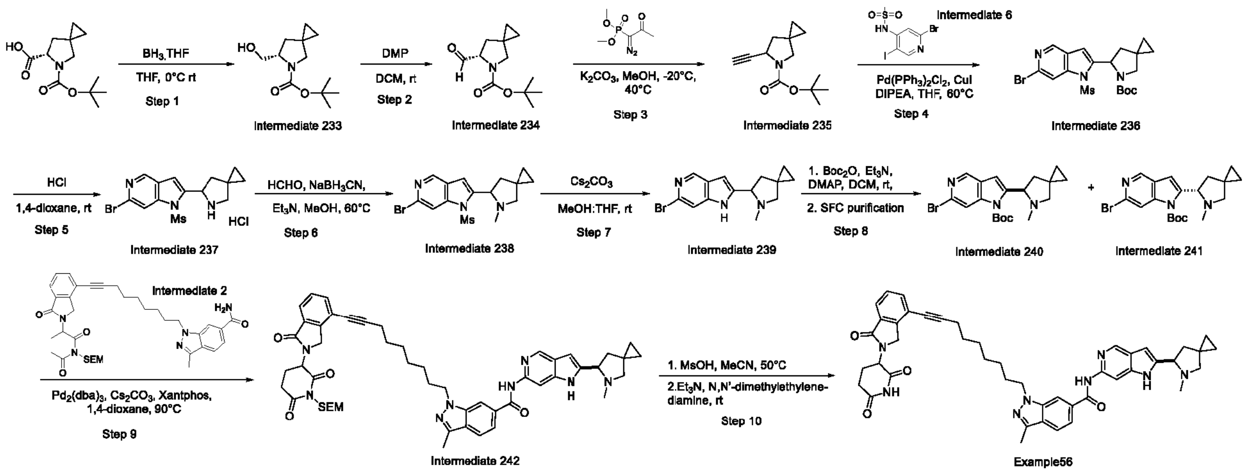


**Scheme 54**

**Step 1**

**Intermediate 233: *tert*-butyl (*S*)-6-(hydroxymethyl)-5-azaspiro [2.4] heptane-5-carboxylate**

To a solution of (*S*)-5-(*tert*-butoxycarbonyl)-5-azaspiro[2.4]heptane-6-carboxylic acid (CAS No: 1129634-44-1, 10.0 g, 41.4 mmol) in tetrahydrofuran (150 mL) was added borane-tetrahydrofuran complex (62.2 mL, 62.2 mmol) in tetrahydrofuran (150 mL) at 0°C and stirred at room temperature for 4h. The reaction mixture was quenched with MeOH (25 mL) and concentrated *in vacuo* to afford tert-butyl (*S*)-6-(hydroxymethyl)-5-azaspiro[2.4]heptane-5-carboxylate (**Intermediate 233**, 9.00 g, 96%) as a brown liquid, which was used for the next step without further purification. $^1$H NMR (400 MHz, DMSO-$d_6$) $\delta$ ppm 0.44-0.63 (m, 4H), 1.39 (s, 9H), 1.57-1.72 (m, 1H), 1.94-2.05 (m, 1H), 2.88-3.00 (m, 1H), 3.33-3.43 (m, 1H), 3.50-3.61 (m, 1H), 3.80-3.86 (m, 1H), 4.26-4.40 (m, 1H), 4.70 (br s, 1H). Mass spec m/z 172.5 [M-56]$^+$.

**Step 2**

**Intermediate 234: *tert*-butyl (*S*)-6-formyl-5-azaspiro [2.4] heptane-5-carboxylate**

To a solution of *tert*-butyl (*S*)-6-(hydroxymethyl)-5-azaspiro[2.4]heptane-5-carboxylate (**Intermediate 233,** 10.0 g, 43.99 mmol) in dichloromethane (150 mL) was added Dess-Martin Periodinane (28.85 g, 65.99 mmol) at 0°C and the

reaction stirred at room temperature for 4h. The reaction mixture was diluted with water (500 mL) and extracted with dichloromethane (3 × 200 mL). The organic layer was separated, dried over anhydrous $Na_2SO_4$, filtered, and concentrated *in vacuo.* The crude material was purified by combi-flash column chromatography, by eluting with 20% ethyl acetate in heptane, to afford *tert*-butyl (*S*)-6-formyl-5-azaspiro[2.4]heptane-5-carboxylate (**Intermediate 234, 6.9** g, 70%) as a yellow liquid. $^1$H NMR (400 MHz, DMSO-$d_6$) δ ppm 0.40-0.48 (m, 1H), 0.51-0.64 (m, 3H), 1.41 (s, 9H), 1.75-1.87 (m, 1H), 2.03-2.14 (m, 1H), 3.19-3.26 (m, 1H), 3.28-3.36 (m, 1H), 4.16-4.25 (m, 1H), 9.48-9.54 (m, 1H).

**Step 3**

**Intermediate 235: *tert*-butyl-6-ethynyl-5-azaspiro [2.4] heptane-5-carboxylate**

To a solution of *tert*-butyl (*S*)-6-formyl-5-azaspiro [2.4] heptane-5-carboxylate (**Intermediate 234**, 6.40 g, 28.0 mmol) in methanol (70 mL) was added potassium carbonate (4.7 g, 34.0 mmol) followed by dimethyl (1-diazo-2-oxopropyl) phosphonate (CAS No: 90965-06-3, 8.2 g, 43.0 mmol) at -20°C. The reaction mixture was stirred at 40°C for 3h. The reaction mixture was diluted with water (500 mL) and extracted with dichloromethane (3 × 100 mL). The organic layer was separated, dried over anhydrous $Na_2SO_4$, filtered, and concentrated *in vacuo.* The crude material was purified by combi-flash column chromatography, by eluting with 5% ethyl acetate in heptane, to afford *tert*-butyl-6-ethynyl-5-azaspiro [2.4] heptane-5-carboxylate (**Intermediate 235**, 5.20 g, 75%) as a yellow solid. $^1$H NMR (400 MHz, DMSO-$d_6$) δ ppm 0.50-0.70 (m, 4H), 1.41 (s, 9H), 1.50-1.71 (m, 1H), 2.27-2.32 (m, 1H), 2.98-3.17 (m, 2H), 3.22-3.37 (m, 1H), 4.40-4.54 (m, 1H).

**Step 4**

**Intermediate 236: *tert*-butyl-6-(6-bromo-1-(methylsulfonyl)-1*H*-pyrrolo[3,2-c]pyridin-2-yl)-5-azaspiro[2.4]heptane-5-carboxylate**

To a solution of *N*-(2-bromo-5-iodopyridin-4-yl)methanesulfonamide (**Intermediate 6**, 7.30 g, 19.36 mmol) in tetrahydrofuran (100 mL) was added *tert*-butyl-6-ethynyl-5-azaspiro[2.4]heptane-5-carboxylate (**Intermediate 235**, 5.14 g, 23.24 mmol) and DIPEA (27.1 mL, 155 mmol). The reaction mixture was purged with argon for 15 min followed by copper (I) iodide (0.37 g, 1.93 mmol) and $Pd(PPh_3)_2Cl_2$ (1.39 g, 1.94 mmol). The reaction mixture was further purged with argon for another 10 min and heated at 60°C for 4h. The reaction mixture was diluted with water (100 mL) and extracted with dichloromethane (3 × 100 mL). The organic layer was separated, dried over anhydrous $Na_2SO_4$, filtered, and concentrated *in vacuo.* The crude material was purified by combi-flash column chromatography, by eluting with 35% ethyl acetate in heptane, to afford tert-butyl-6-(6-bromo-1-(methylsulfonyl)-1H-pyrrolo[3,2-c]pyridin-2-yl)-5-azaspiro[2.4]heptane-5-carboxylate (**Intermediate 236**, 6.0 g, 66%) as yellow solid. $^1$H NMR (401 MHz, DMSO-$d_6$) δ ppm 0.28-0.52 (m, 2H), 0.53-0.72 (m, 2H), 1.16-1.28 (m, 2H), 1.40 (s, 9H), 3.37 (s, 3H), 3.53-3.66 (m, 2H), 5.30-5.43 (m, 1H), 6.81 (s, 1H), 7.98 (s, 1H), 8.72 (s, 1H).

**Step 5**

**Intermediate 237: 6-bromo-1-(methylsulfonyl)-2-(5-azaspiro[2.4]heptan-6-yl)-1*H*-pyrrolo[3,2-c]pyridine hydrochloride**

To a solution of *tert*-butyl-6-(6-bromo-1-(methylsulfonyl)-1*H*-pyrrolo[3,2-c]pyridin-2-yl)-5-azaspiro[2.4]heptane-5-carboxylate (**Intermediate 236**, 6.5 g, 14.0 mmol) in 1,4-dioxane (60 mL) was added 4M HCl in 1,4-dioxane (70 mL). The reaction mixture was stirred at room temperature for 16h. The reaction mixture was concentrated *in vacuo* to afford 6-bromo-1-(methylsulfonyl)-2-(5-azaspiro [2.4] heptan-6-yl)-1*H*-pyrrolo[3,2-c]pyridine hydrochloride (**Intermediate 237**, 5.0 g, 98%) as a yellow solid, which was used for next step without further purification. $^1$H NMR (400 MHz, DMSO-$d_6$) δ ppm 0.68-0.76 (m, 2H), 0.78-0.92 (m, 2H), 2.26-2.35 (m, 2H), 3.07-3.16 (m, 1H), 3.25-3.35 (m, 1H), 3.82 (s, 3H), 5.35-5.46 (m, 1H), 7.46 (s, 1H), 8.03 (s, 1H), 8.84 (s, 1H), 9.68 (br s, 1H), 9.54 (br s, 1H). Mass spec m/z 370.1 $[M+H]^+$.

**Step 6**

**Intermediate 238: 6-bromo-2-(5-methyl-5-azaspiro[2.4]heptan-6-yl)-1-(methylsulfonyl)-1*H*-pyrrolo[3,2-c]pyridine**

To a cooled (0°C) solution of 6-bromo-1-(methylsulfonyl)-2-(5-azaspiro[2.4]heptan-6-yl)-1*H*-pyrrolo[3,2-c]pyridine hydrochloride (**Intermediate 237**, 6.5 g, 18.0 mmol) in methanol (70 mL) was added triethylamine (12.4 mL, 88.5 mmol) and formaldehyde (1.95 mL, 52.9 mmol). After stirring for 5 min, sodium cyanoborohydride (2.62 g, 35.4 mmol) was

added and the reaction mixture was heated at 60°C for 4h. The reaction mixture was diluted with water (500 mL) and extracted with dichloromethane (3 × 250 mL). The organic layer was separated, dried over anhydrous $Na_2SO_4$, filtered, and concentrated *in vacuo.* The crude material was purified by combi-flash column chromatography, by eluting with 35% ethyl acetate in heptane, to afford 6-bromo-2-(5-methyl-5-azaspiro [2.4]heptan-6-yl)-1-(methylsulfonyl)-1*H*-pyrrolo[3,2-c] pyridine (**Intermediate 238, 4.8** g, 71%) as a yellow solid. $^1$H NMR (400 MHz, DMSO-$d_6$) δ ppm 0.48-0.54 (m, 1H), 0.56-0.58 (m, 2H), 0.59-0.72 (m, 1H), 1.68-1.73 (m, 1H), 2.33 (s, 3H), 2.40-2.46 (m, 1H), 2.60-2.66 (m, 1H), 2.78-2.82 (m, 1H), 3.56 (s, 3H), 4.02-4.08 (m, 1H), 6.99 (s, 1H), 7.96 (s, 1H), 8.70 (s, 1H).

**Step 7**

**Intermediate 239: 6-bromo-2-(5-methyl-5-azaspiro[2.4]heptan-6-yl)-1*H*-pyrrolo [3,2-c]pyridine**

To a solution of 6-bromo-2-(5-methyl-5-azaspiro[2.4]heptan-6-yl)-1-(methylsulfonyl)-1H-pyrrolo [3,2-c]pyridine (**Intermediate 238, 4.8** g, 12.0 mmol) in methanol (50 mL) and tetrahydrofuran (50 mL) was added cesium carbonate (8.1 g, 25.0 mmol). The reaction mixture was stirred at room temperature for 4h. The reaction mixture was quenched with water (100 mL) and extracted ethyl acetate (2 × 200 mL). The organic layer was separated, dried over anhydrous $Na_2SO_4$, filtered, and concentrated *in vacuo* to afford (6-bromo-2-(5-methyl-5-azaspiro[2.4]heptan-6-yl)-1*H*-pyrrolo[3,2-c]pyridine (**Intermediate 239, 2.8** g, 73%) as a yellow solid, which was used for next step without further purification. $^1$H NMR (400 MHz, DMSO-$d_6$) δ ppm 0.48-0.56 (m, 2H), 0.58-0.72 (m, 2H), 1.94-2.07 (m, 2H), 2.14 (s, 3H), 2.84-2.92 (m, 1H), 3.55-3.59 (m, 1H), 3.62-3.70 (m, 1H), 6.48 (s, 1H), 7.44 (s, 1H), 8.49 (s, 1H), 11.68 (br s, 1H). Mass spec m/z 306.2 $[M+H]^+$.

**Step 8**

**Intermediate 240: tert-butyl-(rel-R)-6-bromo-2-(5-methyl-5-azaspiro[2.4]heptan-6-yl)-1*H*-pyrrolo[3,2-c] pyridine-1-carboxylate and Intermediate 241: tert-butyl-(rel-*S*)-6-bromo-2-(5-methyl-5-azaspiro[2.4]heptan-6-yl)-1*H*-pyrrolo[3,2-c] pyridine-1-carboxylate**

To a cooled (0°C) solution of 6-bromo-2-(5-methyl-5-azaspiro[2.4]heptan-6-yl)-1*H*-pyrrolo [3,2-c]pyridine (**Intermediate 239**, 5.6 g, 18.0 mmol) in dichloromethane (60 mL) was added triethylamine (10.0 mL, 73.0 mmol) and di-tert-butyl dicarbonate (8.1 g, 37.0 mmol) followed by 4-dimethylaminopyridine (0.45 g, 3.7 mmol). The reaction mixture was stirred at room temperature for 4h. The reaction mixture was diluted with water (300 mL) and extracted with dichloromethane (3 × 200 mL). The organic layer was separated, dried over anhydrous $Na_2SO_4$, filtered, and concentrated *in vacuo.* The resulting material was purified by combi-flash column chromatography, by eluting with 45% ethyl acetate in heptane to afford crude product. This material was further purified by SFC chiral purification (Column: Chiralpak IK 250*30mm, 5 μm. Isocratic elution 25% Mobile Phase (A): $CO_2$, Mobile Phase (B): MeCN+isopropylalcohol) to afford *tert*-butyl-(rel-*S*)-6-bromo-2-(5-methyl-5-azaspiro[2.4]heptan-6-yl)-1*H*-pyrrolo[3,2-c] pyridine-1-carboxylate (**Intermediate 241**, 1.9 g, 26%) and *tert*-butyl-(rel-R)-6-bromo-2-(5-methyl-5-azaspiro[2.4]heptan-6-yl)-1H-pyrrolo[3,2-c] pyridine-1-carboxylate **(Intermediate 240,** 1.40 g, 19%) as a white solid.

**Intermediate 241** exhibited:

1st eluting peak, retention time: 7.67 mins.

$^1$H NMR (400 MHz, DMSO-$d_6$) δ ppm 0.34-0.42 (m, 1H), 0.46-0.64 (m, 3H), 1.56-1.61 (m, 1H), 1.64 (s, 9H), 2.33 (s, 3H), 2.40 - 2.47 (m, 1H), 2.61-2.66 (m, 1H), 2.78-2.80 (m, 1H), 4.10-4.16 (m, 1H), 6.89 (s, 1H), 8.04 (s, 1H), 8.63 (s, 1H). Mass spec m/z 408.10 $[M+H]^+$.

**Intermediate 240** exhibited:

2$^{nd}$ eluting peak, retention time: 9.99 mins.

$^1$H NMR (400 MHz, DMSO-$d_6$) δ ppm 0.34-0.42 (m, 1H), 0.46-0.64 (m, 3H), 1.56-1.61 (m, 1H), 1.64 (s, 9H), 2.33 (s, 3H), 2.40 - 2.47 (m, 1H), 2.61-2.66 (m, 1H), 2.78-2.80 (m, 1H), 4.10-4.16 (m, 1H), 6.89 (s, 1H), 8.04 (s, 1H), 8.63 (s, 1H). Mass spec m/z 408.10 $[M+H]^+$.

**Step 9**

**Intermediate 242: *tert*-butyl 6-(1-(9-(2-(2,6-dioxo-1-((2-(trimethylsilyl)ethoxy)methyl)piperidin-3-yl)-1-oxoisoindolin-4-yl)non-8-yn-1-yl)-3-methyl-1*H*-indazole-6-carboxamido)-2-((rel-*R*)-5-methyl-5-azaspiro[2.4]heptan-6-yl)-1*H*-pyrrolo[3,2-c]pyridine-1-carboxylate**

To a mixture of *tert*-butyl (rel-*R*)-6-bromo-2-(5-methyl-5-azaspiro [2.4]heptan-6-yl)-1*H*-pyrrolo [3,2-c]pyridine-1-carboxylate (**Intermediate 240**, 0.4 g, 0.98 mmol) and 1-(9-(2-(2,6-dioxo-1-((2-(trimethylsilyl)ethoxy)methyl)piperidin-3-yl)-1-oxoisoindolin-4-yl)non-8-yn-1-yl)-3-methyl-1*H*-indazole-6-carboxamide (**Intermediate 2**, 0.52 g, 0.78 mmol) in 1,4-dioxane (15 mL) was added cesium carbonate (0.96 g, 2.95 mmol) at room temperature. The reaction mixture was purged with argon for 15 min. then $Pd_2(dba)_3$ (0.13 g, 0.14 mmol) and Xantphos (0.17 g, 0.29 mmol) were added. The reaction mixture was further purged with argon for another 10 min and heated at 90°C for 2h. The reaction mixture was filtered through celite bed and washed with ethyl acetate (100mL). The filtrate was concentrated *in vacuo* to obtain the crude compound. The crude material was purified by combi-flash column chromatography, by eluting with 90% ethyl acetate in heptane, to afford *tert*-butyl 6-(1-(9-(2-(2,6-dioxo-1-((2-(trimethylsilyl)ethoxy)methyl)piperidin-3-yl)-1-oxoisoindolin-4-yl) non-8-yn-1-yl)-3-methyl-1*H*-indazole-6-carboxamido)-2-((rel-*R*)-5-methyl-5-azaspiro[2.4]heptan-6-yl)-1*H*-pyrrolo[3,2-c] pyridine-1-carboxylate (**Intermediate 242**, **0.58** g, 59%) as a yellow solid. $^1H$ NMR (400 MHz, DMSO-$d_6$) δ ppm -0.06 (s, 9H), 0.40-0.44 (m, 1H), 0.48-0.65 (m, 3H), 0.75-0.86 (m, 2H), 1.24-1.44 (m, 6H), 1.51-1.56 (m, 2H), 1.60-1.64 (m, 2H), 1.70 (s, 9H), 1.81-1.93 (m, 2H), 2.19 (s, 3H), 2.34 (s, 3H), 2.42-2.46 (m, 2H), 2.61-2.69 (m, 2H), 2.73-2.83 (m, 2H), 2.99-3.11 (m, 2H), 3.47-3.54 (m, 2H), 4.13-4.26 (m, 2H), 4.37-4.48 (m, 3H), 4.97-5.10 (m, 2H), 5.24-5.28 (m, 1H), 6.85 (s, 1H), 7.47-7.53 (m, 1H), 7.58-7.62 (m, 1H), 7.68-7.74 (m, 2H), 7.76-7.81 (m, 1H), 8.42 (s, 1H), 8.62 (s, 1H), 8.97 (s, 1H), 10.74 (s, 1H). Mass spec m/z 994.7 $[M+H]^+$.

**Step 10**

**Example 56: 1-(9-(2-(2,6-dioxopiperidin-3-yl)-1-oxoisoindolin-4-yl)non-8-yn-1-yl)-3-methyl-*N*-(2-((rel-*R*)-5-methyl-5-azaspiro[2.4]heptan-6-yl)-1*H*-pyrrolo[3,2-c]pyridin-6-yl)-1*H*-indazole-6-carboxamide**

To a solution of *tert*-butyl 6-(1-(9-(2-(2,6-dioxo-1-((2-(trimethylsilyl)ethoxy)methyl)piperidin-3-yl)-1-oxoisoindolin-4-yl)non-8-yn-1-yl)-3-methyl-1*H*-indazole-6-carboxamido)-2-((rel-*R*)-5-methyl-5-azaspiro[2.4]heptan-6-yl)-1*H*-pyrrolo[3,2-c]pyridine-1-carboxylate (**Intermediate 242,** 0.58 g, 0.58 mmol) in acetonitrile (15 mL) was added methanesulfonic acid (0.58 g, 5.9 mmol) at room temperature and the reaction heated at 50°C for 2h. To the reaction mixture *N*, *N*'-dimethylethylenediamine (0.29 g, 2.9 mmol) and triethylamine (1.64 mL, 11.7 mmol) were added. The reaction mixture was stirred at room temperature for 3h. The reaction mixture was concentrated *in vacuo.* The crude compound was quenched with water (50 mL) and the resultant precipitate collected by filtration and dried *in vacuo.* The crude material was purified by preparative HPLC (Method A) to afford 1-(9-(2-(2,6-dioxopiperidin-3-yl)-1-oxoisoindolin-4-yl)non-8-yn-1-yl)-3-methyl-N-(2-((rel-R)-5-methyl-5-azaspiro[2.4]heptan-6-yl)-1*H*-pyrrolo[3,2-c]pyridin-6-yl)-1*H*-indazole-6-carboxamide (**Example 56**, 0.19 g, 43%) as an off white solid. $^1H$ NMR (400 MHz, DMSO-$d_6$) δ ppm 0.50-0.73 (m, 4H), 1.25-1.45 (m, 6H), 1.49-1.60 (m, 2H), 1.82-1.91 (m, 2H), 1.95-2.10 (m, 3H), 2.16 (s, 3H), 2.40-2.48 (m, 4H), 2.52 (s, 3H), 2.54-2.61 (m, 1H), 2.84-2.96 (m, 2H), 3.59-3.63 (m, 1H), 4.22-4.35 (m, 1H), 4.36-4.46 (m, 3H), 5.11-5.16 (m, 1H), 6.44 (s, 1H), 7.46-7.52 (m, 1H), 7.58-7.63 (m, 1H), 7.68-7.80 (m, 3H), 8.25 (s, 1H), 8.43 (s, 1H), 8.52 (s, 1H), 10.52 (s, 1H), 11.00 (s, 1H), 11.47 (s, 1H). Mass spec m/z 765.0 $[M+H]^+$.

**Example 57 was synthesized following Scheme 55**

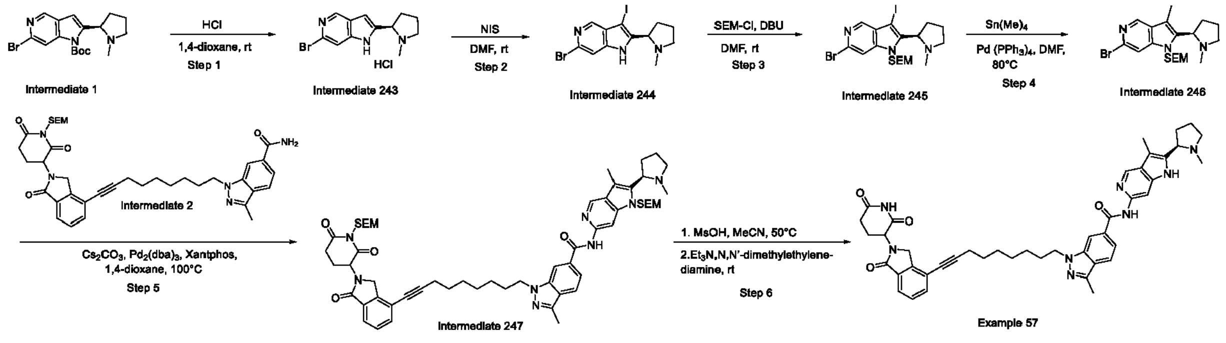


**Scheme 55**

**Step 1**

**Intermediate 243: (R)-6-bromo-2-(1-methylpyrrolidin-2-yl)-1*H*-pyrrolo[3,2-c]pyridine hydrochloride**

To a solution of *tert*-butyl (R)-6-bromo-2-(1-methylpyrrolidin-2-yl)-1*H*-pyrrolo[3,2-c]pyridine-1-carboxylate (**Intermediate 1,** 10.0 g, 26.3 mmol) in 1, 4-dioxane (100 mL) was added 4M HCl in 1,4-dioxane (100 mL) at 0°C. The reaction

mixture was stirred at room temperature for 16h. The reaction mixture was concentrated *in vacuo* to obtain the crude compound. The crude material was washed with ether (100 mL) to afford (R)-6-bromo-2-(1-methylpyrrolidin-2-yl)-1*H*-pyrrolo[3, 2-c]pyridine hydrochloride (**Intermediate 243**, 8.00 g) as an off white solid, which was used for the next step without further purification. Mass spec: m/z: 280.5 $[M+H]^+$.

**Step** 2

**Intermediate 244: (*R*)-6-bromo-3-iodo-2-(1-methylpyrrolidin-2-yl)-1*H*-pyrrolo [3,2-c] pyridine**

To a solution of (*R*)-6-bromo-2-(1-methylpyrrolidin-2-yl)-1*H*-pyrrolo[3,2-c]pyridine hydrochloride (**Intermediate 243**, 2.50 g, 8.9 mmol) in dimethylformamide (20 mL) was added *N*-iodosuccinimide (4.2 g, 18 mmol). The reaction mixture was stirred at room temperature for 16h. The reaction mixture was concentrated *in vacuo.* The crude compound was quenched with water (50 mL) and the resultant precipitate collected by filtration and dried *in vacuo* to afford (R)-6-bromo-3-iodo-2-(1-methylpyrrolidin-2-yl)-1H-pyrrolo [3, 2-c] pyridine (**Intermediate 244**, 1.50 g, 41%) as a brown solid, which was used for the next step without further purification. $^1$H NMR (400 MHz, DMSO-$d_6$) δ ppm 1.69-1.97 (m, 3H), 2.15 (s, 3H), 2.17-2.20 (m, 1H), 2.29-2.35 (m, 1H), 3.16-3.20 (m, 1H), 3.46-3.50 (m, 1H), 7.45 (s, 1H), 8.26 (s, 1H), 11.96 (br s, 1H). Mass spec m/z 406.0 $[M+H]^+$.

**Step 3**

**Intermediate 245: (R)-6-bromo-3-iodo-2-(1-methylpyrrolidin-2-yl)-1-((2-(trimethylsilyl) ethoxy) methyl)-1*H*-pyrrolo [3, 2-c] pyridine**

To a solution of (*R*)-6-bromo-3-iodo-2-(1-methylpyrrolidin-2-yl)-1*H*-pyrrolo [3, 2-c] pyridine (**Intermediate 244,** 1.40 g, 3.4 mmol) in dimethylformamide (15 mL) was added DBU (1.9 g, 12 mmol) at 0°C followed by SEM-Cl (2.2 mL, 12 mmol) under nitrogen atmosphere. The reaction mixture was stirred at room temperature for 16h then quenched with ice cold water (100 mL) and extracted with ethyl acetate (2 × 100 mL). The organic layer was separated, washed with brine solution (100 mL), dried over anhydrous $Na_2SO_4$, filtered and concentrated *in vacuo.* The crude material was purified by combi-flash chromatography, by eluting with 15% ethyl acetate in heptane, to afford (*R*)-6-bromo-3-iodo-2-(1-methylpyrrolidin-2-yl)-1-((2-(trimethylsilyl) ethoxy) methyl)-1H-pyrrolo [3, 2-c] pyridine (**Intermediate 245**, 1.30 g, 70%) as a colourless liquid. $^1$H NMR (400 MHz, DMSO-$d_6$) δ ppm -0.10 (s, 9H), 0.72-0.88 (m, 2H), 1.78-1.96 (m, 2H), 1.98-2.04 (m, 1H), 2.07 (s, 3H), 2.09-2.14 (m, 1H), 2.16-2.24 (m, 1H), 3.13-3.16 (m, 1H), 3.42-3.50 (m, 1H), 3.52-3.60 (m, 1H), 3.65-3.69 (m, 1H), 5.65-7.68 (m, 1H), 5.92-5.96 (m, 1H), 7.82 (s, 1H), 8.26 (s, 1H). Mass spec m/z 536.0 $[M+H]^+$.

**Step** 4

**Intermediate 246: (*R*)-6-bromo-3-methyl-2-(1-methylpyrrolidin-2-yl)-1-((2-(trimethylsilyl) ethoxy) methyl)-1*H*-pyrrolo [3, 2-c] pyridine**

To a solution of (R)-6-bromo-3-iodo-2-(1-methylpyrrolidin-2-yl)-1-((2-(trimethylsilyl) ethoxy) methyl)-1H-pyrrolo [3, 2-c] pyridine (**Intermediate 245,** 1.00 g, 1.9 mmol) in dimethylformamide (10 mL) was added tetramethyl tin (1.1 g, 5.6 mmol) and the mixture purged with argon for 15 min. To the reaction mixture $Pd(PPh_3)_4$ (0.22 g, 0.19 mmol) was added and the mixture purged with argon for 10 min. The reaction mixture was heated at 80°C for 3h. The reaction mixture was poured into ice cold water (30 mL) and extracted with ethyl acetate (3 × 50 mL). The organic layer was separated, dried over anhydrous $Na_2SO_4$, filtered, and concentrated *in vacuo.* The crude material was purified by combi-flash chromatography, by eluting with 40% ethyl acetate in heptane, to afford (*R*)-6-bromo-3-methyl-2-(1-methylpyrrolidin-2-yl)-1-((2-(trimethylsilyl) ethoxy) methyl)-1*H*-pyrrolo [3, 2-c] pyridine (**Intermediate 246**, 0.40 g, 51%) as yellow sticky solid. $^1$H NMR (400 MHz, DMSO-$d_6$) δ ppm -0.07 (s, 9H), 0.74-0.89 (m, 2H), 1.77-2.04 (m, 3H), 2.09 (s, 3H), 2.13-2.22 (m, 1H), 2.31 (s, 3H), 3.13-3.17 (m, 1H), 3.40-3.59 (m, 4H), 5.58-5.62 (m, 1H), 5.80-5.82 (m, 1H), 7.77 (s, 1H), 8.53 (s, 1H). Mass spec m/z 424.20 $[M+H]^+$.

**Step 5**

**Intermediate 247: 1-(9-(2-(2,6-dioxo-1-((2-(trimethylsilyl)ethoxy)methyl)piperidin-3-yl)-1-oxoisoindolin-4-yl) non-8-yn-1-yl)-3-methyl-*N*-(3-methyl-2-((*R*)-1-methylpyrrolidin-2-yl)-1-((2-(trimethylsilyl)ethoxy)methyl)-1*H*-pyrrolo[3,2-c]pyridin-6-yl)-1*H*-indazole-6-carboxamide**

To a solution of 1-(9-(2-(2,6-dioxo-1-((2-(trimethylsilyl)ethoxy)methyl)piperidin-3-yl)-1-oxoisoindolin-4-yl)non-8-yn-1-yl)-3-methyl-1*H*-indazole-6-carboxamide (**Intermediate 2,** 0.42 g, 0.72 mmol) in 1,4-dioxane (15 mL) was added (R)-6-bromo-3-methyl-2-(1-methylpyrrolidin-2-yl)-1-((2-(trimethylsilyl)ethoxy)methyl)-1*H*-pyrrolo[3,2-c]pyridine (**Intermediate 246**, 0.40 g, 0.91 mmol) followed by cesium carbonate (0.89 g, 2.72 mmol) at room temperature. The reaction mixture was purged with argon for 15 min followed by $Pd_2(dba)_3$ (0.13 g, 0.13 mmol) and Xantphos (0.16 g, 0.27 mmol). The reaction mixture was further purged with argon for 10 min and heated at 100°C for 2h. The reaction mixture was concentrated *in vacuo* to obtain the crude compound. The crude material was purified by combi-flash chromatography, by eluting with 80% ethyl acetate in heptane, to afford 1-(9-(2-(2,6-dioxo-1-((2-(trimethylsilyl)ethoxy)methyl)piperidin-3-yl)-1-oxoisoindolin-4-yl)non-8-yn-1-yl)-3-methyl-*N*(3-methyl-2-((*R*)-1-methylpyrrolidin-2-yl)-1-((2-(trimethylsilyl)ethoxy) methyl)-1*H*-pyrrolo[3,2-c]pyridin-6-yl)-1*H*-indazole-6-carboxamide (**Intermediate 247**, 0.42 g, 46%) as a yellow solid. $^1H$ NMR (400 MHz, DMSO-$d_6$) δ ppm -0.06 (s, 9H), -0.05 (s, 9H), 0.76-0.96 (m, 4H), 1.22-1.44 (m, 6H), 1.48-1.59 (m, 2H), 1.80-1.94 (m, 5H), 2.00-2.09 (m, 2H), 2.12 (s, 3H), 2.15-2.23 (m, 2H), 2.33 (s, 3H), 2.41-2.46 (m, 3H), 2.74-2.80 (m, 2H), 3.02-3.10 (m, 1H), 3.15-3.20 (m, 1H), 3.45-3.64 (m, 6H), 4.22-4.26 (m, 1H), 4.37-4.47 (m, 3H), 4.97-5.09 (m, 2H), 5.24-5.30 (m, 1H), 5.52-5.56 (m, 1H), 5.84-5.88 (m, 1H), 7.46-7.52 (m, 1H), 7.58-7.62 (m, 1H), 7.68-7.82 (m, 3H), 8.41 (s, 1H), 8.45 (s, 1H), 8.55 (s, 1H), 10.65 (s, 1H). Mass spec m/z 1013.64 $[M+H]^+$.

**Step 6**

**Example 57: 1-(9-(2-(2,6-dioxopiperidin-3-yl)-1-oxoisoindolin-4-yl)non-8-yn-1-yl)-3-methyl-*N*-(3-methyl-2-((*R*)-1-methylpyrrolidin-2-yl)-1*H*-pyrrolo[3,2-c]pyridin-6-yl)-1*H*-indazole-6-carboxamide**

To a solution of 1-(9-(2-(2,6-dioxo-1-((2-(trimethylsilyl)ethoxy)methyl)piperidin-3-yl)-1-oxoisoindolin-4-yl)non-8-yn-1-yl)-3-methyl-*N*-(3-methyl-2-((*R*)-1-methylpyrrolidin-2-yl)-1-((2-(trimethylsilyl)ethoxy)methyl)-1*H*-pyrrolo[3,2-c]pyridin-6-yl)-1*H*-indazole-6-carboxamide (**Intermediate 247**, 0.38 g, 0.37 mmol) in acetonitrile (10 mL) was added methanesulfonic acid (0.49 mL, 7.49 mmol) at room temperature and the reaction mixture was heated at 50°C for 2h. After cooling to room temperature, *N*, *N*'-dimethylethylenediamine (0.45 mL, 3.75 mmol) followed by triethylamine (2.10 mL, 14.99 mmol) were added and the reaction stirred for 3h. The reaction mixture was poured into ice cold water (20 mL) and the resultant precipitate collected by filtration and dried *in vacuo* to obtain the crude compound. The crude material was purified by preparative HPLC (Method A) to afford 1-(9-(2-(2,6-dioxopiperidin-3-yl)-1-oxoisoindolin-4-yl)non-8-yn-1-yl)-3-methyl-*N*-(3-methyl-2-((*R*)-1-methylpyrrolidin-2-yl)-1*H*-pyrrolo[3,2-c]pyridin-6-yl)-1*H*-indazole-6-carboxamide (**Example 57**, 0.12 g , 43%) as an off white solid. $^1H$ NMR (400 MHz, DMSO-$d_6$) δ ppm 1.27-1.44 (m, 6H), 1.47-1.60 (m, 2H), 1.78-2.02 (m, 6H), 2.07-2.11 (m, 1H), 2.13 (s, 3H), 2.23-2.26 (m, 1H), 2.27 (s, 3H), 2.42-2.47 (m, 3H), 2.49 (s, 3H), 2.54-2.62 (m, 1H), 2.85-2.94 (m, 1H), 3.14-3.18 (m, 1H), 3.39-3.43 (m, 1H), 4.25-4.33 (m, 1H), 4.37-4.46 (m, 3H), 5.10-5.15 (m, 1H), 7.44-7.53 (m, 1H), 7.58-7.60 (m, 1H), 7.67-7.80 (m, 3H), 8.20 (s, 1H), 8.43 (s, 1H), 8.49 (s, 1H), 10.50 (s, 1H), 11.00 (s, 1H), 11.06 (s, 1H). Mass spec m/z 753.2 $[M+H]^+$.

**Example 58 was synthesized following Scheme 56**

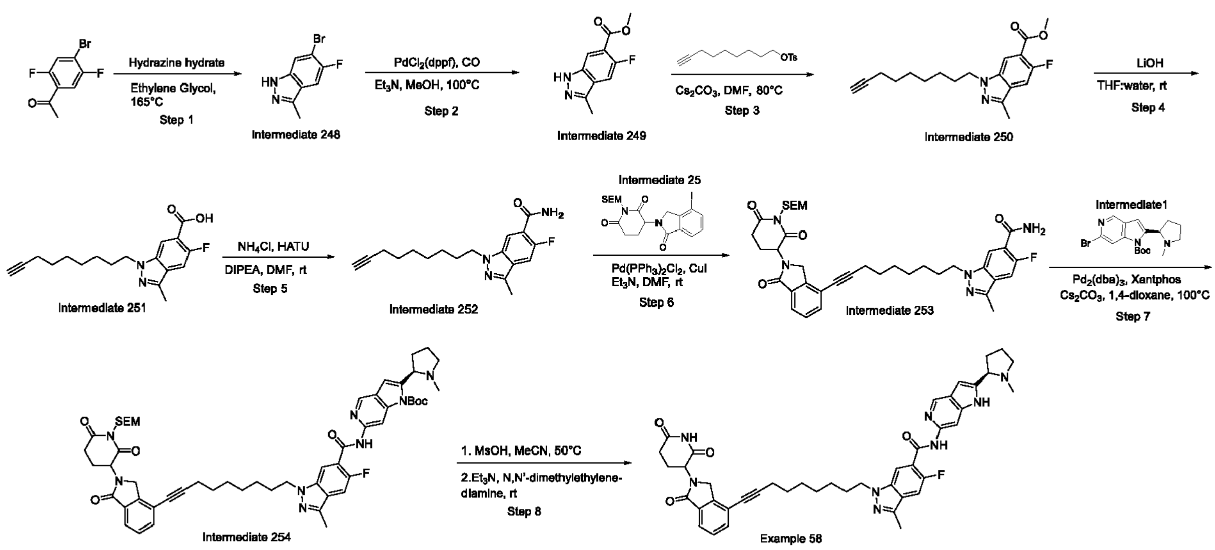


**Scheme 56**

**Step 1**

**Intermediate 248: 6-bromo-5-fluoro-3-methyl-1*H*-indazole**

To a solution of 1-(4-bromo-2,5-difluorophenyl)ethan-1-one (CAS No: 123942-11-0, 5.00 g, 21.27 mmol) in ethylene glycol (50 mL, 892.0 mmol) was added hydrazine monohydrate (1.69 mL, 53.18 mmol) at room temperature. The reaction mixture was heated at 165°C for 16h. The reaction mixture was quenched with water (1000 mL) and the resultant precipitate collected by filtration. The obtained solid was washed with water (3 × 250 mL) and *n*-pentane (3 × 60 mL) and then dried *in vacuo* to afford 6-bromo-5-fluoro-3-methyl-1*H*-indazole (**Intermediate 248**, 2.50 g, 51%) as an off white solid, which was used for the next step without further purification. $^1$H NMR (400 MHz, DMSO-$d_6$) δ ppm 2.46 (s, 3H), 7.72 (d, *J*=8.93 Hz, 1H), 7.82 (d, *J*=5.62 Hz, 1H), 12.83 (br s, 1H). Mass spec m/z 231.3 $[M+H]^+$.

**Step 2**

**Intermediate 249: methyl 5-fluoro-3-methyl-1*H*-indazole-6-carboxylate**

To a solution of 6-bromo-5-fluoro-3-methyl-1*H*-indazole (**Intermediate 248**, 2.50 g, 11.0 mmol) in methanol (40 mL) was added triethylamine (4.6 mL, 33.0 mmol) followed by $PdCl_2$(dppf) (0.81 g, 1.1 mmol) at room temperature. The reaction mixture was stirred under CO atmosphere (100 psi) at 100°C for 16h. The reaction mixture was filtered through celite bed and concentrated *in vacuo.* The crude material was purified by combi-flash chromatography, by eluting with 30% ethyl acetate in heptane, to afford methyl 5-fluoro-3-methyl-1*H*-indazole-6-carboxylate (**Intermediate 249**, 1.60 g, 70%) as an off white solid. $^1$H NMR (400 MHz, DMSO-$d_6$) δ ppm 2.48 (s, 3H), 3.88 (s, 3H), 7.67 (d, *J*=10.96 Hz, 1H), 7.98 (d, *J*=5.26 Hz, 1H), 13.05 (br s, 1H). Mass spec m/z 209.5 $[M+H]^+$.

**Step 3**

**Intermediate 250: methyl 5-fluoro-3-methyl-1-(non-8-yn-1-yl)-1*H*-indazole-6-carboxylate**

To a solution of methyl 5-fluoro-3-methyl-1*H*-indazole-6-carboxylate **(Intermediate 249,** 1.60 g, 7.7 mmol) in dimethylformamide (13 mL) was added non-8-yn-1-yl 4-methylbenzenesulfonate (CAS 87462-64-4, 2.9 g, 10.0 mmol) followed by cesium carbonate (7.50 g, 23.0 mmol). The reaction mixture was stirred at 80°C for 2h. The reaction mixture was diluted with water (50 mL) and extracted with ethyl acetate (3 × 200 mL). The organic layer was separated, washed with brine solution (3 × 200 mL), dried over anhydrous $Na_2SO_4$, filtered, and concentrated *in vacuo* to obtain the crude compound as a mixture of regioisomers. The regioisomers were separated by combi-flash chromatography, by eluting with 30% ethyl acetate, to afford desired regioisomer methyl 5-fluoro-3-methyl-1-(non-8-yn-l-yl)-1*H*-indazole-6-carboxylate **(Intermediate 250,** 1.60 g, 63%) as a colourless liquid. $^1$H NMR (400 MHz, DMSO-$d_6$) δ ppm 1.16-1.33 (m, 6H), 1.34-1.46 (m, 2H), 1.74-1.81 (m, 2H), 2.06-2.15 (m, 2H), 2.48 (s, 3H), 2.69-2.72 (m, 1H), 3.89 (s, 3H), 4.38 (t, *J*=7.02 Hz, 2H), 7.66 (d, *J*=10.96 Hz, 1H), 8.16 (d, *J*=5.70 Hz, 1H). Mass spec m/z 331.3 $[M+H]^+$.

**Step 4**

**Intermediate 251: 5-fluoro-3-methyl-1-(non-8-yn-1-yl)-1*H*-indazole-6-carboxylic acid**

To a solution of methyl 5-fluoro-3-methyl-1-(non-8-yn-1-yl)-1*H*-indazole-6-carboxylate (**Intermediate 250,** 1.5 g, 4.5 mmol) in methanol (11 mL) and tetrahydrofuran (11 mL) was added lithium hydroxide (0.44 g, 18.0 mmol) in water (5 mL) at room temperature. The reaction mixture was stirred at room temperature for 16h. The reaction mixture was concentrated *in vacuo.* The reaction mixture was diluted with water (25 mL) and the aqueous layer was acidified by adjusted to pH~4 with 0.5 N HCl. The resultant precipitate collected by filtration and dried *in vacuo* to afford 5-fluoro-3-methyl-1-(non-8-yn-1-yl)-1*H*-indazole-6-carboxylic acid (**Intermediate 251**, 0.90 g, 63%) as a white solid, which was used for the next step without further purification. $^1$H NMR (400 MHz, DMSO-$d_6$) δ ppm 1.17-1.32 (m, 6H), 1.34-1.44 (m, 2H), 1.74-1.81 (m, 2H), 2.08-2.13 (m, 2H), 2.47 (s, 3H), 2.68-2.70 (m, 1H), 4.37 (t, *J*=7.00 Hz, 2H), 7.60 (d, *J*=10.88 Hz, 1H), 8.11 (d, *J*=5.63 Hz, 1H), 13.32 (br s, 1H). Mass spec m/z 317.1 $[M+H]^+$.

**Step 5**

**Intermediate 252: 5-fluoro-3-methyl-1-(non-8-yn-1-yl)-1H indazole-6-carboxamide**

To a solution of 5-fluoro-3-methyl-1-(non-8-yn-1-yl)-1*H*-indazole-6-carboxylic acid (**Intermediate 251,** 1.1 g, 3.5

mmol) in dimethylformamide (11 mL) were added HATU (2.0 g, 5.2 mmol) and DIPEA (1.8 mL, 10.0 mmol) at room temperature and stirred for 20 min. Then ammonium chloride (0.93 g, 17.0 mmol) was added and the reaction mixture was stirred at room temperature for 16h. The reaction mixture was quenched with water (200 mL) to obtain a precipitate which was filtered and dried *in vacuo* to afford 5-fluoro-3-methyl-1-(non-8-yn-1-yl)-1*H*-indazole-6-carboxamide (**Intermediate 252**, 0.85 g, 74%) as a white solid, which was used in the next step without further purification. $^1$H NMR (400 MHz, DMSO-$d_6$) δ ppm 1.19-1.31 (m, 6H), 1.35-1.46 (m, 2H), 1.75-1.81 (m, 2H), 2.09-2.14 (m, 2H), 2.46 (s, 3H), 2.70 (s, 1H), 4.33 (t, *J*=6.80 Hz, 2H), 7.57 (d, *J*=10.40 Hz, 1H), 7.66 (br s, 1H), 7.75 (br s, 1H), 7.87 (d, *J*=6.00 Hz, 1H). Mass spec m/z 317.1 $[M+H]^+$.

**Step 6**

**Intermediate 253: 1-(9-(2-(2, 6-dioxo-1-((2-(trimethylsilyl) ethoxy) methyl) piperidin-3-yl)-1-oxoisoindolin-4-yl) non-8-yn-1-yl)-5-fluoro-3-methyl-1*H*-indazole-6-carboxamide**

To a mixture of 5-fluoro-3-methyl-1-(non-8-yn-1-yl)-1*H*-indazole-6-carboxamide (**Intermediate 252**, 0.40 g, 1.26 mmol) and 3-(4-iodo-1-oxoisoindolin-2-yl)-1-((2-(trimethylsilyl)ethoxy)methyl)piperidine-2,6-dione (**Intermediate 25**, 0.63 g, 1.26 mmol) in dimethylformamide (5 mL) was added triethylamine (6.40 mL, 45.66 mmol). The reaction mixture was purged with argon gas for 15 min. To the reaction mixture $PdCl_2(PPh_3)_2$ (0.08 g, 0.12 mmol) and copper (I) iodide (0.02 g, 0.12 mmol) were added. Then the reaction mixture was purged with argon gas for another 10 min and stirred at room temperature for 2h. The reaction mixture was diluted with water (200 mL) and extracted with ethyl acetate (3 × 150 mL). The organic layer was separated, dried over anhydrous $Na_2SO_4$, filtered, and concentrated *in vacuo.* The crude material was purified by combi-flash chromatography, by eluting with 80% ethyl acetate in heptane, to afford 1-(9-(2-(2, 6-dioxo-1-((2-(trimethylsilyl) ethoxy) methyl) piperidin-3-yl)-1-oxoisoindolin-4-yl) non-8-yn-1-yl)-5-fluoro-3-methyl-1*H*-indazole-6-carboxamide (**Intermediate 253**, 0.70 g, 81%) as a yellow solid. $^1$H NMR (400 MHz, DMSO-$d_6$) δ ppm -0.04 (s, 9H), 0.77-0.87 (m, 2H), 1.17-1.44 (m, 6H), 1.50-1.57 (m, 2H), 1.74-1.82 (m, 2H), 2.01-2.09 (m, 1H), 2.40-2.42 (m, 1H), 2.45 (s, 3H), 2.46-2.48 (m, 2H), 2.75-2.82 (m, 1H), 3.01-3.12 (m, 1H), 3.46-3.57 (m, 2H), 4.22-4.37 (m, 3H), 4.43-4.47 (m, 1H), 4.99-5.10 (m, 2H), 5.25-5.29 (m, 1H), 7.49-7.63 (m, 3H), 7.66 (br s, 1H), 7.70-7.72 (m, 1H), 7.74 (br s, 1H), 7.88 (d, J=5.26 Hz, 1H). Mass spec m/z 686.0 $[M+H]^+$.

**Step 7**

**Intermediate 254: *tert*-butyl 6-(1-(9-(2-(2,6-dioxo-1-((2-(trimethylsilyl)ethoxy)methyl)piperidin-3-yl)-1-oxoisoindolin-4-yl)non-8-yn-1-yl)-5-fluoro-3-methyl-1*H*-indazole-6-carboxamido)-2-((*R*)-1-methylpyrrolidin-2-yl)-1*H*-pyrrolo[3,2-*c*]pyridine-1-carboxylate**

To a solution of 1-(9-(2-(2, 6-dioxo-1-((2-(trimethylsilyl) ethoxy) methyl) piperidin-3-yl)-1-oxoisoindolin-4-yl) non-8-yn-1-yl)-5-fluoro-3-methyl-1*H*-indazole-6-carboxamide (**Intermediate 253**, 0.65 g, 0.94 mmol) in 1,4-dioxane (10 mL) was added tert-butyl (*R*)-6-bromo-2-(1-methylpyrrolidin-2-yl)-1*H*-pyrrolo[3,2-c]pyridine-1-carboxylate (**Intermediate 1,** 0.45 g, 1.18 mmol) followed by cesium carbonate (1.15 g, 3.55 mmol) at room temperature. The reaction mixture was purged with argon for 15 min. To the reaction mixture $Pd_2(dba)_3$ (0.16 g, 0.17 mmol) and Xantphos (0.21 g, 0.35 mmol) were added. Then the reaction mixture was purged with argon for 10 min and stirred at 100°C for 2h. The reaction mixture was filtered through celite bed and washed with ethyl acetate (50 mL). The filtrate was concentrated *in vacuo.* The crude material was purified by combi-flash column chromatography, by eluting with 100% ethyl acetate in heptane, to afford *tert*-butyl 6-(1-(9-(2-(2,6-dioxo-1-((2-(trimethylsilyl)ethoxy)methyl)piperidin-3-yl)-1-oxoisoindolin-4-yl) non-8-yn-1-yl)-5-fluoro-3-methyl-1*H*-indazole-6-carboxamido)-2-((*R*)-1-methylpyrrolidin-2-yl)-1*H*-pyrrolo[3,2-*c*]pyridine-1-carboxylate (**Intermediate 254,** 0.48 g, 41%) as yellow solid. $^1$H NMR (400 MHz, DMSO-$d_6$) δ ppm -0.05 (s, 9H), 0.75-0.87 (m, 2H), 1.22-1.44 (m, 6H), 1.48-1.63 (m, 4H), 1.69 (s, 9H), 1.71-1.87 (m, 4H), 1.95-2.09 (m, 2H), 2.36 (s, 3H), 2.47 (s, 3H), 2.73-2.79 (m, 1H), 2.98-3.19 (m, 3H), 3.41-3.57 (m, 3H), 3.87-3.97 (m, 1H), 4.22-4.46 (m, 5H), 4.97-5.10 (m, 2H), 5.24-5.29 (m, 1H), 6.75 (s, 1H), 7.46-7.54 (m, 1H), 7.57-7.65 (m, 2H), 7.70 (d, *J*=7.89 Hz, 1H), 7.98 (d, *J*=5.26 Hz, 1H), 8.57 (s, 1H), 8.95 (s, 1H), 10.78 (br s, 1H). Mass spec m/z 986.7 $[M+H]^+$.

**Step 8**

**Example 58: 1-(9-(2-(2,6-dioxopiperidin-3-yl)-1-oxoisoindolin-4-yl)non-8-yn-1-yl)-5-fluoro-3-methyl-*N*-(2-((*R*)-1-methylpyrrolidin-2-yl)-1*H*-pyrrolo[3,2-*c*]pyridin-6-yl)-1*H*-indazole-6-carboxamide**

To a solution of tert-butyl 6-(1-(9-(2-(2,6-dioxo-1-((2-(trimethylsilyl)ethoxy)methyl)piperidin-3-yl)-1-oxoisoindolin-4-yl)non-8-yn-1-yl)-5-fluoro-3-methyl-1*H*-indazole-6-carboxamido)-2-((*R*)-1-methylpyrrolidin-2-yl)-1*H*-pyrrolo[3,2-c]

pyridine-1-carboxylate (**Intermediate 254,** 0.48 g, 0.48 mmol) in acetonitrile (10 mL) was added methanesulfonic acid (0.32 mL, 4.86 mmol) at room temperature and the reaction stirred at 50°C for 2h. To the reaction mixture *N*, *N*'-dimethylethylenediamine (0.29 mL, 2.43 mmol) and triethylamine (1.36 mL, 9.72 mmol) were added. The reaction mixture was stirred at room temperature for 3h. The reaction mixture was concentrated. The crude compound was quenched with water (50 mL) to get a solid precipitate which was filtered and dried in *vacuo* to afford the crude product. The crude material was purified by preparative HPLC (Method A) to afford 1-(9-(2-(2,6-dioxopiperidin-3-yl)-1-oxoisoindolin-4-yl)non-8-yn-1-yl)-5-fluoro-3-methyl-*N*-(2-((*R*)-1-methylpyrrolidin-2-yl)-1*H*-pyrrolo[3,2-*c*]pyridin-6-yl)-1*H*-indazole-6-carboxamide (**Example 58**, 0.08 g, 23%) as a white solid. $^1$H NMR (400 MHz, DMSO-$d_6$) δ ppm 1.23-1.45 (m, 7H), 1.50-1.60 (m, 2H), 1.77-1.94 (m, 5H), 1.97-2.04 (m, 1H), 2.11-2.17 (m, 1H), 2.18 (s, 3H), 2.23-2.32 (m, 1H), 2.48 (s, 3H), 2.53-2.62 (m, 2H), 2.85-2.95 (m, 1H), 3.12-3.19 (m, 1H), 3.34-3.36 (m, 2H), 4.27-4.46 (m, 4H), 5.11-5.16 (m, 1H), 6.40 (s, 1H), 7.48-7.53 (m, 1H), 7.59-7.64 (m, 2H), 7.60-7.70 (m, 1H), 8.00 (d, J=5.50 Hz, 1H), 8.25 (s, 1H), 8.49 (s, 1H), 10.48 (s, 1H), 10.99 (br s, 1H), 11.40 (s, 1H). Mass spec m/z 756.9 [M+H]$^+$.

**Example 59 was synthesized following Scheme 57**

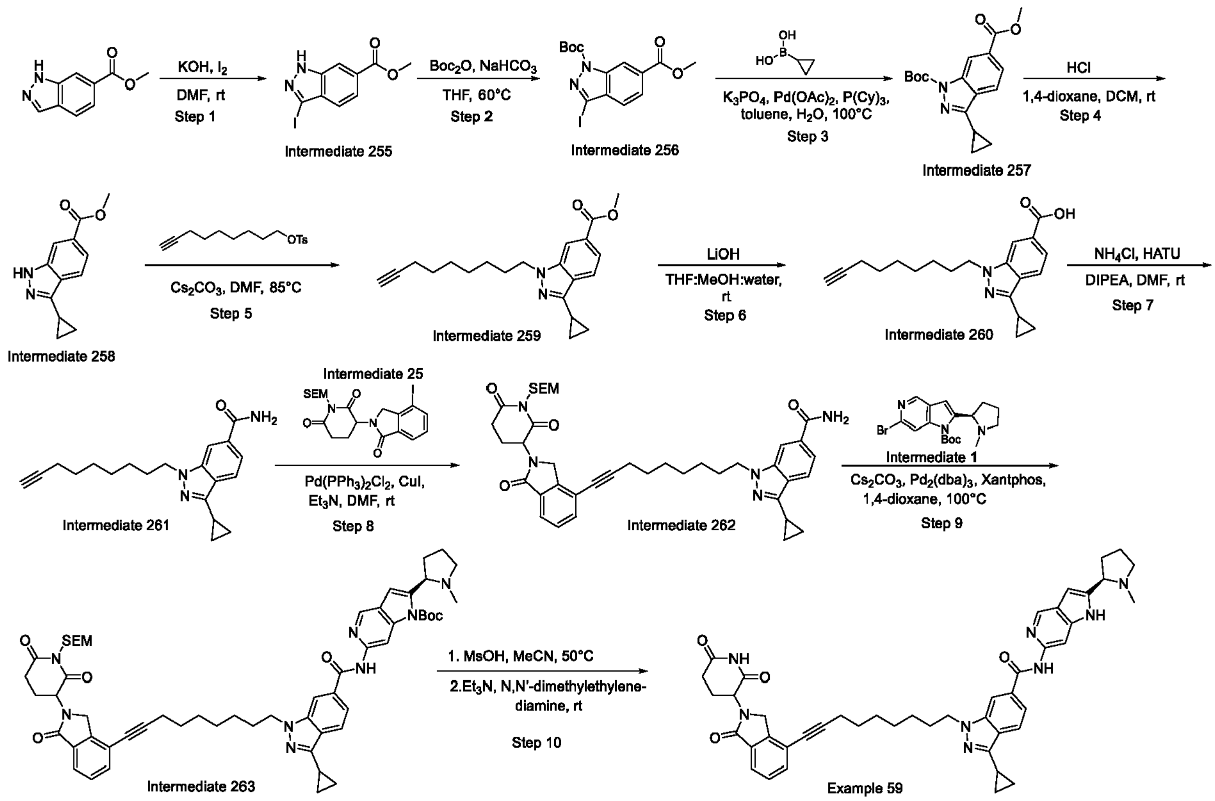


**Scheme 57**

**Step 1**

**Intermediate 255: methyl 3-iodo-1*H*-indazole-6-carboxylate**

To a solution of methyl 1*H*-indazole-6-carboxylate (CAS No: 170487-40-8, 25.0 g, 141.91 mmol) in dimethylformamide (200 mL) was added potassium hydroxide (31.86 g, 567.63 mmol) and iodine (39.62 g, 156.10 mmol) and the reaction mixture was stirred at room temperature for 16h. The reaction mixture was diluted with ethyl acetate (500 mL) and washed with ice-cold water (3 × 500 mL). The organic layer was separated, dried over anhydrous $Na_2SO_4$, filtered, and concentrated *in vacuo* to afford methyl 3-iodo-1*H*-indazole-6-carboxylate (**Intermediate 255**, 9.25 g, 22%) as an off-white solid, which was used for the next step without further purification. $^1$H NMR (400 MHz, DMSO-$d_6$) δ ppm 3.90 (s, 3H), 7.56 (d, *J*=8.33 Hz, 1H), 7.75 (d, *J*=8.33 Hz, 1H), 8.16 (s, 1H), 13.86 (br s, 1H). Mass spec m/z 302.97 [M+H]$^+$.

**Step 2**

**Intermediate 256: 1-(tert-butyl) 6-methyl 3-iodo-1*H*-indazole-1, 6-dicarboxylate**

To a solution of methyl 3-iodo-1*H*-indazole-6-carboxylate (**Intermediate 255**, 9.20 g, 30 mmol) in THF (90 mL) was added sodium bicarbonate (5.1 g, 61 mmol) followed by di-tert-butyl dicarbonate (11 g, 46 mmol) at room temperature. The reaction mixture was heated at 60°C for 2h. The reaction mixture was then concentrated *in vacuo.* The crude residue was quenched with water (100 mL) to get a solid precipitate which was filtered, washed with water (50 mL) and dried *in vacuo* to afford 1-(*tert*-butyl) 6-methyl 3-iodo-1*H*-indazole-1, 6-dicarboxylate (**Intermediate 256**, 18.0 g) as an off-white solid which was used for the next step without further purification. $^1$H NMR (400 MHz, DMSO-$d_6$) δ ppm 1.67 (s, 9H), 3.93 (s, 3H), 7.69 (d, *J*=8.33 Hz, 1H), 7.99 (d, *J*=8.77 Hz, 1H), 8.70 (s, 1H). Mass spec: m/z: 403.1 $[M+H]^+$.

**Step 3**

**Intermediate 257: 1-(*tert*-butyl) 6-methyl 3-cyclopropyl-1*H*-indazole-1,6-dicarboxylate**

To a solution of 1-(tert-butyl) 6-methyl 3-iodo-1*H*-indazole-1, 6-dicarboxylate (**Intermediate 256,** 15.0 g, 37.29 mmol) in toluene:water (1:1, 210 mL) was added potassium phosphate (32.3 g, 149.2 mmol). The reaction mixture was purged with argon gas for 15 min then cyclopropylboronic acid (CAS No: 411235-57-9, 6.54 g, 74.59 mmol), $Pd(OAc)_2$ (1.23 g, 7.46 mmol) and tricyclohexylphosphine (3.13 g, 11.19 mmol) were added. The reaction mixture was purged with argon gas for 10 min and heated at 100°C for 16h. The reaction mixture was then extracted with ethyl acetate (2 × 200 mL). The organic layer was separated, dried over anhydrous $Na_2SO_4$, filtered, and concentrated *in vacuo.* The crude material was purified by combi-flash column chromatography, by eluting with 12% ethyl acetate in heptane, to afford 1-(tert-butyl) 6-methyl 3-cyclopropyl-1*H*-indazole-1,6-dicarboxylate (**Intermediate 257,** 7.1 g, 60%) as yellow solid. $^1$H NMR (400 MHz, DMSO-$d_6$) δ ppm 1.01-1.12 (m, 4H), 1.62 (s, 9H), 2.35-2.43 (m, 1H), 3.90 (s, 3H), 7.89 (d, *J*=8.33 Hz, 1H), 8.03 (d, *J*=8.33 Hz, 1H), 8.65 (s, 1H). Mass spec: m/z 317.3 $[M+H]^+$.

**Step 4**

**Intermediate 258: methyl 3-cyclopropyl-1*H*-indazole-6-carboxylate**

To a solution of 1-(*tert*-butyl) 6-methyl 3-cyclopropyl-1*H*-indazole-1,6-dicarboxylate (**Intermediate 257,** 7.00 g, 22 mmol) in dichloromethane (30 mL) was added 4M HCl in 1,4-dioxane (70 mL) at room temperature and the reaction mixture was stirred for 16h. The reaction mixture was quenched with saturated $NaHCO_3$ solution (100 mL) and extracted with ethyl acetate (2 × 100 mL). The organic layer was separated, dried over anhydrous $Na_2SO_4$, filtered, and concentrated *in vacuo* to afford methyl 3-cyclopropyl-1*H*-indazole-6-carboxylate (**Intermediate 258**, 4.5 g, 94%,) as an orange solid, which was used for the next step without further purification. $^1$H NMR (400 MHz, DMSO-$d_6$) δ ppm 0.92-1.04 (m, 4H), 2.27-2.36 (m, 1H), 3.89 (s, 3H), 7.64 (dd, *J*=8.50, 1.38 Hz, 1H), 7.89 (d, *J*=8.38 Hz, 1H), 8.06 (s, 1H), 12.93 (s, 1H). Mass spec: m/z 217.15 $[M+H]^+$.

**Step 5**

**Intermediate 259: methyl 3-cyclopropyl-1-(non-8-yn-1-yl)-1H-indazole-6-carboxylate**

To a solution of methyl 3-cyclopropyl-1*H*-indazole-6-carboxylate (**Intermediate 258,** 3.5 g, 16 mmol) in dimethylformamide (35 mL) was added cesium carbonate (9.4 g, 49 mmol) followed by non-8-yn-1-yl 4-methylbenzenesulfonate (CAS No: 87462-64-4, 5.7 g, 19 mmol). The reaction mixture was heated at 85°C for 4h. The reaction mixture was quenched with ice cold water (50 mL) and extracted with ethyl acetate (2 × 50 mL). The organic layers were separated, dried over anhydrous $Na_2SO_4$, filtered, and concentrated *in vacuo* to obtain the crude compound as a mixture of regioisomers. The regioisomers were separated by combi-flash chromatography, by eluting with 6% ethyl acetate in heptane, to afford methyl 3-cyclopropyl-1-(non-8-yn-1-yl)-1*H*-indazole-6-carboxylate (**Intermediate 259,** 3.3 g, 60%) as yellow oil. $^1$H NMR (400 MHz, DMSO-$d_6$) δ ppm 0.90-1.05 (m, 4H), 1.14-1.45 (m, 8H), 1.70-1.82 (m, 2H), 2.06-2.14 (m, 2H), 2.25-2.35 (m, 1H), 2.65-2.75 (m, 1H), 3.90 (s, 3H), 4.32-4.40 (m, 2H), 7.60-7.69 (m, 1H), 7.82-7.92 (m, 1H), 8.21 (s, 1H). Mass spec: m/z: 339.3 $[M+H]^+$.

**Step 6**

**Intermediate 260: 3-cyclopropyl-1-(non-8-yn-1-yl)-1*H*-indazole-6-carboxylic acid**

To a solution of methyl 3-cyclopropyl-1-(non-8-yn-1-yl)-1*H*-indazole-6-carboxylate (**Intermediate 59,** 3.3 g, 9.8 mmol) in tetrahydrofuran:methanol:water (2:2:1, 50 mL) was added lithium hydroxide (0.94 g, 39 mmol) and the reaction mixture was stirred at room temperature for 16h. The reaction mixture was then concentrated *in vacuo,* water (50 mL) was added and the mixture acidified to pH ~4 with 1N HCl and the aqueous phase extracted with ethyl acetate (2 × 50 mL). The organic layer was separated, dried over $Na_2SO_4$, and concentrated *in vacuo* to afford 3-cyclopropyl-1-(non-8-yn-1-yl)-1*H*-indazole-6-carboxylic acid (**Intermediate 260,** 3.0 g) as a yellow solid, which was used for the next step without further purification. $^1$H NMR (401 MHz, DMSO-$d_6$) δ ppm 0.92-1.04 (m, 4H), 1.16-1.32 (m, 6H), 1.33-1.43 (m, 2H), 1.72-1.79 (m, 2H), 2.09-2.13 (m, 2H), 2.26-2.33 (m, 1H), 2.72 (t, *J*=2.63 Hz, 1H), 4.35 (t, *J*=6.91 Hz, 2H), 7.64 (d, *J*=8.19 Hz, 1H), 7.81 (d, *J*=8.31 Hz, 1H), 8.16 (s, 1H). Mass spec: m/z 325.25 $[M+H]^+$.

**Step 7**

**Intermediate 261: 3-cyclopropyl-1-(non-8-yn-1-yl)-1*H*-indazole-6-carboxamide**

To a solution of 3-cyclopropyl-1-(non-8-yn-1-yl)-1*H*-indazole-6-carboxylic acid (**Intermediate 260**, 3.00 g, 9.25 mmol) in dimethylformamide (30 mL) was added HATU (5.55 g, 13.87 mmol) and DIPEA (5.75 mL, 32.37 mmol) at room temperature and stirred for 10 min. Ammonium chloride (1.62 mL, 46.24 mmol) was then added at 0°C and the reaction mixture was stirred at room temperature for 16h. The reaction mixture was then diluted with ice cold water (100 mL). The solid precipitate obtained was filtered and dried under vacuum to afford 3-cyclopropyl-1-(non-8-yn-1-yl)-1*H*-indazole-6-carboxamide (**Intermediate 261,** 2.5 g, 84%) as a white solid, which was used for next step without further purification. $^1$H NMR (400 MHz, DMSO-$d_6$) δ ppm 0.91-1.04 (m, 4H), 1.17-1.43 (m, 7H), 1.72-1.82 (m, 2H), 2.07-2.15 (m, 2H), 2.23-2.35 (m, 2H), 2.66-2.73 (m, 1H), 4.30 (t, *J*=7.02 Hz, 2H), 7.41 (br s, 1H), 7.58 (d, *J*=8.33 Hz, 1H), 7.79 (d, *J*=8.33 Hz, 1H), 8.02 (br s, 1H), 8.10 (s, 1H). Mass spec: m/z 324.28 $[M+H]^+$.

**Step 8**

**Intermediate 262: 3-cyclopropyl-1-(9-(2-(2, 6-dioxo-1-((2-(trimethylsilyl) ethoxy) methyl) piperidin-3-yl)-1-oxoisoindolin-4-yl) non-8-yn-1-yl)-1*H*-indazole-6-carboxamide**

To a solution of 3-cyclopropyl-1-(non-8-yn-1-yl)-1*H*-indazole-6-carboxamide **(Intermediate 261, 0.70** g, 2.16 mmol) in dimethylformamide (3 mL) was added 3-(4-iodo-1-oxo-isoindolin-2-yl)-1-(2-trimethylsilylethoxymethyl)piperidine-2,6-dione **(Intermediate 25,** 1.19 g, 2.38 mmol) followed by triethylamine (11.2 mL, 80.09 mmol). The reaction mixture was purged with argon for 15 min followed by the addition of copper(I) iodide (0.082 g, 0.43 mmol) and $Pd(PPh_3)_2Cl_2$ (0.15 g, 0.21 mmol), further purged with argon for 10 min and stirred at room temperature for 2h. The reaction mixture was quenched with ice cold water (50 mL) and extracted with ethyl acetate (2 × 50 mL). The organic layer was separated, dried over anhydrous $Na_2SO_4$, and concentrated *in vacuo.* The crude material was purified by combi-flash column chromatography, by eluting with 3% MeOH in DCM, to afford 3-cyclopropyl-1-(9-(2-(2, 6-dioxo-1-((2-(trimethylsilyl) ethoxy) methyl) piperidin-3-yl)-1-oxoisoindolin-4-yl) non-8-yn-1-yl)-1*H*-indazole-6-carboxamide (**Intermediate 262,** 0.80 g, 53%) as an off white solid. $^1$H NMR (401 MHz, DMSO-$d_6$) δ ppm -0.04 (s, 9H), 0.78-0.87 (m, 2H), 0.89-1.02 (m, 4H), 1.19-1.27 (m, 2H), 1.29-1.41 (m, 4H), 1.48-1.56 (m, 2H), 1.72-1.83 (m, 2H), 1.99-2.08 (m, 1H), 2.23-2.30 (m, 1H), 2.39-2.47 (m, 4H), 2.75-2.80 (m, 1H), 3.00-3.12 (m, 1H), 3.47-3.55 (m, 2H), 4.26-4.33 (m, 2H), 4.43-4.47 (m, 1H), 4.99-5.09 (m, 2H), 5.25-5.30 (m, 1H), 7.44 (br s, 1H), 7.49-7.54 (m, 1H), 7.56-7.64 (m, 2H), 7.71 (d, *J*=6.97 Hz, 1H), 7.78 (d, *J*=8.44 Hz, 1H), 8.04 (br s, 1H), 8.10 (s, 1H). Mass spec: m/z: 696.47 $[M+H]^+$.

**Step 9**

**Intermediate 263: *tert*-butyl 6-(3-cyclopropyl-1-(9-(2-(2,6-dioxo-1-((2-(trimethylsilyl) ethoxy)methyl)piperidin-3-yl)-1-oxoisoindolin-4-yl)non-8-yn-1-yl)-1*H*-indazole-6-carboxamido)-2-((*R*)-1-methylpyrrolidin-2-yl)-1*H*-pyrrolo[3,2-c]pyridine-1-carboxylate**

To a solution of 3-cyclopropyl-1-(9-(2-(2,6-dioxo-1-((2-(trimethylsilyl)ethoxy)methyl)piperidin-3-yl)-1-oxoisoindolin-4-yl non-8-yn-1-yl)-1*H*-indazole-6-carboxamide (**Intermediate 262,** 0.8 g, 1.15 mmol) in 1,4-dioxane (15 mL) was added *tert*-butyl (*R*)-6-bromo-2-(1-methylpyrrolidin-2-yl)-1*H*-pyrrolo[3,2-c]pyridine-1-carboxylate (**Intermediate 1,** 0.48 g, 1.26 mmol) followed by cesium carbonate (1.12 g, 3.45 mmol) at room temperature. The reaction mixture was purged with argon for 15 min followed by $Pd_2(dba)_3$ (0.16 g, 0.17 mmol) and Xantphos (0.19 g, 0.34 mmol). The reaction mixture was purged with argon for another 10 min and heated at 100°C for 2h. The reaction mixture was then concentrated *in vacuo*

to obtain crude compound. The crude material was purified by combi-flash column chromatography, by eluting with 3% MeOH in DCM, to afford *tert*-butyl 6-(3-cyclopropyl-1-(9-(2-(2,6-di oxo-1-((2-(trimethylsilyl)ethoxy)methyl)piperidin-3-yl)-1-oxoisoindolin-4-yl)non-8-yn-1-yl)-1*H*-indazole-6-carboxamido)-2-((R)-1-methylpyrrolidin-2-yl)-1*H*-pyrrolo[3,2-c] pyridine-1-carboxylate (**Intermediate 263**, 0.6 g, 52%) as yellow solid. $^1$H NMR (400 MHz, DMSO-$d_6$) δ ppm -0.05 (s, 9H), 0.73-0.88 (m, 2H), 0.89-1.06 (m, 4H), 1.16-1.44 (m, 6H), 1.48-1.57 (m, 2H), 1.58-1.66 (m, 2H), 1.70 (s, 9H), 1.73-1.88 (m, 3H), 1.98-2.08 (m, 1H), 2.25-2.33 (m, 1H), 2.36 (s, 3H), 2.40-2.46 (m, 3H), 2.54-2.56 (m, 2H), 2.74-2.80 (m, 2H), 2.99-3.19 (m, 2H), 3.48-3.54 (m, 2H), 3.86-3.97 (m, 1H), 4.22-4.26 (m, 1H), 4.33-4.47 (m, 2H), 4.96-5.10 (m, 2H), 5.24-5.28 (m, 1H), 6.76 (s, 1H), 7.50 (t, *J*=7.67 Hz, 1H), 7.60 (d, *J*=7.46 Hz, 1H), 7.65-7.74 (m, 2H), 7.84 (d, *J*=8.29 Hz, 1H), 8.41 (s, 1H), 8.60 (s, 1H), 8.97 (s, 1H), 10.73 (s, 1H). Mass spec: m/z: 995.61 [M+H]$^+$.

**Step 10**

**Example 59: 3-cyclopropyl-1-(9-(2-(2, 6-dioxopiperidin-3-yl)-1-oxoisoindolin-4-yl) non-8-yn-1-yl)-*N*-(2-((*R*)-1-methylpyrrolidin-2-yl)-1*H*-pyrrolo [3, 2-c] pyridin-6-yl)-1*H*-indazole-6-carboxamide**

To a solution of tert-butyl 6-(3-cyclopropyl-1-(9-(2-(2,6-dioxo-1-((2-(trimethylsilyl)ethoxy)methyl)piperidin-3-yl)-1-oxoisoindolin-4-yl)non-8-yn-1-yl)-1*H*-indazole-6-carboxamido)-2-((*R*)-1-methylpyrrolidin-2-yl)-1*H*-pyrrolo[3,2-c] pyridine-1-carboxylate (**Intermediate 263**, 0.55 g, 0.55 mmol) in acetonitrile (6 mL) was added methanesulfonic acid (0.36 mL, 5.52 mmol) at room temperature and the reaction mixture was heated at 50°C for 2h. *N,N'*-dimethylethylenediamine (0.33 mL, 2.76 mmol) was then added, followed by triethylamine (1.55 mL, 11.05 mmol) and the reaction stirred at room temperature for 4h. The reaction mixture was then poured into ice cold water (50 mL) to get the solid precipitate which was filtered and dried under vacuum to obtain crude compound. The crude material was then purified by preparative HPLC (Method A) to afford 3-cyclopropyl-1-(9-(2-(2,6-dioxopiperidin-3-yl)-1-oxoisoindolin-4-yl)non-8-yn-1-yl)-*N*-(2-((*R*)-1-methylpyrrolidin-2-yl)-1*H*-pyrrolo[3,2-c] pyridin-6-yl)-1*H*-indazole-6-carboxamide (**Example 59**, 0.14 g, 34%) as an off white solid. $^1$H NMR (400 MHz, DMSO-$d_6$) δ ppm 0.91-1.05 (m, 4H), 1.23-1.44 (m, 7H), 1.49-1.58 (m, 2H), 1.77-2.02 (m, 6H), 2.12-2.15 (m, 1H), 2.17 (s, 3H), 2.23-2.31 (m, 2H), 2.41-2.46 (m, 2H), 2.56-2.63 (m, 2H), 2.84-2.95 (m, 1H), 3.11-3.18 (m, 1H), 4.26-4.47 (m, 4H), 5.10-5.16 (m, 1H), 6.40 (s, 1H), 7.49 (t, *J*=7.57 Hz, 1H), 7.59 (d, *J*=6.88 Hz, 1H), 7.67-7.75 (m, 2H), 7.84 (d, *J*=8.50 Hz, 1H), 8.25 (s, 1H), 8.41 (s, 1H), 8.51 (s, 1H), 10.51 (s, 1H), 10.97 (br s, 1H), 11.38 (s, 1H). Mass spec: m/z: 765.2 [M+H]$^+$.

**Example 60 was synthesized following Scheme 58**

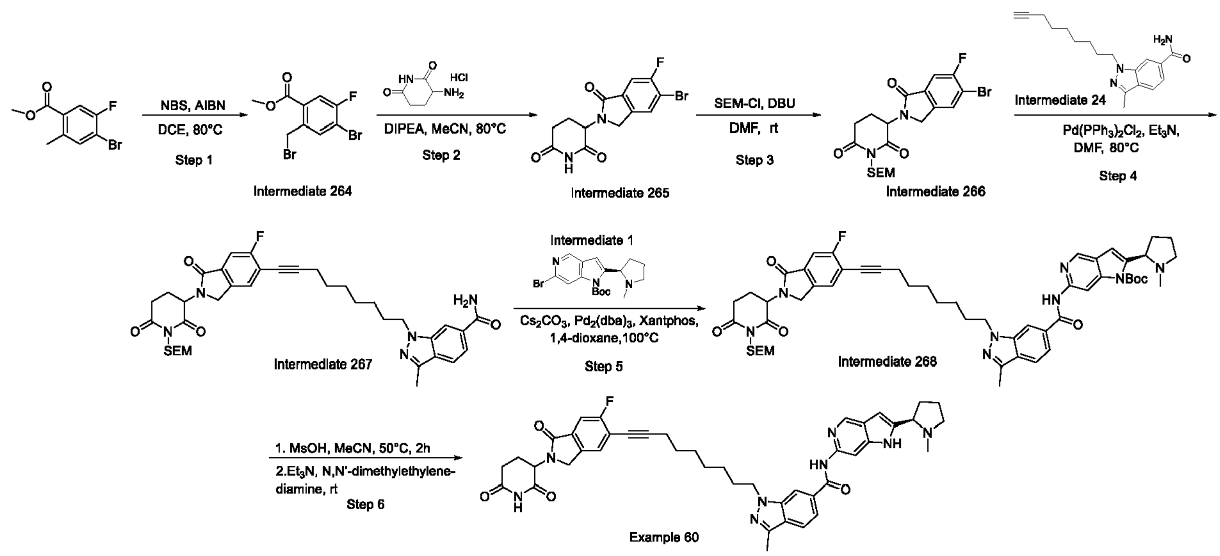


**Scheme 58**

**Step 1**

**Intermediate 264: methyl 4-bromo-2-(bromomethyl)-5-fluorobenzoate**

To a solution of methyl 4-bromo-5-fluoro-2-methylbenzoate (CAS No: 1352889-89-4, 5.00 g, 20 mmol) in dichloroethane (50 mL) was added *N*-bromosuccinimide (7.2 g, 40 mmol) followed by AIBN (0.66 g, 4.0 mmol). The reaction mixture was stirred at 80°C for 12h. The reaction mixture was poured into water (60 mL) and extracted with ethyl acetate (3 × 100 mL). The organic layer was separated, dried over $Na_2SO_4$, and concentrated *in vacuo.* The crude material was purified by combi-flash column chromatography, by eluting with 10% ethyl acetate in heptane, to afford methyl 4-bromo-2-(bromomethyl)-5-fluorobenzoate (**Intermediate 264**, 3.8 g, 58%) as an off white solid. $^1H$ NMR (400 MHz, DMSO-$d_6$) δ ppm 3.88 (s, 3H), 4.97 (s, 2H), 7.78 (d, *J*=9.21 Hz, 1H), 8.05 (d, *J*=7.02 Hz, 1H).

**Step 2**

**Intermediate 265: 3-(5-bromo-6-fluoro-1-oxoisoindolin-2-yl)piperidine-2,6-dione**

To a solution of methyl 4-bromo-2-(bromomethyl)-5-fluorobenzoate **(Intermediate 264,** 3.5 g, 11.0 mmol) in acetonitrile (40 mL) was added 3-aminopiperidine-2, 6-dione hydrochloride (CAS No: 24666-56-6, 2.1 g, 13 mmol) followed by DIPEA (4.2 g, 32 mmol). The reaction mixture was stirred at 80°C for 12h. The reaction mixture was poured into cold water (50 mL), the resultant precipitate collected by filtration and dried *in vacuo* to afford 3-(5-bromo-6-fluoro-1-oxoisoindolin-2-yl) piperidine-2, 6-dione (**Intermediate 265,** 3.00 g, 82%) as a blue solid, which was used for the next step without further purification. $^1H$ NMR (400 MHz, DMSO-$d_6$) δ ppm 1.97-2.06 (m, 1H), 2.33-2.43 (m, 1H), 2.56-2.62 (m, 1H), 2.84-2.98 (m, 1H), 4.29-4.50 (m, 2H), 5.09-5.14 (m, 1H), 7.70 (d, *J*=7.45 Hz, 1H), 8.04 (d, *J*=5.70 Hz, 1H), 11.00 (br s, 1H). Mass spec m/z 343.0 $[M+2H]^+$.

**Step 3**

**Intermediate 266: 3-(5-bromo-6-fluoro-1-oxoisoindolin-2-yl)-1-((2-(trimethylsilyl)ethoxy)methyl) piperidine-2, 6-dione**

To a solution of 3-(5-bromo-6-fluoro-1-oxoisoindolin-2-yl) piperidine-2,6-dione **(Intermediate 265,** 2.7 g, 7.9 mmol) in dimethylformamide (30 mL) was added DBU (4.3 g, 28 mmol) at 0°C followed by 2-(trimethylsilyl)ethoxymethyl chloride (5.1 mL, 28 mmol). The reaction mixture was stirred at room temperature for 16h under nitrogen atmosphere. The reaction mixture was quenched with water (100 mL), diluted with ethyl acetate (200 mL) and filtered through celite bed. The organic layer was separated, washed with water (150 mL), dried over anhydrous $Na_2SO_4$, filtered, and concentrated *in vacuo.* The crude material was purified by combi-flash chromatography, by eluting with 50% ethyl acetate in heptane, to afford 3-(5-bromo-6-fluoro-1-oxoisoindolin-2-yl)-1-((2-(trimethylsilyl)ethoxy)methyl) piperidine-2, 6-dione (**Intermediate 266,** 1.7 g, 46%) as brown solid. $^1H$ NMR (400 MHz, DMSO-$d_6$) δ ppm 0.02 (s, 9H), 1.81-1.86 (m, 2H), 2.04-2.10 (m, 1H), 2.36-2.42 (m, 1H), 2.76-2.81 (m, 1H), 3.01-3.08 (m, 1H), 3.49-3.55 (m, 2H), 4.29-4.33 (m, 1H), 4.46-4.50 (m, 1H), 5.01-5.08 (m, 2H), 5.20-5.24 (m, 1H), 7.71 (d, *J*=7.45 Hz, 1H), 8.05 (d, *J*=5.70 Hz, 1H). Mass spec m/z 469.0 $[M-H]^+$.

**Step 4**

**Intermediate 267: 1-(9-(2-(2,6-dioxo-1-((2-(trimethylsilyl)ethoxy)methyl)piperidin-3-yl)-6-fluoro-1-oxoisoindolin-5-yl) non-8-yn-1-yl)-3-methyl-1*H*-indazole-6-carboxamide**

To a solution of 3-(5-bromo-6-fluoro-1-oxoisoindolin-2-yl)-1-((2-(trimethylsilyl)ethoxy)methyl)piperidine-2, 6-dione (**Intermediate 266**, 0.50 g, 1.06 mmol) in dimethylformamide (5 mL) was added 3-methyl-1-(non-8-yn-1-yl)-1*H*-indazole-6-carboxamide (**Intermediate 24,** 1.57 g, 5.30 mmol) followed by triethylamine (0.44 mL, 3.18 mmol) at room temperature. The reaction mixture was purged with argon for 15 min followed by the addition of $PdCl_2(PPh_3)_2$ (0.076 g, 0.10 mmol). The reaction mixture was further purged with argon for 10 min and stirred at 80°C for 16h. The reaction mixture was poured into water (30 mL) and extracted with ethyl acetate (3 × 50 mL). The organic layer was separated, dried over $Na_2SO_4$, and concentrated *in vacuo.* The crude material was purified by combi-flash chromatography, by eluting with 100% ethyl acetate in heptane, to afford 1-(9-(2-(2, 6-dioxo-1-((2-(trimethylsilyl) ethoxy) methyl) piperidin-3-yl)-6-fluoro-1-oxoisoindolin-5-yl) non-8-yn-1-yl)-3-methyl-1*H*-indazole-6-carboxamide (**Intermediate 267**, 0.50 g, 69%) as an off white solid. $^1H$ NMR (400 MHz, DMSO-$d_6$) δ ppm 0.00 (s, 9H), 0.83-0.90 (m, 2H), 1.24-1.47 (m, 6H), 1.52-1.60 (m, 2H), 1.82-1.90 (m, 2H), 2.04-2.12 (m, 1H), 2.34-2.44 (m, 1H), 2.45-2.48 (m, 2H), 2.51 (s, 3H), 2.77-2.86 (m, 1H), 3.03-3.14 (m, 1H), 3.50-3.58 (m, 2H), 4.25-4.40 (m, 3H), 4.42-4.50 (m, 1H), 5.03-5.11 (m, 2H), 5.24-5.28 (m, 1H), 7.45 (br s, 1H), 7.58-7.64 (m, 2H), 7.72-7.77 (m, 2H), 8.07 (br s, 1H), 8.15 (s, 1H). Mass spec m/z 688.56 $[M+H]^+$.

**Step 5**

**Intermediate 268: *tert*-butyl 6-(1-(9-(2-(2,6-dioxo-1-((2-(trimethylsilyl)ethoxy)methyl)piperidin-3-yl)-6-fluoro-1-oxoisoindolin-5-yl)non-8-yn-1-yl)-3-methyl-1*H*-indazole-6-carboxamido)-2-((*R*)-1-methylpyrrolidin-2-yl)-1*H*-pyrrolo[3,2-*c*]pyridine-1-carboxylate**

To a solution of 1-(9-(2-(2, 6-dioxo-1-((2-(trimethylsilyl) ethoxy) methyl) piperidin-3-yl)-6-fluoro-1-oxoisoindolin-5-yl) non-8-yn-1-yl)-3-methyl-1*H*-indazole-6-carboxamide (**Intermediate 267**, 0.50 g, 0.73 mmol) in 1,4-dioxane (15 mL) was added tert-butyl (*R*)-6-bromo-2-(1-methylpyrrolidin-2-yl)-1*H*-pyrrolo[3,2-c]pyridine-1-carboxylate (**Intermediate 1**, 0.35 g, 0.92 mmol) followed by cesium carbonate (0.60 g, 1.84 mmol) at room temperature. The reaction mixture was purged with argon for 15 min followed by addition of $Pd_2(dba)_3$ (0.13 g, 0.14 mmol) and Xantphos (0.16 g, 0.27 mmol). The reaction mixture was purged with argon for another 10 min and stirred at 100°C for 2h. The reaction mixture was concentrated *in vacuo* to obtain the crude compound. The crude material was purified by combi-flash column chromatography, by eluting with 80% ethyl acetate in heptane, to afford *tert*-butyl 6-(1-(9-(2-(2,6-dioxo-1-((2-(trimethylsilyl)ethoxy)methyl)piperidin-3-yl)-6-fluoro-1-oxoisoindolin-5-yl)non-8-yn-1-yl)-3-methyl-1*H*-indazole-6-carboxamido)-2-((*R*)-1-methylpyrrolidin-2-yl)-1*H*-pyrrolo[3,2-*c*]pyridine-1-carboxylate (**Intermediate 268**, 0.30 g, 33%) as a yellow solid. $^1$H NMR (400 MHz, DMSO-$d_6$) δ ppm -0.03 (s, 9H), 0.79-0.88 (m, 2H), 1.21-1.47 (m, 6H), 1.49-1.66 (m, 4H), 1.70 (s, 9H), 1.74-1.78 (m, 2H), 1.83-1.93 (m, 2H), 2.01-2.09 (m, 1H), 2.28-2.33 (m, 3H), 2.36 (s, 3H), 2.52 (s, 3H), 2.64-2.68 (m, 1H), 2.74-2.82 (m, 1H), 2.99-3.17 (m, 2H), 3.48-3.56 (m, 2H), 3.90-3.96 (m, 1H), 4.22-4.27 (m, 1H), 4.35-4.46 (m, 3H), 4.98-5.09 (m, 2H), 5.20-5.26 (m, 1H), 6.76 (s, 1H), 7.55 (d, *J*=8.40 Hz, 1H), 7.66-7.74 (m, 2H), 7.78 (d, *J*=8.40 Hz, 1H), 8.43 (s, 1H), 8.61 (s, 1H), 8.97 (s, 1H), 10.75 (br s, 1H). Mass spec m/z 986.6 $[M+H]^+$.

**Step 6**

**Example 60: 1-(9-(2-(2,6-dioxopiperidin-3-yl)-6-fluoro-1-oxoisoindolin-5-yl)non-8-yn-1-yl)-3-methyl-*N*-(2-((R)-1-methylpyrrolidin-2-yl)-1*H*-pyrrolo[3,2-c]pyridin-6-yl)-1*H*-indazole-6-carboxamide**

To a solution of *tert*-butyl 6-(1-(9-(2-(2,6-dioxo-1-((2-(trimethylsilyl)ethoxy)methyl)piperidin-3-yl)-6-fluoro-1-oxoisoindolin-5-yl)non-8-yn-1-yl)-3-methyl-1*H*-indazole-6-carboxamido)-2-((*R*)-1-methylpyrrolidin-2-yl)-1*H*-pyrrolo[3,2-c] pyridine-1-carboxylate (**Intermediate 268**, 0.30 g, 0.30 mmol) in acetonitrile (10 mL) was added methanesulfonic acid (0.20 mL, 3.04 mmol) at room temperature and the reaction mixture was stirred at 50°C for 2h. After cooling to room temperature, *N*, *N*'-dimethylethylenediamine (0.18 mL, 1.52 mmol) and triethylamine (0.85 mL, 6.07 mmol) were added and the reaction stirred at room temperature for 3h. The reaction mixture was poured into ice cold water (20 mL) and the resultant precipitate collected by filtration and dried *in vacuo.* The crude compound was purified by preparative HPLC (Method A) to afford 1-(9-(2-(2,6-dioxopiperidin-3-yl)-6-fluoro-1-oxoisoindolin-5-yl)non-8-yn-1-yl)-3-methyl-*N*-(2-((*R*)-1-methylpyrrolidin-2-yl)-1*H*-pyrrolo[3,2-c]pyridin-6-yl)-1*H*-indazole-6-carboxamide (**Example 60**, 0.13 g, 56%) as an off white solid. $^1$H NMR (400 MHz, DMSO-$d_6$) δ ppm 1.31-1.40 (m, 6H), 1.52-1.56 (m, 2H), 1.84-2.01 (m, 6H), 2.14-2.16 (m, 1H), 2.17 (s, 3H), 2.25-2.28 (m, 1H), 2.31-2.33 (m, 1H), 2.46 (s, 3H), 2.56-2.63 (m, 2H), 2.85-2.94 (m, 1H), 3.09-3.16 (m, 1H), 3.31-3.35 (m, 2H), 4.31-4.43 (m, 4H), 5.08-5.12 (m, 1H), 6.40 (s, 1H), 7.55 (d, J=8.00 Hz, 1H), 7.67-7.79 (m, 3H), 8.25 (s, 1H), 8.44 (s, 1H), 8.52 (s, 1H), 10.52 (s, 1H), 10.99 (s, 1H), 11.38 (s, 1H). Mass spec m/z 757.1 $[M+H]^+$.

**Example 61 was synthesized following Scheme 59**

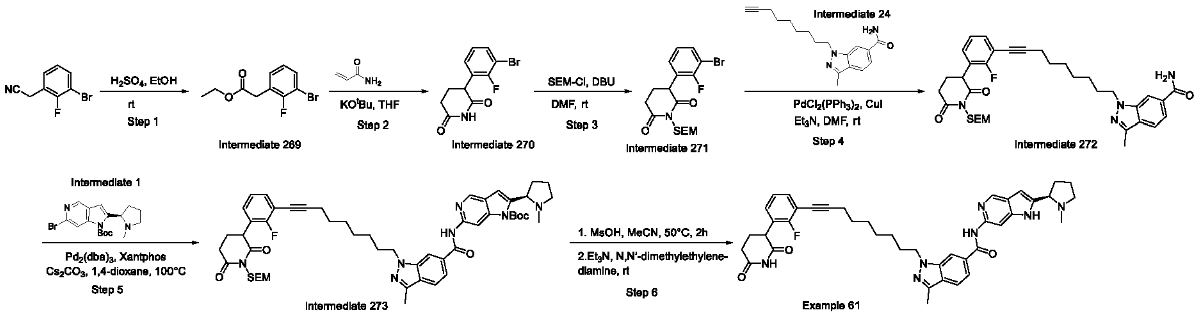


**Scheme 59**

**Step 1**

**Intermediate 269: ethyl 2-(3-bromo-2-fluorophenyl)acetate**

To a solution of 2-(3-bromo-2-fluorophenyl) acetonitrile (CAS No: 874285-03-7, 15.0 g, 70.08 mmol) in ethanol (150 mL) was added sulfuric acid (25 mL) and the reaction mixture was stirred at room temperature for 3h. The reaction mixture was concentrated *in vacuo.* The obtained residue was poured into water (1500 mL), adjusted to pH~4 by addition of 1M aqueous HCl and extracted with dichloromethane (3 × 500 mL). The organic layer was separated, dried over $Na_2SO_4$, filtered and concentrated *in vacuo* to afford ethyl 2-(3-bromo-2-fluorophenyl) acetate (**Intermediate 269**, 14.0 g, 73%) as colourless oil, which was used for the next step without further purification. $^1$H NMR (400 MHz, DMSO-$d_6$) δ ppm 1.19 (t, *J*=7.02 Hz, 3H), 3.79 (s, 2H), 4.08-4.14 (m, 2H), 7.10-7.14 (m, 1H), 7.35-7.38 (m, 1H), 7.60-7.63 (m, 1H). Mass spec m/z 260.1 [M+H]$^+$.

**Step 2**

**Intermediate 270: 3-(3-bromo-2-fluorophenyl) piperidine-2, 6-dione**

To a solution of ethyl 2-(3-bromo-2-fluorophenyl) acetate (**Intermediate 269**, 5.00 g, 19 mmol) in tetrahydrofuran (50 mL) was added acrylamide (CAS No: 79-06-1, 1.00 g, 19 mmol) followed by KO$^t$Bu (2.1 mL, 23 mmol) at 0°C. The reaction mixture was stirred at room temperature for 12h. The reaction mixture was diluted with water (100 mL) and extracted with ethyl acetate (3 × 100 mL). The organic layer was separated, dried over $Na_2SO_4$, filtered and concentrated *in vacuo.* The crude material was purified by combi-flash chromatography, by eluting with 100% ethyl acetate in heptane, to afford 3-(3-bromo-2-fluorophenyl) piperidine-2, 6-dione (**Intermediate 270**, 2.00 g, 37%) as an off white solid. $^1$H NMR (401 MHz, DMSO-$d_6$) δ ppm 1.98-2.08 (m, 1H), 2.19-2.31 (m, 1H), 2.55-2.60 (m, 1H), 2.68-2.82 (m, 1H), 4.12-4.17 (m, 1H), 7.14-7.18 (m, 1H), 7.33-7.40 (m, 1H), 7.62-7.69 (m, 1H), 10.96 (br s, 1H). Mass spec m/z 287.79 [M+2H]$^+$.

**Step 3**

**Intermediate 271: 3-(3-bromo-2-fluorophenyl)-1-((2-(trimethylsilyl) ethoxy) methyl) piperidine-2, 6-dione**

To a cooled (0°C) solution of 3-(3-bromo-2-fluorophenyl) piperidine-2, 6-dione (**Intermediate 270,** 2.00 g, 7.0 mmol) in dimethylformamide (20 mL) was added DBU (3.7 g, 24 mmol) followed by 2-(trimethylsilyl)ethoxymethyl chloride (4.1 g, 24 mmol). The reaction mixture was stirred at room temperature for 16h. The reaction mixture was diluted with water (100 mL) and extracted with ethyl acetate (3 × 100 mL). The organic layer was separated, dried over anhydrous $Na_2SO_4$, filtered and concentrated *in vacuo.*

The crude material was purified by combi-flash chromatography, by eluting with 30% ethyl acetate in heptane, to afford 3-(3-bromo-2-fluorophenyl)-1-((2-(trimethylsilyl) ethoxy) methyl) piperidine-2, 6-dione (**Intermediate 271**, 2.6 g, 89%) as an off white solid. $^1$H NMR (400 MHz, DMSO-$d_6$) δ ppm -0.02 (s, 9H), 0.79-0.89 (m, 2H), 1.99-2.08 (m, 1H), 2.18-2.30 (m, 1H), 2.70-2.79 (m, 1H), 2.85-2.97 (m, 1H), 3.48-3.56 (m, 2H), 4.26-4.30 (m, 1H), 5.09 (s, 2H), 7.14-7.18 (m, 1H), 7.33-7.37 (m, 1H), 7.63-7.67 (m, 1H).

**Step 4**

**Intermediate 272: 1-(9-(3-(2, 6-dioxo-1-((2-(trimethylsilyl)ethoxy)methyl) piperidin-3-yl)-2-fluorophenyl) non-8-yn-1-yl)-3-methyl-1*H*-indazole-6-carboxamide**

To a solution of 3-(3-bromo-2-fluorophenyl)-1-((2-(trimethylsilyl) ethoxy) methyl) piperidine-2,6-dione (**Intermediate 271**, 0.70 g, 2 mmol) in dimethylformamide (5.0 mL) was added 3-methyl-1-(non-8-yn-1-yl)-1*H*-indazole-6-carboxamide (**Intermediate 24,** 0.50 g, 2.00 mmol) followed by triethylamine (0.7 mL, 5 mmol) at room temperature. The reaction mixture was purged with argon for 15 min then copper (I) iodide (0.03 g, 0.2 mmol) and $PdCl_2(PPh_3)_2$ (0.1 g, 0.2 mmol) were added. The reaction mixture was purged with argon for 10 min and stirred at room temperature for 3h. The reaction mixture was diluted with water (100 mL) and extracted with ethyl acetate (3 × 100 mL). The organic layer was separated, dried over $Na_2SO_4$, and concentrated *in vacuo.* The crude material was purified by combi-flash column chromatography, by eluting with 80% ethyl acetate in heptane, to afford 1-(9-(3-(2, 6-dioxo-1-((2-(trimethylsilyl) ethoxy) methyl) piperidin-3-yl)-2-fluorophenyl) non-8-yn-1-yl)-3-methyl-1*H*-indazole-6-carboxamide (**Intermediate 272**, 0.70 g, 70%) as a yellow solid. $^1$H NMR (400 MHz, DMSO-$d_6$) δ ppm -0.03 (s, 9H), 0.80-0.87 (m, 2H), 1.19-1.43 (m, 6H), 1.45-1.56 (m, 2H), 1.77-1.88 (m, 2H), 1.96-2.06 (m, 1H), 2.15-2.25 (m, 1H), 2.41-2.45 (m, 2H), 2.49 (s, 3H), 2.70-2.77 (m, 1H), 2.85-2.92 (m, 1H), 3.48-3.57 (m, 2H), 4.18-4.22 (m, 1H), 4.31-4.35 (m, 2H), 5.09 (s, 2H), 7.12-7.18 (m, 1H), 7.26-7.31 (m,

1H), 7.34-7.44 (m, 2H), 7.59 (d, *J*=8.40 Hz, 1H), 7.72 (d, *J*=8.40 Hz, 1H), 8.02 (br s, 1H), 8.12 (s, 1H). Mass spec m/z 633.40 $[M+H]^+$.

**Step 5**

**Intermediate 273: *tert*-butyl 6-(1-(9-(3-(2,6-dioxo-1-((2-(trimethylsilyl)ethoxy)methyl)piperidin-3-yl)-2-fluorophenyl)non-8-yn-1-yl)-3-methyl-1*H*-indazole-6-carboxamido)-2-((*R*)-1-methylpyrrolidin-2-yl)-1*H* pyrrolo[3,2-*c*]pyridine-1-carboxylate**

To a solution of 1-(9-(3-(2, 6-dioxo-1-((2-(trimethylsilyl) ethoxy) methyl) piperidin-3-yl)-2-fluorophenyl) non-8-yn-1-yl)-3-methyl- 1*H*-indazole-6-carboxamide (**Intermediate 272**, 0.40 g, 0.63 mmol) in 1,4-dioxane (10 mL) was added tert-butyl (*R*)-6-bromo-2-(1-methylpyrrolidin-2-yl)-1*H*-pyrrolo[3,2-*c*]pyridine-1-carboxylate (**Intermediate 1,** 0.19 g, 0.50 mmol) followed by cesium carbonate (0.62 g, 1.89 mmol) at room temperature. The reaction mixture was purged with argon for 15 min then $Pd_2(dba)_3$ (0.059 g, 0.063 mmol) and Xantphos (0.11 g, 0.18 mmol) were added. The reaction mixture was purged with argon for 10 min and stirred at 100°C for 2h. The reaction mixture was concentrated *in vacuo* to obtain crude compound. The crude material was purified by combi-flash column chromatography, by eluting with 90% ethyl acetate in heptane, to afford *tert*-butyl 6-(1-(9-(3-(2,6-dioxo-1-((2-(trimethylsilyl)ethoxy)methyl)piperidin-3-yl)-2-fluorophenyl)non-8-yn-1-yl)-3-methyl-1*H*-indazole-6-carboxamido)-2-((*R*)-1-methylpyrrolidin-2-yl)-1*H*-pyrrolo[3,2-c] pyridine-1-carboxylate (**Intermediate 273,** 0.56 g, 95%) as a white solid. $^1H$ NMR (400 MHz, DMSO-$d_6$) δ ppm -0.05 (s, 9H), 0.78-0.87 (m, 2H), 1.23-1.44 (m, 6H), 1.51-1.66 (m, 2H), 1.68-1.70 (m, 2H), 1.71 (s, 9H), 1.74-1.78 (m, 2H), 1.82-1.92 (m, 2H), 2.01-2.09 (m, 1H), 2.28-2.33 (m, 2H), 2.36 (s, 3H), 2.40-2.44 (m, 2H), 2.50 (s, 3H), 2.65-2.77 (m, 1H), 2.82-2.95 (m, 1H), 3.12-3.16 (m, 1H), 3.45-3.57 (m, 2H), 3.90-4.06 (m, 1H), 4.16-4.21 (m, 1H), 4.40 (t, *J*=6.58 Hz, 2H), 5.08 (s, 2H), 6.76 (s, 1H), 7.09-7.16 (m, 1H), 7.27 (t, *J*=6.80 Hz, 1H), 7.36 (t, *J*=6.80 Hz, 1H), 7.70-7.74 (m, 1H), 7.76-7.84 (m, 1H), 8.43 (s, 1H), 8.61 (s, 1H), 8.98 (s, 1H), 10.76 (s, 1H). Mass spec m/z 932.73 $[M+H]^+$.

**Step 6**

**Example 61: 1-(9-(3-(2, 6-dioxopiperidin-3-yl)-2-fluorophenyl) non-8-yn-1-yl)-3-methyl-*N*-(2-((*R*)-1-methylpyrrolidin-2-yl)-1*H*-pyrrolo [3, 2-*c*] pyridin-6-yl)-1*H*-indazole-6-carboxamide**

To a solution of *tert*-butyl 6-(1-(9-(3-(2,6-dioxo-1-((2-(trimethylsilyl)ethoxy)methyl)piperidin-3-yl)-2-fluorophenyl)non-8-yn-1-yl)-3-methyl-1*H*-indazole-6-carboxamido)-2-((*R*)-1-methylpyrrolidin-2-yl)-1*H*-pyrrolo[3,2-c]pyridine-1-carboxylate (**Intermediate 273,** 0.40 g, 0.43 mmol) in acetonitrile (10 mL) was added methanesulfonic acid (0.42 mL, 6.43 mmol) at room temperature and the reaction mixture was stirred at 50°C for 2h. To the reaction mixture was added *N*, *N*'-dimethylethylenediamine (0.31 mL, 2.57 mmol) followed by triethylamine (0.87 g, 8.58 mmol) and the reaction stirred at room temperature for 3h. The reaction mixture was poured into ice cold water (100 mL), the resultant precipitate collected by filtration and dried *in vacuo* to afford the crude compound. The crude material was purified by preparative HPLC (Method A) to afford 1-(9-(3-(2, 6-dioxopiperidin-3-yl)-2-fluorophenyl) non-8-yn-1-yl)-3-methyl-*N*-(2-((2-((*R*)-1-methylpyrrolidin-2-yl)-1H-pyrrolo [3, 2-*c*] pyridin-6-yl)-1*H*-indazole-6-carboxamide (**Example 61,** 0.11 g, 37%) as an off white solid. $^1H$ NMR (400 MHz, DMSO-$d_6$) δ ppm 1.31-1.40 (m, 6H), 1.52-1.56 (m, 2H), 1.84-2.01 (m, 6H), 2.17 (s, 3H), 2.22-2.43 (m, 3H), 2.45-2.48 (m, 2H), 2.51 (s, 3H), 2.56-2.60 (m, 1H), 2.66-2.74 (m, 1H), 3.12-3.16 (m, 1H), 3.32-3.34 (m, 1H), 4.03-4.07 (m, 1H), 4.38-4.42 (m, 2H), 6.40 (s, 1H), 7.09-7.14 (m, 1H), 7.25-7.28 (m, 1H), 7.32-7.36 (m, 1H), 7.72-7.79 (m, 2H), 8.25 (s, 1H), 8.44 (s, 1H), 8.52 (s, 1H), 10.52 (s, 1H), 10.89 (s, 1H), 11.38 (s, 1H). Mass spec m/z 702.1 $[M+H]^+$.

**Example 62 was synthesized following Scheme 60**

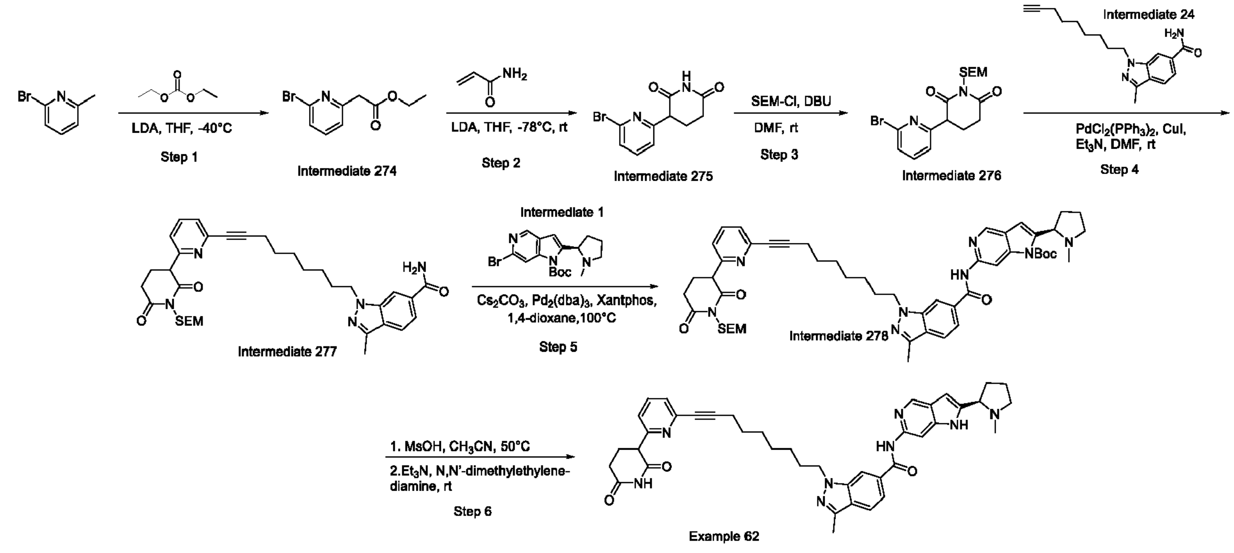


## Scheme 60

### Step 1

**Intermediate 274: ethyl 2-(6-bromopyridin-2-yl) acetate**

To a cooled (-40°C) solution of 2-bromo-6-methylpyridine (CAS No: 5315-25-3, 10.0 g, 58.13 mmol) in anhydrous tetrahydrofuran (100 mL) was added lithium diisopropylamide (2M in THF solution, 116 mL, 116.27 mmol) and the reaction mixture was stirred for 10 min at -40°C. Then diethyl carbonate (CAS No: 105-58-8, 13.73 g, 116.27 mmol) w was added and the mixture was stirred at -40°C for 2h. The reaction mixture was quenched with saturated $NH_4Cl$ solution (200 mL) and extracted with ethyl acetate (3 × 200 mL). The combined organic layers were separated, dried over anhydrous $Na_2SO_4$, filtered, and concentrated *in vacuo*. The crude material was purified by combi-flash chromatography, by eluting with 20% ethyl acetate in heptane, to afford ethyl 2-(6-bromopyridin-2-yl) acetate (**Intermediate 274,** 5.00 g, 35%) as a colourless liquid. $^1$H NMR (401 MHz, DMSO-*d*$_6$) δ ppm 1.18 (t, *J*=7.20 Hz, 3H), 3.85 (s, 2H), 4.10 (q, *J*=7.20 Hz, 2H), 7.42 (d, *J*=7.60 Hz, 1H), 7.55 (d, *J*=8.00 Hz, 1H), 7.74 (t, *J*=7.60 Hz, 1H). Mass spec m/z 244.09 [M+H]$^+$.

### Step 2

**Intermediate 275: 3-(6-bromopyridin-2-yl)piperidine-2,6-dione**

To a cooled (-78°C) solution of ethyl-2-(6-bromopyridin-2-yl)acetate **(Intermediate 274, 3.30** g, 14 mmol) in tetrahydrofuran (20 mL) was added acrylamide (CAS No: 79-06-1, 1.00 g, 14 mmol) and the reaction mixture was stirred for 10 min at -78°C. Then lithium diisopropylamide (2M in THF solution, 16 mL, 16 mmol) was added and the mixture was stirred at -78°C for 1h, then allowed to warm to room temperature and stirred for a further 3h. The reaction mixture was quenched with saturated $NH_4Cl$ solution (20 mL) and extracted with ethyl acetate (2 × 25 mL). The combined organic layers were separated, dried over anhydrous $Na_2SO_4$, filtered, and concentrated *in vacuo*. The crude material was purified by combi-flash chromatography, by eluting with 50% ethyl acetate in heptane, to afford 3-(6-bromopyridin-2-yl) piperidine-2,6-dione (**Intermediate 275,** 0.90 g, 25%) as a white solid. $^1$H NMR (400 MHz, DMSO-*d*$_6$) δ ppm 2.06-2.14 (m, 1H), 2.19-2.31 (m, 1H), 2.54-2.58 (m, 1H), 2.61-2.68 (m, 1H), 4.02-4.08 (m, 1H), 7.42 (d, *J*=7.60 Hz, 1H), 7.57 (d, *J*=8.0 Hz, 1H), 7.75 (t, *J*=7.60 Hz, 1H), 10.91 (br s, 1H). Mass spec m/z 269.2 [M+H]$^+$.

### Step 3

**Intermediate 276: 3-(6-bromopyridin-2-yl)-1-((2-(trimethylsilyl) ethoxy) methyl) piperidine-2, 6-dione**

To a cooled (0°C) solution of 3-(6-bromopyridin-2-yl) piperidine-2,6-dione (**Intermediate 275,** 0.70 g, 2.60 mmol) in dimethylformamide (10 mL) was added DBU (0.60 mL, 3.90 mmol), followed by 2-(trimethylsilyl)ethoxymethyl chloride (0.72 mL, 3.90 mmol). The reaction mixture was stirred at room temperature for 16h, then quenched with water (50 mL) and extracted with ethyl acetate (100 mL). The organic layer was separated, dried over anhydrous $Na_2SO_4$, filtered, and

concentrated *in vacuo.* The crude material was purified by combi-flash chromatography, by eluting with 50% ethyl acetate in heptane, to afford 3-(6-bromopyridin-2-yl)-1-((2-(trimethylsilyl)ethoxy)methyl)piperidine-2, 6-dione (**Intermediate 276**, 0.72 g, 45%) as an off-white solid. $^1$H NMR (400 MHz, DMSO-$d_6$) δ ppm -0.03 (s, 9H), 0.79-0.88 (m, 2H), 2.06-2.17 (m, 1H), 2.20-2.32 (m, 1H), 2.57-2.58 (m, 1H), 2.61-2.68 (m, 1H), 3.47-3.58 (m, 2H), 4.21-4.24 (m, 1H), 5.08 (s, 2H), 7.43 (d, *J*=7.60 Hz, 1H), 7.58 (d, *J*=8.0 Hz, 1H), 7.76 (t, *J*=8.0 Hz, 1H). Mass spec m/z 399.0 [M+H]$^+$.

**Step 4**

**Intermediate 277: 1-(9-(6-(2, 6-dioxo-1-((2-(trimethylsilyl) ethoxy) methyl) piperidin-3-yl) pyridin-2-yl) non-8-yn-1-yl)-3-methyl-1*H*-indazole-6-carboxamide**

To a solution of 3-methyl-1-(non-8-yn-1-yl)-1*H*-indazole-6-carboxamide (**Intermediate 24**, 0.52 g, 1.75 mmol) in dimethylformamide (15 mL) was added 3-(6-bromopyridin-2-yl)-1-((2-(trimethylsilyl)ethoxy)methyl)piperidine-2,6-dione (**Intermediate 276**, 0.70 g, 1.75 mmol) followed by triethylamine (8.84 mL, 63.09 mmol). The reaction mixture was purged with argon for 15 min, then $PdCl_2(PPh_3)_2$ (0.12 g, 0.173 mmol) and copper (I) iodide (0.034 g, 0.17 mmol) were added. The reaction mixture was purged with argon for 10 min and then stirred at room temperature for 16h. The reaction mixture was diluted with water (200 mL) and extracted with ethyl acetate (3 × 90 mL). The combined organic layers were separated, dried over anhydrous $Na_2SO_4$, and concentrated *in vacuo.* The crude material was purified by combi-flash column chromatography, by eluting with 80% ethyl acetate in heptane, to afford 1-(9-(6-(2,6-dioxo-1-((2-(trimethylsilyl)ethoxy) methyl)piperidin-3-yl) pyridin-2-yl)non-8-yn-1-yl)-3-methyl-1*H*-indazole-6-carboxamide (**Intermediate 277**, 0.55 g, 51%) as a yellow solid. $^1$H NMR (400 MHz, DMSO-$d_6$) δ ppm -0.04 (s, 9H), 0.80-0.84 (m, 2H), 1.07-1.12 (m, 2H), 1.22-1.42 (m, 6H), 1.46-1.52 (m, 2H), 1.73-1.83 (m, 2H), 2.36-2.43 (m, 2H), 2.49 (s, 3H), 2.81-2.86 (m, 2H), 3.53 (t, *J*=7.60 Hz, 2H), 4.12-4.20 (m, 1H), 4.34 (t, *J*=6.40 Hz, 2H), 5.08 (s, 2H), 7.28-7.34 (m, 2H), 7.40 (br s, 1H), 7.54-7.65 (m, 1H), 7.70-7.78 (m, 2H), 8.02 (br s, 1H), 8.12 (s, 1H). Mass spec m/z 616.1 [M+H]$^+$.

**Step 5**

**Intermediate 278: *tert*-butyl 6-(1-(9-(6-(2,6-dioxo-1-((2-(trimethylsilyl)ethoxy)methyl)piperidin-3-yl)pyridin-2-yl) non-8-yn-1-yl)-3-methyl-1*H*-indazole-6-carboxamido)-2-((*R*)-1-methylpyrrolidin-2-yl)-1*H*-pyrrolo[3,2-c]pyridine-1-carboxylate**

To a solution of 1-(9-(6-(2, 6-dioxo-1-((2-(trimethylsilyl) ethoxy) methyl) piperidin-3-yl) pyridin-2-yl) non-8-yn-1-yl)-3-methyl-1*H*-indazole-6-carboxamide (**Intermediate 277**, 0.50 g, 0.81 mmol) in 1,4-dioxane (15 mL) was added *tert*-butyl-(*R*)-6-bromo-2-(1-methylpyrrolidin-2-yl)-1*H*-pyrrolo[3,2-c]pyridine-1-carboxylate (**Intermediate 1**, 0.37 g, 0.97 mmol) followed by cesium carbonate (0.79 g, 2.43 mmol). The reaction mixture was purged with argon for 15 min then $Pd_2(dba)_3$ (0.11 g, 0.12 mmol) and Xantphos (0.14 g, 0.24 mmol) were added. The reaction mixture was further purged with argon for 10 min and heated at 100°C for 2h. The reaction mixture was filtered through a celite bed and the filter pad was washed with ethyl acetate (100 mL). The filtrate was concentrated *in vacuo* and the crude material purified by combi-flash chromatography, by eluting with 90% ethyl acetate in heptane, to afford *tert*-butyl 6-(1-(9-(6-(2,6-dioxo-1-((2-(trimethylsilyl)ethoxy)methyl)piperidin-3-yl)pyridin-2-yl)non-8-yn-1-yl)-3-methyl-1*H*-indazole-6-carboxamido)-2-((*R*)-1-methylpyrrolidin-2-yl)-1*H*-pyrrolo[3,2-c]pyridine-1-carboxylate (**Intermediate 278**, 0.54 g, 73%) as a yellow solid. $^1$H NMR (401 MHz, DMSO-$d_6$) δ ppm -0.05 (s, 9H), 0.77-0.85 (m, 2H), 1.23-1.42 (m, 6H), 1.48-1.56 (m, 2H), 1.59-1.67 (m, 2H), 1.71 (s, 9H), 1.74-1.80 (m, 2H), 1.82-1.92 (m, 2H), 2.30-2.34 (m, 1H), 2.36 (s, 3H), 2.38-2.42 (m, 3H), 2.51 (s, 3H), 2.55-2.57 (m, 1H), 2.69-2.80 (m, 2H), 3.09-3.18 (m, 1H), 3.48-3.55 (m, 2H), 3.89-3.97 (m, 1H), 4.15-4.20 (m, 1H), 4.38-4.44 (m, 2H), 5.07 (s, 2H), 6.77 (s, 1H), 7.29-7.34 (m, 2H), 7.71-7.76 (m, 2H), 7.77-7.82 (m, 1H), 8.44 (s, 1H), 8.62 (s, 1H), 8.99 (s, 1H), 10.79 (s, 1H). Mass spec m/z 914.9 [M+H]$^+$.

**Step 6**

**Example 62: 1-(9-(6-(2, 6-dioxopiperidin-3-yl) pyridin-2-yl) non-8-yn-1-yl)-3-methyl-*N*-(2-((*R*)-1-methylpyrrolidin-2-yl)-1*H*-pyrrolo [3, 2-c] pyridin-6-yl)-1*H* indazole-6-carboxamide**

To a solution of *tert*-butyl 6-(1-(9-(6-(2,6-dioxo-1-((2-(trimethylsilyl)ethoxy)methyl)piperidin-3-yl)pyridin-2-yl) non-8-yn-1-yl)-3-methyl-1*H*-indazole-6-carboxamido)-2-((*R*)-1-methylpyrrolidin-2-yl)-1*H*-pyrrolo[3,2-c]pyridine-1-carboxylate (**Intermediate 278,** 0.54 g, 0.59 mmol) in acetonitrile (2mL) was added methanesulfonic acid (0.39 mL, 5.90 mmol) and the reaction mixture was heated at 50°C for 2h. The reaction mixture was cooled to room temperature, then N, N'-dimethylethylenediamine (0.35 mL, 2.95 mmol) followed by triethylamine (1.65 mL, 11.80 mmol) were added and the mixture stirred at room temperature for 3h. The reaction mixture was then concentrated *in vacuo* and the obtained residue

was poured into water (50 mL). The resultant precipitate was collected by filtration and dried *in vacuo.* The crude material was purified by preparative HPLC (Method A) to afford 1-(9-(6-(2, 6-dioxopiperidin-3-yl) pyridin-2-yl) non-8-yn-1-yl)-3-methyl-*N*-(2-((*R*)-1-methylpyrrolidin-2-yl)-1*H*-pyrrolo [3, 2-c] pyridin-6-yl)-1*H*-indazole-6-carboxamide (**Example 62**, 0.074 g, 18%) as an off white solid. $^1$H NMR (400 MHz, DMSO-$d_6$) δ ppm 1.27-1.43 (m, 6H), 1.48-1.57 (m, 2H), 1.78-1.96 (m, 5H), 2.05-2.12 (m, 1H), 2.13-2.16 (m, 1H), 2.18 (s, 3H), 2.22-2.31 (m, 2H), 2.40-2.44 (m, 2H), 2.51 (s, 3H), 2.53-2.56 (m, 2H), 3.12-3.18 (m, 1H), 3.32-3.34 (m, 1H), 3.98-4.02 (m, 1H), 4.38-4.44 (m, 2H), 6.41 (s, 1H), 7.28-7.33 (m, 2H), 7.70-7.76 (m, 2H), 7.77-7.81 (m, 1H), 8.26 (s, 1H), 8.44 (s, 1H), 8.52 (s, 1H), 10.54 (s, 1H), 10.88 (br s, 1H), 11.40 (br s, 1H). Mass spec m/z 685.2 [M+H]$^+$.

**Example 63 was synthesized following Scheme 61**

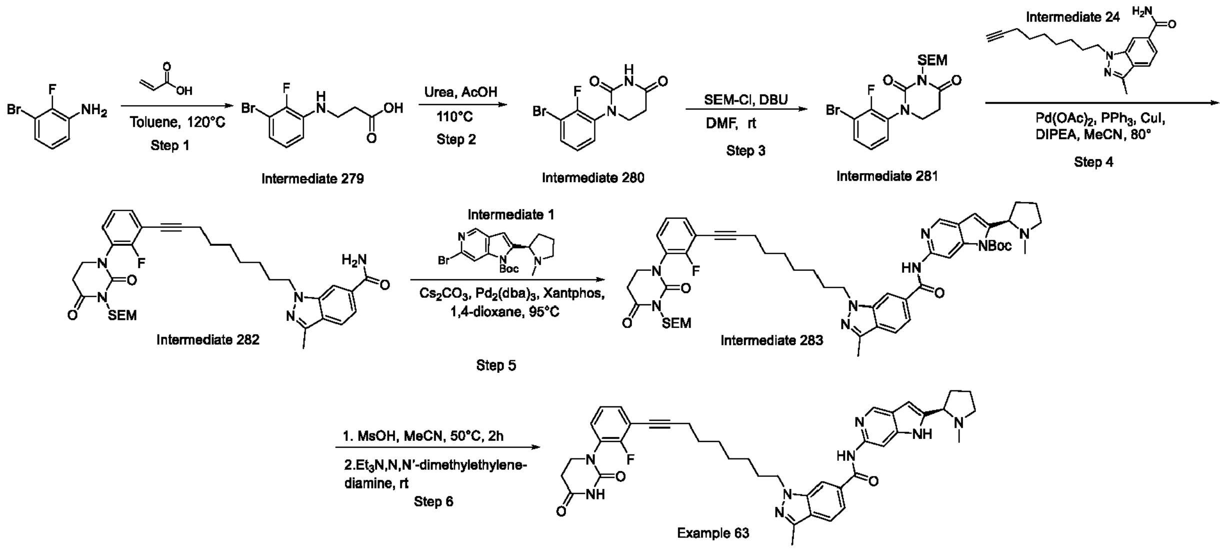


**Scheme 61**

**Step 1**

**Intermediate 279: 3-((3-bromo-2-fluorophenyl) amino) propanoic acid**

To a solution of 3-bromo-2-fluoroaniline (CAS No. 58534-95-5, 2.00 g, 10.5 mmol) in toluene (20 mL) was added acrylic acid (CAS No. 79-10-7, 1.14 g, 15.8 mmol) at room temperature. The reaction mixture was heated at 120°C for 18h. The reaction mixture was concentrated *in vacuo.* The crude residue was poured into saturated $NaHCO_3$ solution (pH~9) and extracted with DCM (2 × 120 mL). The aqueous layer was acidified by adjusted to pH~5 with 1N HCl. The resultant precipitate was filtered, washed with heptane (60 mL) and then dried *in vacuo* to afford 3-((3-bromo-2-fluorophenyl) amino) propanoic acid (**Intermediate 279,** 2.00 g, 73%) as an off white solid which was used for the next step without further purification. $^1$H NMR (400 MHz, DMSO-$d_6$) δ ppm 2.52-2.61 (m, 2H), 3.35-3.51 (m, 2H), 5.72 (br s, 1H), 6.67-6.85 (m, 2H), 6.86-6.98 (m, 1H), 12.25 (br s, 1H). Mass spec m/z 262.2 [M+H]$^+$.

**Step 2**

**Intermediate 280: 1-(3-bromo-2-fluorophenyl) dihydropyrimidine-2,4(1*H*, *3H)*-dione**

A solution of 3-((3-bromo-2-fluorophenyl) amino) propanoic acid (**Intermediate 279,** 2.00 g, 7.6 mmol) in acetic acid (15 mL) was heated at 110°C for 15 min then urea (2.3 g, 38 mmol) was added and the reaction mixture was stirred at 110°C for 20h. The reaction mixture was concentrated *in vacuo.* The obtained residue was poured into saturated $NaHCO_3$ solution (pH-9), the resultant precipitate collected by filtration, washed with heptane (50 mL) then dried *in vacuo* to afford 1-(3-bromo-2-fluorophenyl) dihydropyrimidine-2, 4(1*H*, 3*H*)-dione (**Intermediate 280**, 1.50 g, 68%) as an off white solid which was used for the next step without further purification. $^1$H NMR (400 MHz, DMSO-$d_6$) δ ppm 2.72 (t, *J*=6.58 Hz, 2H), 3.74(t, *J*=6.58 Hz, 2H), 7.22 (t, *J*=7.89 Hz, 1H), 7.47 (t, *J*=7.24 Hz, 1H), 7.66 (t, *J*=7.02 Hz, 1H), 10.53 (br s, 1H). Mass spec

m/z 287.2 $[M+H]^+$.

**Step 3**

**Intermediate 281: 1-(3-bromo-2-fluorophenyl)-3-((2-(trimethylsilyl) ethoxy) methyl) dihydropyrimidine-2, 4(1*H*, 3*H*)-dione**

To a solution of 1-(3-bromo-2-fluorophenyl) dihydropyrimidine-2, 4(1*H*, 3*H*)-dione (**Intermediate 280**, 1.40 g, 4.9 mmol) in dimethylformamide (20 mL) was added DBU (2.6 g, 17 mmol) at 0°C followed by 2-(trimethylsilyl)methoxymethyl chloride (2.8 g, 17 mmol). The reaction mixture was stirred at room temperature for 16h. The reaction mixture was poured into ice cold water (60 mL) and extracted with ethyl acetate (2 × 90 mL). The organic layer was separated, dried over anhydrous $Na_2SO_4$, filtered, and concentrated *in vacuo.* The crude material was purified by combi-flash chromatography, by eluting with 40% ethyl acetate in heptane, to afford 1-(3-bromo-2-fluorophenyl)-3-((2-(trimethylsilyl) ethoxy) methyl) dihydropyrimidine-2, 4(1*H*, 3*H*)-dione (**Intermediate 281,** 1.78 g, 87%) as a colourless gum. $^1$H NMR (400 MHz, DMSO-$d_6$) δ ppm -0.01 (s, 9H), 0.85 (t, *J*=7.89 Hz, 2H), 2.91 (t, *J*=6.58 Hz, 2H), 3.56 (t, *J*=7.89 Hz, 2H), 3.76 (t, *J*=6.58 Hz, 2H), 5.11 (s, 2H), 7.25 (t, *J*=8.11 Hz, 1H), 7.49 (t, *J*=7.24 Hz, 1H), 7.70 (t, *J*=7.02 Hz, 1H).

**Step 4**

**Intermediate 282: 1-(9-(3-(2, 4-dioxo-3-((2-(trimethylsilyl) ethoxy) methyl) tetrahydropyrimidin-1(2*H*)-yl)-2-fluorophenyl) non-8-yn-1-yl)-3-methyl-1*H* indazole-6-carboxamide**

To a solution of 1-(3-bromo-2-fluorophenyl)-3-((2-(trimethylsilyl) ethoxy) methyl) dihydropyrimidine-2,4(1*H*, 3*H*)-dione (**Intermediate 281,** 1.47 g, 3.53 mmol) in acetonitrile (15 mL) was added 3-methyl-1-(non-8-yn-1-yl)-1*H*-indazole-6-carboxamide (**Intermediate 24,** 0.70 g, 2.35 mmol) followed by DIPEA (1.52 g, 11.77 mmol) at room temperature. The reaction mixture was purged with argon for 15 min followed the addition of triphenylphosphine (0.18 g, 0.70 mmol), copper (I) iodide (0.047 g, 0.23 mmol) and palladium acetate (0.058 g, 0.35 mmol). The reaction mixture was purged with argon for 10 min then heated at 80°C for 16h. The reaction mixture was concentrated *in vacuo* to obtain the crude compound. The crude material was purified by combi-flash column chromatography, by eluting with 10% MeOH in DCM, to afford 1-(9-(3-(2, 4-dioxo-3-((2-(trimethylsilyl) ethoxy) methyl) tetrahydropyrimidin-1(2*H*)-yl)-2-fluorophenyl) non-8-yn-1-yl)-3 -methyl-1*H*-indazole-6-carboxamide (**Intermediate 282, 0.44** g, 29%) as a yellow gum. $^1$H NMR (400 MHz, DMSO-$d_6$) δ ppm -0.07 (s, 9H), 0.80 (t, *J*=7.89 Hz, 2H), 1.16-1.40 (m, 6H), 1.42-1.52 (m, 2H), 1.73-1.83 (m, 2H), 2.36-2.43 (m, 2H), 2.45 (s, 3H), 2.81-2.86 (m, 2H), 3.51 (t, *J*=7.67 Hz, 2H), 3.63-3.72 (m, 2H), 4.29 (t, *J*=6.80 Hz, 2H), 5.06 (s, 2H), 7.17 (t, *J*=7.60 Hz 1H), 7.34-7.42 (m, 3H), 7.55 (d, *J*=8.33 Hz, 1H), 7.68 (d, *J*=8.33 Hz, 1H), 7.98 (br s, 1H), 8.08 (s, 1H). Mass spec m/z 634.44 $[M+H]^+$.

**Step 5**

**Intermediate 283: tert-butyl (*R*)-6-(1-(9-(3-(2,4-dioxo-3-((2-(trimethylsilyl)ethoxy)methyl)tetrahydropyrimidin-1(2*H*)-yl)-2-fluorophenyl)non-8-yn-1-yl)-3-methyl-1*H*-indazole-6-carboxamido)-2-(1-methylpyrrolidin-2-yl)-1*H*-pyrrolo[3,2-c]pyridine-1-carboxylate**

To a solution of 1-(9-(3-(2, 4-dioxo-3-((2-(trimethylsilyl) ethoxy) methyl) tetrahydropyrimidin-1(2H)-yl)-2-fluorophenyl) non-8-yn-1-yl)-3-methyl-1H-indazole-6-carboxamide (**Intermediate 282,** 0.42 g, 0.67 mmol) in 1,4-dioxane (3 mL) was added *tert*-butyl (*R*)-6-bromo-2-(1-methylpyrrolidin-2-yl)-1H-pyrrolo[3,2-c]pyridine-1-carboxylate (**Intermediate 1,** 0.32 g, 0.84 mmol) followed by cesium carbonate (0.43 g, 1.26 mmol) at room temperature. The reaction mixture was purged with argon for 15 min followed by the addition of $Pd_2(dba)_3$ (0.11 g, 0.12 mmol) and Xantphos (0.076 g, 0.12 mmol). The reaction mixture was purged with argon for 10 min and then heated at 95°C for 2h. The reaction mixture was concentrated *in vacuo* to obtain crude compound. The crude material was purified by combi-flash column chromatography, by eluting with 10% MeOH in DCM, to afford *tert*-butyl (*R*)-6-(1-(9-(3-(2,4-dioxo-3-((2-(trimethylsilyl)ethoxy) methyl)tetrahydropyrimidin-1(2*H*)-yl)-2-fluorophenyl)non-8-yn-1-yl)-3-methyl-1*H*-indazole-6-carboxamido)-2-(1-methylpyrrolidin-2-yl)-1*H*-pyrrolo[3,2-c]pyridine-1-carboxylate (**Intermediate 283,** 0.40 g, 51%) as an off white solid. $^1$H NMR (400 MHz, DMSO-$d_6$) δ ppm -0.04 (s, 9H), 0.78-0.86 (m, 2H), 1.22-1.46 (m, 7H), 1.47-1.57 (m, 2H), 1.70 (s, 9H), 1.73-1.80 (m, 2H), 1.81-1.91 (m, 2H), 2.30-2.34 (m, 1H), 2.36 (s, 3H), 2.42-2.46 (m, 2H), 2.49 (s, 3H), 2.83-2.92 (m, 2H), 3.11-3.19 (m, 1H), 3.49-3.59 (m, 3H), 3.68-3.74 (m, 2H), 3.89-3.96 (m, 1H), 4.40 (t, *J*=6.80 Hz, 2H), 5.09 (s, 2H), 6.76 (s, 1H), 7.17-7.23 (m, 1H), 7.36-7.44 (m, 2H), 7.69-7.81 (m, 2H), 8.43 (s, 1H), 8.61 (s, 1H), 8.98 (s, 1H), 10.76 (s, 1H). Mass spec m/z 933.63 $[M+H]^+$.

**Step 6**

**Example 63: (*R*)-1-(9-(3-(2, 4-dioxotetrahydropyrimidin-1(2*H*)-yl)-2-fluorophenyl) non-8-yn-1-yl)-3-methyl-*N*-(2-(1-methylpyrrolidin-2-yl)-1H-pyrrolo [3, 2-c] pyridin-6-yl)-1*H*-indazole-6-carboxamide**

To a cooled (0°C) solution of *tert*-butyl (*R*)-6-(1-(9-(3-(2,4-dioxo-3-((2-(trimethylsilyl)ethoxy)methyl)tetrahydropyrimidin-1(2*H*)-yl)-2-fluorophenyl)non-8-yn-1-yl)-3-methyl-1*H*-indazole-6-carboxamido)-2-(1-methylpyrrolidin-2-yl)-1*H*-pyrrolo[3,2-c]pyridine-1-carboxylate **(Intermediate 283, 0.30** g, 0.32 mmol) in acetonitrile (3 mL) was added methane sulfonic acid (0.32 g, 3.21 mmol) and the reaction mixture was then heated at 50°C for 2h. The reaction mixture was cooled to room temperature then *N*, *N'*-dimethylethylenediamine (0.18 g, 1.93 mmol), followed by triethylamine (0.68 g, 6.43 mmol), were added. The reaction was stirred at room temperature for 2h. The reaction mixture was poured into ice cold water (50 mL), the resultant precipitate collected by filtration and dried *in vacuo* to afford the crude compound. The crude material was purified by preparative HPLC (Method A) to afford (*R*)-1-(9-(3-(2,4-dioxotetrahydropyrimidin-1(2*H*)-yl)-2-fluorophenyl)non-8-yn-1-yl)-3-methyl-*N*-(2-(1-methylpyrrolidin-2-yl)-1*H*-pyrrolo[3, 2-c]pyridin-6-yl)-1*H*-indazole-6-carboxamide **(Example 63,** 0.075 g, 23%) as an off white solid. $^1$H NMR (400 MHz, DMSO-$d_6$) δ ppm 1.34-1.48 (m, 7H), 1.56-1.62 (m, 2H), 1.90-1.97 (m, 5H), 2.31-2.33 (m, 1H), 2.35 (s, 3H), 2.38-2.40 (m, 1H), 2.52 (s, 3H), 2.54-2.56 (m, 1H), 2.73-2.79 (m, 2H), 3.19-3.24 (m, 1H), 3.38-3.42 (m, 1H), 3.77 (t, *J*=6.80 Hz, 2H), 4.47(t, *J*=7.00 Hz, 2H), 6.47 (s, 1H), 7.25 (t, *J*=7.80 Hz, 1H), 7.42-7.49 (m, 2H), 7.79-7.86 (m, 2H), 8.32 (s, 1H), 8.51 (s, 1H), 8.59 (s, 1H), 10.58 (s, 1H), 10.60 (s, 1H). Mass spec m/z 703.10 $[M+H]^+$.

**Example 64 was synthesized following Scheme 62**

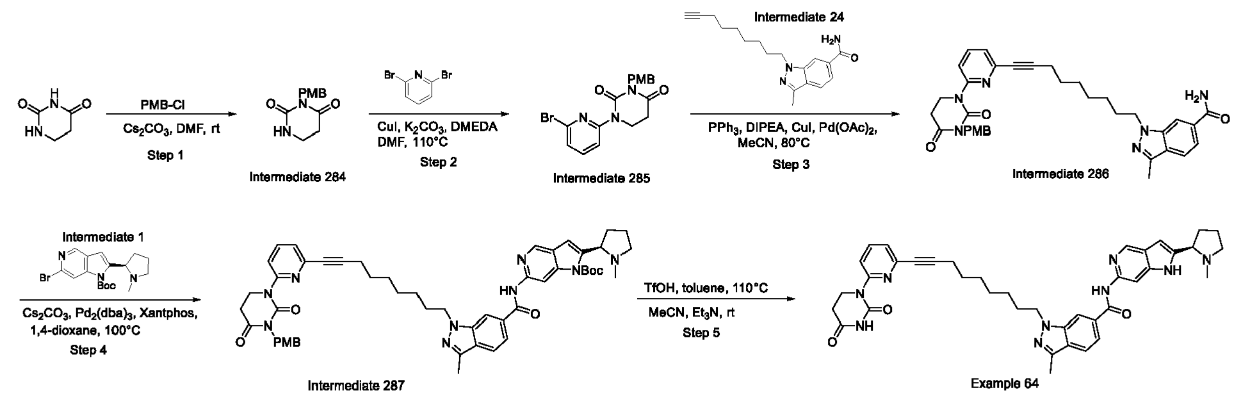


**Scheme 62**

**Step 1**

**Intermediate 284: 3-(4-methoxybenzyl) dihydropyrimidine-2,4(1*H*, 3*H*)-dione**

To a solution of dihydropyrimidine-2,4(1*H*,3H)-dione (CAS No: 504-07-4, 5.00 g, 43.82 mmol) in dimethylformamide (150 mL) was added cesium carbonate (28.56 g, 87.64 mmol) followed by 4-methoxybenzyl chloride (CAS No: 824-94-2, 4.06 mL, 28.48 mmol). The reaction mixture was stirred at room temperature for 16h. The reaction mixture was quenched with ice cold water (1500 mL) and stirred for 30 min, the resultant precipitate collected by filtration and dried *in vacuo* to afford 3-(4-methoxybenzyl) dihydropyrimidine-2,4(1*H*,3*H*)-dione **(Intermediate 284,** 4.50 g, 44%) as an off white solid, which was used for the next step without further purification. $^1$H NMR (400 MHz, DMSO-$d_6$) δ ppm 2.62 (t, *J*=6.80 Hz, 2H), 3.19-3.23 (m, 2H), 3.71 (s, 3H), 4.71 (s, 2H), 6.83-6.85 (m, 2H), 7.16-7.18 (m, 2H), 7.80 (br s, 1H).

**Step 2**

**Intermediate 285: 1-(6-bromopyridin-2-yl)-3-(4-methoxybenzyl) dihydropyrimidine-2,4(1*H*, 3*H*)-dione**

To a solution of 2, 6-dibromopyridine (CAS No: 626-05-1, 7.00 g, 29.5 mmol) in dimethylformamide (10 mL) was added 3-(4-methoxybenzyl)dihydropyrimidine-2,4(1*H*, 3*H*)-dione **(Intermediate 284,** 4.15 g, 17.7 mmol) followed by

$K_2CO_3$ (12.3 g, 88.6 mmol). The reaction mixture was purged with argon gas for 15 min then copper (I) iodide (0.56 g, 2.95 mmol) and *N*, *N'*-dimethylethylenediamine (0.53 g, 5.91 mmol) were added. The reaction mixture was heated at 110°C for 16h. The reaction mixture was filtered and the precipitate washed with ethyl acetate (100 mL). The filtrate was washed with ice cold water (100 mL). The organic layer was separated, dried over $Na_2SO_4$, and concentrated *in vacuo.* The crude material was purified by combi-flash column chromatography, by eluting with 20% ethyl acetate in heptane, to afford 1-(6-bromopyridin-2-yl)-3-(4-methoxybenzyl) dihydropyrimidine-2,4(1*H*,3*H*)-dione **(Intermediate 285,** 3.4 g, 29%) as an off white solid. $^1$H NMR (400 MHz, DMSO-$d_6$) δ ppm 2.88 (t, *J*=6.80 Hz, 2H), 3.72 (s, 3H), 4.02 (t, *J*=6.80 Hz, 2H), 4.83 (s, 2H), 6.84-6.86 (m, 2H), 7.22-7.25 (m, 2H), 7.42-7.47 (m, 1H), 7.76-.7.79 (m, 2H).

**Step 3**

**Intermediate 286: 1-(9-(6-(3-(4-methoxybenzyl)-2,4-dioxotetrahydropyrimidin-1(2*H*)-yl)pyridin-2-yl)non-8-yn-1-yl)-3-methyl-1*H*-indazole-6-carboxamide**

To a solution of 1-(6-bromopyridin-2-yl)-3-(4-methoxybenzyl)dihydropyrimidine-2,4(1*H*,3*H*)-dione **(Intermediate 285,** 1.5 g, 3.8 mmol) in acetonitrile (25 mL) was added 3-methyl-1-(non-8-yn-1-yl)-1*H*-indazole-6-carboxamide **(Intermediate 24,** 3.8 mmol, 5.8 mmol) followed by DIPEA (3.3 mL, 19 mmol) at room temperature. The reaction mixture was purged with argon gas for 15 min then triphenylphosphine (0.30 g, 1.2 mmol), copper (I) iodide (0.07 g, 0.38 mmol) and palladium (II) acetate (0.095 g, 0.58 mmol) were added. The reaction mixture was further purged with argon gas for another 10 min and then heated at 80°C for 16h. The reaction mixture was concentrated *in vacuo* to obtain crude compound. The crude material was purified by combi-flash chromatography, by eluting with 15% ethyl acetate in heptane, to afford 1-(9-(6-(3-(4-methoxybenzyl)-2,4-dioxotetrahydropyrimidin-1(2*H*)-yl)pyridin-2-yl)non-8-yn-1-yl)-3-methyl-1*H*-indazole-6-carboxamide **(Intermediate 286,** 1.5 g, 64%) as a yellow gummy liquid. $^1$H NMR (400 MHz, DMSO-$d_6$) δ ppm 1.21-1.42 (m, 6H), 1.46-1.56 (m, 2H), 1.76-1.87 (m, 2H), 2.40 (t, *J*=7.20 Hz, 2H), 2.48 (s, 3H), 2.85 (t, *J*=6.80 Hz, 2H), 3.71 (s, 3H), 3.99 (t, *J*=6.80 Hz, 2H), 4.33 (t, *J*=7.20 Hz, 2H), 4.83 (s, 2H), 6.84-6.87 (m, 2H), 7.22-7.26 (m, 3H), 7.41 (br s, 1H), 7.56-7.66 (m, 2H), 7.69-7.80 (m, 2H), 8.03 (br s, 1H), 8.12 (s, 1H). Mass spec m/z 607.38 $[M+H]^+$.

**Step 4**

**Intermediate 287: *tert*-butyl (*R*)-6-(1-(9-(6-(3-(4-methoxybenzyl)-2,4-dioxotetrahydropyrimidin-1(2*H*)-yl)pyridin-2-yl)non-8-yn-1-yl)-3-methyl-1*H*-indazole-6-carboxamido)-2-(1-methylpyrrolidin-2-yl)-1*H*-pyrrolo[3,2-c]pyridine-1-carboxylate**

To a solution of 1-(9-(6-(3-(4-methoxybenzyl)-2,4-dioxotetrahydropyrimidin-1(2*H*)-yl)pyridin-2-yl)non-8-yn-1-yl)-3-methyl-1*H*-indazole-6-carboxamide **(Intermediate 286, 0.70** g, 1.15 mmol) in 1,4-dioxane (20 mL) was added *tert*-butyl (*R*)-6-bromo-2-(1-methylpyrrolidin-2-yl)-1*H*-pyrrolo[3,2-c]pyridine-1-carboxylate **(Intermediate 1,** 0.48 g, 1.27 mmol) followed by cesium carbonate (1.14 g, 3.46 mmol) at room temperature. The reaction mixture was purged with argon for 15 min followed by the addition of $Pd_2(dba)_3$ (0.16 g, 0.17 mmol) and Xantphos (0.20 g, 0.34 mmol). The reaction mixture was purged with argon for 10 min and then heated at 100°C for 2h. The reaction mixture was concentrated *in vacuo* to obtain crude compound. The crude material was purified by combi-flash chromatography, by eluting with 3% MeOH in DCM, to afford *tert*-butyl (*R*)-6-(1-(9-(6-(3-(4-methoxybenzyl)-2,4-dioxotetrahydropyrimidin-1(2*H*)-yl)pyridin-2-yl)non-8-yn-1-yl)-3-methyl-1*H*-indazole-6-carboxamido)-2-(1-methylpyrrolidin-2-yl)-1*H*-pyrrolo[3,2-c]pyridine-1-carboxylate **(Intermediate 287,** 0.6 g, 57%) as off brown solid. $^1$H NMR (400 MHz, DMSO-$d_6$) δ ppm 1.21-1.42 (m, 6H), 1.47-1.56 (m, 2H), 1.59-1.66 (m, 1H), 1.70 (s, 9H), 1.82-1.92 (m, 3H), 2.35 (s, 3H), 2.45 (s, 3H), 2.82-2.86 (m, 2H), 3.13-3.18 (m, 3H), 3.36-3.56 (m, 3H), 3.71 (s, 3H), 3.89-4.02 (m, 3H), 4.06-4.10 (m, 2H), 4.38-4.42 (m, 2H), 6.76 (s, 1H), 6.83-6.86 (m, 2H), 7.17-7.26 (m, 3H), 7.61-7.64 (m, 1H), 7.68-7.81 (m, 3H), 8.43 (s, 1H), 8.60 (s, 1H), 8.98 (s, 1H), 10.75 (br s, 1H). Mass spec m/z 906.57 $[M+H]^+$.

**Step 5**

**Example 64: (*R*)-1-(9-(6-(2,4-dioxotetrahydropyrimidin-1(2*H*)-yl)pyridin-2-yl)non-8-yn-1-yl)-3-methyl-N (2-(1-methylpyrrolidin-2-yl)-1H pyrrolo[3,2-c]pyridin-6-yl)-1*H*-indazole-6-carboxamide**

To a solution of *tert*-butyl (*R*)-6-(1-(9-(6-(3-(4-methoxybenzyl)-2,4-dioxotetrahydropyrimidin-1(2*H*)-yl)pyridin-2-yl)non-8-yn-1-yl)-3-methyl-1*H*-indazole-6-carboxamido)-2-(1-methylpyrrolidin-2-yl)-1*H*-pyrrolo[3,2-c]pyridine-1-carboxylate **(Intermediate 287,** 0.50 g, 0.55 mmol) in toluene (3 mL) was added trifluoromethanesulfonic acid (0.49 mL, 5.52 mmol) then the reaction was heated to 110°C for 3h. The toluene layer was decanted and the resulting residue was taken up in acetonitrile (10 mL). Triethylamine (1.55 mL, 11.04 mmol) was added and the reaction was stirred at room

temperature for 10 min then concentrated *in vacuo.* The obtained residue was diluted with ice cold water (50 mL), the resultant precipitate collected by filtration and dried *in vacuo* to afford the crude compound. The crude material was purified by preparative HPLC (Method A) to afford (R)-1-(9-(6-(2,4-dioxotetrahydropyrimidin-1(2*H*)-yl)pyridin-2-yl)non-8-yn-1-yl)-3-methyl-*N*-(2-(1-methylpyrrolidin-2-yl)-1*H*-pyrrolo[3,2-c]pyridin-6-yl)-1*H*-indazole-6-carboxamide **(Example 64,** 0.039 g, 10%) as an off white solid. $^1$H NMR (400 MHz, DMSO-$d_6$) δ ppm 1.23-1.43 (m, 7H), 1.48-1.57 (m, 2H), 1.76-1.97 (m, 5H), 2.12-2.16 (m, 1H), 2.17 (s, 3H), 2.24-2.30 (m, 1H), 2.40-2.44 (m, 2H), 2.52 (s, 3H), 2.63-2.67 (m, 2H), 3.12-3.18 (m, 1H), 3.98-4.02 (m, 2H), 4.38-4.42 (m, 2H), 6.40 (s, 1H), 7.18-7.20 (m, 1H), 7.66-7.81 (m, 4H), 8.25 (s, 1H), 8.43 (s, 1H), 8.52 (s, 1H), 10.52 (br s, 2H), 11.38 (br s, 1H). Mass spec m/z 686.0 [M+H]$^+$.

**Example 65 was synthesized following Scheme 63**

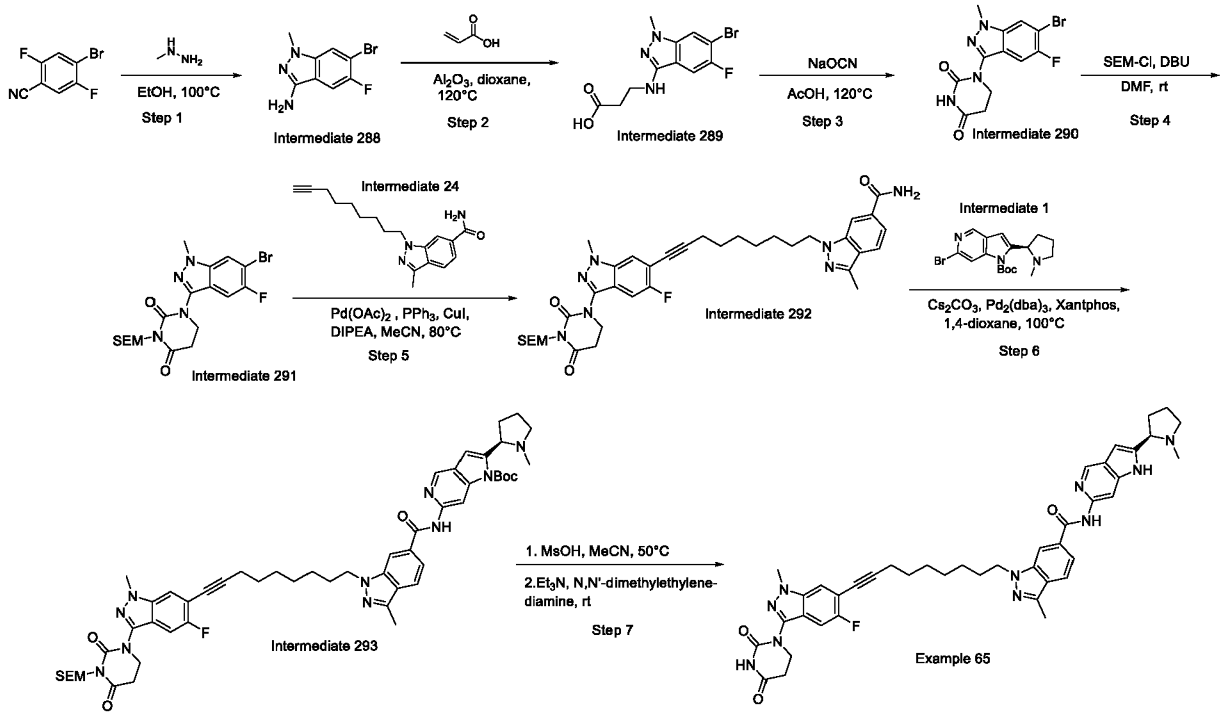


**Scheme 63**

**Step 1**

**Intermediate 288: 6-bromo-5-fluoro-1-methyl-1*H*-indazol-3-amine**

To a solution of 4-bromo-2,5-difluorobenzonitrile (CAS No: 133541-45-4, 5.00 g, 22.93 mmol) in ethanol (50 mL) was added methyl hydrazine (5.28 g, 114.68 mmol) and the reaction mixture was heated at 100°C for 16h. The reaction mixture was concentrated *in vacuo* and the resultant residue was diluted with water (300 mL). The resultant precipitate was collected by filtration and dried *in vacuo* to afford 6-bromo-5-fluoro-1-methyl-1*H*-indazol-3-amine **(Intermediate 288,** 3.00 g, 54%) as a white solid, which was used for the next step without further purification. $^1$H NMR (400 MHz, DMSO-$d_6$) δ ppm 3.71 (s, 3H), 5.51 (s, 2H), 7.62 (d, *J*=9.21 Hz, 1H), 7.81 (d, *J*=5.70 Hz, 1H). Mass spec m/z 244.2 [M+H]$^+$.

**Step 2**

**Intermediate 289: 3-((6-bromo-5-fluoro-1-methyl-1*H*-indazol-3-yl) amino) propanoic acid**

To a solution of 6-bromo-5-fluoro-1-methyl-1*H*-indazol-3-amine **(Intermediate 288,** 3.00 g, 12.3 mmol) in 1, 4-dioxane (30 mL) was added acrylic acid (CAS No: 79-10-7, 2.66 g, 36.9 mmol) followed by aluminium oxide (1.5 mL, 49.2 mmol) and the reaction mixture was heated at 120°C for 16h. The reaction mixture was filtered through a celite bed and the filter pad was washed with dichloromethane (50 mL). The filtrate was concentrated *in vacuo* and the resultant crude

material purified by combi-flash chromatography, by eluting with 50% ethyl acetate in heptane, to afford 3-((6-bromo-5-fluoro-1-methyl-1*H*-indazol-3-yl)amino)propanoic acid **(Intermediate 289, 2.30** g, 52%) as an off-white solid. $^1$H NMR (400 MHz, DMSO-$d_6$) δ ppm 2.60 (t, *J*=6.40 Hz, 2H), 3.44 (t, *J*=6.40 Hz, 2H), 3.75 (s, 3H), 6.16-6.25 (m, 1H), 7.65 (d, *J*=9.20 Hz, 1H), 7.82 (d, *J*=5.60 Hz, 1H), 12.19 (br s, 1H). Mass spec m/z 316.2 $[M+H]^+$.

**Step 3**

**Intermediate 290: 1-(6-bromo-5-fluoro-1-methyl-1H indazol-3-yl)dihydropyrimidine-2,4(1*H*, 3*H*)-dione**

To a solution of 3-((6-bromo-5-fluoro-1-methyl-1*H*-indazol-3-yl) amino)propanoic acid **(Intermediate 289,** 1.90 g, 6.0 mmol) in acetic acid (20 mL) was added sodium cyanate (0.88 g, 18 mmol) and the reaction mixture was heated at 120°C for 48h. The reaction mixture was concentrated *in vacuo* to afford crude 1-(6-bromo-5-fluoro-1-methyl-1*H*-indazol-3-yl) dihydropyrimidine-2,4(1*H*,3*H*)-dione **(Intermediate 290,** 0.60 g, 27%) as a grey solid, which was used for the next step without further purification. $^1$H NMR (400 MHz, DMSO-$d_6$) δ ppm 2.78 (t, *J*=6.40 Hz, 2H), 3.94 (t, *J*=6.40 Hz, 2H), 4.02 (s, 3H), 7.63 (d, *J*=9.20 Hz, 1H), 8.18 (d, *J*=5.60 Hz, 1H), 10.61 (s, 1H). Mass spec m/z 341.08 $[M+H]^+$.

**Step 4**

**Intermediate 291: 1-(6-bromo-5-fluoro-1-methyl-1H indazol-3-yl)-3-((2-(trimethylsilyl)ethoxy)methyl) dihydropyrimidine-2, 4(1*H*, 3*H*)-dione**

To a cooled (0°C) solution of 1-(6-bromo-5-fluoro-1-methyl-1*H*-indazol-3-yl)dihydropyrimidine-2,4(1*H*,3*H*)-dione **(Intermediate 290,** 1.00 g, 2.93 mmol) in dimethylformamide (4 mL) was added DBU (1.35 mL, 8.79 mmol) followed by 2-(trimethylsilyl)ethoxymethyl chloride (1.1 mL, 5.86 mmol). The reaction mixture was stirred at room temperature for 16h. The reaction mixture was diluted with water (200 mL) and extracted with ethyl acetate (3 × 100 mL). The combined organic layers were separated, dried over anhydrous $Na_2SO_4$, filtered, and concentrated *in vacuo.* The crude material was purified by combi-flash chromatography, by eluting with 50% ethyl acetate in heptane, to afford 1-(6-bromo-5-fluoro-1-methyl-1*H*-indazol-3-yl)-3-((2-(trimethylsilyl)ethoxy) methyl)dihydropyrimidine-2, 4(1*H*, 3*H*)-dione **(Intermediate 291,** 0.90 g, 65%) as a yellow liquid. $^1$H NMR (400 MHz, DMSO-$d_6$) δ ppm -0.01 (s, 9H), 0.84-0.88 (m, 2H), 2.92-2.96 (m, 2H), 3.56-3.62 (m, 2H), 3.91-3.94 (m, 2H), 4.01 (s, 3H), 5.15 (s, 2H), 7.56 (d, *J*=8.7 Hz, 1H), 8.19 (d, *J*=5.8 Hz, 1H).

**Step 5**

**Intermediate 292: 1-(9-(3-(2,4-dioxo-3-((2-(trimethylsilyl)ethoxy)methyl)tetrahydropyrimidin-1(2*H*)-yl)-5-fluoro-1-methyl-1*H*-indazol-6-yl)non-8-yn-1-yl)-3-methyl-1*H*-indazole-6-carboxamide**

To a solution of 3-methyl-1-(non-8-yn-1-yl)-1*H*-indazole-6-carboxamide **(Intermediate 24,** 0.63 g, 2.1 mmol) in acetonitrile (20 mL) was added 1-(6-bromo-5-fluoro-1-methyl-1*H*-indazol-3-yl)-3-((2-(trimethylsilyl)ethoxy) methyl)dihydropyrimidine-2,4(1*H*,3*H*-dione **(Intermediate 291,** 1.00 g, 2.1 mmol) followed by DIPEA (2 mL, 11 mmol). The reaction mixture was purged with argon for 15 min, then copper (I) iodide (0.043 g, 0.21 mmol), $Pd(OAc)_2$ (0.053 g, 0.32 mmol) and triphenylphosphine (0.17 g, 0.64 mmol) were added. The reaction mixture was further purged with argon for 10 min and then heated at 80°C for 4h. The reaction mixture was diluted with water (100 mL) and extracted with ethyl acetate (3 × 50 mL). The combined organic layers were separated, dried over anhydrous $Na_2SO_4$, filtered, and concentrated *in vacuo.* The crude material was purified by combi-flash chromatography, by eluting with 2% MeOH in dichloromethane, to afford 1-(9-(3-(2,4-dioxo-3-((2-(trimethylsilyl)ethoxymethyl)tetrahydropyrimidin-1(2*H*)-yl)-5-fluoro-1-methyl-1*H*-indazol-6-yl) non-8-yn-1-yl)-3-methyl-1*H*-indazole-6-carboxamide **(Intermediate 292,** 0.80 g, 47%) as a yellow solid. $^1$H NMR (400 MHz, DMSO-$d_6$) δ ppm -0.01 (s, 9H), 0.84-0.88 (m, 2H), 1.23-1.46 (m, 7H), 1.52-1.58 (m, 2H), 1.78-1.88 (m, 2H), 2.47 (s, 3H), 2.52-2.57 (m, 1H), 2.92-2.96 (m, 2H), 3.57-3.62 (m, 2H), 3.90-3.94 (m, 2H), 3.99 (s, 3H), 4.32-4.36 (m, 2H), 5.15 (s, 2H), 7.38-7.42 (m, 2H), 7.59 (d, *J*=8.80 Hz, 1H), 7.72 (d, *J*=8.80 Hz, 1H), 7.82-7.84 (m, 1H), 8.03 (br s, 1H), 8.12 (s, 1H). Mass spec m/z 688.3 $[M+H]^+$.

**Step 6**

**Intermediate 293: Synthesis of *tert*-butyl-(*R*)-6-(1-(9-(3-(2,4-dioxo-3-((2-(trimethylsilyl)ethoxy)methyl)tetrahydropyrimidin-1(2*H*)-yl)-5-fluoro-1-methyl-1*H*-indazol-6-yl)non-8-yn-1-yl)-3-methyl-1*H*-indazole-6-carboxamido)-2-(1-methylpyrrolidin-2-yl)-1*H*-pyrrolo[3,2-c]pyridine-1-carboxylate**

To a solution of 1-(9-(3-(2, 4-dioxo-3-((2-(trimethylsilyl) ethoxy)methyl)tetrahydropyrimidin-1(2*H*)-yl)-5-fluoro-1-methyl-1*H*-indazol-6-yl) non-8-yn-1-yl)-3-methyl-1*H*-indazole-6-carboxamide **(Intermediate 292,** 0.50 g, 0.73 mmol) in 1,4-dioxane (10 mL) was added *tert*-butyl (R)-6-bromo-2-(1-methylpyrrolidin-2-yl)-1*H*-pyrrolo[3,2-c]pyridine-1-carboxylate **(Intermediate 1,** 0.30 g, 0.79 mmol) followed by cesium carbonate (0.35 g, 1.82 mmol). The reaction mixture was purged with argon for 15 min then $Pd_2(dba)_3$ (0.10 g, 0.11 mmol) and Xantphos (0.13 g, 0.22 mmol) were added. The reaction mixture was further purged with argon for 10 min and then heated at 100°C for 2h. The reaction mixture was concentrated *in vacuo* and the crude material was purified by combi-flash chromatography, by eluting with 70% ethyl acetate in heptane, to afford *tert*-butyl (*R*)-6-(1-(9-(3-(2,4-dioxo-3-((2-(trimethylsilyl)ethoxy)methyl)tetrahydropyrimidin-1(2*H*)-yl)-5-fluoro-1-methyl-1*H*-indazol-6-yl)non-8-yn-1-yl)-3-methyl-1*H*-indazole-6-carboxamido)-2-(1-methylpyrrolidin-2-yl)-1*H*-pyrrolo[3,2-c]pyridine-1-carboxylate **(Intermediate 293,** 0.56 g, 78%) as a yellow solid. $^1$H NMR (400 MHz, DMSO-$d_6$) δ ppm -0.02 (s, 9H), 0.82-0.90 (m, 2H), 1.25-1.48 (m, 6H), 1.51-1.62 (m, 2H), 1.70 (s, 9H), 1.73-1.78 (m, 2H), 1.85-1.88 (m, 3H), 2.32-2.34 (m, 1H), 2.35 (s, 3H), 2.49 (s, 3H), 2.53-2.56 (m, 2H), 2.91-2.94 (m, 2H), 3.12-3.16 (m, 1H), 3.56-3.60 (m, 3H), 3.90-3.94 (m, 3H), 3.97 (s, 3H), 4.40-4.43 (m, 2H), 5.14 (s, 2H), 6.75 (s, 1H), 7.35-7.43 (m, 1H), 7.69-7.74 (m, 1H), 7.76-7.84 (m, 2H), 8.43 (s, 1H), 8.59 (s, 1H), 8.97 (s, 1H), 10.76 (br s, 1H).

**Step 7**

**Example 65: (*R*)-1-(9-(3-(2,4-dioxotetrahydropyrimidin-1(2*H*)-yl)-5-fluoro-1-methyl-1*H*-indazol-6-yl)non-8-yn-1-yl)-3-methyl-*N*-(2-(1-methylpyrrolidin-2-yl)-1*H*-pyrrolo[3,2-c]pyridin-6-yl)-1*H*-indazole-6-carboxamide**

To a solution of *tert*-butyl-(*R*)-6-(1-(9-(3-(2,4-dioxo-3-((2-(trimethylsilyl)ethoxy)methyl)tetrahydropyrimidin-1(2*H*)-yl)-5-fluoro-1-methyl-1*H*-indazol-6-yl)non-8-yn-1-yl)-3-methyl-1*H*-indazole-6-carboxamido)-2-(1-methylpyrrolidin-2-yl)-1*H*-pyrrolo[3,2-c]pyridine-1-carboxylate **(Intermediate 293,** 0.56 g, 0.57 mmol) in acetonitrile (15 mL) was added methanesulfonic acid (0.37 mL, 5.67 mmol) and the reaction mixture was heated at 50°C for 2h. The reaction mixture was cooled to room temperature, then *N*,*N*'-dimethylethylenediamine (0.34 mL, 2.83 mmol) followed by triethylamine (1.59 mL, 11.35 mmol) were added and the mixture stirred at room temperature for 3h then concentrated *in vacuo.* The obtained residue was diluted with water (50 mL) and the resultant precipitate was collected by filtration and dried *in vacuo.* The crude material was purified by preparative HPLC (Method A) to afford (*R*)-1-(9-(3-(2,4-dioxotetrahydropyrimidin-1(2*H*)-yl)-5-fluoro-1-methyl-1*H*-indazol-6-yl)non-8-yn-1-yl)-3-methyl-N (2-(1-methylpyrrolidin-2-yl)-1H-pyrrolo[3,2-c]pyridin-6-yl)-1*H*-indazole-6-carboxamide **(Example 65,** 0.075 g, 17%) as an off-white solid. $^1$H NMR (400 MHz, DMSO-$d_6$) δ ppm 1.27-1.48 (m, 7H), 1.51-1.60 (m, 2H), 1.82-1.95 (m, 5H), 2.12-2.16 (m, 1H), 2.18 (s, 3H), 2.24-2.30 (m, 1H), 2.51 (s, 3H), 2.73-2.77 (m, 2H), 3.12-3.18 (m, 1H), 3.34-3.45 (m, 2H), 3.88-3.92 (m, 2H), 3.96 (s, 3H), 4.38-4.44 (m, 2H), 6.40 (s, 1H), 7.43-7.45 (m, 1H), 7.70-7.83 (m, 3H), 8.25 (s, 1H), 8.44 (s, 1H), 8.51 (s, 1H), 10.52 (s, 1H), 10.57 (s, 1H), 11.38 (s, 1H). Mass spec m/z 756.9 $[M+H]^+$.

**Example 66 was synthesized following Scheme 64**

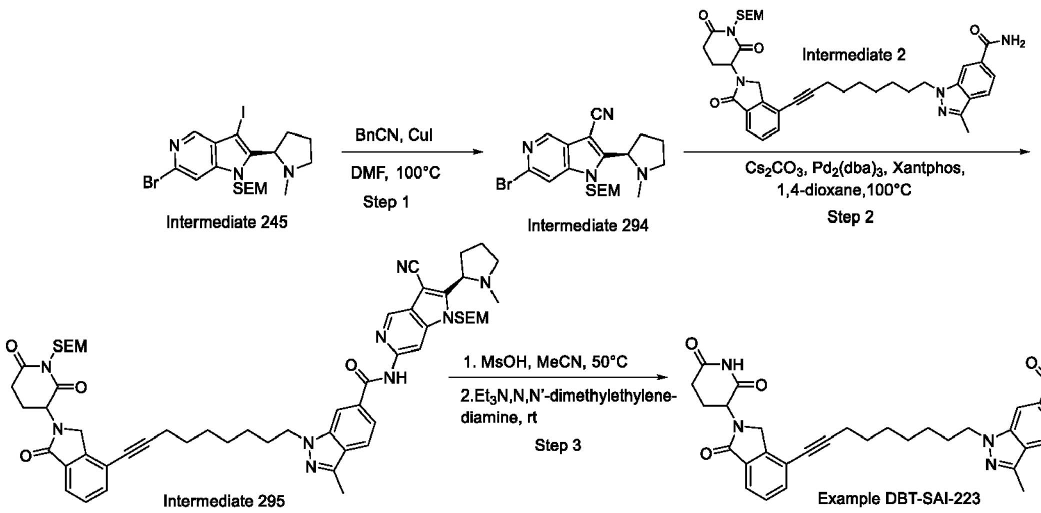


**Scheme 64**

**Step 1**

**Intermediate 294: (*R*)-6-bromo-2-(1-methylpyrrolidin-2-yl)-1-((2-(trimethylsilyl)ethoxy)methyl)-1*H*-pyrrolo [3, 2-c] pyridine-3-carbonitrile**

To a solution of (*R*)-6-bromo-3-iodo-2-(1-methylpyrrolidin-2-yl)-1-((2-(trimethylsilyl)ethoxy)methyl)-1*H*-pyrrolo [3,2-c]pyridine **(Intermediate 245,** 1.00 g, 1.9 mmol) in dimethylformamide (10 mL) was added copper (I) iodide (0.43 g, 2.2 mmol) and benzyl cyanide (1.8 g, 15.0 mmol). The reaction mixture was heated at 100°C for 16h. The reaction mixture was diluted with water (100 mL) and extracted with ethyl acetate (3 × 100 mL). The organic layer was separated, dried over anhydrous $Na_2SO_4$, filtered, and concentrated *in vacuo.* The crude material was purified by combi-flash chromatography, by eluting with 30% ethyl acetate in heptane, to afford (*R*)-6-bromo-2-(1-methylpyrrolidin-2-yl)-1-((2-(trimethylsilyl)ethoxy) methyl)-1*H*-pyrrolo[3,2-c]pyridine-3-carbonitrile **(Intermediate 294, 0.2** g, 20%) as a yellow liquid. $^1H$ NMR (400 MHz, DMSO-$d_6$) δ ppm -0.08 (s, 9H), 0.81-0.84 (m, 2H), 1.80-1.97 (m, 3H), 2.26 (s, 3H), 2.34-2.36 (m, 2H), 3.16-3.22 (m, 1H), 3.51-3.59 (m, 2H), 3.78-3.82 (m, 1H), 5.74-5.78 (m, 2H), 8.12 (s, 1H), 8.68 (s, 1H). Mass spec m/z 435.1 $[M+H]^+$.

**Step 2**

**Intermediate 295: N (3-cyano-2-((R)-1-methylpyrrolidin-2-yl)-1-((2-(trimethylsilyl)ethoxy)methyl)-1*H*-pyrrolo [3,2-c]pyridin-6-yl)-1-(9-(2-(2,6-dioxo-1-((2-(trimethylsilyl)ethoxy)methyl)piperidin-3-yl)-1-oxoisoindolin-4-yl) non-8-yn-1-yl)-3-methyl-1*H*-indazole-6-carboxamide**

To a solution of (*R*)-6-bromo-2-(1-methylpyrrolidin-2-yl)-1-((2-(trimethylsilyl)ethoxy)methyl)-1*H*-pyrrolo[3,2-c] pyridine-3-carbonitrile **(Intermediate 294,** 0.25 g, 0.57 mmol) in 1,4-dioxane (3 mL) was added 1-(9-(2-(2,6-dioxo-1-((2-(trimethylsilyl)ethoxy)methyl)piperidin-3-yl)-1-oxoisoindolin-4-yl)non-8-yn-1-yl)-3-methyl-1*H*-indazole-6-carboxamide **(Intermediate 2,** 0.30 g, 0.45 mmol) followed by cesium carbonate (0.56 g, 1.72 mmol) at room temperature. The reaction mixture was purged with argon for 15 min followed by addition of $Pd_2(dba)_3$ (0.10 g, 0.11 mmol) and Xantphos (0.06 g, 0.11 mmol). The reaction mixture was further purged with argon for another 10 min and heated at 100°C for 2h. The reaction mixture was filtered through celite bed and washed with ethyl acetate (100 mL). The filtrate was concentrated *in vacuo.* The crude material was washed with heptane and dried *in vacuo* to afford *N*-(3-cyano-2-((*R*)-1-methylpyrrolidin-2-yl)-1-((2-(trimethylsilyl)ethoxy)methyl)-1*H*-pyrrolo[3,2-c]pyridin-6-yl)-1-(9-(2-(2,6-dioxo-1-((2-(trimethylsilyl)ethoxy) methyl)piperidin-3-yl)-1-oxoisoindolin-4-yl)non-8-yn-1-yl)-3-methyl-1*H*-indazole-6-carboxamide **(Intermediate 295, 0.23** g) as yellow solid, which was carried forward into next step without further purification. Mass spec m/z 1023.6 $[M+H]^+$.

**Step 3**

**Example 66: *N*-(3-cyano-2-((*R*)-1-methylpyrrolidin-2-yl)-1*H*-pyrrolo[3,2-c]pyridin-6-yl)-1-(9-(2-(2,6-dioxopiperidin-3-yl)-1-oxoisoindolin-4-yl)non-8-yn-1-yl)-3-methyl-1*H*-indazole-6-carboxamide**

To a cooled (0°C) solution of *N*-(3-cyano-2-((*R*)-1-methylpyrrolidin-2-yl)-1-((2-(trimethylsilyl)ethoxy)methyl)-1*H*-

pyrrolo[3,2-c]pyridin-6-yl)-1-(9-(2-(2,6-dioxo-1-((2-(trimethylsilyl)ethoxy)methyl)piperidin-3-yl)-1-oxoisoindolin-4-yl) non-8-yn-1-yl)-3-methyl-1*H*-indazole-6-carboxamide **(Intermediate 295,** 0.23 g, 0.22 mmol) in acetonitrile (2 mL) was added methanesulfonic acid (0.22 mL, 3.37 mmol) and the reaction then heated at 50°C for 2h. The reaction mixture was cooled to room temperature then *N,N'*-dimethylethylenediamine (0.21 mL, 1.80 mmol) and triethylamine (0.63 mL, 4.49 mmol) were added. The reaction mixture was stirred at room temperature for 3h then concentrated *in vacuo.* The crude compound was diluted with ice cold water (80 mL), the resultant precipitate collected by filtration and dried *in vacuo.* The crude material was purified by preparative HPLC (Method A) to afford *N*-(3-cyano-2-((*R*)-1-methylpyrrolidin-2-yl)-1*H*-pyrrolo[3,2-c]pyridin-6-yl)-1-(9-(2-(2,6-dioxopiperidin-3-yl)-1-oxoisoindolin-4-yl)non-8-yn-1-yl)-3-methyl-1*H*-indazole-6-carboxamide **(Example 66,** 0.006 g, 2%) as an off white solid. $^1$H NMR (400 MHz, DMSO-$d_6$) δ ppm 1.24-1.45 (m, 6H), 1.50-1.59 (m, 2H), 1.82-2.02 (m, 6H), 2.24 (s, 3H), 2.26-2.31 (m, 1H), 2.36-2.44 (m, 2H), 2.45 (s, 3H), 2.55-2.61 (m, 2H), 2.84-2.95 (m, 1H), 3.19-3.22 (m, 2H), 3.58-3.62 (m, 1H), 4.24-4.35 (m, 1H), 4.36-4.46 (m, 3H), 5.10-5.15 (m, 1H), 7.47-7.52 (m, 1H), 7.57-7.60 (m, 1H), 7.68-7.76 (m, 2H), 7.77-7.81 (m, 1H), 8.38-8.41 (s, 1H), 8.45 (s, 1H), 8.67 (s, 1H), 10.81 (br s, 1H), 10.99 (br s, 1H), 12.52 (br s, 1H). Mass spec m/z 764.0 $[M+H]^+$.

**Example 67: *N*-(3-chloro-2-((*R*)-1-methylpyrrolidin-2-yl)-1*H*-pyrrolo[3,2-c]pyridin-6-yl)-1-(9-(2-(2,6-dioxopiperidin-3-yl)-7-fluoro-1-oxoisoindolin-4-yl)non-8-yn-1-yl)-3-methyl-1H indazole-6-carboxamide**

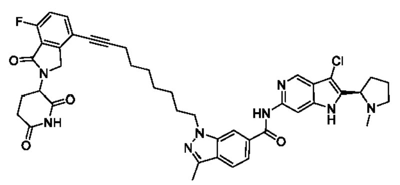

To a solution of 1-(9-(2-(2,6-dioxopiperidin-3-yl)-7-fluoro-1-oxoisoindolin-4-yl)non-8-yn-1-yl)-3-methyl-*N*-(2-((*R*)-1-methylpyrrolidin-2-yl)-1*H*-pyrrolo[3,2-*c*]pyridin-6-yl)-1*H*-indazole-6-carboxamide **(Example 52,** 0.20 g, 0.3 mmol) in dimethylformamide (2.0 mL) was added *N*-chlorosuccinimide (0.05 g, 0.4 mmol) and the reaction mixture was stirred at room temperature for 6h. The reaction mixture was diluted with water (80 mL) and extracted with ethyl acetate (3 × 100 mL). The organic layer was separated, dried over anhydrous $Na_2SO_4$, filtered and concentrated *in vacuo.* The crude material was purified by preparative HPLC (Method A) to afford *N*-(3-chloro-2-((*R*)-1-methylpyrrolidin-2-yl)-1*H*-pyrrolo[3,2-c]pyridin-6-yl)-1-(9-(2-(2,6-dioxopiperidin-3-yl)-7-fluoro-1-oxoisoindolin-4-yl)non-8-yn-1-yl)-3-methyl-1*H*-indazole-6-carboxamide **(Example 67,** 0.04 g, 20%) as an off white solid. $^1$H NMR (400 MHz, DMSO-$d_6$) δ ppm 1.28-1.40 (m, 6H), 1.51-1.56 (m, 2H), 1.85-1.99 (m, 6H), 2.17 (s, 3H), 2.18-2.20 (m, 1H), 2.30-2.33 (m, 1H), 2.42 (s, 3H), 2.43-2.47 (m, 1H), 2.56-2.64 (m, 3H), 2.84-2.96 (m, 1H), 3.16-3.20 (m, 1H), 3.51-3.55 (m, 1H), 4.27-4.32 (m, 1H), 4.38-4.47 (m, 3H), 5.06-5.12 (m, 1H), 7.26 (d, *J*=8.80 Hz, 1H), 7.61-7.64 (m, 1H), 7.71-7.80 (m, 2H), 8.31 (s, 1H), 8.44 (s, 1H), 8.51 (s, 1H), 10.67 (s, 1H), 10.99 (s, 1H), 11.70 (s, 1H). Mass spec m/z 791.0 $[M+H]^+$.

**Example 68 was synthesized following Scheme 65**

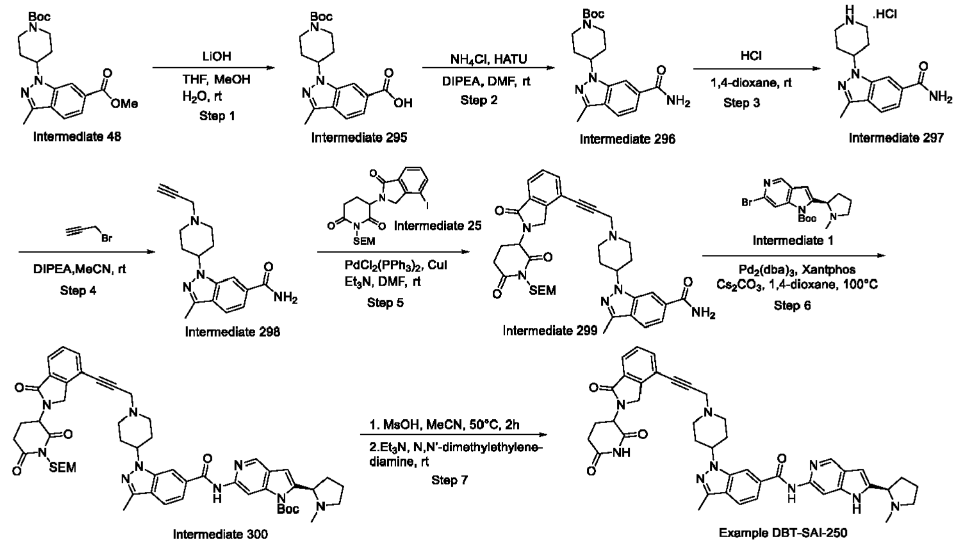


## Scheme 65

### Step 1

#### Intermediate 295: 1-(1-(*tert*-butoxycarbonyl) piperidin-4-yl)-3-methyl-1*H*-indazole-6-carboxylic acid

To a solution of methyl 1-(1-(*tert*-butoxycarbonyl)piperidin-4-yl)-3-methyl-1H-indazole-6-carboxylate **(Intermediate 48,** 14.0 g, 37.49 mmol) in tetrahydrofuran (50 mL), methanol (50 mL) and water (50 mL) was added lithium hydroxide (2.70 g, 112.5 mmol). The reaction mixture was stirred at room temperature for 16h then concentrated *in vacuo.* The obtained residue was diluted with water (100 mL) and extracted with dichloromethane (2 × 50 mL). The aqueous layer was acidified to pH~5 by addition of aqueous citric acid solution. After stirring, for 10 min, the resultant precipitate was collected by filtration and dried *in vacuo* to afford 1-(1-(*tert*-butoxycarbonyl) piperidin-4-yl)-3-methyl-1*H*-indazole-6-carboxylic acid **(Intermediate 295,** 12.5 g, 93%) as an off-white solid which was used for the next step without further purification. $^1$H NMR (400 MHz, DMSO-$d_6$) δ ppm 1.43 (s, 9H), 1.86-1.97 (m, 4H), 2.52 (s, 3H), 2.98-3.04 (m, 2H), 4.06-4.10 (m, 2H), 4.84-4.93 (m, 1H), 7.65-7.69 (m, 1H), 7.70-7.74 (m, 1H), 8.26 (s, 1H). Mass spec m/z 360.30 [M+H]$^+$.

### Step 2

#### Intermediate 296: *tert*-butyl 4-(6-carbamoyl-3-methyl-1*H*-indazol-1-yl) piperidine-1-carboxylate

To a solution of 1-(1-(*tert*-butoxycarbonyl) piperidin-4-yl)-3-methyl-1*H*-indazole-6-carboxylic acid **(Intermediate 295,** 12.5 g, 34.8 mmol) in dimethylformamide (130 mL) was added HATU (26.4 g, 69.6 mmol) and the reaction mixture stirred at room temperature for 15 min. Ammonium chloride (9.30 g, 174.0 mmol) and DIPEA (20 mL, 104.0 mmol) were then added and the reaction mixture was stirred at room temperature for 16h. The reaction mixture was then poured into ice cold water (250 mL), the resultant precipitate was collected by filtration and dried *in vacuo* to afford *tert*-butyl 4-(6-carbamoyl-3-methyl-1*H*-indazol-1-yl) piperidine-1-carboxylate **(Intermediate 296,** 11.0 g, 88%) as an off white solid, which was used for next step without further purification. $^1$H NMR (400 MHz, DMSO-$d_6$) δ ppm 1.43 (s, 9H), 1.90-1.98 (m, 4H), 2.53 (s, 3H), 2.97-3.14 (m, 2H), 4.04-4.14 (m, 2H), 4.73-4.86 (m, 1H), 7.43 (br s, 1H), 7.59 (d, *J*=8.80 Hz, 1H), 7.73 (d, *J*=8.80 Hz, 1H), 8.04 (br s, 1H), 8.19 (s, 1H). Mass spec m/z 359.20 [M+H]$^+$.

### Step 3

#### Intermediate 297: 3-methyl-1-(piperidin-4-yl)-1*H*-indazole-6-carboxamide hydrochloride

To a solution of *tert*-butyl 4-(6-carbamoyl-3-methyl-1*H*-indazol-1-yl) piperidine-1-carboxylate **(Intermediate 296, 9.00** g, 25.1 mmol) in 1, 4-dioxane (90 mL) was added 4M HCl in 1,4-dioxane (250 mL). The reaction mixture was stirred at room temperature for 16h. The reaction mixture was concentrated *in vacuo* and the obtained solid washed with diethyl ether (2 × 100 mL) and dried *in vacuo* to afford 3-methyl-1-(piperidin-4-yl)-1*H*-indazole-6-carboxamide

hydrochloride **(Intermediate 297,** 9.00 g, 85%) as an off-white solid, which was used for the next step without further purification. $^1$H NMR (400 MHz, DMSO-$d_6$) δ ppm 2.01-2.10 (m, 2H), 2.29-2.41 (m, 2H), 2.50 (s, 3H), 3.05-3.18 (m, 2H), 3.38-3.44 (m, 2H), 4.92-4.98 (m, 1H), 7.44 (br s, 1H), 7.61 (d, *J*=8.20 Hz, 1H), 7.73 (d, J=8.20 Hz, 1H), 8.09 (br s, 1H), 8.31 (s, 1H), 9.08 (br s, 1H), 9.27 (br s, 1H). Mass spec m/z 259.30 $[M+H]^+$.

**Step 4**

**Intermediate 298: 3-methyl-1-(1-(prop-2-yn-1-yl)piperidin-4-yl)-1*H*-indazole-6-carboxamide**

To a cooled (0°C) solution of 3-methyl-1-(piperidin-4-yl)-1*H*-indazole-6-carboxamide hydrochloride **(Intermediate 297,** 1.00 g, 3.0 mmol) in acetonitrile (10 mL) was added DIPEA (1.00 g, 10.0 mmol) followed by 3-bromoprop-1-yne (CAS No: 106-96-7, 0.40 g, 3.0 mmol). The reaction mixture was stirred at room temperature for 1h then poured into ice cold water (80 mL) and extracted with ethyl acetate (3 × 100 mL). The combined organic layers were dried over anhydrous $Na_2SO_4$, filtered and concentrated *in vacuo.* The crude material was purified by combi-flash column chromatography, by eluting with 7% of MeOH in DCM, to afford 3-methyl-1-(1-(prop-2-yn-1-yl) piperidin-4-yl)-1*H*-indazole-6-carboxamide **(Intermediate 298,** 0.50 g, 30%) as an off white solid. $^1$H NMR (400 MHz, DMSO-$d_6$) δ ppm 1.88-1.94 (m, 2H), 2.09-2.18 (m, 2H), 2.36-2.46 (m, 2H), 2.49 (s, 3H), 2.92-2.96 (m, 2H), 3.15 (s, 1H), 3.37 (s, 2H), 4.46-4.58 (m, 1H), 7.41 (br s, 1H), 7.60 (d, *J*=8.40 Hz, 1H), 7.72 (d, *J*=8.40 Hz, 1H), 8.03 (br s, 1H), 8.20 (s, 1H). Mass spec m/z 297.18 $[M+H]^+$.

**Step 5**

**Intermediate 299: 1-(1-(3-(2-(2,6-dioxo-1-((2-(trimethylsilyl)ethoxy)methyl)piperidin-3-yl)-1-oxoisoindolin-4-yl) prop-2-yn-1-yl) piperidin-4-yl)-3-methyl-1*H*-indazole-6-carboxamide**

To a solution of 3-methyl-1-(1-(prop-2-yn-1-yl)piperidin-4-yl)-1*H*-indazole-6-carboxamide **(Intermediate 298,** 0.5 g, 1.68 mmol) in dimethylformamide (1 mL) was added 3-(4-iodo-1-oxoisoindolin-2-yl)-1-((2-(trimethylsilyl)ethoxy) methyl)piperidine-2,6-dione **(Intermediate 25, 0.84** g, 1.68 mmol) followed by triethylamine (8.74 mL, 62.42 mmol). The reaction mixture was purged with argon for 15 min, then copper (I) iodide (0.05 g, 0.25 mmol) and $PdCl_2(PPh_3)_2$ (0.18 g, 0.25 mmol) were added. The reaction mixture was purged with argon for another 10 min and then stirred at room temperature for 1h. The reaction mixture was then quenched with ice cold water (80 mL) and extracted with ethyl acetate (3 × 50 mL). The combined organic layers were dried over anhydrous $Na_2SO_4$ and concentrated *in vacuo.* The crude material was purified by combi-flash column chromatography, by eluting with 7% MeOH in DCM, to afford 1-(1-(3-(2-(2, 6-dioxo-1-((2-(trimethylsilyl) ethoxy) methyl) piperidin-3-yl)-1-oxoisoindolin-4-yl) prop-2-yn-1-yl) piperidin-4-yl)-3-methyl-1*H*-indazole-6-carboxamide **(Intermediate 299, 0.38** g, 34%) as a yellow solid. $^1$H NMR (400 MHz, DMSO-$d_6$) δ ppm -0.05 (s, 9H), 0.75-0.88 (m, 2H), 1.92-1.96 (m, 2H), 2.06-2.23 (m, 3H), 2.44-2.47 (m, 2H), 2.53 (s, 3H), 2.76-2.80 (m, 2H), 2.98-3.09 (m, 3H), 3.45-3.57 (m, 2H), 3.69 (s, 2H), 4.31-4.42 (m, 1H), 4.52-4.64 (m, 2H), 5.00-5.10 (m, 2H), 5.25-5.30 (m, 1H), 7.41 (br s, 1H), 7.55-7.63 (m, 2H), 7.68-7.81 (m, 3H), 8.00 (br s, 1H), 8.20 (s, 1H). Mass spec m/z 669.10 $[M+H]^+$.

**Step 6**

**Intermediate 300: tert-butyl 6-(1-(1-(3-(2-(2,6-dioxo-1-((2-(trimethylsilyl)ethoxy)methyl)piperidin-3-yl)-1-oxoisoindolin-4-yl)prop-2-yn-1-yl)piperidin-4-yl)-3-methyl-1*H*-indazole-6-carboxamido)-2-((*R*)-1-methylpyrrolidin-2-yl)-1*H*-pyrrolo[3,2-c]pyridine-1-carboxylate**

To a solution of 1-(1-(3-(2-(2, 6-dioxo-1-((2-(trimethylsilyl) ethoxy) methyl) piperidin-3-yl)-1-oxoisoindolin-4-yl) prop-2-yn-1-yl) piperidin-4-yl)-3-methyl-1*H*-indazole-6-carboxamide **(Intermediate 299,** 0.34 g, 0.50 mmol) in 1,4-dioxane (3 mL) was added *tert*-butyl (*R*)-6-bromo-2-(1-methylpyrrolidin-2-yl)-1*H*-pyrrolo[3,2-c]pyridine-1-carboxylate **(Intermediate 1,** 0.23 g, 0.61 mmol) followed by cesium carbonate (0.34 g, 1.01 mmol). The reaction mixture was purged with argon for 15 min, then $Pd_2(dba)_3$ (0.06 g, 0.07 mmol) and Xantphos (0.09 g, 0.15 mmol) were added. The reaction mixture was further purged with argon for 10 min and stirred at 100°C for 2h. The reaction mixture was concentrated *in vacuo* and the crude material was purified by combi-flash chromatography, by eluting with 7% MeOH in DCM, to afford tert-butyl 6-(1-(1-(3-(2-(2,6-dioxo-1-((2-(trimethylsilyl)ethoxy)methyl)piperidin-3-yl)-1-oxoisoindolin-4-yl)prop-2-yn-1-yl)piperidin-4-yl)-3-methyl-1*H*-indazole-6-carboxamido)-2-((*R*)-1-methylpyrrolidin-2-yl)-1*H*-pyrrolo[3,2-c]pyridine-1-carboxylate **(Intermediate 300,** 0.25 g, 31%) as yellow gummy solid. $^1$H NMR (400 MHz, DMSO-$d_6$) δ ppm -0.07 (s, 9H), 0.75-0.82 (m, 2H), 1.64-1.68 (m, 2H), 1.70 (s, 9H), 1.73-1.82 (m, 3H), 2.06-2.28 (m, 4H), 2.35 (s, 3H), 2.38-2.44 (m, 2H), 2.52 (s, 3H), 2.55-2.64 (m, 2H), 2.97-3.08 (m, 3H), 3.09-3.17 (m, 2H), 3.45-3.53 (m, 2H), 3.71 (s, 2H), 3.87-3.96 (m, 1H), 4.34-4.38 (m, 1H), 4.54-4.70 (m, 2H), 4.96-5.07 (m, 2H), 5.21-5.30 (m, 1H), 6.75 (s, 1H), 7.56-7.60 (m, 1H), 7.68-7.74 (m, 1H), 7.75-7.82 (m, 3H), 8.48 (s, 1H), 8.59 (s, 1H), 8.97 (s, 1H), 10.72 (s, 1H). Mass spec m/z 968.80

[M+H]+.

**Step 7**

**Example 68: 1-(1-(3-(2-(2,6-dioxopiperidin-3-yl)-1-oxoisoindolin-4-yl)prop-2-yn-1-yl)piperidin-4-yl)-3-methyl-*N*-(2-((*R*)-1-methylpyrrolidin-2-yl)-1*H*-pyrrolo[3,2-*c*]pyridin-6-yl)-1*H*-indazole-6-carboxamide**

To a solution of *tert*-butyl 6-(1-(1-(3-(2-(2,6-dioxo-1-((2-(trimethylsilyl)ethoxy)methyl)piperidin-3-yl)-1-oxoisoindolin-4-yl)prop-2-yn-1-yl)piperidin-4-yl)-3-methyl-1H-indazole-6-carboxamido)-2-((R)-1-methylpyrrolidin-2-yl)-1H-pyrrolo[3,2-c]pyridine-1-carboxylate **(Intermediate 300, 0.23** g, 0.23 mmol) in acetonitrile (3 mL) was added methanesulfonic acid (0.24 g, 2.37 mmol) and the reaction mixture was stirred at 50°C for 2h. The reaction mixture was cooled to room temperature, then *N,N'*-dimethylethylenediamine (0.13 g, 1.42 mmol) followed by triethylamine (0.50 g, 4.75 mmol) were added and the mixture stirred at room temperature for 2h. The reaction mixture was poured into ice cold water (30 mL), the resultant precipitate was collected by filtration and dried *in vacuo.* The crude material was purified by preparative HPLC (Method A) to afford 1-(1-(3-(2-(2,6-dioxopiperidin-3-yl)-1-oxoisoindolin-4-yl)prop-2-yn-1-yl)piperidin-4-yl)-3-methyl-*N*-(2-((*R*)-1-methylpyrrolidin-2-yl)-1*H*-pyrrolo[3,2-*c*]pyridin-6-yl)-1*H*-indazole-6-carboxamide **(Example 68,** 0.04 g, 19%) as white solid. $^1$H NMR (400 MHz, DMSO-$d_6$) $\delta$ ppm 1.76-1.95 (m, 3H), 1.97-2.05 (m, 2H), 2.06-2.14 (m, 2H), 2.17 (s, 3H), 2.20-2.30 (m, 2H), 2.40-2.46 (m, 2H), 2.53 (s, 3H), 2.54-2.65 (m, 3H), 2.81-2.91 (m, 1H), 3.01-3.08 (m, 2H), 3.11-3.18 (m, 1H), 3.32-3.35 (m, 1H), 3.72 (s, 2H), 4.37-4.43 (m, 1H), 4.52-4.58 (m, 1H), 4.64-4.73 (m, 1H), 5.11-5.16 (m, 1H), 6.40 (s, 1H), 7.55-7.60 (m, 1H), 7.71-7.81 (m, 4H), 8.25 (s, 1H), 8.49 (s, 1H), 8.50 (s, 1H), 10.49 (s, 1H), 10.98 (s, 1H), 11.38 (s, 1H). Mass spec m/z 738.20 [M+H]+.

**Example 69 was synthesized following Scheme 66**

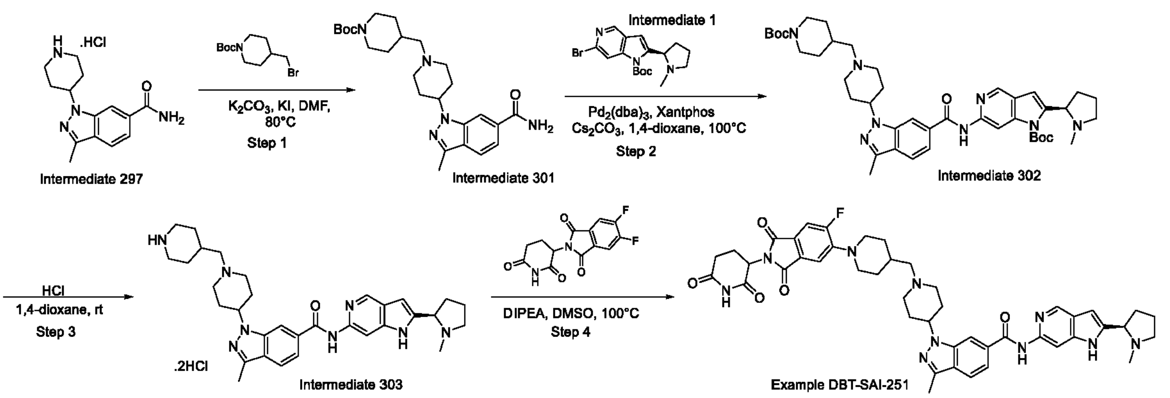


**Scheme 66**

**Step 1**

**Intermediate 301: *tert*-butyl 4-((4-(6-carbamoyl-3-methyl-1*H*-indazol-1-yl) piperidin-1-yl) methyl) piperidine-1-carboxylate**

To a solution of 3-methyl-1-(4-piperidyl) indazole-6-carboxamide hydrochloride **(Intermediate 297,** 5.00 g, 17.0 mmol) in dimethylformamide (50 mL) was added *tert*-butyl 4-(bromomethyl)piperidine-1-carboxylate (CAS No: 158407-04-6, 5.66 g, 20.4 mmol) followed by potassium carbonate (7.03 g, 50.9 mmol) and potassium iodide (0.28 g, 1.70 mmol). The reaction mixture was stirred at 80°C for 16h then concentrated *in vacuo.* The obtained residue was poured into ice cold water (800 mL), the resultant precipitate was collected by filtration and dried *in vacuo* to afford *tert*-butyl 4-((4-(6-carbamoyl-3-methyl-1*H*-indazol-1-yl)piperidin-1-yl)methyl)piperidine-1-carboxylate **(Intermediate 297,** 3.90 g, 50%) as an off white solid which was used for the next step without further purification. $^1$H NMR (400 MHz, DMSO-$d_6$) $\delta$ ppm 1.39 (s, 9H), 1.62-1.76 (m, 4H), 1.82-1.90 (m, 2H), 2.06-2.20 (m, 5H), 2.52 (s, 3H), 2.60-2.80 (m, 3H), 2.87-3.05 (m, 2H), 3.85-4.05 (m, 3H), 4.46-4.60 (m, 1H), 7.41 (br s, 1H), 7.58-7.62 (m, 1H), 7.70-7.74 (m, 1H), 8.03 (br s, 1H), 8.18 (s, 1H). Mass spec m/z 456.3 [M+H]+.

**Step 2**

**Intermediate 302: *tert*-butyl (*R*)-6-(1-(1-((1-(*tert*-butoxycarbonyl) piperidin-4-yl) methyl) piperidin-4-yl)-3-methyl-1*H*-indazole-6-carboxamido)-2-(1-methylpyrrolidin-2-yl)-1*H*-pyrrolo[3, 2-*c*] pyridine-1-carboxylate**

To a solution of *tert*-butyl 4-((4-(6-carbamoyl-3-methyl-1*H*-indazol-1-yl)piperidin-1-yl)methyl)piperidine-1-carboxylate **(Intermediate 301,** 1.92 g, 4.21 mmol) in 1,4-dioxane (20 mL) was added *tert*-butyl (*R*)-6-bromo-2-(1-methylpyrrolidin-2-yl)-1*H*-pyrrolo[3,2-c]pyridine-1-carboxylate **(Intermediate 1,** 2.00 g, 5.26 mmol) followed by cesium carbonate (4.06 g, 21.0 mmol). The reaction mixture was purged with argon for 15 min, then $Pd_2(dba)_3$ (0.74 g, 0.78 mmol) and Xantphos (0.94 g, 1.58 mmol) were added. The reaction mixture was purged with argon for a further 10 min and then stirred at 100°C for 2h. The reaction mixture was filtered through celite, the filter pad was washed with ethyl acetate (100 mL) and the filtrate was concentrated *in vacuo.* The crude material was purified by combi-flash chromatography, by eluting with 10% MeOH in DCM, to afford *tert*-butyl (*R*)-6-(1-(1-((1-(*tert*-butoxycarbonyl)piperidin-4-yl)methyl)piperidin-4-yl)-3-methyl-1*H*-indazole-6-carboxamido)-2-(1-methylpyrrolidin-2-yl)-1*H*-pyrrolo[3,2-*c*]pyridine-1-carboxylate **(Intermediate 302,** 1.60 g, 40%) as a yellow solid. $^1H$ NMR (400 MHz, DMSO-$d_6$) δ ppm 0.89-1.03 (m, 2H), 1.07-1.10 (m, 1H), 1.39 (s, 9H), 1.55-1.66 (m, 3H), 1.70 (s, 9H), 1.75-1.80 (m, 3H), 1.88-1.94 (m, 2H), 2.07-2.24 (m, 6H), 2.28-2.33 (m, 1H), 2.35 (s, 3H), 2.39-2.44 (m, 1H), 2.52 (s, 3H), 2.92-3.02 (m, 2H), 3.08-3.16 (m, 1H), 3.36-3.42 (m, 1H), 3.85-4.00 (m, 3H), 4.60-4.70 (m, 1H), 6.77 (s, 1H), 7.71 (d, *J*=8.40 Hz, 1H), 7.77 (d, *J*=8.40 Hz, 1H), 8.45 (s, 1H), 8.61 (s, 1H), 8.99 (s, 1H), 10.78 (br s, 1H). Mass spec m/z 755.5 $[M+H]^+$.

**Step 3**

**Intermediate 303: (*R*)-3-methyl-*N*-(2-(1-methylpyrrolidin-2-yl)-1*H*-pyrrolo[3,2-c]pyridin-6-yl)-1-(1-(piperidin-4-ylmethyl)piperidin-4-yl)-1*H*-indazole-6-carboxamide dihydrochloride**

To a cooled (0°C) solution of *tert*-butyl (*R*)-6-(1-(1-((1-(*tert*-butoxycarbonyl)piperidin-4-yl)methyl)piperidin-4-yl)-3-methyl-1*H*-indazole-6-carboxamido)-2-(1-methylpyrrolidin-2-yl)-1*H*-pyrrolo[3,2-c]pyridine-1-carboxylate **(Intermediate 302,** 1.50 g, 1.99 mmol) in 1,4-dioxane (18 mL) was added 4M HCl in 1,4-dioxane (20 mL, 80.0 mmol). The reaction mixture was stirred at room temperature for 16h then concentrated *in vacuo* to afford (*R*)-3-methyl-*N*-(2-(1-methylpyrrolidin-2-yl)-1*H*-pyrrolo[3,2-c]pyridin-6-yl)-1-(1-(piperidin-4-ylmethyl)piperidin-4-yl)-1*H*-indazole-6-carboxamide dihydrochloride **(Intermediate 303,** 1.10 g) as a yellow solid which was used for the next step without further purification. $^1H$ NMR (400 MHz, DMSO-$d_6$) δ ppm 1.41-1.57 (m, 2H) 2.01-2.10 (m, 2H), 2.14-2.21 (m, 2H), 2.26 (s, 3H), 2.33-2.44 (m, 2H), 2.56 (s, 3H), 2.65-2.77 (m, 3H), 2.84-2.92 (m, 3H), 3.05-3.12 (m, 2H), 3.17-3.33 (m, 5H), 3.72-3.76 (m, 3H), 4.75-4.80 (m, 1H), 5.01-5.08 (m, 2H), 7.32 (s, 1H), 7.83-7.88 (m, 1H), 7.90-7.96 (m, 1H), 8.44-8.48 (m, 1H), 9.00 (s, 1H), 9.14 (s, 1H), 10.59 (br s, 1H), 11.65 (br s, 1H), 12.40 (br s, 1H), 13.43 (br s, 1H). Mass spec m/z 555.3 $[M+H]^+$.

**Step 4**

**Example 69: 1-(1-((1-(2-(2,6-dioxopiperidin-3-yl)-6-fluoro-1,3-dioxoisoindolin-5-yl)piperidin-4-yl)methyl)piperidin-4-yl)-3-methyl-*N*-(2-((*R*)-1-methylpyrrolidin-2-yl)-1*H*-pyrrolo[3,2-c]pyridin-6-yl)-1*H*-indazole-6-carboxamide**

To a solution of (*R*)-3-methyl-*N*-(2-(1-methylpyrrolidin-2-yl)-1*H*-pyrrolo [3, 2-c] pyridin-6-yl)-1-(1-(piperidin-4-ylmethyl) piperidin-4-yl)-1*H*-indazole-6-carboxamide dihydrochloride **(Intermediate 303,** 0.30 g, 0.51 mmol) in dimethyl sulfoxide (8 mL) was added 2-(2,6-dioxopiperidin-3-yl)-5,6-difluoroisoindoline-1,3-dione (CAS No: 1496997-41-1, 0.15 g, 0.51 mmol) followed by DIPEA (0.44 mL, 2.5 mmol). The reaction mixture was stirred at 100°C for 2h then concentrated *in vacuo.* The obtained residue was poured into ice cold water (80 mL), the resultant precipitate collected by filtration and dried *in vacuo* to afford the crude compound. This material was purified by preparative HPLC (Method A) to afford 1-(1-((1-(2-(2,6-dioxopiperidin-3-yl)-6-fluoro-1,3-dioxoisoindolin-5-yl)piperidin-4-yl)methyl)piperidin-4-yl)-3-methyl-*N*-(2-((*R*)-1-methylpyrrolidin-2-yl)-1*H*-pyrrolo[3,2-c]pyridin-6-yl)-1*H*-indazole-6-carboxamide **(Example 69,** 0.012 g, 3%) as a yellow solid. $^1H$ NMR (400 MHz, DMSO-$d_6$) δ ppm 1.24-1.36 (m, 2H), 1.73-1.98 (m, 8H), 2.00-2.07 (m, 1H), 2.10-2.16 (m, 2H), 2.18 (s, 3H), 2.20-2.25 (m, 3H), 2.26-2.31 (m, 3H), 2.46-2.48 (m, 1H), 2.53 (s, 3H), 2.55-2.63 (m, 2H), 2.86-2.97 (m, 3H), 2.99-3.07 (m, 2H), 3.11-3.19 (m, 1H), 3.58-3.68 (m, 2H), 4.62-4.72 (m, 1H), 5.08-5.12 (m, 1H), 6.41 (s, 1H), 7.44-7.46 (m, 1H), 7.69-7.80 (m, 3H), 8.25 (s, 1H), 8.46 (s, 1H), 8.53 (s, 1H), 10.56 (br s, 1H), 11.10 (br s, 1H), 11.40 (br s, 1H). Mass spec m/z 829.1 $[M+H]^+$.

**Example 70: 1-(1-((1-(2-(2,6-dioxopiperidin-3-yl)-1,3-dioxoisoindolin-5-yl)piperidin-4-yl)methyl)piperidin-4-yl)-3-methyl-*N*-(2-((*R*)-1-methylpyrrolidin-2-yl)-1*H*-pyrrolo[3,2-c]pyridin-6-yl)-1*H*-indazole-6-carboxamide**

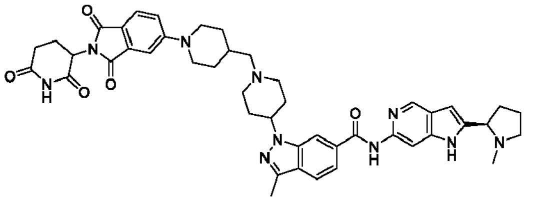

To a solution of (*R*)-3-methyl-*N*-(2-(1-methylpyrrolidin-2-yl)-1*H*-pyrrolo[3,2-c]pyridin-6-yl)-1-(1-(piperidin-4-ylmethyl)piperidin-4-yl)-1*H*-indazole-6-carboxamide dihydrochloride **(Intermediate 303,** 0.35 g, 0.63 mmol) in dimethyl sulfoxide (8.0 mL) was added 2-(2,6-dioxopiperidin-3-yl)-5-fluoroisoindoline-1,3-dione (CAS No: 835616-61-0, 0.14 g, 0.50 mmol) followed by DIPEA (3.31 mL, 18.93 mmol) and the reaction mixture was heated under microwave irradiation at 100°C for 2h. The reaction mixture was poured into ice cold water (50 mL), the resultant precipitate was collected by filtration, washed with pentane (2 × 40 mL) and dried *in vacuo.* The crude material was purified by preparative HPLC (Method A) to afford 1-(1-((1-(2-(2,6-dioxopiperidin-3-yl)-1,3-dioxoisoindolin-5-yl)piperidin-4-yl)methyl)piperidin-4-yl)-3-methyl-N (2-((R)-1-methylpyrrolidin-2-yl)-1*H*-pyrrolo[3,2-c]pyridin-6-yl)-1*H*-indazole-6-carboxamide **(Example 70,** 0.045 g, 9%) as a yellow solid. $^1$H NMR (400 MHz, DMSO-$d_6$) δ ppm 1.11-1.25 (m, 2H), 1.76-2.06 (m, 9H), 2.12-2.15 (m, 2H), 2.17 (s, 3H), 2.18-2.22 (m, 2H), 2.23-2.29 (m, 3H), 2.53 (s, 3H), 2.54-2.63 (m, 3H), 2.84-3.07 (m, 6H), 3.11-3.18 (m, 1H), 4.04-4.12 (m, 2H), 4.62-4.70 (m, 1H), 5.05-5.09 (m, 1H), 6.41 (s, 1H), 7.22-7.26 (m, 1H), 7.32 (s, 1H), 7.64-7.68 (m, 1H), 7.71-7.74 (m, 1H), 7.76-7.80 (m, 1H), 8.25 (s, 1H), 8.46 (s, 1H), 8.53 (s, 1H), 10.57 (br s, 1H), 11.09 (br s, 1H), 11.41 (br s, 1H). Mass spec m/z 811.1 [M+H]$^+$.

**Example 71: 1-(1-((1-(2-(2,6-dioxopiperidin-3-yl)-1,3-dioxoisoindolin-4-yl)piperidin-4-yl)methyl)piperidin-4-yl)-3-methyl-*N*-(2-((*R*)-1-methylpyrrolidin-2-yl)-1*H*-pyrrolo[3,2-*c*]pyridin-6-yl)-1*H*-indazole-6-carboxamide**

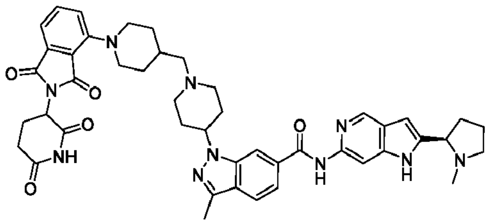

To a solution of (*R*)-3-methyl-*N*-(2-(1-methylpyrrolidin-2-yl)-1*H*-pyrrolo[3,2-*c*]pyridin-6-yl)-1-(1-(piperidin-4-ylmethyl)piperidin-4-yl)-1*H*-indazole-6-carboxamide dihydrochloride **(Intermediate 303,** 0.40 g, 0.68 mmol) in dimethyl sulfoxide (8.0 mL) was added 2-(2,6-dioxopiperidin-3-yl)-4-fluoroisoindoline-1,3-dione (CAS No: 835616-60-9, 0.19 g, 0.68 mmol) followed by DIPEA (0.59 mL, 3.4 mmol) and the reaction mixture was stirred at 100°C for 4h. The reaction mixture was poured into ice cold water (80 mL), the resultant precipitate was collected by filtration and dried *in vacuo.* The crude material was purified by preparative HPLC (Method A) to afford 1-(1-((1-(2-(2,6-dioxopiperidin-3-yl)-1,3-dioxoisoindolin-4-yl)piperidin-4-yl)methyl)piperidin-4-yl)-3-methyl-*N*-(2-((*R*)-1-methylpyrrolidin-2-yl)-1*H*-pyrrolo[3,2-*c*]pyridin-6-yl)-1*H*-indazole-6-carboxamide **(Example 71,** 0.18 g, 34%) as a yellow solid. $^1$H NMR (400 MHz, DMSO-$d_6$) δ ppm 1.30-1.43 (m, 2H), 1.72-1.83 (m, 2H), 1.84-1.97 (m, 6H), 2.00-2.06 (m, 1H), 2.10-2.16 (m, 2H), 2.18 (s, 3H), 2.19-2.32 (m, 6H), 2.53 (s, 3H), 2.56-2.63 (m, 2H), 2.86-2.95 (m, 3H), 3.02-3.08 (m, 2H), 3.10-3.20 (m, 1H), 3.32-3.37 (m, 1H), 3.70-3.74 (m, 2H), 4.66-4.70 (m, 1H), 5.07-5.12 (m, 1H), 6.41 (s, 1H), 7.31-7.36 (m, 2H), 7.66-7.74 (m, 2H), 7.76-7.80 (m, 1H), 8.25 (s, 1H), 8.47 (s, 1H), 8.53 (s, 1H), 10.55 (br s, 1H), 11.08 (br s, 1H), 11.39 (br s, 1H). Mass spec m/z 811.1 [M+H]$^+$.

**Example 72 was synthesized following Scheme 67**

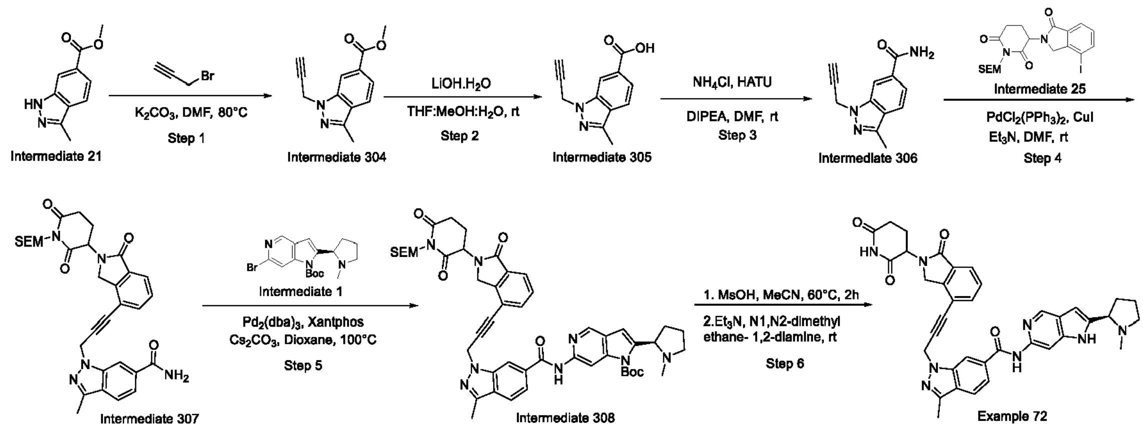


## Scheme 67

**Step** 1

**Intermediate 304: methyl 3-methyl-1-(prop-2-yn-1-yl)-1*H*-indazole-6-carboxylate**

To a solution of methyl 3-methyl-1*H*-indazole-6-carboxylate **(Intermediate 21, 5.0** g, 28.0 mmol) in dimethylformamide (50 mL) was added 3-bromoprop-1-yne (CAS No: 106-96-7, 4.4 g, 37.0 mmol) followed by potassium carbonate (12.0 g, 85.0 mmol) and the reaction mixture was stirred at 80°C for 2h. The reaction mixture was quenched with water (200 mL) and extracted with ethyl acetate (2 × 200 mL). The combined organic layers were separated, dried over anhydrous $Na_2SO_4$, filtered, and concentrated *in vacuo* to get the crude mixture of regioisomers. The regioisomers were separated by combi-flash chromatography, by eluting with 10% ethyl acetate in heptane, to afford the desired regioisomer methyl 3-methyl-1-(prop-2-yn-1-yl)-1*H*-indazole-6-carboxylate **(Intermediate 304,** 3.70 g, 57%) as a pale yellow solid. $^1$H NMR (400 MHz, DMSO-$d_6$) δ ppm 2.52 (s, 3H), 3.37 (s, 1H), 3.91 (s, 3H), 5.37 (s, 2H), 7.71 (d, *J*=8.29 Hz, 1H), 7.86 (d, J=8.29 Hz, 1H), 8.33 (s, 1H). Mass spec m/z 229.4 $[M+H]^+$.

**Step 2**

**Intermediate 305: 3-methyl-1-(prop-2-yn-1-yl)-1*H*-indazole-6-carboxylic acid**

To a solution of methyl 3-methyl-1-(prop-2-yn-1-yl)-1*H*-indazole-6-carboxylate **(Intermediate 304,** 3.70 g, 16.0 mmol) in tetrahydrofuran (20 mL), methanol (20 mL) and water (10 mL) was added lithium hydroxide (1.9 g, 81.0 mmol). The reaction mixture was stirred at room temperature for 2h then concentrated *in vacuo.* The obtained residue was poured in water (30 mL) and acidified to pH~4 with saturated solution of citric acid. The resultant precipitate was collected by filtration, washed with water (50 mL) and dried *in vacuo* to afford 3-methyl-1-(prop-2-yn-1-yl)-1*H*-indazole-6-carboxylic acid **(Intermediate 305,** 3.55 g) as an off white solid, which was used for the next step without further purification. $^1$H NMR (400 MHz, DMSO-$d_6$) δ ppm 2.54 (s, 3H), 3.36 (s, 1H), 5.34 (s, 2H), 7.69-7.71 (m, 1H), 7.80-7.84 (m, 1H), 8.29 (s, 1H). Mass spec m/z 215.1 $[M+H]^+$.

**Step 3**

**Intermediate 306: 3-methyl-1-(prop-2-yn-1-yl)-1*H*-indazole-6-carboxamide**

To a solution of 3-methyl-1-(prop-2-yn-1-yl)-1*H*-indazole-6-carboxylic acid **(Intermediate 305,** 3.55 g, 16.6 mmol) in dimethylformamide (35 mL) was added HATU (9.55 g, 24.9 mmol) and the reaction mixture was stirred for 15 min. Ammonium chloride (4.43 g, 82.9 mmol) and DIPEA (11.7 mL, 66.3 mmol) were then added and the reaction mixture was stirred at room temperature for 16h. The reaction mixture was quenched with ice cold water (200 mL) and extracted with ethyl acetate (2 × 200 mL). The combined organic layers were separated, dried over anhydrous $Na_2SO_4$, filtered, and concentrated *in vacuo.* The crude material was purified by preparative HPLC (Method A) to afford 3-methyl-1-(prop-2-yn-1-yl)-1*H*-indazole-6-carboxamide **(Intermediate 306,** 1.20 g, 34%) as an off white solid. $^1$H NMR (400 MHz, DMSO-$d_6$) δ ppm 2.49 (s, 3H), 3.35 (s, 1H), 5.27 (s, 2H), 7.45 (br s, 1H), 7.65 (d, *J*=8.20 Hz, 1H), 7.78 (d, *J*=8.20 Hz, 1H), 8.04 (br s, 1H), 8.20 (s, 1H). Mass spec m/z 214.4 $[M+H]^+$.

**Step 4**

**Intermediate 307: 1-(3-(2-(2, 6-dioxo-1-((2-(trimethylsilyl)ethoxy)methyl) piperidin-3-yl)-1-oxoisoindolin-4-yl) prop-2-yn-1-yl)-3-methyl-1*H*-indazole-6-carboxamide**

To a solution of 3-methyl-1-(prop-2-yn-1-yl)-1*H*-indazole-6-carboxamide **(Intermediate 306,** 0.60 g, 2.81 mmol) in dimethylformamide (5.0 mL) was added 3-(4-iodo-1-oxoisoindolin-2-yl)-1-((2-(trimethylsilyl)ethoxy)methyl)piperidine-2,6-dione **(Intermediate 25,** 1.40 g, 2.81 mmol) followed by triethylamine (14.6 mL, 104.11 mmol). The reaction mixture was purged with argon for 15 min, then copper (I) iodide (0.084 g, 0.42 mmol) and $PdCl_2(PPh_3)_2$ (0.20 g, 0.28 mmol) were added. The reaction mixture was purged with argon for another 10 min and stirred at room temperature for 2h. The reaction mixture was diluted with water (100 mL) and extracted with ethyl acetate (3 × 100 mL). The combined organic layers were separated, dried over $Na_2SO_4$, and concentrated *in vacuo.* The crude material was purified by combi-flash chromatography, by eluting with 60% ethyl acetate in heptane, to afford 1-(3-(2-(2,6-dioxo-1-((2-(trimethylsilyl)ethoxy) methyl)piperidin-3-yl)-1-oxoisoindolin-4-yl) prop-2-yn-1-yl)-3-methyl-1*H*-indazole-6-carboxamide **(Intermediate 307,** 0.80 g, 49%) as a white solid. $^1H$ NMR (400 MHz, DMSO-$d_6$) δ ppm -0.04 (s, 9H), 0.78-0.89 (m, 2H), 1.99-2.06 (m, 1H), 2.26-2.38 (m, 1H), 2.52 (s, 3H), 2.79-2.84 (m, 1H), 3.01-3.12 (m, 1H), 3.50-3.56 (m, 2H), 4.12-4.19 (m, 1H), 4.32-4.36 (m, 1H), 5.06 (s, 2H), 5.21-5.26 (m, 1H), 5.59 (s, 2H), 7.39 (br s, 1H), 7.51-7.56 (m, 1H), 7.66-7.68 (m, 2H), 7.74-7.76 (m, 1H), 7.78-7.81 (m, 1H), 8.08 (br s, 1H), 8.32 (s, 1H). Mass spec m/z 584.1 $[M+H]^+$.

**Step 5**

**Intermediate 308: *tert*-butyl-6-(1-(3-(2-(2,6-dioxo-1-((2-(trimethylsilyl)ethoxy)methyl)piperidin-3-yl)-1-oxoisoindolin-4-yl)prop-2-yn-1-yl)-3-methyl-1*H*-indazole-6-carboxamido)-2-((*R*)-1-methylpyrrolidin-2-yl)-1*H*-pyrrolo[3,2-*c*]pyridine-1-carboxylate**

To a solution of 1-(3-(2-(2, 6-dioxo-1-((2-(trimethylsilyl) ethoxy) methyl) piperidin-3-yl)-1-oxoisoindolin-4-yl) prop-2-yn-1-yl)-3-methyl-1H-indazole-6-carboxamide **(Intermediate 307,** 0.73 g, 1.26 mmol) in 1,4-dioxane (20 mL) was added *tert*-butyl (*R*)-6-bromo-2-(1-methylpyrrolidin-2-yl)-1*H*-pyrrolo[3,2-*c*]pyridine-1-carboxylate **(Intermediate 1,** 0.60 g, 1.57 mmol) followed by cesium carbonate (1.55 g, 4.73 mmol). The reaction mixture was purged with argon for 15 min, then $Pd_2(dba)_3$ (0.22 g, 0.23 mmol) and Xantphos (0.28 g, 0.47 mmol) were added. The reaction mixture was purged with argon for another 10 min and then stirred at 100°C for 2h. The reaction mixture was concentrated *in vacuo* and the crude residue purified by combi-flash chromatography, by eluting with 5% MeOH in dichloromethane, to afford *tert*-butyl-6-(1-(3-(2-(2,6-dioxo-1-((2-(trimethylsilyl)ethoxy)methyl)piperidin-3-yl)-1-oxoisoindolin-4-yl)prop-2-yn-1-yl)-3-methyl-1*H*-indazole-6-carboxamido)-2-((*R*)-1-methylpyrrolidin-2-yl)-1*H*-pyrrolo[3,2-*c*]pyridine-1-carboxylate **(Intermediate 308,** 0.90 g, 82%) as a yellow solid. $^1H$ NMR (400 MHz, DMSO-$d_6$) δ ppm 0.02 (s, 9H), 0.78-0.88 (m, 2H), 1.62-1.68 (m, 2H), 1.77 (s, 9H), 1.84-1.89 (m, 2H), 1.97-2.11 (m, 1H), 2.23-2.30 (m, 1H), 2.36-2.40 (m, 1H), 2.44 (s, 3H), 2.46-2.48 (m, 1H), 2.57 (s, 3H), 2.79-2.89 (m, 1H), 3.12-3.18 (m, 1H), 3.45-3.51 (m, 2H), 3.95-4.05 (m, 1H), 4.14-4.20 (m, 1H), 4.34-4.38 (m, 1H), 4.91-4.98 (m, 2H), 5.16-5.24 (m, 1H), 5.71 (s, 2H), 6.84 (s, 1H), 7.50-7.60 (m, 1H), 7.68-7.82 (m, 2H), 7.81-7.87 (m, 2H), 8.68 (s, 1H), 8.73 (s, 1H), 9.05 (s, 1H), 10.90 (br s, 1H). Mass spec m/z 884.8 $[M+H]^+$.

**Step 6**

**Example 72: 1-(3-(2-(2,6-dioxopiperidin-3-yl)-1-oxoisoindolin-4-yl)prop-2-yn-1-yl)-3-methyl-N (2-((R)-1-methylpyrrolidin-2-yl)-1H pyrrolo[3,2-c]pyridin-6-yl)-1*H*-indazole-6-carboxamide**

To a solution of *tert*-butyl 6-(1-(3-(2-(2,6-dioxo-1-((2-(trimethylsilyl)ethoxy)methyl)piperidin-3-yl)-1-oxoisoindolin-4-yl)prop-2-yn-1-yl)-3-methyl- 1*H*-indazole-6-carboxamido)-2-((*R*)-1-methylpyrrolidin-2-yl)-1*H*-pyrrolo[3,2-c]pyridine-1-carboxylate **(Intermediate 308, 0.90** g, 0.10 mmol) in acetonitrile (2.0 mL) was added methanesulfonic acid (0.06 mL, 1.01 mmol) and the reaction mixture was stirred at 60°C for 2h. The reaction mixture was cooled to room temperature, then *N,N'*-dimethylethylenediamine (0.06 mL, 0.50 mmol) followed by triethylamine (0.28 mL, 2.03 mmol) were added and the mixture stirred at room temperature for 4h. The reaction mixture was concentrated *in vacuo* and the resultant residue was diluted with water (50 mL). The resultant precipitate was collected by filtration and dried *in vacuo.* The crude solid was purified by preparative HPLC (Method A) to afford 1-(3-(2-(2,6-dioxopiperidin-3-yl)-1-oxoisoindolin-4-yl) prop-2-yn-1-yl)-3-methyl-*N*-(2-((*R*)-1-methylpyrrolidin-2-yl)-1*H*-pyrrolo[3,2-c]pyridin-6-yl)-1*H*-indazole-6-carboxamide **(Example 72,** 0.01 g, 16%) as a yellow solid. $^1H$ NMR (400 MHz, DMSO-$d_6$) δ ppm 1.76-2.00 (m, 5H), 2.17 (s, 3H), 2.23-2.34 (m, 3H), 2.46-2.49 (m, 1H), 2.55 (s, 3H), 2.79-2.89 (m, 1H), 3.12-3.18 (m, 1H), 4.14-4.20 (m, 1H), 4.34-4.38 (m, 1H), 5.06-5.12 (m, 1H), 5.65 (s, 2H), 6.41 (s, 1H), 7.52 (t, J=7.60 Hz, 1H), 7.66-7.69 (m, 1H), 7.74 (d, J=7.60 Hz, 1H), 7.81-7.87 (m, 2H), 8.23 (s, 1H), 8.52 (s, 1H), 8.60 (s, 1H), 10.55 (s, 1H), 11.00 (br s, 1H), 11.40 (s, 1H). Mass spec m/z 655.1

[M+H]+.

**Example 73 was synthesised following Scheme 68**

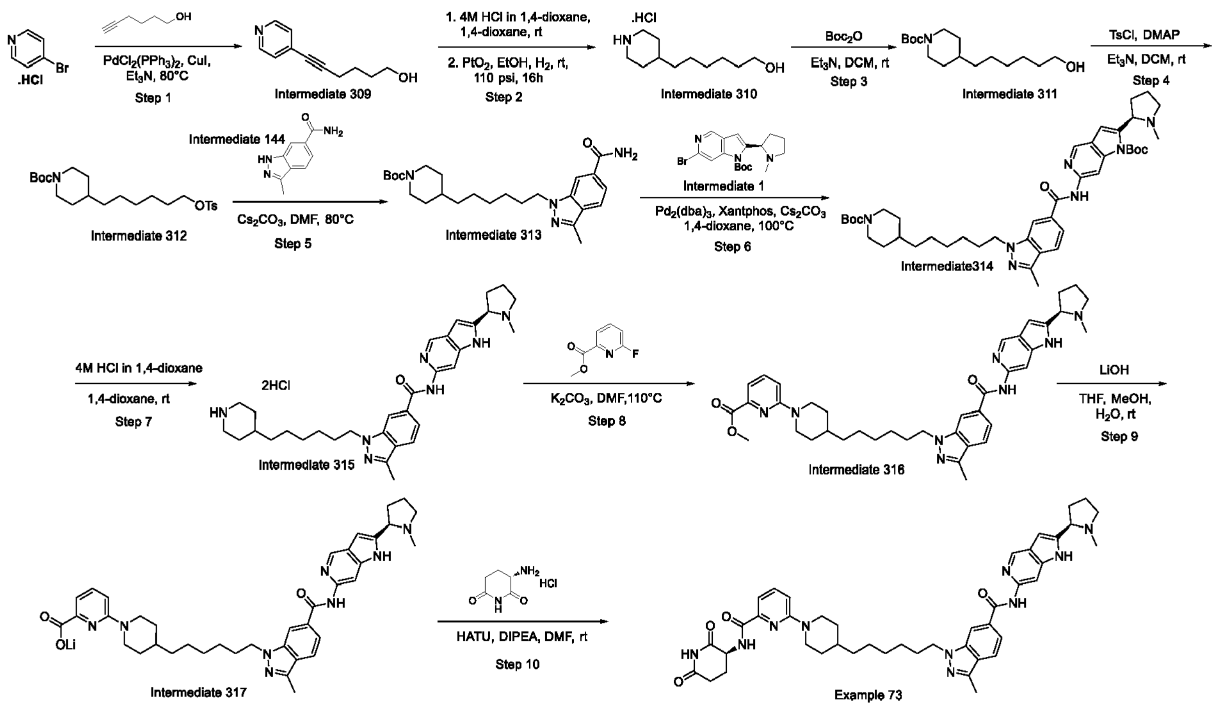


# Scheme 68

**Step 1**

**Intermediate 309: 6-(pyridin-4-yl)hex-5-yn-1-ol**

To a solution of 4-bromopyridine hydrochloride (CAS No: 19524-06-2, 13.0 g, 82.3 mmol) in dimethylformamide (6 mL) was added hex-5-yn-1-ol (CAS No: 928-90-5, 9.69 g, 98.7 mmol) followed by triethylamine (20 mL, 143.0 mmol) and the reaction mixture was purged with argon for 15 min. Copper(I) iodide (1.60 g, 8.23 mmol) and $PdCl_2(PPh_3)_2$ (5.83 g, 8.23 mmol) were added and the reaction mixture was further purged with argon for 10 min and then heated at 80°C for 3h. The reaction mixture was concentrated *in vacuo.* The obtained residue was diluted with aqueous saturated sodium thiosulfate solution (200 mL) and extracted with ethyl acetate (3 × 500 mL). The combined organic phases were dried over anhydrous $Na_2SO_4$, filtered, and concentrated *in vacuo.* The crude material was purified by combi-flash chromatography by eluting with 100% ethyl acetate in heptane to afford 6-(pyridin-4-yl) hex-5-yn-1-ol **(Intermediate 309,** 8.00 g, 55%) as a yellow liquid. $^1$H NMR (400 MHz, DMSO-$d_6$) δ ppm 1.48-1.61 (m, 4H), 2.48 (t, J=7.20 Hz, 2H), 3.34-3.45 (m, 2H), 4.39-4.46 (m, 1H), 7.32-7.38 (m, 2H), 7.54-7.64 (m, 2H). Mass spec: m/z: 176.0 [M+H]+.

**Step 2**

**Intermediate 310: 6-(piperidin-4-yl)hexan-1-ol hydrochloride**

To a solution of 6-(pyridin-4-yl)hex-5-yn-1-ol **(Intermediate 309,** 10.0 g, 57.1 mmol) in 1, 4-dioxane (5 mL) was added 4M hydrochloric acid in 1,4-dioxane (5 mL) at room temperature and the reaction stirred for 30 min. The reaction mixture was concentrated *in vacuo* and the obtained residue was dissolved in ethanol (5.0 mL, 86.0 mmol) platinum(IV) oxide (2.59 g, 11.4 mmol) was added and the reaction mixture was stirred at room temperature for 16h under $H_2$ atmosphere at 110 psi. The reaction mixture was filtered through celite, washed with methanol (100 mL) and the filtrate was concentrated *in vacuo* to afford 6-(piperidin-4-yl)hexan-1-ol hydrochloride **(Intermediate 310,** 8.60 g, 81%) as a yellow liquid, which was used for the next step without further purification. $^1$H NMR (400 MHz, DMSO-$d_6$) δ 1.14-1.54 (m, 13H), 1.72-1.76 (m, 2H), 2.72-2.80 (m, 2H), 3.12-3.20 (m, 2H), 3.34-3.38 (m, 2H), 3.89 (br s, 1H), 8.89 (br s, 1H), 9.12 (br s,

1H). Mass spec: m/z: 186.3 [M+H]+.

**Step 3**

**Intermediate 311: *tert*-butyl 4-(6-hydroxyhexyl) piperidine-1-carboxylate**

To a solution of 6-(piperidin-4-yl)hexan-1-ol hydrochloride **(Intermediate 310,** 8.60 g, 46 mmol) in dichloromethane (5 mL) was added triethylamine (33 mL, 230 mmol) followed by di-tert-butyl dicarbonate (16 mL, 70 mmol). The reaction mixture was stirred at room temperature for 7h under a nitrogen atmosphere. The reaction mixture was quenched with water (30 mL) and extracted with dichloromethane (2 × 30 mL). The combined organic phases were dried over anhydrous $Na_2SO_4$, filtered, and concentrated *in vacuo* to afford tert-butyl 4-(6-hydroxyhexyl)piperidine-1-carboxylate **(Intermediate 311,** 8.00 g, 60%)) as a yellow liquid, which was used for the next step without further purification. $^1$H NMR (400 MHz, DMSO-$d_6$) δ 0.84-0.94 (m, 2H), 1.06-1.24 (m, 8H), 1.26-1.31 (m, 1H), 1.38 (s, 9H), 1.55-1.65 (m, 4H), 2.58-2.74 (m, 2H), 3.34-3.40 (m, 2H), 3.86-3.94 (m, 2H), 4.29-4.32 (m, 1H). Mass spec: m/z: 186.23 [M+H]+.

**Step 4**

**Intermediate 312: *tert*-butyl 4-(6-(tosyloxy)hexyl) piperidine-1-carboxylate**

To a solution of *tert*-butyl 4-(6-hydroxyhexyl)piperidine-1-carboxylate **(Intermediate 311,** 8.00 g, 28.0 mmol) in dichloromethane (100 mL) was added triethylamine (19.5 mL, 140.0 mmol) and 4-toluenesulfonyl chloride (8.02 g, 42.0 mmol) followed by 4-dimethylaminopyridine (0.36 g, 2.80 mmol) at 0°C. The reaction mixture was stirred at room temperature for 16h under nitrogen atmosphere. The reaction mixture was quenched with water (300 mL) and extracted with dichloromethane (2 × 300 mL). The combined organic phases were dried over anhydrous $Na_2SO_4$ filtered, and concentrated *in vacuo.* The crude material was purified by combi-flash chromatography by eluting with 50% ethyl acetate in heptane to afford *tert*-butyl 4-(6-(tosyloxy)hexyl)piperidine-1-carboxylate **(Intermediate 312, 9.00** g, 73%) as a yellow liquid. $^1$H NMR (400 MHz, DMSO-$d_6$) δ 0.84-0.94 (m, 2H), 1.06-1.24 (m, 8H), 1.26-1.31 (m, 1H), 1.38 (s, 9H), 1.49-1.63 (m, 4H), 2.42 (s, 3H), 2.58-2.72 (m, 2H), 3.85-3.95 (m, 2H), 3.96-4.05 (m, 2H), 7.43-7.52 (m, 2H), 7.74-7.82 (m, 2H). Mass spec: m/z: 439.9 [M+H]+.

**Step 5**

**Intermediate 313: *tert*-butyl 4-(6-(6-carbamoyl-3-methyl-1*H*-indazol-1-yl)hexyl)piperidine-1-carboxylate**

To a solution of tert-butyl 4-(6-(tosyloxy)hexyl)piperidine-1-carboxylate **(Intermediate 312,** 7.50 g, 17.0 mmol) in dimethylformamide (50 mL) was added 3-methyl-1H-indazole-6-carboxamide **(Intermediate 144,** 3.0 g, 17.0 mmol) followed by cesium carbonate (17 g, 51.0 mmol) at room temperature and the reaction mixture was heated at 80°C for 5h. The reaction mixture was quenched with water (300 mL) and extracted with ethyl acetate (2 × 300 mL). The combined organic phases were dried over anhydrous $Na_2SO_4$, filtered and concentrated *in vacuo* to get the crude mixture which was separated by combi-flash chromatography by eluting with 90-95% ethyl acetate in heptane to afford *tert*-butyl 4-(6-(6-carbamoyl-3-methyl-1*H*-indazol-1-yl)hexyl)piperidine-1-carboxylate **(Intermediate 313,** 3.50 g, 46%) as a yellow liquid. $^1$H NMR (400 MHz, DMSO-$d_6$) δ 0.86-0.94 (m, 2H), 1.08-1.34 (m, 6H), 1.38 (s, 9H), 1.54-1.60 (m, 2H), 1.76-1.86 (m, 2H), 2.51 (s, 3H), 2.60-2.68 (m, 2H), 3.16-3.29 (m, 3H), 3.86-3.92 (m, 2H), 4.33 (t, J=6.40 Hz, 2H), 7.32 (br s, 1H), 7.60 (d, J=8.29 Hz, 1H), 7.72 (d, J=8.29 Hz, 1H), 7.96 (br s, 1H), 8.11 (br s, 1H). Mass spec: m/z: 443.0 [M+H]+.

**Step 6**

**Intermediate 314: *tert*-butyl (*R*)-6-(1-(6-(1-(*tert*-butoxycarbonyl)piperidin-4-yl)hexyl)-3-methyl-1*H*-indazole-6-carboxamido)-2-(1-methylpyrrolidin-2-yl)-1*H*-pyrrolo[3,2-c]pyridine-1-carboxylate**

To a solution of *tert*-butyl 4-(6-(6-carbamoyl-3-methyl-1*H*-indazol-1-yl)hexyl)piperidine-1-carboxylate (Comp-**Intermediate 313,** 2.33 g, 5.26 mmol) in 1,4-dioxane (50 mL) was added *tert*-butyl (*R*)-6-bromo-2-(1-methylpyrrolidin-2-yl)-1*H*-pyrrolo[3,2-c]pyridine-1-carboxylate **(Intermediate 1,** 2.50 g, 6.57 mmol) followed by cesium carbonate (6.43 g, 19.7 mmol) at room temperature. The reaction mixture was purged with argon for 15 min then $Pd_2(dba)_3$ (0.93 g, 0.98 mmol) and Xantphos (1.18 g, 1.97 mmol) were added. The reaction mixture was further purged with argon for 10 min and heated at 100°C for 2h. Progress of the reaction was monitored by TLC. After completion, the reaction mixture was filtered through celite bed, washed with ethyl acetate (50 mL) and the filtrate was concentrated in *vacuo.* The crude material was purified by combi-flash chromatography by eluting with 95% ethyl acetate in heptane to afford *tert*-butyl

(*R*)-6-(1-(6-(1-(*tert*-butoxycarbonyl)piperidin-4-yl)hexyl)-3-methyl-1*H*-indazole-6-carboxamido)-2-(1-methylpyrrolidin-2-yl)-1*H*-pyrrolo[3,2-c]pyridine-1-carboxylate **(Intermediate 314, 1.10** g, 23%) as a yellow solid. $^1$H NMR (400 MHz, DMSO-$d_6$) δ 0.82-0.92 (m, 2H), 1.08-1.33 (m, 9H), 1.35 (s, 9H), 1.50-1.57 (m, 3H), 1.59-1.66 (m, 1H), 1.71 (s, 9H), 1.74-1.89 (m, 5H), 2.29-2.34 (m, 1H), 2.36 (s, 3H), 2.38-2.43 (m, 1H), 2.52 (s, 3H), 3.11-3.17 (m, 1H), 3.80-3.95 (m, 3H), 4.36-4.44 (m, 2H), 6.77 (s, 1H), 7.71-7.75 (m, 2H), 7.77-7.81 (m, 1H), 8.62 (s, 1H), 8.99 (s, 1H), 10.76 (s, 1H). Mass spec: m/z: 742.3 [M+H]$^+$.

**Step 7**

**Intermediate 315: (*R*)-3-methyl-*N*-(2-(1-methylpyrrolidin-2-yl)-1*H*-pyrrolo[3,2-c] pyridin-6-yl)-1-(6-(piperidin-4-yl)hexyl)-1*H*-indazole-6-carboxamide dihydrochloride**

To a solution of *tert*-butyl (*R*)-6-(1-(6-(1-(*tert*-butoxycarbonyl) piperidin-4-yl) hexyl)-3-methyl-1*H*-indazole-6-carboxamido),-2-(1-methylpyrrolidin-2-yl)-1*H*-pyrrolo[3,2-c]pyridine-1-carboxylate **(Intermediate 314,** 1.10 g, 1.6 mmol) in 1,4-dioxane (10 mL) was added 4M hydrochloric acid in 1,4-dioxane (20 mL, 80.0 mmol) at room temperature and the reaction mixture was stirred at room temperature under nitrogen atmosphere for 16h. The reaction mixture was concentrated in *vacuo* to afford (R)-3-methyl-*N*-(2-(1-methylpyrrolidin-2-yl)-1*H*-pyrrolo[3,2-c]pyridin-6-yl)-1-(6-(piperidin-4-yl),hexyl)-1*H*-indazole-6-carboxamide dihydrochloride **(Intermediate 315,** 1.00 g, 91%) as an off white solid, which was used for the next step without further purification. $^1$H NMR (400 MHz, DMSO-$d_6$) δ 1.10-1.35 (m, 10H), 1.42-1.50 (m, 1H), 1.68-1.76 (m, 2H), 1.80-1.92 (m, 2H), 2.13-2.25 (m, 2H), 2.38-2.42 (m, 1H), 2.49 (s, 3H), 2.55 (s, 3H), 2.56-2.58 (m, 1H), 2.66-2.80 (m, 2H), 3.12-3.20 (m, 2H), 3.80-3.95 (m, 2H), 4.42-4.46 (m, 2H), 4.74-4.80 (m, 1H), 7.29 (s, 1H), 7.81-7.93 (m, 2H), 8.44 (s, 1H), 8.59 (s, 1H), 8.76-8.91 (m, 2H), 9.13 (s, 1H), 11.48 (br s, 1H), 12.30 (br s, 1H), 13.29 (br s, 1H). Mass spec: m/z: 542 [M+H]$^+$.

**Step 8**

**Intermediate 316: methyl (*R*)-6-(4-(6-(3-methyl-6-((2-(1-methylpyrrolidin-2-yl)-1*H*-pyrrolo[3,2-c]pyridin-6-yl)carbamoyl)-1*H*-indazol-1-yl)hexyl)piperidin-1-yl)picolinate**

To a solution of (*R*)-3-methyl-*N*-(2-(1-methylpyrrolidin-2-yl)-1*H*-pyrrolo[3,2-c]pyridin-6-yl)-1-(6-(piperidin-4-yl) hexyl)-1*H*-indazole-6-carboxamide dihydrochloride **(Intermediate 315,** 1.00 g, 1.8 mmol) in dimethylformamide (15 mL) were added methyl 6-fluoropicolinate (CAS No: 455-71-0, 0.29 g, 1.8 mmol) and potassium carbonate (0.77 g, 5.5 mmol) and the reaction mixture was heated at 110°C for 16h. Water (100 mL) was added and a solid precipitated, which was filtered and dried under vacuum to afford methyl (*R*)-6-(4-(6-(3-methyl-6-((2-(1-methylpyrrolidin-2-yl)-1*H*-pyrrolo[3,2-c]pyridin-6-yl)carbamoyl)-1*H*-indazol-1-yl)hexyl)piperidin-1-yl)picolinate **(Intermediate 316, 0.70** g, 60%) as a brown solid, which was used for the next step without further purification. $^1$H NMR (400 MHz, DMSO-$d_6$) δ 0.98-1.08 (m, 2H), 1.14-1.35 (m, 9H), 1.38-1.48 (m, 1H), 1.64-1.72 (m, 2H), 1.76-1.97 (m, 5H), 2.12-2.14 (m, 1H), 2.17 (s, 3H), 2.23-2.31 (m, 1H), 2.51 (s, 3H), 2.67-2.79 (m, 2H), 3.10-3.19 (m, 1H), 3.81 (s, 3H), 4.24-4.34 (m, 2H), 4.36-4.44 (m, 2H), 6.40 (s, 1H), 6.97-6.99 (m, 1H), 7.21-7.23 (m, 1H), 7.58-7.62 (m, 1H), 7.70-7.81 (m, 2H), 8.25 (s, 1H), 8.44 (s, 1H), 8.52 (s, 1H), 10.52 (br s, 1H), 11.37 (br s, 1H). Mass spec: m/z: 677.0 [M+H]$^+$.

**Step 9**

**Intermediate 317: Lithium salt of (R)-6-(4-(6-(3-methyl-6-((2-(1-methylpyrrolidin-2-yl)-1*H*-pyrrolo[3,2-c]pyridin-6-yl)carbamoyl)-1*H*-indazol-1-yl)hexyl)piperidin-1-yl)picolinic acid**

To a solution of methyl (*R*)-6-(4-(6-(3-methyl-6-((2-(1-methylpyrrolidin-2-yl)-1*H*-pyrrolo[3,2-c]pyridin-6-yl)carbamoyl)-1*H*-indazol-1-yl)hexyl)piperidin-1-yl)picolinate **(Intermediate 316, 0.70** g, 1.0 mmol) in tetrahydrofuran (10 mL), methanol (10 mL) and water (5 mL) was added lithium hydroxide (0.05 g, 2.0 mmol) and the reaction mixture was stirred at room temperature for 5h. The reaction mixture was directly concentrated *in vacuo* to afford as lithium salt of (*R*)-6-(4-(6-(3-methyl-6-((2-(1-methylpyrrolidin-2-yl)-1*H*-pyrrolo[3,2-c]pyridin-6-yl)carbamoyl)-1*H*-indazol-1-yl)hexyl)piperidin-1-yl)picolinic acid **(Intermediate 317, 0.50** g, 70%) as a yellow solid, which was used for the next step without further purification. $^1$H NMR (400 MHz, DMSO-$d_6$) δ 0.93-1.06 (m, 2H), 1.07-1.34 (m, 9H), 1.52-1.60 (m, 2H), 1.74-1.94 (m, 6H), 2.12-2.14 (m, 1H), 2.16 (s, 3H), 2.21-2.29 (m, 1H), 2.52 (s, 3H), 2.59-2.68 (m, 2H), 3.11-3.18 (m, 1H), 4.02-4.08 (m, 2H), 4.38-4.44 (m, 2H), 6.35 (s, 1H), 6.74-6.80 (m, 1H), 7.21-7.27 (m, 1H), 7.47-7.54 (m, 1H), 7.72-7.78 (m, 2H), 8.14 (s, 1H), 8.41 (s, 1H), 8.44 (s, 1H), 8.54 (br s, 1H), 10.61 (br s, 1H). Mass spec: m/z: 663.0 [M+H]$^+$.

**Step 10**

**Example 73: 1-(6-(1-(6-(((*S*)-2,6-dioxopiperidin-3-yl)carbamoyl)pyridin-2-yl)piperidin-4-yl)hexyl)-3-methyl-*N*-(2-((*R*)-1-methylpyrrolidin-2-yl)-1*H*-pyrrolo[3,2-c]pyridin-6-yl)-1*H*-indazole-6-carboxamide**

To a solution of the lithium salt of (*R*)-6-(4-(6-(3-methyl-6-((2-(1-methylpyrrolidin-2-yl)-1*H*-pyrrolo[3,2-c]pyridin-6-yl)carbamoyl)-1*H*-indazol-1-yl)hexyl)piperidin-1-yl)picolinic acid **(Intermediate 317,** 0.50 g, 0.7 mmol) in dimethylformamide (15 mL) was added HATU (0.4 g, 0.9 mmol) and the reaction stirred at room temperature for 15 min. (*S*)-3-aminopiperidine-2,6-dione hydrochloride (CAS No: 25181-50-4, 0.1 g, 0.7 mmol) and *N,N*-diisopropylethylamine (0.6 mL, 4 mmol) were added and the reaction mixture was stirred at room temperature for 16h. The reaction mixture was poured into ice cold water (100 mL) to get a precipitate which was filtered and dried *in vacuo* to obtain the crude compound. The crude material was purified through preparative HPLC (Method A) to afford 1-(6-(1-(6-(((*S*)-2,6-dioxopiperidin-3-yl) carbamoyl)pyridin-2-yl)piperidin-4-yl)hexyl)-3-methyl-*N*-(2-((*R*)-1-methylpyrrolidin-2-yl)-1*H*-pyrrolo[3,2-c]pyridin-6-yl)-1*H*-indazole-6-carboxamide **(Example 73,** 0.21 g, 40%) as an off white solid. $^1$H NMR (400 MHz, DMSO-$d_6$) δ 1.00-1.11 (m, 2H), 1.14-1.35 (m, 9H), 1.38-1.48 (m, 1H), 1.65-1.74 (m, 2H), 1.80-2.04 (m, 6H), 2.12-2.14 (m, 1H), 2.17 (s, 3H), 2.22-2.31 (m, 2H), 2.52 (s, 3H), 2.53-2.55 (m, 1H), 2.70-2.81 (m, 3H), 3.11-3.18 (m, 1H), 4.32-4.43 (m, 4H), 4.67-4.76 (m, 1H), 6.40 (s, 1H), 6.96 (d, J=8.80 Hz, 1H), 7.24 (d, *J*=7.20 Hz, 1H), 7.62 (t, J=7.20 Hz, 1H), 7.71-7.81 (m, 2H), 8.25 (s, 1H), 8.44 (s, 1H), 8.52 (s, 1H), 8.71 (d, J=8.80 Hz, 1H), 10.53 (s, 1H), 10.87 (s, 1H), 11.38 (s, 1H). Mass spec: m/z: 773.2 [M+H]$^+$.

**Example 74 was synthesised following Scheme 69**

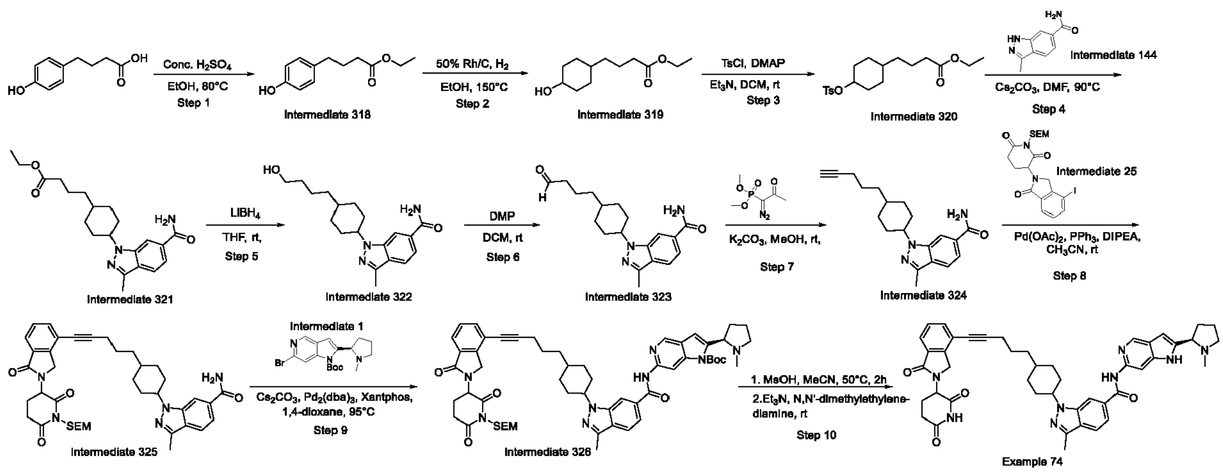


## Scheme 69

**Step** 1

**Intermediate 318: ethyl 4-(4-hydroxyphenyl)butanoate**

To a solution of 4-(4-hydroxyphenyl)butanoic acid (CAS No: 7021-11-6, 25.0 g, 138.73 mmol) in ethanol (250 mL) was added sulfuric acid (6.80 g, 69.36 mmol) at 0°C and the reaction mixture was heated at 80°C for 2h. The reaction mixture was concentrated *in vacuo* to obtain crude compound. The crude material was purified by combi-flash chromatography by eluting with 35% ethyl acetate in heptane to afford ethyl 4-(4-hydroxyphenyl) butanoate **(Intermediate 318,** 23.0 g, 80%) as a liquid. $^1$H NMR (400 MHz, DMSO-$d_6$) δ 1.19 (t, J=7.20 Hz, 3H), 1.73-1.80 (m, 2H), 2.26 (t, J=7.60 Hz, 2H), 2.46 (t, *J*=7.60 Hz, 2H), 4.06 (q, *J*=7.20 Hz, 2H), 6.67 (d, *J*=8.40 Hz, 2H), 6.97 (d, *J*=8.40 Hz, 2H), 9.14 (s, 1H). Mass spec: m/z: 209.10 [M+H]$^+$.

**Step 2**

**Intermediate 319: ethyl 4-(4-hydroxycyclohexyl)butanoate**

To a solution of ethyl 4-(4-hydroxyphenyl) butanoate **(Intermediate 318,** 15.0 g, 72.03 mmol) in ethanol (150 mL) was added 50% Rh/C (0.5 mL, 30.0 mmol) at room temperature under a nitrogen atmosphere. The atmosphere was then

changed to hydrogen and the reaction mixture was heated at 150°C for 72h under a hydrogen atmosphere at 180 psi. The reaction mixture was filtered through celite, washed with methanol (100 mL) and concentrated *in vacuo.* The crude material was purified by combi-flash column chromatography by eluting with 30% ethyl acetate in heptane to afford ethyl 4-(4-hydroxycyclohexyl) butanoate **(Intermediate 319,** 10.0 g, 65%) as a yellow liquid.

$^1$H NMR (400 MHz, DMSO-$d_6$) δ 0.79-0.93 (m, 1H), 1.03-1.07 (m, 2H), 1.11-1.23 (m, 2H), 1.17 (t, *J*=7.20 Hz, 3H), 1.27-1.43 (m, 3H), 1.45-1.59 (m, 3H), 1.62-1.68 (m, 1H), 1.76-1.80 (m, 1H), 2.19-2.29 (m, 2H), 3.26-3.32 (m, 1H), 3.71 (br s, 1H), 4.05 (q, *J*=7.20 Hz, 2H).

**Step 3**

**Intermediate 320: ethyl 4-(4-(tosyloxy)cyclohexyl)butanoate**

To a solution of ethyl 4-(4-hydroxycyclohexyl)butanoate **(Intermediate 319,** 9.00 g, 42.0 mmol) in dichloromethane (100 mL) was added triethylamine (20.0 mL, 130.0 mmol) and 4-dimethylaminopyridine (2.7 g, 21.0 mmol) at 0°C. After stirring for 5 min, p-toluenesulfonyl chloride (9.4 mL, 63.0 mmol) was added and the reaction stirred at room temperature for 16h. The reaction mixture was poured in ice cold water (120 mL) and extracted with dichloromethane (2 × 150 mL). The combined organic phases were dried over anhydrous $Na_2SO_4$, filtered, and concentrated *in vacuo.* The crude material was purified by combi-flash chromatography by eluting with 50% ethyl acetate in heptane to afford ethyl 4-(4-(tosyloxy)cyclohexyl)butanoate **(Intermediate 320,** 10.0 g, 65% yield) as a yellow liquid. $^1$H NMR (400 MHz, DMSO-$d_6$) δ 0.82-0.96 (m, 1H), 1.05-1.12 (m, 4H), 1.13 (t, *J*=7.20 Hz, 3H), 1.34-1.55 (m, 5H), 1.62-1.66 (m, 2H), 1.74-1.78 (m, 1H), 2.20-2.25 (m, 2H), 2.41 (s, 3H), 4.02 (q, *J*=7.20 Hz, 2H), 4.31-4.39 (m, 1H), 7.46 (d, *J*=7.80 Hz, 2H), 7.78 (d, *J*=7.80 Hz, 2H).

**Step 4**

**Intermediate 321: ethyl 4-(4-(6-carbamoyl-3-methyl-1*H*-indazol-1-yl) cyclohexyl)butanoate**

To a solution of ethyl 4-(4-(tosyloxy) cyclohexyl)butanoate **(Intermediate 320,** 9.6 g, 26.0 mmol) in dimethylformamide (70 mL) was added 3-methyl-1*H*-indazole-6-carboxamide **(Intermediate 144,** 3.50 g, 20.0 mmol) followed by cesium carbonate (17.0 g, 50.0 mmol) and the reaction mixture was heated at 90°C for 5h. The reaction mixture was then poured into ice cold water (100 mL) and extracted with ethyl acetate (3 × 120 mL). combined organic phases were dried over anhydrous $Na_2SO_4$, filtered, and concentrated *in vacuo* to get the crude which was purified by combi-flash chromatography by eluting with 70% ethyl acetate in heptane to afford ethyl 4-(4-(6-carbamoyl-3-methyl-1H-indazol-1-yl) cyclohexyl) butanoate **(Intermediate 321, 2.50** g, 34%) as a yellow liquid. $^1$H NMR (400 MHz, DMSO-$d_6$) δ 1.19 (t, J=7.60 Hz, 3H), 1.24-1.31 (m, 1H), 1.40-1.48 (m, 1H), 1.54-1.64 (m, 2H), 1.67-1.79 (m, 4H), 1.84-1.99 (m, 2H), 1.99-2.12 (m, 1H), 2.29-2.35 (m, 2H), 2.36 (s, 3H), 2.74-2.76 (m, 1H), 2.89-2.92 (m, 1H), 4.12 (q, J=7.60 Hz, 2H), 4.51-4.64 (m, 1H), 7.42 (br s, 1H), 7.59-7.62 (m, 1H), 7.73-7.75 (m, 1H), 8.04 (br s, 1H), 8.15-8.17 (m,1H). Mass spec: m/z: 372.64 $[M+H]^+$.

**Step 5**

**Intermediate 322: 1-(4-(4-hydroxybutyl) cyclohexyl)-3-methyl-1*H*-indazole-6-carboxamide**

To a solution of ethyl 4-(4-(6-carbamoyl-3-methyl-1*H*-indazol-1-yl)cyclohexyl)butanoate **(Intermediate 321, 2.50** g, 6.7 mmol) in tetrahydrofuran (25 mL) was added 2M Lithium borohydride solution in tetrahydrofuran (17 mL, 34.0 mmol) at 0°C and the reaction mixture was stirred at room temperature for 32h. The reaction mixture was poured into ice cold water (80 mL) and extracted with ethyl acetate (2 × 150 mL). The combined organic phases were dried over anhydrous $Na_2SO_4$, filtered, and concentrated in *vacuo.* The crude material was purified by combi-flash chromatography by eluting with ethyl acetate to afford 1-(4-(4-hydroxybutyl) cyclohexyl)-3-methyl-1*H*-indazole-6-carboxamide **(Intermediate 322, 1.80** g, 81%) as an off white solid. $^1$H NMR (400 MHz, DMSO-$d_6$) δ 1.18-1.28 (m, 2H), 1.30-1.48 (m, 5H), 1.64-1.77 (m, 4H), 1.86-1.96 (m, 2H), 1.98-2.12 (m, 1H), 2.52 (s, 3H), 3.36-3.44 (m, 2H), 4.32-4.36 (m, 1H), 4.51-4.62 (m, 1H), 7.40 (br s, 1H), 7.59-7.62 (m, 1H), 7.70-7.74 (m, 1H), 8.03 (br s, 1H), 8.10-8.17 (m, 1H). Mass spec: m/z: 330.2 $[M+H]^+$.

**Step 6**

**Intermediate 323: 3-methyl-1-(4-(4-oxobutyl) cyclohexyl)-1*H*-indazole-6-carboxamide**

To a solution of 1-(4-(4-hydroxybutyl)cyclohexyl)-3-methyl-1*H*-indazole-6-carboxamide **(Intermediate 322,** 1.1

g, 3.3 mmol) in dichloromethane (10 mL) was added Dess-Martin Periodinane (2.2 g, 5.0 mmol) at 0°C and the reaction mixture was stirred at room temperature for 1h. The reaction mixture was poured into saturated $NaHCO_3$ solution (100 mL) and extracted with dichloromethane (2 × 90 mL). The combined organic phases were dried over anhydrous $Na_2SO_4$, filtered, and concentrated *in vacuo* to afford 3-methyl-1-(4-(4-oxobutyl) cyclohexyl)-1*H*-indazole-6-carboxamide **(Intermediate 323,** 0.90 g, 82%) as a solid, which was used for the next step without further purification.

Mass spec: m/z: 328.1 $[M+H]^+$.

**Step 7**

**Intermediate 324: 3-methyl-1-(4-(pent-4-yn-1-yl)cyclohexyl)-1*H*-indazole-6-carboxamide**

To a solution of 3-methyl-1-(4-(4-oxobutyl) cyclohexyl)-1*H*-indazole-6-carboxamide **(Intermediate 323,** 0.90 g, 2.74 mmol) in methanol (10 mL) was added potassium carbonate (0.79 g, 5.49 mmol) at 0°C, followed by dimethyl (1-diazo-2-oxopropyl)phosphonate (0.79 g, 4.12 mmol), and the reaction mixture was stirred at room temperature for 2h. The reaction mixture was poured into water (90 mL) and extracted with ethyl acetate (2 × 100 mL). The combined organic phases were dried over anhydrous $Na_2SO_4$, filtered, and concentrated *in vacuo.* The crude material was purified by combi-flash chromatography by eluting with 5% of MeOH in DCM to afford 3-methyl-1-(4-(pent-4-yn-1-yl)cyclohexyl)-1*H*-indazole-6-carboxamide **(Intermediate 324,** 0.31 g, 35%) as a yellow solid. $^1H$ NMR (400 MHz, DMSO-$d_6$) δ 1.13-1.25 (m, 1H), 1.29-1.41 (m, 1H), 1.43-1.55 (m, 3H), 1.65-1.78 (m, 3H), 1.83-1.97 (m, 2H), 1.98-2.11 (m, 1H), 2.15-2.24 (m, 2H), 2.52 (s, 3H), 2.76-2.78 (m, 1H), 3.63-3.69 (m, 2H), 4.43-4.62 (m, 1H), 7.42 (br s, 1H), 7.57-7.60 (m, 1H), 7.71-7.73 (m, 1H), 8.04 (br s, 1H), 8.14-8.16 (m, 1H). Mass spec: m/z: 324.2 $[M+H]^+$.

**Step 8**

**Intermediate 325: 1-(4-(5-(2-(2,6-dioxo-1-((2-(trimethylsilyl)ethoxy)methyl)piperidin-3-yl)-1-oxoisoindolin-4-yl) pent-4-yn-1-yl)cyclohexyl)-3-methyl-1*H*-indazole-6-carboxamide**

To a solution of 3-methyl-1-(4-(pent-4-yn-1-yl) cyclohexyl)-1*H*-indazole-6-carboxamide **(Intermediate 324,** 0.30 g, 0.93 mmol) and 3-(4-iodo-1-oxoisoindolin-2-yl)-1-((2-(trimethylsilyl)ethoxy)methyl)piperidine-2,6-dione **(Intermediate 25,** 0.49 g, 0.97 mmol) in acetonitrile (10 mL) was added *N,N*-diisopropylethylamine (0.9 mL, 4.89 mmol). The reaction mixture was purged with argon gas for 15 min. Triphenylphosphine (0.07 g, 0.29 mmol) and palladium acetate (0.02 g, 0.14 mmol) were then added and the reaction mixture was further purged with argon gas for 10 min and stirred at room temperature for 1h. The reaction mixture was concentrated *in vacuo* and the resulting crude material was purified by combi-flash chromatography by eluting with 7% of MeOH in DCM to afford 1-(4-(5-(2-(2,6-dioxo-1-((2-(trimethylsilyl) ethoxy)methyl)piperidin-3-yl)-1-oxoisoindolin-4-yl)pent-4-yn-1-yl)cyclohexyl)-3-methyl-1*H*-indazole-6-carboxamide **(Intermediate 326,** 0.30 g, 44%) as a solid. $^1H$ NMR (400 MHz, DMSO-$d_6$) δ -0.03 (s, 9H), 0.79-0.86 (m, 2H), 1.24-1.28 (m, 4H), 1.40-1.42 (m, 1H), 1.57-1.79 (m, 5H), 1.85-2.13 (m, 4H), 2.46 (s, 3H), 2.65-2.78 (m, 1H), 2.98-3.09 (m, 1H), 3.10-3.20 (m, 2H), 3.46-3.54 (m, 2H), 3.58-3.64 (m, 1H), 4.25-4.30 (m, 1H), 4.44-4.64 (m, 2H), 4.97-5.10 (m, 2H), 5.25-5.30 (m, 1H), 7.41 (br s, 1H), 7.53-7.60 (m, 2H), 7.66-7.68 (m, 1H), 7.70-7.74 (m, 2H), 8.03 (br s, 1H), 8.12-8.16 (m, 1H). Mass spec: m/z: 696.2 $[M+H]^+$.

**Step 9**

**Intermediate 326: 6-(1-(4-(5-(2-(2,6-dioxo-1-((2-(trimethylsilyl)ethoxy)methyl)piperidin-3-yl)-1-oxoisoindolin-4-yl)pent-4-yn-1-yl)cyclohexyl)-3-methyl-1*H*-indazole-6-carboxamido)-2-((*R*)-1-methylpyrrolidin-2-yl)-1*H*-pyrrolo [3,2-c]pyridine-1-carboxylate**

To a solution of 1-(4-(5-(2-(2,6-dioxo-1-((2-(trimethylsilyl)ethoxy)methyl)piperidin-3-yl)-1-oxoisoindolin-4-yl) pent-4-yn-1-yl)cyclohexyl)-3-methyl-1*H*-indazole-6-carboxamide **(Intermediate 325,** 0.20 g, 0.29 mmol) in 1,4-dioxane (2 mL) was added tert-butyl (*R*)-6-bromo-2-(1-methylpyrrolidin-2-yl)-1*H*-pyrrolo[3,2-c]pyridine-1-carboxylate **(Intermediate 1,** 0.14 g, 0.36 mmol) followed by cesium carbonate (0.18 g, 0.55 mmol) at room temperature and the reaction mixture was purged with argon for 15 min followed by $Pd_2(dba)_3$ (0.04 g, 0.05 mmol) and Xantphos (0.06 g, 0.11 mmol). The reaction mixture was further purged with argon for 10 min and then heated at 95°C for 1h. The reaction mixture was concentrated *in vacuo* to obtain crude compound. The crude material was purified by combi-flash chromatography by eluting with 7% of MeOH in DCM to afford tert-butyl 6-(1-(4-(5-(2-(2,6-dioxo-1-((2-(trimethylsilyl)ethoxy)methyl)piperidin-3-yl)-1-oxoisoindolin-4-yl)pent-4-yn-1-yl)cyclohexyl)-3-methyl-1*H*-indazole-6-carboxamido)-2-((*R*)-1-methylpyrrolidin-2-yl)-1*H*-pyrrolo[3,2-c]pyridine-1-carboxylate **(Intermediate 326**, 0.20 g, 55%) as a solid. $^1H$ NMR (400 MHz, DMSO-$d_6$) δ -0.04 (s, 9H), 0.75-0.88 (m, 2H), 1.22-1.26 (m, 1H), 1.40-1.50 (m, 2H), 1.57-1.68 (m, 8H), 1.71 (s, 9H),

1.72-1.78 (m, 4H), 2.02 (s, 3H), 2.05-2.12 (m, 2H), 2.30-2.33 (m, 2H), 2.39 (s, 3H), 2.40-2.42 (m, 1H), 2.67-2.76 (m, 1H), 2.96-3.17 (m, 2H), 3.44-3.63 (m, 3H), 3.88-3.97 (m, 1H), 3.98-4.07 (m, 1H), 4.26-4.30 (m, 1H), 4.46-4.52 (m, 1H), 4.63-4.70 (m, 1H), 5.01-5.07 (m, 2H), 5.22-5.32 (m, 1H), 6.77 (s, 1H), 7.53-7.58 (m, 1H), 7.66-7.80 (m, 4H), 8.42 (s, 1H), 8.61 (s, 1H), 8.98 (s, 1H), 10.77 (br s, 1H). Mass spec: m/z: 995.57 $[M+H]^+$.

**Step 10**

**Example 74: 1-(4-(5-(2-(2,6-dioxopiperidin-3-yl)-1-oxoisoindolin-4-yl) pent-4-yn-1-yl) cyclohexyl)-3-methyl-*N*-(2-((*R*)-1-methylpyrrolidin-2-yl)-1*H*-pyrrolo[3,2-c]pyridin-6-yl)-1*H*-indazole-6-carboxamide**

To a solution of tert-butyl 6-(1-(4-(5-(2-(2,6-dioxo-1-((2-(trimethylsilyl)ethoxy)methyl)piperidin-3-yl)-1-oxoisoindolin-4-yl)pent-4-yn-1-yl)cyclohexyl)-3-methyl-1H-indazole-6-carboxamido)-2-((R)-1-methylpyrrolidin-2-yl)-1*H*-pyrrolo[3,2-c]pyridine-1-carboxylate **(Intermediate 326,** 0.24 g, 0.24 mmol) in acetonitrile (3 mL) was added methanesulfonic acid (0.24 g, 2.41 mmol) at room temperature and heated at 50°C for 2h. *N*, *N'*-dimethylethylenediamine (0.13 g, 1.44 mmol) and triethylamine (0.51 g, 4.82 mmol) were added and the reaction stirred at room temperature for 2h. Progress of the reaction was monitored by TLC. After completion, the reaction mixture was concentrated in *vacuo* to obtain crude compound which was poured into water (50 mL), the solid precipitated out, which was filtered and dried under vacuo to get the crude compound which was purified through Prep-HPLC in acidic buffer to afford 1-(4-(5-(2-(2,6-dioxopiperidin-3-yl)-1-oxoisoindolin-4-yl)pent-4-yn-1-yl)cyclohexyl)-3-methyl-*N*-(2-((*R*)-1-methylpyrrolidin-2-yl)-1*H*-pyrrolo[3,2-c]pyridin-6-yl)-1*H*-indazole-6-carboxamide **(Example 74,** 0.1 g, 38%) as a white solid. $^1$H NMR (400 MHz, DMSO-$d_6$) δ 1.58-1.68 (m, 4H), 1.72-1.85 (m, 7H), 1.86-2.02 (m, 4H), 2.09-2.16 (m, 2H), 2.18 (s, 3H), 2.24-2.30 (m, 1H), 2.44-2.47 (m, 1H), 2.48 (s, 3H), 2.53-2.60 (m, 3H), 2.82-2.93 (m, 1H), 3.12-3.19 (m, 1H), 3.34-3.36 (m, 2H), 4.27-4.37 (m, 1H), 4.44-4.52 (m, 1H), 4.67-4.75 (m, 1H), 5.10-5.15 (m, 1H), 6.40 (s, 1H), 7.49-7.56 (m, 1H), 7.63-7.68 (m, 1H), 7.69-7.71 (m, 2H), 7.73-7.78 (m, 1H), 8.25 (s, 1H), 8.42 (s, 1H), 8.52 (s, 1H), 10.54 (br s, 1H), 10.98 (br s, 1H), 11.38 (br s, 1H). Mass spec: m/z: 765.0 $[M+H]^+$.

**Examples 75, 76 and 77 were synthesized following Scheme 70**

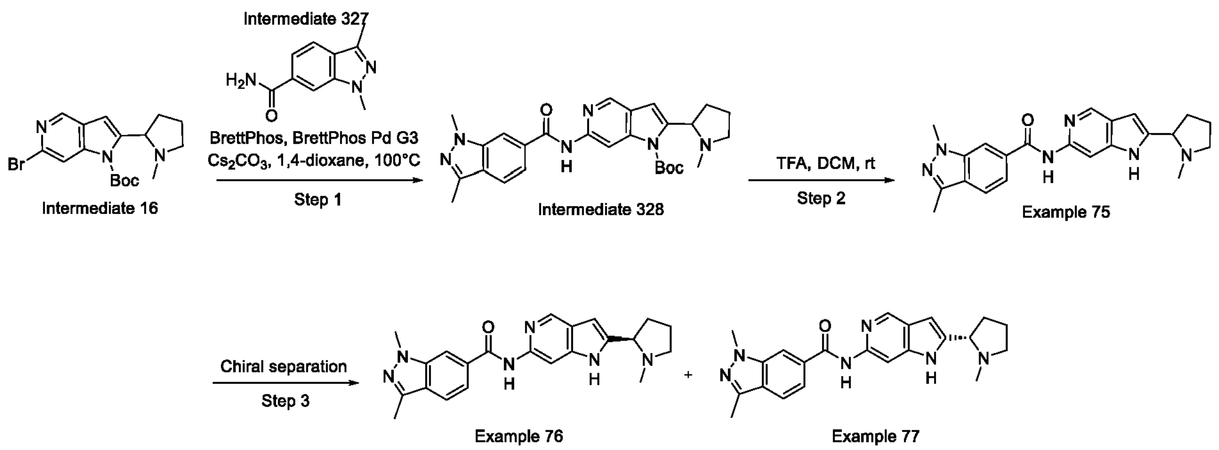


**Scheme 70**

**Step 1**

**Intermediate 328: rac-tert-butyl 6-(1,3-dimethyl-1H-indazole-6-carboxamido)-2-(1-methylpyrrolidin-2-yl)-1*H*-pyrrolo[3,2-c]pyridine-1-carboxylate**

A solution of rac-tert-butyl 6-bromo-2-(1-methylpyrrolidin-2-yl)-1H-pyrrolo[3,2-c]pyridine-1-carboxylate **(Intermediate 16,** 10 g, 25.1 mmol), 1,3-dimethyl-1H-indazole-6-carboxamide **(Intermediate 327:** 5.98 g, 30.0 mmol), Brettphos (6.74 g, 12.6 mmol), Brettphos Pd G3 (11.4 g, 12.6 mmol) and $Cs_2CO_3$ (40.9 g, 125.5 mmol) in 1,4-dioxane (300 mL) was stirred for 15h at 100°C under nitrogen atmosphere. The reaction mixture was quenched with water and the resulting solution was extracted three times with EtOAc. The organic layers were combined, washed with brine, dried over anhydrous $Na_2SO_4$, filtered, and concentrated under reduced pressure. The crude material was purified by flash

chromatography on silica gel using DCM/MeOH (95/5) as eluent to afford rac-tert-butyl 6-(1,3-dimethyl-1H-indazole-6-carboxamido)-2-(1-methylpyrrolidin-2-yl)-1H-pyrrolo[3,2-c]pyridine-1-carboxylate **(Intermediate 328:** 6 g, 42%) as a yellow oil. Mass spec: m/z 489 $[M+H]^+$.

**Step 2**

**Example 75: rac-1,3-dimethyl-N-(2-(1-methylpyrrolidin-2-yl)-1H-pyrrolo[3,2-*c*]pyridin-6-yl)-1*H*-indazole-6-carboxamide**

To a solution of rac-tert-butyl 6-(1,3-dimethyl-1H-indazole-6-carboxamido)-2-(1-methylpyrrolidin-2-yl)-1H-pyrrolo[3,2-c]pyridine-1-carboxylate **(Intermediate 328:** 6 g, 11.1 mmol) in DCM (150 mL) was added TFA (30 mL). The reaction mixture was stirred for 1h at rt. The reaction was concentrated under reduced pressure and the crude material was chromatographed by reverse-phase flash chromatography (C18 aq) using $MeCN/H_2O$ (37/63) to provide rac-1,3-dimethyl-N-(2-(1-methylpyrrolidin-2-yl)-1H-pyrrolo[3,2-c]pyridin-6-yl)-1H-indazole-6-carboxamide **(Example 75,** 4.6 g, 60%) as a light yellow solid. Mass spec: m/z 389 $[M+H]^+$; $^1H$ NMR (300 MHz, MeOH-$d_4$) δ ppm 8.93 (s, 1H), 8.28 (s, 1H), 7.89 (t, J = 8.7 Hz, 2H), 7.79 (d, J = 8.7 Hz, 1H), 7.24 (s, 1H), 4.81 - 4.83 (m, 1H), 4.10 (s, 3H), 3.80 (br, 1H), 3.31 - 3.44 (m, 1H), 2.95 (br, 3H), 2.62 - 2.76 (m, 1H), 2.49 - 2.58 (m, 4H), 2.30 - 2.41 (m, 2H).

**Step 3**

**Example 76: (R)-1,3-dimethyl-N-(2-(1-methylpyrrolidin-2-yl)-1H-pyrrolo[3,2-*c*]pyridin-6-yl)-1*H*-indazole-6-carboxamide and Example 77: (S)-1,3-dimethyl-N-(2-(1-methylpyrrolidin-2-yl)-1H-pyrrolo[3,2-c]pyridin-6-yl)-1H-indazole-6-carboxamide**

Rac-1,3-dimethyl-N-(2-(1-methylpyrrolidin-2-yl)-1H-pyrrolo[3,2-c]pyridin-6-yl)-1H-indazole-6-carboxamide **(Intermediate 328, 5.46** g, 13.4 mmol) was separated into the enantiomeric **Example 76** and **Example 77** using the following conditions: column: Chiral Art Amylose-C 30 × 250 mm, 5.0 μm; Eluent: $CO_2$/MeOH (1% of 2M solution $NH_3$ in MeOH) 1/1; Flow rate: 85 mL/min to provide:

**Example 76: (R)-1,3-dimethyl-N-(2-(1-methylpyrrolidin-2-yl)-1H-pyrrolo[3,2-c]pyridin-6-yl)-1H-indazole-6-carboxamide**

First eluting compound (retention time = 7.39 min)
$^1H$ NMR (400 MHz, MeOH-$d_4$) δ ppm 8.51 (s, 1H), 8.21 (s, 1H), 8.13 (s, 1H), 7.80 (d, J = 8.4 Hz, 1H), 7.71 (d, J = 7.2 Hz, 1H), 6.50 (s, 1H), 4.06 (s, 3H), 3.42 (t, J = 8.4Hz, 1H), 3.19 - 3.24 (m, 1H), 2.56 (s, 3H), 2.31 - 2.41 (m, 1H), 2.24 - 2.29 (m, 4H), 1.94 - 2.05 (m, 2H), 1.86 - 1.93 (m, 1H). Mass spec: m/z: 389 $[M+H]^+$.

**Example 77: (S)-1,3-dimethyl-N-(2-(1-methylpyrrolidin-2-yl)-1H-pyrrolo[3,2-c]pyridin-6-yl)-1H-indazole-6-carboxamide**

Second eluting compound (retention time = 12.1 min)
$^1H$ NMR (400 MHz, MeOH-$d_4$) δ ppm 8.53 (s, 1H), 8.17 - 8.21 (m, 2H), 7.71 - 7.85 (m, 2H), 6.53 (s, 1H), 4.08 (s, 3H), 3.45 (t, J = 8.00 Hz, 1H), 3.22 (t, J = 7.2 Hz, 1H), 2.58 (s, 3H), 2.26 - 2.44 (m, 5H), 2.01 - 2.09 (m, 3H). Mass spec: m/z: 389 $[M+H]^+$.

**Example 76 was also prepared following Scheme 71**

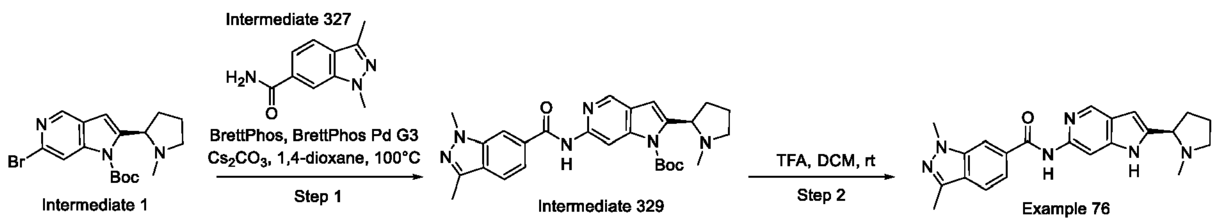


**Scheme 71**

**Step 1**

**Intermediate 329: tert-butyl (R)-6-(1,3-dimethyl-1H-indazole-6-carboxamido)-2-(1-methylpyrrolidin-2-yl)-1H-pyrrolo[3,2-c]pyridine-1-carboxylate**

A 50 mL round-bottom flask was charged with tert-butyl (R)-6-bromo-2-(1-methylpyrrolidin-2-yl)-1H-pyrrolo[3,2-c]pyridine-1-carboxylate **(Intermediate 1,** 400 mg, 0.999 mmol), 1,3-dimethyl-1H-indazole-6-carboxamide **(Intermediate 327,** 227 mg, 1.20 mmol), Brettphos Pd G3 (453 mg, 0.499 mmol), Brettphos (322 mg, 0.599 mmol), $Cs_2CO_3$ (1.63 g, 5.00 mmol), 1,4-dioxane (10 mL) under nitrogen. The reaction was stirred for 15h at 100°C under nitrogen and concentrated under reduced pressure. The residue was chromatographed on a silica gel column with MeOH/DCM (4/96) to provide tert-butyl (R)-6-(1,3-dimethyl-1H-indazole-6-carboxamido)-2-(1-methylpyrrolidin-2-yl)-1H-pyrrolo[3,2-c]pyridine-1-carboxylate **(Intermediate 329,** 350 mg, 68.1%) as a yellow solid. Mass spec: m/z: 489.1 $[M+H]^+$.

**Step 2**

**Example 76: (R)-1,3-dimethyl-N-(2-(1-methylpyrrolidin-2-yl)-1H-pyrrolo[3,2-c]pyridin-6-yl)-1H-indazole-6-carboxamide**

A 100 mL round-bottom flask was charged with tert-butyl (R)-6-(1,3-dimethyl-1H-indazole-6-carboxamido)-2-(1-methylpyrrolidin-2-yl)-1H-pyrrolo[3,2-c]pyridine-1-carboxylate **(Intermediate 329,** 350 mg, 0.645 mmol), DCM (10 mL) and TFA (3 mL) and the reaction was stirred for 2h at room temperature and then concentrated under reduced pressure to provide crude. The crude was chromatographed on a silica gel column with MeCN/water (50/50) to provide (R)-1,3-dimethyl-N-(2-(1-methylpyrrolidin-2-yl)-1H-pyrrolo[3,2-c]pyridin-6-yl)-1H-indazole-6-carboxamide (148.9 mg, 59.33%) as a off-white solid. $^1H$ NMR (400 MHz, MeOH-$d_4$) δ ppm 8.51 (s, 1H), 8.21 (s, 1H), 8.13 (s, 1H), 7.80 (d, J = 8.4 Hz, 1H), 7.71 (d, J = 7.2 Hz, 1H), 6.50 (s, 1H), 4.06 (s, 3H), 3.42 (t, J = 8.4Hz, 1H), 3.19 - 3.24 (m, 1H), 2.56 (s, 3H), 2.31 - 2.41 (m, 1H), 2.24 - 2.29 (m, 4H), 1.94 - 2.05 (m, 2H), 1.86 - 1.93 (m, 1H). Mass spec: m/z: 389 $[M+H]^+$.

**Intermediate 327 was synthesised following Scheme 72**

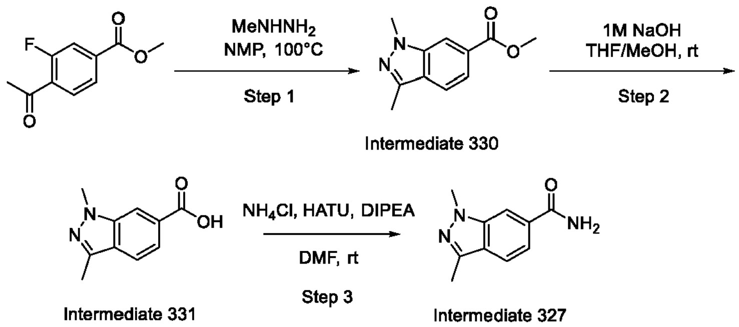


Scheme 72

**Step** 1

**Intermediate 330:** Methyl **1,3-dimethyl-1H-indazole-6-carboxylate**

To a solution of methyl 4-acetyl-3-fluorobenzoate (CAS: 1059549-72-2, 5.00 g, 24.2 mmol) in NMP (35 mL) was added methylhydrazine (1.5 mL, 29.1 mmol) and the mixture was stirred overnight at 100°C in a sealed tube. The solution was cooled to rt, diluted with $H_2O$ (35 mL) and the aqueous layer was extracted with EtOAc (40 mL). The organic layer was washed three times with $H_2O$, dried over anhydrous $MgSO_4$, filtered, and concentrated to dryness to afford methyl 1,3-dimethyl-1H-indazole-6-carboxylate **(Intermediate 330:** 4.3 g, 87%) as an orange oil which was used in Step 2 without further purification. Mass spec: m/z: 205 $[M+H]^+$; $^1H$ NMR (400 MHz, DMSO-$d_6$): δ ppm 8.21 (s, 1H), 7.81 (d, J=8.5 Hz, 1H), 7.65 (dd, J=8.5, 1.3 Hz, 1H), 4.03 (s, 3H), 3.90 (s, 3H), 2.50 (s, 3H).

**Step 2**

**Intermediate 331: 1,3-dimethyl-1H-indazole-6-carboxylic acid**

To a solution of methyl 1,3-dimethylindazole-6-carboxylate **(Intermediate 330:** 4.3 g, 20.0 mmol) in a mixture of THF (50 mL)/MeOH (50 mL) was added a NaOH solution (60 mL, 60.0 mmol, 1M in water) dropwise over 14 min. The resulting mixture was stirred at rt for 45 min. The solvents were removed under reduced pressure and the residue was acidified with a solution of HCl (60 mL, 1N in $H_2O$). The precipitate was filtered, rinsed with $H_2O$ (20 mL) and dried under reduced pressure at 40°C overnight to afford 1,3-dimethyl-1H-indazole-6-carboxylic acid **(Intermediate 331:** 3.85 g, quant.) as a yellow solid. Mass spec: m/z: 191 $[M+H]^+$; $^1H$ NMR (400 MHz, DMSO-$d_6$): δ ppm 13.04 (s, 1H), 8.19 (t, J=1.1 Hz, 1H), 7.79 (dd, J=8.3, 0.8 Hz, 1H), 7.65 (dd, J=8.4, 1.3 Hz, 1H), 4.03 (s, 3H), 2.51 (s, 3H).

**Step 3**

**Intermediate 327: 1,3-dimethyl-1H-indazole-6-carboxamide**

A 50mL round-bottom flask was charged with 1,3-dimethylindazole-6-carboxylic acid **(Intermediate 331,** 200 mg, 1.05 mmol), $NH_4Cl$ (281 mg, 5.26 mmol), HATU (600 mg, 1.58 mmol), DIPEA (408 mg, 3.16 mmol) in DMF (5 mL) and the reaction was stirred overnight at room temperature. The volatiles were removed under reduced pressure and the mixture was chromatographed on a C18 silica column, eluting with MeCN/water (with 0.5% TFA) (87/13) to provide 1,3-dimethyl-1H-indazole-6-carboxamide **(Intermediate 327,** 133 mg, 60%) as a white solid. $^1H$ NMR (400 MHz, DMSO-$d_6$) δ ppm 8.11 (t, J = 1.1 Hz, 1H), 8.02 (s, 1H), 7.74 (dd, J = 8.4, 0.8 Hz, 1H), 7.60 (dd, J = 8.4, 1.4 Hz, 1H), 7.41 (s, 1H), 4.00 (s, 3H), 2.49 (s, 3H). Mass spec: m/z: 190.1$[M+H]^+$.

**Examples 78 and 79 were synthesized following Scheme 73**

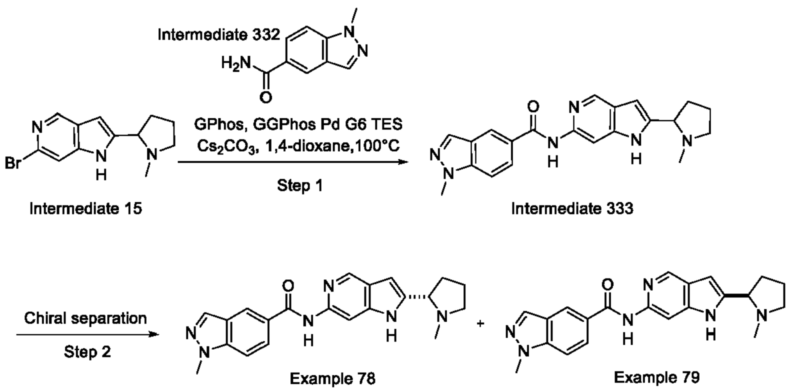


**Scheme 73**

**Step 1**

**Intermediate 333: rac-1-methyl-N-(2-(1-methylpyrrolidin-2-yl)-1H-pyrrolo[3,2-c]pyridin-6-yl)-1H-indazole-5-carboxamide**

To a solution of rac-2-{6-bromo-1H-pyrrolo[3,2-c]pyridin-2-yl}-1-methylpyrrolidine **(Intermediate 15,** 100 mg, 0.321 mmol) in 1,4-dioxane (2 mL) were added 1-methylindazole-5-carboxamide **(Intermediate 332,** 75.0 mg, 0.385 mmol), $Cs_2CO_3$ (314 mg, 0.963 mmol), [3-(tert-butoxy)-6-methoxy-2',6'-bis(propan-2-yl)-[1,1'-biphenyl]-2-yl]dicyclohexylphosphane Gphos (17.0 mg, 0.032 mmol) and Pd GPhos G6 TES (30.0 mg, 0.032 mmol). The reaction mixture was stirred at 100°C for 3h under nitrogen atmosphere and quenched with water. The resulting mixture was concentrated under reduced pressure and the crude material was chromatographed by reverse-phase flash chromatography (C18 aq) using MeCN/$H_2O$ (75/25) to provide rac-1-methyl-N-(2-(1-methylpyrrolidin-2-yl)-1H-pyrrolo[3,2-c]pyridin-6-yl)-1H-indazole-5-carboxamide **(Intermediate 333,** 50 mg 37%) as a brown oil. Mass spec: m/z 375 $[M+H]^+$.

**Step 2**

**Example 78: 1-methyl-N-{2-[(rel-2S)-1-methylpyrrolidin-2-yl]-1H-pyrrolo[3,2-c]pyridin-6-yl}indazole-5-carboxamide and Example 79: 1-methyl-N-{2-[(rel-2R)-1-methylpyrrolidin-2-yl]-1H-pyrrolo[3,2-c]pyridin-6-yl}indazole-5-carboxamide**

Rac-1-methyl-N-(2-(1-methylpyrrolidin-2-yl)-1H-pyrrolo[3,2-c]pyridin-6-yl)-1H-indazole-5-carboxamide **(Intermediate 333,** 50 mg) was separated into the enantiomeric **Examples 78** and **79** using the following conditions: column: Chiral Art Cellulose-SB 30 × 250 mm, 5.0 um; Eluent: $CO_2$/IPA (1% of 2M solution $NH_3$ in MeOH) 6/4; Flow rate: 85 mL/min to provide:

**Example 79: 1-methyl-N-{2-[(rel-2S)-1-methylpyrrolidin-2-yl]-1H-pyrrolo[3,2-c]pyridin-6-yl}indazole-5-carboxamide**

First eluting compound (retention time = 5.33 min)
Mass spec: m/z: 375 $[M+H]^+$; $^1H$ NMR (400 MHz, DMSO-$d_6$) δ ppm 11.36 (s, 1H), 10.45 (s, 1H), 8.51 - 8.56 (m, 2H), 8.21 (s, 2H), 8.08 - 8.10 (m, 1H), 7.72 - 7.74 (m, 1H), 6.40 (s, 1H), 4.10 (s, 3H), 3.33 (s, 1H), 3.10 - 3.19 (m, 1H), 2.26 - 2.28 (m, 1H), 2.15 - 2.17 (m, 4H), 1.88 - 1.90 (m, 3H).

**Example 79: 1-methyl-N-{2-[(rel-2R)-1-methylpyrrolidin-2-yl]-1H-pyrrolo[3,2-c]pyridin-6-yl}indazole-5-carboxamide**

Second eluting compound (retention time = 6.42 min)
Mass spec: m/z: 375 $[M+H]^+$; $^1H$ NMR (400 MHz, DMSO-$d_6$) δ ppm 11.36 (s, 1H), 10.45 (s, 1H), 8.51 - 8.56 (m, 2H), 8.21 (s, 2H), 8.08 - 8.11 (m, 1H), 7.72 - 7.74 (m, 1H), 6.40 (s, 1H), 4.10 (s, 3H), 3.33 (s, 1H), 3.10 - 3.19 (m, 1H), 2.26 - 2.30 (m, 1H), 2.09 - 2.20 (m, 4H), 1.75 - 2.01 (m, 3H).

**Intermediate 332: 1-methyl-1H-indazole-5-carboxamide**

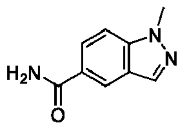

A stirred solution of 1-methyl-1H-indazole-5-carboxylic acid (CAS No: 1176754-31-6, 2.50 g, 14.2 mmol) in anhydrous DCM (125 mL), with a few drops of DMF, was cooled to 0°C before addition of thionyl chloride (1.1 mL, 15.6 mmol). The reaction mixture was stirred 4h at rt until consumption of starting material observed. The mixture was concentrated to dryness and re-dissolved in DCM (110 mL). A solution of $NH_4OH$ (11 mL, 71.0 mmol, 25% in water) was added and the mixture was stirred vigorously overnight at rt. Water was added and the mixture was extracted three times with EtOAc. The combined organic layers were dried over a phase separator and concentrated to dryness. The residue was taken up in DCM and the precipitate was filtered, washed with DCM and dried under vacuum at 40°C to afford 1-methyl-1H-indazole-5-carboxamide **(Intermediate 332:** 1.6 g, 64%) as a beige solid. $^1H$ NMR (400 MHz, DMSO-$d_6$) δ ppm 8.35 (dd, *J*= 1.5, 0.7 Hz, 1H), 8.17 (d, *J*= 0.9 Hz, 1H), 7.98 (s, 1H), 7.93 (dd, *J*= 8.8, 1.6 Hz, 1H), 7.67 (d, *J*= 8.8 Hz, 1H), 7.27 (s, 1H), 4.07 (s, 3H). Mass spec: m/z: 176 $[M+H]^+$.

**Example 80: N-{3-chloro-2-[(2R)-1-methylpyrrolidin-2-yl]-1H-pyrrolo[3,2-c]pyridin-6-yl}-1,3-dimethylindazole-6-carboxamide**

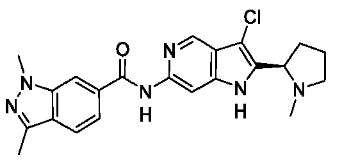

To a stirred solution of 1,3-dimethyl-N-{2-[(2R)-1-methylpyrrolidin-2-yl]-1H-pyrrolo[3,2-c]pyridin-6-yl}indazole-6-carboxamide **(Example 76,** 90 mg, 0.232 mmol) in DMF (2 mL) was added NCS (46 mg, 0.348 mmol) dropwise

at 0°C . The reaction mixture was stirred overnight at rt, then concentrated under reduced pressure. The residue was purified by preparative HPLC (Column: XBridge OBD, 30 × 150 mm, 5μm; Eluent: MeCN in Water (with 10 mmol/L $NH_4HCO_3$) from 34 to 64%; flow rate: 60 mL/min) to afford N-{3-chloro-2-[(2R)-1-methylpyrrolidin-2-yl]-1H-pyrrolo[3,2-c]pyridin-6-yl}-1,3-dimethylindazole-6-carboxamide **(Example 80,** 52.8 mg, 55%) as an off-white solid. $^1$H NMR (400 MHz, MeOH-$d_4$) δ ppm 8.52 (s, 1H), 8.28 (s, 1H), 8.17 (s, 1H), 7.83 - 7.85 (m, 1H), 7.72 - 7.74 (m, 1H), 4.09 (s, 3H), 3.67 - 3.73 (m, 1H), 3.22 - 3.26 (m, 1H), 2.64 (s, 3H), 2.42 - 2.47 (m, 1H), 2.27 (s, 4H), 1.92 - 2.12 (m, 3H). Mass spec: m/z: 423 [M+H]$^+$.

**Example 81: N-{3-chloro-2-[(2S)-1-methylpyrrolidin-2-yl]-1H-pyrrolo[3,2-c]pyridin-6-yl}-1,3-dimethylindazole-6-carboxamide**

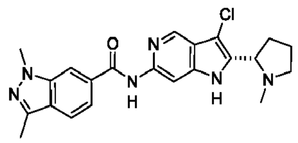

Following an analogous procedure to **Example 80,** using 1,3-dimethyl-N-{2-[(2S)-1-methylpyrrolidin-2-yl]-1H-pyrrolo[3,2-c]pyridin-6-yl}indazole-6-carboxamide **(Example 77,** 200 mg, 0.515 mmol), afforded N-{3-chloro-2-[(2S)-1-methylpyrrolidin-2-yl]-1H-pyrrolo[3,2-c]pyridin-6-yl}-1,3-dimethylindazole-6-carboxamide **(Example 81:** 64.8 mg, 31%) as an off-white solid. $^1$H NMR (400 MHz, MeOH-$d_4$) δ ppm 8.50 (s, 1H), 8.30 (s, 1H), 8.16 (s, 1H), 7.82 - 7.84 (m, 1H), 7.71 - 7.77 (m, 1H), 4.08 (s, 3H), 3.68 - 3.80 (m, 1H), 3.22 - 3.26 (m, 1H), 2.58 (s, 3H), 2.38 - 2.47 (m, 1H), 2.27 (s, 4H), 1.92 - 2.11 (m, 3H). Mass spec: m/z: 423 [M+H]$^+$.

**Example 82: (R)-1,3-dimethyl-N-(2-(1-methylpyrrolidin-2-yl)-3-(trifluoromethyl)-1H-pyrrolo[3,2-c]pyridin-6-yl)-1H-indazole-6-carboxamide**

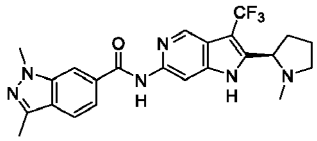

To a solution of (R)-1,3-dimethyl-N-(2-(1-methylpyrrolidin-2-yl)-1H-pyrrolo[3,2-c]pyridin-6-yl)-1H-indazole-6-carboxamide **(Example 76,** 400 mg, 0.978 mmol) in MeCN (10 mL) was added $K_2CO_3$ (270 mg, 1.96 mmol) and 2,8-difluoro-5-(trifluoromethyl)-5H-dibenzo[b,d]thiophen-5-ium trifluoromethanesulfonate (643 mg, 1.47 mmol) and the mixture was stirred at 80°C overnight. Water (1 mL) was added and the mixture chromatographed on a C18 column, eluting with water/MeCN (25/75) to provide crude product. The crude product was purified by Chiral-HPLC (Column: YMC-Actus Triart C18 ExRS, 30*150 mm, 5μm; Mobile Phase A: Water (+10mmol/L $NH_4HCO_3$ + 0.05% $NH_3.H_2O$), Mobile Phase B: MeCN; Flow rate: 60 mL/min Gradient: 5% B to 20% B in 8 min) to provide (R)-1,3-dimethyl-N-(2-(1-methylpyrrolidin-2-yl)-3-(trifluoromethyl)-1H-pyrrolo[3,2-c]pyridin-6-yl)-1H-indazole-6-carboxamide **(Example 82,** 3.1 mg, 0.7%) as a white solid. 1H NMR (400 MHz, DMSO-d6) δ ppm 12.01 (br, 1H), 10.88 (br, 1H), 8.43 - 8.52 (m, 2H), 7.73 - 7.82 (m, 2H), 6.53 - 6.66 (m, 1H), 4.07 (s, 3H), 3.45 - 3.53 (m, 1H), 3.15 - 3.25 (m, 1H), 2.55 (s, 3H), 2.35 - 2.40 (m, 2H), 2.12 - 2.26 (m, 3H), 1.70 - 2.05 (m, 3H). Mass spec: m/z: 457.1 [M+H]$^+$.

**Example 83: (S)-1,3-dimethyl-N-(2-(1-methylpyrrolidin-2-yl)-3-(trifluoromethyl)-1H-pyrrolo[3,2-c]pyridin-6-yl)-1H-indazole-6-carboxamide**

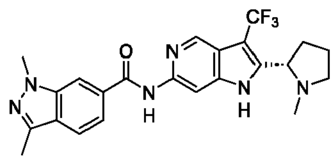

**Example 83** was synthesised in an analogous manner to **Example 82,** using (S)-1,3-dimethyl-N-(2-(1-methylpyrrolidin-2-yl)-1H-pyrrolo[3,2-c]pyridin-6-yl)-1H-indazole-6-carboxamide **(Example 77)** as the amide and 2,8-difluoro-5-(trifluoromethyl)-5H-dibenzo[b,d]thiophen-5-ium trifluoromethanesulfonate (CAS No: 1961266-44-3), to afford (S)-1,3-dimethyl-N-(2-(1-methylpyrrolidin-2-yl)-3-(trifluoromethyl)-1H-pyrrolo[3,2-c]pyridin-6-yl)-1H-indazole-6-carboxamide **(Example 83,** 15 mg, 3%) as a white solid. $^1H$ NMR (400 MHz, DMSO-$d_6$) δ ppm 12.15 (s, 1H), 10.76 (s, 1H), 8.62 (s, 1H), 8.43 (d, J = 14Hz, 2H), 7.74 - 7.82 (m, 2H), 4.06 (s, 3H), 3.62 - 3.65 (m, 1H), 3.20 - 3.24 (m, 1H), 2.50 (s, 3H), 2.30 - 2.35 (m, 1H), 2.20 - 2.24 (m, 4H), 1.90 - 2.00 (m, 1H), 1.70 - 1.90 (m, 2H). Mass spec: m/z: 457 [M+H]$^+$.

**Examples 84 and 85 were synthesized following Scheme 74**

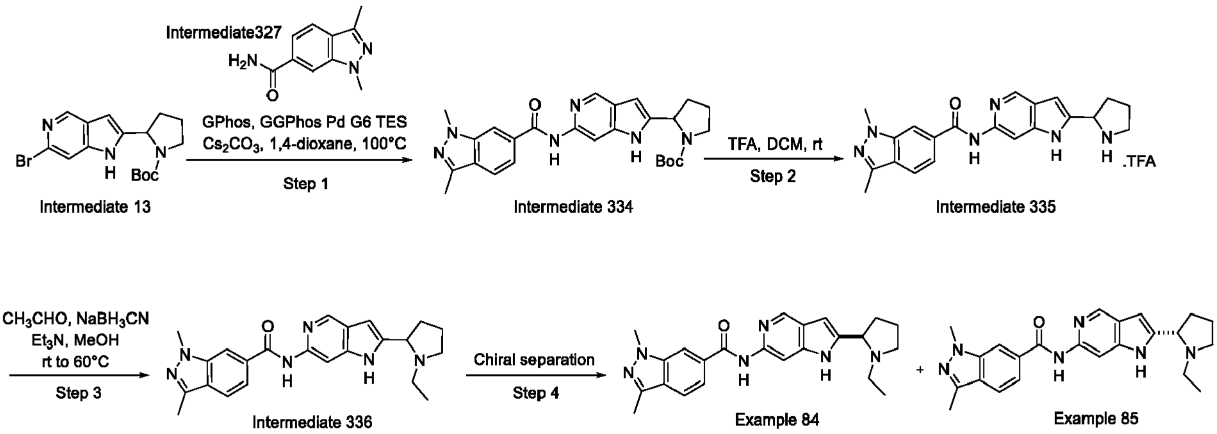


Scheme 74

**Step** 1

**Intermediate 334: rac-tert-butyl 2-[6-(1,3-dimethylindazole-6-amido)-1H-pyrrolo[3,2-c]pyridin-2-yl]pyrrolidine-1-carboxylate**

To a solution of rac-tert-butyl 2-{6-bromo-1H-pyrrolo[3,2-c]pyridin-2-yl}pyrrolidine-1-carboxylate **(Intermediate 13:** 700 mg, 1.91 mmol), 1,3-dimethylindazole-6-carboxamide **(Intermediate 327,** 434 mg, 2.29 mmol) , $Cs_2CO_3$ (1.25 g, 3.82 mmol) in 1,4-dioxane (20 mL) were added Gphos (103 mg, 0.191 mmol) and Gphos Pd G6 TES (180 mg, 0.191 mmol) . The reaction mixture was stirred overnight at 100°C under nitrogen atmosphere and quenched with water (80 mL). The resulting solution was extracted three times with EtOAc and the combined organic layers were washed with brine, dried over anhydrous $Na_2SO_4$, filtered, and concentrated under reduced pressure. The crude material was purified by flash chromatography on silica gel using DCM/MeOH (95/5) as eluent to afford rac-tert-butyl 2-[6-(1,3-dimethylindazole-6-amido)-1H-pyrrolo[3,2-c]pyridin-2-yl]pyrrolidine-1-carboxylate **(Intermediate 334:** 700 mg, 80%) as a yellow oil. Mass spec: m/z: 475 [M+H]$^+$.

**Step 2**

**Intermediate 335: 1,3-dimethyl-N-[2-(pyrrolidin-2-yl)-1H-pyrrolo[3,2-c]pyridin-6-yl]indazole-6-carboxamide trifluoroacetate**

To a solution of rac-tert-butyl 2-[6-(1,3-dimethylindazole-6-amido)-1H-pyrrolo[3,2-c]pyridin-2-yl]pyrrolidine-1-carboxylate **(Intermediate 334:** 700 mg, 1.48 mmol) in DCM (30 mL) was added TFA (10 mL). The reaction mixture was stirred for 2h at rt and concentrated under reduced pressure to provide rac-1,3-dimethyl-N-[2-(pyrrolidin-2-yl)-1H-pyrrolo[3,2-c]pyridin-6-yl]indazole-6-carboxamide trifluoroacetate **(Intermediate 335:** 600 mg) as a brown oil which was used in the next step without further purification. Mass spec: m/z: 375 [M+H]$^+$.

**Step 3**

**Intermediate 336: rac-N-[2-(1-ethylpyrrolidin-2-yl)-1H-pyrrolo[3,2-c]pyridin-6-yl]-1,3-dimethylindazole-6-carboxamide**

To a solution of rac-1,3-dimethyl-N-[2-(pyrrolidin-2-yl)-1H-pyrrolo[3,2-c]pyridin-6-yl]indazole-6-carboxamide **(Intermediate 335:** 120 mg, 0.320 mmol) in MeOH (4 mL) was added acetaldehyde (0.32 mL, 1.60 mmol, 5M in THF) and $Et_3N$ (97.3 mg, 0.960 mmol). The reaction mixture was stirred for 1h at rt. Then, $NaBH_3CN$ (48.5 mg, 0.771 mmol) was added and the reaction mixture was stirred for 1h at 60°C. The mixture was concentrated under reduced pressure and the crude material was chromatographed by reverse-phase flash chromatography (C18 aq) using MeCN/$H_2O$ (35/65) to provide rac-N-[2-(1-ethylpyrrolidin-2-yl)-1H-pyrrolo[3,2-c]pyridin-6-yl]-1,3-dimethylindazole-6-carboxamide **(Intermediate 336, 60** mg, 36%) as a brown oil. Mass spec: m/z: 403 $[M+H]^+$.

**Step 4**

**Example 84: N-{2-[(rel-2R)-1-ethylpyrrolidin-2-yl]-1H-pyrrolo[3,2-c]pyridin-6-yl}-1,3-dimethylindazole-6-carboxamide and Example 85: N-{2-[(rel-2S)-1-ethylpyrrolidin-2-yl]-1H-pyrrolo[3,2-c]pyridin-6-yl}-1,3-dimethylindazole-6-carboxamide**

Rac-N-[2-(1-ethylpyrrolidin-2-yl)-1H-pyrrolo[3,2-c]pyridin-6-yl]-1,3-dimethylindazole-6-carboxamide **(Intermediate 336,** 60 mg) was separated into the enantiomeric **Example 84** and **Example 85** using the following conditions: column: ChiralPak IF 2 × 25 cm, 5.0 um; Eluent: MTBE (0.5% of 2M solution $NH_3$ in MeOH)/EtOH 95/5; Flow rate: 20 mL/min to provide:

**Example 84: N-{2-[(rel-2R)-1-ethylpyrrolidin-2-yl]-1H-pyrrolo[3,2-c]pyridin-6-yl}-1,3-dimethylindazole-6-carboxamide**

First eluting compound (retention time = 11.8 min)
Mass spec: m/z: 403 $[M+H]^+$; $^1H$ NMR (400 MHz, MeOH-$d_4$) δ ppm 8.57 (s, 1H), 8.22 - 8.25 (m, 2H), 7.87 - 7.88 (m, 1H), 7.79 - 7.80 (m, 1H), 6.54 (s, 1H), 4.13 (s, 3H), 3.60 - 3.62 (m, 1H), 3.39 (s, 1H), 2.77 - 2.79 (m, 1H), 2.63 (s, 3H), 2.33 - 2.38 (m, 3H), 1.99 - 2.11 (m, 3H), 1.05 - 1.19 (m, 3H).

**Example 85: N-{2-[(rel-2S)-1-ethylpyrrolidin-2-yl]-1H-pyrrolo[3,2-c]pyridin-6-yl}-1,3-dimethylindazole-6-carboxamide**

Second eluting compound (retention time = 16.7 min)
Mass spec: m/z: 403 $[M+H]^+$; $^1H$ NMR (400 MHz, MeOH-$d_4$) δ ppm 8.52 (s, 1H), 8.17 - 8.20 (m, 2H), 7.83 - 7.85 (m, 1H), 7.72 - 7.74 (m, 1H), 6.50 (s, 1H), 4.09 (s, 3H), 3.60 - 3.62 (m, 1H), 3.39 (s, 1H), 2.75 - 2.77 (m, 1H), 2.58 (s, 3H), 2.33 - 2.38 (m, 3H), 1.99 - 2.11 (m, 3H), 1.05 - 1.19 (m, 3H).

**Examples 86 and 87 were synthesized following Scheme 75**

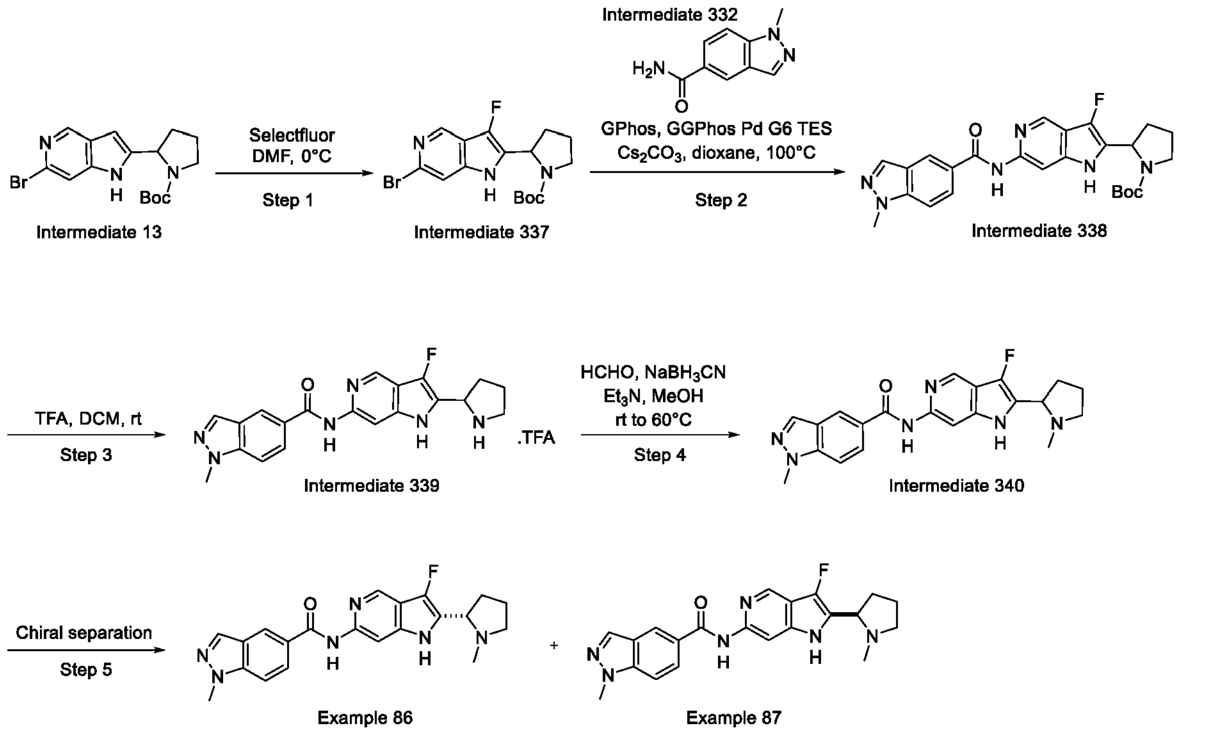

Intermediate 13 Intermediate 337 Intermediate 338

Intermediate 339 Intermediate 340

Example 86 + Example 87

**Scheme 75**

**Step 1**

**Intermediate 337: rac-tert-butyl 2-{6-bromo-3-fluoro-1H-pyrrolo[3,2-c]pyridin-2-yl}pyrrolidine-1-carboxylate**

To a solution of rac-tert-butyl 2-{6-bromo-1H-pyrrolo[3,2-c]pyridin-2-yl}pyrrolidine-1-carboxylate **(Intermediate 13:** 6.40 g, 0.015 mol) in DMF (4 mL) was added selectfluor (5.55 g, 0.015 mol). The reaction mixture was stirred at 0°C for 3h and quenched with water. The mixture was concentrated under reduced pressure and the residue was chromatographed by reverse-phase flash chromatography (C18 silica) using MeCN/$H_2O$ (55/45) to provide rac-tert-butyl 2- f 6-bromo-3-fluoro-1H-pyrrolo[3,2-c]pyridin-2-yl}pyrrolidine-1-carboxylate **(Intermediate 337:** 800 mg, 12%) as a yellow oil. Mass spec: m/z 384 $[M+H]^+$.

**Step 2**

**Intermediate 338: rac-tert-butyl 2-[3-fluoro-6-(1-methylindazole-5-amido)-1H-pyrrolo[3,2-c]pyridin-2-yl]pyrrolidine-1-carboxylate**

To a solution of rac-tert-butyl 2- f 6-bromo-3-fluoro-1H-pyrrolo[3,2-c]pyridin-2-yl}pyrrolidine-1-carboxylate **(Intermediate 337:** 750 mg, 1.75 mmol) in 1,4-dioxane (8 mL) were added 1-methylindazole-5-carboxamide **(Intermediate 332:** 410 mg, 2.11 mmol), $Cs_2CO_3$ (1.71 g, 5.25 mmol), GPhos (94.0 mg, 0.175 mmol) and GPhos Pd G6 TES (166 mg, 0.175 mmol) under nitrogen atmosphere. The reaction mixture was stirred at 100°C for 3h and then quenched with water. The resulting solution was extracted three times with EtOAc and the combined organic layers were washed with brine, dried over anhydrous $Na_2SO_4$, filtered, and concentrated under reduced pressure. The crude material was purified by flash chromatography on silica gel using DCM/MeOH (96/4) as eluent to afford rac-tert-butyl 2-[3-fluoro-6-(1-methylindazole-5-amido)-1H-pyrrolo[3,2-c]pyridin-2-yl]pyrrolidine-1-carboxylate **(Intermediate 338:** 350 mg, 37%) as a yellow solid. Mass spec: m/z 479 $[M+H]^+$.

**Step 3**

**Intermediate 339: rac-N-[3-fluoro-2-(pyrrolidin-2-yl)-1H-pyrrolo[3,2-c]pyridin-6-yl]-1-methylindazole-5-carboxamide trifluoroacetate**

To a solution of rac-tert-butyl 2-[3-fluoro-6-(1-methylindazole-5-amido)-1H-pyrrolo[3,2-c]pyridin-2-yl]pyrrolidine-1-carboxylate **(Intermediate 338:** 350 mg, 0.811 mmol) in DCM (3 mL) was added TFA (1 mL). The reaction mixture was stirred at rt for 2h and concentrated under reduced pressure to provide rac-N-[3-fluoro-2-(pyrrolidin-2-yl)-1H-pyrrolo [3,2-c]pyridin-6-yl]-1-methylindazole-5-carboxamide trifluoroacetate (**Intermediate 339**: 250 mg, 90%) as a yellow oil which was used in Step 4 without further purification. Mass spec: m/z 379 $[M+H]^+$.

**Step 4**

**Intermediate 340: rac-N-(3-fluoro-2-(1-methylpyrrolidin-2-yl)-1H-pyrrolo[3,2-c]pyridin-6-yl)-1-methyl-1H-indazole-5-carboxamide**

To a solution of rac-N-[3-fluoro-2-(pyrrolidin-2-yl)-1H-pyrrolo[3,2-c]pyridin-6-yl]-1-methylindazole-5-carboxamide trifluoroacetate (**Intermediate 339**: 25 mg, 0.059 mmol) in MeOH (1 mL) were added paraformaldehyde (8.86 mg, 0.295 mmol) and $Et_3N$ (29.9 mg, 0.295 mmol). The reaction mixture was stirred at rt for 30 min prior addition of $NaBH_3CN$ (11.1 mg, 0.177 mmol). The reaction mixture was stirred at 60°C for 1h and quenched with water. The mixture was concentrated under reduced pressure and the residue was chromatographed by reverse-phase flash chromatography (C18) using $MeCN/H_2O$ (86/14) to provide rac-N-(3-fluoro-2-(1-methylpyrrolidin-2-yl)-1H-pyrrolo[3,2-c]pyridin-6-yl)-1-methyl-1H-indazole-5-carboxamide (**Intermediate 340:** 20 mg, 77%) as a white solid. Mass spec: m/z 393 $[M+H]^+$.

**Step 5**

**Example 86: N-{3-fluoro-2-[(rel-2S)-1-methylpyrrolidin-2-yl]-1H-pyrrolo[3,2-c]pyridin-6-yl}-1-methylindazole-5-carboxamide and Example 87: N-{3-fluoro-2-[(rel-2R)-1-methylpyrrolidin-2-yl]-1H-pyrrolo[3,2-c]pyridin-6-yl}-1-methylindazole-5-carboxamide**

Rac-N-(3-fluoro-2-(1-methylpyrrolidin-2-yl)-1H-pyrrolo[3,2-c]pyridin-6-yl)-1-methyl-1H-indazole-5-carboxamide (**Intermediate 340**, 20 mg) was separated into the enantiomeric **Example 86** and **Example 87** using the following conditions: column: Chiral Art Cellulose-SC 2 × 25 cm, 5.0 um; Eluent: (Hexane/DCM 3/1 (0.5% 2M $NH_3$ in MeOH))/IPA 90/10; Flow rate: 20 mL/min to provide:

**Example 86: N-{3-fluoro-2-[(rel-2S)-1-methylpyrrolidin-2-yl]-1H-pyrrolo[3,2-c]pyridin-6-yl}-1-methylindazole-5-carboxamide**

First eluting compound (retention time = 9.1 min)
Mass spec: m/z: 393 $[M+H]^+$; $^1H$ NMR (400 MHz, MeOH-$d_4$) δ ppm 8.48 - 8.55 (m, 1H), 8.48 (s, 1H), 8.20 - 8.21 (m, 1H), 8.18 - 8.20 (m, 1H), 8.04 - 8.07 (m, 1H), 7.67 - 7.70 (m, 1H), 4.12 (s, 3H), 3.58 - 3.62 (m, 1H), 3.18 - 3.20 (m, 1H), 2.38 - 2.43 (m, 1H), 2.23 - 2.28 (m, 4H), 2.05 - 2.10 (m, 2H), 1.90 - 2.00 (m, 1H).

**Example 87: N-{3-fluoro-2-[(rel-2R)-1-methylpyrrolidin-2-yl]-1H-pyrrolo[3,2-c]pyridin-6-yl1-1-methylindazole-5-carboxamide**

Second eluting compound (retention time = 13.3 min)
Mass spec: m/z: 393 $[M+H]^+$; $^1H$ NMR (400 MHz, MeOH-$d_4$) δ ppm 8.56 (s, 1H), 8.48 (s, 1H), 8.20 - 8.21 (m, 2H), 8.04 - 8.07 (m, 1H), 7.68 - 7.70 (m, 1H), 4.12 (s, 3H), 3.59 - 3.63 (m, 1H), 3.19 - 3.23 (m, 1H), 2.41 - 2.43 (m, 1H), 2.22 - 2.28 (m, 4H), 1.92 - 2.11 (m, 3H).

**Examples 88 and 89 were made following Scheme 76**

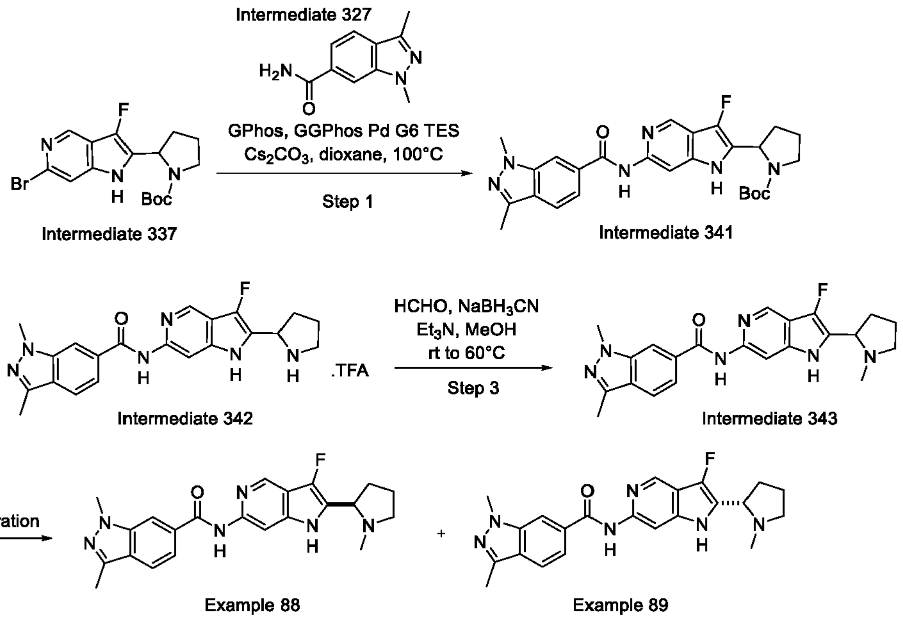


## Scheme 76

### Step 1

**Intermediate 341: rac-tert-butyl 2-[6-(1,3-dimethylindazole-6-amido)-3-fluoro-1H-pyrrolo[3,2-c]pyridin-2-yl]pyrrolidine-1-carboxylate**

Following an analogous procedure to **Scheme 75, Step 2,** using rac-tert-butyl 2-{6-bromo-3-fluoro-1H-pyrrolo [3,2-c]pyridin-2-yl}pyrrolidine-1-carboxylate as the bromine intermediate (**Intermediate 337**: 140 mg, 0.328 mmol) and 1,3-dimethylindazole-6-carboxamide as the amine (**Intermediate 327:** 83 mg, 0.393 mmol), afforded after purification by reverse-phase flash chromatography (C18 aq) using MeCN/$H_2O$ (72/28) rac-tert-butyl 2-[6-(1,3-dimethylindazole-6-amido)-3-fluoro-1H-pyrrolo[3,2-c]pyridin-2-yl]pyrrolidine-1-carboxylate (**Intermediate 341:** 100 mg, 56%) as a yellow oil. Mass spec: m/z: 493 $[M+H]^+$.

### Step 2

**Intermediate 342: rac-N-[3-fluoro-2-(pyrrolidin-2-yl)-1H-pyrrolo[3,2-c]pyridin-6-yl]-1,3-dimethylindazole-6-carboxamide trifluoroacetate**

Following an analogous procedure to **Scheme 75, Step 3,** using rac-tert-butyl 2-[6-(1,3-dimethylindazole-6-amido)-3-fluoro-1H-pyrrolo[3,2-c]pyridin-2-yl]pyrrolidine-1-carboxylate (**Intermediate 341**: 100 mg, 0.182 mmol), afforded rac-N-[3-fluoro-2-(pyrrolidin-2-yl)-1H-pyrrolo[3,2-c]pyridin-6-yl]-1,3-dimethylindazole-6-carboxamide trifluoroacetate (**Intermediate 342**: 75 mg, 96%) as a yellow oil. Mass spec: m/z: 393 $[M+H]^+$.

### Step 3

**Intermediate 343: rac-N-(3-fluoro-2-(1-methylpyrrolidin-2-yl)-1H-pyrrolo[3,2-c]pyridin-6-yl)-1,3-dimethyl-1H-indazole-6-carboxamide**

Following an analogous procedure to **Scheme 75, Step 4,** using rac-tert-butyl 2-[6-(1,3-dimethylindazole-6-amido)-3-fluoro-1H-pyrrolo[3,2-c]pyridin-2-yl]pyrrolidine-1-carboxylate trifluoroacetate (**Intermediate 342:** 70 mg, 0.176 mmol), afforded rac-N-(3-fluoro-2-(1-methylpyrrolidin-2-yl)-1H-pyrrolo[3,2-c]pyridin-6-yl)-1,3-dimethyl-1H-indazole-6-carboxamide (**Intermediate 343**: 50 mg, 65%). Mass spec: m/z: 407 $[M+H]^+$.

### Step 4

**Example 88: N-{3-fluoro-2-[(rel-2R)-1-methylpyrrolidin-2-yl]-1H-pyrrolo[3,2-c]pyridin-6-yl}-1,3-dimethylindazole-6-carboxamide and Example 89: N-{3-fluoro-2-[(rel-2S)-1-methylpyrrolidin-2-yl]-1H-pyrrolo[3,2-c]pyridin-6-yl}-1,3-dimethylindazole-6-carboxamide**

Rac-N-(3-fluoro-2-(1-methylpyrrolidin-2-yl)-1H-pyrrolo[3,2-c]pyridin-6-yl)-1,3-dimethyl-1H-indazole-6-carboxamide (**Intermediate 343**, 50 mg) was separated into the enantiomeric **Example 88** and **Example 89** using the following conditions: column: Chiral Art Amylose-SA 2 × 25 cm, 5.0 um; Eluent: (Hexane (0.5% 2M $NH_3$ in MeOH))/EtOH 85/15; Flow rate: 20 mL/min to provide:

**Example 88: N-{3-fluoro-2-[(rel-2R)-1-methylpyrrolidin-2-yl]-1H-pyrrolo[3,2-c]pyridin-6-yl1-1,3-dimethylindazole-6-carboxamide**

First eluting compound (retention time = 11.3 min)
Mass spec: m/z: 407 $[M+H]^+$; $^1H$ NMR (400 MHz, DMSO-$d_6$) δ ppm 11.29 (s, 1H), 10.64 (s, 1H), 8.59 (s, 1H), 8.42 (s, 1H), 8.25 - 8.26 (m, 1H), 7.72 - 7.81 (m, 2H), 4.05 (s, 3H), 3.33 - 3.47 (m, 1H), 3.14 - 3.16 (m, 1H), 2.50 - 2.51 (m, 3H), 2.24 - 2.31 (m, 1H), 2.13 - 2.22 (m, 4H), 1.91 - 1.95 (m, 3H).

**Example 89: N-{3-fluoro-2-[(rel-2S)-1-methylpyrrolidin-2-yl]-1H-pyrrolo[3,2-c]pyridin-6-yl}-1,3-dimethylindazole-6-carboxamide**

Second eluting compound (retention time = 14.2 min)
Mass spec: m/z: 407 $[M+H]^+$; $^1H$ NMR (400 MHz, DMSO-$d_6$) δ ppm 11.29 (s, 1H), 10.64 (s, 1H), 8.59 (s, 1H), 8.42 (s, 1H), 8.25 (s, 1H), 7.72 - 7.81 (m, 2H), 4.05 (s, 3H), 3.47 - 3.49 (m, 1H), 3.13 - 3.16 (m, 1H), 2.50 - 2.67 (m, 3H), 2.29 - 2.33 (m, 1H), 2.13 - 2.27 (m, 4H), 1.82 - 1.97 (m, 3H).

**Examples 90 and 91 were synthesized following Scheme 77**

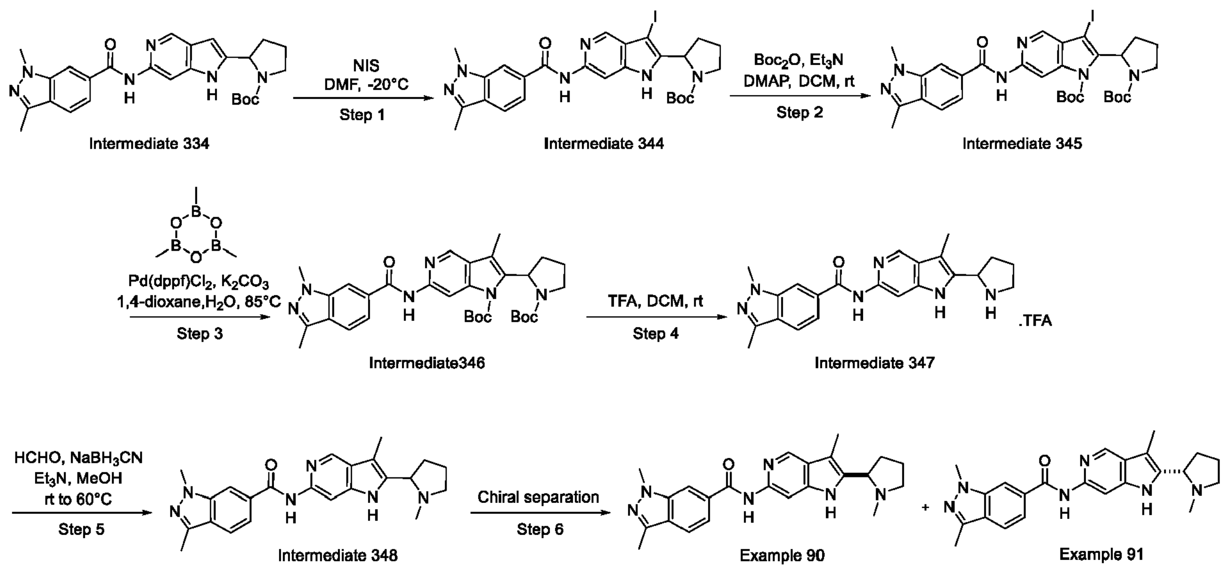


Scheme 77

**Step 1**

**Intermediate 344: rac-tert-butyl 2-[6-(1,3-dimethylindazole-6-amido)-3-iodo-1H-pyrrolo[3,2-c]pyridin-2-yl]pyrrolidine-1-carboxylate**

To a solution of rac-tert-butyl 2-[6-(1,3-dimethylindazole-6-amido)-1H-pyrrolo[3,2-c]pyridin-2-yl]pyrrolidine-1-carboxylate (**Intermediate 334**, 250 mg, 0.473 mol) in DMF (5 mL) cooled to -20°C was added NIS (128 mg, 0.567 mmol). The reaction mixture was stirred at -20°C for 2h and then quenched with water. The resulting solution was extracted three

times with EtOAc and the combined organic layers were washed with brine, dried over anhydrous $Na_2SO_4$, filtered, and concentrated under reduced pressure. The crude material was purified by flash chromatography on silica gel using petroleum ether/EtOAc (52/48) as eluent to afford rac-tert-butyl 2-[6-(1,3-dimethylindazole-6-amido)-3-iodo-1H-pyrrolo [3,2-c]pyridin-2-yl]pyrrolidine-1-carboxylate (**Intermediate 344**, 200 mg, 63%) as a yellow oil. Mass spec: m/z 601 $[M+H]^+$.

**Step 2**

**Intermediate 345: rac-tert-butyl 2-[1-(tert-butoxycarbonyl)-6-[N-(tert-butoxycarbonyl)1,3-dimethylindazole-6-amido]-3-iodopyrrolo[3,2-c]pyridin-2-yl] pyrrolidine- 1-carboxylate**

To a solution of rac-tert-butyl 2-[6-(1,3-dimethylindazole-6-amido)-3-iodo-1H-pyrrolo[3,2-c]pyridin-2-yl]pyrrolidine-1-carboxylate (**Intermediate 344,** 200 mg, 0.299 mmol) in DCM (10 mL) were added $(Boc)_2O$ (130 mg, 0.599 mmol), DMAP (10.9 mg, 0.090 mmol) and $Et_3N$ (121 mg, 1.20 mmol). The reaction mixture was stirred at rt for 2h and then quenched with water. The resulting solution was extracted three times with EtOAc and the combined organic layers were washed with brine, dried over anhydrous $Na_2SO_4$, filtered, and concentrated under reduced pressure. The crude material was purified by flash chromatography on silica gel using petroleum ether/EtOAc (6/4) as eluent to afford rac-tert-butyl 2-[1-(tert-butoxycarbonyl)-6-[N-(tert-butoxycarbonyl)1,3-dimethylindazole-6-amido]-3-iodopyrrolo[3,2-c]pyridin-2-yl] pyrrolidine-1-carboxylate (**Intermediate 345,** 200 mg, 77%) as a yellow oil. Mass spec: m/z 801 $[M+H]^+$.

**Step 3**

**Intermediate 346: rac-tert-butyl 2-(1-(tert-butoxycarbonyl)pyrrolidin-2-yl)-6-(1,3-dimethyl-1H-indazole-6-carboxamido)-3-methyl-1H-pyrrolo[3,2-c]pyridine-1-carboxylate**

To a solution of rac-tert-butyl 2-[1-(tert-butoxycarbonyl)-6-[N-(tert-butoxycarbonyl)1,3-dimethylindazole-6-amido]-3-iodopyrrolo[3,2-c]pyridin-2-yl]pyrrolidine-1-carboxylate (**Intermediate 345**, 200 mg, 0.230 mmol) in 1,4-dioxane (4 mL)/$H_2O$ (1 mL) were added trimethyl-1,3,5,2,4,6-trioxatriborinane (34.5 mg, 0.275 mmol), $Pd(dppf)Cl_2$ (33.7 mg, 0.046 mmol) and $K_2CO_3$ (63.5 mg, 0.460 mmol). The reaction mixture was stirred at 80°C overnight under nitrogen atmosphere and quenched with water. The resulting solution was extracted three times with EtOAc and the combined organic layers were washed with brine, dried over anhydrous $Na_2SO_4$, filtered, and concentrated under reduced pressure. The crude material was purified by flash chromatography on silica gel using petroleum ether/EtOAc (75/25) as eluent to afford rac-tert-butyl 2-[1-(tert-butoxycarbonyl)-6-[N-(tert-butoxycarbonyl)1,3-dimethylindazole-6-amido]-3-methylpyrrolo[3,2-c] pyridin-2-yl]pyrrolidine-1-carboxylate (**Intermediate 346**, 100 mg, 51%) as a yellow oil. Mass spec: m/z 689 $[M+H]^+$.

**Step 4**

**Intermediate 347: rac-1,3-dimethyl-N-[3-methyl-2-(pyrrolidin-2-yl)-1H-pyrrolo[3,2-c]pyridin-6-yl]indazole-6-carboxamide trifluoroacetate**

To a solution of rac-tert-butyl 2-[1-(tert-butoxycarbonyl)-6-[N-(tert-butoxycarbonyl)1,3-dimethylindazole-6-amido]-3-methylpyrrolo[3,2-c]pyridin-2-yl]pyrrolidine-1-carboxylate (**Intermediate 346**, 100 mg, 0.116 mmol) in DCM (3 mL) was added TFA (1 mL). The reaction mixture was stirred at rt for 2h and concentrated under reduced pressure to afford rac-1,3-dimethyl-N-[3-methyl-2-(pyrrolidin-2-yl)-1H-pyrrolo[3,2-c]pyridin-6-yl]indazole-6-carboxamide trifluoroacetate (**Intermediate 347**, 55 mg, 89%) as a yellow solid, which was used in Step 5 without further purification. Mass spec: m/z 389 $[M+H]^+$.

**Step 5**

**Intermediate 348: rac-1,3-dimethyl-N-(3-methyl-2-(1-methylpyrrolidin-2-yl)-1H-pyrrolo[3,2-c]pyridin-6-yl)-1H-indazole-6-carboxamide**

To a solution of rac-1,3-dimethyl-N-[3-methyl-2-(pyrrolidin-2-yl)-1H-pyrrolo[3,2-c]pyridin-6-yl]indazole-6-carboxamide trifluoroacetate (**Intermediate 347**, 40.0 mg, 0.082 mmol) in MeOH (1 mL) were added paraformaldehyde (12.4 mg, 0.411 mmol) and $Et_3N$ (24.9 mg, 0.246 mmol). The reaction mixture was stirred at rt for 30 min prior addition of $NaBH_3CN$ (15.4 mg, 0.246 mmol). The reaction mixture was stirred at 60°C for 2h and quenched with water. The mixture was concentrated under reduced pressure and the residue was chromatographed by reverse-phase flash chromatography (C18) using MeCN/$H_2O$ (75/25) to provide rac-1,3-dimethyl-N-(3-methyl-2-(1-methylpyrrolidin-2-yl)-1H-pyrrolo [3,2-c]pyridin-6-yl)-1H-indazole-6-carboxamide (**Intermediate 348,** 25 mg). Mass spec: m/z 403 $[M+H]^+$.

**Step 6**

**Example 90: 1,3-dimethyl-N-{3-methyl-2-[(rel-2R)-1-methylpyrrolidin-2-yl]-1H-pyrrolo[3,2-c]pyridin-6-yl}indazole-6-carboxamide and Example 91: 1,3-dimethyl-N-{3-methyl-2-[(rel-2S)-1-methylpyrrolidin-2-yl]-1H-pyrrolo[3,2-c]pyridin-6-yl}indazole-6-carboxamide**

Rac-1,3-dimethyl-N-(3-methyl-2-(1-methylpyrrolidin-2-yl)-1H-pyrrolo[3,2-c]pyridin-6-yl)-1H-indazole-6-carboxamide (**Intermediate 348, 25** mg) was separated into the enantiomeric **Example 90** and **Example 91** using the following conditions: column: Chiral Art Amylose-SA 2 × 25 cm, 5.0 um; Eluent: Hexane (0.5% 2M $NH_3$ in MeOH)/EtOH 75/25; Flow rate: 20 mL/min to provide:

**Example 90: 1,3-dimethyl-N-{3-methyl-2-[(rel-2R)-1-methylpyrrolidin-2-yl]-1H-pyrrolo[3,2-c]pyridin-6-yl}indazole-6-carboxamide**

First eluting compound (retention time = 11.3 min)
Mass spec: m/z: 403 $[M+H]^+$; $^1H$ NMR (400 MHz, MeOH-$d_4$) δ ppm 8.58 (s, 1H), 8.17 - 8.20 (m, 2H), 7.83 - 7.85 (m, 1H), 7.71 - 7.74 (m, 1H), 4.08 - 4.27 (m, 4H), 3.41 - 3.55 (m, 1H), 2.78 - 2.91 (m, 1H), 2.51 - 2.62 (m, 3H), 2.48 - 2.50 (m, 3H), 2.35 - 2.47 (m, 4H), 2.06 - 2.34 (m, 3H).

**Example 91: 1,3-dimethyl-N-{3-methyl-2-[(rel-2S)-1-methylpyrrolidin-2-yl]-1H-pyrrolo[3,2-c]pyridin-6-yl}indazole-6-carboxamide**

Second eluting compound (retention time = 14.4 min)
Mass spec: m/z: 403 $[M+H]^+$; $^1H$ NMR (400 MHz, MeOH-$d_4$) δ ppm 8.64 (s, 1H), 8.18 - 8.24 (m, 2H), 7.83 - 7.86 (m, 1H), 7.72 - 7.74 (m, 1H), 4.53 - 4.58 (m, 1H), 4.08 - 4.10 (m, 3H), 3.47 - 3.71 (m, 1H), 3.02 - 3.18 (m, 1H), 2.61 - 2.71 (m, 3H), 2.52 - 2.60 (m, 3H), 2.31 - 2.51 (m, 5H), 2.15 - 2.30 (m, 2H).

**Examples 92 and 93 were synthesized following Scheme 78**

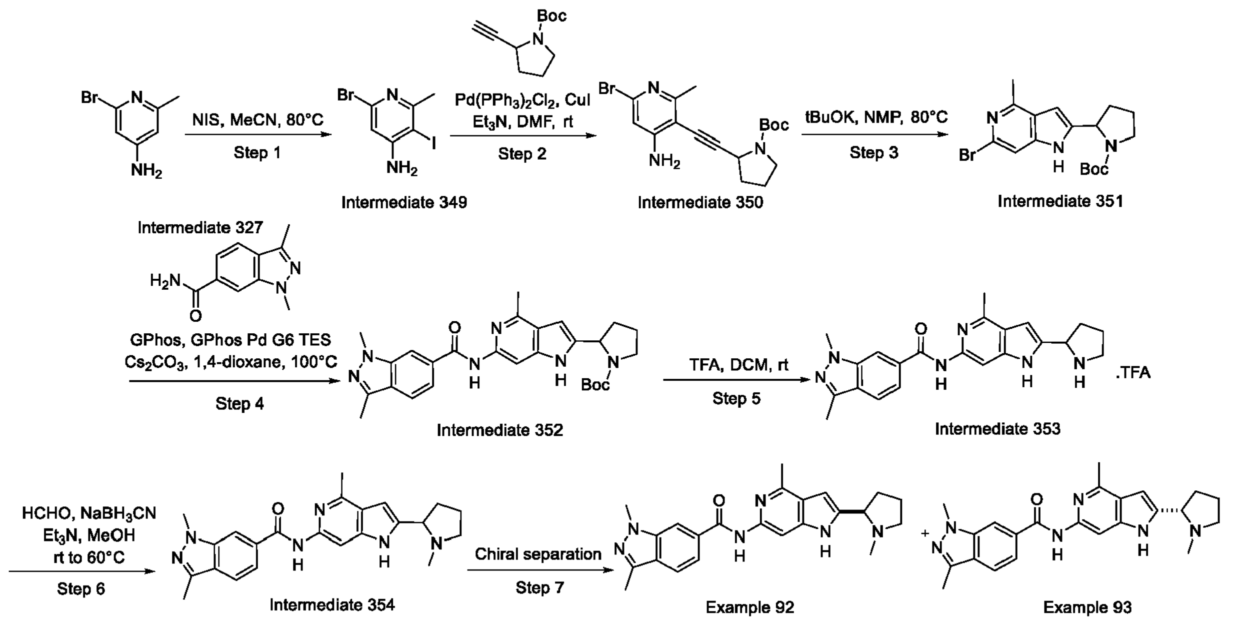


**Scheme 78**

**Step 1**

**Intermediate 349: 2-bromo-5-iodopyridin-4-amine**

To a solution of 2-bromo-6-methylpyridin-4-amine (CAS: 79055-59-7, 4 g, 21.4 mmol) in MeCN (15 mL) was added NIS (5.77 g, 25.7 mmol). The reaction mixture was stirred overnight at 80°C and then quenched with water (200 mL). The resulting solution was extracted three times with EtOAc and the combined organic layers were washed with brine, dried over anhydrous $Na_2SO_4$, filtered, and concentrated under reduced pressure. The crude material was purified by flash chromatography on silica gel using petroleum ether/EtOAc (9/91) as eluent to provide 6-bromo-3-iodo-2-methylpyridin-4-amine (**Intermediate 349**: 1.1 g, 16%) as a white solid. Mass spec: m/z: 313 $[M+H]^+$.

**Step 2**

**Intermediate 350: rac-tert-butyl 2-[2-(4-amino-6-bromo-2-methylpyridin-3-yl)ethynyl] pyrrolidine- 1-carboxylate**

A solution of 6-bromo-3-iodo-2-methylpyridin-4-amine (**Intermediate 349**, 1.1 g, 3.52 mmol), rac-tert-butyl 2-ethynylpyrrolidine-1-carboxylate (CAS: 316141-37-4, 824 mg, 4.22 mmol), $Pd(PPh_3)_2Cl_2$ (247 mg, 0.352 mmol), CuI (66.9 mg, 0.352 mmol) and $Et_3N$ (1.07g, 10.5 mmol) in DMF (15 mL) was stirred overnight at rt under nitrogen atmosphere. The mixture was concentrated under reduced pressure and the residue was chromatographed by reverse-phase flash chromatography (C18) using MeCN/$H_2O$ (38/62) to provide rac-tert-butyl 2-[2-(4-amino-6-bromo-2-methylpyridin-3-yl) ethynyl]pyrrolidine-1-carboxylate (**Intermediate 350**, 250 mg, 16%) as a brown oil. Mass spec: m/z: 380 $[M+H]^+$.

**Step 3**

**Intermediate 351: rac-tert-butyl 2-{6-bromo-4-methyl-1H-pyrrolo[3,2-c]pyridin-2-yl}pyrrolidine-1-carboxylate)**

To a solution of rac-tert-butyl 2-[2-(4-amino-6-bromo-2-methylpyridin-3-yl)ethynyl]pyrrolidine-1-carboxylate (**Intermediate 350**: 250 mg, 0.657 mmol) in NMP (5 mL) was added a solution of tBuOK (1.97 mL, 1.97 mmol, 1M in THF). The reaction mixture was stirred for 4h at 80°C, then quenched with water. The resulting solution was extracted three times with EtOAc and the combined organic layers washed with brine, dried over anhydrous $Na_2SO_4$, filtered, and concentrated under reduced pressure. The crude material was purified by flash chromatography on silica gel using petroleum ether/EtOAc (1/1) as eluent to provide rac-tert-butyl 2-{6-bromo-4-methyl-1H-pyrrolo[3,2-c]pyridin-2-yl}pyrrolidine-1-carboxylate) (**Intermediate 351**, 130 mg, 55%) as a colorless oil. Mass spec: m/z: 380 $[M+H]^+$.

**Step 4**

**Intermediate 352: rac-tert-butyl 2-[6-(1,3-dimethylindazole-6-amido)-4-methyl-1H-pyrrolo[3,2-c]pyridin-2-yl] pyrrolidine-1-carboxylate**

A solution of rac-tert-butyl 2-{6-bromo-4-methyl-1H-pyrrolo[3,2-c]pyridin-2-yl}pyrrolidine-1-carboxylate (**Intermediate 351**, 130 mg, 0.342 mmol), 1,3-dimethylindazole-6-carboxamide (**Intermediate 327**, 77.6 mg, 0.410 mmol), GPhos (18.35 mg, 0.034 mmol), GPhos Pd G6 TES (32.3 mg, 0.034 mmol) and $Cs_2CO_3$ (334 mg, 1.03 mmol) in 1,4-dioxane (3 mL) was stirred for 3h at 100°C under nitrogen atmosphere. The reaction mixture was concentrated under reduced pressure and the crude material was purified by flash chromatography on silica gel using DCM/MeOH (97/3) as eluent to provide rac-tert-butyl 2-[6-(1,3-dimethylindazole-6-amido)-4-methyl-1H-pyrrolo[3,2-c]pyridin-2-yl]pyrrolidine-1-carboxylate (**Intermediate 352**, 130 mg, 80%) as a brown oil. Mass spec: m/z: 489 $[M+H]^+$.

**Step 5**

**Intermediate 353: rac-1,3-dimethyl-N-[4-methyl-2-(pyrrolidin-2-yl)-1H-pyrrolo[3,2-c]pyridin-6-yl]indazole-6-carboxamide trifluoroacetate**

To a solution of rac-tert-butyl 2-[6-(1,3-dimethylindazole-6-amido)-4-methyl-1H-pyrrolo[3,2-c]pyridin-2-yl]pyrrolidine-1-carboxylate (**Intermediate 352**, 130 mg, 0.266 mmol) in DCM (4 mL) was added TFA (0.8 mL). The reaction mixture was stirred for 2h at rt, then concentrated under reduced pressure to provide rac-1,3-dimethyl-N-[4-methyl-2-(pyrrolidin-2-yl)-1H-pyrrolo[3,2-c]pyridin-6-yl]indazole-6-carboxamide trifluoroacetate (**Intermediate 353**, 100 mg) as a colorless oil which was used in Step 6 without further purification. Mass spec: m/z: 389 $[M+H]^+$.

**Step 6**

**Intermediate 354: rac-1,3-dimethyl-N-[4-methyl-2-(1-methylpyrrolidin-2-yl)-1H-pyrrolo[3,2-c]pyridin-6-yl]indazole-6-carboxamide**

To a solution of rac-1,3-dimethyl-N-[4-methyl-2-(1-methylpyrrolidin-2-yl)-1H-pyrrolo[3,2-c]pyridin-6-yl]indazole-6-carboxamide (**Intermediate 353:** 100 mg, 0.257 mmol) in MeOH (4 mL) were added paraformaldehyde (38.7 mg, 1.29 mmol) and $Et_3N$ (78.2 mg, 0.771 mmol). The reaction mixture was stirred for 1h at rt prior addition of $NaBH_3CN$ (48.5 mg, 0.771 mmol). The reaction mixture was stirred for 1h at 60°C, then concentrated under reduced pressure. The residue was chromatographed by reverse-phase flash chromatography (C18) using MeCN/$H_2O$ (35/65) to afford rac-1,3-dimethyl-N-[4-methyl-2-(1-methylpyrrolidin-2-yl)-1H-pyrrolo[3,2-c]pyridin-6-yl]indazole-6-carboxamide (**Intermediate 354:** 60 mg, 68%) as a brown oil. Mass spec: m/z: 403 [M+H]+.

**Step 7**

**Example 92: 1,3-dimethyl-N-{4-methyl-2-[(rel-2R)-1-methylpyrrolidin-2-yl]-1H-pyrrolo[3,2-c]pyridin-6-yl}indazole-6-carboxamide and Example 93: 1,3-dimethyl-N-{4-methyl-2-[(rel-2S)-1-methylpyrrolidin-2-yl]-1H-pyrrolo [3,2-c] pyridin-6-yl}indazole-6-carboxamide**

Rac-1,3-dimethyl-N-[4-methyl-2-(1-methylpyrrolidin-2-yl)-1H-pyrrolo[3,2-c]pyridin-6-yl]indazole-6-carboxamide (**Intermediate 354, 60** mg) was separated into the enantiomeric **Example 92** and **Example 93** using the following conditions: column: ChiralPak IF 2 × 25 cm, 5.0 um; Eluent: MTBE (0.5% 2M $NH_3$ in MeOH)/EtOH 95/5; Flow rate: 17 mL/min to provide:

**Example 92: 1,3-dimethyl-N-{4-methyl-2-[(rel-2R)-1-methylpyrrolidin-2-yl]-1H-pyrrolo[3,2-c]pyridin-6-yl}indazole-6-carboxamide**

First eluting compound (retention time = 5.4 min)
Mass spec: m/z: 403 $[M+H]^+$; $^1H$ NMR (400 MHz, DMSO-$d_6$) δ ppm 11.32 (s, 1H), 10.41 (s, 1H), 8.42 (s, 1H), 8.10 (s, 1H), 7.74 - 7.80 (m, 2H), 6.41 (s, 1H), 4.06 (s, 3H), 3.39 - 3.41 (m, 4H), 3.14 - 3.19 (m, 1H), 2.60 (s, 3H), 2.25 - 2.27 (m, 1H), 2.17 (s, 4H), 1.80 - 1.91 (m, 3H).

**Example 93: 1,3-dimethyl-N-{4-methyl-2-[(rel-2S)-1-methylpyrrolidin-2-yl]-1H-pyrrolo[3,2-c]pyridin-6-yl}indazole-6-carboxamide**

Second eluting compound (retention time = 10.6 min)
Mass spec: m/z: 403 $[M+H]^+$; $^1H$ NMR (400 MHz, DMSO-$d_6$) δ ppm 11.32 (s, 1H), 10.41 (s, 1H), 8.42 (s, 1H), 8.10 (s, 1H), 7.74 - 7.78 (m, 2H), 6.41 (s, 1H), 4.06 (s, 3H), 3.39 - 3.41 (m, 4H), 3.14 - 3.16 (m, 1H), 2.60 (s, 3H), 2.25 - 2.27 (m, 1H), 2.17 (s, 4H), 1.80 - 1.91 (m, 3H).

**Example 94 was synthesised following Scheme 79**

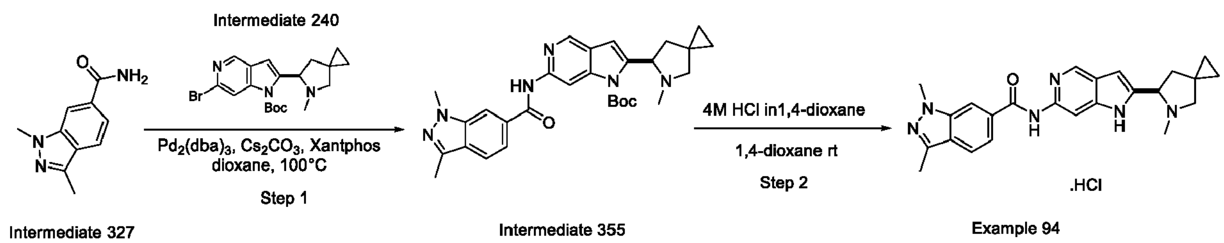


Scheme 79

**Step 1**

**Intermediate 356: *tert*-butyl (rel-*R*)-6-(1, 3-dimethyl-1*H*-indazole-6-carboxamido)-2-(5-methyl-5-azaspiro [2.4] heptan-6-yl)-1*H*-pyrrolo[3, 2-c]pyridine-1-carboxylate**

To a solution of 1,3-dimethyl-1*H*-indazole-6-carboxamide (**Intermediate 327**, 0.19 g, 1.04 mmol) and *tert*-butyl (rel-*R*)-6-bromo-2-(5-methyl-5-azaspiro[2.4]heptan-6-yl)-1*H*-pyrrolo[3,2-c]pyridine-1-carboxylate (**Intermediate 240, 0.40** g, 1.30 mmol) in 1,4-dioxane (8 mL) was added cesium carbonate (1.27 g, 3.91 mmol) at room temperature and the reaction mixture was purged with argon for 15 min. $Pd_2(dba)_3$ (0.18 g, 0.19 mmol) and Xantphos (0.23 g, 0.39 mmol) were then added and the reaction mixture was further purged with argon for 10 min and heated at 100°C for 2h. The reaction mixture was filtered through celite and washed with ethyl acetate (100 mL) and filtrate was concentrated *in vacuo.* The crude material was purified by combi-flash column chromatography by eluting with 2% MeOH in DCM to afford *tert*-butyl (rel-*R*)-6-(1, 3-dimethyl-1*H*-indazole-6-carboxamido)-2-(5-methyl-5-azaspiro [2.4] heptan-6-yl)-1*H*-pyrrolo [3,2-c] pyridine-1-carboxylate (**Intermediate 355**, 0.30 g, 47%) as an off white solid. $^1$H NMR (400 MHz, DMSO-$d_6$) δ ppm 0.35-0.65 (m, 4H), 1.09-1.19 (m, 1H), 1.58-1.67 (m, 1H), 1.71 (s, 9H), 2.35 (s, 3H), 2.45 (s, 3H), 2.61-2.65 (m, 1H), 2.77-2.82 (m, 1H), 4.05 (s, 3H), 4.16-4.21 (m, 1H), 6.86 (s, 1H), 7.71-7.75 (m, 1H), 7.78-7.83 (m, 1H), 8.42 (s, 1H), 8.63 (s, 1H), 8.98 (s, 1H), 10.74 (s, 1H). Mass spec: m/z: 415.3 [M-Boc]$^+$.

**Step 2**

**Example 94: (rel-*R*)-1,3-dimethyl-*N*-(2-(5-methyl-5-azaspiro [2.4] heptan-6-yl)-1*H*-pyrrolo[3, 2-c] pyridin-6-yl)-1*H*-indazole-6-carboxamide hydrochloride**

To a solution of *tert*-butyl (*R*)-6-(1,3-dimethyl-1*H*-indazole-6-carboxamido)-2-(5-methyl-5-azaspiro [2.4] heptan-6-yl)-1*H*-pyrrolo[3,2-c] pyridine-1-carboxylate (**Intermediate 355**, 0.30 g, 0.58 mmol) in 1,4-dioxane (6 mL) was added 4M HCl in 1,4-dioxane (5 mL) and the reaction mixture was stirred at room temperature for 3h. The reaction mixture was concentrated *in vacuo* to afford (*R*)-1, 3-dimethyl-*N*-(2-(5-methyl-5-azaspiro [2.4] heptan-6-yl)-1*H*-pyrrolo [3,2-c] pyridin-6-yl)-1*H*-indazole-6-carboxamide hydrochloride (**Example 94**, 0.05 g, 21%) as an off white solid. $^1$H NMR (400 MHz, DMSO-$d_6$) δ ppm 0.51-0.75 (m, 4H), 2.01-2.14 (m, 2H), 2.18 (s, 3H), 2.54 (s, 3H), 2.56-2.58 (m, 1H), 2.89-2.93 (m, 1H), 3.61-3.65 (m, 1H), 4.07 (s, 3H), 6.46 (s, 1H), 7.74-7.78 (m, 1H), 7.79-7.83 (m, 1H), 8.27 (s, 1H), 8.44 (s, 1H), 8.55 (s, 1H), 10.53 (s, 1H), 11.49 (s, 1H). Mass spec: m/z: 413.2 [M-H]$^-$.

**Example 95 was synthesised following Scheme 80**

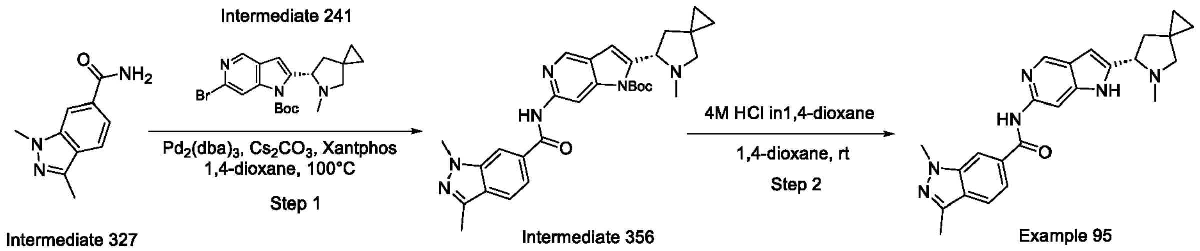


**Scheme 80**

**Step 1**

**Intermediate 356: *tert*-butyl (rel-*S*)-6-(1,3-dimethyl-1*H*-indazole-6-carboxamido)-2-(5-methyl-5-azaspiro [2.4] heptan-6-yl)-1*H*-pyrrolo[3,2-c]pyridine-1-carboxylate**

**Intermediate 356** was synthesised in analogous manner to **Intermediate 355,** following **Step 1** of **Scheme 79,** using 1,3-dimethyl-1*H*-indazole-6-carboxamide **(Intermediate 327)** as the amide and *tert*-butyl (rel-*S*)-6-bromo-2-(5-methyl-5-azaspiro[2.4]heptan-6-yl)-1*H*-pyrrolo[3,2-c]pyridine-1-carboxylate **(Intermediate 241)** as the aryl halide, to afford *tert*-butyl (rel-*S*)-6-(1,3-dimethyl-1*H*-indazole-6-carboxamido)-2-(5-methyl-5-azaspiro [2.4] heptan-6-yl)-1*H*-pyrrolo[3,2-c]pyridine-1-carboxylate **(Intermediate 356).** 1H NMR (400 MHz, DMSO-*d6*) δ ppm 0.40-0.65 (m, 4H), 1.09-1.19 (m, 1H), 1.58-1.67 (m, 1H), 1.71 (s, 9H), 2.35 (s, 3H), 2.45 (s, 3H), 2.61-2.65 (m, 1H), 2.77-2.82 (m, 1H), 4.05 (s, 3H), 4.15-4.21 (m, 1H), 6.86 (s, 1H), 7.71-7.75 (m, 1H), 7.78-7.83 (m, 1H), 8.42 (s, 1H), 8.64 (s, 1H), 8.98 (s, 1H), 10.74 (s, 1H). Mass spec: m/z: 515.2 [M+H]$^+$.

**Step 2**

**Example 95: (rel-S)-1,3-dimethyl-N-(2-(5-methyl-5-azaspiro[2.4]heptan-6-yl)-1H-pyrrolo[3,2-c]pyridin-6-yl)-1*H*-indazole-6-carboxamide**

To a solution of tert-butyl (rel-S)-6-(1, 3-dimethyl-1H-indazole-6-carboxamido)-2-(5-methyl-5-azaspiro[2.4] heptan-6-yl)-1H-pyrrolo[3,2-c] pyridine-1-carboxylate (**Intermediate 356**, 0.26 g, 0.63 mmol) in 1,4-dioxane (5.0 mL) was added 4M hydrochloric acid in 1,4-dioxane (5.0 mL) and the reaction mixture was stirred at room temperature for 5h. The reaction mixture was concentrated *in vacuo* to obtain crude compound. The crude material was purified by preparative HPLC (Method A) to afford (rel-*S*)-1,3-dimethyl-*N*-(2-(5-methyl-5-azaspiro[2.4]heptan-6-yl)-1*H*-pyrrolo[3, 2-c]pyridin-6-yl)-1*H*-indazole-6-carboxamide (**Example 95,** 0.055 g, 21%) as an off-white solid. 1H NMR (400 MHz, DMSO-d6) δ 0.50-0.59 (m, 2H), 0.60-0.72 (m, 2H), 2.00-2.09 (m, 2H), 2.17 (s, 3H), 2.51 (s, 3H), 2.52-2.54 (m, 1H), 2.88-2.94 (m, 1H), 3.58-3.64 (m, 1H), 4.05 (s, 3H), 6.44 (s, 1H), 7.72-7.76 (m, 1H), 7.78-7.81 (m, 1H), 8.25 (s, 1H), 8.42 (s, 1H), 8.53 (s, 1H), 10.51 (s, 1H), 11.47 (br s, 1H). Mass spec: m/z: 415.2 $[M+H]^+$.

**Example 96 was synthesised following Scheme 81**

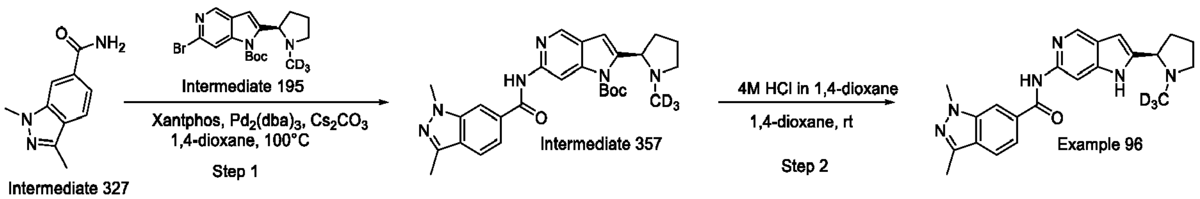


**Scheme 81**

**Step 1**

**Intermediate 357: *tert*-butyl (*R*)-6-(1,3-dimethyl-1*H*-indazole-6-carboxamido)-2-(1-(methyl-d3) pyrrolidin-2-yl)-1*H*-pyrrolo [3, 2-c] pyridine-1-carboxylate**

To a solution of 1,3-dimethyl-1*H*-indazole-6-carboxamide (**Intermediate 327**, 0.098 g, 0.52 mmol) in 1,4-dioxane (5 mL) was added *tert*-butyl (*R*)-6-bromo-2-(1-(methyl-d3)pyrrolidin-2-yl)-1*H*-pyrrolo[3,2-c]pyridine-1-carboxylate (**Intermediate 195**, 0.25 g, 0.65 mmol) followed by cesium carbonate (0.64 g, 1.96 mmol) at room temperature. The reaction mixture was purged with argon for 15 min then $Pd_2(dba)_3$ (0.061 g, 0.065 mmol) and Xantphos (0.12 g, 0.19 mmol) were added. The reaction mixture was further purged with argon for 10 min and then stirred at 100°C for 2h. The reaction mixture was filtered through celite and washed with ethyl acetate (50 mL) and concentrated in *vacuo.* The crude material was purified by combi-flash chromatography by eluting with 90% ethyl acetate in heptane to afford *tert*-butyl (*R*)-6-(1,3-dimethyl-1*H*-indazole-6-carboxamido)-2-(1-(methyl-d3)pyrrolidin-2-yl)-1*H*-pyrrolo[3,2-c]pyridine-1-carboxylate (**Intermediate 357**, 0.20 g, 62%) as a yellow solid. $^1$H NMR (400 MHz, DMSO-$d_6$) δ 1.57-1.65 (m, 1H), 1.71 (s, 9H), 1.74-1.81 (m, 2H), 2.31-2.43 (m, 2H), 2.51 (s, 3H), 3.09-3.17 (m, 1H), 3.90-3.96 (m, 1H), 4.05 (s, 3H), 6.76 (s, 1H), 7.73 (d, *J*=8.40 Hz, 1H), 7.80 (d, *J*=8.40 Hz, 1H), 8.41 (s, 1H), 8.61 (s, 1H), 8.98 (s, 1H), 10.74 (s, 1H). Mass spec: m/z: 492.0 $[M+H]^+$.

**Step 2**

**Example 96: (*R*)-1,3-dimethyl-*N*-(2-(1-(methyl-$d_3$)pyrrolidin-2-yl)-1*H*-pyrrolo[3,2-c]pyridin-6-yl)-1*H*-indazole-6-carboxamide**

To a solution of *tert*-butyl (*R*)-6-(1, 3-dimethyl-1*H*-indazole-6-carboxamido)-2-(1-(methyl-$d_3$) pyrrolidin-2-yl)-1*H*-pyrrolo[3,2-c]pyridine-1-carboxylate **(Intermediate 357, 0.20** g, 0.40 mmol) in 1,4-dioxane (4 mL) was added 4M hydrochloric acid in 1,4-dioxane (4 mL) at 0°C and the reaction mixture was stirred at room temperature for 3h. The reaction mixture was concentrated in *vacuo* to obtain the crude compound. The crude material was purified by preparative HPLC (Method A) to afford (*R*)-1,3-dimethyl-*N*-(2-(1-(methyl-$d_3$) pyrrolidin-2-yl)-1*H*-pyrrolo [3,2-c] pyridin-6-yl)-1*H*-indazole-6-carboxamide (**Example 96,** 0.045 g, 28%) as an off-white solid. $^1$H NMR (400 MHz, DMSO-$d_6$) δ 1.77-1.96 (m,

3H), 2.11-2.20 (m, 1H), 2.22-2.31 (m, 1H), 2.52 (s, 3H), 3.11-3.17 (m, 1H), 3.32-3.35 (m, 1H), 4.05 (s, 3H), 6.40 (s, 1H), 7.74 (d, *J*=8.40 Hz, 1H), 7.81 (d, *J*=8.40 Hz, 1H), 8.25 (s, 1H), 8.42 (s, 1H), 8.52 (s, 1H), 10.50 (s, 1H), 11.38 (s, 1H). Mass spec: m/z: 392.0 [M+H]+.

**Example 97 was synthesised following Scheme 82**

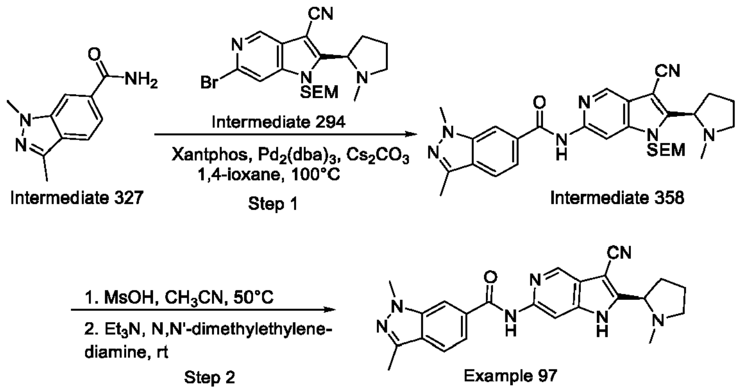


**Scheme 82**

**Step 1**

**Intermediate 358: (*R*)-*N*-(3-cyano-2-(1-methylpyrrolidin-2-yl)-1-((2-(trimethylsilyl) ethoxy) methyl)-1*H*-pyrrolo [3, 2-c]pyridin-6-yl)-1, 3-dimethyl-1*H*-indazole-6-carboxamide**

To a solution of (*R*)-6-bromo-2-(1-methylpyrrolidin-2-yl)-1-((2-(trimethylsilyl)ethoxy)methyl)-1*H*-pyrrolo[3,2-c] pyridine-3-carbonitrile (**Intermediate 294**, 0.16 g, 0.37 mmol) in 1,4-dioxane (2.0 mL) was added 1,3-dimethyl-1*H*-indazole-6-carboxamide (**Intermediate 327, 0.05** g, 0.29 mmol) followed by cesium carbonate (0.36 g, 1.1 mmol) at room temperature. The reaction mixture was purged with argon for 15 min then $Pd_2(dba)_3$ (0.05 g, 0.05 mmol) and Xantphos (0.06 g, 0.11 mmol) were added. The reaction mixture was further purged with argon for 10 min and then stirred at 100°C for 2h. The reaction mixture was filtered through celite, washed with ethyl acetate (100 mL) and filtrate was concentrated *in vacuo.* The crude material was purified by combi-flash chromatography by eluting with 90% ethyl acetate in heptane to afford (*R*)-*N*-(3-cyano-2-(1-methylpyrrolidin-2-yl)-1-((2-(trimethylsilyl) ethoxy) methyl)-1*H*-pyrrolo[3, 2-c]pyridin-6-yl)-1,3-dimethyl-1*H*-indazole-6-carboxamide (**Intermediate 358, 0.03** g, 20%) as a yellow solid. $^1$H NMR (400 MHz, DMSO-$d_6$) δ - 0.06 (s, 9H), 0.87-0.93 (m, 2H), 1.82-1.98 (m, 4H), 2.28 (s, 3H), 2.32-2.41 (m, 2H), 3.16-3.22 (m, 1H), 3.57 (s, 3H), 3.59-3.65 (m, 2H), 4.06 (s, 3H), 5.69-5.83 (m, 2H), 7.74 (d, *J*=8.40, 1H), 7.81 (d, *J*=8.40 Hz, 1H), 8.46 (s, 1H), 8.61 (s, 1H), 8.72 (s, 1H), 10.93 (br s, 1H). Mass spec: m/z: 544.3 [M+H]+.

**Step 2**

**Example 97: (*R*)-*N*-(3-cyano-2-(1-methylpyrrolidin-2-yl)-1*H*-pyrrolo [3,2-c] pyridin-6-yl)-1, 3-dimethyl-1*H*-indazole-6-carboxamide**

To a solution of (*R*)-*N*-(3-cyano-2-(1-methylpyrrolidin-2-yl)-1-((2-(trimethylsilyl) ethoxy) methyl)-1*H*-pyrrolo [3, 2-c] pyridin-6-yl)-1, 3-dimethyl-1*H*-indazole-6-carboxamide (**Intermediate 358, 0.03** g, 0.06 mmol) in acetonitrile (2.0 mL) was added methanesulfonic acid (0.05 mL, 0.8 mmol) at room temperature and the reaction mixture was stirred at 50°C for 2h. After completion of the reaction, the mixture was cooled to room temperature then *N*, *N*'-dimethylethylenediamine (0.05 mL, 0.4 mmol) followed by triethylamine (0.2 mL, 1 mmol) were added and stirred at room temperature for 3h. The reaction mixture was concentrated *in vacuo,* the obtained residue was poured into ice cold water (40 mL) and extracted with ethyl acetate (3 × 50 mL). The organic layer was separated, dried over anhydrous $Na_2SO_4$, and concentrated in *vacuo.* The crude material was purified by preparative HPLC (Method A) to afford (*R*)-*N*-(3-cyano-2-(1-methylpyrrolidin-2-yl)-1*H*-pyrrolo [3,2-c] pyridin-6-yl)-1,3-dimethyl-1*H*-indazole-6-carboxamide (**Example 97**, 0.006 g, 30%) as an off-white solid. $^1$H NMR (400 MHz, DMSO-$d_6$) δ 1.84-2.01 (m, 3H), 2.24 (s, 3H), 2.28-2.34 (m, 1H), 2.37-2.44 (m, 1H), 2.52 (s, 3H), 3.17-3.24 (m, 1H), 3.56-3.65 (m, 1H), 4.06 (s, 3H), 7.75 (d, *J*=8.40 Hz, 1H), 7.81 (d, *J*=8.40 Hz, 1H), 8.41 (s, 1H), 8.44 (s,

1H), 8.68 (s, 1H), 10.80 (br s, 1H). Mass spec: m/z: 414.2 $[M+H]^+$.

**Example 98: Efficacy *in vitro***

**MLLT1 Degradation Assay:**

MLLT1 degradation efficacy of compounds was tested in an automated chemiluminescent detection system (JESS, Cat# 004-650). MV4-11 (#CRL-9591, ATCC) cells were seeded at a density of 1.2 million cells/well in a non-treated 6-well cell culture plate in 2.4 mL of complete medium (IMDM + 10% FBS + 1X Penicillin-Streptomycin). 10 mM DMSO stocks were prepared, and cells were treated with three concentrations of test compounds viz. 1000, 100 and 10 nM at a final DMSO concentration of 0.1% and incubated for 24h at 37°C, 5% $CO_2$. Post incubation, cells were centrifuged, washed with HBSS and lysed in 70 uL of RIPA buffer supplemented with Protease inhibitors (sc-24948; Santa Cruz). Subsequently, protein estimation was done using BCA protein assay kit (23225; Pierce). 4 ug protein lysate (4 uL of 1ug/uL lysate) was loaded into each well of the JESS cartridge (SM-WO01; Bio-techne) and the JESS run was performed using 1:900 dilution of anti-human MLLT1 antibody (14893; Cell signaling technology) and 1:1200 dilution of anti-human GAPDH antibody (2118; Cell signaling technology) with anti-rabbit secondary detection module (DM-001; Bio-Techne). Post run, data were analysed using "Compass for SW" software for chemiluminescent detection. The peak area for each band was used for the calculation of MLLT1 degradation with respect to DMSO control. Data is shown as "MLLT1 % degradation".
**Category A:** A ≥ 75% degradation
**Category B:** 50% ≤ B < 75% degradation
**Category C:** 25% ≤ C < 50% degradation
**Category D:** D < 25% degradation

**Table 1**

| Example Number | MLLT1 % Degradation @ 1 μM | MLLT1 % Degradation @ 0.1 μM | MLLT1 % Degradation @ 0.01 μM |
|---|---|---|---|
| 1 | A | A | A |
| 2 | A | A | B |
| 3 | A | A | B |
| 4 | B | D | D |
| 5 | C | B | B |
| 6 | A | B | C |
| 9 | A | C | D |
| 10 | C | C | C |
| 11 | B | B | B |
| 15 | A | A | D |
| 16 | A | A | A |
| 18 | A | A | A |
| 20 | A | C | C |
| 21 | A | A | A |
| 22 | A | A | A |
| 23 | A | A | A |
| 24 | A | B | ND |
| 25 | A | A | ND |
| 26 | A | A | ND |
| 27 | A | A | A |
| 28 | C | C | ND |
| 29 | A | A | B |

(continued)

| Example Number | MLLT1 % Degradation @ 1 μM | MLLT1 % Degradation @ 0.1 μM | MLLT1 % Degradation @ 0.01 μM |
|---|---|---|---|
| 30 | A | A | A |
| 31 | A | B | C |
| 32 | B | A | B |
| 33 | A | B | B |
| 34 | A | B | D |
| 35 | B | D | C |
| 36 | A | A | A |
| 38 | B | A | B |
| 39 | A | A | A |
| 40 | A | A | A |
| 41 | B | C | D |
| 42 | A | A | B |
| 43 | A | B | C |
| 44 | A | A | B |
| 45 | A | A | A |
| 46 | B | A | A |
| 47 | B | D | D |
| 48 | A | B | C |
| 49 | A | A | A |
| 50 | A | A | A |
| 51 | A | B | C |
| 52 | A | A | A |
| 53 | A | A | A |
| 54 | A | A | A |
| 55 | A | A | B |
| 56 | A | A | A |
| 57 | A | A | A |
| 58 | A | A | A |
| 59 | A | A | A |
| 60 | A | B | C |
| 61 | A | A | C |
| 62 | A | B | B |
| 63 | A | B | D |
| 65 | A | B | C |
| 66 | A | A | A |
| 67 | A | A | A |
| 68 | B | A | A |
| 69 | A | A | A |
| 70 | A | A | A |

(continued)

| Example Number | MLLT1 % Degradation @ 1 μM | MLLT1 % Degradation @ 0.1 μM | MLLT1 % Degradation @ 0.01 μM |
|---|---|---|---|
| 71 | A | A | B |
| 72 | B | A | A |
| 73 | A | A | B |
| 74 | A | B | B |
| ND = not determined | | | |

**MV4-11 Cellular Proliferative Assays**

**Assay Format A**

An MV4-11 proliferation assay was used to test the ability of compounds to reduce cell viability. MV4-11 cells (ATCC #CRL9591) were grown in IMDM medium (11510596, Gibco) supplemented with 10% fetal bovine serum (F7524, Gibco) and 1% Penicillin/Streptomycin (15140-122, Gibco) at 37°C with 5% $CO_2$. Cells were seeded in 384-well Elplasia plates (4447, Corning) at a density of 50 cells/well and treated with vehicle (DMSO) or compounds (10 serial semi-log dilutions, 30 μM as top concentration).

After 120 hours of treatment, cells were incubated for 10 min at RT with the CellTiter-Glo (CTG) reagent (G7571, Promega). Measurement of the luminescence signal was performed on a microplate reader (Ensight, PerkinElmer). CTG reagent measures the amount of ATP, which is directly proportional to the number of cells, to determine the number of viable cells in culture.

The data were analysed as follows:

$$\text{Data normalization, } N(x) = \frac{x - \langle cr \rangle}{\langle sr \rangle - \langle cr \rangle} \times 100$$

where x is the measured raw signal value of a well, (cr) is the median of the measured signal values for the Central Reference (DMSO) wells on a plate, (sr) is the median of the measured signal values for the Scale Reference (reference compound) wells on a plate.

$$\text{Curve fitting, } S_0 + \frac{S_{\text{inf}} - S_0}{1 + \left(\frac{AC_{50}}{10^C}\right)^n}$$

where the experimental set-up assumes the Hill parameters in certain ranges: $S_0$ ~ Central Reference (CR) from normalization (= Bottom), $S_{inf}$ ~ Scale Reference (SR) from normalization (= Top), nHill ~ 1, $AC_{50}$ ~ within the measured concentration range.

**Assay Format B**

MV4-11 cells at a confluency of -50-80% was used for cell growth assay. 600 cells/well in a 384-well opaque cell culture plate (6007680; Perkin Elmer) were seeded in 40 uL of complete medium (IMDM + 10% FBS + 1X Penicillin-Streptomycin). Cells were incubated overnight at 37°C, 5% $CO_2$. 10 mM DMSO stocks were prepared, cells were treated with 10 concentrations of test compounds starting from 1 uM or 100 nM, 3-fold dilution in 10 uL (5x concentration) of complete medium. Final DMSO concentration was 0.3%. Culture plates were centrifuged at 40 × g, 30s. Cells were incubated with compounds for 168 h (7 days). After 7 days, 20 uL CTG 2.0 reagent (G9242; Promega) was added per well and luminescence reading was taken in Envision Xcite 2105 multimode plate reader. Data was analyzed in GraphPad Prism 10.0 and data was shown as % viability with respect to DMSO control and dose-response curves were generated using four parameter logistic regression. Assay quality was determined by z-factor calculation using positive and negative controls.

$IC_{50}$ was ≤ 100 nM for each of the Examples set out in the Table below:

**Table 2**

| Example Number | Assay format |
|---|---|
| 1 | A |
| 2 | A |
| 3 | A |
| 7 | A |
| 8 | A |
| 11 | A |
| 12 | A |
| 13 | A |
| 14 | A |
| 15 | A |
| 16 | A |
| 17 | A |
| 18 | A |
| 19 | A |
| 21 | A |
| 22 | A |
| 23 | A |
| 25 | A |
| 26 | A |
| 27 | A |
| 29 | A |
| 30 | A |
| 31 | A |
| 32 | A |
| 33 | A |
| 36 | A |
| 38 | A |
| 39 | B |
| 40 | B |
| 42 | A |
| 44 | A |
| 45 | A |
| 46 | A |
| 47 | A |
| 48 | A |
| 52 | B |
| 53 | B |
| 54 | B |
| 55 | B |

(continued)

| Example Number | Assay format |
|---|---|
| 56 | B |
| 57 | B |
| 58 | B |
| 59 | B |
| 61 | B |
| 66 | B |
| 67 | B |
| 68 | B |
| 69 | B |
| 70 | B |
| 71 | B |
| 72 | B |
| 74 | B |

**Comparison of PROTAC and small molecule activity**

Comparative Example compound 76 was prepared as described above and tested following the protocol of Assay Format A. The result is provided in Table 3 together with the results of a number of representative compounds of the invention.

**Table 3**

| Experimental Example No | MV4-11 antiproliferative $IC_{50}$ (nM) | Assay method |
|---|---|---|
| 1 | 4 | A |
| 15 | 12 | A |
| 16 | 9 | A |
| 21 | 11 | A |
| 22 | 8 | A |
| 25 | 11 | A |
| 26 | 14 | A |
| 30 | 6 | A |
| 32 | 15 | A |
| 44 | 5 | A |
| 45 | 10 | A |
| 46 | 7 | A |
| 76 | 40 | A |

**Inhibition data**

This procedure is intended to measure the interaction between YEATS proteins and histone peptides modified by an appropriate epigenetic mark and can be used to assay compounds which bind to the YEATS proteins and inhibit this interaction. The assay uses 6His tagged MLLT1 or MLLT3 with an anti-6His antibody conjugated with $Eu^{3+}$ chelate (donor) and a biotinylated peptide with Streptavidin conjugated XL-665 (acceptor). When the donor and acceptor labels are in close proximity, by binding of the peptide by MLLT1 or MLLT3, the excitation of the donor (337 nm) triggers a Fluorescence

Resonance Energy Transfer (FRET) to the acceptor which then fluoresces at specific wavelength (665 nm).

**HTRF Protein-Peptide Titration**

This step optimises the protein/peptide ratio that will be used in HTRF screening assay in order to allow the best signal-to-noise ratio. Each new purified protein should be titrate this way as the signal-to-noise ratio may differ from different protein batches. Displacement assay for MLLT1 and MLLT3 was performed by using H3K9cro peptide (ARTKQTAR(Kcrot)STGGKAPRKQLYK(biotin)).

A serial dilution of 4X solution of biotinylated peptide was prepared in assay buffer (25 mM HEPES, 20 mM NaCl, 0.05 % BSA, 0.05 % Tween-20, pH 7.0) covering concentrations from 3.2 μM to 0.2 nM. and 5 μL of each dilution were transferred horizontally to fill 15 columns of a white ProxiPlate-384 Plus shallow well Microplate (Perkin Elmer, USA). A sixteenth column of buffer was also included for a background reference. In parallel, a serial dilution of the 6His-tag protein of interest was prepared in the same way and 5 μL of each dilution were vertically added to the wells containing the peptide dilutions. Buffer has been transferred to the last line of the plate in order to make a no-protein reference. The microplate has been covered and incubated 1 hour at room temperature. A 2X detection mix was prepared in assay buffer by diluting streptavidin conjugated with XL-665 (610SAXLB, Cisbio Bioassays, France) to 1/2000 and anti-6His(Eu) chelate (LANCE Eu-W1024 Anti-6xHis, AD0111, Perkinelmer LAS (UK)LTD) to 1/20000, 10 μL were added to each well and the plate has been incubated for 1 to 24 hours at room temperature in the dark. The plate was read in the Pherastar using the HTRF module with dual emission protocol (A = ex. 320nm em. 665nm, B = ex. 320nm em. 620nm).

The HTRF ratio was calculated as channel A/B * 10000 and the specific signal as follow: specific signal = HTRF Ratio Positive signal - HTRF Ratio Negative signal (without protein). The concentrations of protein and peptide that allow the maximum specific signal without saturation were selected to perform HRTF screening assay described below.

**HTRF $IC_{50}$ Determination Assay**

The compounds were dissolved in DMSO at a stock concentration of 10 mM and dispensed into microplate using the Echo acoustic liquid handler (Labcyte, Beckman Coulter, USA). The assay covers a window of compound concentrations from 20 μM to 20 nM spread in 11 data points in duplicate. Solvent and compound controls were added to each plate in order to calculate the background signal.

Ten microliters of 2x mix of 6His-protein and biotinylated peptide, the concentrations were determined above, were added to the dispensed compounds and incubated for one hour at room temperature in the dark. Ten microliters of the 2X detection mix was added to each well and the plate was incubated for another hour at room temperature in the dark and the plate was read in order to calculate the HTRF specific signal. The % inhibition values as follows:

$$\% I = \frac{positive\ control - signal}{positive\ control} \times 100$$

The $IC_{50}$ values were determined by plotting % inhibition vs compound concentration and fitting the data to a non-linear sigmoidal dose response equation (4 parameter fit):

$$I_i = I_o + \frac{I_{complete} - I_o}{1 + \left(\frac{IC_{50}}{c_i}\right)^s}$$

$I_i$: Inhibition at given concentration $c_i$
$I_{complete}$: Complete absence of binding, bottom of curve
$I_0$: No inhibition of binding, top of curve
$IC_{50}$: Concentration at which inhibition is 50%
$c_i$: concentration i in range tested
$s$: Hill coefficient (slope at $IC_{50}$)

Calculations were carried out in Excel and GraphPad Prism templates.

***Results***

**Table 4** exhibits the ranges of $IC_{50}$ (nM) of the compounds when tested in the MLLT1 and MLLT3 assays.

**Category A:** $IC_{50} \leq 100$ nM; **Category B**: 100 nM < $IC_{50} \leq 1000$ nM; **Category C:** 1000 nM < $IC_{50} \leq 10000$ nM;

**Table 4**

| Example Number | MLLT1 HTRF $IC_{50}$ nM | MLLT3 HTRF $IC_{50}$ nM |
|---|---|---|
| 75 | A | B |
| 76 | A | A |
| 77 | B | C |
| 78 | C | C |
| 79 | A | A |
| 80 | A | A |
| 81 | B | C |
| 84 | B | B |
| 85 | C | ND |
| 86 | C | C |
| 87 | A | A |
| 88 | A | A |
| 89 | B | C |
| 90 | A | A |
| 91 | C | C |
| 92 | A | A |
| 93 | C | C |

Comparative Example compound 76 was prepared as described above and tested following the protocol of the HTRF $IC_{50}$ Assay. The result is provided in Table 5 together with the results of a number of representative compounds.

**Table 5**

| Example Number | MLLT1 HTRF $IC_{50}$ nM | MLLT3 HTRF $IC_{50}$ nM |
|---|---|---|
| 76 | 43 | 81 |
| 80 | 6 | 22 |
| 88 | 17 | 37 |
| 90 | 12 | 45 |
| 92 | 37 | 63 |

In one embodiment, the invention provides compounds according to the following aspects.

1. A compound which is a Proteolysis Targeting Chimera (PROTAC) or a pharmaceutically acceptable salt thereof, wherein the PROTAC has the structure:

M-LINK-U

wherein U is an E3 ubiquitin ligase binding moiety, LINK is a moiety that covalently links M and U, and M is an MLLT1 and/or MLLT3 binder of formula (I):

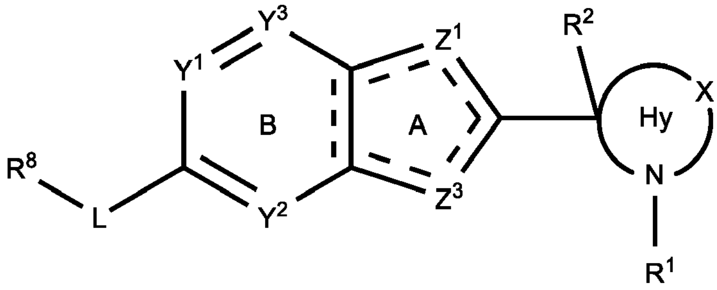

(I)

wherein:

one of $Z^1$ and $Z^3$ is -N(H)- and the other is N or -C($R^4$)-, $Y^1$ is N, $Y^2$ is N or -C($R^6$)-, and $Y^3$ is N or -C($R^5$)-;
Hy is a 4- to 7-membered heterocyclic ring containing X and at least one N atom, wherein: ring Hy is linked to ring A via a C atom within ring Hy, said C atom also being linked to $R^2$; a N atom within ring Hy is substituted by $R^1$; X is a bond, -N($R^{11}$)-, O, S, -S(O)$_2$-, -S(O)(N$R^{11}$)-, or -C($R^{11}$)$_2$-; and the rest of ring Hy is unsubstituted or substituted by one or two $R^3$;
L is -C(O)N(H)-, wherein the C atom of L is bonded to $R^8$, and the N atom of L is bonded to ring B;
$R^1$ is H, $C_{1-4}$ cycloalkyl, or $C_{1-4}$ alkyl which is itself unsubstituted or substituted with one $C_{1-4}$ alkoxy or one, two or three halo;
$R^2$ is H or methyl;
each $R^3$ is independently selected from $C_{1-4}$ alkyl, $C_{1-4}$ alkoxy, phenyl, a 5- to 6-membered heteroaryl ring and halo, or (i) two $R^3$ linked to adjacent C atoms in ring Hy form, together with the C atoms to which they are attached, a $C_{5-6}$ cycloalkyl ring, or (ii)
two $R^3$ linked to the same C atom in ring Hy form, together with the C atom to which they are attached, a $C_{3-6}$ cycloalkyl ring or a $C_{3-6}$ heterocycloalkyl ring;
$R^4$ and $R^6$ are independently selected from H, halo, CN, $C_{1-4}$ alkoxy, and $C_{1-4}$ alkyl which is itself unsubstituted or substituted by one, two or three halo;
$R^5$ is selected from H, halo, $C_{1-4}$ alkoxy, $C_{3-5}$ cycloalkyl and $C_{1-4}$ alkyl which is itself unsubstituted or substituted by one, two or three halo;
$R^8$ is an 8- to 10-membered heteroaryl ring, the group $R^8$ being unsubstituted or substituted by one, two or three substituents independently selected from halo, $C_{1-4}$ alkoxy, $R^{10}$, -($C_{1-4}$ alkylene)-$R^{10}$, =O, -CN, and $C_{1-4}$ alkyl which is itself unsubstituted or substituted by one or two $R^9$;
each $R^9$ is independently selected from halo and $C_{1-4}$ alkoxy;
$R^{10}$ is a group selected from a 5- to 6-membered heteroaryl ring, a 3- to 6-membered cycloalkyl ring, a 4- to 6-membered heterocyclyl ring, and a phenyl ring, the group $R^{10}$ being unsubstituted or substituted by one or two substituents independently selected from $C_{1-4}$ alkyl, $C_{1-4}$ alkoxy, and halo; and
each $R^{11}$ is independently selected from H, $C_{1-4}$ alkyl, and $C_{1-4}$ cycloalkyl;
wherein M is bonded to LINK via a C or N atom within group $R^8$ such that a hydrogen atom on the C or N atom within group $R^8$ is replaced with a bond to LINK.

2. A compound according to aspect 1, wherein LINK is (a) a single bond, or (b) a chemical linker group represented by formula (L):

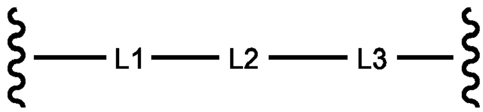

(L)

wherein:

LINK is attached to M via L1;
LINK is attached to U via L3;
L1 and L3 are each independently selected from a single bond, -N(R')-, -C(O)N(R')-, -O-, -N(R')C(O)-, -C(O)-,

-$S(O_2)N(R')$-, -$N(R')S(O_2)$-, -($C_{1-6}$ alkylene)-, ethynyl, -($C_{2-6}$ alkenylene)-, a divalent 3- to 6-membered cycloalkyl ring and a divalent 4- to 7-membered heterocyclyl ring;
L2 is represented by the formula -$(L4)_m$-;
each L4 is independently selected from a divalent 3- to 6-membered cycloalkyl ring, a divalent 4- to 7-membered heterocyclyl ring and a unit of formula:

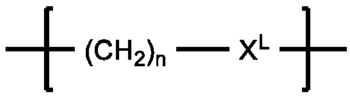
or
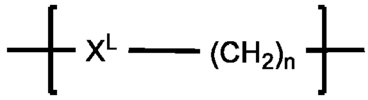
;

each $X^L$ is independently selected from a single bond, -N(R')-, -O-, -C(O)-, -S-, -SO-, - $SO_2$-, -C(R")=C(R")- and -C(H)≡C(H)-;
n is selected from 1 to 4;
m is selected from 1 to 30;
each R' is independently selected from H and $C_{1-4}$ alkyl; and
each R" is independently selected from H and halo.

3. A compound according to aspect 2, wherein each L4 is independently selected from a divalent 4- to 7-membered heterocyclyl ring and a unit of formula

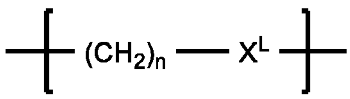
or
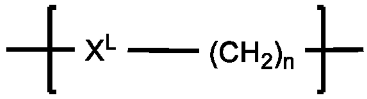
;

wherein each $X^L$ is independently selected from a single bond, -O-, or -S-; and
n is selected from 1 or 2.

4. A compound according to aspect 2 or 3, wherein each divalent 4- to 7-membered heterocyclyl ring of L1, L2 and L3 when present contains at least one N atom.

5. A compound according to aspect 4, wherein each divalent 4- to 7-membered heterocyclyl ring of L1, L2 and L3 when present contains one or two N atoms.

6. A compound according to aspect 5, wherein each divalent 4- to 7-membered heterocyclyl ring of L1, L2 and L3 when present is independently selected from piperidinyl and diazinanyl.

7. A compound according to any one of aspects 2 to 6, wherein L2 is selected from -$(CH_2)_m$-, -$(CH_2CH_2O)_m$-, -$(OCH_2CH_2)_m$-, -$(CH_2CH_2S)_m$-, -$(SCH_2CH_2)_m$-, and a divalent 4- to 7-membered heterocyclyl ring.

8. A compound according to any one of aspects 2 to 7, wherein m is selected from 1 to 10.

9. A compound according to any one of aspects 2 to 8, wherein L1 and L3 are each independently selected from a single bond, -($C_{1-6}$ alkylene)-, -N(R')-, -C(O)N(R')-, -O-, -N(R')C(O)-, -C(O)-, -$S(O_2)N(R')$-, -$N(R')S(O_2)$-, ethynyl, and a divalent 4- to 7-membered heterocyclyl ring.

10. A compound according to any one of aspects 2 to 9, wherein L1 and L3 are each independently selected from a single bond, -($C_{1-6}$ alkylene)-, -N(R')-, -C(O)N(R')-, - N(R')C(O)-, -O-, ethynyl, and a divalent 4- to 7-membered heterocyclyl ring.

11. A compound according to any one of aspects 2 to 10, wherein L1 and L3 are each independently selected from a single bond, -($C_{1-6}$ alkylene)-, -N(R')-, -C(O)N(R')-, -N(R')C(O)-, -O-, ethynyl, piperidinyl and diazinanyl.

12. A compound according to any one of aspects 1 to 11, wherein U is a CRBN or VHL E3 ubiquitin ligase binding moiety.

13. A compound according to aspect 12, wherein U is a CRBN E3 ubiquitin ligase binding moiety.

14. A compound according to aspect 12, wherein U is a VHL E3 ubiquitin ligase binding moiety.

15. A compound according to any one of aspects 1 to 12, wherein U is:

(a) a CRBN E3 ubiquitin ligase binding moiety of formula (U1):

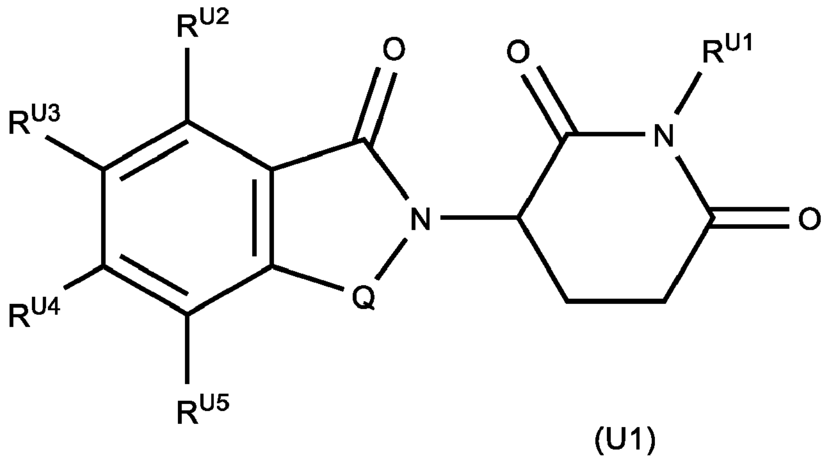

(U1)

wherein:

$R^{U1}$ is H;
Q is selected from -CH($R^{U6}$)-, -N($R^{U6}$)-, -O-, -C(O)-, -NH-CH($R^{U6}$)-, -N=C($R^{U6}$)-, or -N=N-;
$R^{U6}$ is H or $C_{1-4}$ alkyl;
$R^{U2}$ and $R^{U5}$ are each independently selected from H, halogen, $C_{1-4}$ alkyl, $NH_2$, $NO_2$, OH, COOH, CN, $CF_3$, and a single bond to LINK;
$R^{U3}$ and $R^{U4}$ are each independently selected from H, halogen, $C_{1-4}$ alkyl, $NH_2$, $NO_2$, OH, COOH, CN, $CF_3$, and a single bond to LINK;
wherein one and only one of $R^{U2}$, $R^{U3}$, $R^{U4}$ and $R^{U5}$ is a single bond to LINK;

(b) a CRBN E3 ubiquitin ligase binding moiety of formula (U4):

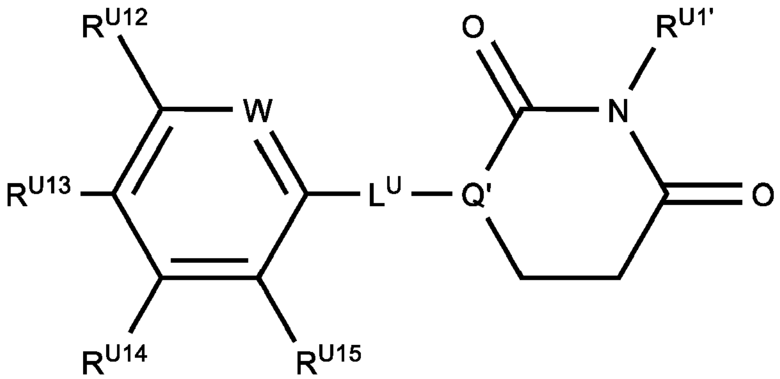

(U4)

wherein:

$R^{U1'}$ is H;
W is N or $CR^{U16}$;
Q' is N and $L^U$ is a single bond, or Q' is CH and $L^U$ is selected from a single bond or -C(O)N(H)-, wherein either (i) the C atom of $L^U$ is bonded to phenyl, and the N atom of $L^U$ is bonded to Q'; or (ii) the C atom of $L^U$ is bonded to Q', and the N atom of $L^U$ is bonded to phenyl;
$R^{U12}$, $R^{U13}$ and $R^{U14}$ are each independently selected from H, halogen, $C_{1-4}$ alkyl, $NH_2$, $NO_2$, OH, COOH,

CN, $CF_3$, and a single bond to LINK;
wherein one and only one of $R^{U12}$, $R^{U13}$ and $R^{U14}$ is a single bond to LINK;
$R^{U15}$ and $R^{U16}$ are each independently selected from H, halogen, $C_{1-4}$ alkyl, $NH_2$, $NO_2$, OH, COOH, CN and $CF_3$;

(c) a CRBN E3 ubiquitin ligase binding moiety of formula (U5):

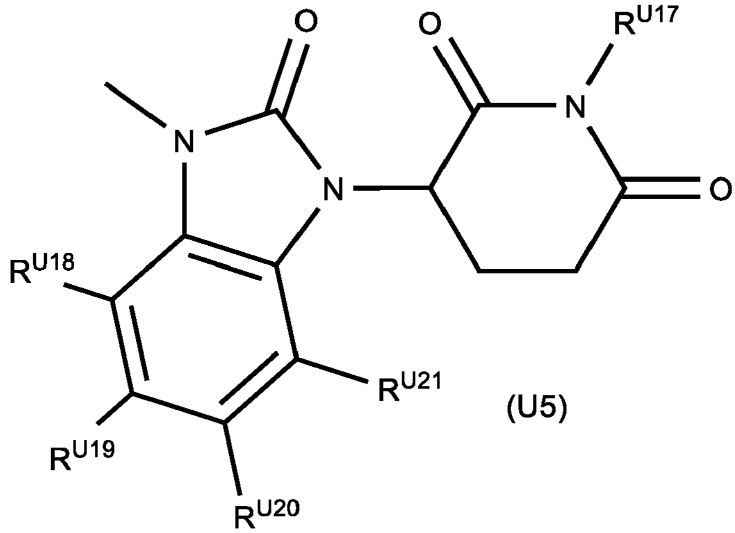

(U5)

wherein:

$R^{U17}$ is H;
$R^{U18}$, $R^{U19}$, $R^{U20}$ and $R^{U21}$ are each independently selected from H, halogen, $C_{1-4}$ alkyl, $NH_2$, $NO_2$, OH, COOH, CN, $CF_3$, and a single bond to LINK;
wherein one and only one of $R^{U18}$, $R^{U19}$, $R^{U20}$ and $R^{U21}$ is a single bond to LINK;
or (d) a VHL E3 ubiquitin ligase binding moiety of formula (U2):

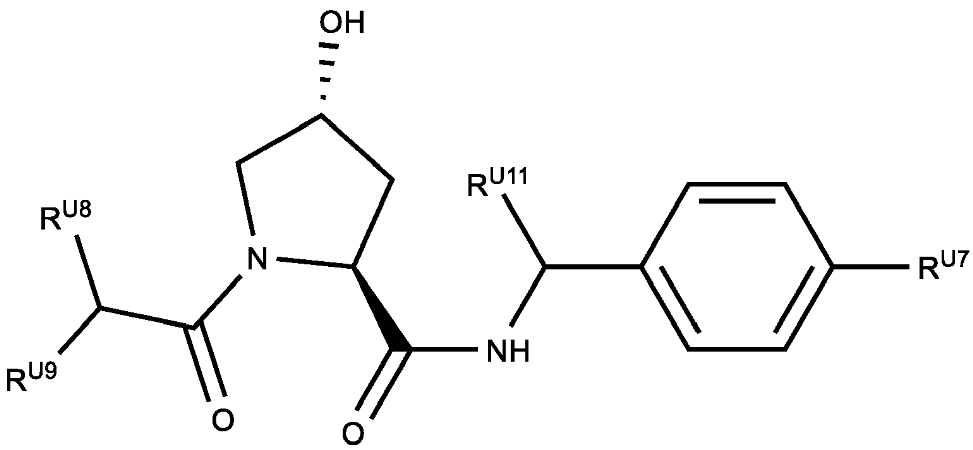

(U2)

wherein:

$R^{U7}$ is a group selected from phenyl, a 5- to 6-membered heteroaryl ring, a 5- to 6-membered heterocyclyl ring, and a 5- to 6-membered cycloalkyl ring, the group $R^{U7}$ being unsubstituted or substituted by $C_{1-4}$ alkyl;
$R^{U11}$ is H or $C_{1-4}$ alkyl;
$R^{U8}$ is selected from $C_{1-4}$ alkyl, phenyl and a 3- to 8-membered cycloalkyl ring; and
$R^{U9}$ is a single bond to LINK.

16. A compound according to aspect 15, wherein U is a VHL E3 ubiquitin ligase binding moiety of formula (U3):

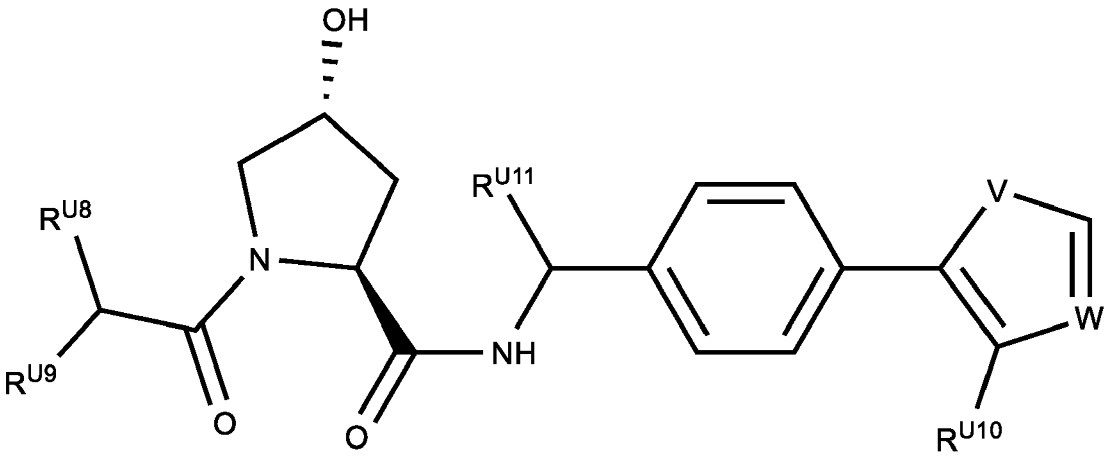

(U3)

wherein:

V is S or O;
W is N or CH;
$R^{U10}$ is H or $C_{1-4}$ alkyl;
$R^{U11}$ is H or $C_{1-4}$ alkyl;
$R^{U8}$ is selected from $C_{1-4}$ alkyl, phenyl and a 3- to 8-membered cycloalkyl ring; and
$R^{U9}$ is a single bond to LINK.

17. A compound according to aspect 16, wherein $R^{U10}$ is H or methyl.

18. A compound according to any one of aspects 15 to 17, wherein $R^{U8}$ is t-butyl.

19. A compound according to aspect 15, wherein U is a CRBN ubiquitin ligase binding moiety of formula (U1), wherein $R^{U6}$ is H or methyl.

20. A compound according to aspect 15 or 19, wherein U is a CRBN ubiquitin ligase binding moiety of formula (U1), wherein Q is selected from -C(O)- and $-CH_2-$.

21. A compound according to any one of aspects 15, 19 and 20, wherein U is a CRBN ubiquitin ligase binding moiety of formula (U1), wherein three of $R^{U2}$, $R^{U3}$, $R^{U4}$ and $R^{U5}$ are H.

22. A compound according to aspect 15, wherein U is a CRBN ubiquitin ligase binding moiety of formula (U4), wherein Q' is N.

23. A compound according to aspect 15 or 22, wherein U is a CRBN ubiquitin ligase binding moiety of formula (U4), wherein Q' is CH.

24. A compound according to aspect 23, wherein $L^U$ is a single bond.

25. A compound according to aspect 23, wherein $L^U$ is -C(O)N(H)-, wherein the C atom of $L^U$ is bonded to phenyl, and the N atom of $L^U$ is bonded to Q'.

26. A compound according to any one of aspects 15 and 22 to 25, wherein U is a CRBN ubiquitin ligase binding moiety of formula (U4), wherein $R^{U15}$ and $R^{U16}$ are H, and two of $R^{U12}$, $R^{U13}$ and $R^{U14}$ are H.

27. A compound according to any one of aspects 15 and 22 to 25, wherein U is a CRBN ubiquitin ligase binding moiety of formula (U4), wherein $R^{U13}$, $R^{U14}$ and $R^{U15}$ are H, $R^{U12}$ is a single bond to LINK, and $R^{U16}$ is selected from H, F and

Me.

28. A compound according to aspect 15, wherein U is a CRBN ubiquitin ligase binding moiety of formula (U5), wherein three of $R^{U18}$, $R^{U19}$, $R^{U20}$ and $R^{U21}$ are H.

29. A compound according to any one of the preceding aspects, wherein the N atom of ring Hy which is substituted by $R^1$ is adjacent to the C atom of ring Hy that is bonded to ring A.

30. A compound according to any one of the preceding aspects, wherein no more than two of $Y^1$, $Y^2$ and $Y^3$ are N.

31. A compound according to any one of the preceding aspects, wherein $Z^1$ is - $C(R^4)$- and $Z^3$ is -N(H)-.

32. A compound according to any one of the preceding aspects, wherein $Z^1$ is - $C(R^4)$-, $Z^3$ is -N(H)-, $Y^1$ is N, $Y^2$ is -$C(R^6)$- and $Y^3$ is -$C(R^5)$-.

33. A compound according to any one of the preceding aspects, wherein X is a bond, -N(Me)-, O or -$CH_2$-.

34. A compound according to any one of the preceding aspects, wherein X is a bond.

35. A compound according to any one of the preceding aspects, wherein M is of formula (II):

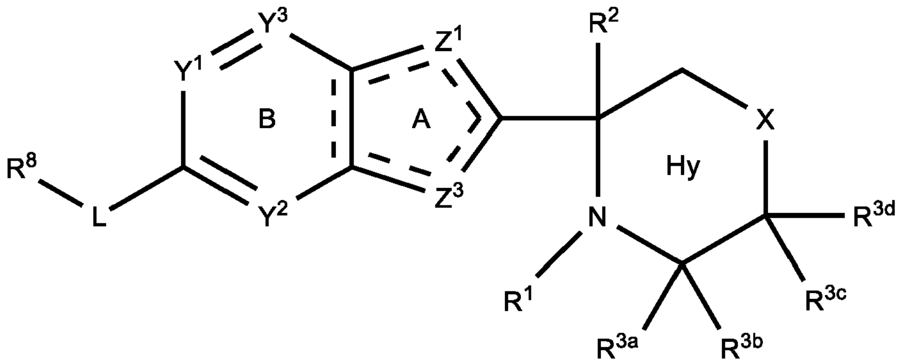

(II)

wherein:

$Z^1$, $Z^3$, $Y^1$, $Y^2$, $Y^3$, $R^1$, $R^2$, $R^8$, X and L are as defined in any one of the preceding aspects;
$R^{3a}$ and $R^{3b}$ are independently selected from H, $C_{1-4}$ alkyl, phenyl, a 5- to 6-membered heteroaryl ring and $C_{1-4}$ alkoxy, or $R^{3a}$ and $R^{3b}$ form, together with the C atom to which they are attached, a $C_{3-6}$ cycloalkyl ring or a $C_{3-6}$ heterocycloalkyl ring,
$R^{3c}$ and $R^{3d}$ are independently selected from H, $C_{1-4}$ alkyl, halo, phenyl, a 5- to 6-membered heteroaryl ring and $C_{1-4}$ alkoxy, or $R^{3c}$ and $R^{3d}$ form, together with the C atom to which they are attached, a $C_{3-6}$ cycloalkyl ring or a $C_{3-6}$ heterocycloalkyl ring,
or
$R^{3a}$ and $R^{3c}$ are H, and $R^{3b}$ and $R^{3d}$ form, together with the C atoms to which they are attached, a $C_{5-6}$ cycloalkyl ring, with the proviso that when X is -N($R^{11}$)-, O, S, -S(O)$_2$- or -S(O)(N$R^{11}$)-, then neither $R^{3c}$ nor $R^{3d}$ are halo;
wherein at least two of $R^{3a}$, $R^{3b}$, $R^{3c}$ and $R^{3d}$ are H; and
wherein M is bonded to LINK via a C or N atom within group $R^8$ such that a hydrogen atom on the C or N atom within group $R^8$ is replaced with a bond to LINK.

36. A compound according to any one of the preceding aspects, wherein $R^4$, $R^5$ and $R^6$ are H.

37. A compound according to any one of aspects 1 to 35, wherein one of $R^4$, $R^5$ and $R^6$ is not H.

38. A compound according to aspect 37, wherein one of $R^4$, $R^5$ and $R^6$ is selected from CN, fluoro, chloro, methoxy, methyl and trifluoromethyl, and the rest are H, with the proviso that $R^5$ and $R^6$ are not CN.

39. A compound according to any one of aspects 1 to 28, wherein M is of formula (III):

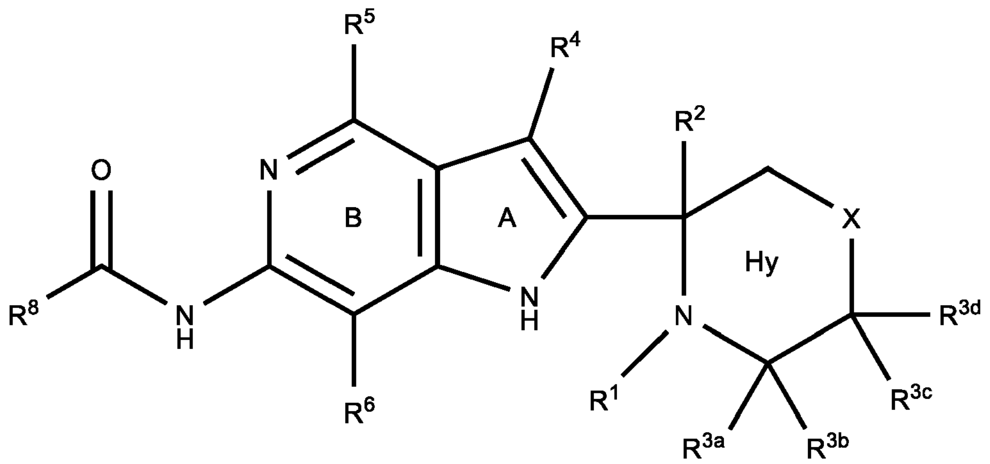

(III)

wherein:

$R^1$ is H or $C_{1-4}$ alkyl which is itself unsubstituted or substituted with one $C_{1-4}$ alkoxy or one, two or three halo, preferably with one $C_{1-4}$ alkoxy;
$R^2$ is H or methyl;
$R^{3a}$ and $R^{3b}$ are independently selected from H, $C_{1-4}$ alkyl, and $C_{1-4}$ alkoxy, or $R^{3a}$ and $R^{3b}$ form, together with the C atom to which they are attached, a $C_{3-6}$ cycloalkyl ring,
$R^{3c}$ and $R^{3d}$ are independently selected from H, $C_{1-4}$ alkyl, halo and $C_{1-4}$ alkoxy, or $R^{3c}$ and $R^{3d}$ form, together with the C atom to which they are attached, a $C_{3-6}$ cycloalkyl ring, or
$R^{3a}$ and $R^{3c}$ are H, and $R^{3b}$ and $R^{3d}$ form, together with the C atoms to which they are attached, a $C_{5-6}$ cycloalkyl ring,
with the proviso that when X is -N(Me)- or O, then neither $R^{3c}$ nor $R^{3d}$ are halo; wherein at least two of $R^{3a}$, $R^{3b}$, $R^{3c}$ and $R^{3d}$ are H;
X is a bond, -N(Me)-, O or $-CH_2-$;
$R^4$ is selected from H, CN, halo, $C_{1-4}$ alkoxy, and $C_{1-4}$ alkyl which is itself unsubstituted or substituted by one, two or three halo;
$R^5$ and $R^6$ are independently selected from H, halo, $C_{1-4}$ alkoxy, and $C_{1-4}$ alkyl which is itself unsubstituted or substituted by one, two or three halo;
$R^8$ is an 8- to 10-membered heteroaryl ring, the group $R^8$ being unsubstituted or substituted by one, two or three substituents independently selected from halo, $C_{1-4}$ alkoxy, $R^{10}$, -($C_{1-4}$ alkylene)-$R^{10}$, =O, -CN, and $C_{1-4}$ alkyl which is itself unsubstituted or substituted by one or two $R^9$;
each $R^9$ is independently selected from halo and $C_{1-4}$ alkoxy;
$R^{10}$ is a group selected from a 5- to 6-membered heteroaryl ring, a 3- to 6-membered cycloalkyl ring, a 4- to 6-membered heterocyclyl ring, and a phenyl ring, the group $R^{10}$ being unsubstituted or substituted by one or two substituents independently selected from $C_{1-4}$ alkyl, $C_{1-4}$ alkoxy, and halo; and
wherein M is bonded to LINK via a C or N atom within group $R^8$ such that a hydrogen atom on the C or N atom within group $R^8$ is replaced with a bond to LINK.

40. A compound according to aspect 39, wherein the C or N atom within group $R^8$ that is bonded to LINK is a C or N atom of the heteroaryl ring of group $R^8$, or a C or N atom of the heteroaryl, cycloalkyl, heterocyclyl or phenyl ring of $R^{10}$.

41. A compound according to any one of aspects 1 to 40, wherein $R^{10}$ is selected from cyclopropyl, cyclobutyl, cyclopentyl, cyclohexyl, imidazolyl, oxadiazolyl, oxetanyl, phenyl, pyrazinyl, pyrazolyl, pyridazinyl, pyridinyl, pyrimidinyl, pyrrolidinyl, thiazinyl, thiazolyl and triazolyl, more preferably pyrazolyl and cyclopropyl, most preferably cyclopropyl.

42. A compound according to aspect 41, wherein $R^8$ is an 8- to 10-membered heteroaryl ring which is unsubstituted or substituted by one or two $C_{1-4}$ alkyl.

43. A compound according to any one of the preceding aspects, wherein Hy is an unsubstituted pyrrolidine ring, $R^1$ is methyl, and $R^2$ is H.

44. A compound according to aspect 1, wherein the PROTAC has a structure selected from:

1-(9-(2-(2,6-dioxopiperidin-3-yl)-1-oxoisoindolin-4-yl)non-8-yn-1-yl)-3-methyl-*N*-(2-((*R*)-1-methylpyrrolidin-2-yl)-1*H*-pyrrolo[3,2-c]pyridin-6-yl)-1*H*-indazole-6-carboxamide;
rac-1-(8-{[2-(2,6-dioxopiperidin-3-yl)-1,3-dioxoisoindol-4-yl]amino}octyl)-3-methyl-N-[2-(1-methylpyrrolidin-2-yl)-1H-pyrrolo[3,2-c]pyridin-6-yl]indazole-6-carboxamide;
1-(8-{[2-(2,6-dioxopiperidin-3-yl)-1,3-dioxoisoindol-4-yl]amino}octyl)-3-methyl-N-{2-[(2R)-1-methylpyrrolidin-2-yl]-1H-pyrrolo[3,2-c]pyridin-6-yl}indazole-6-carboxamide;
1-(8-{[2-(2,6-dioxopiperidin-3-yl)-1,3-dioxoisoindol-4-yl]amino}octyl)-3-methyl-N-{2-[(2S)-1-methylpyrrolidin-2-yl]-1H-pyrrolo[3,2-c]pyridin-6-yl}indazole-6-carboxamide;
2-(8-((2-(2,6-dioxopiperidin-3-yl)-1,3-dioxoisoindolin-4-yl)amino)octyl)-3-methyl-N-(2-((R)-1-methylpyrrolidin-2-yl)-1H-pyrrolo[3,2-c]pyridin-6-yl)-2H-indazole-6-carboxamide;
3-[4-({4-[2-(2,6-dioxopiperidin-3-yl)-6-fluoro-1,3-dioxoisoindol-5-yl]piperazin-1-yl}methyl)piperidin-1-yl]-1-methyl-N-{2-[(2R)-1-methylpyrrolidin-2-yl]-1H-pyrrolo[3,2-c]pyridin-6-yl}indazole-5-carboxamide;
1-[1-(3-{[2-(2,6-dioxopiperidin-3-yl)-1,3-dioxoisoindol-4-yl]amino}propyl)piperidin-4-yl]-3-methyl-N-{2-[(2R)-1-methylpyrrolidin-2-yl]-1H-pyrrolo[3,2-c]pyridin-6-yl}indazole-6-carboxamide;
1-(8-{[2-(2,6-dioxopiperidin-3-yl)-1,3-dioxoisoindol-5-yl]oxy}octyl)-3-methyl-N-{2-[(2R)-1-methylpyrrolidin-2-yl]-1H-pyrrolo[3,2-c]pyridin-6-yl}indazole-6-carboxamide;
1-{8-[3-(2,4-dioxo-1,3-diazinan-1-yl)-2-methylphenoxy]octyl}-3-methyl-N-{2-[(2R)-1-methylpyrrolidin-2-yl]-1H-pyrrolo[3,2-c]pyridin-6-yl}indazole-6-carboxamide;
1-{6-[3-(2,4-dioxo-1,3-diazinan-1-yl)-2-methylphenoxy]hexyl}-3-methyl-N-{2-[(2R)-1-methylpyrrolidin-2-yl]-1H-pyrrolo[3,2-c]pyridin-6-yl}indazole-6-carboxamide;
1-{7-[1-(2,6-dioxopiperidin-3-yl)-3-methyl-2-oxo-1,3-benzodiazol-4-yl]hept-6-yn-1-yl}-3-methyl-N- f 2-[(2R)-1-methylpyrrolidin-2-yl]-1H-pyrrolo[3,2-c]pyridin-6-yl}indazole-6-carboxamide;
1-(8-((2-(2,6-dioxopiperidin-3-yl)-1,3-dioxoisoindolin-4-yl)oxy)octyl)-3-methyl-N-(2-((R)-1-methylpyrrolidin-2-yl)-1H-pyrrolo[3,2-c]pyridin-6-yl)-1H-indazole-6-carboxamide;
1-(3-((2-(2,6-dioxopiperidin-3-yl)-1,3-dioxoisoindolin-4-yl)amino)propyl)-3-methyl-N-(2-((R)-1-methylpyrrolidin-2-yl)-1H-pyrrolo[3,2-c]pyridin-6-yl)-1H-indazole-6-carboxamide;
1-(8-{[2-(2,6-dioxopiperidin-3-yl)-1,3-dioxoisoindol-5-yl]amino}octyl)-3-methyl-N-{2-[(2R)-1-methylpyrrolidin-2-yl]-1H-pyrrolo[3,2-c]pyridin-6-yl}indazole-6-carboxamide;
1-(8-{[2-(2,6-dioxopiperidin-3-yl)-1,3-dioxoisoindol-5-yl]amino}octyl)-3-methyl-N-{2-[(2S)-1-methylpyrrolidin-2-yl]-1H-pyrrolo[3,2-c]pyridin-6-yl}indazole-6-carboxamide;
1-(8-{[2-(2,6-dioxopiperidin-3-yl)-1-oxo-3H-isoindol-4-yl]amino}octyl)-3-methyl-N-{2-[(2R)-1-methylpyrrolidin-2-yl]-1H-pyrrolo[3,2-c]pyridin-6-yl}indazole-6-carboxamide;
1-(8-{[2-(2,6-dioxopiperidin-3-yl)-1-oxo-3H-isoindol-4-yl]amino}octyl)-3-methyl-N-{2-[(2S)-1-methylpyrrolidin-2-yl]-1H-pyrrolo[3,2-c]pyridin-6-yl}indazole-6-carboxamide;
N-(3-chloro-2-((R)-1-methylpyrrolidin-2-yl)-1H-pyrrolo[3,2-c]pyridin-6-yl)-1-(8-((2-(2,6-dioxopiperidin-3-yl)-1,3-dioxoisoindolin-4-yl)amino)octyl)-3-methyl-1H-indazole-6-carboxamide;
1-(4-{4-[2-(2,6-dioxopiperidin-3-yl)-1,3-dioxoisoindol-5-yl]piperazin-1-yl}butyl)-3-methyl-N-{2-[(2R)-1-methylpyrrolidin-2-yl]-1H-pyrrolo[3,2-c]pyridin-6-yl}indazole-6-carboxamide;
1-(4-{4-[2-(2,6-dioxopiperidin-3-yl)-1,3-dioxoisoindol-5-yl]piperazin-1-yl}butyl)-3-methyl-N-{2-[(2S)-1-methylpyrrolidin-2-yl]-1H-pyrrolo[3,2-c]pyridin-6-yl}indazole-6-carboxamide;
1-(4-((2-(2,6-dioxopiperidin-3-yl)-1,3-dioxoisoindolin-4-yl)amino)butyl)-3-methyl-N-(2-((R)-1-methylpyrrolidin-2-yl)-1H-pyrrolo[3,2-c]pyridin-6-yl)-1H-indazole-6-carboxamide;
1-(6-{[2-(2,6-dioxopiperidin-3-yl)-1,3-dioxoisoindol-4-yl]amino}hexyl)-3-methyl-N-{2-[(2R)-1-methylpyrrolidin-2-yl]-1H-pyrrolo[3,2-c]pyridin-6-yl}indazole-6-carboxamide;
1-(5-(4-(2-(2,6-dioxopiperidin-3-yl)-6-fluoro-1,3-dioxoisoindolin-5-yl)piperazin-1-yl)pentyl)-3-methyl-N-(2-((R)-1-methylpyrrolidin-2-yl)-1H-pyrrolo[3,2-c]pyridin-6-yl)-1H-indazole-6-carboxamide;
1-(2-(2-(4-(2-(2,6-dioxopiperidin-3-yl)-6-fluoro-1,3-dioxoisoindolin-5-yl)piperazin-1-yl)ethoxy)ethyl)-3-methyl-*N*-(2-((*R*)-1-methylpyrrolidin-2-yl)-1*H*-pyrrolo[3,2-c]pyridin-6-yl)-1*H*-indazole-6-carboxamide;
1-(2-(2-(2-((2-(2,6-dioxopiperidin-3-yl)-1,3-dioxoisoindolin-4-yl)amino)ethoxy)ethoxy)ethyl)-3-methyl-*N*-(2-((R)-1-methylpyrrolidin-2-yl)-1*H*-pyrrolo[3,2-c]pyridin-6-yl)-1*H*-indazole-6-carboxamide;
1-(2-(2-((2-(2,6-dioxopiperidin-3-yl)-1,3-dioxoisoindolin-4yl)amino)ethoxy)ethyl)-3-methyl-*N*-(2-((*R*)-1-methylpyrrolidin-2-yl)-1*H*-pyrrolo[3,2-c]pyridin-6-yl)-1*H*-indazole-6-carboxamide;
1-(3-(1-(2-(2,6-dioxopiperidin-3-yl)-1,3-dioxoisoindolin-4-yl)piperidin-4-yl)propyl)-3-methyl-*N*-(2-((*R*)-1-methylpyrrolidin-2-yl)-1*H*-pyrrolo[3,2-c]pyridin-6-yl)-1*H*-indazole-6-carboxamide;
1-(3-(1-(4-((2,6-dioxopiperidin-3-yl)carbamoyl)-2-fluorophenyl)piperidin-4-yl)propyl)-3-methyl-N-(2-((*R*)-1-methylpyrrolidin-2-yl)-1H-pyrrolo[3,2-c]pyridin-6-yl)-1H-indazole-6-carboxamide;
1-(5-(1-(2-(2,6-dioxopiperidin-3-yl)-1,3-dioxoisoindolin-4-yl)piperidin-4-yl)pentyl)-3-methyl-*N*-(2-((*R*)-1-methylpyrrolidin-2-yl)-1*H*-pyrrolo[3,2-c]pyridin-6-yl)-1*H*-indazole-6-carboxamide;
1-(7-(2-(2, 6-dioxopiperidin-3-yl)-1-oxoisoindolin-4-yl) hept-6-yn-1-yl)-3-methyl-*N*-(2-((*R*)-1-methylpyrrolidin-2-yl)-1*H*-pyrrolo [3, 2-c] pyridin-6-yl)-1*H*-indazole-6-carboxamide;
1-(3-((3-(2-(2,6-dioxopiperidin-3-yl)-1-oxoisoindolin-4-yl)prop-2-yn-1-yl)oxy)propyl)-3-methyl-*N*-(2-((*R*)-1-methylpyrrolidin-2-yl)-1*H*-pyrrolo[3,2-c]pyridin-6-yl)-1*H*-indazole-6-carboxamide;
1-(5-(2-(2,6-dioxopiperidin-3-yl)-1-oxoisoindolin-4-yl)pent-4-yn-1-yl)-3-methyl-*N*-(2-((*R*)-1-methylpyrrolidin-2-yl)-1*H*-pyrrolo[3,2-c]pyridin-6-yl)-1*H*-indazole-6-carboxamide;
1-(7-(2-(2,6-dioxopiperidin-3-yl)-1,3-dioxoisoindolin-4-yl)hept-6-yn-1-yl)-3-methyl-*N*-(2-((*R*)-1-methylpyrrolidin-2-yl)-1*H*-pyrrolo[3,2-c]pyridin-6-yl)-1*H*-indazole-6-carboxamide;
1-(7-(3-((2,6-dioxopiperidin-3-yl)carbamoyl)-2-fluorophenyl)hept-6-yn-1-yl)-3-methyl-*N*-(2-((*R*)-1-methylpyrrolidin-2-yl)-1*H*-pyrrolo[3,2-c]pyridin-6-yl)-1*H*-indazole-6-carboxamide;
1-(7-(3-((2,6-dioxopiperidin-3-yl)carbamoyl)-2-methylphenyl)hept-6-yn-1-yl)-3-methyl-*N*-(2-((*R*)-1-methylpyrrolidin-2-yl)-1*H*-pyrrolo[3,2-c]pyridin-6-yl)-1*H*-indazole-6-carboxamide;
1-(8-((2-(2,6-dioxopiperidin-3-yl)-1,3-dioxoisoindolin-4-yl)amino)octyl)-*N*-(2-((*R*)-1-methylpyrrolidin-2-yl)-1*H*-pyrrolo[3,2-c]pyridin-6-yl)-1*H*-indazole-5-carboxamide;
1-(8-((2-(2,6-dioxopiperidin-3-yl)-1,3-dioxoisoindolin-4-yl)amino)octyl)-N-(2-((S)-1-methylpyrrolidin-2-yl)-1H-pyrrolo[3,2-c]pyridin-6-yl)-1H-indazole-5-carboxamide;
1-(9-(2-(2,6-dioxopiperidin-3-yl)-1-oxoisoindolin-4-yl)non-8-yn-1-yl)-*N*-(2-((*R*)-1-methylpyrrolidin-2-yl)-1*H*-pyrrolo[3, 2-*c*]pyridin-6-yl)-1*H*-indazole-5-carboxamide;
1-(9-(1-(2,6-dioxopiperidin-3-yl)-3-methyl-2-oxo-2,3-dihydro-1*H*-benzo[d]imidazol-5-yl)non-8-yn-1-yl)-3-methyl-*N*-(2-((R)-1-methylpyrrolidin-2-yl)-1*H*-pyrrolo[3,2-c]pyridin-6-yl)-1*H*-indazole-6-carboxamide;
1-((E)-9-chloro-9-(1-(2,6-dioxopiperidin-3-yl)-3-methyl-2-oxo-2,3-dihydro-1*H*-benzo[d]imidazol-5-yl)non-8-en-1-yl)-3-methyl-*N*-(2-((*R*)-1-methylpyrrolidin-2-yl)-1*H*-pyrrolo[3,2-*c*]pyridin-6-yl)-1*H*-indazole-6-carboxamide;
1-((Z)-9-chloro-9-(1-(2,6-dioxopiperidin-3-yl)-3-methyl-2-oxo-2,3-dihydro-1*H*-benzo[d]imidazol-5-yl)non-8-en-1-yl)-3-methyl-*N*-(2-((*R*)-1-methylpyrrolidin-2-yl)-1*H*-pyrrolo[3,2-*c*]pyridin-6-yl)-1*H*-indazole-6-carboxamide;
1-(9-chloro-9-(1-(2,6-dioxopiperidin-3-yl)-3-methyl-2-oxo-2,3-dihydro-1*H*-benzo[d]imidazol-5-yl)non-8-en-1-yl)-3-methyl-*N*-(2-((*R*)-1-methylpyrrolidin-2-yl)-1*H*-pyrrolo[3,2-c]pyridin-6-yl)-1*H*-indazole-6-carboxamide;
1-(9-(6-(((*S*)-2,6-dioxopiperidin-3-yl)carbamoyl)pyridin-3-yl)non-8-yn-1-yl)-3-methyl-N-(2-((*R*)-1-methylpyrrolidin-2-yl)-1H-pyrrolo[3,2-c]pyridin-6-yl)-1H-indazole-6-carboxamide;
1-(9-(6-(((*S*)-2,6-dioxopiperidin-3-yl)carbamoyl)pyridin-2-yl)non-8-yn-1-yl)-3-methyl-N-(2-((*R*)-1-methylpyrrolidin-2-yl)-1H-pyrrolo[3,2-c]pyridin-6-yl)-1H-indazole-6-carboxamide;
1-(9-(2-(((*S*)-2,6-dioxopiperidin-3-yl)carbamoyl)pyridin-4-yl)non-8-yn-1-yl)-3-methyl-N-(2-((*R*)-1-methylpyrrolidin-2-yl)-1H-pyrrolo[3,2-c]pyridin-6-yl)-1H-indazole-6-carboxamide;
1-(9-(2-(2,6-dioxopiperidin-3-yl)-1-oxoisoindolin-4-yl)non-8-yn-1-yl)-3-methyl-*N*-(2-((R)-1-(methyl-d3)pyrrolidin-2-yl)-1*H*-pyrrolo[3,2-c]pyridin-6-yl)-1*H*-indazole-6-carboxamide;
1-(8-(2-(2,6-dioxopiperidin-3-yl)-1-oxoisoindolin-4-yl)oct-7-yn-1-yl)-3-methyl-*N*-(2-((R)-1-methylpyrrolidin-2-yl)-1*H*-pyrrolo[3,2-c]pyridin-6-yl)-1*H*-indazole-6-carboxamide;
1-(6-(2-(2,6-dioxopiperidin-3-yl)-1-oxoisoindolin-4-yl)hex-5-yn-1-yl)-3-methyl-*N*-(2-((*R*)-1-methylpyrrolidin-2-yl)-1*H*-pyrrolo[3,2-c]pyridin-6-yl)-1*H*-indazole-6-carboxamide;
1-(5-(4-(2-(2,6-dioxopiperidin-3-yl)-1,3-dioxoisoindolin-4-yl)piperazin-1-yl)pentyl)-3-methyl-*N*-(2-((*R*)-1-methylpyrrolidin-2-yl)-1*H*-pyrrolo[3,2-c]pyridin-6-yl)-1*H*-indazole-6-carboxamide;
1-(9-(2-(2,6-dioxopiperidin-3-yl)-1-oxoisoindolin-4-yl)non-8-yn-1-yl)-*N*-(2-((R)-1-ethylpyrrolidin-2-yl)-1*H*-pyrrolo[3,2-c]pyridin-6-yl)-3-methyl-1*H*-indazole-6-carboxamide;
1-(5-((3-(2-(2,6-dioxopiperidin-3-yl)-1-oxoisoindolin-4-yl)prop-2-yn-1-yl)oxy)pentyl)-3-methyl-*N*-(2-((R)-1-methylpyrrolidin-2-yl)-1*H*-pyrrolo[3,2-c]pyridin-6-yl)-1*H*-indazole-6-carboxamide;
1-(2-((6-(2-(2,6-dioxopiperidin-3-yl)-1-oxoisoindolin-4-yl)hex-5-yn-1-yl)oxy)ethyl)-3-methyl-*N*-(2-((R)-1-methylpyrrolidin-2-yl)-1*H*-pyrrolo[3,2-c]pyridin-6-yl)-1*H*-indazole-6-carboxamide;
1-(9-(3-(((S)-2,6-dioxopiperidin-3-yl)carbamoyl)-2-fluorophenyl)non-8-yn-1-yl)-3-methyl-*N*-(2-((R)-1-methylpyrrolidin-2-yl)-1*H*-pyrrolo[3,2-c]pyridin-6-yl)-1*H*-indazole-6-carboxamide;
1-(9-(2-(2,6-dioxopiperidin-3-yl)-7-fluoro-1-oxoisoindolin-4-yl)non-8-yn-1-yl)-3-methyl-*N*-(2-((*R*)-1-methylpyrrolidin-2-yl)-1*H*-pyrrolo[3,2-c]pyridin-6-yl)-1*H*-indazole-6-carboxamide;
*N*-(3-chloro-2-((R)-1-methylpyrrolidin-2-yl)-1*H*-pyrrolo[3,2-c]pyridin-6-yl)-1-(9-(2-(2,6-dioxopiperidin-3-yl)-1-oxoisoindolin-4-yl)non-8-yn-1-yl)-3-methyl-1*H*-indazole-6-carboxamide;
1-(10-(2-(2,6-dioxopiperidin-3-yl)-1-oxoisoindolin-4-yl) dec-9-yn-1-yl)-3-methyl-*N*-(2-((R)-1-methylpyrrolidin-2-yl)-1*H*-pyrrolo[3,2-c]pyridin-6-yl)-1*H*-indazole-6-carboxamide;
1-(9-(2-(2, 6-dioxopiperidin-3-yl)-1-oxoisoindolin-4-yl) non-8-yn-1-yl)-*N*-(2-((R)-1-methylpyrrolidin-2-yl)-1*H*-pyrrol[3, 2-c]pyridin-6-yl)-1*H*-indazole-6-carboxamide;
1-(9-(2-(2,6-dioxopiperidin-3-yl)-1-oxoisoindolin-4-yl)non-8-yn-1-yl)-3-methyl-*N*-(2-((*R*)-5-methyl-5-azaspiro[2.4]heptan-6-yl)-1*H*-pyrrolo[3,2-c]pyridin-6-yl)-1*H*-indazole-6-carboxamide;
1-(9-(2-(2,6-dioxopiperidin-3-yl)-1-oxoisoindolin-4-yl)non-8-yn-1-yl)-3-methyl-*N*-(3-methyl-2-((*R*)-1-methylpyrrolidin-2-yl)-1*H*-pyrrolo[3,2-c]pyridin-6-yl)-1*H*-indazole-6-carboxamide;
1-(9-(2-(2,6-dioxopiperidin-3-yl)-1-oxoisoindolin-4-yl)non-8-yn-1-yl)-5-fluoro-3-methyl-*N*-(2-((*R*)-1-methylpyrrolidin-2-yl)-1*H*-pyrrolo[3,2-*c*]pyridin-6-yl)-1*H*-indazole-6-carboxamide;
3-cyclopropyl-1-(9-(2-(2, 6-dioxopiperidin-3-yl)-1-oxoisoindolin-4-yl) non-8-yn-1-yl)-*N*-(2-((*R*)-1-methylpyrrolidin-2-yl)-1*H*-pyrrolo [3, 2-c] pyridin-6-yl)-1*H*-indazole-6-carboxamide;
1-(9-(2-(2,6-dioxopiperidin-3-yl)-6-fluoro-1-oxoisoindolin-5-yl)non-8-yn-1-yl)-3-methyl-*N*-(2-((*R*)-1-methylpyrrolidin-2-yl)-1*H*-pyrrolo[3,2-c]pyridin-6-yl)-1*H*-indazole-6-carboxamide;
1-(9-(3-(2, 6-dioxopiperidin-3-yl)-2-fluorophenyl) non-8-yn-1-yl)-3-methyl-*N*-(2-((*R*)-1-methylpyrrolidin-2-yl)-1*H*-pyrrolo [3, 2-*c*] pyridin-6-yl)-1*H*-indazole-6-carboxamide;
1-(9-(6-(2, 6-dioxopiperidin-3-yl) pyridin-2-yl) non-8-yn-1-yl)-3-methyl-*N*-(2-((*R*)-1-methylpyrrolidin-2-yl)-1*H*-pyrrolo [3, 2-c] pyridin-6-yl)-1*H*-indazole-6-carboxamide;
(*R*)-1-(9-(3-(2, 4-dioxotetrahydropyrimidin-1(2*H*)-yl)-2-fluorophenyl) non-8-yn-1-yl)-3-methyl-*N*-(2-(1-methylpyrrolidin-2-yl)-1H-pyrrolo [3, 2-c] pyridin-6-yl)-1*H*-indazole-6-carboxamide;
(*R*)-1-(9-(6-(2,4-dioxotetrahydropyrimidin-1(2*H*)-yl)pyridin-2-yl)non-8-yn-1-yl)-3-methyl-*N*-(2-(1-methylpyrrolidin-2-yl)-1*H*-pyrrolo[3,2-c]pyridin-6-yl)-1*H*-indazole-6-carboxamide;
(*R*)-1-(9-(3-(2,4-dioxotetrahydropyrimidin-1(2*H*)-yl)-5-fluoro-1-methyl-1*H*-indazol-6-yl)non-8-yn-1-yl)-3-methyl-*N*-(2-(1-methylpyrrolidin-2-yl)-1*H*-pyrrolo[3,2-c]pyridin-6-yl)-1*H*-indazole-6-carboxamide;
*N*-(3-cyano-2-((*R*)-1-methylpyrrolidin-2-yl)-1*H*-pyrrolo[3,2-c]pyridin-6-yl)-1-(9-(2-(2,6-dioxopiperidin-3-yl)-1-oxoisoindolin-4-yl)non-8-yn-1-yl)-3-methyl-1*H*-indazole-6-carboxamide;
*N*-(3-chloro-2-((*R*)-1-methylpyrrolidin-2-yl)-1*H*-pyrrolo[3,2-c]pyridin-6-yl)-1-(9-(2-(2,6-dioxopiperidin-3-yl)-7-fluoro-1-oxoisoindolin-4-yl)non-8-yn-1-yl)-3-methyl-1*H*-indazole-6-carboxamide;
1-(1-(3-(2-(2,6-dioxopiperidin-3-yl)-1-oxoisoindolin-4-yl)prop-2-yn-1-yl)piperidin-4-yl)-3-methyl-*N*-(2-((*R*)-1-methylpyrrolidin-2-yl)-1*H*-pyrrolo[3,2-c]pyridin-6-yl)-1*H*-indazole-6-carboxamide;
1-(1-((1-(2-(2,6-dioxopiperidin-3-yl)-6-fluoro-1,3-dioxoisoindolin-5-yl)piperidin-4-yl)methyl)piperidin-4-yl)-3-methyl-*N*-(2-((*R*)-1-methylpyrrolidin-2-yl)-1*H*-pyrrolo[3,2-c]pyridin-6-yl)-1*H*-indazole-6-carboxamide;
1-(1-((1-(2-(2,6-dioxopiperidin-3-yl)-1,3-dioxoisoindolin-5-yl)piperidin-4-yl)methyl)piperidin-4-yl)-3-methyl-*N*-(2-((*R*)-1-methylpyrrolidin-2-yl)-1*H*-pyrrolo[3,2-*c*]pyridin-6-yl)-1*H*-indazole-6-carboxamide;
1-(1-((1-(2-(2,6-dioxopiperidin-3-yl)-1,3-dioxoisoindolin-4-yl)piperidin-4-yl)methyl)piperidin-4-yl)-3-methyl-*N*-(2-((*R*)-1-methylpyrrolidin-2-yl)-1*H*-pyrrolo[3,2-*c*]pyridin-6-yl)-1*H*-indazole-6-carboxamide;
1-(3-(2-(2,6-dioxopiperidin-3-yl)-1-oxoisoindolin-4-yl)prop-2-yn-1-yl)-3-methyl-*N*-(2-((*R*)-1-methylpyrrolidin-2-yl)-1*H*-pyrrolo[3,2-*c*]pyridin-6-yl)-1*H*-indazole-6-carboxamide;
1-(6-(1-(6-(((S)-2,6-dioxopiperidin-3-yl)carbamoyl)pyridin-2-yl)piperidin-4-yl)hexyl)-3-methyl-N-(2-((R)-1-methylpyrrolidin-2-yl)-1H-pyrrolo[3,2-c]pyridin-6-yl)-1H-indazole-6-carboxamide; and
1-(4-(5-(2-(2,6-dioxopiperidin-3-yl)-1-oxoisoindolin-4-yl) pent-4-yn-1-yl) cyclohexyl)-3-methyl-N-(2-((R)-1-methylpyrrolidin-2-yl)-1H-pyrrolo[3,2-c] pyridin-6-yl)-1H-indazole-6-carboxamide.

45. A compound according to any one of aspects 1 to 28, wherein M is of formula (IV):

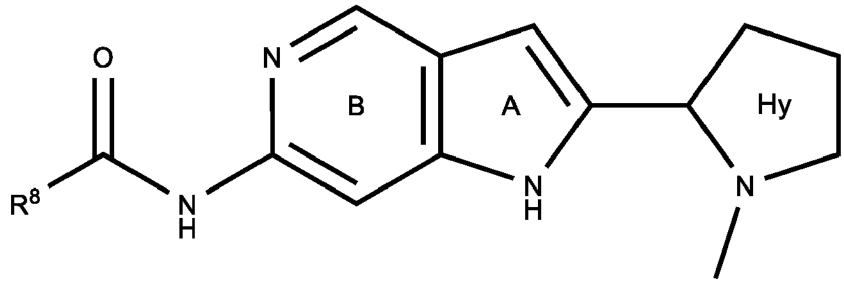

(IV)

wherein $R^8$ is an 8- to 10-membered heteroaryl ring, the group $R^8$ being unsubstituted or substituted by one, two or

three substituents independently selected from halo, $C_{1-4}$ alkyl and $R^{10}$;
$R^{10}$ is a 3-membered cycloalkyl ring;
wherein M is bonded to LINK via a C or N atom within group $R^8$ such that a hydrogen atom on the C or N atom within group $R^8$ is replaced with a bond to LINK.

46. A compound according to any one of aspects 1 to 45, wherein $R^8$ is indazolyl.

47. A compound according to any one of the preceding aspects, wherein the group $R^8$ is unsubstituted or substituted by one cyclopropyl group or one or two methyl groups.

48. A compound according to any one of the preceding aspects, wherein the group $R^8$ is an indazolyl group which is unsubstituted or substituted by one or two methyl groups.

49. A compound according to any one of aspects 48 to 50, wherein $R^8$ is selected from one of the following structures:

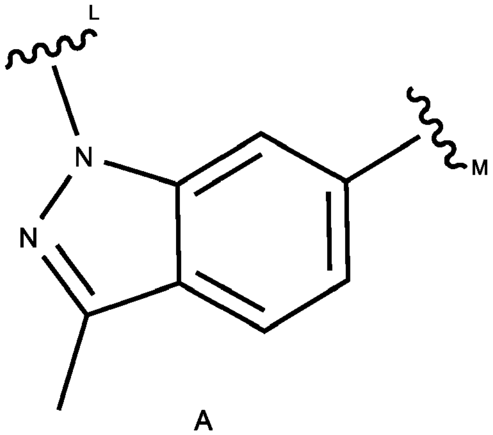

A

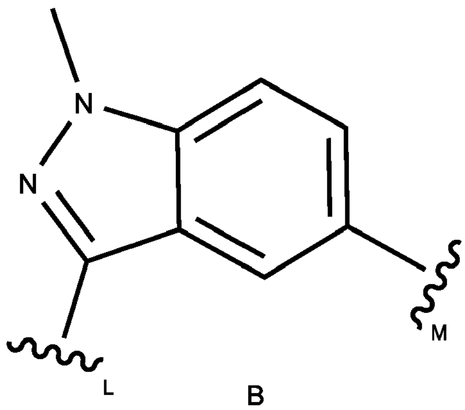

B

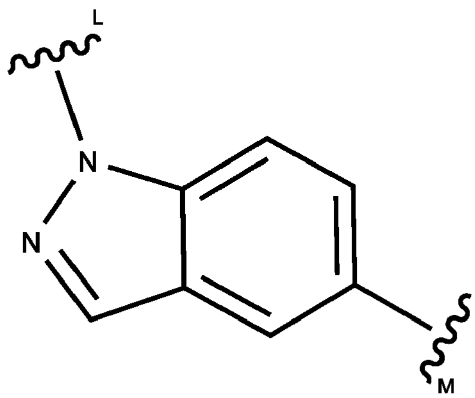

C

wherein:

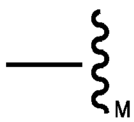

represents the point of attachment of $R^8$ to the rest of moiety M;

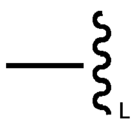

represents the point of attachment of $R^8$ to LINK.

50. A compound according to any one of aspects 1 to 35 and 39 to 43, wherein at least one of $R^4$, $R^5$ and $R^6$ is not H.

51. A compound which is a bicyclic compound of formula (I') or a pharmaceutically acceptable salt thereof:

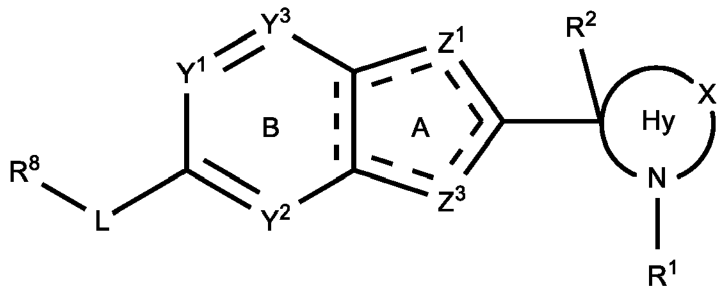

(I')

wherein:

one of $Z^1$ and $Z^3$ is -N(H)- and the other is N or -C($R^4$)-, $Y^1$ is N, $Y^2$ is N or -C($R^6$)-, and $Y^3$ is N or -C($R^5$)-;
Hy is a 4- to 7-membered heterocyclic ring containing X and at least one N atom, wherein: ring Hy is linked to ring A via a C atom within ring Hy, said C atom also being linked to $R^2$; a N atom within ring Hy is substituted by $R^1$; X is a bond, -N($R^{11}$)-, O, S, -S(O)$_2$-, -S(O)(N$R^{11}$)-, or -C($R^{11}$)$_2$-; and the rest of ring Hy is unsubstituted or substituted by one or two $R^3$;
L is -C(O)N(H)-, wherein the C atom of L is bonded to $R^8$, and the N atom of L is bonded to ring B;
$R^1$ is H, $C_{1-4}$ cycloalkyl, or $C_{1-4}$ alkyl which is itself unsubstituted or substituted with one $C_{1-4}$ alkoxy or one, two or three halo;
$R^2$ is H or methyl;
each $R^3$ is independently selected from $C_{1-4}$ alkyl, $C_{1-4}$ alkoxy, phenyl, a 5- to 6-membered heteroaryl ring and halo, or (i) two $R^3$ linked to adjacent C atoms in ring Hy form, together with the C atoms to which they are attached, a $C_{5-6}$ cycloalkyl ring, or (ii) two $R^3$ linked to the same C atom in ring Hy form, together with the C atom to which they are attached, a $C_{3-6}$ cycloalkyl ring or a $C_{3-6}$ heterocycloalkyl ring;
$R^4$ and $R^6$ are independently selected from H, halo, CN, $C_{1-4}$ alkoxy, and $C_{1-4}$ alkyl which is itself unsubstituted or substituted by one, two or three halo;
$R^5$ is selected from H, halo, $C_{1-4}$ alkoxy, $C_{3-5}$ cycloalkyl and $C_{1-4}$ alkyl which is itself unsubstituted or substituted by one, two or three halo;
at least one of $R^4$, $R^5$ and $R^6$ is not H;
$R^8$ is an 8- to 10-membered heteroaryl ring, the group $R^8$ being unsubstituted or substituted by one, two or three substituents independently selected from halo, $C_{1-4}$ alkoxy, $R^{10}$, -($C_{1-4}$ alkylene)-$R^{10}$, =O, -CN, and $C_{1-4}$ alkyl which is itself unsubstituted or substituted by one or two $R^9$;
each $R^9$ is independently selected from halo and $C_{1-4}$ alkoxy;
$R^{10}$ is a group selected from a 5- to 6-membered heteroaryl ring, a 3- to 6-membered cycloalkyl ring, a 4- to 6-membered heterocyclyl ring, and a phenyl ring, the group $R^{10}$ being unsubstituted or substituted by one or two substituents independently selected from $C_{1-4}$ alkyl, $C_{1-4}$ alkoxy, and halo;
each $R^{11}$ is independently selected from H, $C_{1-4}$ alkyl, and $C_{1-4}$ cycloalkyl.

52. A compound according to any one of the preceding aspects, wherein the bond from ring Hy to ring A is in the "up" position and the bond from ring Hy to $R^2$ is in the "down" position relative to ring Hy, as depicted below:

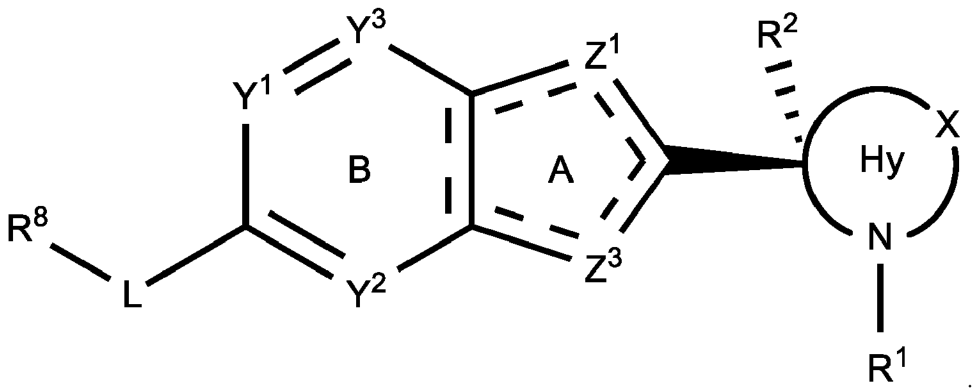

53. A compound according to aspect 51 which is selected from:

N-{3-chloro-2-[(2R)-1-methylpyrrolidin-2-yl]-1H-pyrrolo[3,2-c]pyridin-6-yl}-1,3-dimethylindazole-6-carboxamide;
N-{3-chloro-2-[(2S)-1-methylpyrrolidin-2-yl]-1H-pyrrolo[3,2-c]pyridin-6-yl}-1,3-dimethylindazole-6-carboxamide;
(R)-1,3-dimethyl-N-(2-(1-methylpyrrolidin-2-yl)-3-(trifluoromethyl)-1H-pyrrolo[3,2-c]pyridin-6-yl)-1H-indazole-6-carboxamide;
(S)-1,3-dimethyl-N-(2-(1-methylpyrrolidin-2-yl)-3-(trifluoromethyl)-1H-pyrrolo[3,2-c]pyridin-6-yl)-1H-indazole-6-carboxamide;
N-{3-fluoro-2-[(rel-2S)-1-methylpyrrolidin-2-yl]-1H-pyrrolo[3,2-c]pyridin-6-yl}-1-methylindazole-5-carboxamide;
N-{3-fluoro-2-[(rel-2R)-1-methylpyrrolidin-2-yl]-1H-pyrrolo[3,2-c]pyridin-6-yl}-1-methylindazole-5-carboxamide;
N-{3-fluoro-2-[(rel-2R)-1-methylpyrrolidin-2-yl]-1H-pyrrolo[3,2-c]pyridin-6-yl}-1,3-dimethylindazole-6-carboxamide;
N-{3-fluoro-2-[(rel-2S)-1-methylpyrrolidin-2-yl]-1H-pyrrolo[3,2-c]pyridin-6-yl}-1,3-dimethylindazole-6-carboxamide;
1,3-dimethyl-N-{3-methyl-2-[(rel-2R)-1-methylpyrrolidin-2-yl]-1H-pyrrolo[3,2-c]pyridin-6-yl}indazole-6-carboxamide;
1,3-dimethyl-N-{3-methyl-2-[(rel-2S)-1-methylpyrrolidin-2-yl]-1H-pyrrolo[3,2-c]pyridin-6-yl}indazole-6-carboxamide;
1,3-dimethyl-N-{4-methyl-2-[(rel-2R)-1-methylpyrrolidin-2-yl]-1H-pyrrolo[3,2-c]pyridin-6-yl}indazole-6-carboxamide;
1,3-dimethyl-N-{4-methyl-2-[(rel-2S)-1-methylpyrrolidin-2-yl]-1H-pyrrolo[3,2-c]pyridin-6-yl}indazole-6-carboxamide; and
(R)-N-(3-cyano-2-(1-methylpyrrolidin-2-yl)-1H-pyrrolo[3,2-c]pyridin-6-yl)-1,3-dimethyl-1H-indazole-6-carboxamide;
and the pharmaceutically acceptable salts thereof.

54. A pharmaceutical composition which comprises a compound as defined in any one of aspects 1 to 53 and a pharmaceutically acceptable carrier or diluent.

55. A compound as defined in any one of aspects 1 to 53 for use in the treatment of the human or animal body by therapy.

56. A compound as defined in any one of aspects 1 to 53 for use in the treatment of cancer.

57. The compound for use according to aspect 56, wherein the cancer is a transcriptionally addicted cancer, such as breast cancer, acute leukemia, prostate cancer, bladder cancer, cholangiocarcinoma, colon adenocarcinoma, esophageal carcinoma, head and neck squamous cell carcinoma, kidney chromophobe, kidney renal clear cell carcinoma, kidney renal papillary cell carcinoma, liver hepatocellular carcinoma, lung adenocarcinoma, pheochromocytoma and paraganglioma, rectum adenocarcinoma, stomach adenocarcinoma, uterine corpus endometrial carcinoma, cervical squamous cell carcinoma and endocervical adenocarcinoma, lung squamous cell carcinoma, neuroblastoma, midline glioma or sarcoma, preferably neuroblastoma or midline glioma.

58. The compound for use according to aspect 56, wherein the cancer is acute myeloid leukaemia (AML), myelodysplastic syndrome (MDS), myeloproliferative neoplasm (MPN) or acute lymphoblastic leukaemia (ALL).

59. The compound for use according to aspect 58, wherein the cancer is mixed-lineage leukaemia (MLL) or rearranged acute leukemia (AML and ALL).

60. The compound for use according to aspect 58, wherein the cancer is NPM1 mutant leukemia.

Alternatively, the invention provides compounds according to the following embodiments.

1. A compound which is a bicyclic compound of formula (I') or a pharmaceutically acceptable salt thereof:

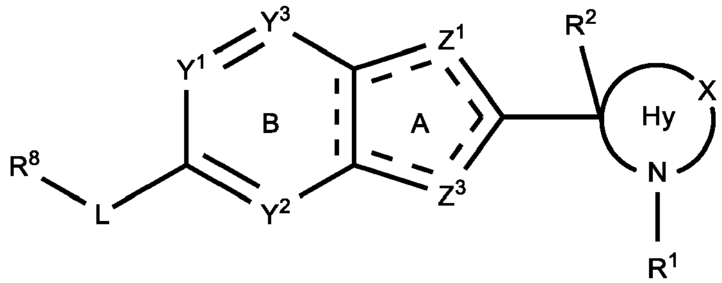

(I')

wherein:

one of $Z^1$ and $Z^3$ is -N(H)- and the other is N or -C($R^4$)-, $Y^1$ is N, $Y^2$ is N or -C($R^6$)-, and $Y^3$ is N or -C($R^5$)-;
Hy is a 4- to 7-membered heterocyclic ring containing X and at least one N atom, wherein: ring Hy is linked to ring A via a C atom within ring Hy, said C atom also being linked to $R^2$; a N atom within ring Hy is substituted by $R^1$; X is a bond, -N($R^{11}$)-, O, S, -S(O)$_2$-, -S(O)($NR^{11}$)-, or -C($R^{11}$)$_2$-; and the rest of ring Hy is unsubstituted or substituted by one or two $R^3$;
L is -C(O)N(H)-, wherein the C atom of L is bonded to $R^8$, and the N atom of L is bonded to ring B;
$R^1$ is H, $C_{1-4}$ cycloalkyl, or $C_{1-4}$ alkyl which is itself unsubstituted or substituted with one $C_{1-4}$ alkoxy or one, two or three halo;
$R^2$ is H or methyl;
each $R^3$ is independently selected from $C_{1-4}$ alkyl, $C_{1-4}$ alkoxy, phenyl, a 5- to 6-membered heteroaryl ring and halo, or (i) two $R^3$ linked to adjacent C atoms in ring Hy form, together with the C atoms to which they are attached, a $C_{5-6}$ cycloalkyl ring, or (ii) two $R^3$ linked to the same C atom in ring Hy form, together with the C atom to which they are attached, a $C_{3-6}$ cycloalkyl ring or a $C_{3-6}$ heterocycloalkyl ring;
$R^4$ and $R^6$ are independently selected from H, halo, CN, $C_{1-4}$ alkoxy, and $C_{1-4}$ alkyl which is itself unsubstituted or substituted by one, two or three halo;
$R^5$ is selected from H, halo, $C_{1-4}$ alkoxy, $C_{3-5}$ cycloalkyl and $C_{1-4}$ alkyl which is itself unsubstituted or substituted by one, two or three halo;
at least one of $R^4$, $R^5$ and $R^6$ is not H;
$R^8$ is an 8- to 10-membered heteroaryl ring, the group $R^8$ being unsubstituted or substituted by one, two or three substituents independently selected from halo, $C_{1-4}$ alkoxy, $R^{10}$, -($C_{1-4}$ alkylene)-$R^{10}$, =O, -CN, and $C_{1-4}$ alkyl which is itself unsubstituted or substituted by one or two $R^9$;
each $R^9$ is independently selected from halo and $C_{1-4}$ alkoxy;
$R^{10}$ is a group selected from a 5- to 6-membered heteroaryl ring, a 3- to 6-membered cycloalkyl ring, a 4- to 6-membered heterocyclyl ring, and a phenyl ring, the group $R^{10}$ being unsubstituted or substituted by one or two substituents independently selected from $C_{1-4}$ alkyl, $C_{1-4}$ alkoxy, and halo;
each $R^{11}$ is independently selected from H, $C_{1-4}$ alkyl, and $C_{1-4}$ cycloalkyl.

2. A compound according to embodiment 1, wherein one of $R^4$, $R^5$ and $R^6$ is selected from halo, $C_{1-4}$ alkoxy, and $C_{1-4}$ alkyl which is itself unsubstituted or substituted by one, two or three halo, and the rest are H.

3. A compound according to embodiment 1 or 2 wherein $R^4$ is selected from halo, $C_{1-4}$ alkoxy, and $C_{1-4}$ alkyl which is

itself unsubstituted or substituted by one, two or three halo.

4. A compound according to embodiment 3, wherein $R^4$ is selected from halo, and $C_{1-4}$ alkyl which is itself substituted by one, two or three halo.

5. A compound according to embodiment 4, wherein $R^4$ is selected from F, Cl and $CF_3$.

6. A compound according to any one of embodiments 1 to 5, wherein $R^5$ is $C_{1-4}$ alkyl.

7. A compound according to embodiment 6, wherein $R^5$ is methyl.

8. A compound according to any one of embodiments 1 to 7, wherein $R^1$ is H, $C_{1-4}$ cycloalkyl, unsubstituted $C_{2-4}$ alkyl, or $C_{1-4}$ alkyl which is itself substituted with one $C_{1-4}$ alkoxy or one, two or three halo.

9. A compound according to any one of embodiments 1 to 7, wherein $R^1$ is $C_{1-4}$ alkyl.

10. A compound according to embodiment 9, wherein $R^1$ is methyl.

11. A compound according to embodiment 8, wherein $R^1$ is H or $C_{2-4}$ alkyl.

12. A compound according to any one of embodiments 1 to 11, wherein $R^2$ is H.

13. A compound according to any one of embodiments 1 to 12, wherein the N atom of ring Hy which is substituted by $R^1$ is adjacent to the C atom of ring Hy that is bonded to ring A.

14. A compound according to any one of embodiments 1 to 13, wherein no more than two of $Y^1$, $Y^2$ and $Y^3$ are N.

15. A compound according to any one of embodiments 1 to 14, wherein $Z^1$ is - $C(R^4)$- and $Z^3$ is -N(H)-.

16. A compound according to any one of embodiments 1 to 15, wherein $Y^2$ is - $C(R^6)$- and $Y^3$ is $-C(R^5)-$.

17. A compound according to any one of embodiments 1 to 16, wherein X is a bond, -N(Me)-, O or $-CH_2-$.

18. A compound according to embodiment 17, wherein X is a bond.

19. A compound according to any one of embodiments 1 to 18, which is a bicyclic compound of formula (II') or a pharmaceutically acceptable salt thereof:

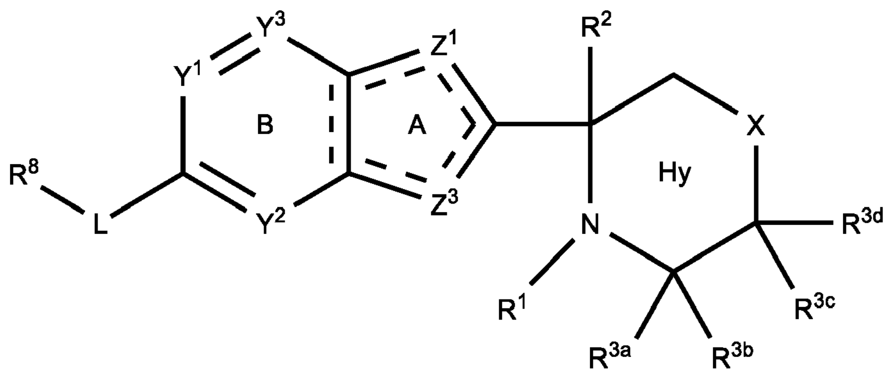

(II')

wherein:

$Z^1$, $Z^3$, $Y^1$, $Y^2$, $Y^3$, $R^1$, $R^2$, $R^8$, X and L are as defined in any one of embodiments 1 to 18;
$R^{3a}$ and $R^{3b}$ are independently selected from H, $C_{1-4}$ alkyl, phenyl, a 5- to 6-membered heteroaryl ring and $C_{1-4}$ alkoxy, or $R^{3a}$ and $R^{3b}$ form, together with the C atom to which they are attached, a $C_{3-6}$ cycloalkyl ring or a $C_{3-6}$ heterocycloalkyl ring,

$R^{3c}$ and $R^{3d}$ are independently selected from H, $C_{1-4}$ alkyl, halo, phenyl, a 5- to 6-membered heteroaryl ring and $C_{1-4}$ alkoxy, or $R^{3c}$ and $R^{3d}$ form, together with the C atom to which they are attached, a $C_{3-6}$ cycloalkyl ring or a $C_{3-6}$ heterocycloalkyl ring,
with the proviso that when X is -N($R^{11}$)-, O, S, -S(O)$_2$- or -S(O)(N$R^{11}$)-, then neither $R^{3c}$ nor $R^{3d}$ are halo;
or
$R^{3a}$ and $R^{3c}$ are H, and $R^{3b}$ and $R^{3d}$ form, together with the C atoms to which they are attached, a $C_{5-6}$ cycloalkyl ring;
wherein at least two of $R^{3a}$, $R^{3b}$, $R^{3c}$ and $R^{3d}$ are H.

20. A compound according to any one of embodiments 1 to 19, which is a bicyclic compound of formula (III') or a pharmaceutically acceptable salt thereof:

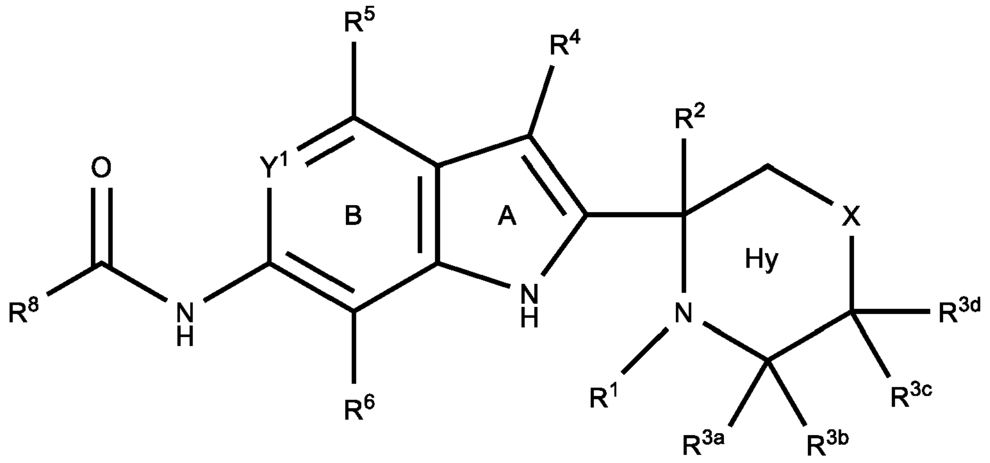

(III')

wherein:
X, $Y^1$, $R^1$, $R^2$, $R^4$, $R^5$, $R^6$, $R^{3a}$, $R^{3b}$, $R^{3c}$, $R^{3d}$ and $R^8$ are as defined in any one of embodiments 1 to 19.

21. A compound according to any one of embodiments 1 to 20, wherein $R^{3a}$ and $R^{3b}$ are independently selected from H, $C_{1-4}$ alkyl, and $C_{1-4}$ alkoxy, or $R^{3a}$ and $R^{3b}$ form, together with the C atom to which they are attached, a $C_{3-6}$ cycloalkyl ring,

$R^{3c}$ and $R^{3d}$ are independently selected from H, $C_{1-4}$ alkyl, halo and $C_{1-4}$ alkoxy, or $R^{3c}$ and $R^{3d}$ form, together with the C atom to which they are attached, a $C_{3-6}$ cycloalkyl ring, with the proviso that when X is -N($R^{11}$)-, O, S, -S(O)$_2$- or -S(O)(N$R^{11}$)-, then neither $R^{3c}$ nor $R^{3d}$ are halo;
or
$R^{3a}$ and $R^{3c}$ are H, and $R^{3b}$ and $R^{3d}$ form, together with the C atoms to which they are attached, a $C_{5-6}$ cycloalkyl ring;
wherein at least two of $R^{3a}$, $R^{3b}$, $R^{3c}$ and $R^{3d}$ are H;

22. A compound according to embodiment 21, wherein $R^{3a}$, $R^{3b}$, $R^{3c}$ and $R^{3d}$ are H.

23. A compound according to any one of embodiments 1 to 22, wherein Hy is a pyrrolidine ring, $R^1$ is methyl, and $R^2$ is H.

24. A compound according to any one of embodiments 1 to 23, wherein the group

$R^8$ is unsubstituted or substituted by one, two or three substituents independently selected from halo, $C_{1-4}$ alkoxy, $R^{10}$, -($C_{1-4}$ alkylene)-$R^{10}$, =O, -CN, and $C_{1-4}$ alkyl which is itself unsubstituted or substituted by one or two $R^9$;
each $R^9$ is independently selected from halo and $C_{1-4}$ alkoxy;
$R^{10}$ is a group selected from a 5- to 6-membered heteroaryl ring, a 3- to 6-membered cycloalkyl ring, a 4- to 6-membered heterocyclyl ring, and a phenyl ring, the group $R^{10}$ being unsubstituted or substituted by one or two substituents independently selected from $C_{1-4}$ alkyl, $C_{1-4}$ alkoxy, and halo.

25. A compound according to any one of embodiments 1 to 24, wherein the group $R^8$ is unsubstituted or substituted by one, two or three substituents independently selected from halo, $C_{1-4}$ alkoxy, $R^{10}$, -($C_{1-4}$ alkylene)-$R^{10}$, =O, -CN, and $C_{1-4}$ alkyl which is itself unsubstituted or substituted by one $R^9$, preferably wherein $R^{10}$ is selected from cyclopropyl, cyclobutyl, cyclopentyl, cyclohexyl, imidazolyl, oxadiazolyl, oxetanyl, phenyl, pyrazinyl, pyrazolyl, pyridazinyl, pyridinyl, pyrimidinyl, pyrrolidinyl, thiazinyl, thiazolyl and triazolyl, more preferably pyrazolyl.

26. A compound according to any one of embodiments 1 to 25, wherein $R^8$ is an 8-to 10- membered heteroaryl ring containing one to three nitrogen atoms, the group $R^8$ being unsubstituted or substituted by one, two or three substituents as defined in claim 25.

27. A compound according to any one of embodiments 1 to 26, wherein $R^8$ is indazolyl which is unsubstituted or substituted by one, two or three substituents as defined in claim 25.

28. A compound according to embodiment 27, wherein $R^8$ is indazolyl which is unsubstituted or substituted by one or two $C_{1-4}$ alkyl.

29. A compound according to embodiment 28, wherein $R^8$ is indazolyl which is unsubstituted or substituted by one or two methyl.

29. A compound according to embodiment 30, wherein $R^8$ is selected from one of the following structures:

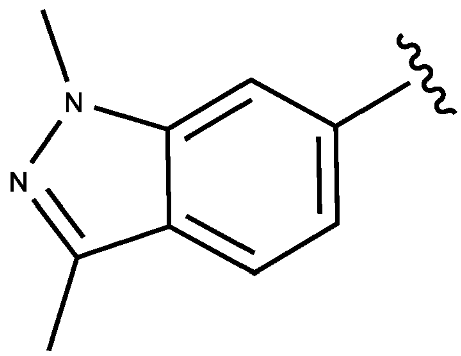

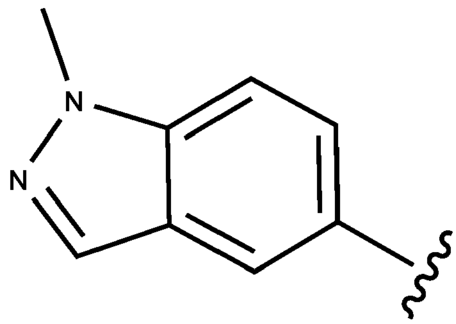

.

30. A compound according to embodiment 1 which is selected from:

N- f 3-chloro-2-[(2R)-1-methylpyrrolidin-2-yl]-1H-pyrrolo[3,2-c]pyridin-6-yl}-1,3-dimethylindazole-6-carboxamide;
N-{3-chloro-2-[(2S)-1-methylpyrrolidin-2-yl]-1H-pyrrolo[3,2-c]pyridin-6-yl}-1,3-dimethylindazole-6-carboxamide;
(R)-1,3-dimethyl-N-(2-(1-methylpyrrolidin-2-yl)-3-(trifluoromethyl)-1H-pyrrolo[3,2-c]pyridin-6-yl)-1H-indazole-6-carboxamide;
(S)-1,3-dimethyl-N-(2-(1-methylpyrrolidin-2-yl)-3-(trifluoromethyl)-1H-pyrrolo[3,2-c]pyridin-6-yl)-1H-indazole-6-carboxamide;
N-{3-fluoro-2-[(rel-2S)-1-methylpyrrolidin-2-yl]-1H-pyrrolo[3,2-c]pyridin-6-yl}-1-methylindazole-5-carboxamide;
N-{3-fluoro-2-[(rel-2R)-1-methylpyrrolidin-2-yl]-1H-pyrrolo[3,2-c]pyridin-6-yl}-1-methylindazole-5-carboxamide;
N-{3-fluoro-2-[(rel-2R)-1-methylpyrrolidin-2-yl]-1H-pyrrolo[3,2-c]pyridin-6-yl}-1,3-dimethylindazole-6-carboxamide;
N-{3-fluoro-2-[(rel-2S)-1-methylpyrrolidin-2-yl]-1H-pyrrolo[3,2-c]pyridin-6-yl}-1,3-dimethylindazole-6-carboxamide;
1,3-dimethyl-N-{3-methyl-2-[(rel-2R)-1-methylpyrrolidin-2-yl]-1H-pyrrolo[3,2-c]pyridin-6-yl}indazole-6-carboxamide;
1,3-dimethyl-N-{3-methyl-2-[(rel-2S)-1-methylpyrrolidin-2-yl]-1H-pyrrolo[3,2-c]pyridin-6-yl}indazole-6-carboxamide;
1,3-dimethyl-N-{4-methyl-2-[(rel-2R)-1-methylpyrrolidin-2-yl]-1H-pyrrolo[3,2-c]pyridin-6-yl}indazole-6-carboxamide;

1,3-dimethyl-N-{4-methyl-2-[(rel-2S)-1-methylpyrrolidin-2-yl]-1H-pyrrolo[3,2-c]pyridin-6-yl}indazole-6-carboxamide; and
(R)-N-(3-cyano-2-(1-methylpyrrolidin-2-yl)-1H-pyrrolo[3,2-c]pyridin-6-yl)-1,3-dimethyl-1H-indazole-6-carboxamide;
and the pharmaceutically acceptable salts thereof.

31. A pharmaceutical composition which comprises a compound as defined in any one of embodiments 1 to 30 and a pharmaceutically acceptable carrier or diluent.

32. A compound as defined in any one of embodiments 1 to 30 for use in the treatment of the human or animal body by therapy.

33. A compound as defined in any one of embodiments 1 to 30 for use in the treatment of cancer.

34. The compound for use according to embodiment 33, wherein the cancer is a transcriptionally addicted cancer, such as breast cancer, acute leukemia, prostate cancer, bladder cancer, cholangiocarcinoma, colon adenocarcinoma, esophageal carcinoma, head and neck squamous cell carcinoma, kidney chromophobe, kidney renal clear cell carcinoma, kidney renal papillary cell carcinoma, liver hepatocellular carcinoma, lung adenocarcinoma, pheochromocytoma and paraganglioma, rectum adenocarcinoma, stomach adenocarcinoma, uterine corpus endometrial carcinoma, cervical squamous cell carcinoma and endocervical adenocarcinoma, lung squamous cell carcinoma, neuroblastoma, midline glioma or sarcoma, preferably neuroblastoma or midline glioma.

35. The compound for use according to embodiment 33, wherein the cancer is acute myeloid leukaemia (AML), myelodysplastic syndrome (MDS), myeloproliferative neoplasm (MPN) or acute lymphoblastic leukaemia (ALL).

36. The compound for use according to embodiment 35, wherein the cancer is mixed-lineage leukaemia (MLL) or rearranged acute leukemia (AML and ALL).

37. The compound for use according to embodiment 35, wherein the cancer is NPM1 mutant leukemia.

## Claims

1. A compound which is a Proteolysis Targeting Chimera (PROTAC) or a pharmaceutically acceptable salt thereof, wherein the PROTAC has the structure:

**M-LINK-U**

wherein U is an E3 ubiquitin ligase binding moiety, LINK is a moiety that covalently links M and U, and M is an MLLT1 and/or MLLT3 binder of formula (I):

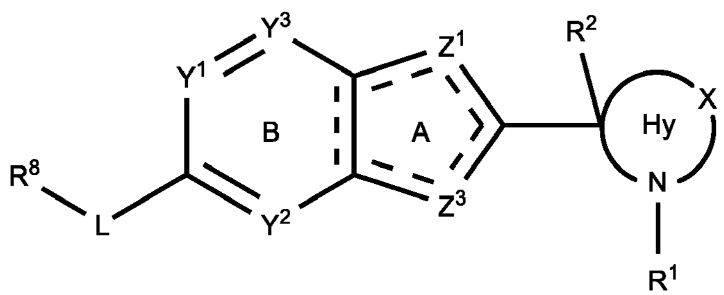

(I)

wherein:
one of $Z^1$ and $Z^3$ is -N(H)- and the other is N or -C($R^4$)-, $Y^1$ is N, $Y^2$ is N or -C($R^6$)-, and $Y^3$ is N or -C($R^5$)-;
Hy is a 4- to 7-membered heterocyclic ring containing X and at least one N atom, wherein: ring Hy is linked to ring A

via a C atom within ring Hy, said C atom also being linked to $R^2$; a N atom within ring Hy is substituted by $R^1$; X is a bond, $-N(R^{11})-$, O, S, $-S(O)_2-$, $-S(O)(NR^{11})-$, or $-C(R^{11})_2-$; and the rest of ring Hy is unsubstituted or substituted by one or two $R^3$;

L is -C(O)N(H)-, wherein the C atom of L is bonded to $R^8$, and the N atom of L is bonded to ring B;

$R^1$ is H, $C_{1-4}$ cycloalkyl, or $C_{1-4}$ alkyl which is itself unsubstituted or substituted with one $C_{1-4}$ alkoxy or one, two or three halo;

$R^2$ is H or methyl;

each $R^3$ is independently selected from $C_{1-4}$ alkyl, $C_{1-4}$ alkoxy, phenyl, a 5- to 6-membered heteroaryl ring and halo, or (i) two $R^3$ linked to adjacent C atoms in ring Hy form, together with the C atoms to which they are attached, a $C_{5-6}$ cycloalkyl ring, or (ii) two $R^3$ linked to the same C atom in ring Hy form, together with the C atom to which they are attached, a $C_{3-6}$ cycloalkyl ring or a $C_{3-6}$ heterocycloalkyl ring;

$R^4$ and $R^6$ are independently selected from H, halo, CN, $C_{1-4}$ alkoxy, and $C_{1-4}$ alkyl which is itself unsubstituted or substituted by one, two or three halo;

$R^5$ is selected from H, halo, $C_{1-4}$ alkoxy, $C_{3-5}$ cycloalkyl and $C_{1-4}$ alkyl which is itself unsubstituted or substituted by one, two or three halo;

$R^8$ is an 8- to 10-membered heteroaryl ring, the group $R^8$ being unsubstituted or substituted by one, two or three substituents independently selected from halo, $C_{1-4}$ alkoxy, $R^{10}$, $-(C_{1-4}$ alkylene$)-R^{10}$, =O, -CN, and $C_{1-4}$ alkyl which is itself unsubstituted or substituted by one or two $R^9$;

each $R^9$ is independently selected from halo and $C_{1-4}$ alkoxy;

$R^{10}$ is a group selected from a 5- to 6-membered heteroaryl ring, a 3- to 6-membered cycloalkyl ring, a 4- to 6-membered heterocyclyl ring, and a phenyl ring, the group $R^{10}$ being unsubstituted or substituted by one or two substituents independently selected from $C_{1-4}$ alkyl, $C_{1-4}$ alkoxy, and halo; and

each $R^{11}$ is independently selected from H, $C_{1-4}$ alkyl, and $C_{1-4}$ cycloalkyl;

wherein M is bonded to LINK via a C or N atom within group $R^8$ such that a hydrogen atom on the C or N atom within group $R^8$ is replaced with a bond to LINK.

**2.** A compound according to claim 1, wherein LINK is (a) a single bond, or (b) a chemical linker group represented by formula (L):

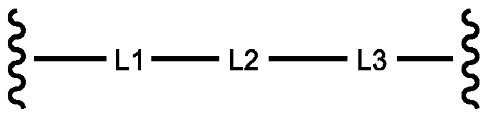

(L)

wherein:

LINK is attached to M via L1;

LINK is attached to U via L3;

L1 and L3 are each independently selected from a single bond, -N(R')-, -C(O)N(R')-, -O-, -N(R')C(O)-, -C(O)-, $-S(O_2)N(R')-$, $-N(R')S(O_2)-$, $-(C_{1-6}$ alkylene$)-$, ethynyl, $-(C_{2-6}$ alkenylene$)-$, a divalent 3- to 6-membered cycloalkyl ring and a divalent 4- to 7-membered heterocyclyl ring;

L2 is represented by the formula $-(L4)_m-$;

each L4 is independently selected from a divalent 3- to 6-membered cycloalkyl ring, a divalent 4- to 7-membered heterocyclyl ring and a unit of formula:

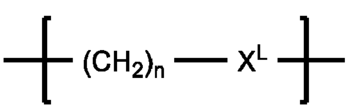 or 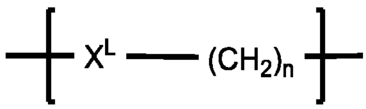 ;

each $X^L$ is independently selected from a single bond, -N(R')-, -O-, -C(O)-, -S-, -SO-, $- SO_2-$, -C(R")=C(R")- and -C(H)≡C(H)-;

n is selected from 1 to 4;

m is selected from 1 to 30;

each R' is independently selected from H and $C_{1-4}$ alkyl; and

each R" is independently selected from H and halo.

**3.** A compound according to claim 1 or 2, wherein U is:

(a) a CRBN E3 ubiquitin ligase binding moiety of formula (U1):

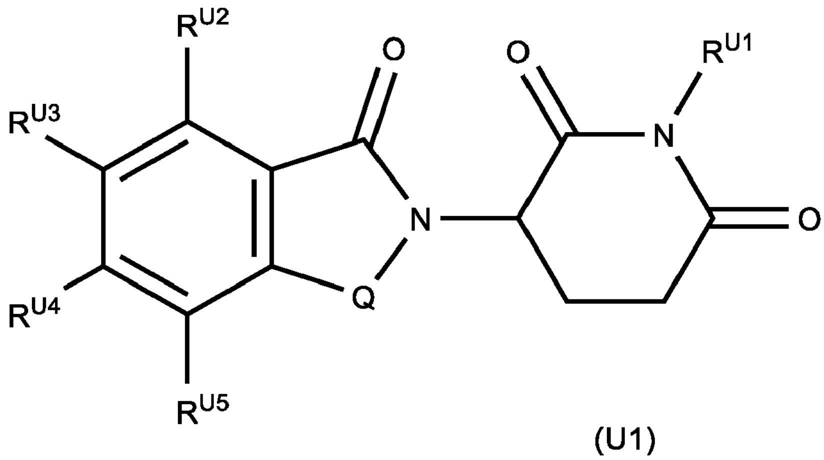

(U1)

wherein:

$R^{U1}$ is H;
Q is selected from -CH($R^{U6}$)-, -N($R^{U6}$)-, -O-, -C(O)-, -NH-CH($R^{U6}$)-, -N=C($R^{U6}$)-, or -N=N-;
$R^{U6}$ is H or $C_{1-4}$ alkyl;
$R^{U2}$ and $R^{U5}$ are each independently selected from H, halogen, $C_{1-4}$ alkyl, $NH_2$, $NO_2$, OH, COOH, CN, $CF_3$, and a single bond to LINK;
$R^{U3}$ and $R^{U4}$ are each independently selected from H, halogen, $C_{1-4}$ alkyl, $NH_2$, $NO_2$, OH, COOH, CN, $CF_3$, and a single bond to LINK;
wherein one and only one of $R^{U2}$, $R^{U3}$, $R^{U4}$ and $R^{U5}$ is a single bond to LINK;

(b) a CRBN E3 ubiquitin ligase binding moiety of formula (U4):

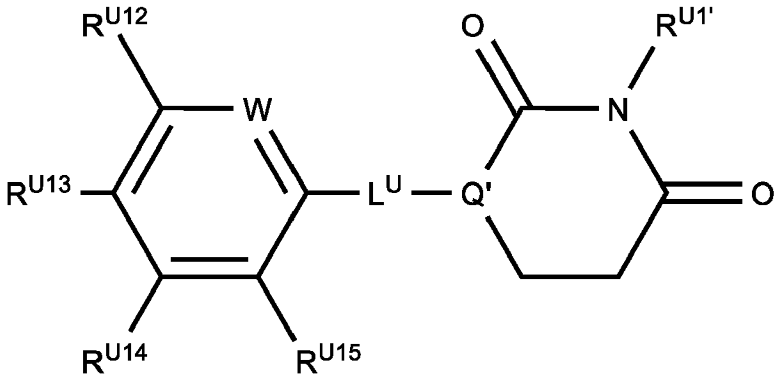

(U4)

wherein:

$R^{U1'}$ is H;
W is Nor $CR^{U16}$;
Q' is N and $L^U$ is a single bond, or Q' is CH and $L^U$ is selected from a single bond or -C(O)N(H)-, wherein either (i) the C atom of $L^U$ is bonded to phenyl, and the N atom of $L^U$ is bonded to Q'; or (ii) the C atom of $L^U$ is bonded to Q', and the N atom of $L^U$ is bonded to phenyl;
$R^{U12}$, $R^{U13}$ and $R^{U14}$ are each independently selected from H, halogen, $C_{1-4}$ alkyl, $NH_2$, $NO_2$, OH, COOH, CN, $CF_3$, and a single bond to LINK;
wherein one and only one of $R^{U12}$, $R^{U13}$ and $R^{U14}$ is a single bond to LINK;

$R^{U15}$ and $R^{U16}$ are each independently selected from H, halogen, $C_{1-4}$ alkyl, $NH_2$, $NO_2$, OH, COOH, CN and $CF_3$;

(c) a CRBN E3 ubiquitin ligase binding moiety of formula (U5):

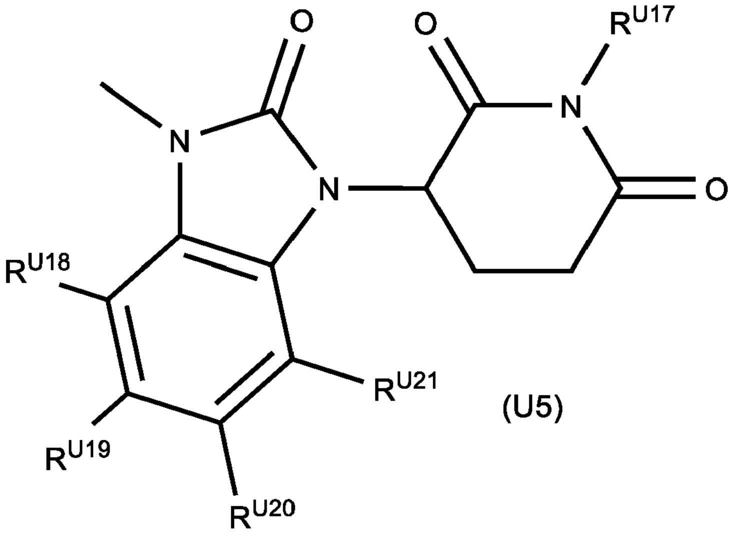

(U5)

wherein:

$R^{U17}$ is H;
$R^{U18}$, $R^{U19}$, $R^{U20}$ and $R^{U21}$ are each independently selected from H, halogen, $C_{1-4}$ alkyl, $NH_2$, $NO_2$, OH, COOH, CN, $CF_3$, and a single bond to LINK;
wherein one and only one of $R^{U18}$, $R^{U19}$, $R^{U20}$ and $R^{U21}$ is a single bond to LINK;
or (d) a VHL E3 ubiquitin ligase binding moiety of formula (U2):

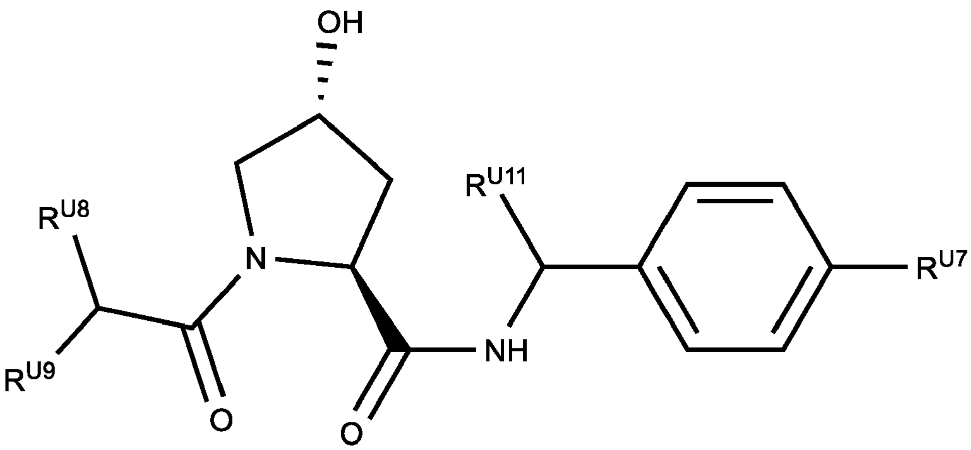

(U2)

wherein:

$R^{U7}$ is a group selected from phenyl, a 5- to 6-membered heteroaryl ring, a 5- to 6-membered heterocyclyl ring, and a 5- to 6-membered cycloalkyl ring, the group $R^{U7}$ being unsubstituted or substituted by $C_{1-4}$ alkyl;
$R^{U11}$ is H or $C_{1-4}$ alkyl;
$R^{U8}$ is selected from $C_{1-4}$ alkyl, phenyl and a 3- to 8-membered cycloalkyl ring; and
$R^{U9}$ is a single bond to LINK.

**4.** A compound according to any one of the preceding claims, wherein the N atom of ring Hy which is substituted by $R^1$ is

adjacent to the C atom of ring Hy that is bonded to ring A.

**5.** A compound according to any one of the preceding claims, wherein $Z^1$ is - $C(R^4)$-, $Z^3$ is -N(H)-, $Y^1$ is N, $Y^2$ is -$C(R^6)$- and $Y^3$ is -$C(R^5)$-.

**6.** A compound according to any one of the preceding claims, wherein M is of formula (II):

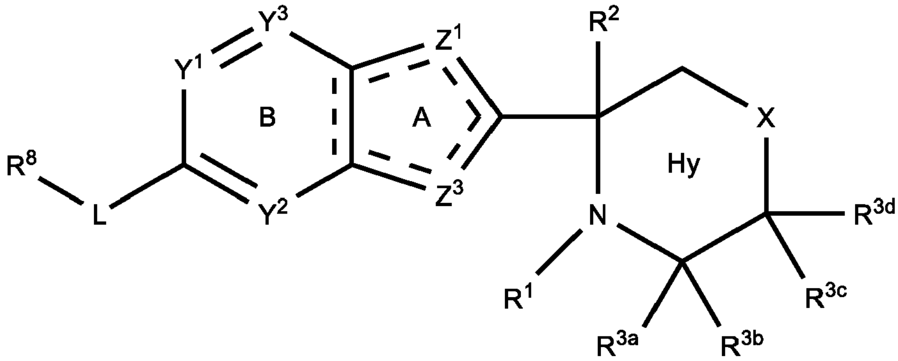

(II)

wherein:

$Z^1$, $Z^3$, $Y^1$, $Y^2$, $Y^3$, $R^1$, $R^2$, $R^8$, X and L are as defined in any one of the preceding claims;
$R^{3a}$ and $R^{3b}$ are independently selected from H, $C_{1-4}$ alkyl, phenyl, a 5- to 6-membered heteroaryl ring and $C_{1-4}$ alkoxy, or $R^{3a}$ and $R^{3b}$ form, together with the C atom to which they are attached, a $C_{3-6}$ cycloalkyl ring or a $C_{3-6}$ heterocycloalkyl ring,
$R^{3c}$ and $R^{3d}$ are independently selected from H, $C_{1-4}$ alkyl, halo, phenyl, a 5- to 6-membered heteroaryl ring and $C_{1-4}$ alkoxy, or $R^{3c}$ and $R^{3d}$ form, together with the C atom to which they are attached, a $C_{3-6}$ cycloalkyl ring or a $C_{3-6}$ heterocycloalkyl ring,
or
$R^{3a}$ and $R^{3c}$ are H, and $R^{3b}$ and $R^{3d}$ form, together with the C atoms to which they are attached, a $C_{5-6}$ cycloalkyl ring, with the proviso that when X is -N($R^{11}$)-, O, S, -$S(O)_2$- or -S(O)($NR^{11}$)-, then neither $R^{3c}$ nor $R^{3d}$ are halo;
wherein at least two of $R^{3a}$, $R^{3b}$, $R^{3c}$ and $R^{3d}$ are H; and
wherein M is bonded to LINK via a C or N atom within group $R^8$ such that a hydrogen atom on the C or N atom within group $R^8$ is replaced with a bond to LINK.

**7.** A compound according to any one of the preceding claims, wherein M is of formula (III):

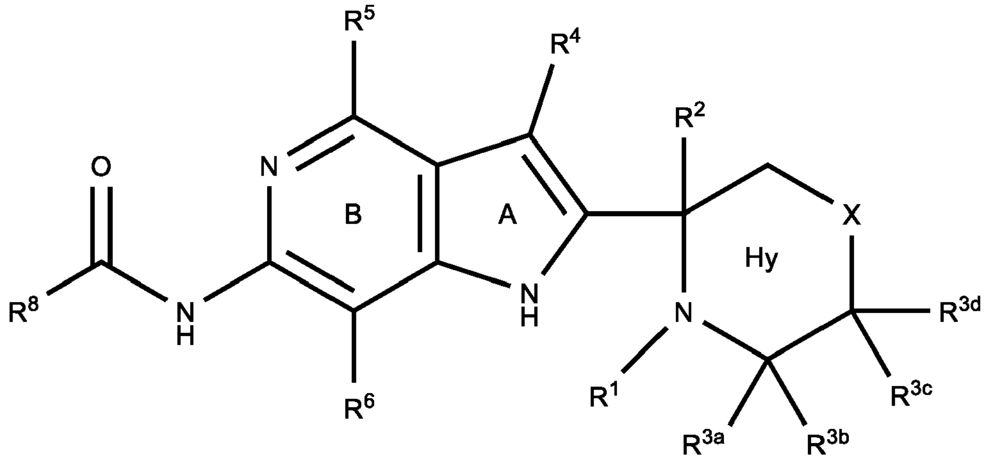

(III)

wherein:

$R^1$ is H or $C_{1-4}$ alkyl which is itself unsubstituted or substituted with one $C_{1-4}$ alkoxy or one, two or three halo, preferably with one $C_{1-4}$ alkoxy;
$R^2$ is H or methyl;
$R^{3a}$ and $R^{3b}$ are independently selected from H, $C_{1-4}$ alkyl, and $C_{1-4}$ alkoxy, or $R^{3a}$ and $R^{3b}$ form, together with the C atom to which they are attached, a $C_{3-6}$ cycloalkyl ring,
$R^{3c}$ and $R^{3d}$ are independently selected from H, $C_{1-4}$ alkyl, halo and $C_{1-4}$ alkoxy, or $R^{3c}$ and $R^{3d}$ form, together with the C atom to which they are attached, a $C_{3-6}$ cycloalkyl ring, or
$R^{3a}$ and $R^{3c}$ are H, and $R^{3b}$ and $R^{3d}$ form, together with the C atoms to which they are attached, a $C_{5-6}$ cycloalkyl ring,
with the proviso that when X is -N(Me)- or O, then neither $R^{3c}$ nor $R^{3d}$ are halo;
wherein at least two of $R^{3a}$, $R^{3b}$, $R^{3c}$ and $R^{3d}$ are H;
X is a bond, -N(Me)-, O or $-CH_2-$;
$R^4$ is selected from H, CN, halo, $C_{1-4}$ alkoxy, and $C_{1-4}$ alkyl which is itself unsubstituted or substituted by one, two or three halo;
$R^5$ and $R^6$ are independently selected from H, halo, $C_{1-4}$ alkoxy, and $C_{1-4}$ alkyl which is itself unsubstituted or substituted by one, two or three halo;
$R^8$ is an 8- to 10-membered heteroaryl ring, the group $R^8$ being unsubstituted or substituted by one, two or three substituents independently selected from halo, $C_{1-4}$ alkoxy, $R^{10}$, $-(C_{1-4}$ alkylene)-$R^{10}$, =O, -CN, and $C_{1-4}$ alkyl which is itself unsubstituted or substituted by one or two $R^9$;
each $R^9$ is independently selected from halo and $C_{1-4}$ alkoxy;
$R^{10}$ is a group selected from a 5- to 6-membered heteroaryl ring, a 3- to 6-membered cycloalkyl ring, a 4- to 6-membered heterocyclyl ring, and a phenyl ring, the group $R^{10}$ being unsubstituted or substituted by one or two substituents independently selected from $C_{1-4}$ alkyl, $C_{1-4}$ alkoxy, and halo; and
wherein M is bonded to LINK via a C or N atom within group $R^8$ such that a hydrogen atom on the C or N atom within group $R^8$ is replaced with a bond to LINK.

**8.** A compound according to claim 1, wherein the PROTAC has a structure selected from:

1-(9-(2-(2,6-dioxopiperidin-3-yl)-1-oxoisoindolin-4-yl)non-8-yn-1-yl)-3-methyl-*N*-(2-((*R*)-1-methylpyrrolidin-2-yl)-1*H*-pyrrolo[3,2-c]pyridin-6-yl)-1*H*-indazole-6-carboxamide;
rac-1-(8-{[2-(2,6-dioxopiperidin-3-yl)-1,3-dioxoisoindol-4-yl]amino}octyl)-3-methyl-N-[2-(1-methylpyrrolidin-2-yl)-1H-pyrrolo[3,2-c]pyridin-6-yl]indazole-6-carboxamide;
1-(8-{[2-(2,6-dioxopiperidin-3-yl)-1,3-dioxoisoindol-4-yl]amino}octyl)-3-methyl-N-{2-[(2R)-1-methylpyrrolidin-2-yl]-1H-pyrrolo[3,2-c]pyridin-6-yl}indazole-6-carboxamide;
1-(8-{[2-(2,6-dioxopiperidin-3-yl)-1,3-dioxoisoindol-4-yl]amino}octyl)-3-methyl-N-{2-[(2S)-1-methylpyrrolidin-2-yl]-1H-pyrrolo[3,2-c]pyridin-6-yl}indazole-6-carboxamide;
2-(8-((2-(2,6-dioxopiperidin-3-yl)-1,3-dioxoisoindolin-4-yl)amino)octyl)-3-methyl-N-(2-((R)-1-methylpyrrolidin-2-yl)-1H-pyrrolo[3,2-c]pyridin-6-yl)-2H-indazole-6-carboxamide;
3-[4-({4-[2-(2,6-dioxopiperidin-3-yl)-6-fluoro-1,3-dioxoisoindol-5-yl]piperazin-1-yl}methyl)piperidin-1-yl]-1-methyl-N-{2-[(2R)-1-methylpyrrolidin-2-yl]-1H-pyrrolo[3,2-c]pyridin-6-yl}indazole-5-carboxamide;
1-[1-(3-{[2-(2,6-dioxopiperidin-3-yl)-1,3-dioxoisoindol-4-yl]amino}propyl)piperidin-4-yl]-3-methyl-N-{2-[(2R)-1-methylpyrrolidin-2-yl]-1H-pyrrolo[3,2-c]pyridin-6-yl}indazole-6-carboxamide;
1-(8-{[2-(2,6-dioxopiperidin-3-yl)-1,3-dioxoisoindol-5-yl]oxy}octyl)-3-methyl-N-{2-[(2R)-1-methylpyrrolidin-2-yl]-1H-pyrrolo[3,2-c]pyridin-6-yl}indazole-6-carboxamide;
1-{8-[3-(2,4-dioxo-1,3-diazinan-1-yl)-2-methylphenoxy]octyl}-3-methyl-N-{2-[(2R)-1-methylpyrrolidin-2-yl]-1H-pyrrolo[3,2-c]pyridin-6-yl}indazole-6-carboxamide;
1-{6-[3-(2,4-dioxo-1,3-diazinan-1-yl)-2-methylphenoxy]hexyl}-3-methyl-N-{2-[(2R)-1-methylpyrrolidin-2-yl]-1H-pyrrolo[3,2-c]pyridin-6-yl}indazole-6-carboxamide;
1-{7-[1-(2,6-dioxopiperidin-3-yl)-3-methyl-2-oxo-1,3-benzodiazol-4-yl]hept-6-yn-1-yl}-3-methyl-N- f 2-[(2R)-1-methylpyrrolidin-2-yl]-1H-pyrrolo[3,2-c]pyridin-6-yl}indazole-6-carboxamide;
1-(8-((2-(2,6-dioxopiperidin-3-yl)-1,3-dioxoisoindolin-4-yl)oxy)octyl)-3-methyl-N-(2-((R)-1-methylpyrrolidin-2-yl)-1H-pyrrolo[3,2-c]pyridin-6-yl)-1H-indazole-6-carboxamide;
1-(3-((2-(2,6-dioxopiperidin-3-yl)-1,3-dioxoisoindolin-4-yl)amino)propyl)-3-methyl-N-(2-((R)-1-methylpyrrolidin-2-yl)-1H-pyrrolo[3,2-c]pyridin-6-yl)-1H-indazole-6-carboxamide;
1-(8-{[2-(2,6-dioxopiperidin-3-yl)-1,3-dioxoisoindol-5-yl]amino}octyl)-3-methyl-N-{2-[(2R)-1-methylpyrrolidin-2-yl]-1H-pyrrolo[3,2-c]pyridin-6-yl}indazole-6-carboxamide;
1-(8-{[2-(2,6-dioxopiperidin-3-yl)-1,3-dioxoisoindol-5-yl]amino}octyl)-3-methyl-N-{2-[(2S)-1-methylpyrrolidin-2-yl]-1H-pyrrolo[3,2-c]pyridin-6-yl}indazole-6-carboxamide;
1-(8-{[2-(2,6-dioxopiperidin-3-yl)-1-oxo-3H-isoindol-4-yl]amino}octyl)-3-methyl-N-{2-[(2R)-1-methylpyrrolidin-2-yl]-1H-pyrrolo[3,2-c]pyridin-6-yl}indazole-6-carboxamide;
1-(8-{[2-(2,6-dioxopiperidin-3-yl)-1-oxo-3H-isoindol-4-yl]amino}octyl)-3-methyl-N-{2-[(2S)-1-methylpyrrolidin-2-yl]-1H-pyrrolo[3,2-c]pyridin-6-yl}indazole-6-carboxamide;
N-(3-chloro-2-((R)-1-methylpyrrolidin-2-yl)-1H-pyrrolo[3,2-c]pyridin-6-yl)-1-(8-((2-(2,6-dioxopiperidin-3-yl)-1,3-dioxoisoindolin-4-yl)amino)octyl)-3-methyl-1H-indazole-6-carboxamide;
1-(4-{4-[2-(2,6-dioxopiperidin-3-yl)-1,3-dioxoisoindol-5-yl]piperazin-1-yl}butyl)-3-methyl-N-{2-[(2R)-1-methylpyrrolidin-2-yl]-1H-pyrrolo[3,2-c]pyridin-6-yl}indazole-6-carboxamide;
1-(4-{4-[2-(2,6-dioxopiperidin-3-yl)-1,3-dioxoisoindol-5-yl]piperazin-1-yl}butyl)-3-methyl-N-{2-[(2S)-1-methylpyrrolidin-2-yl]-1H-pyrrolo[3,2-c]pyridin-6-yl}indazole-6-carboxamide;
1-(4-((2-(2,6-dioxopiperidin-3-yl)-1,3-dioxoisoindolin-4-yl)amino)butyl)-3-methyl-N-(2-((R)-1-methylpyrrolidin-2-yl)-1H-pyrrolo[3,2-c]pyridin-6-yl)-1H-indazole-6-carboxamide;
1-(6- f [2-(2,6-dioxopiperidin-3-yl)-1,3-dioxoisoindol-4-yl]amino}hexyl)-3-methyl-N-{2-[(2R)-1-methylpyrrolidin-2-yl]-1H-pyrrolo[3,2-c]pyridin-6-yl}indazole-6-carboxamide;
1-(5-(4-(2-(2,6-dioxopiperidin-3-yl)-6-fluoro-1,3-dioxoisoindolin-5-yl)piperazin-1-yl)pentyl)-3-methyl-N-(2-((R)-1-methylpyrrolidin-2-yl)-1H-pyrrolo[3,2-c]pyridin-6-yl)-1H-indazole-6-carboxamide;
1-(2-(2-(4-(2-(2,6-dioxopiperidin-3-yl)-6-fluoro-1,3-dioxoisoindolin-5-yl)piperazin-1-yl)ethoxy)ethyl)-3-methyl-*N*-(2-((*R*)-1-methylpyrrolidin-2-yl)-1*H*-pyrrolo[3,2-c]pyridin-6-yl)-1*H*-indazole-6-carboxamide;
1-(2-(2-(2-((2-(2,6-dioxopiperidin-3-yl)-1,3-dioxoisoindolin-4-yl)amino)ethoxy)ethoxy)ethyl)-3-methyl-*N*-(2-((R)-1-methylpyrrolidin-2-yl)-1*H*-pyrrolo[3,2-c]pyridin-6-yl)-1*H*-indazole-6-carboxamide;
1-(2-(2-((2-(2,6-dioxopiperidin-3-yl)-1,3-dioxoisoindolin-4yl)amino)ethoxy)ethyl)-3-methyl-*N*-(2-((*R*)-1-methylpyrrolidin-2-yl)-1*H*-pyrrolo[3,2-c]pyridin-6-yl)-1*H*-indazole-6-carboxamide;
1-(3-(1-(2-(2,6-dioxopiperidin-3-yl)-1,3-dioxoisoindolin-4-yl)piperidin-4-yl)propyl)-3-methyl-*N*-(2-((*R*)-1-methylpyrrolidin-2-yl)-1*H*-pyrrolo[3,2-c]pyridin-6-yl)-1*H*-indazole-6-carboxamide;
1-(3-(1-(4-((2,6-dioxopiperidin-3-yl)carbamoyl)-2-fluorophenyl)piperidin-4-yl)propyl)-3-methyl-N-(2-((*R*)-1-methylpyrrolidin-2-yl)-1H-pyrrolo[3,2-c]pyridin-6-yl)-1H-indazole-6-carboxamide;
1-(5-(1-(2-(2,6-dioxopiperidin-3-yl)-1,3-dioxoisoindolin-4-yl)piperidin-4-yl)pentyl)-3-methyl-*N*-(2-((R)-1-methylpyrrolidin-2-yl)-1*H*-pyrrolo[3,2-c]pyridin-6-yl)-1*H*-indazole-6-carboxamide;
1-(7-(2-(2,6-dioxopiperidin-3-yl)-1-oxoisoindolin-4-yl)hept-6-yn-1-yl)-3-methyl-*N*-(2-((*R*)-1-methylpyrrolidin-2-yl)-1*H*-pyrrolo [3, 2-c] pyridin-6-yl)-1*H*-indazole-6-carboxamide;
1-(3-((3-(2-(2,6-dioxopiperidin-3-yl)-1-oxoisoindolin-4-yl)prop-2-yn-1-yl)oxy)propyl)-3-methyl-*N*-(2-((*R*)-1-methylpyrrolidin-2-yl)-1*H*-pyrrolo[3,2-c]pyridin-6-yl)-1*H*-indazole-6-carboxamide;
1-(5-(2-(2,6-dioxopiperidin-3-yl)-1-oxoisoindolin-4-yl)pent-4-yn-1-yl)-3-methyl-*N*-(2-((*R*)-1-methylpyrrolidin-2-yl)-1*H*-pyrrolo[3,2-c]pyridin-6-yl)-1*H*-indazole-6-carboxamide;
1-(7-(2-(2,6-dioxopiperidin-3-yl)-1,3-dioxoisoindolin-4-yl)hept-6-yn-1-yl)-3-methyl-*N*-(2-((*R*)-1-methylpyrrolidin-2-yl)-1*H*-pyrrolo[3,2-c]pyridin-6-yl)-1*H*-indazole-6-carboxamide;
1-(7-(3-((2,6-dioxopiperidin-3-yl)carbamoyl)-2-fluorophenyl)hept-6-yn-1-yl)-3-methyl-*N*-(2-((*R*)-1-methylpyrrolidin-2-yl)-1*H*-pyrrolo[3,2-c]pyridin-6-yl)-1*H*-indazole-6-carboxamide;
1-(7-(3-((2,6-dioxopiperidin-3-yl)carbamoyl)-2-methylphenyl)hept-6-yn-1-yl)-3-methyl-*N*-(2-((*R*)-1-methylpyrrolidin-2-yl)-1*H*-pyrrolo[3,2-c]pyridin-6-yl)-1*H*-indazole-6-carboxamide;
1-(8-((2-(2,6-dioxopiperidin-3-yl)-1,3-dioxoisoindolin-4-yl)amino)octyl)-*N*-(2-((*R*)-1-methylpyrrolidin-2-yl)-1*H*-pyrrolo[3,2-c]pyridin-6-yl)-1*H*-indazole-5-carboxamide;
1-(8-((2-(2,6-dioxopiperidin-3-yl)-1,3-dioxoisoindolin-4-yl)amino)octyl)-N-(2-((S)-1-methylpyrrolidin-2-yl)-1H-pyrrolo[3,2-c]pyridin-6-yl)-1H-indazole-5-carboxamide;
1-(9-(2-(2,6-dioxopiperidin-3-yl)-1-oxoisoindolin-4-yl)non-8-yn-1-yl)-*N*-(2-((*R*)-1-methylpyrrolidin-2-yl)-1*H*-pyrrolo[3, 2-*c*]pyridin-6-yl)-1*H*-indazole-5-carboxamide;
1-(9-(1-(2,6-dioxopiperidin-3-yl)-3-methyl-2-oxo-2,3-dihydro-1*H*-benzo[d]imidazol-5-yl)non-8-yn-1-yl)-3-methyl-*N*-(2-((R)-1-methylpyrrolidin-2-yl)-1*H*-pyrrolo[3,2-c]pyridin-6-yl)-1*H*-indazole-6-carboxamide;
1-((E)-9-chloro-9-(1-(2,6-dioxopiperidin-3-yl)-3-methyl-2-oxo-2,3-dihydro-1*H*-benzo[d]imidazol-5-yl)non-8-en-1-yl)-3-methyl-*N*-(2-((*R*)-1-methylpyrrolidin-2-yl)-1*H*-pyrrolo[3,2-*c*]pyridin-6-yl)-1*H*-indazole-6-carboxamide;
1-((Z)-9-chloro-9-(1-(2,6-dioxopiperidin-3-yl)-3-methyl-2-oxo-2,3-dihydro-1*H*-benzo[d]imidazol-5-yl)non-8-en-1-yl)-3-methyl-*N*-(2-((*R*)-1-methylpyrrolidin-2-yl)-1*H*-pyrrolo[3,2-*c*]pyridin-6-yl)-1*H*-indazole-6-carboxamide;
1-(9-chloro-9-(1-(2,6-dioxopiperidin-3-yl)-3-methyl-2-oxo-2,3-dihydro-1*H*-benzo[d]imidazol-5-yl)non-8-en-1-yl)-3-methyl-*N*-(2-((*R*)-1-methylpyrrolidin-2-yl)-1*H*-pyrrolo[3,2-*c*]pyridin-6-yl)-1*H*-indazole-6-carboxamide;
1-(9-(6-(((*S*)-2,6-dioxopiperidin-3-yl)carbamoyl)pyridin-3-yl)non-8-yn-1-yl)-3-methyl-N-(2-((*R*)-1-methylpyrrolidin-2-yl)-1H-pyrrolo[3,2-c]pyridin-6-yl)-1H-indazole-6-carboxamide;
1-(9-(6-(((*S*)-2,6-dioxopiperidin-3-yl)carbamoyl)pyridin-2-yl)non-8-yn-1-yl)-3-methyl-N-(2-((*R*)-1-methylpyrrolidin-2-yl)-1H-pyrrolo[3,2-c]pyridin-6-yl)-1H-indazole-6-carboxamide;
1-(9-(2-(((*S*)-2,6-dioxopiperidin-3-yl)carbamoyl)pyridin-4-yl)non-8-yn-1-yl)-3-methyl-N-(2-((*R*)-1-methylpyrrolidin-2-yl)-1H-pyrrolo[3,2-c]pyridin-6-yl)-1H-indazole-6-carboxamide;
1-(9-(2-(2,6-dioxopiperidin-3-yl)-1-oxoisoindolin-4-yl)non-8-yn-1-yl)-3-methyl-*N*-(2-((R)-1-(methyl-d3)pyrrolidin-2-yl)-1*H*-pyrrolo[3,2-c]pyridin-6-yl)-1*H*-indazole-6-carboxamide;
1-(8-(2-(2,6-dioxopiperidin-3-yl)-1-oxoisoindolin-4-yl)oct-7-yn-1-yl)-3-methyl-*N*-(2-((R)-1-methylpyrrolidin-2-yl)-1*H*-pyrrolo[3,2-c]pyridin-6-yl)-1*H*-indazole-6-carboxamide;
1-(6-(2-(2,6-dioxopiperidin-3-yl)-1-oxoisoindolin-4-yl)hex-5-yn-1-yl)-3-methyl-*N*-(2-((*R*)-1-methylpyrrolidin-2-yl)-1*H*-pyrrolo[3,2-c]pyridin-6-yl)-1*H*-indazole-6-carboxamide;
1-(5-(4-(2-(2,6-dioxopiperidin-3-yl)-1,3-dioxoisoindolin-4-yl)piperazin-1-yl)pentyl)-3-methyl-*N*-(2-((*R*)-1-methylpyrrolidin-2-yl)-1*H*-pyrrolo[3,2-c]pyridin-6-yl)-1*H*-indazole-6-carboxamide;
1-(9-(2-(2,6-dioxopiperidin-3-yl)-1-oxoisoindolin-4-yl)non-8-yn-1-yl)-*N*-(2-((R)-1 - ethylpyrrolidin-2-yl)-1*H*-pyrrolo[3,2-c]pyridin-6-yl)-3-methyl-1*H*-indazole-6-carboxamide;
1-(5-((3-(2-(2,6-dioxopiperidin-3-yl)-1-oxoisoindolin-4-yl)prop-2-yn-1-yl)oxy)pentyl)-3-methyl-*N*-(2-((R)-1-methylpyrrolidin-2-yl)-1*H*-pyrrolo[3,2-c]pyridin-6-yl)-1*H*-indazole-6-carboxamide;
1-(2-((6-(2-(2,6-dioxopiperidin-3-yl)-1-oxoisoindolin-4-yl)hex-5-yn-1-yl)oxy)ethyl)-3-methyl-*N*-(2-((R)-1-methylpyrrolidin-2-yl)-1*H*-pyrrolo[3,2-c]pyridin-6-yl)-1*H*-indazole-6-carboxamide;
1-(9-(3-(((S)-2,6-dioxopiperidin-3-yl)carbamoyl)-2-fluorophenyl)non-8-yn-1-yl)-3-methyl-*N*-(2-((R)-1-methylpyrrolidin-2-yl)-1*H*-pyrrolo[3,2-c]pyridin-6-yl)-1*H*-indazole-6-carboxamide;
1-(9-(2-(2,6-dioxopiperidin-3-yl)-7-fluoro-1-oxoisoindolin-4-yl)non-8-yn-1-yl)-3-methyl-*N*-(2-((*R*)-1-methylpyrrolidin-2-yl)-1*H*-pyrrolo[3,2-c]pyridin-6-yl)-1*H*-indazole-6-carboxamide;
*N*-(3-chloro-2-((R)-1-methylpyrrolidin-2-yl)-1*H*-pyrrolo[3,2-c]pyridin-6-yl)-1-(9-(2-(2,6-dioxopiperidin-3-yl)-1-oxoisoindolin-4-yl)non-8-yn-1-yl)-3-methyl-1*H*-indazole-6-carboxamide;
1-(10-(2-(2,6-dioxopiperidin-3-yl)-1-oxoisoindolin-4-yl) dec-9-yn-1-yl)-3-methyl-*N*-(2-((R)-1-methylpyrrolidin-2-yl)-1*H*-pyrrolo[3,2-c]pyridin-6-yl)-1*H*-indazole-6-carboxamide;
1-(9-(2-(2, 6-dioxopiperidin-3-yl)-1-oxoisoindolin-4-yl) non-8-yn-1-yl)-*N*-(2-((*R*)-1-methylpyrrolidin-2-yl)-1*H*-pyrrol[3, 2-c]pyridin-6-yl)-1*H*-indazole-6-carboxamide;
1-(9-(2-(2,6-dioxopiperidin-3-yl)-1-oxoisoindolin-4-yl)non-8-yn-1-yl)-3-methyl-*N*-(2-((*R*)-5-methyl-5-azaspiro[2.4]heptan-6-yl)-1*H*-pyrrolo[3,2-c]pyridin-6-yl)-1*H*-indazole-6-carboxamide;
1-(9-(2-(2,6-dioxopiperidin-3-yl)-1-oxoisoindolin-4-yl)non-8-yn-1-yl)-3-methyl-*N*-(3-methyl-2-((*R*)-1-methylpyrrolidin-2-yl)-1*H*-pyrrolo[3,2-c]pyridin-6-yl)-1*H*-indazole-6-carboxamide;
1-(9-(2-(2,6-dioxopiperidin-3-yl)-1-oxoisoindolin-4-yl)non-8-yn-1-yl)-5-fluoro-3-methyl-*N*-(2-((*R*)-1-methylpyrrolidin-2-yl)-1*H*-pyrrolo[3,2-*c*]pyridin-6-yl)-1*H*-indazole-6-carboxamide;
3-cyclopropyl-1-(9-(2-(2, 6-dioxopiperidin-3-yl)-1-oxoisoindolin-4-yl) non-8-yn-1-yl)-*N*-(2-((*R*)-1-methylpyrrolidin-2-yl)-1*H*-pyrrolo [3, 2-c] pyridin-6-yl)-1*H*-indazole-6-carboxamide;
1-(9-(2-(2,6-dioxopiperidin-3-yl)-6-fluoro-1-oxoisoindolin-5-yl)non-8-yn-1-yl)-3-methyl-*N*-(2-((*R*)-1-methylpyrrolidin-2-yl)-1*H*-pyrrolo[3,2-c]pyridin-6-yl)-1*H*-indazole-6-carboxamide;
1-(9-(3-(2, 6-dioxopiperidin-3-yl)-2-fluorophenyl) non-8-yn-1-yl)-3-methyl-*N*-(2-((R)-1-methylpyrrolidin-2-yl)-1*H*-pyrrolo [3, 2-*c*] pyridin-6-yl)-1*H*-indazole-6-carboxamide;
1-(9-(6-(2, 6-dioxopiperidin-3-yl) pyridin-2-yl) non-8-yn-1-yl)-3-methyl-*N*-(2-((*R*)-1-methylpyrrolidin-2-yl)-1*H*-pyrrolo [3, 2-c] pyridin-6-yl)-1*H*-indazole-6-carboxamide;
(*R*)-1-(9-(3-(2, 4-dioxotetrahydropyrimidin-1(2*H*)-yl)-2-fluorophenyl) non-8-yn-1-yl)-3-methyl-*N*-(2-(1-methylpyrrolidin-2-yl)-1H-pyrrolo [3, 2-c] pyridin-6-yl)-1*H*-indazole-6-carboxamide;
(*R*)-1-(9-(6-(2,4-dioxotetrahydropyrimidin-1(2*H*)-yl)pyridin-2-yl)non-8-yn-1-yl)-3-methyl-*N*-(2-(1-methylpyrrolidin-2-yl)-1*H*-pyrrolo[3,2-c]pyridin-6-yl)-1*H*-indazole-6-carboxamide;
(*R*)-1-(9-(3-(2,4-dioxotetrahydropyrimidin-1(2*H*)-yl)-5-fluoro-1-methyl-1*H*-indazol-6-yl)non-8-yn-1-yl)-3-methyl-*N*-(2-(1-methylpyrrolidin-2-yl)-1*H*-pyrrolo[3,2-c]pyridin-6-yl)-1*H*-indazole-6-carboxamide;
*N*-(3-cyano-2-((*R*)-1-methylpyrrolidin-2-yl)-1*H*-pyrrolo[3,2-c]pyridin-6-yl)-1-(9-(2-(2,6-dioxopiperidin-3-yl)-1-oxoisoindolin-4-yl)non-8-yn-1-yl)-3-methyl-1*H*-indazole-6-carboxamide;
*N*-(3-chloro-2-((*R*)-1-methylpyrrolidin-2-yl)-1*H*-pyrrolo[3,2-*c*]pyridin-6-yl)-1-(9-(2-(2,6-dioxopiperidin-3-yl)-7-fluoro-1-oxoisoindolin-4-yl)non-8-yn-1-yl)-3-methyl-1*H*-indazole-6-carboxamide;
1-(1-(3-(2-(2,6-dioxopiperidin-3-yl)-1-oxoisoindolin-4-yl)prop-2-yn-1-yl)piperidin-4-yl)-3-methyl-*N*-(2-((*R*)-1-methylpyrrolidin-2-yl)-1*H*-pyrrolo[3,2-*c*]pyridin-6-yl)-1*H*-indazole-6-carboxamide;
1-(1-((1-(2-(2,6-dioxopiperidin-3-yl)-6-fluoro-1,3-dioxoisoindolin-5-yl)piperidin-4-yl)methyl)piperidin-4-yl)-3-methyl-*N*-(2-((*R*)-1-methylpyrrolidin-2-yl)-1*H*-pyrrolo[3,2-c]pyridin-6-yl)-1*H*-indazole-6-carboxamide;
1-(1-((1-(2-(2,6-dioxopiperidin-3-yl)-1,3-dioxoisoindolin-5-yl)piperidin-4-yl)methyl)piperidin-4-yl)-3-methyl-*N*-

(2-((*R*)-1-methylpyrrolidin-2-yl)-1*H*-pyrrolo[3,2-*c*]pyridin-6-yl)-1*H*-indazole-6-carboxamide; 1-(1-((1-(2-(2,6-dioxopiperidin-3-yl)-1,3-dioxoisoindolin-4-yl)piperidin-4-yl)methyl)piperidin-4-yl)-3-methyl-*N*-(2-((R)-1-methylpyrrolidin-2-yl)-1H-pyrrolo[3,2-*c*]pyridin-6-yl)-1*H*-indazole-6-carboxamide; 1-(3-(2-(2,6-dioxopiperidin-3-yl)-1-oxoisoindolin-4-yl)prop-2-yn-1-yl)-3-methyl-*N*-(2-((*R*)-1-methylpyrrolidin-2-yl)-1*H*-pyrrolo[3,2-*c*]pyridin-6-yl)-1*H*-indazole-6-carboxamide; 1-(6-(1-(6-(((S)-2,6-dioxopiperidin-3-yl)carbamoyl)pyridin-2-yl)piperidin-4-yl)hexyl)-3-methyl-N-(2-((R)-1-methylpyrrolidin-2-yl)-1H-pyrrolo[3,2-c]pyridin-6-yl)-1H-indazole-6-carboxamide; and 1-(4-(5-(2-(2,6-dioxopiperidin-3-yl)-1-oxoisoindolin-4-yl) pent-4-yn-1-yl) cyclohexyl)-3-methyl-N-(2-((R)-1-methylpyrrolidin-2-yl)-1H-pyrrolo[3,2-c]pyridin-6-yl)-1H-indazole-6-carboxamide.

**9.** A compound according to any one of claims 1 to 7, wherein M is of formula (IV):

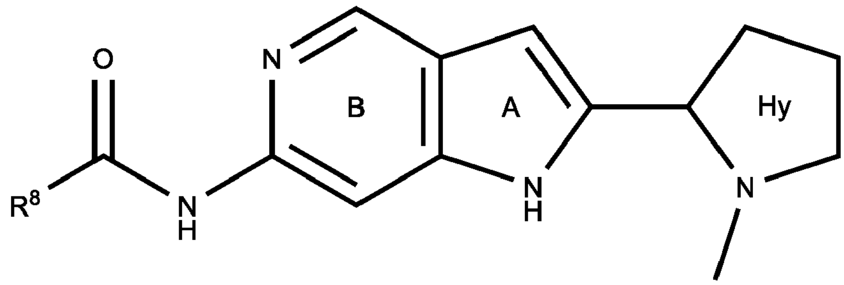

(IV)

wherein $R^8$ is an 8- to 10-membered heteroaryl ring, the group $R^8$ being unsubstituted or substituted by one, two or three substituents independently selected from halo, $C_{1-4}$ alkyl and $R^{10}$;
$R^{10}$ is a 3-membered cycloalkyl ring;
wherein M is bonded to LINK via a C or N atom within group $R^8$ such that a hydrogen atom on the C or N atom within group $R^8$ is replaced with a bond to LINK.

**10.** A compound which is a bicyclic compound of formula (I') or a pharmaceutically acceptable salt thereof:

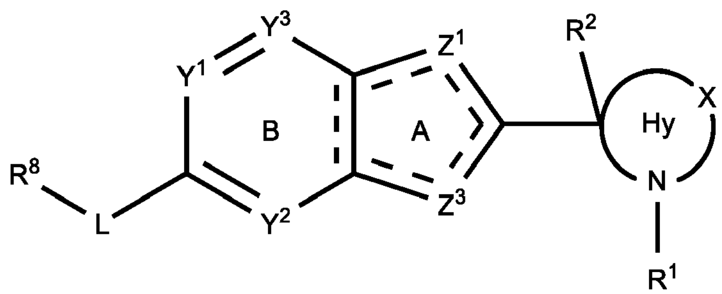

(I')

wherein:

one of $Z^1$ and $Z^3$ is -N(H)- and the other is N or -C($R^4$)-, $Y^1$ is N, $Y^2$ is N or -C($R^6$)-, and $Y^3$ is N or -C($R^5$)-;
Hy is a 4- to 7-membered heterocyclic ring containing X and at least one N atom,
wherein: ring Hy is linked to ring A via a C atom within ring Hy, said C atom also being linked to $R^2$; a N atom within ring Hy is substituted by $R^1$; X is a bond, -N($R^{11}$)-, O, S, -S(O)$_2$-, -S(O)(N$R^{11}$)-, or -C($R^{11}$)$_2$-; and the rest of ring Hy is unsubstituted or substituted by one or two $R^3$;
L is -C(O)N(H)-, wherein the C atom of L is bonded to $R^8$, and the N atom of L is bonded to ring B;
$R^1$ is H, $C_{1-4}$ cycloalkyl, or $C_{1-4}$ alkyl which is itself unsubstituted or substituted with one $C_{1-4}$ alkoxy or one, two or three halo;
$R^2$ is H or methyl;
each $R^3$ is independently selected from $C_{1-4}$ alkyl, $C_{1-4}$ alkoxy, phenyl, a 5- to 6-membered heteroaryl ring and halo, or (i) two $R^3$ linked to adjacent C atoms in ring Hy form, together with the C atoms to which they are attached,

a $C_{5-6}$ cycloalkyl ring, or (ii) two $R^3$ linked to the same C atom in ring Hy form, together with the C atom to which they are attached, a $C_{3-6}$ cycloalkyl ring or a $C_{3-6}$ heterocycloalkyl ring;
$R^4$ and $R^6$ are independently selected from H, halo, CN, $C_{1-4}$ alkoxy, and $C_{1-4}$ alkyl which is itself unsubstituted or substituted by one, two or three halo;
$R^5$ is selected from H, halo, $C_{1-4}$ alkoxy, $C_{3-5}$ cycloalkyl and $C_{1-4}$ alkyl which is itself unsubstituted or substituted by one, two or three halo;
at least one of $R^4$, $R^5$ and $R^6$ is not H;
$R^8$ is an 8- to 10-membered heteroaryl ring, the group $R^8$ being unsubstituted or
substituted by one, two or three substituents independently selected from halo, $C_{1-4}$ alkoxy, $R^{10}$, -($C_{1-4}$ alkylene)-$R^{10}$, =O, -CN, and $C_{1-4}$ alkyl which is itself unsubstituted or
substituted by one or two $R^9$;
each $R^9$ is independently selected from halo and $C_{1-4}$ alkoxy;
$R^{10}$ is a group selected from a 5- to 6-membered heteroaryl ring, a 3- to 6-membered cycloalkyl ring, a 4- to 6-membered heterocyclyl ring, and a phenyl ring, the group $R^{10}$ being unsubstituted or substituted by one or two substituents independently selected from $C_{1-4}$ alkyl, $C_{1-4}$ alkoxy, and halo;
each $R^{11}$ is independently selected from H, $C_{1-4}$ alkyl, and $C_{1-4}$ cycloalkyl.

**11.** A pharmaceutical composition which comprises a compound as defined in any one of claims 1 to 10 and a pharmaceutically acceptable carrier or diluent.

**12.** A compound as defined in any one of claims 1 to 10 for use in the treatment of the human or animal body by therapy.

**13.** A compound as defined in any one of claims 1 to 10 for use in the treatment of cancer.

**14.** The compound for use according to claim 13, wherein the cancer is a transcriptionally addicted cancer, such as breast cancer, acute leukemia, prostate cancer, bladder cancer, cholangiocarcinoma, colon adenocarcinoma, esophageal carcinoma, head and neck squamous cell carcinoma, kidney chromophobe, kidney renal clear cell carcinoma, kidney renal papillary cell carcinoma, liver hepatocellular carcinoma, lung adenocarcinoma, pheochromocytoma and paraganglioma, rectum adenocarcinoma, stomach adenocarcinoma, uterine corpus endometrial carcinoma, cervical squamous cell carcinoma and endocervical adenocarcinoma, lung squamous cell carcinoma, neuroblastoma, midline glioma or sarcoma, preferably neuroblastoma or midline glioma.

**15.** The compound for use according to claim 13, wherein the cancer is acute myeloid leukaemia (AML), myelodysplastic syndrome (MDS), myeloproliferative neoplasm (MPN) or acute lymphoblastic leukaemia (ALL), for instance wherein the cancer is mixed-lineage leukaemia (MLL), rearranged acute leukemia (AML and ALL) or NPM1 mutant leukemia.

Europäisches Patentamt
European Patent Office
Office européen des brevets

# EUROPEAN SEARCH REPORT

Application Number
EP 24 18 3691

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>A | WO 2022/240830 A1 (BRIDGE MEDICINES [US]) 17 November 2022 (2022-11-17)<br>* claims 18-19, 25-33; examples 1,2,3-9, 28-72, 77-98; tables 1,2 *<br>----- | 10-15<br>1-9 | INV.<br>C07D471/04<br>A61K47/55<br>A61P35/00<br>A61K31/4745 |
| A | WO 2024/054749 A2 (BRIDGE MEDICINES [US]) 14 March 2024 (2024-03-14)<br>* claims; examples; tables 1-3 *<br>----- | 1-15 | |
| Y | WO 2023/183412 A1 (BAYLOR COLLEGE MEDICINE [US]) 28 September 2023 (2023-09-28)<br>* page 23, line 25 - page 27, line 15; claims; figures; examples *<br>----- | 1-15 | |
| Y | TROUP ROBERT I. ET AL: "Current strategies for the design of PROTAC linkers: a critical review", EXPLORATION OF TARGETED ANTI-TUMOR THERAPY, vol. 1, no. 5, 30 October 2020 (2020-10-30), XP055828975, DOI: 10.37349/etat.2020.00018 Retrieved from the Internet: URL:https://www.explorationpub.com/uploads/Article/A100218/100218.pdf><br>* the whole document *<br>-----<br>-/-- | 1-15 | TECHNICAL FIELDS SEARCHED (IPC)<br>C07D |

The present search report has been drawn up for all claims

| Place of search | Date of completion of the search | Examiner |
|---|---|---|
| Munich | 9 December 2024 | Gavriliu, Daniela |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

3

page 1 of 2

Europäisches Patentamt
European Patent Office
Office européen des brevets

# EUROPEAN SEARCH REPORT

Application Number
EP 24 18 3691

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | KRASAVIN MIKHAIL ET AL: "Synthesis of novel glutarimide ligands for the E3 ligase substrate receptor Cereblon (CRBN): Investigation of their binding mode and antiproliferative effects against myeloma cell lines", EUROPEAN JOURNAL OF MEDICINAL CHEMISTRY, vol. 246, 1 December 2022 (2022-12-01), page 114990, XP093208891, AMSTERDAM, NL ISSN: 0223-5234, DOI: 10.1016/j.ejmech.2022.114990 * the whole document * ----- | 1-15 | |

TECHNICAL FIELDS SEARCHED (IPC)

The present search report has been drawn up for all claims

3

| Place of search | Date of completion of the search | Examiner |
|---|---|---|
| Munich | 9 December 2024 | Gavriliu, Daniela |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

# ANNEX TO THE EUROPEAN SEARCH REPORT ON EUROPEAN PATENT APPLICATION NO.

EP 24 18 3691

This annex lists the patent family members relating to the patent documents cited in the above-mentioned European search report. The members are as contained in the European Patent Office EDP file on
The European Patent Office is in no way liable for these particulars which are merely given for the purpose of information.

09-12-2024

| Patent document cited in search report | Publication date | Patent family member(s) | Publication date |
|---|---|---|---|
| WO 2022240830 A1 | 17-11-2022 | AU 2022272294 A1 | 30-11-2023 |
| | | BR 112023023578 A2 | 12-03-2024 |
| | | CA 3218317 A1 | 17-11-2022 |
| | | CN 117858877 A | 09-04-2024 |
| | | CO 2023017195 A2 | 18-03-2024 |
| | | CR 20230578 A | 17-06-2024 |
| | | EP 4337662 A1 | 20-03-2024 |
| | | IL 308430 A | 01-01-2024 |
| | | JP 2024518824 A | 07-05-2024 |
| | | KR 20240047955 A | 12-04-2024 |
| | | WO 2022240830 A1 | 17-11-2022 |
| WO 2024054749 A2 | 14-03-2024 | NONE | |
| WO 2023183412 A1 | 28-09-2023 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2020041331 A
- WO 2019140003 A
- WO 2013106643 A
- US 20160045607 A
- WO 2014187777 A
- US 20140356322 A
- US 9249 A
- US 153 A
- US 20160058872 A
- US 20150291562 A


### Non-patent literature cited in the description


- *Cell*, 2013, vol. 153, 17
- *Cell*, 2017, vol. 168, 629
- *Cell Mol Life Sci*, 2018, vol. 75, 3931
- *Nat Rev Mol Cell Biol*, 2012, vol. 13, 543
- *Nature*, 2020, vol. 577, 121
- *Nature*, 2017, vol. 543, 270
- *Front. Pediatr.*, vol. 5, 4
- *New Engl J Med*, 2016, vol. 374, 2209
- *Cancer Disc*, 2022, vol. 12, 2684
- *Cell Rep.*, 16 February 2021, vol. 34 (7), 108749
- *ACS Cent Sci*, 2021, vol. 7, 815
- *Angew. Chem. Int. Ed.*, 2018, vol. 57, 16302
- **GREG T. HERMANSON**. Bioconjugate Techniques. Academic Press Inc., 1996
- **GU et al.** *BioEssays*, 2018, vol. 40, 1700247
- **SUN et al.** *Signal Transduction and Targeted Therapy*, 2019, vol. 4, 64
- **WINTER et al.** *Science*, 19 June 2015, 1376
- *Cell Rep*, 2021, vol. 16, 1087
- **EISENHAUER et al.** *European Journal of Cancer*, 2009, vol. 45, 228-247
- *Blood*, 2007, vol. 109, 1815
- *Leukemia Res*, 2018, vol. 68, 32
- *CHEMICAL ABSTRACTS*, 7598-35-8
- *CHEMICAL ABSTRACTS*, 316141-37-4
- *CHEMICAL ABSTRACTS*, 73365-02-3
- *CHEMICAL ABSTRACTS*, 90965-06-3
- *CHEMICAL ABSTRACTS*, 10160-28-8
- *CHEMICAL ABSTRACTS*, 191732-72-6
- *CHEMICAL ABSTRACTS*, 625446-22-2
- *CHEMICAL ABSTRACTS*, 835616-60-9
- *CHEMICAL ABSTRACTS*, 1092351-82-0
- *CHEMICAL ABSTRACTS*, 1211568-27-2
- *CHEMICAL ABSTRACTS*, 1496997-41-1
- *CHEMICAL ABSTRACTS*, 109384-19-2
- *CHEMICAL ABSTRACTS*, 83948-53-2
- *CHEMICAL ABSTRACTS*, 50816-19-8
- *CHEMICAL ABSTRACTS*, 64567-60-8
- *CHEMICAL ABSTRACTS*, 53222-92-7
- *CHEMICAL ABSTRACTS*, 53963-10-3
- *CHEMICAL ABSTRACTS*, 913297-44-6
- *CHEMICAL ABSTRACTS*, 62595-74-8
- *CHEMICAL ABSTRACTS*, 63478-76-2
- *CHEMICAL ABSTRACTS*, 50816-20-1
- *CHEMICAL ABSTRACTS*, 5054-59-1
- *CHEMICAL ABSTRACTS*, 835616-61-0
- *CHEMICAL ABSTRACTS*, 2093387-36-9
- *CHEMICAL ABSTRACTS*, 1814936-54-3
- *CHEMICAL ABSTRACTS*, 57260-71-6
- *CHEMICAL ABSTRACTS*, 110-52-1
- *CHEMICAL ABSTRACTS*, 164365-88-2
- *CHEMICAL ABSTRACTS*, 142356-33-0
- *CHEMICAL ABSTRACTS*, 54512-75-3
- *CHEMICAL ABSTRACTS*, 111-44-4
- *CHEMICAL ABSTRACTS*, 1076199-21-7
- *CHEMICAL ABSTRACTS*, 164332-88-1
- *CHEMICAL ABSTRACTS*, 164149-27-3
- *CHEMICAL ABSTRACTS*, 369-25-5
- *CHEMICAL ABSTRACTS*, 24666-56-6
- *CHEMICAL ABSTRACTS*, 137076-22-3
- *CHEMICAL ABSTRACTS*, 42516-28-9
- *CHEMICAL ABSTRACTS*, 504-63-2
- *CHEMICAL ABSTRACTS*, 106-96-7
- *CHEMICAL ABSTRACTS*, 5390-04-5
- *CHEMICAL ABSTRACTS*, 447464-03-1

- *CHEMICAL ABSTRACTS*, 133232-56-1
- *CHEMICAL ABSTRACTS*, 473416-12-5
- *CHEMICAL ABSTRACTS*, 142356-35-2
- *CHEMICAL ABSTRACTS*, 61700-61-6
- *CHEMICAL ABSTRACTS*, 87462-64-4
- *CHEMICAL ABSTRACTS*, 305790-48-1
- *CHEMICAL ABSTRACTS*, 845306-08-3
- *CHEMICAL ABSTRACTS*, 25181-50-4
- *CHEMICAL ABSTRACTS*, 910044-07-4
- *CHEMICAL ABSTRACTS*, 30766-03-1
- *CHEMICAL ABSTRACTS*, 871-91-0
- *CHEMICAL ABSTRACTS*, 928-90-5
- *CHEMICAL ABSTRACTS*, 111-29-5
- *CHEMICAL ABSTRACTS*, 252948-15-5
- *CHEMICAL ABSTRACTS*, 2438239-34-8
- *CHEMICAL ABSTRACTS*, 13019-22-2
- *CHEMICAL ABSTRACTS*, 906000-44-0
- *CHEMICAL ABSTRACTS*, 1129634-44-1
- *CHEMICAL ABSTRACTS*, 123942-11-0
- *CHEMICAL ABSTRACTS*, 170487-40-8
- *CHEMICAL ABSTRACTS*, 411235-57-9
- *CHEMICAL ABSTRACTS*, 1352889-89-4
- *CHEMICAL ABSTRACTS*, 874285-03-7
- *CHEMICAL ABSTRACTS*, 79-06-1
- *CHEMICAL ABSTRACTS*, 5315-25-3
- *CHEMICAL ABSTRACTS*, 105-58-8
- *CHEMICAL ABSTRACTS*, 58534-95-5
- *CHEMICAL ABSTRACTS*, 79-10-7
- *CHEMICAL ABSTRACTS*, 504-07-4
- *CHEMICAL ABSTRACTS*, 824-94-2
- *CHEMICAL ABSTRACTS*, 626-05-1
- *CHEMICAL ABSTRACTS*, 133541-45-4
- *CHEMICAL ABSTRACTS*, 158407-04-6
- *CHEMICAL ABSTRACTS*, 19524-06-2
- *CHEMICAL ABSTRACTS*, 455-71-0
- *CHEMICAL ABSTRACTS*, 7021-11-6
- *CHEMICAL ABSTRACTS*, 1059549-72-2
- *CHEMICAL ABSTRACTS*, 1176754-31-6
- *CHEMICAL ABSTRACTS*, 1961266-44-3
- *CHEMICAL ABSTRACTS*, 79055-59-7